# EUROPEAN PATENT APPLICATION

(11) **EP 1 770 085 A1**
(43) Date of publication of application: **04.04.2007**
(21) Application number: 05766156.3
(22) Date of filing: 19.07.2005
(51) Int. Cl.: C07C 269/04, C07C 269/06, C07C 271/22, C07C 271/34, C07C 271/54, C07C 311/16, C07C 317/44, C07C 323/42, C07C 333/04, C07D 207/08, C07D 207/34, C07C 209/42, C07D 213/68, C07D 213/81, C07D 213/82, C07D 215/48, C07D 215/54, C07D 217/26, C07D 231/38, C07D 233/88, C07D 235/24

(54) **DIAMINE DERIVATIVE, PROCESS FOR PRODUCING THE SAME AND FUNGICIDE CONTAINING THE DERIVATIVE AS ACTIVE INGREDIENT**

(30) Priority: 21.07.2004 JP 2004213208; 27.01.2005 JP 2005019032
(71) Applicant: Mitsui Chemicals, Inc., Minato-ku Tokyo 105-7117 (JP)
(72) Inventor: KAKIMOTO, Takeshi, c/o Mitsui Chemicals, Inc., Omuta-shi, Fukuoka 836-8610 (JP); KAMEKAWA, Hisato, c/o Mitsui Chemicals, Inc., Omuta-shi, Fukuoka 836-8610 (JP); CHIBA, Yutaka, c/o Mitsui Chemicals, Inc., Mobara-shi, Chiba 297-0017 (JP); KOHNO, Toshiyuki, c/o Mitsui Chemicals, Inc., Mobara-shi, Chiba 297-0017 (JP); KOBAYASHI, Yumi, c/o Mitsui Chemicals, Inc., Mobara-shi, Chiba 297-0017 (JP); TOMURA, Naofumi, c/o Mitsui Chemicals, Inc., Mobara-shi, Chiba 297-0017 (JP); ARAKI, Natsuko, c/o Mitsui Chemicals, Inc., Mobara-shi, Chiba 297-0017 (JP); YOSHIDA, Masako, c/o Mitsui Chemicals, Inc., Sodegaura-shi, Chiba 299-0265 (JP); AOKI, Yoji, c/o Mitsui Chemicals, Inc., Mobara-shi, Chiba 297-0017 (JP); BANBA, SHINICHI, c/o Mitsui Chemicals, Inc., Mobara-shi, Chiba 297-0017 (JP)
(74) Representative: Stuart, Ian Alexander
(86) International application number: PCT/JP2005/013233
(87) International publication number: WO 2006/009134

(57) **Abstract**

It is an object of the invention to provide a novel fungicide which exhibits a wide controlling spectrum against pathogens of various crops, and solves the toleration problem.

The diamine derivative represented by the formula (1) and a process for preparation of the same, fungicides comprising the same as an active ingredient are disclosed: 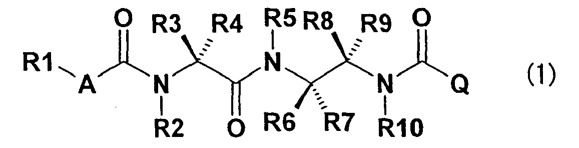 [wherein
R1 is substituents such as an alkyl group having 1 to 6 carbon atoms and the like, R2 and R5 are each independently substituents such as hydrogen atom, an alkyl group having 1 to 6 carbon atoms and the like, R3 and R4 are each independently substituents such as hydrogen atom, an alkyl group having 1 to 6 carbon atoms and the like, or R3 and R4 may be bonded to each other to form a hydrocarbon ring having 3 to 6 carbon atoms, R6, R7, R8 and R9 are each independently substituents such as hydrogen atom, an alkyl group having 1 to 6 carbon atoms and the like, R10 is a substituent such as hydrogen atom, an alkyl group having 1 to 6 carbon atoms and the like, A is an oxygen atom or a sulfur atom, and Q is an aryl group or a heterocycle].

## Description

### Technical Field

The present invention relates to a novel diamine derivative, a process of preparation thereof, and a fungicide comprising the diamine derivative as an active ingredient.

### Background Art

Disease control has played an important role in the cultivation of crops, and various fungicides have been developed and used. However, said fungicides are not always satisfactory in view of the fungicidal activity or the activity against the disease in useful crops. It is recently known that the resistant phytopathogens has appeared due to the frequent use of agricultural and horticultural fungicides, and certain existing agents do not show sufficient activity against such bacteria. In addition, it has been desired to develop a novel fungicide which is safe from the environmental problems and can control harmful microorganisms in a low concentration of said agents. It has been reported that diamine derivatives which differ from the compound of the invention is described as being useful as controlling agents against harmful organisms in WO03008372. The diamine derivative as described in WO03008372 exhibits a controlling activity against only rice blast disease, whereas the diamine derivatives of the present invention have a wide spectrum of disease control, and have different chemical structures from the compounds as described in the specification of the above-mentioned publication.

### Disclosure of the Invention

It is an object of the present invention to provide a novel agricultural and horticultural fungicide which exhibits a wide spectrum of disease control against pathogens of various crops, and also solves the resistant phytopathogens problems which have been gradually intensified.

The inventors synthesized various kinds of diamine derivatives, and examined their physiological activities. As a result, they found that the compound of the invention has a wide spectrum of disease control, and possess a very remarkable fungicidal activity, and also do not do harm to useful crops at all, and thus have completed the invention.

The invention relates to the followings:
[1]. A diamine derivative represented by the formula (1) : 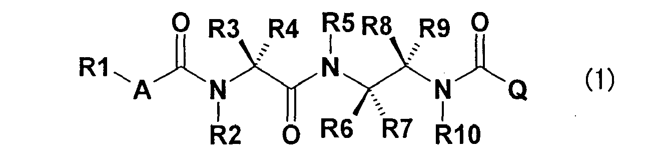 [wherein
   R1 is an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, an alkynyl group having 2 to 6 carbon atoms, an aryl group, a heterocycle, an arylalkyl group having 1 to 6 carbon atoms on alkyl moieties, or a heteroarylalkyl group having 1 to 6 carbon atoms on alkyl moieties;
   R2 and R5 are each independently a hydrogen atom, an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, an alkynyl group having 2 to 6 carbon atoms, an acyl group, an aryl group, a heterocycle, an arylalkyl group having 1 to 6 carbon atoms on alkyl moieties, or a heteroarylalkyl group having 1 to 6 carbon atoms on alkyl moieties;
   R3 and R4 are each independently a hydrogen atom, an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, an alkynyl group having 2 to 6 carbon atoms, an aryl group, a heterocycle, an arylalkyl group having 1 to 6 carbon atoms on alkyl moieties, or a heteroarylalkyl group having 1 to 6 carbon atoms on alkyl moieties, or R3 and R4 may be bonded to each other to form a hydrocarbon ring having 3 to 6 carbon atoms;
   R6, R7, R8 and R9 are each independently a hydrogen atom, an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, an alkynyl group having 2 to 6 carbon atoms, provided that at least one substituent represents an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, an alkynyl group having 2 to 6 carbon atoms;
   R10 is a hydrogen atom, an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, an alkynyl group having 2 to 6 carbon atoms, an acyl group;
   A is an oxygen atom or a sulfur atom; and
   Q is an aryl group or a heterocycle].
[2]. A fungicide comprising the diamine derivative as described in the above [1] as an active ingredient.
[3]. A process for preparing the diamine derivative as described in [1], comprising reacting a compound represented by the formula (2) 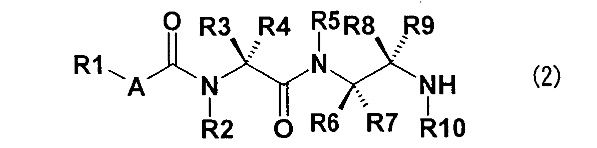 [wherein R1, R2, R3, R4, R5, R6, R7, R8, R9, R10 and A have the same meanings as in [1]] with a compound represented by the formula (3) 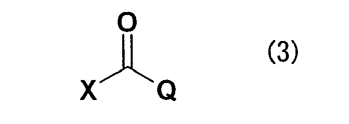 [wherein Q has the same meaning as in [1], and X is a leaving group].
[4]. A process for preparing the diamine derivative as described in [1], comprising reacting the compound represented by the formula (2) with a compound represented by the formula (4) 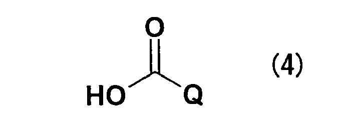 [wherein Q has the same meaning as in [1]].
[5]. A process for preparing the diamine derivative as described in [1], comprising reacting a compound represented by the formula (5) 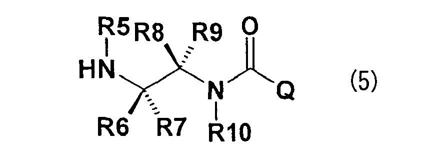 [wherein R5, R6, R7, R8, R9, R10 and Q have the same meanings as in [1]] with a compound represented by the formula (6) 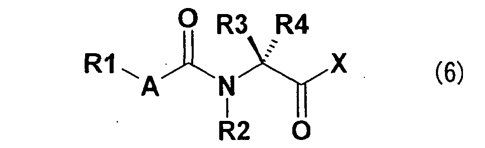 [wherein R1, R2, R3, R4 and A have the same meanings as in [1], and X is a leaving group].
[6]. A process for preparing the diamine derivative as described in [1], comprising reacting the compound represented by the formula (5) with a compound represented by the formula (7) 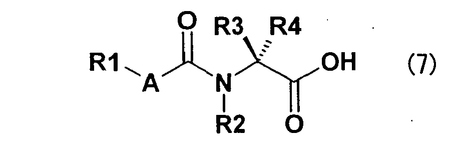 [wherein R1, R2, R3, R4 and A have the same meanings as in [1]].
[7]. A process for preparing the diamine derivative as described in [1], comprising reacting a compound represented by the formula (8) 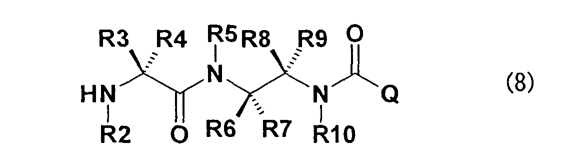 [wherein R2, R3, R4, R5, R6, R7, R8, R9, R10 and Q have the same meanings as in [1]] with a compound represented by the formula (9) 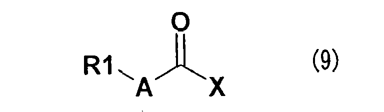 [wherein R1 and A have the same meanings as in [1], and X is a leaving group].

### Best Mode for Carrying Out the Invention

The present invention is illustrated in detail as follows.

For the diamine derivative as represented by the formula (1) and the process for the preparation thereof, examples of representative substituents of said derivatives may be mentioned as follows, but not limited thereto. The alkyl group having 1 to 6 carbon atoms includes, for example, a methyl group, an ethyl group, a propyl group, a butyl group, a pentyl group, a hexyl group and the like, each of which may be substituted. The cycloalkyl group having 3 to 6 carbon atoms includes, for example, a cyclopropyl group, a cyclobutyl group, a cyclopentyl group, a cyclohexyl group and the like, each of which may be substituted. The alkenyl group having 2 to 6 carbon atoms includes, for example, vinyl group, a propenyl group, a butenyl group, a pentenyl group, a hexenyl group and the like, each of which may be substituted. The cycloalkenyl group having 3 to 6 carbon atoms includes, for example, a cyclopropenyl group, a cyclobutenyl group, a cyclopentenyl group, a cyclohexenyl group and the like, each of which may be substituted. The alkynyl group having 2 to 6 carbon atoms includes, for example, an ethynyl group, a propynyl group, a butynyl group, a pentynyl group, a hexynyl group and the like, each of which may be substituted. The aryl group includes, for example, a phenyl group, a naphthyl group and the like, each of which may be substituted. The heterocycle represets a heterocycle having 1 to 15 carbon atoms and at least one selected from a nitrogen atom, an oxygen atom and a sulfur atom, and includes, for example, furan, thiophene, oxazole, pyrrole, 1H-pyrazole, 3H-pyrazole, imidazole, thiazole, oxazole, isoxazole, isothiazole, [1,2,3]oxadiazole, [1,2,4]oxadiazole, [1,3,4] oxadiazole, furazane, [1,2,3]thiadiazole, [1,2,4]thiadiazole, [1,2,5] thiadiazole, [1,3,4]thiadiazole, 1H-[1,2,3]triazole, 2H-[1,2,3]triazole, 1H-[1,2,4]triazole, 2H-[1,2,4]triazole, 1H-tetrazole, 5H-tetrazole, 2,3-dihydro-1H-pyrrole, 2,5-dihydro-1H-pyrrole, 3,4-dihydro-2H-pyrrole, pyrrolidine, 2,3-dihydrofuran, 2,5-dihydrofuran, tetrahydrofuran, 2,3-dihydrothiophene, 2,5-dihydrothiophene, tetrahydrothiophen, pyrazolidine, pyridine, pyrane, thiopyrane, pyridazine, pyrimidine, pyrazine, [1,2,3]triazine, [1,2,4]triazine, [1,3,5]triazine, 2H-[1,2,3]oxadiazine, 2H-[1,2,4]oxadiazine, 2H-[1,2,5]oxadiazine, 2H-[1,2,6]oxadiazine, 4H-[1,2,3]oxadiazine, 4H-[1,2,4]oxadiazine, 4H-[1,2,5]oxadiazine, 4H-[1,2,6]oxadiazine, 4H-[1,3,4]oxadiazine, 4H-[1,3,5]oxadiazine, 2H-[1,2,3]thiadiazine, 2H-[1,2,4]thiadiazine, 2H-[1,2,5]thiadiazine, 2H-[1,2,6]thiadiazine, 4H-[1,2,3]thiadiazine, 4H-[1,2,4]thiadiazine, 4H-[1,2,5]thiadiazine, 4H-[1,2,6]thiadiazine, 4H-[1,3,4]thiadiazine, 4H-[1,3,5]thiadiazine, 2,3-dihydropyridine, 3,4-dihydropyridine, 1,3-dioxane, 1,4-dioxane, piperidine, 3,4-dihydro-2H-pyran, 3,6-dihydro-2H-pyran, tetrahydropyran, 3,4-dihydro-2H-thiopyran, 3,6-dihydro-2H-thiopyran, tetrahydrothiopyran, morpholine, piperazine, cromane, cromene, isobenzofuran, indolidine, indole, 3H-indole, dihydroindole, isoindole, 1H-indazole, 2H-indazole, purine, quinoline, isoquinoline, 4H-quinolidine, phthalazine, naphthyridine, quinoxaline, quinazoline, cinnoline, carbazole, acridine, phenazine, phenanthroline, phenothiazine, phenoxazine, indoline, isoindoline, benzofuran, dihydrobenzofuran, benzothiophene, benzo[c]thiophene, benzo[c]isothiazole, benzo[c]isoxazole, dihydrobenzothiophene, benzoxazole, benzoisoxazole, benzimidazole, benzothiazole, benzisothiazole, 1H-benzotriazole, 2H-benzotriazole, benzo[1,4]dioxane, 2,3-dihydrobenzo[1,4]dioxane, benzo[1,3]dioxol, β-carboline, benzo[1,2,5]oxadiazole, benzo[1,2,5]thiadiazole, pteridine, imidazo[1,2-a]pyridine, imidazo[1,2-a]pyridine, pyrazolo[1,5-a]pyrimidine, [1,2,4]triazo[1,5-a]pyrimidine and the like, each of which may be substituted. The acyl group includes, for example, an alkylcarbonyl group such as an acetyl group and the like, and an arylcarbonyl group such as a benzoyl group and the like, each of which may be substituted. The substituents of an alkyl group having 1 to 6 carbon atoms include, for example, an alkyl group such as a methyl group, an ethyl group, a propyl group, a butyl group and the like; a cycloalkyl group such as a cyclopropyl group, a cyclobutyl group, a cyclopentyl group, a cyclohexyl group and the like; a halogen-substituted alkyl group such as a trifluoromethyl group, a difluoromethyl group, a bromodifluoromethyl group, a trifluoroethyl group and the like; an alkoxy group such as a methoxy group, an ethoxy group, a propoxy group, a butoxy group and the like; a halogen-substituted alkoxy group such as a trifluoromethoxy group, a difluoromethoxy group, a trifluoroethoxy group and the like; an alkylthio group such as a methylthio group, an ethylthio group, a propylthio group, a butylthio group and the like; a halogen-substituted alkylthio group such as a trifluoromethylthio group, a difluoromethylthio group, a trifluoroethylthio group and the like; an alkylsulfinyl group such as a methanesulfinyl group, an ethanesulfinyl group, a propanesulfinyl group, a butanesulfinyl group and the like; a halogen-substituted alkylsulfinyl group such as a trifluormethanesulfinyl group, a difluormethanesulfinyl group, a trifluorethanesulfinyl group and the like; an alkylsulfonyl group such as a methansulfonyl group, an ethanesulfonyl group, a propanesulfonyl group, a butanesulfonyl group and the like; a halogen-substituted alkylsulfonyl group such as a trifluormethanesulfonyl group, a difluoromethanesulfonyl group, a trifluoroethanesulfonyl and the like; an alkylsulfoneamide group such as a methanesulfoneamide group, an ethanesulfoneamide, a propanesulfoneamide, butanesulfoneamide and the like; a halogen-substitued alkylsulfoneamide group such as a trifluoromethanesulfoneamide group, a difluoromethanesulfoneamide group, a trifluoroethanesulfoneamide group and the like; an aminomethyl group such as a methylamino group, a phenylaminomethyl group, an aminomethyl group and the like; an amide group such as an acetylamino group, a benzoylamino group and the like; a halogen atom such as a fluorine atom, a chlorine atom, bromine atom, an iodine atom and the like; and an acyl group such as an acetyl group, a benzoyl group and the like, provided that the case where the alkyl group having 1 to 6 carbon atoms is substituted by an aryl group or a heterocycle is excluded. The substituents of the cycloalkyl group having 3 to 6 carbon atoms, the alkenyl group having 2 to 6 carbon atoms, the cycloalkeny group having 3 to 6 carbon atoms, the alkynyl group having 2 to 6 carbon atoms, the aryl group, the heterocycle, the arylalkyl group having 1 to 6 carbon atom in the alkyl moiety, or the heteroarylalkyl group having 1 to 6 carbon atom in the alkyl moiety, include, for example, an alkyl group such as a methyl group, an ethyl group, a propyl group, a butyl group and the like; a cycloalkyl group such as a cyclopropyl group, a cyclobutyl group, a cyclopentyl group or a cyclohexyl group and the like; a halogen-substituted alkyl group such as a trifluoromethyl group, a difluoromethyl group,a bromodifluoromethyl group, a trifluoroethyl group and the like; an alkoxy group such as a methoxy group, an ethoxy group, a propoxy group, a butoxy group and the like; a halogen-substituted alkoxy group such as a trifluoromethoxy group, a difluoromethoxy group, a trifluoroethoxy group and the like; an alkylthio group such as a methylthio group, an ethylthio group, a propylthio group, a butylthio group and the like; a halogen-substituted alkylthio group such as a trifluoromethylthio group, a difluoromethylthio group, a trifluoroethylthio group and the like; an alkylsulfinyl group such as a methanesulfinyl group, ethanesulfinyl group, propanesulfinyl group or butanesulfinyl group and the like; a halogen-substituted alkylsulfinyl group such as a trifluormethanesulfinyl group, a difluormethanesulfinyl group or a trifluorethanesulfinyl group and the like; an alkylsulfonyl group such as a methansulfonyl group, an ethanesulfonyl group, a propanesulfonyl group, a butanesulfonyl group and the like; a halogen-substituted alkylsulfonyl group such as a trifluormethanesulfonyl group, a difluoromethanesulfonyl group or trifluoroethanesulfonyl and the like; an alkylsulfoneamide group such as a methanesulfoneamide group, ethanesulfoneamide, propanesulfoneamide or butanesulfoneamide and the like; a halogen-substitued alkylsulfoneamide group such as a trifluoromethanesulfoneamide group, a difluoromethanesulfoneamide group or a trifluoroethanesulfoneamide group and the like; an aryl group such as a phenyl group or a naphthal group and the like; a heterocycle such as furan, thiophene, oxazole, pyrrole, 1H-pyrazole, 3H-pyrazole, imidazole, thiazole, isoxazole, isothiazole, tetrahydrofuran, pyrazolidine, pyridine, pyrane, pyrimidine, pyridine and the like; an aminomethyl group such as a methylamino group, a phenylaminomethyl group, an aminomethyl group and the like; an amide group such as an acetylamino group, a benzoylamino group and the like; a halogen atom such as a fluorine atom, a chlorine atom, a bromine atom, an iodine atom and the like; an acyl group such as an acetyl group, a benzoyl group and the like, respectively.

X in the compounds represented by the formulae (3), (6) and (9) is a leaving group such as, for example, a halogen atom, typically such as a chlorine atom, an alkoxy group, typically such as a methoxy group and an ethoxy group, an aryloxy group, typically such as a phenoxy group, an acyloxy group, typically such as an acetyloxy group and a benzoyloxy group, an alkoxycarbonyloxy group, typically such as a methoxycarbonyloxy group, an arylcarbonyloxy group, typically such as a phenylcarbonyloxy group, N-hydroxy succinimide, 1-hydroxybenzotriazole and an imidazole group and the like, respectively.

The compound represented by the formula (1) according to the invention is a novel compound and the compound represented by the formula (1) can be prepared by the method as shown in the Reaction scheme (1): 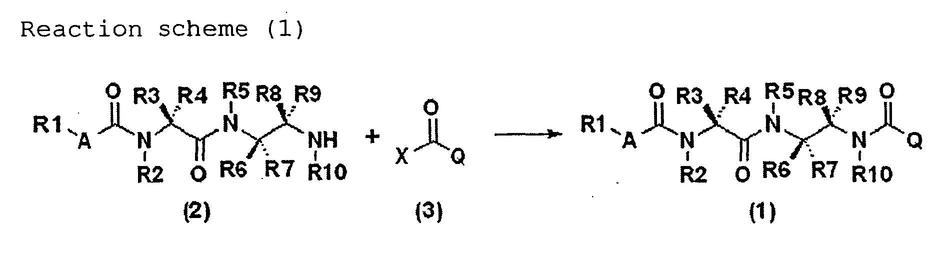 [wherein, R1, R2, R3, R4, R5, R6, R7, R8, R9, R10 and A have the same meanings as in the formula (2), Q and X have the same meanings as in the formula (3)].

In the reaction scheme (1), the diamine derivative represented by the formula (1) can be prepared by reacting the amine derivative represented by the formula (2) and a salt thereof with a known carbonyl compound represented by the formula (3) with or without a base and a metal reagent such as trialkylaluminum and the like, without a solvent or in a solvent.

Examples of bases used for the reaction as shown in the reaction scheme (1) include alkaline metal hydroxides such as sodium hydroxide, potassium hydroxide and the like; alkaline earth metal hydroxides such as magnesium hydroxide, calcium hydroxide and the like; alkaline metal hydrides such as sodium hydride, potassium hydride and the like; alkaline metal alcoholates such as sodium methoxide, sodium ethoxide and the like; alkaline metal oxides such as sodium oxide and the like; carbonates such as potassium carbonate, sodium carbonate and the like; phosphates such as tripotassium phosphate, trisodium phosphate, dipotassium monohydrogen phosphate, disodium monohydrogen phosphate and the like; acetates such as sodium acetate, potassium acetate and the like; organic bases such as pyridine, 4-(dimethylamino)pyridine, triethylamine, imdazole, diazabicycloundecene and the like.

The amounts of these bases are not particularly limited, and the bases can be used as a solvent when said organic bases had been used.

When these bases are used, the solvents used in the reaction, include for example water, alcohols such as methanol, ethanol, propanol, butanol and the like; halogenated hydrocarbons such as dichloromethane, chloroform and the like; aromatic hydrocarbons such as benzene, toluene, xylene and the like; aliphatic hydrocarbons such as hexane, heptane and the like; non-protonic polaric solvents such as dimethylformamide(DMF), dimethylacetamide(DMA), dimethylsulfoxide(DMSO), 1,3-dimethyl-2-imiazolidinone(DMI), 1-methyl-2-pyrrolidone(NMP) and the like; ethers such as ethylether, isopropylether, 1,2-dimethoxyethane(DME), tetrahydrofuran(THF), dioxane and the like; nitriles such as acetonitrile, propionitrile and the like.

The metal reagents used in the reaction represented by the reaction scheme (1) include for example halogenated alkylamino magnesium (Bodroux reaction) obtained by a Grignard reagent and alkylamine, aluminum lithium hydride, trimethylaluminum, triethylaluminum and the like. The amounts of these metal reagents used are not particularly limited.

When these metal reagents are used, solvents used in the reaction, include for example halogenated hydrocarbons such as dichloromethane, chloroform and the like; aromatic hydrocarbons such as benzene, toluene, xylene and the like; aliphatic hydrocarbons such as hexane, heptane and the like; ethers such as ethylether, isopropylether, 1,2-dimethoxyethane(DME), tetrahydrofuran(THF), dioxane and the like; and the like.

The reaction temperature and the reaction time of the above-described reaction can be widely varied. Generally, the reaction temperature is preferably -78 to 200°C, more preferably -78 to 100°C, and the reaction time is preferably 0.01 to 50 hours, more preferably 0.1 to 15 hours.

The equivalent amounts of the carbonyl compound represented by the formula (3) are preferably 1 to 2 equivalents, more preferably 1 to 1.2 equivalents, based on the amine derivative represented by the formula (2).

The amine derivative compound represented by the formula (2) in the reaction scheme (1) and a salt thereof can be prepared by the method as shown in the reaction scheme (2): 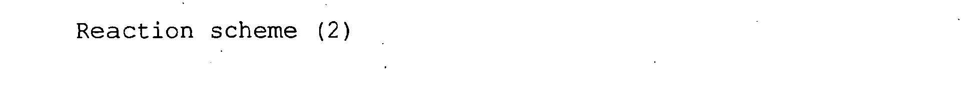 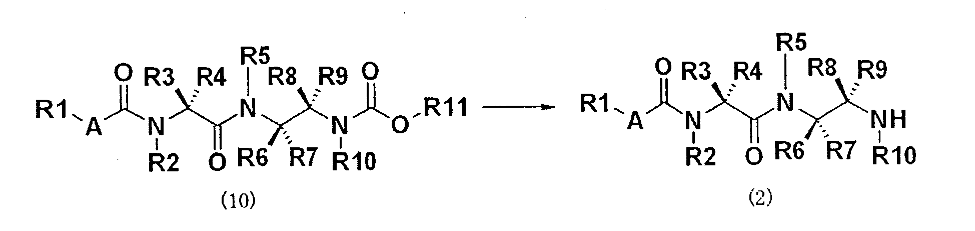 [wherein, R1, R2, R3, R4, R5, R6, R7, R8, R9, R10 and A have the same meanings as in the formula (2), R11 is a t-butyl group or a benzyl group which may be substituted].

In the reaction scheme (2), the diamine derivative represented by the formula (2) can be prepared by reacting the diamine derivative represented by the formula (10) with an acid, or by hydrogenation.

As the acid in this case, hydrochloric acid, hydrobromic acid, sulfuric acid, nitric acid, acetic acid, trifluoroacetic acid and the like can be used. The amount of these acids is not particularly limited, and can be also used as a solvent.

Hydrogenation can be carried out in a suitable solvent in the presence of a catalyst under hydrogen atmosphere at normal pressure or elevated pressure. The catalyst includes, for example, a palladium catalyst such as palladium-carbon, a nickel catalyst such as Raney-nickel and the like, a cobalt catalyst, a ruthenium catalyst, a rhodium catalyst, a platinum catalyst and the like. The solvent includes, for example, water, alcohols such as methanol, ethanol and the like; an aromatic hydrocarbon such as benzene, toluene, and the like; branched or cyclic ethers such as ether, dioxane, tetrahydrofuran and the like; ester such as ethyl acetate and the like. The reaction temperature and the reaction time of the above-described reaction can be widely varied. Generally, the reaction temperature may be appropriately selected within the range of -20°C to a reflux temperature of the used solvents, and the reaction time within the range of from several minutes to 96 hours, respectively.

The diamine derivative represented by the formula (10) in the reaction scheme (2) and a salt thereof can be prepared by the method as shown in the reaction scheme (3): 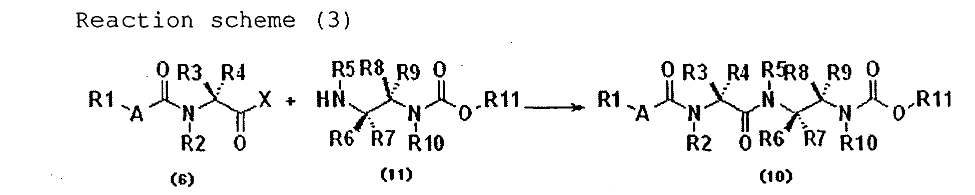 [wherein, R1, R2, R3, R4, A and X have the same meanings as in the formula (6), and R5, R6, R7, R8, R9, R10 and R11 have the same meanings as in the formula (10)].

In the reaction scheme (3), the diamine derivative represented by the formula (10) can be prepared by reacting the amine derivative represented by the formula (11) and a salt thereof with a known amino acid derivative represented by the formula (6) with or without a base, and in the presence of a metal reagent such as trialkylaluminum and the like without a solvent or in a solvent.

As the base in this reaction, the same bases as used in the reaction scheme (1) can be used. The amount of these bases is not particularly limited, and the bases can be used as a solvent when said organic bases had been used.

As the metal reagent in this reaction, the same reagents as used in the reaction scheme (1) can be used. The amounts of these metal reagents are not particularly limited.

As the organic solvent in this reaction, the same solvents as used in the reaction scheme (1) can be used.

The amount of the compound represented by the formula (6) is 1 to 4 equivalents, and preferably 1 to 2 equivalents, based on the amine derivative represented by the formula (11) .

The reaction temperature and the reaction time of the above-described reaction can be widely varied. Generally, the reaction temperature is -20 to 200°C, and preferably 0 to 100°C, and the reaction time is 0.01 to 50 hours, and preferably 0.1 to 15 hours.

The compound represented by the formula (6) in the reaction scheme (3) can be prepared by a conventional method wherein amino acid derivative represented by the formula (7) is reacted with thionyl chloride, oxalyl chloride, phosgene, phosphorus oxychloride, phosphorus trichloride, phosphorus pentachloride, thionyl bromide, phoshorus tribromide, diethylaminosulfur trifluoride, 1,1'-carbonylbis-1H-imidazole and the like.

The compound represented by the formula (6) in the reaction scheme (3) can be also prepared by a conventional method wherein amino acid derivative represented by the formula (7) is reacted with alcohols such as methylalcohol, ethylalcohol and the like or phenols such as phenol or nitrophenol and the like.

The compound represented by the formula (6) in the reaction scheme (3) can be prepared by a conventional method wherein amino acid derivative represented by the formula (7) is reacted with chloroformic esters such as methyl chloroformate, phenyl chloroformate and the like.

The compound represented by the formula (6) in the reaction scheme (3) can be prepared by a conventional method wherein amino acid derivative represented by the formula (7) is reacted with N-hydroxysuccinimide, 1-hydroxybenzotriazol and the like.

The amine derivative represented by the formula (11) in the reaction scheme (3) and a salt thereof are commercially available and can be easily prepared by a Gabriel method, a Delphine method, a known amine synthesizing method such as reduction of a cyano group, amide, imine, oxime and the like, and the method as described in Tetrahedron Asymmetry, Volume 11, page 1907 (2000).

The diamine derivative compound represented by the formula (10) can be prepared by the method as shown in the reaction scheme (4): 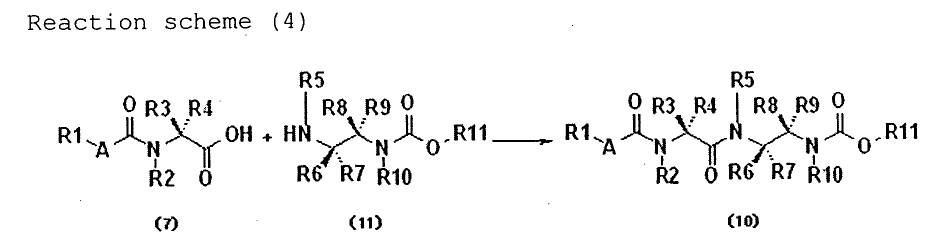 [wherein, R1, R2, R3, R4, and A have the same meanings as in the formula (7), and R5, R6, R7, R8, R9, R10 and R11 have the same meanings as in the formula (10)].

In the reaction scheme (4), the amine derivative represented by the formula (10) can be prepared as by reacting the amine derivative represented by the formula (11) and a salt thereof with a known amino acid derivative represented by the formula (7), without a solvent or in a solvent.

As the condensing agent in this reaction, N,N'-dicyclohexylcarbodiimide, 1,1'-carbonylbis-1H-imidazole, 1-ethyl-3-(3-dimethylaminopropyl)carbodiimide-hydrochloride, 2-chloro-1,3-dimethylimidazolium chloride and the like can be used.

The amount of condensing agent is 1 to 3 equivalents, and preferably 1 to 1.5 equivalents, based on the compound represented by the formula (7).

As the organic solvents in this reaction, the same solvents as used in the reaction scheme (1) can be used.

The amount of carboxylic acid derivative represented by the formula (7) is 1 to 2 equivalents, and preferably 1 to 1.2 equivalents, based on the amine derivative represented by the formula (11).

The reaction temperature and the reaction time of the above-described reaction can be widely varied. Generally, the reaction temperature is -20 to 200°C, preferably 0 to 100°C, and the reaction time is 0.01 to 50 hours, preferably 0.1 to 15 hours.

The compound represented by the formula (7) in the reaction scheme (4) can be prepared by a conventional method wherein the corresponding amino acids are reacted with chloroformic esters, carbonates such as O-methyl-O-(p-nitrophenyl)carbonate and the like; and the like.

The compound represented by the formula (3) in the reaction scheme (1) can be prepared by a conventional method in which a known carboxylic acid derivative represented by the formula (4) is reacted with thionyl chloride, oxalyl chloride, phosgene, phosphorus oxychloride, phosphorus trichloride, phosphorus pentachloride,thionyl bromide, phoshorus tribromide, diethylaminosulfur trifluoride, 1,1'-carbonylbis-1H-imidazole and the like.

The compound represented by the formula (3) in the reaction scheme (1) can be also prepared by a conventional method wherein a known carboxylic acid derivative represented by the formula (4) is reacted with alcohols such as methylalcohol, ethylalcohol and the like or phenols such as phenol or nitrophenol and the like.

The compound represented by the formula (3) in the reaction scheme (1) can be also prepared by a conventional method wherein a known carboxylic acid derivative represented by the formula (4) is reacted with chloroformic esters such as methyl chloroformate, phenyl chloroformate and the like.

The compound represented by the formula (3) in the reaction scheme (1) can be also prepared by a conventional method wherein a known carboxylic acid derivative represented by the formula (4) is reacted with N-hydroxysuccinimide, 1-hydroxybenzotriazole and the like.

The compound represented by the formula (1) according to the invention can be also prepared by the method as shown in the reaction scheme (5): 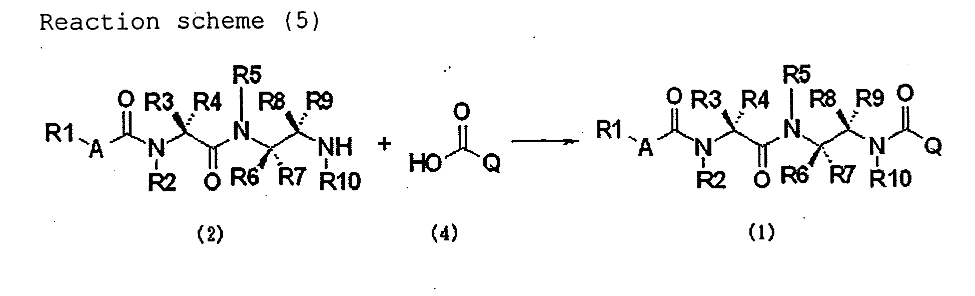 [wherein, R1, R2, R3, R4, R5, R6, R7, R8, R9, R10 and A have the same meanings as in the formula (2), and Q has the same meanings as in the formula (3)].

In the reaction scheme (5), the diamine derivative represented by the formula (1) can be prepared by reacting the amine derivative represented by the formula (2) and a salt thereof with a known carboxylic acid derivative represented by the formula (4) without a solvent or in a solvent.

As condensing agents in this reaction, N,N'-dicyclohexylcarbodiimide, 1,1'-carbonylbis-1H-imidazole, 1-ethyl-3-(3-dimethylaminopropyl)carbodiimide-hydrochloride, 2-chloro-1,3-dimethylimidazolium chloride and the like can be used.

The amount of condensing agents are 1 to 3 equivalents, and preferably 1 to 1.5 equivalents, based on the compound represented by the formula (4).

As the organic solvents in this reaction, the same solvents as used in the reaction scheme (1) can be used.

The amount of the carboxylic acid derivative represented by the formula (4) is 1 to 2 equivalents, and preferably 1 to 1.2 equivalents, based on the amine derivative represented by the formula (2).

The reaction temperature and the reaction time of the above-described reaction can be widely varied. Generally, the reaction temperature is -20 to 200°C, preferably 0 to 100°C, and the reaction time is 0.01 to 50 hours, preferably 0.1 to 15 hours.

The compound represented by the formula (1) according to the invention can be also prepared by the method as shown in the reaction scheme (6): 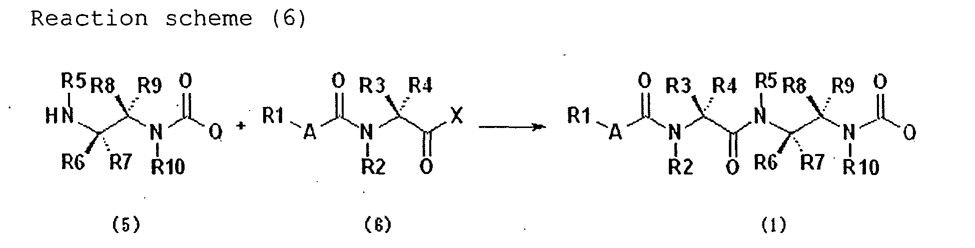 [wherein, R5, R6, R7, R8, R9, R10 and Q have the same meanings as in the formula (5) and R1, R2, R3, R4, A and X have the same meanings as in the formula (6)].

In the reaction scheme (6), the diamine derivative represented by the formula (1) can be prepared as by reacting the amine derivative represented by the formula (5) and a salt thereof with the known compound represented by the formula (6) with or without a base in the presence of a metal reagent such as trialkylaluminum and the like without a solvent or in a solvent.

As the base in this reaction, the same bases as used in the reaction scheme (1) can be used. The amount of these bases is not particularly limited. They can be used as a solvent when said organic bases had been used.

As the metal reagent in this reaction, the same reagents as used in the reaction scheme (1) can be used. The amounts of these metal reagents are not particularly limited.

As the organic solvent in this reaction, the same solvents as used in the reaction scheme (1) can be used.

The amount of compound represented by the formula (6) is 1 to 4 equivalents, and preferably 1 to 2 equivalents, based on the amine derivative represented by the formula (5).

The reaction temperature and the reaction time of the above-described reaction can be widely varied. Generally, the reaction temperature is -20 to 200°C, preferably 0 to 100°C, and the reaction time is 0.01 to 50 hours, preferably 0.1 to 15 hours.

The compound represented by the formula (1) according to the invention can be also prepared by the method as shown in the reaction scheme (7): 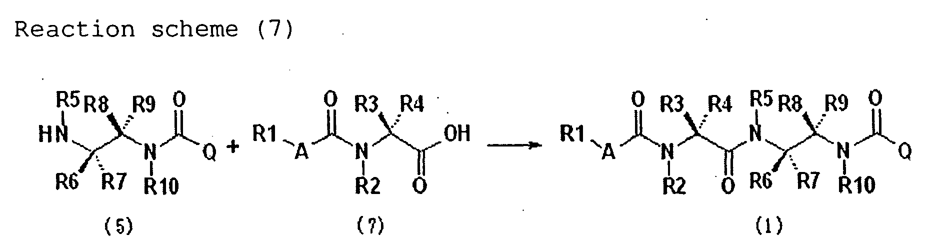 [wherein, R5, R6, R7, R8, R9, R10 and Q have the same meanings as in the formula (5) and R1, R2, R3, R4 and A have the same meanings as in the formula (7)].

In the reaction scheme (7), the diamine derivative represented by the formula (1) can be prepared by reacting the amine derivative represented by the formula (5) and a salt thereof with a known carboxylic acid derivative represented by the formula (7) without a solvent or in a solvent.

As the condensing agent in this reaction, N,N'-dicyclohexylcarbodiimide, 1,1'-carbonylbis-1H-imidazole, 1-ethyl-3-(3-dimethylaminopropyl)carbodiimide-hydrochloride, 2-chloro-1,3-dimethylimidazolium chloride and the like can be used.

The amount of the condensing agent is 1 to 3 equivalents, and preferably 1 to 1.5 equivalents, based on the compound represented by the formula (7).

As the organic solvent in this reaction, the same solvents as used in the reaction scheme (1) can be used.

The amount of carboxylic acid derivative represented by the formula (7) is 1 to 2 equivalents, and preferably 1 to 1.2 equivalents, based on the amine derivative represented by the formula (5).

The reaction temperature and the reaction time of the above-described reaction can be widely varied. Generally, the reaction temperature is -20 to 200°C, preferably 0 to 100°C, and the reaction time is 0.01 to 50 hours, preferably 0.1 to 15 hours.

The compound represented by the formula (1) according to the invention can be also prepared by the method as shown in the reaction scheme (8): 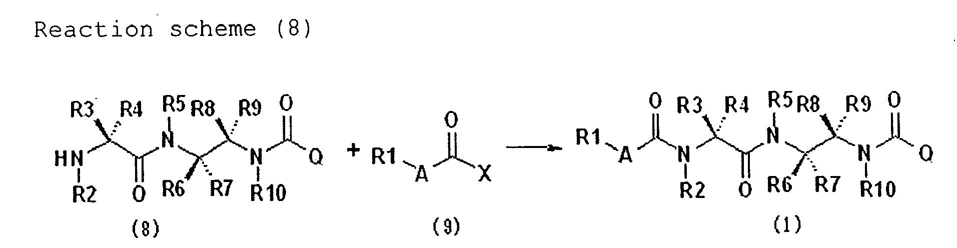 [wherein, R2, R3, R4, R5, R6, R7, R8, R9, R10 and Q have the same meanings as in the formula (8) and R1 and A have the same meanings as in the formula (9)].

In the reaction scheme (8), the diamine derivative represented by the formula (1) can be prepared as by reacting the amine derivative represented by the formula (8) and a salt thereof with the known compounds represented by the formula (9) with or without a base without a solvent or in a solvent.

As the bases in this reaction, the same bases as used in the reaction scheme (1) can be used. The amount of these bases is not particularly limited, and the bases can be used as a solvent when said organic bases had been used.

As the organic solvents in this reaction, the same solvents as used in the reaction scheme (1) can be used.

The amount of the compound represented by the formula (9) is 1 to 4 equivalents, and preferably 1 to 2 equivalents, based on diamine derivative represented by the formula (8).

The reaction temperature and the reaction time of the above-described reaction can be widely varied. Generally, the reaction temperature is -20 to 200°C, preferably 0 to 100°C, and the reaction time is 0.01 to 50 hours, preferably 0.1 to 15 hours.

The diamine derivative represented by the formula (8) in the reaction scheme (8) and a salt thereof can be prepared by the method as shown in the reaction scheme (9): 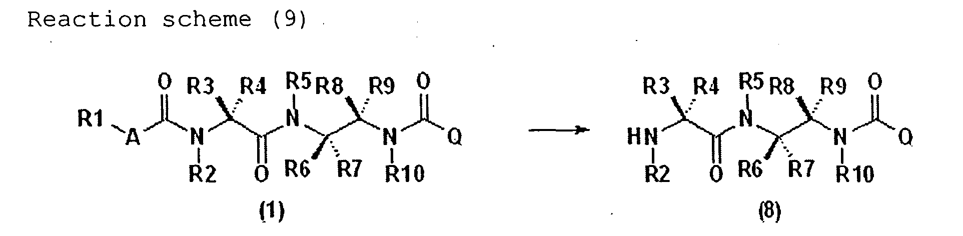 [wherein, R2, R3, R4, R5, R6, R7, R8, R9, R10 and Q have the same meanings as in the formula (8), A has the same meaning as in the formula (9), and R1 is a t-butyl group or a benzyl group which may be substituted].

In the reaction scheme (9), the diamine derivative represented by the formula (8) can be prepared by reacting the diamine derivative represented by the formula (1) with acids, or by hydrogenation.

As the acids in this case, hydrochloric acid, hydrobromic acid, sulfuric acid, nitric acid, acetic acid,trifluoroacetic acid and the like can be used. The amount of these acids is not particularly limited, and the acids can be also used as a solvent.

As the catalysts in the hydrogenation, the same catalysts as used in the process in the reaction scheme (2) can be used.

As the solvents in the reaction as shown in the reaction scheme (9) the same solvents as used in the process in the reaction scheme (1) can be used.

The reaction temperature and the reaction time of the above-described reaction can be widely varied. Generally, the reaction temperature may be appropriately selected within the range of -20°C to a reflux temperature of the used solvents, and the reaction time within the range of from several minutes to 96 hours, respectively.

The compound represented by the formula (9) in the reaction scheme (8) can be prepared by a conventional method wherein the corresponding alcohols were reacted with phosgenes such as phosgene, triphosgene and the like, and chloroformic esters such as phenylchloroformate and the like; and the like.

The diamine derivative represented by the formula (1) contains asymmetric carbon depending on the kinds of substituents, and may exist as an optical isomer, a diastereoisomer, a racemate or a mixture thereof in any proportions. The invention embraces such isomers and mixtures thereof in arbitrary proportion.

The term "fungicide" as used herein refers to an agent which combats microorganisms (pathogens) such as bacteria, fungi, viruses and the like, which are pathogens, including for example, industrial fungicides for the asepsis·the control of fungi, agricultural and horticultural fungicides, medical·disinfecting fungicides and the like. The diamine derivatives of the invention have very remarkable control effect as agricultural and horticultural fungicides.

The agricultural and horticultural fungicide comprising the diamine derivative represented by the formula (1) of the invention as an active ingredient refers to an agent which is used to protect crops from the attack of plant pathogens, and can be used against disease on various plants including vegetables, fruit trees, rice, cereals, flowering plants, turf, caused by fungi belonging to Oomycetes, Ascomycetes, Deuteromycetes, Basidiomycetes, Plasmodiophoromycete, and other pathogens. Disease names (pathogen names) are specifically illustrated by the following non-limiting examples. For example, rice blast disease (Pyricularia oryzae), Heliminthosporium blight (Cochliobolus miyabeanus), damping-off (Rhizoctonia solani), Bakanae disease (Gibberella fujikuroi), Seedling damping-off caused by the genus Pythium (Pythium graminicola, etc.), Barley powdery mildew (Erysiphe graminis f.sp.hordei; f.sp.tritici), Leaf stripe (Pyrenophora graminea), net blotch of barley (Pyrenophora teres), scab (Gibberella zeae), cereal rusts (Puccinia striiformis; P. graminis; P. recondita; P. hordei), snow rot (Typhula sp.; Micronectriella nivalis), smut (Ustilago tritici; U. nuda), eyespot disease (Pseudocercosporella herpotrichoides), scald (Rhynchosporium secalis), Septoria tritici blotch (Septoria tritici),Glum blotch (Leptosphaeria nodorum), snow mold caused by the genus Pythium (Pythium iwayamai, etc.), grape downy mildew (Plasmopara viticola), powdery mildew (Uncinula necator), anthracnose (Elsinoe ampelina), ripe rot (Glomerella cingulata), rust (Phakopsora ampelopsidis), powdery mildew on apple-tree (Podosphaera leucotricha), apple scab (Venturia inaequalis), apple leaf spot (Alternaria mali), Japanese apple rust (Gymnosporangium yamadae), blossom blight (Sclerotinia mali), apple canker (Valsa mali), black spot on pear (Alternaria kikuchiana), pear scab (Venturia nashicola), pear rust (Gymnosporangium haraeanum), rust on western pear (Phytophthora cactorum), brown rot of peach (Sclerotinia cinerea), peach scab (Cladosporium carpophilum), phomopsis leaf blight (Phomopsis sp.), root rot (Phytophthora sp.), anthracnose on persimmon (Gloeosporium kaki), angular leaf spot (Cercospora kaki; Mycosphaerella nawae), downy mildew in cucurbits (Pseudoperonospora cubensis), rot (Phytophthora melonis, Phytophthora nicotianae, Phytophthora drechsleri), powdery mildew (Sphaerotheca fuliginea), anthracnose (Colletotrichum lagenarium), gummy stem blight (Mycosphaerella melonis), tomato late blight (Phytophthora infestans), seedling damping-off (Pythium vezans, Rhizoctonia solani), early blight (Alternaria solani), leaf mold (Cladosporium fulvam), root rot (Pythium myriotylum, Pythium dissotocum), eggplant powdery mildew (Erysiphe cichoracoarum), late blight (Phytophthora infestans), brown rot (Phytophthora capsici), leaf spot(Alternaria japonica), white spot (Cerocosporella barassicae), clubroot (Plasmodiophora brassicae), downy mildew (Peronospora brassicae), white tip on a stone-leek (Phytophthora porri), leek rust (Puccinia allii), root spot of soybean(Phytophthora megasperma), downy mildew (Peronospora manshurica), purple seed stain (Cercospora kikuchii), anthracdose (Elsinoe glycines), pod and stem blight (Diaporthe phaseololum), bean anthracnose (Colletotrichum lindemuthianum), leaf spot on peanut (Mycosphaerella personatum), leaf spot (Cercospora arachidicola), powdery mildew on pea (Erysiphe pisi), downy mildew (Peronospora pisi), potato late blight (Phytophthora infestans), Early blight lesion on tuber (Alternaria solani), Japanese net blotch on tea (Exobasidium reticulatum), white scab (Elsinoe leucospila), brown spot on tobacco (Alternaria longipes), powdery mildew (Erysiphe cichoracearum), anthracdose (Colletotrichum tabacum), leaf spot on sugar beet(Cercospora beticola), downy mildew (Peronospora schachtii), rose black spot (Diplocarpon rosae), downy mildew (Peronospora sparsa), root rot (Phytophthora megasperma), powdery mildew (Sphaerotheca pannosa), leaf blotch on Chrysanthemum (Septoria chrysanthemi-indici), white rust (Puccinia horiana), root rot (Phytophthora cactorum), powdery mildew of strawberry (Sphaerotheca humuli), rot (Phytophthora nicotianae), fruit spot(Pythium ultimum), grey-mold rot (Botrytis cinerea) on cucumber, tomato, strawberry, grape and the like, scleorotinum disease (white mold) (Sclerotinia sclerotiorum), brawn patch on turf (Rhizoctonia solani), dollar-spot (Sclerotinia homoeocarpa), curvularia leaf speckle (Curvularia geniculata), rust on grass (Puccinia zoysiae), Helimintohosporium blight (Cochiliobolus sp.), scald (Rhynchosporium secalis) ,rice blast disease (Pyricularia oryzae), root rot (Gaeumannomyces graminis), anthracdose (Colletotrichum graminicola), gray snow mold (Typhula incarnate), black snow mold (Typhula ishikariensis), snow scald (Sclerotinia borealis), fairy ring (Marasmius oreades, etc.) and the genus Pythium (Pythium aphanidermatum, etc.), may be mentioned.

The diamine derivative represented by the formula (1) shows a strong fungicidal activity against a number of the Oomycete class such as pathogens of grape downy mildew (Plasmopara viticola), downy mildew in cucurbits (Pseudoperonospora cubensis), late blight in potato and tomato (Phytophthora infestans), Pythium disease in tea (Pythium aphanidermatum, etc,) and the like. The present derivative also exhibits a very remarkably good controlling effect against many kinds of the plant blights caused by the pathogenes of the genus Plasmopara, the genus Pseudoperonospora, the genus Peronospora and the genus Pythium, downy mildew, seedling damping-off, Pythium diseases and the like. The present compounds also show a very strong fungicidal activity against rice blast disease pathogen (Pyricularia oryzae), and thus also have a good controlling effect againt rice blast disease.

The active compound represented by the formula (1) has a good compatibility with plants in a concentration of the active compound to be required to control plant pathogens. Therefore, applications by treatment using an agent for the above-ground of plants, by treatment using an agent for the understock and seeds, by soil treatment and the like, can be made.

The compound of the invention, that is, the diamine derivative represented by the formula (1), can be used together with agricultural chemicals such as other fungicides or insecticides, herbicides, plant growth regulating agents and the like, soil-modifying agents or fertilizer substances, as well as as a combined formulation with other agricultural chemicals.

The compound of the invention may be used as it is, but it is preferred that it is used in a composition form wherein the compound is mixed with a carrier including a solid or liquid diluent. As used herein, the term "carriers" refers to a synthetic or natural, inorganic or organic substance which is combined to aid the active components to reach the site to be treated and to ease the storage, tranfer and handling of the active component compound.

The suitable solid carrier includes, for example, an inorganic substance such as clays, such as montmorillonite, kaolinite and bentonite and the like, diatomaceous earth, white clay, talc, vermiculite, quartz, calcium carbonate, silica gel, ammonium sulfate and the like; vegetable organic substances including soybean powder, saw dust, wheat flour and the like, and urea; and the like.

The suitable liquid carrier includes, for example, aromatic hydrocarbons such as toluene, xylene, cumene and the like; paraffin-based hydrocarbons such as kerosene, mineral oil and the like; halogen-based hydrocarbons such as carbon tetrachloride, chloroform, dichloroethane and the like; ketones such as acetone, methylethylketone and the like; ethers such as dioxane, tetrahydrofuran, diethyleneglycol dimethylether and the like; alcohols such as methanol, ethanol, propanol, ethyleneglycol and the like; dimethylformamide, dimethylsulfoxide, and water; and the like.

To further increase the effect of the compound according to the invention, the compound may be used alone or in combination with the auxiliary agents as describd below, depending on the purpose, considering the formulation of the preparations and the surface to be applied and the like.

The adjuvants, for the purpose of emulsifying, dispersing, spreading, wetting, binding, stabilization and the like, include, for example, an anionic surfactant such as lignin sulfonate, alkylbenzenesulfonate, alkyl sulfate, polyoxyalkylenealkyl sulfonate, polyoxyalkylene alkyl phosphate and the like; a non-ionic surfactant such as polyoxyalkylenealkyl ether, polyoxyalkylenealkylaryl ether, polyoxyalkylenealkylamine, polyoxyalkylenealkylamide, polyoxyalkylenealkylthioether, polyoxyalkylene aliphatic ester, glycerin fatty acid ester, sorbitan fatty acid ester, polyoxyalkylenesorbitan fatty acid ester, polyoxypropylenepolyoxyethylene block polymer and the like; a lubricant such as calcium stearate, wax and the like; a stabilizer such as isopropyl hydrodiene phosphate and the like; methylcellulose, carboxymethyl cellulose, casein and arabic gum; and the like. However, the components are not limited to those as mentioned above.

The active components of the compounds according to the invention are generally used in an amount of 0.5 to 20% by weight as powders, 5 to 50% by weight as emulsions, 10 to 90% by weight as wettable powders, 0.1 to 20% by weight as granules, and 10 to 90% by weight as flowable formulations. The carriers for each formulation are generally used in an amount of 60 to 99% by weight as powders, 40 to 95% by weight as emulsions, 10 to 90% by weight as wettable powders, 80 to 99% by weight as granules, and 10 to 90% by weight as flowable formulations. The auxiliary agents are generally used in an amount of 0.1 to 20% by weight as powders, 1 to 20% by weight as emulsions, 0.1 to 20% by weight as wettable powders, 0.1 to 20% by weight as granules, and 0.1 to 20% by weight as flowable formulations.

When the compounds of the invention are used with at least one selected from other fungicides and/or pesticides, the compounds of the invention and at least one selected from other fungicides and/or pesticides may be in the combined composition form, or both the compounds of the invention and at least one selected from other fungicides and/or pesticides are mixed to use simultaneously upon treatment of the agricultural agents.

### EXAMPLES

Hereinbelow, the invention will be illustrated with reference to the following Examples and Test Examples.

### Example 1

### N-[1,1-dimethyl-2-[(4-methylbenzoyl)amino]ethyl][(1-methylethyloxycarbonyl)amino]acetamide [compound No. 14]

To a solution of 2-[(1-methylethyloxycarbonyl)amino]acetic acid (0.24 g, 1.49 mmol) in THF (3 ml) was added 1,1'-carbonylbis-1H-imidazole (0.25 g, 1.54 mmol) at room temperature, and the mixture was stirred for 1 hour. To the reaction solution was added N-(2-amino-2-methyl propyl)4-methylbenzamide (0.25 g, 1.21 mmol) at room temperature, and the mixture was stirred at room temperature for 12 hours. The reaction solution was washed sequentially with a 5% aqueous citric acid solution, a saturated saline solution, a saturated aqueous sodium bicarbonate solution and a saturated saline solution and then dried over anhydrous magnesium sulfate. The inorganic salt was filtered, then the filtrate was concentrated under reduced pressure, and the resulting residue was purified by silica gel column chromatography (n-hexane:ethyl acetate = 1:1) to give 0.29 g of the desired product as a white solid (yield 69%).

### Example 2

### N-[2-methyl-1-(S)-[[(4-methylbenzoyl)amino]methyl]propyl]2-(S)-[(1,1-dimethylethyloxycarbonyl)amino] valeramide (compound No. 40)

To a solution of 2-(S)-[(1,1-dimethylethyloxycarbonyl)amino]valeric acid (2.54 g, 11.69 mmol) in dichloromethane (50 ml) was added 1,1'-carbonylbis-1H-imidazole (1.90 g, 11.72 mmol) at room temperature and the mixture was stirred for 1 hour. To the reaction solution were added N-[2-(S)-amino-3-methylbutyl] 4-methylbenzamide hydrochloride (2.00 g, 7.79 mmol) and triethylamine (0.87 g, 8.60 mmol) at room temperature and then the mixture was stirred at room temperature for 12 hours. The reaction solution was washed sequentially with a 5% aqueous citric acid solution, a saturated saline solution, a saturated aqueous sodium bicarbonate solution and a saturated saline solution and then dried over anhydrous magnesium sulfate. The inorganic salt was filtered and then the filtrate was concentrated under reduced pressure. The resulting crude product was washed with diisopropyl ether to give 2.86g of the desired product as a white solid (yield 88%).

### Example 3

### N-[2-methyl-1-(S)-[[(4-methylbenzoly)amino]methyl]propyl]2-(S)-[(1,1-dimethylethyloxycarbonyl)amino]-2-phenyl acetamide (compound No. 60)

To a solution of 2-(S)-[(1,1-dimethylethyloxycarbonyl)amino]2-phenyl acetamide (2.93 g, 11.66 mmol) in dichloromethane (50 ml) was added 1,1'-carbonhylbis-1H-imidazole (1.90 g, 11.72 mmol) at room temperature and the mixture was stirred for 1 hour. To the reaction solution were added N-[2-(S)-amino-3-methylbutyl] 4-methylbenzamide hydrochloride (2.00 g, 7.79 mmol) and triethylamine (0.87 g, 8.60 mmol) at room temperature and then the mixture was stirred at room temperature for 12 hours. The reaction solution was washed sequentially with a 5% aqueous citric acid solution, a saturated saline solution, a saturated aqueous sodium bicarbonate solution and a saturated saline solution and then dried over anhydrous magnesium sulfate. The inorganic salt was filtered and then the filtrate was concentrated under reduced pressure. The resulting crude product was washed with diisopropyl ether to give 3.06 g of the desired product as a white solid (yield 87%).

### Example 4

### N-[2-methyl-1-(S)-[[(4-methylbenzoyl)amino]methyl]propyl] 2-(RS)-[(1,1-dimethylethyloxycarbonyl)amino]-2-(tetrahydrofuran-3-yl)acetamide (Compound No. 69)

To a solution of 2-(RS)-[(1,1-dimethylethyloxycarbonyl)amino]-2-(tetrahydrofuran-3-yl)acetic acid (1.44 g, 5.87 mmol), N-[2-(S)-amino-3-methylbutyl] 4-methylbenzamide hydrochloride (1.26 g, 4.89 mmol), N-hydroxysuccinimide (0.64 g, 6.36 mmol), dicyclohexylcarbodiimide (1.30 g, 6.36 mol) in dichloromethane (50 ml) was added triethylamine (0.59 g, 5.87 mmol) at room temperature and the mixture was stirred at room temperature for 5 hours. The resulting crystal of dicyclohexylurea was removed by filtration, and the filtrate was concentrated under reduced pressure. The resulting residue was purified by silica gel column chromatography (n-hexane:ethyl acetate = 1:1) to give 1.23 g of the desired product as a white solid (yield 56%).

### Example 5

### N-[2-methyl-1-(S)-[[(4-methylbenzoyl)amino]methyl]propyl] 2-(S)-[(2-methylpropyloxycarbonyl)amino]-4-pentynamide (Compound No. 76)

To a solution of 2-(S)-[(2-methylpropyloxycarbonyl)amino]-4-pentynoic acid (0.50 g, 2.30 mmol) in dichloromethane (10 ml) was added 1,1'-carbonylbis-1H-imidazole (0.41 g, 2.50 mmol) at room temperature and the mixture was stirred for 2 hours. To the reaction solution were added N-[2-(S)-amino-3-methylbutyl] 4-methylbenzoic acid (0.27 g, 1.10 mmol) and then imidazole (0.22 g, 3.20 mmol), the reaction solution was stirred at room temperature overnight. To the reaction solution was added ethyl acetate. Then, the mixture was washed sequentially with a 5% aqueous citric acid solution, water, a 2 N aqueous sodium hydroxide solution and water and dried over anhydrous magnesium sulfate. The inorganic salt was filtered and then the filtrate was concentrated under reduced pressure. The resulting crude product was washed with diisopropyl ether to give 0.20 g of the desired product as a white solid (yield 43%).

### Example 6

### N-[2-methyl-1-(S)-[[(2-methylbenzoyl)amino]methyl]propyl] 3-methyl-2-(S)-[(1-methylethyloxycarbonyl)amino]butyramide (Compound No. 143)

To a solution of 3-methyl-2-(S)-[(1-methylethyloxycarbonyl)amino]butyric acid (0.51 g, 2.49 mmol) in tetrahydrofuran (5 ml) was added 1,1'-carbonylbis-1H-imidazole (0.40 g, 2.49 mmol), and the mixture was stirred at room temperature for 30 minutes. To the reaction solution were added a solution of N-[2-(S)-amino-3-methylbutyl] 2-methylbenzamide hydrochloride (0.58 g, 2.27 mmol) and triethylamine (0.34 g, 3.41 mmol) in tetrahydrofuran (10 ml) and the mixture was stirred overnight. The solvent was concentrated under reduced pressure and the residue was dissolved in ethyl acetate (20 ml) and washed sequentially with a 5% aqueous citric acid solution, a saturated aqueous sodium bicarbonate solution and a saturated saline solution and then dried over anhydrous sodium sulfate. The inorganic salt was filtered and then the filtrate was concentrated under reduced pressure. The resulting crude product was washed with diisopropyl ether to give 0.55 g of the desired product as a white solid (yield 60%).

### Example 7

### N-[2-methyl-1-(RS)-[[(4-methylbenzoyl)amino]methyl]propyl] 3-methyl-2-(S)-[(l-methylethyloxycarbonyl)amino]butyramide (Compound No. 151)

To a solution of 3-methyl-2-(S)-[(1-methylethyloxycarbonyl)amino]butyric acid (0.50 g, 2.46 mmol) in THF (5 ml) was added 1,1'-carbonylbis-1H-imidazole (0.41 g, 2.53 mmol) at room temperature and the mixture was stirred for 1.5 hours. To the reaction solution were added N-[2-(RS)-amino-3-methylbutyl] 4-methylbenzamide hydrochloride (0.64 g, 2.49 mmol) and triethylamine (0.29 g, 2.87 mmol) at room temperature, and the mixture was stirred at room temperature for 12 hours. The reaction solution was washed sequentially with a 5% aqueous citric acid solution, a saturated saline solution, a 1 N aqueous sodium hydroxide solution and a saturated saline solution and dried over anhydrous magnesium sulfate. The inorganic salt was filtered, then the filtrate was concentrated under reduced pressure, and the resulting residue was purified by silica gel column chromatography (n-hexane:ethyl acetate = 1:1) to give 0.62 g of the desired product as a white solid (yield 62%).

### Example 8

### N-[1-(S)-[[(2-chlorobenzoyl)amino]methyl]-2-methylpropyl] 3-methyl-2-(S)-[(1-methylethyloxycarbonyl)amino]butyramide (Compound No. 196)

To a solution of 3-methyl-2-(S)-[(1-methylethyloxycarbonyl)amino]butyric acid (0.30 g, 1.58 mmol) in dichloromethane (10 ml) was added N-methylmorpholine (0.17 ml,1.58 mmol) at -15°C, and to the mixture was added 2-methylpropyloxycarbonylchloride (0.22 ml, 1.73 mmol) at the same temperature. After stirring for 5 minutes, to the reaction solution were added a solution of N-[2-(S)-amino-3-methylbutyl] 2-chlorobenzamide hydrochloride.(0.40 g, 1.44 mmol) and triethylamine (0.29 g, 2.88 mmol) in dichloromethane (5 ml) at the same temperature and the mixture was stirred for 20 minutes and then allowed to a room temperature. The reaction solution was washed with water, a 5% aqueous citric acid solution, a saturated aqueous sodium bicarbonate solution and a saturated saline solution and then dried over anhydrous sodium sulfate. The inorganic salt was filtered and then the filtrate was concentrated under reduced pressure. The resulting crude product was washed with diisopropyl ether to give 0.56 g of the desired product as a white solid (yield 91%).

### Example 9

### N-[1-(S)-[[(4-chlorobenzoyl)amino]methyl]-2-methylpropyl] 3-methyl-2-(S)-[(2-methylpropyloxycarbonyl)amino]butyramide (Compound No. 205)

To a solution of N-[2-(S)-amino-3-methylbutyl] 4-chlorobenzamide hydrochloride (0.50 g, 1.80 mmol), 3-methyl-2-(S)-[(2-methylpropyloxycarbonyl)amino]butyric acid (0.59 g, 2.70 mmol), N-hydroxysuccinimide (0.33 g, 2.88 mmol), dicyclohexylcarbodiimide (0.60 g, 2.88 mmol) in dichloromethane (20 ml) was added triethylamine (0.27 g, 2.70 mmol) at room temperature and the mixture was stirred at room temperature overnight. The resulting precipitate of dicyclohexylurea was filtered, and concentrated under reduced pressure, and the resulting residue was purified by silica gel column chromatography (n-hexane:ethyl acetate = 1:1). The resulting crude product was washed with diisopropyl ether to give 0.36 g of the desired product as a white solid (yield 46%).

### Example 10

### N-[3-methyl-2-(R)-[(4-methylbenzoyl)amino]butyl] 3-methyl-2-(S)-[(2-methylpropyloxycarbonyl)amino]butyramide (Compound No. 274)

### (a) N-[3-methyl-2-(R)-[(4-methylbenzoyl)amino]butyl] 2-(S)-amino-3-methylbutyramide

A solution of N-[3-methyl-2-(R)-[(4-methylbenzoyl)amino]butyl] 2-(S)-[(benzyloxycarbonyl)amino]-3-methylbutyramide (Compound No. 276, 0.93 g, 2.05 mmol), 10% palladium on carbon (0.14 g, 15% by weight), concentrated hydrochloric acid (3 ml) in methanol (50 ml) was stirred under hydrogen atmosphere at 40°C for 5 hours. The reactor was purged with nitrogen and the catalyst was removed by Celite filtration. The filtrate was concentrated under reduced pressure, and the resulting residue was dissolved in a 2 N hydrochloric acid solution and washed with dichloromethane. The aqueous layer was adjusted to pH12 with sodium hydroxide and then extracted with dichloromethane. The organic layer was washed with a 2 N aqueous sodium hydroxide solution and then dried over anhydrous magnesium sulfate. The inorganic salt was filtered and the filtrate was concentrated under reduced pressure to give 0.67 g of the desired product as a yellow oily substance (yield 87%).

### (b) N-[3-methyl-2-(R)-[(4-methylbenzoyl)amino]butyl] 3-methyl-2-(S)-[(2-methylpropyloxycarbonyl)amino]butyramide (Compound No. 274)

To a solution of N-[3-methyl-2-(R)-[(4-methylbenzoyl)amino]butyl] 2-(S)-amino-3-methylbutyramide (0.27 g, 0.76 mmol) and triethylamine (0.09 g, 0.91 mmol) in dichloromethane (30 ml) was added dropwise 2-methylpropyloxycarbonylchloride (0.12 g, 0.84 mmol) under ice-cooling, and the mixture was stirred at room temperature for 5 hours. The reaction solution was washed sequentially with a 5% aqueous citric acid solution, a saturated saline solution, a 2 N aqueous sodium hydroxide solution and a saturated saline solution and then dried over anhydrous magnesium sulfate. The inorganic salt was filtered and the filtrate was concentrated under reduced pressure. The resulting crude product was washed with n-hexane and diisopropyl ether to give 0.08 g of the desired product as a white solid (yield 25%).

### Example 11

### N-[3-methyl-2-(R)-[(4-methylbenzoyl)amino]butyl] 2-(S)-[(benzyloxycarbonyl)amino]-3-methylbutyramide (Compound No. 276)

### (a) N-[3-methyl-2-(R)-[(1,1-dimethylethyloxycarbonyl)amino]butyl]2-(S)-[(benzyloxycarbonyl)amino]-3-methylbutyramide

To a solution of 2-(S)-[(benzyloxycarbonyl)amino]-3-methylbutyric acid (4.66 g, 18.54 mmol) in dichloromethane (100 ml) was added 1,1'-carbonylbis-1H-imidazole (3.01 g, 18.54 mmol) at room temperature and the mixture was stirred for 1 hour. To the reaction solution was added 1,1-dimethylethyl N-[1-(R)-(aminomethyl)-2-methylpropyl]carbamate (2.-50 g, 12.36 mmol) at room temperature and the mixture was stirred at room temperature for 5 hours. The reaction solution was washed sequentially with a 5% aqueous citric acid solution, a saturated saline solution, a 2 N aqueous sodium hydroxide solution and a saturated saline solution and then dried over anhydrous magnesium sulfate. The inorganic salt was filtered and then the filtrate was concentrated under reduced pressure. The resulting crude product was washed with diisopropyl ether to give 2.51 g of the desired product as a white solid (yield 47%).

### (b) N-[2-(R)-amino-3-methylbutyl] 2-(S)-[(benzyloxycarbonyl)amino]-3-methylbutyramide

To a solution of N-[3-methyl-2-(R)-[(1,1-dimethylethyloxycarbonyl)amino]butyl] 2-(S)-[(benzyloxycarbonyl)amino]-3-methylbutyramide (2.50 g, 5.74 mmol) in ethyl acetate (30 ml) was added a 4 N hydrochloric acid ethyl acetate solution (30 ml) at room temperature and the mixture was stirred for 5 hours. The reaction solution was extracted with water, and aqueous layer was washed with ethyl acetate. An aqueous layer was adjusted to pH 12 with sodium hydroxide and extracted with dichloromethane. The organic layer was washed with a 2 N aqueous sodium hydroxide solution and dried over anhydrous magnesium sulfate. The inorganic salt was filtered and the filtrate was concentrated under reduced pressure to give 1.83 g of the desired product as a yellow solid (yield 86%).

### (c) N-[3-methyl-2-(R)-[(9-methylbenzoyl)amino]butyl] 2-(S)-[(benzyloxycarbonyl)amino]-3-methylbutyramide (Compound No. 276)

To a solution of N-[2-(R)-amino-3-methylbutyl] 2-(S)-[(benzyloxycarbonyl)amino]-3-methylbutyramide (1.80 g, 5.37 mmol) and triethylamine (0.61 g, 5.91 mmol) in dichloromethane (50 ml) was added dropwise a solution of 4-methylbenzoylchloride (0.91 g, 5.91 mmol) in dichloromethane (10 ml) under ice-cooling. The reaction solution was stirred at room temperature for 2 hours and then washed with water. The organic layer was dried over saturated magnesium sulfate and then inorganic salt was removed by filtration, and the filtrate was concentrated under reduced pressure and the resulting crude product was crystallized from n-hexane to give 1.16 g of the desired product as a white solid (yield 48%).

### Example 12

### N-[3-methyl-2-(S)-[(4-methylbenzoyl)amino]butyl] 2-(S)-[(benzyloxycarbonyl)amino]-3-methylbutyramide (Compound No. 281)

### (a) N-[3-methyl-2-(S)-[(1,1-dimethylethyloxycarbonyl)amino]butyl]2-(S)-[(benzyloxycarbonyl)amino]-3-methylbutyramide

To a solution of 2-(S)-[(benzyloxycarbonyl)amino]-3-methylbutyric acid (4.51 g, 17.94 mmol) in dichloromethane (100 ml) was added 1,1'-carbonylbis-1H-imidazole (2.91 g, 17.94 mmol) at room temperature, and the mixture was stirred for 1 hour. To the reaction solution was added 1,1-dimethylethyl N-[1-(S)-(aminomethyl)-2-methylpropyl]carbamate (2.42 g, 11.96 mmol) at room temperature, and the mixture was stirred at room temperature for 5 hours. The reaction solution was washed sequentially with a 5% aqueous citric acid solution, a saturated saline solution, a 2 N aqueous sodium hydroxide solution and a saturated saline solution and then dried over anhydrous magnesium sulfate. The inorganic salt was filtered and the filtrate was concentrated under reduced pressure. The resulting crude product was washed with diisopropyl ether to give 2.96 g of the desired product as a white solid (yield 57%).

### (b) N-[2-(S)-amino-3-methylbutyl] 2-(S)-[(benzyloxycarbonyl)amino]-3-methylbutyramide

To a solution of N-[3-methyl-2-(S)-[(1,1-dimethylethyloxycarbonyl)amino]butyl] 2-(S)-[(benzyloxycarbonyl)amino]-3-methylbutyramide (2.73 g, 6.27 mmol) in ethyl acetate (30 ml) was added 4 N hydrochloric acid ethyl acetate (30 ml) at room temperature, and the mixture was stirred for 5 hours. The reaction solution was extracted with water and the aqueous layer was washed with ethyl acetate. The aqueous layer was adjusted to pH12 with sodium hydroxide, and then extracted with dichloromethane. The organic layer was washed with a 2 N aqueous sodium hydroxide solution and then dried over anhydrous magnesium sulfate. The inorganic salt was filtered and the filtrate was concentrated under reduced pressure to give 1.89g of the desired product as a light yellow solid (yield 90%).

### (c) N-[3-methyl-2-(S)-[(4-methylbenzoyl)amino]butyl] 2-(S)-[(benzyloxycarbonyl)amino]-3-methylbutyramide (Compound No. 281)

To a solution of 4-methylbenzoic acid (1.00 g, 7.33 mmol) in dichloromethane (50 ml) was added 1,1'-carbonylbis-1H-imidazole (1.19 g, 7.33 mmol) at room temperature, and the mixture was stirred for 2 hours. To the reaction solution was added N-[2-(S)-amino-3-methylbutyl] 2-(S)-[(benzyloxycarbonyl)amino]-3-methylbutyramide (1.89 g, 5.64 mmol), and the reaction solution was stirred at room temperature for 5 hours. The reaction solution was washed sequentially with a 5% aqueous citric acid solution, a saturated saline solution, a 2 N aqueous sodium hydroxide solution and a saturated saline solution and then dried over anhydrous magnesium sulfate. The inorganic salt was filtered and then the filtrate was concentrated under reduced pressure. The resulting crude product was washed with diisopropyl ether to give 1.25 g of the desired product as a white solid (yield 44%).

### Example 13

### N-[2-(S)-methyl-1-(S)-[[(4-methylbenzoyl)amino]methyl]butyl] 3-methyl-2-(S)-[(methyloxycarbonyl)amino]butyramide (Compound No. 306)

To a solution of N-[2-(S)-methyl-l-(S)-[[(4-methylbenzoyl)amino]methyl]butyl] 2-(S)-amino-3-methylbutyramide hydrochloride (0.50 g, 1.35 mmol) in dichloromethane (10 ml)was added methyloxycarbonylchloride (0.15 g, 1.59 mmol) and triethylamine (0.36 g, 3.56 mmol) at room temperature, and the mixture was stirred at room temperature for 12 hours. The reaction solution was washed sequentially with a 5% aqueous citric acid solution, a saturated saline solution, a 1 N aqueous sodium hydroxide solution and a saturated saline solution and then dried over anhydrous magnesium sulfate. The inorganic salt was filtered and then the filtrate was concentrated under reduced pressure. The resulting crude product was washed with diisopropyl ether to give 0.35 g of the desired product as a white solid (yield 66%).

### Example 14

### N-[1-(S)-[[(benzofuran-2-carbonyl)amino]methyl]-2-methylpropyl] 2-(S)-[(1,1-dimethylethyloxycarbonyl)amino]propionamide (Compound No. 601)

To a solution of 2-(S)-[(1,1-dimethylethyloxycarbonyl)amino]propionic acid (0.46 g, 2.44 mmol) in dichloromethane (20 ml) was added 1,1'-carbonylbis-1H-imidazole (0.40 g, 2.44 mmol), and the mixture was stirred at room temperature for 30 minutes. To the reaction solution was added N-[2-(S)-amino-3-methylbutyl]benzofuran-2-carboxamide (0.50 g, 2.02 mmol) was stirred overnight. The solvent was concentrated under reduced pressure, and the resulting residue was dissolved in ethyl acetate (20 ml), washed sequentially with a 5% aqueous citric acid solution, a saturated aqueous sodium bicarbonate solution, water and a saturated saline solution and then dried over anhydrous magnesium sulfate. The inorganic salt was filtered and then the filtrate was concentrated under reduced pressure. The resulting crude product was washed with diisopropyl ether to give 0.59 g of the desired product as a white solid (yield 70%).

### Example 15

### N-[1-(S)-[[(benzofuran-2-carbonyl)amino]methyl]-2-methylpropyl] 2-(S)-[(1-methylethyloxycarbonyl)amino]propionamide (Compound No. 599)

### (a) N-[1-(S)-[[(benzofuran-2-carbonyl)amino]methyl]-2-methylpropyl 2-(S)-aminopropionamide

To a solution of N-[1-(S)-[[(benzofuran-2-carbonyl)amino]methyl]-2-methylpropyl] 2-(S)-[(1,1-dimethylethyloxycarbonyl)amino]propionamide (Compound No. 12, 1.71 g, 4.10 mmol) in ethyl acetate (5 ml) was added a 4 N hydrochloric acid/ethyl acetate solution (11 ml) at room temperature, and the mixture was stirred overnight. The reaction solution was extracted with water and the resulting aqueous layer was washed with ethyl acetate. The aqueous layer was adjusted to pH12 with sodium hydroxide and extracted with dichloromethane and then the resulting organic layer was dried over anhydrous magnesium sulfate. The inorganic salt was filtrated, and the filtrate was concentrated under reduced pressure to give 1.24 g of the desired product as a white solid (yield 95%).

### (b) N-[1-(S)-[[(benzofuran-2-carbonyl)amino]methyl]-2-methylpropyl]2-(S)-[(1-methylethyloxycarbonyl)amino]propionamide (Compound No. 599)

To a solution of N-[1-(S)-[[(benzofuran-2-carbonyl)amino]methyl]-2-methylpropyl 2-(S)-aminopropionamide (0.23 g, 0.72 mmol) in dichloromethane (5 ml) was added 1-methylethyloxycarbonylchloride (0.10 g, 0.79 mmol). To the reaction solution was added dropwise a solution of triethylamine (0.08 g, 0.79 mmol) in dichloromethane (1 ml), and the mixture was stirred at room temperature for 2 hours. The reaction solution was washed sequentially with a 5% aqueous citric acid solution and water and then dried over anhydrous magnesium sulfate. The inorganic salt was filtered and the filtrate was concentrated under reduced pressure. The resulting crude product was purified by silica gel column chromatography (n-hexane:ethyl acetate = 1:1) to give 0.28g of the desired product as a white solid (yield 96%).

### Example 16

### N-[1-(S)-[[(benzofuran-2-carbonyl)amino]methyl]-2-methylpropyl] 2-[(1,1-dimethylethyloxycarbonyl)amino]-2-methylpropionamide (Compound No. 605)

To a solution of 2-[(1,1-dimethylethyloxycarbonyl)amino]-2-methylpropionic acid (2.80 g, 13.78 mmol), N-[2-(S)-amino-3-methylbutyl]benzofuran-2-carboxamide (2.26 g, 9.18 mmol) and N-hydroxysuccinimide (1.69 g, 14.69 mmol) in dichloromethane (50 ml)was added dicyclohexylcarbodiimide (DCC: 3.03 g, 14.69 mmol) and triethylamine (1.39 g, 13.78 mmol). The mixture was stirred overnight. The resulting crystal of dicyclohexylurea was removed by filtration and then the filtrate was washed sequentially with a 5% aqueous citric acid solution, a saturated aqueous sodium bicarbonate solution and water and dried over anhydrous magnesium sulfate. The inorganic salt was filtered and the filtrate was concentrated under reduced pressure. The resulting crude product was purified by silica gel column chromatography (n-hexane:ethyl acetate = 1:1) to give 3.39 g of the desired product as a white solid (yield 86%).

### Example 17

### N-[2-(RS)-[[(benzofuran-2-carbonyl)amino]-1-methyl]ethyl] [3-methyl-2-(S)-[N-(1,1-dimethylethyloxycarbonyl)amino]]butyramide (Compound No. 652)

To a solution of 3-methyl-2-(S)-[(1,1-dimethylethyloxycarbonyl)amino]butyric acid (0.55 g, 2.55 mmol) in dichloromethane (50 ml) was added 1,1'-carbonylbis-1H-imidazole (0.41 g, 2.55 mmol), and the mixture was stirred at room temperature for 1 hour. To the reaction solution was added N-[2-(RS)-aminopropyl]benzofuran-2-carboxamide hydrochloride (0.50 g, 1.96 mmol), imidazole (0.44 g, 6.47 mmol), and the mixture was stirred at room temperature for 5 hours. The reaction solution was washed sequentially with a 5% aqueous citric acid solution, a saturated saline solution, a saturated aqueous sodium bicarbonate solution and a saturated saline solution and then dried over anhydrous magnesium sulfate. The inorganic salt was filtered and the filtrate was concentrated under reduced pressure. The resulting crude product was washed with a mixed solution of diisopropyl ether and n-hexane to give 0.60 g of the desired product as a white crystal (yield 73%).

### Example 18

### N-[3-methyl-2-(S)-[(benzofuran-2-carbonyl)amino]butyl] 2-(S)-[(benzyloxycarbonyl)amino]-3-methylbutyramide (Compound No. 678)

To a solution of benzofuran-2-carboxylic acid (1.73 g, 10.68 mmol) in dichloromethane (50 ml) was added 1,1'-carbonylbis-1H-imidazole (1.76 g, 10.68 mmol) at room temperature, and the mixture was stirred at room temperature for 1 hour. To the reaction solution was added N-[2-(S)-amino-3-methylbutyl] 2-(S)-[(benzyloxycarbonyl)amino]-3-methylbutyramide (3.41 g, 10.17 mmol), and the mixture was stirred at room temperature for 5 hours. The reaction solution was washed sequentially with a 5% aqueous citric acid solution, a saturated saline solution, a 2 N aqueous sodium hydroxide solution and a saturated saline solution and then dried over anhydrous magnesium sulfate. The inorganic salt was filtered and the filtrate was concentrated under reduced pressure. The resulting crude product was washed with a mixed solution of diisopropyl ether and n-hexane to give 3.60 g of the desired product as a light yellow crystal (yield 74%).

### Example 19

### N-[[2-(S)-[(benzofuran-2-carbonyl)amino]-3-methyl]butyl][2-(S)-[(1-methylethyloxycarbonyl)amino]-3-methyl]butyramide (Compound No. 675)

### (a) N-[3-methyl-2-(S)-[(benzofuran-2-carbonyl)amino]butyl] 2-(S)-amino-3-methylbutyramide

A solution of N-[3-methyl-2-(S)-[(benzofuran-2-carbonyl)amino]butyl] 2-(S)-[(benzyloxycarbonyl)amino]-3-methylbutyramide (Compound No. 86, 3.40 g, 7.09 mmol), 10% palladium on carbon (0.51 g, 15% by weight) and concentrated hydrochloric acid (3 ml) in methanol (50 ml) was stirred under hydrogen atmosphere at 40°C for 5 hours. The reactor was purged with nitrogen and then the catalyst was removed by Celite filtration. The filtrate was concentrated under reduced pressure and the resulting residue was dissolved in a 2 N hydrochloric acid solution, and washed with dichloromethane. An aqueous layer was adjusted to pH 12 with sodium hydroxide and extracted with dichloromethane. The organic layer was washed with a 2 N aqueous sodium hydroxide solution and dried over anhydrous magnesium sulfate. The inorganic salt was filtered and the filtrate was concentrated under reduced pressure to give 1.82 g of the desired product as a white solid (yield 74%).

### (b) N-[[2-(S)-[(benzofuran-2-carbonyl)amino]-3-methyl]butyl][2-(S)-[(1-methylethyloxycarbonyl)amino]-3-methyl]butyramide (Compound No. 675)

To a solution of N-[3-methyl-2-(S)-[(benzofuran-2-carbonyl)amino]butyl] 2-(S)-amino-3-methylbutyramide (0.80 g, 2.32 mmol) and triethylamine (0.26 g, 2.55 mmol) in dichloromethane (50 ml) was added 1-methylethyloxycarbonylchloride (0.31 g, 2.55 mmol) under ice-cooling and the mixture was stirred at room temperature for 2 hours. The reaction solution was washed sequentially with a 5% aqueous citric acid solution, a saturated saline solution, a 2 N aqueous sodium hydroxide solution and a saturated saline solution and then dried over anhydrous magnesium sulfate. The inorganic salt was filtered and then the filtrate was concentrated under reduced pressure. The resulting crude product was washed with diisopropyl ether to give 0.58g of the desired product as a white crystal (yield 58%).

### Example 20

### N-[1-(S)-[[(quinoline-2-carbonyl)amino]methyl]-2-(S)-methylbutyl] 2-(S)-(methyloxycarbonylamino)-3-methylbutyramide (Compound No. 698)

To a solution of 3-methyl-2-[(methyloxycarbonyl)amino]butyric acid (0.30 g, 1.71 mmol), N-[2-(S)-amino-3-methylpenthyl] quinoline-2-carboxamide (0.46 g, 1.70 mmol) in dichloromethane (10 ml) was added 1-(3-dimethylaminopropyl)-3-ethylcarbodiimide hydrochloride (EDC: 0.33 g, 1.72 mmol) at room temperature, and the mixture was stirred for 5 hours and then allowed to stand overnight. The reaction solution was washed with water and then the organic layer was dried over anhydrous sodium sulfate. The inorganic salt was separated by filtration and then concentrated under reduced pressure, and the resulting crude product was washed with diisopropyl ether to give 0.51 g of the desired product as a white solid (yield 70%).

### Example 21

### N-[1-(S)-[[(benzofuran-2-carbonyl)amino]methyl]-2,2-dimethylpropyl] [3-methyl-2-(S)-[N-(ethyloxycarbonyl)amino]]butyramide (Compound No. 714)

To a solution of 3-methyl-2-(S)-[(ethyloxycarbonyl)amino]butyric acid (0.47 g, 2.50 mmol) in dichloromethane (50 ml) was added 1,1'-carbonylbis-1H-imidazole (0.38 g, 2.50 mmol) at room temperature and the mixture was stirred for 1 hour. To the reaction solution was added N-[2-(S)-amino-3-3,3-dimethylbutyl]benzofuran-2-carboxamide (0.50 g, 1.92 mmol) and the mixture was stirred at room temperature for 5 hours. The reaction solution was washed sequentially with a 5% aqueous citric acid solution, a saturated saline solution, a 2 N aqueous sodium hydroxide solution and a saturated saline solution and then dried over anhydrous magnesium sulfate. The inorganic salt was filtered and then the filtrate was concentrated under reduced pressure. The resulting crude product was washed with diisopropyl ether to give 0.74 g of the desired product as a white crystal (yield 89%).

### Example 22

### N-[2-methyl-1-(S)-[[(benzofuran-2-carbonyl)amino]methyl]propyl] [3-methyl-2-(S)-[N-(ethyloxycarbonyl)amino]]butyramide (Compound No. 772)

To a solution of 3-methyl-2-(S)-[(ethyloxycarbonyl)amino]butyric acid (0.40 g, 2.11 mmol) in dichloromethane (50 ml) was added 1,1-carbonylbis-1H-imidazole (0.32 g, 1.94 mmol), and the mixture was stirred at room temperature for 2 hours. To the reaction solution was added N-[2-(S)-amino-3-methylbutyl]benzofuran-2-carboxamide (0.40 g, 1.62 mmol), and the mixture was stirred at room temperature for 5 hours. The reaction solution was washed sequentially with a 5% aqueous citric acid solution, a saturated saline solution, a 2 N aqueous sodium hydroxide solution and a saturated saline solution and then dried over anhydrous magnesium sulfate. The inorganic salt was filtered and then the filtrate was concentrated under reduced pressure. The resulting crude product was washed with diisopropyl ether to give 0.33 g of the desired product as a white crystal (yield 49%).

### Example 23

### N-[2-methyl-1-(S)-[[(benzofuran-2-carbonyl)amino]methyl]propyl][3-methyl-2-(S)-[N-(1-methylethyloxycarbonyl)amino]]butyramide (Compound No. 775)

To a solution of 3-methyl-2-(S)-[-(1-methylethyloxycarbonyl)amino]butyric acid (0.39 g, 1.92 mmol), N-[2-(S)-amino-3-methylbutyl]benzofuran-2-carboxamide hydrochloride (0.50 g, 1.77 mmol), N-hydroxysuccinimide (0.21 g, 2.12 mmol) and dicyclohexylcarbodiimide (0.44 g, 2.12 mmol) in dichloromethane (50 ml) was added triethylamine (0.35 g, 3.54 mmol) at room temperature and the mixture was stirred at room temperature overnight. The resulting precipitation of dicyclohexylurea was filtrated, and the filtrate was concentrated under reduced pressure. The resulting residue was purified by silica gel column chromatography (n-hexane:ethyl acetate = 4:1). The resulting crude product was washed with diisopropyl ether to give 0.38 g of the desired product as a white crystal (yield 50%).

### Example 24

### N-[1-(S)-[[(benzofuran-2-carbonyl)amino]methyl]-2= methylpropyl] 3-methyl-2-(S)-[(vinyloxycarbonyl)amino]butyramide (Compound No. 782)

### (a) N-[1-(S)-[[(benzofuran-2-carbonyl)amino]methyl]-2-methylpropyl [3-methyl-2-(S)-amino]butyramide

To a solution of N-[1-(S)-[[(benzofuran-2-carbonyl)amino]methyl]-2-methylpropyl] 3-methyl-2-(S)-[(1,1-dimethylethyloxycarbonyl)amino]butyramide (19.53 g, 43.83 mmol) in ethyl acetate (100 ml) was added a 4 N hydrochloric acid/ethyl acetate solution (117 ml) at room temperature, and the mixture was stirred overnight. The reaction solution was extracted with water and the aqueous layer was washed with ethyl acetate. The aqueous layer was adjusted to pH12 with sodium hydroxide, and extracted with dichloromethane, and dried over anhydrous magnesium sulfate. The inorganic salt was filtered and the filtrate was concentrated under reduced pressure to give 12.63 g of the desired product as a white solid (yield 83%).

### (b) N-[1-(S)-[[(benzofuran-2-carbonyl)amino]methyl]-2-methylpropyl] 3-methyl-2-(S)-[(vinyloxycarbonyl)amino]butyramide (Compound No. 782)

To a solution of N-[1-(S)-[[(benzofuran-2-carbonyl)amino]methyl]-2-methylpropyl [3-methyl-2-(S)-amino]butyramide (0.46 g, 1.34 mmol) in dichloromethane (10 ml) was added triethylamine (0.41 g, 4.02 mmol). To the reaction solution was added dropwise a solution of vinyloxycarbonylchloride (0.17 g, 1.61 mmol) in dichloromethane (10 ml) under ice-cooling, and the mixture was stirred at room temperature for 2 hours. The reaction solution was washed sequentially with a 5% aqueous citric acid solution, a saturated aqueous sodium bicarbonate solution and water and dried over anhydrous sodium sulfate. The inorganic salt was filtered and then the filtrate was concentrated under reduced pressure. The resulting crude product was washed with diisopropyl ether to give 0.46g of the desired product as a white solid (yield 83%).

### Example 25

### N-[1-(S)-[[(benzofuran-2-carbonyl)amino]methyl]-2-methylpropyl] 3-methyl-2-(S)-[[(tetrahydrofuran-2-methyl)oxycarbonyl]amino]butyramide (Compound No. 786)

To a solution of N-[1-(S)-[[(benzofuran-2-carbonyl)amino]methyl]-2-methylpropyl 3-methyl-2-(S)-aminobutyramide (0.46 g, 1.34 mmol) in dichloromethane (10 ml) was added dropwise a solution of O-[tetrahydrofuran-2-methyl] O-[4-nitrophenyl]carbonate (0.43 g, 1.61 mmol) in dichloromethane (10 ml) under ice-cooling, and the mixture was stirred at room temperature for 5 days. The reaction solution was washed sequentially with a 5% aqueous citric acid solution, a saturated aqueous sodium bicarbonate solution and water and dried over anhydrous sodium sulfate. The inorganic salt was filtered and the filtrate was concentrated under reduced pressure. The resulting crude product was washed with diisopropyl ether to give 0.52 g of the desired product as a white solid (yield 82%).

### Example 26

### N-[1-(S)-[[(benzofuran-2-carbonyl)amino]methyl]-2-methylpropyl] [3-methyl-2-(S)-(ethylthiocarbonylamino)]butyramide (Compound No. 793)

To a solution of N-[1-(S)-[(benzoxazol-2-yl)carbonylaminomethyl]-2-methylpropyl] 3-methyl-2-(S)-aminobutyramide hydrochloride (0.30 g, 0.79 mmol), ethylthiocarbonylchloride (0.10 g, 0.80 mmol) in dichloromethane (15 ml) was added a solution of triethylamine (0.24 g, 2.37 mmol) in dichloromethane (5 ml) at 5°C, and the mixture was stirred at room temperature for 5 hours and allowed to stand overnight. To the reaction solution was added ethyl acetate, and the mixture was washed with water and dried over anhydrous sodium sulfate. The inorganic salt was filtered and then the filtrate was concentrated under reduced pressure. The resulting crude product was washed with diisopropyl ether to give 0.21 g of the desired product as a white solid (yield 60%).

### Example 27

N-[2-methyl-1-(S)-[[[(1,4-benzodioxane)-2-(RS)-carbonyl]amino]methyl]propyl] [3-methyl-2-(S)-[N-(1-methylethyloxycarbonyl)amino]]butyramide (Compound No. 899)

To a solution of 3-methyl-2-(S)-[(1-methylethyloxycarbonyl)amino]butyric acid (0.37 g, 1.83 mmol), N-[2-(S)-amino-3-methylbutyl] 1,3-benzodioxane-2-(RS)-carboxamide hydrochloride (0.50 g, 1.66 mmol), N-hydroxysuccinimide (0.20 g, 1.99 mmol) and dicyclohexylcarbodiimide (0.41 g, 1.99 mmol) in dichloromethane (50 ml) was added triethylamine (0.33 g, 3.32 mmol) at room temperature, and the mixture was stirred at room temperature overnight. The resulting precipitate of dicyclohexylurea was filtered, and the filtrate was concentrated under reduced pressure. The resulting residue was purified by silica gel column chromatography (n-hexane:ethyl acetate = 4:1). The resulting crude product was washed with diisopropyl ether to give 0.71g of the desired product as white semi-solid (yield 95%).

### Example 28

### N-[1-(S)-[(benzoxadiazol-4-yl)carbonylaminomethyl]-2-methylpropyl] 3-methyl-2-(S)-[(1-methylethyl)oxycarbonylamino]butyramide (Compound No. 929)

### (a) N-[2-(S)-(2,2-dimethylethyloxycarbonyl)amino-3-methyl]butyl phthalimide

To solution of 1,1-dimethylethyl N-[(1-hydroxymethyl-2-methyl)propyl]carbamate (30.00 g, 147.58 mmol), phthalimide (21.72 g, 147.58 mmol) and triphenylphosphine (42.36 g, 162.34 mmol) in tetrahydrofuran (300 ml) was added dropwise a solution of diethyl azodicarboxylate (DEAD) (28.32 g, 162.34 mmol) in tetrahydrofuran (50 ml) under ice-cooling. The temperature of the reaction solution was slowly elevated, and the mixture was stirred at room temperature for 5 hours. The reaction solution was concentrated under reduced pressure to give 45.91 g of a white solid. The obtained white solid was purified by silica gel column chromatography to give 34.40 g of the desired product as a white crystal (yield 70%).

### (b) N-[2-(S)-amino-3-methyl]butyl phthalimidehydrochloride

To a suspension of N-[2-(S)-(2,2-dimethylethyloxycarbonyl)amino-3-methyl]butyl phthalimide (23.05 g, 69.34 mmol) in ethyl acetate (50 ml) was added a 4 N hydrochloric acid/ethyl acetate solution (150 ml, 600 mmol as hydrochloric acid) at room temperature, and the mixture was stirred at room temperature for 5 hours. The precipitated white crystal was collected by filtration, and the obtained crystal was washed with ethyl acetate (100 ml). The resulting crystal was dried under reduced pressure to give 16.74 g of the desired product as a white crystal.

### (c) N-[2-methyl-1-(S)-[(phthalimide-1-yl)methyl]propyl [3-methyl-2-(S)-[N-(1-methylethyloxycarbonyl)amino]]butyramide

To a solution of 3-methyl-2-(S)-[(1-methylethyloxycarbonyl)amino]butyric acid (17.06 g, 83.95 mmol) in dichloromethane (350 ml) was added 1,1'-carbonylbis-1H-imidazole (11.62 g, 83.95 mmol) at room temperature, and the mixture was stirred at room temperature for 2 hours. To the reaction solution were added N-[2-(S)-amino-3-methyl]butyl phthalimidehydrochloride (15.00 g, 64.58 mmol) and imidazole (14.42 g, 213.11 mmol) at room temperature and the mixture was stirred at room temperature for 7 hours. The reaction solution was washed sequentially with a 5% aqueous citric acid solution, water and a saturated aqueous bicarbonate solution and then dried over anhydrous magnesium sulfate. The inorganic salt was filtered and then the filtrate was concentrated under reduced pressure. The resulting white crystal was washed with diisopropyl ether to give 18.67 g of the desired product as a white crystal (yield 69%, melting point 240.9°C).

### (d) N-[1-(S)-aminomethyl-2-methylpropyl] 3-methyl-2-(S)-[(1-methylethyl)oxycarbonylamino]butyramide

To a solution of N-[2-methyl-1-(S)-[(phthalimide-1-yl)methyl]propyl [3-methyl-2-(S)-[N-(1-methylethyloxycarbonyl)amino]]butyramide (10.00 g, 23.95 mmol) in ethanol (250 ml) was added hydrazine monohydrate (2.52 g, 50.30 mmol) at room temperature, and the mixture was stirred under heating and reflux for 5 hours. The reaction solution was returned to room temperature, and the precipitated crystal was removed by filtration and washed with ethanol. The washed filtrate was concentrated under reduced pressure, and the resulting residue was dissolved in dichloromethane and washed with a 2 N aqueous sodium hydroxide solution. The insoluble white precipitate was removed by Celite filtration. It was extracted with a 5% aqueous citric acid solution and the extract was washed with dichloromethane. The pH of the aqueous layer was adjusted to 12 or higher with a 2 N aqueous sodium hydroxide solution under ice-cooling and then the aqueous layer was extracted with dichloromethane. The organic layer was washed with a 2 N aqueous sodium hydroxide solution and dried over anhydrous magnesium sulfate. The inorganic salt was filtrated, and the filtrate was concentrated under reduced pressure to give 5.88 g of the desired product as a white crystal (yield 85%, melting point 106.3 °C).

### (e) N-[1-(S)-[(benzoxadiazol-4-yl)carbonylaminomethyl]-2-methylpropyl]3-methyl-2-(S)-[(1-methylethyl)oxycarbonylamino]butyramide (Compound No. 929)

To a solution of N-[1-(S)-aminomethyl-2-methylpropyl] 3-methyl-2-(S)-[(1-methylethyl)oxycarbonylamino]butyramide (0.50 g, 1.99 mmol) and triethylamine (0.26 g, 2.57 mmol) in dichloromethane (10 ml) was added a solution of benzoxadiazole-4-carbonylchloride (0.37 g, 2.03 mmol) in dichloromethane (10 ml) at 5°C. The mixture was returned to room temperature, stirred for 5 hours and then allowed to stand overnight. To the reaction solution was added ethyl acetate, and the mixture was washed with water and dried over anhydrous sodium sulfate. The inorganic salt was filtered and the filtrate was concentrated under reduced pressure, and the resulting crude product was washed with diisopropyl ether to give 0.60 g of the desired product as a white solid (yield 88%).

### Example 29

### N-[1-(S)-[[(6-chloropyridine-2-carbonyl)amino]methyl]-2-methylpropyl] 3-methyl-2-(S)-[(1-methylethyloxycarbonyl)amino]butyramide (Compound No. 953)

To a solution of 3-methyl-2-(S)-[(1-methylethyloxycarbonyl)amino]butyric acid (0.20 g, 0.99 mmol) in dichloromethane (5 ml) was added 1,1'-carbonylbis-1H-imidazole (0.16 g, 0.99 mmol), and the mixture was stirred at room temperature for 30 minutes. To the reaction solution was added N-[2-(S)-amino-3-methylbutyl] 6-chloropyridine-2-carboxamide (0.20 g, 0.83 mmol), and the mixture was stirred overnight. The solvent was concentrated under reduced pressure, and the resulting residue was dissolved in ethyl acetate (20 ml), washed sequentially with a 5% aqueous citric acid solution, a saturated aqueous sodium bicarbonate solution, water and a saturated saline solution and then dried over anhydrous sodium sulfate. The inorganic salt was filtered and then the filtrate was concentrated under reduced pressure. The resulting crude product was washed with diisopropyl ether to give 0.20 g of the desired product as a white solid (yield 56%).

### Example 30

### N-[1-(S)-[[(thiophene-2-carbonyl)amino]methyl]-2-methylpropyl] 3-methyl-2-(S)-[(2-methylpropyloxycarbonyl)amino]butyramide (Compound No. 1020)

To a solution of 3-methyl-2-(S)-[(2-methylpropyloxycarbonyl)amino]butyric acid (0.52 g, 2.42 mmol) in dichloromethane (10 ml) was added N-methylmorpholine (0.27 ml, 2.42 mmol) at -15°C. To the mixture was added dropwise 2-methylpropyloxycarbonylchloride (0.33 ml, 2.58 mmol) and then the mixture was stirred at - 15°C for 5 minutes. To the reaction solution was added a solution of N-[2-(S)-amino-3-methylbutyl] thiophene-2-carboxamide hydrochloride (0.40 g, 1.61 mmol) and triethylamine (0.33 g, 3.22 mmol) in dichloromethane (5 ml) at -15°C and stirred for 20 minutes. The reaction solution was concentrated under reduced pressure, and the resulting residue was dissolved in ethyl acetate (20 ml), washed sequentially with water, a 5% aqueous citric acid solution, a saturated aqueous sodium bicarbonate solution, and a saturated saline solution and then dried over anhydrous sodium sulfate. The inorganic salt was filtered and then the filtrate was concentrated under reduced pressure. The resulting crude product was washed with diisopropyl ether to give 0.54 g of the desired product as a white solid (yield 82%).

Hereinbelow, the preparation methods of the intermediates are illustrated with reference to the following Reference Examples.

### Reference Example 1

### 1,1-dimethylethyl N-[1-(S)-(aminomethyl)-2-methylpropyl]carbamate

To a solution of 1,1-dimethylethyl-N-[1-(S)- (hydroxymethyl)-2-methylpropyl]carbamate (25.00 g, 122.98 mmol) in tetrahydrofuran (250 ml) was added phthalimide (18.10 g, 123.02 mmol) and triphenylphosphine (35.50 g, 135.35 mmol) and the mixture was stirred under ice-cooling. Then, to the mixture was added dropwise a solution of diethyl azodicarboxylate (23.60 g, 135.51 mmol) in tetrahydrofuran (10 ml). The mixture was stirred under ice-cooling for 1 hour, and further stirred at room temperature for 4 hours. The solvent was concentrated under reduced pressure and then dried. To the resulting white solid of the resulting N-[2-(S)-[(1,1-dimethylethyloxycarbonyl)amino]-3-methylbutyl]phthalimide was added ethanol (500 ml) and pyrazine monohydrate (13.1 g, 261.69 mmol) and the mixture was refluxed at 100°C for 2 hours. The precipitated white crystal of phthaloylhydrazide was removed by filtration and the reaction solution was allowed to stand overnight. The precipitated phthaloylhydrazide was further removed by filtration. The solvent was concentrated under reduced pressure and then dried. To the residue was added dichloromethane (500 ml), and the mixture was washed with a 2 N aqueous sodium hydroxide solution (500 ml). The organic layer was extracted with a 5% aqueous citric acid solution (500 ml) and the aqueous layer was washed with dichloromethane (500 ml). The pH of the aqueous layer was adjusted to 12 using sodium hydroxide (10.40 g), extracted with dichloromethane (500 ml), washed with a 2 N aqueous sodium hydroxide solution (500 ml) and then dried over anhydrous sodium sulfate. The inorganic salt was filtered and the filtrate was concentrated under reduced pressure to give 21.95 g of the desired product as a white solid (yield 88%).

### Reference Example 2

### N-[3-methyl-2-(S)-[(1,1-dimethylethyloxycarbonyl)amino]butyl] 4-cyanobenzamide

To a solution of 1,1-dimethylethyl N-[1-(S)-(aminomethyl)-2-methylpropyl]carbamate (2.00 g, 9.89 mmol) in dichloromethane (20 ml) was added triethylamine (2.00 g, 2.76 ml), and the mixture was cooled to 0°C. To the reaction solution was added dropwise a solution of 4-cyanobenzoylchloride (2.59 g, 14.84 mmol) in dichloromethane (20 ml). The mixture was returned to room temperature and stirred for 2 hours. The reaction solution was washed sequentially with water, a 5% aqueous citric acid solution and a saturated aqueous sodium bicarbonate solution, and then dried over anhydrous sodium sulfate. The inorganic salt was filtered and then the filtrate was concentrated under reduced pressure. The resulting crude product was recrystallized using n-hexane and ethyl acetate to give 3.05 g of the desired product as a white solid (yield 93%).

### Reference Example 3

### N-[2-(S)-amino-3-methylbutyl] 4-cyanobenzamide hydrochloride

N-[2-(S)-[(1,1-dimethylethyloxycarbonyl)amino]-3-methylbutyl] 4-cyanobenzamide (2.85 g, 8.60 mmol) was dissolved in ethyl acetate (10 ml), and a 4 N hydrochloric acid/ethyl acetate solution (13 ml) was added thereto. The mixture was stirred at room temperature overnight. The precipitated salt was washed with ethyl acetate, collected by filtration and dried to give 2.03 g of the desired product as a white solid (yield 88%).

### Reference Example 4

### N-[3-methyl-2-(S)-[(1,1-dimethylethyloxycarbonyl)amino]butyl]benzofurancarboxamide

To a solution of benzofuran-2-carboxylic acid (15.0 g, 92.5 mmol) in dichloromethane (300 ml) was added 1,1'-carbonylbis-1H-imidazole (15.8 g, 97.6 mmol) at room temperature, and the mixture was stirred for 2 hours. To the reaction solution was added 1,1-dimethylethyl N-[1-(S)-(aminomethyl)-2-methylpropyl]carbamate (19.7 g, 92.5 mmol) was stirred for 5 hours and allowed to stand overnight. The reaction solution was washed sequentially with a 5% aqueous citric acid solution, water, a saturated aqueous sodium bicarbonate solution and water and then the organic layer was dried over anhydrous sodium sulfate. The inorganic salt was separated by filtration, and concentrated under reduced pressure. The resulting crude product was recrystallized using diisopropyl ether to give 26.9 g of the desired product as a white solid (yield 84%).

### Reference Example 5

### N-[3-methyl-2-(S)-aminobutyl]benzofurancarboxamide hydrochloride

N-[3-methyl-2-(S)-[(1,1-dimethylethyloxycarbonyl)amino]butyl]benzofurancarboxamide (28.2 g, 77.5 mmol) was dissolved in ethyl acetate (50 ml), a 4 N hydrochloric acid/ethyl acetate solution (200 ml) was added thereto and then the mixture was stirred at room temperature for 6 hours. The precipitated salt was washed with ethyl acetate and collected by filtration and then dried to give 20.8 g of the desired product as a white solid (yield 95%).

The compounds represented by the formula (1) which can be prepared in the same manner as in Examples 1 to 30 are listed in Table 1 as follows. Among these compounds, several chemical properties on certain compounds are listed in Table 2. In Table 1, Me represents a methyl group, Et represents an ethyl group, nPr represents a normal propyl group, iPr represents an isopropyl group, nBu represents a normal butyl group, iBu represents an isobutyl group, sBu represents a secondary butyl group, tBu represents a tertiary butyl group, neoPen represents a 2,2-dimethylpropyl group, 2-EtHex represents a 2-ethylhexyl group, MOE represents a methoxy ethyl group, cPr-CH₂ represents a cyclopropylmethyl group, cHex-CH₂ represents a cyclohexylmethyl group, CF₃ represents a trifluoromethyl group, CF₃CH₂ represents a 2,2,2-trifluoroethyl group, ClCH₂ represents a chloromethyl group, CH₃CHCl represents a chloroethyl group, CCl₃CH₂ represents a 2,2,2-trichloroethyl group, CCl₃C(Me)₂ represents a 2,2,2-trichloro-1,1-dimethylethyl group, 2-BocAE represents a 2-(N-tertiary butyloxycarbonyl)aminoethyl group, POCEt represents a 1-(isopropoxycarbonyl)ethyl group, 1-Me-1-MeS-Et represents a 1-methyl-1-methylthioethyl group, Ph represents a phenyl group, 4-MePh represents a 4-methylphenyl group, Bn represents a benzyl group, α-Me-Bn represents a α-methylbenzyl group, 1-NP represents a 1-naphthyl group, 2-NP represents a 2-naphthyl group, cPen represents a cyclopenthyl group, cHex represents a cyclohexyl group, Ac represents an acetyl group, Bz represents a benzoyl group, vinyl represents an ethenyl group, allyl represents a 2-propenyl group, propargyl represents a 2-propinyl group, 2-THF represents a tetrahydrofuran-2-yl group, 3-THF represents a tetrahydrofuran-3-yl group, THF-2-CH₂ represents a tetrahydrofuran-2-ylmethyl group, THF-3-CH₂ represents a tetrahydrofuran-3-ylmethyl group, 2-TPrepresents a thiophen-2-yl group, 4-Py represents a pyridin-4-yl group, Pyr-2-CH₂ represents a pyrrolidin-2-ylmethyl group, Boc-Pyr-2-CH₂ represents a (N-tertiary butyloxycarbonylpyrrolidin)-2-ylmethyl group, (-)Ment represents a (1R,2S,5R)-2-isopropyl-5-methylcyclohexan-1-yl group, (+)Ment represents a (1S,2R,5S)-2-isopropyl-5-methylcyclohexan-1-yl group, 3-furanyl-CH₂ represents a (furan-3-yl)methyl group, 4,5-DEP-2-CH₂ represents a (4,5-diethoxypyridin-2-yl)methyl group.

The diamine derivative represented by the formula (1) contains asymmetric carbon depending on the kinds of substituents, and may exist as an optical isomer, a diastereoisomer, a racemate or a mixture thereof in arbitrary proportions.

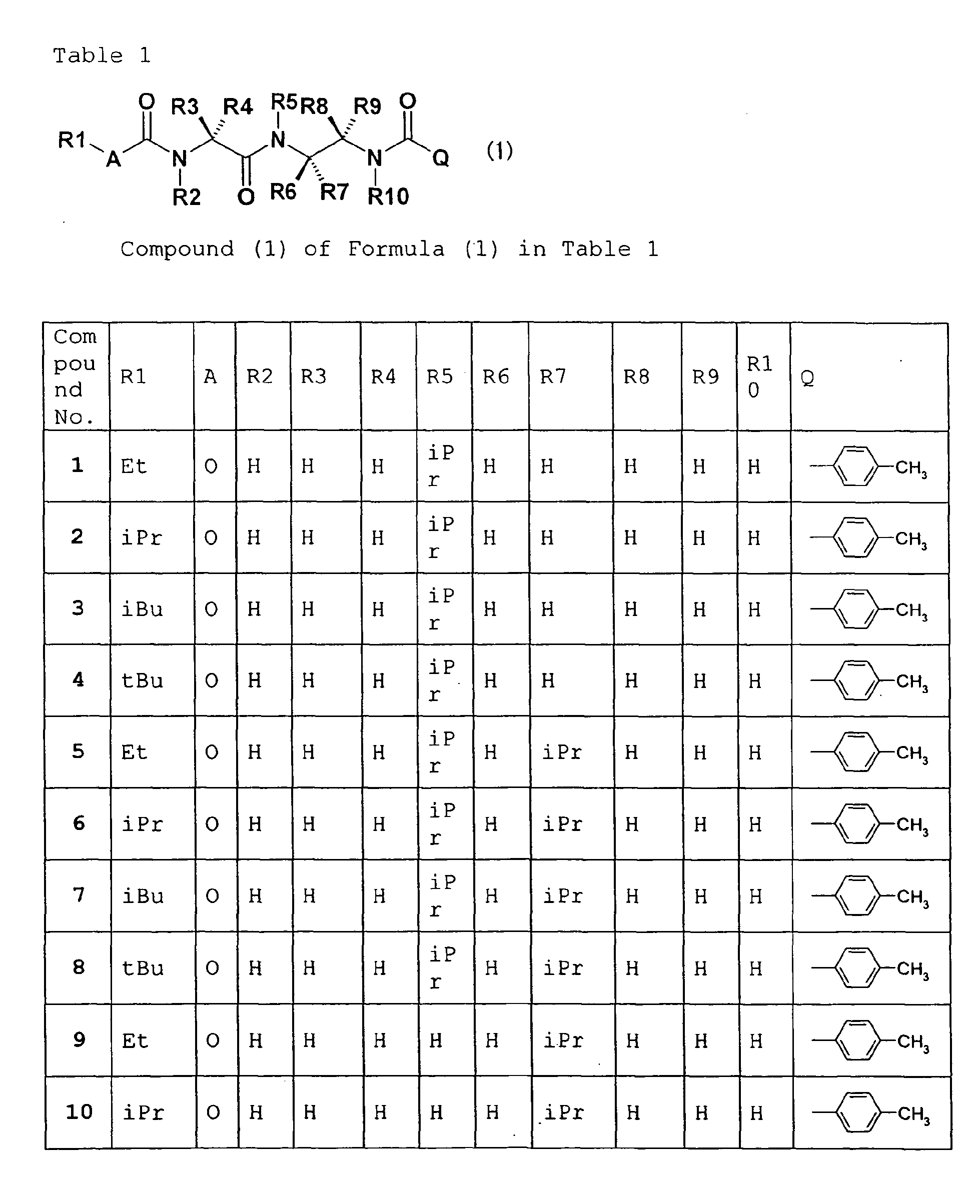

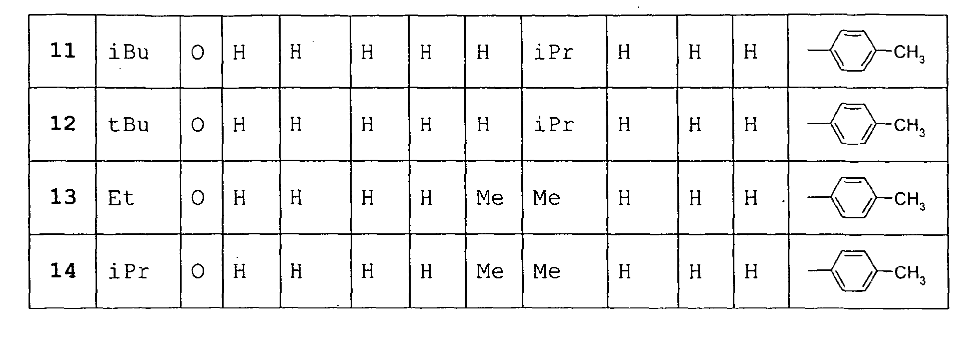

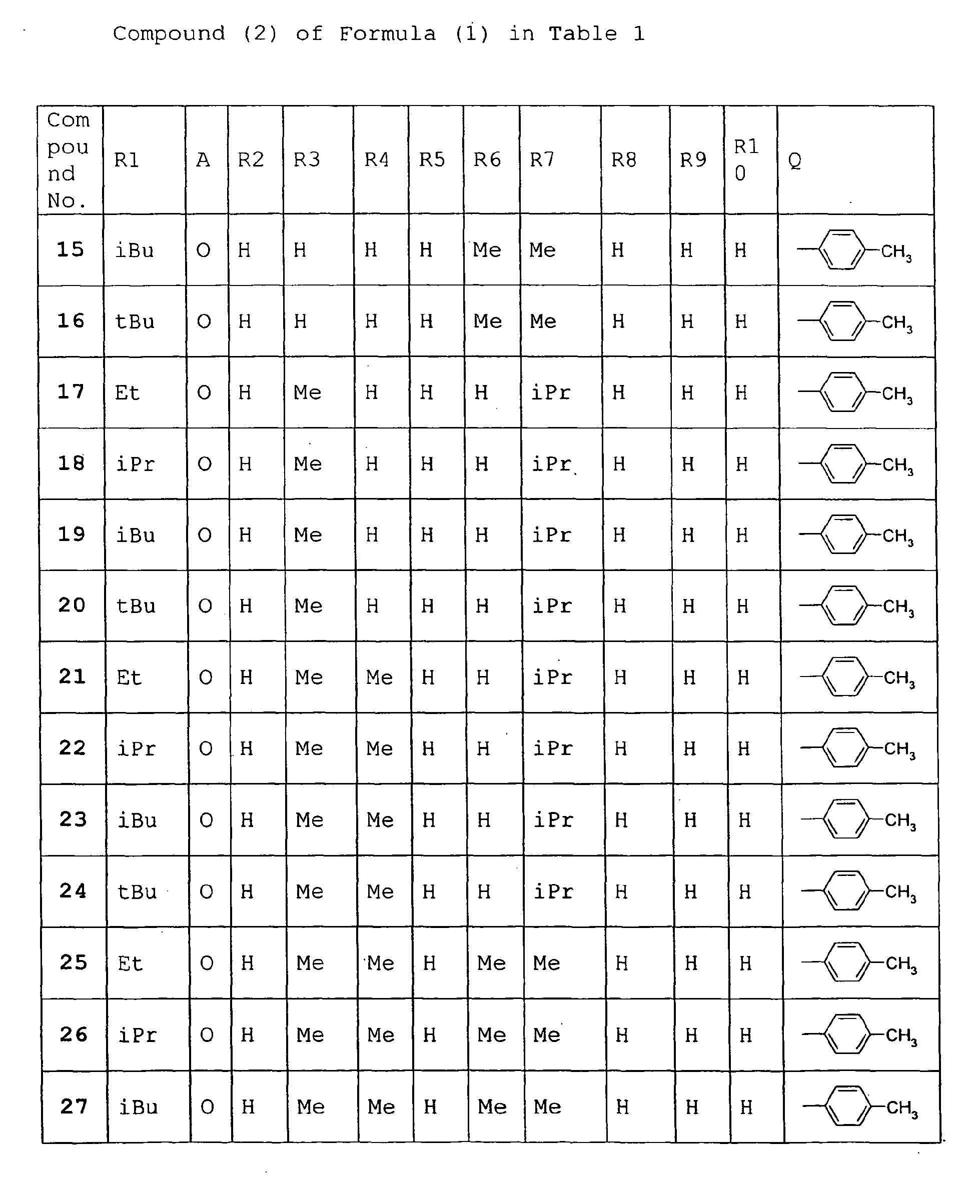

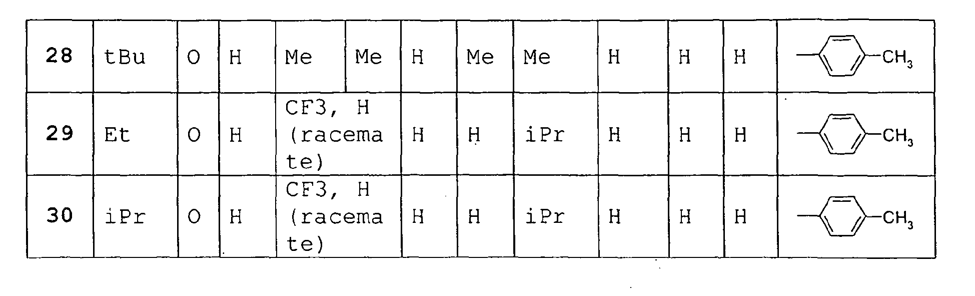

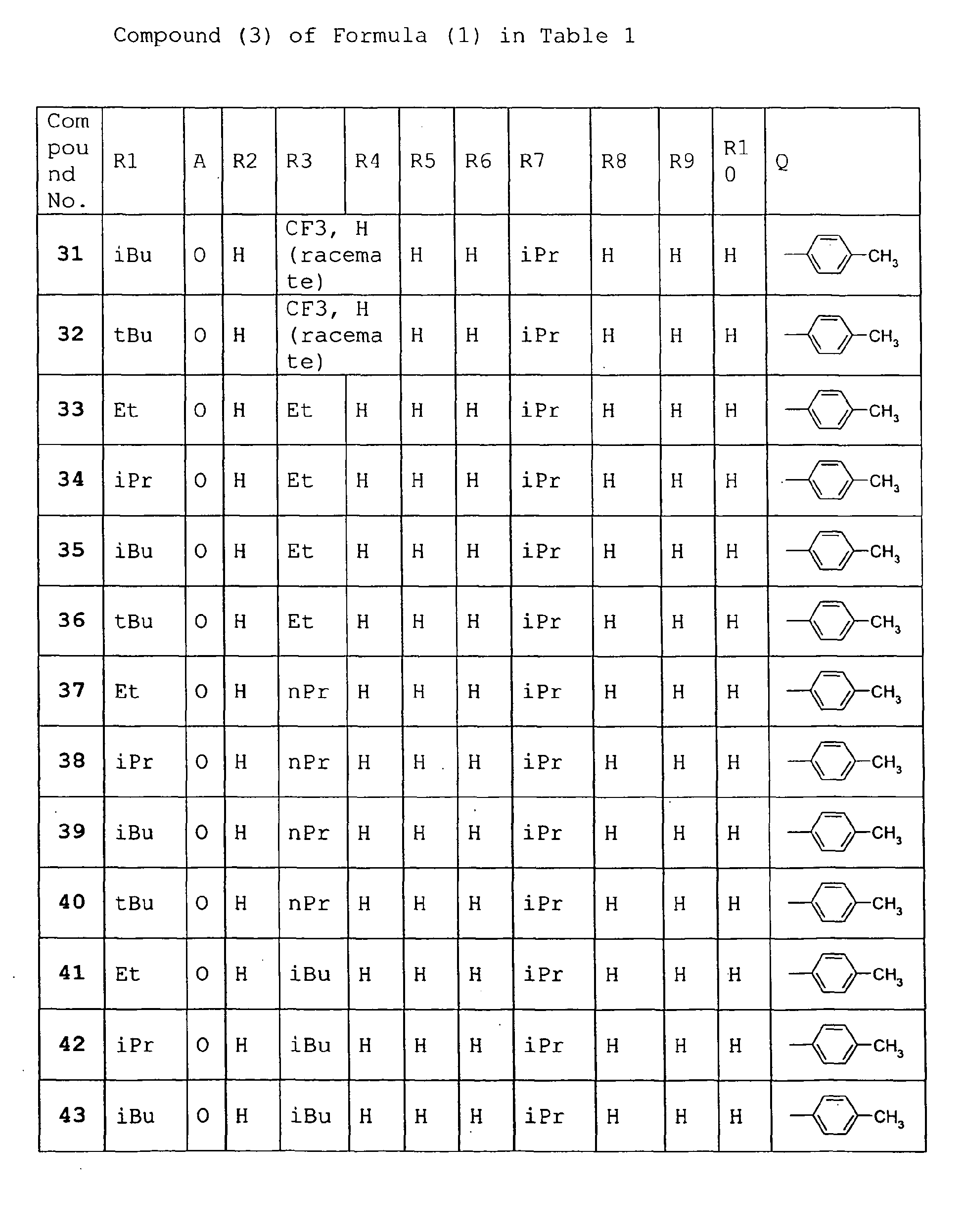

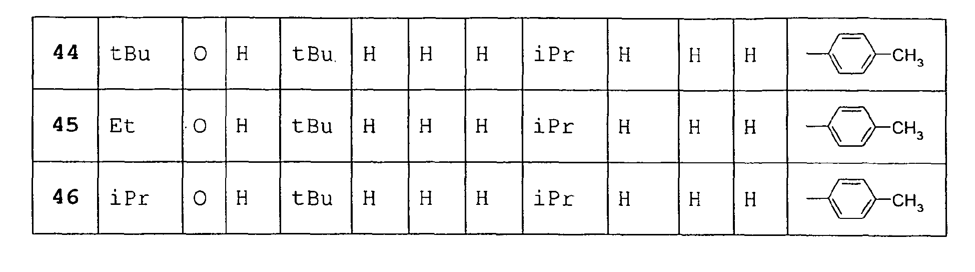

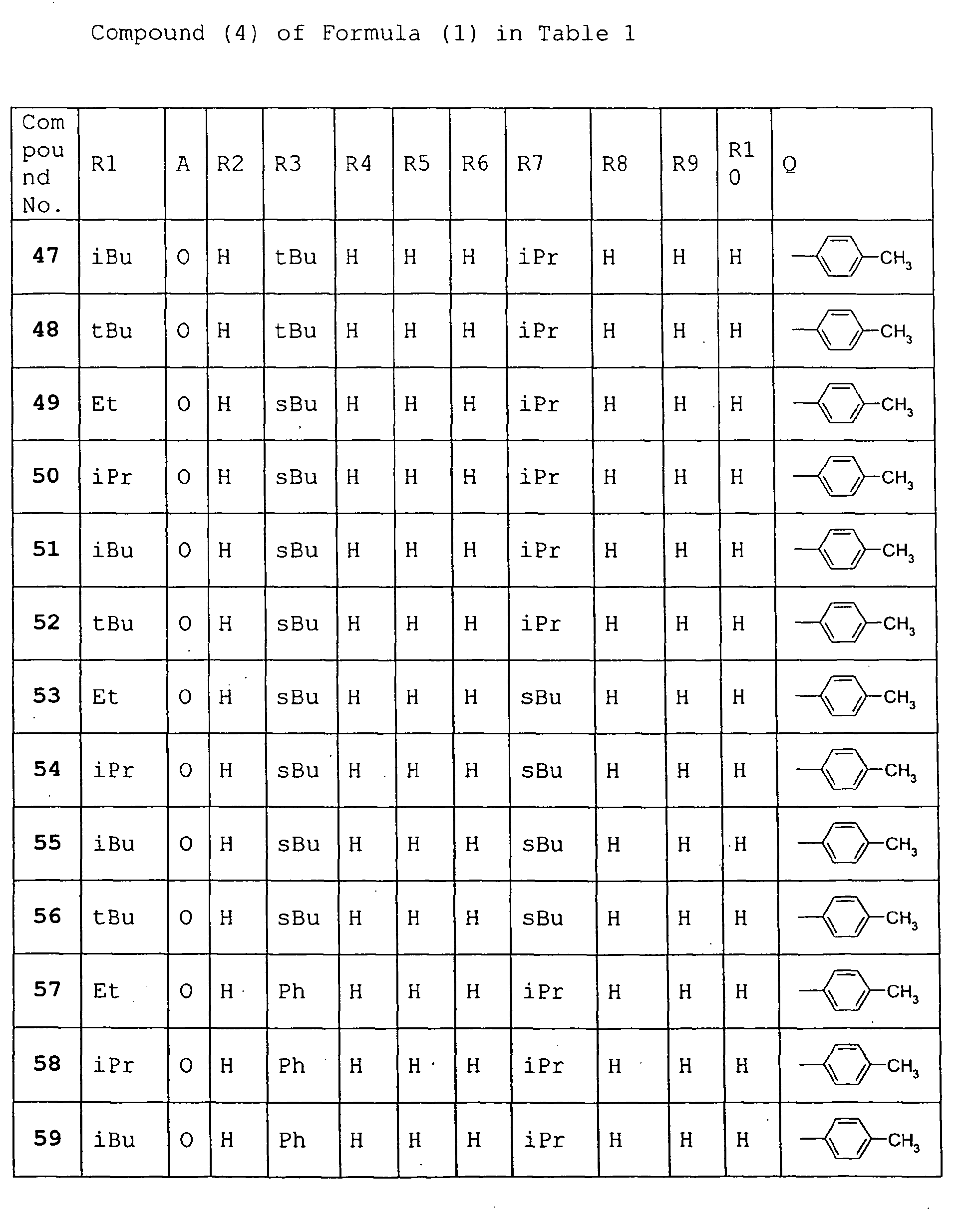

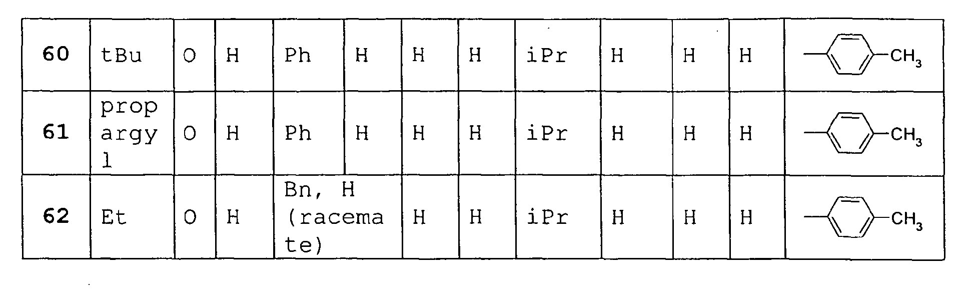

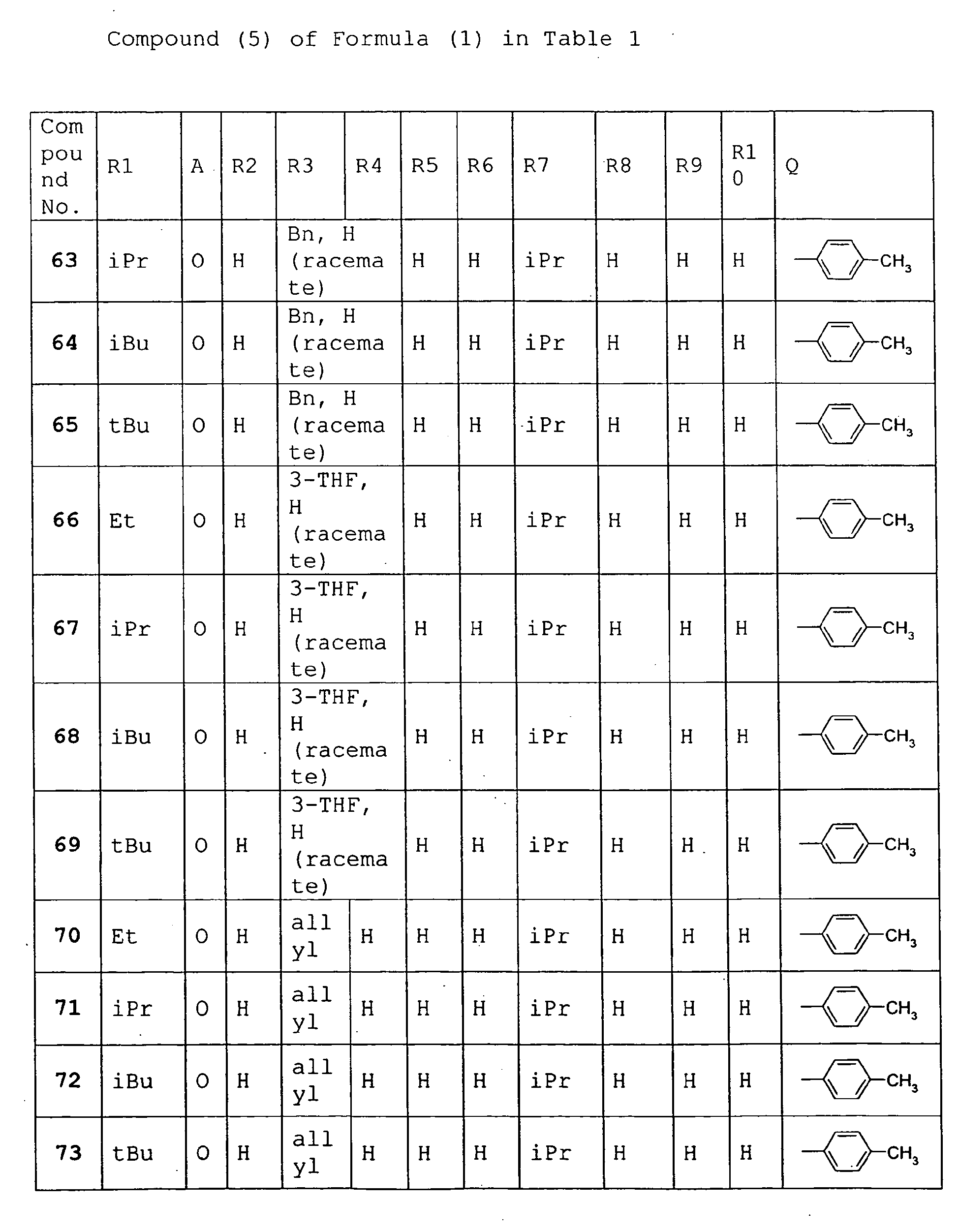

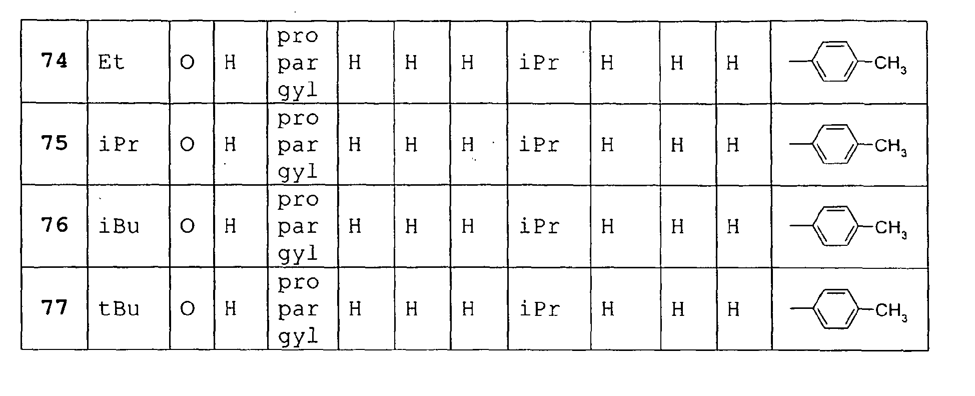

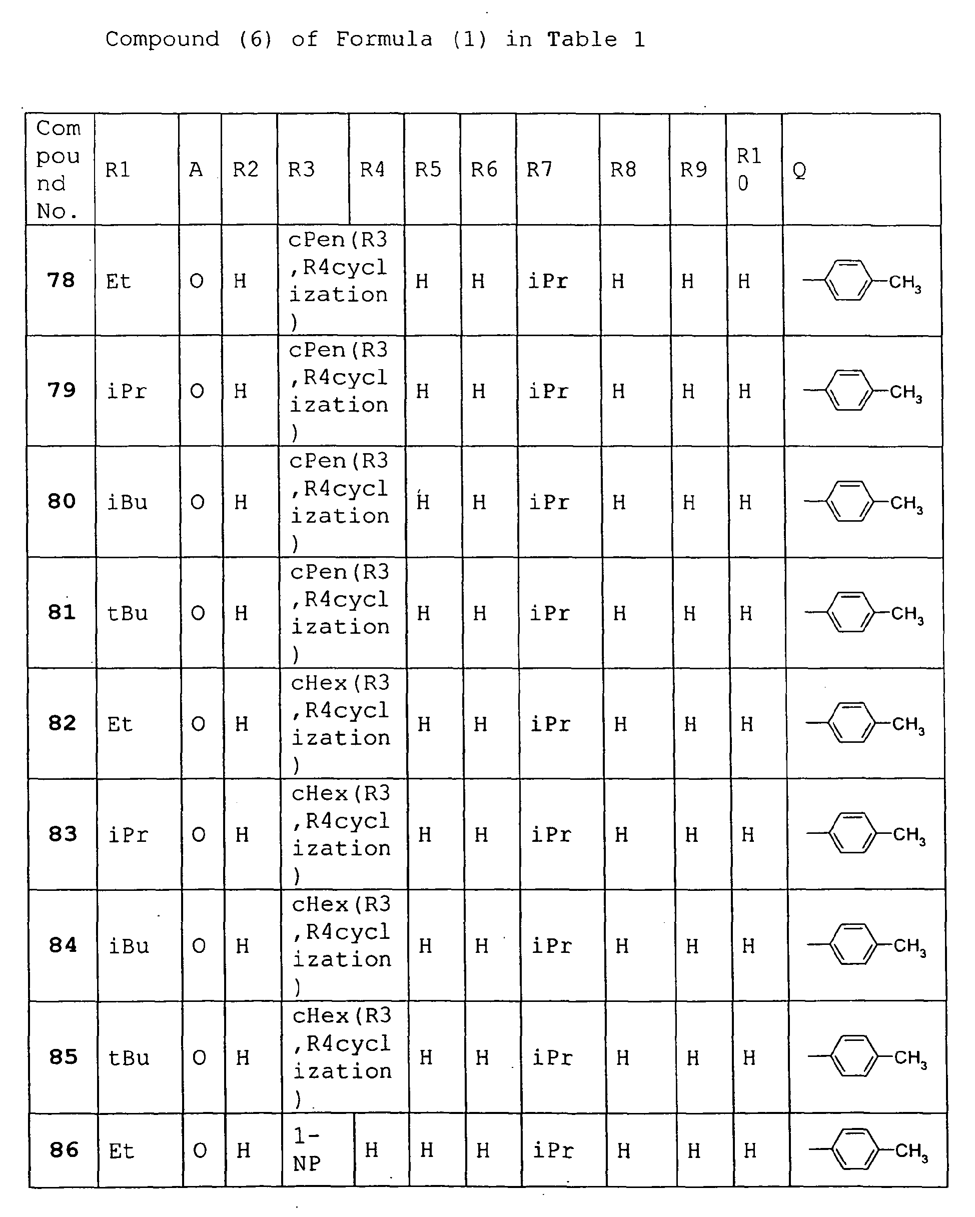

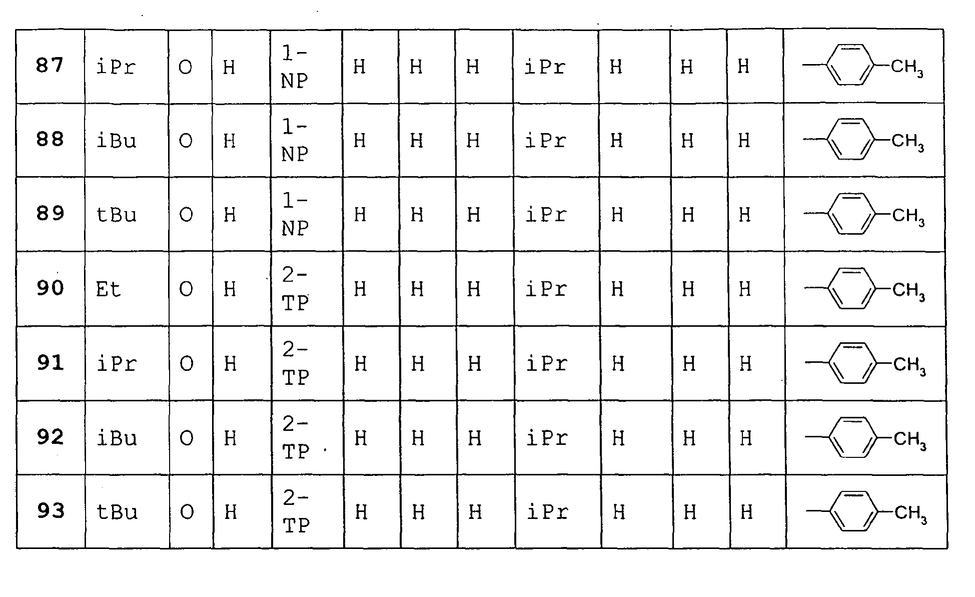

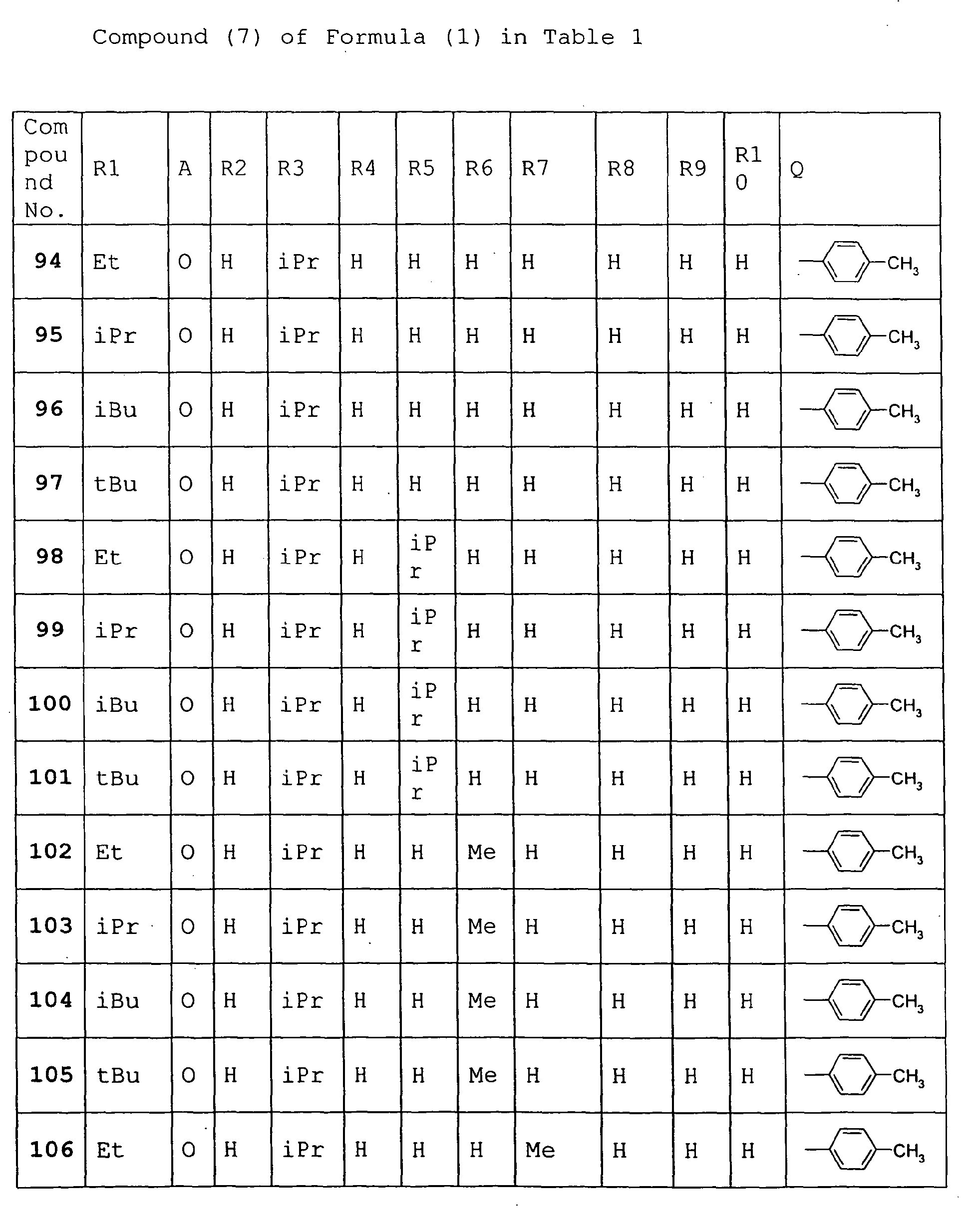

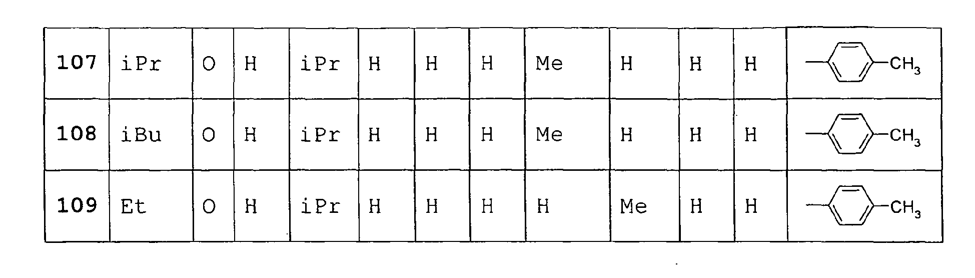

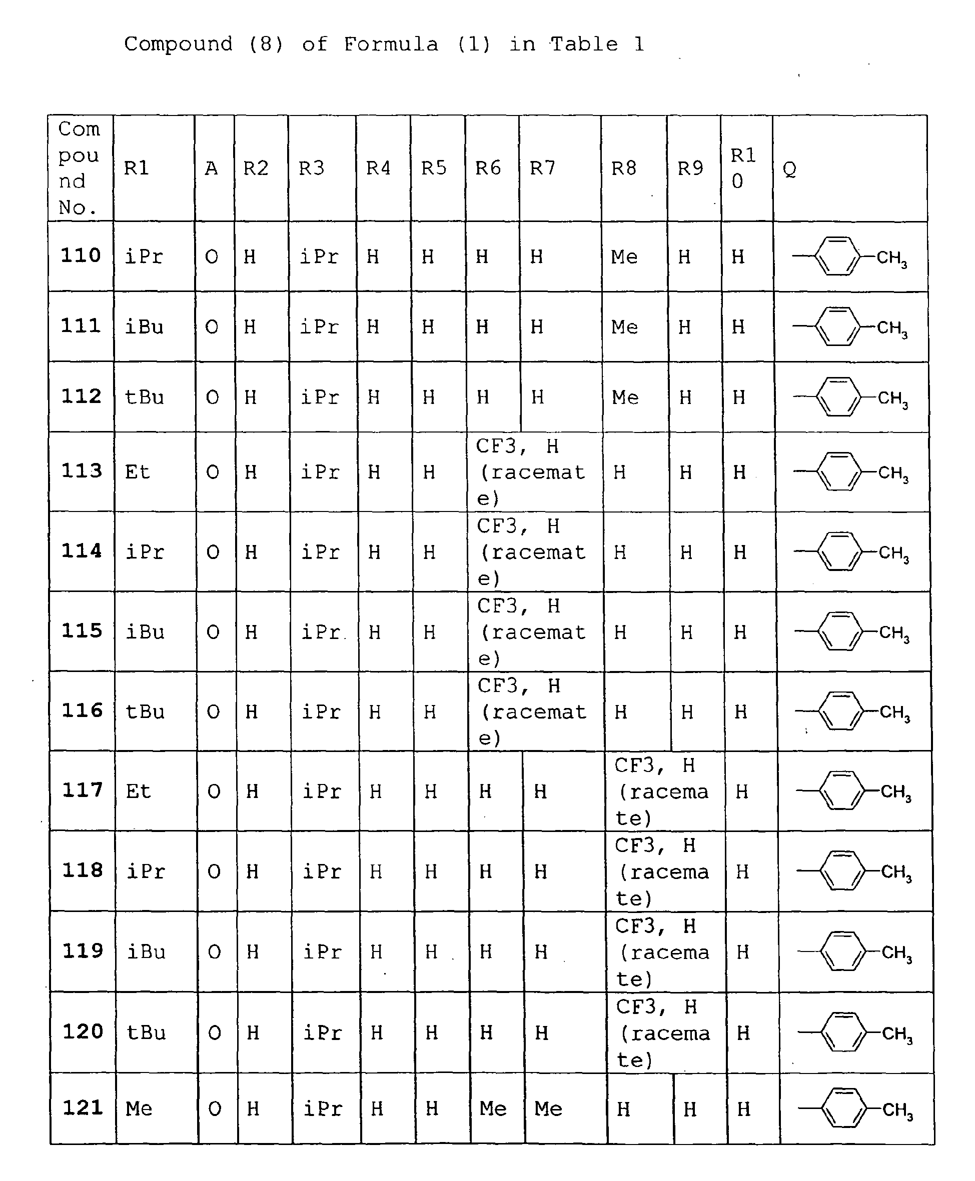

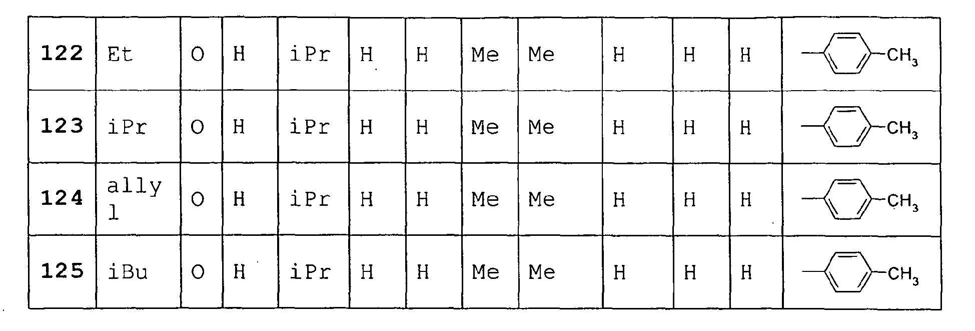

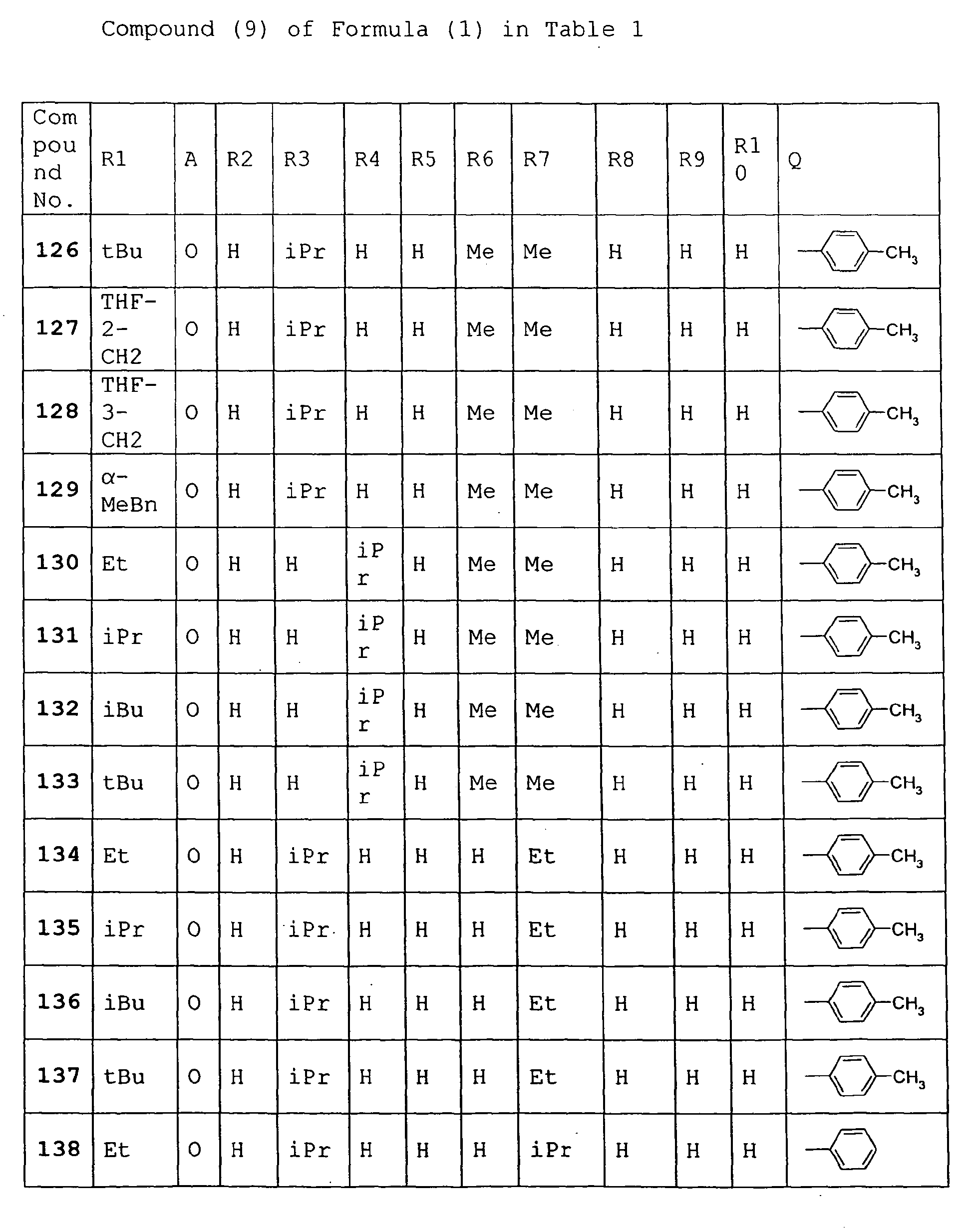

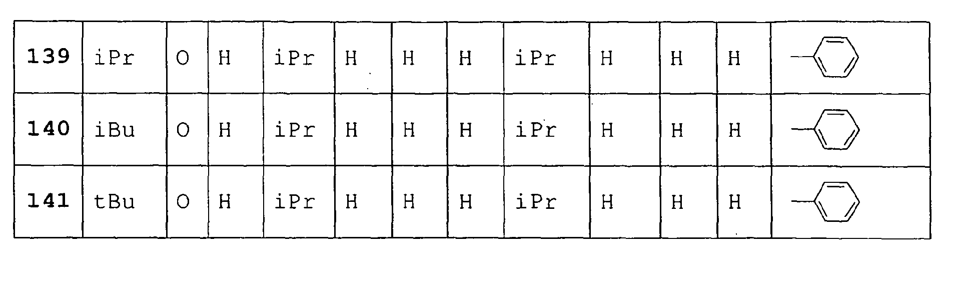

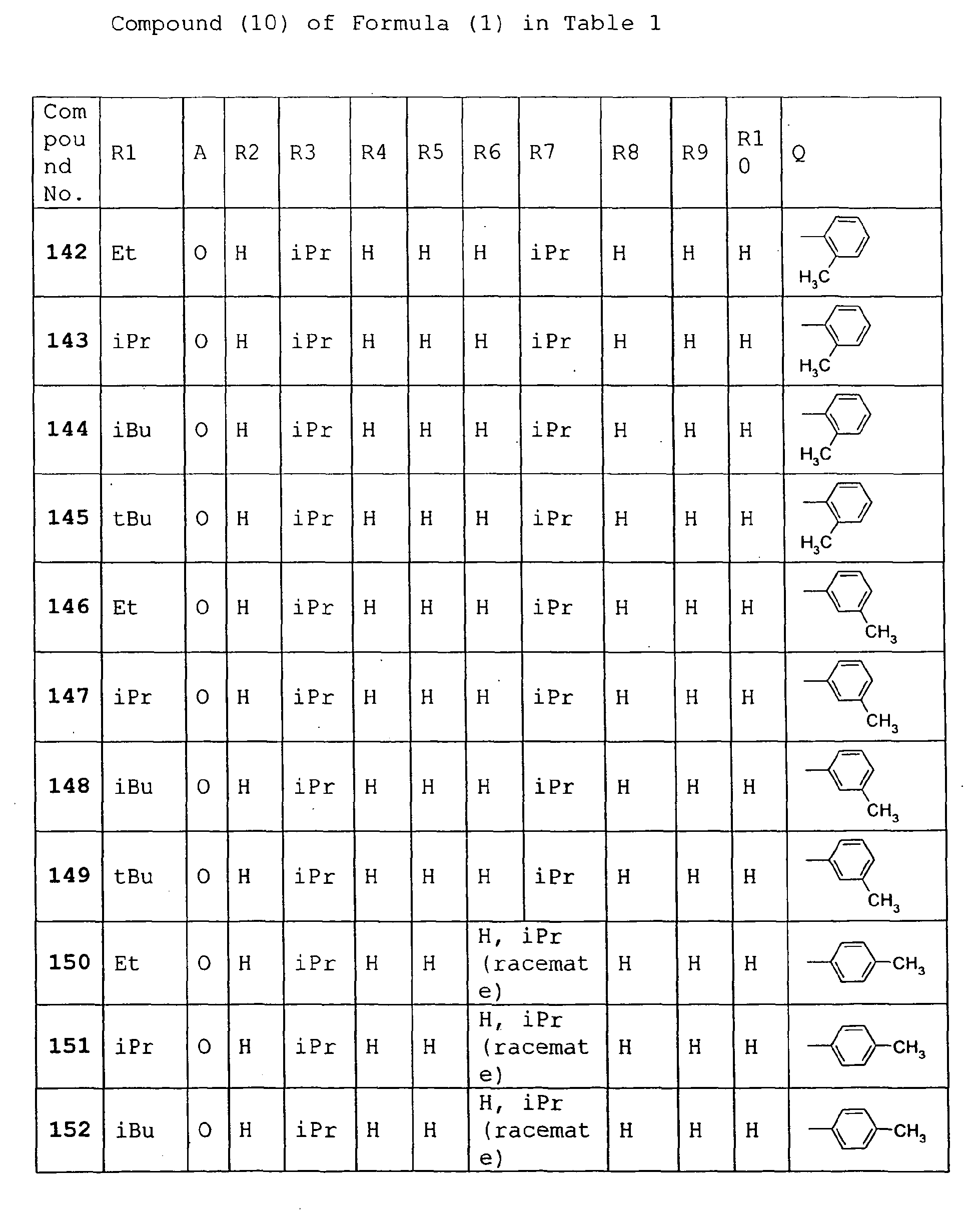

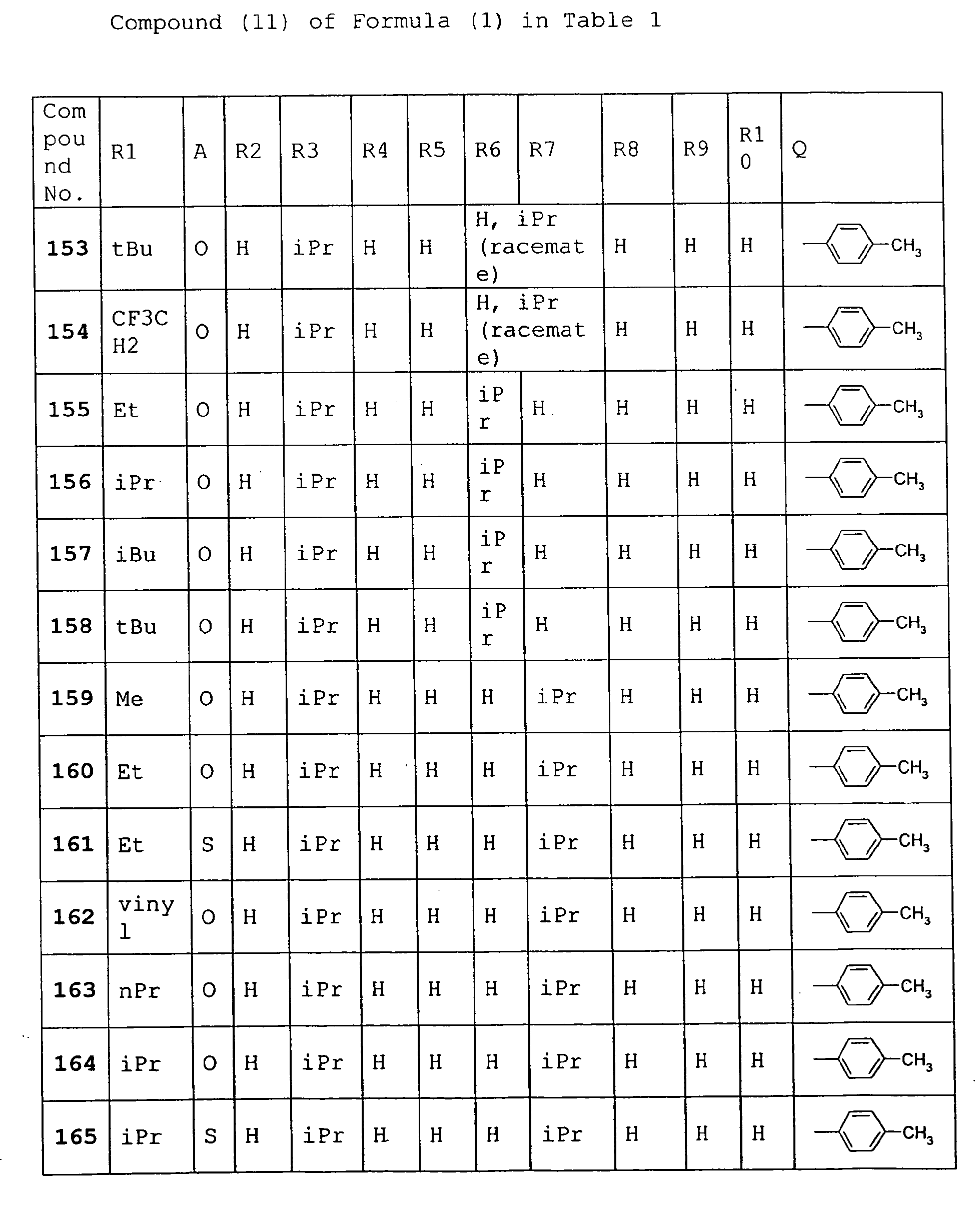

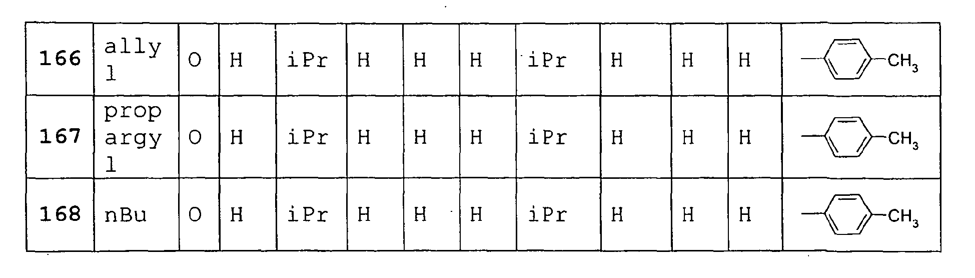

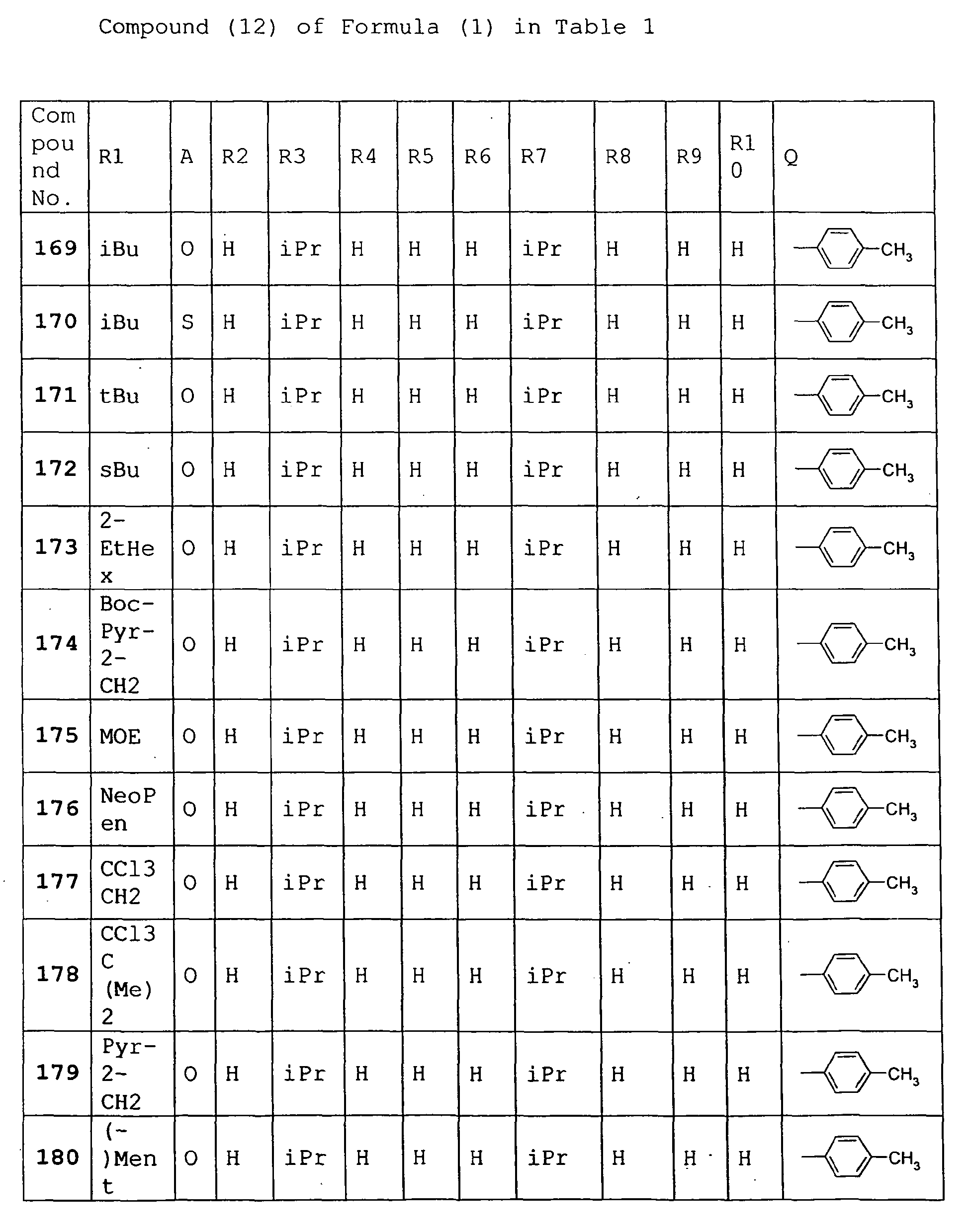

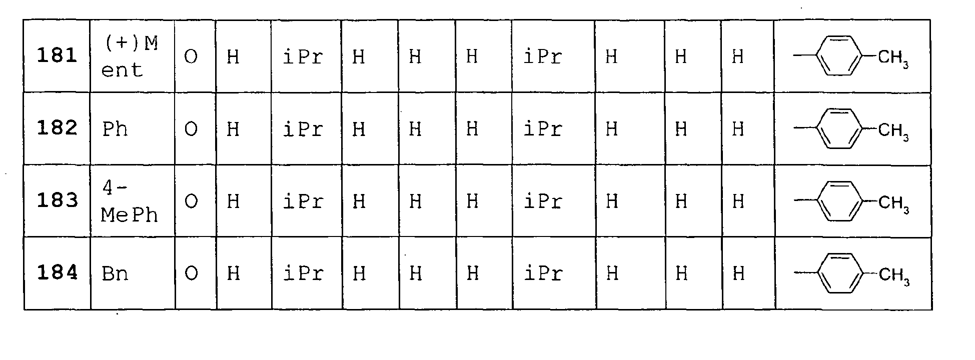

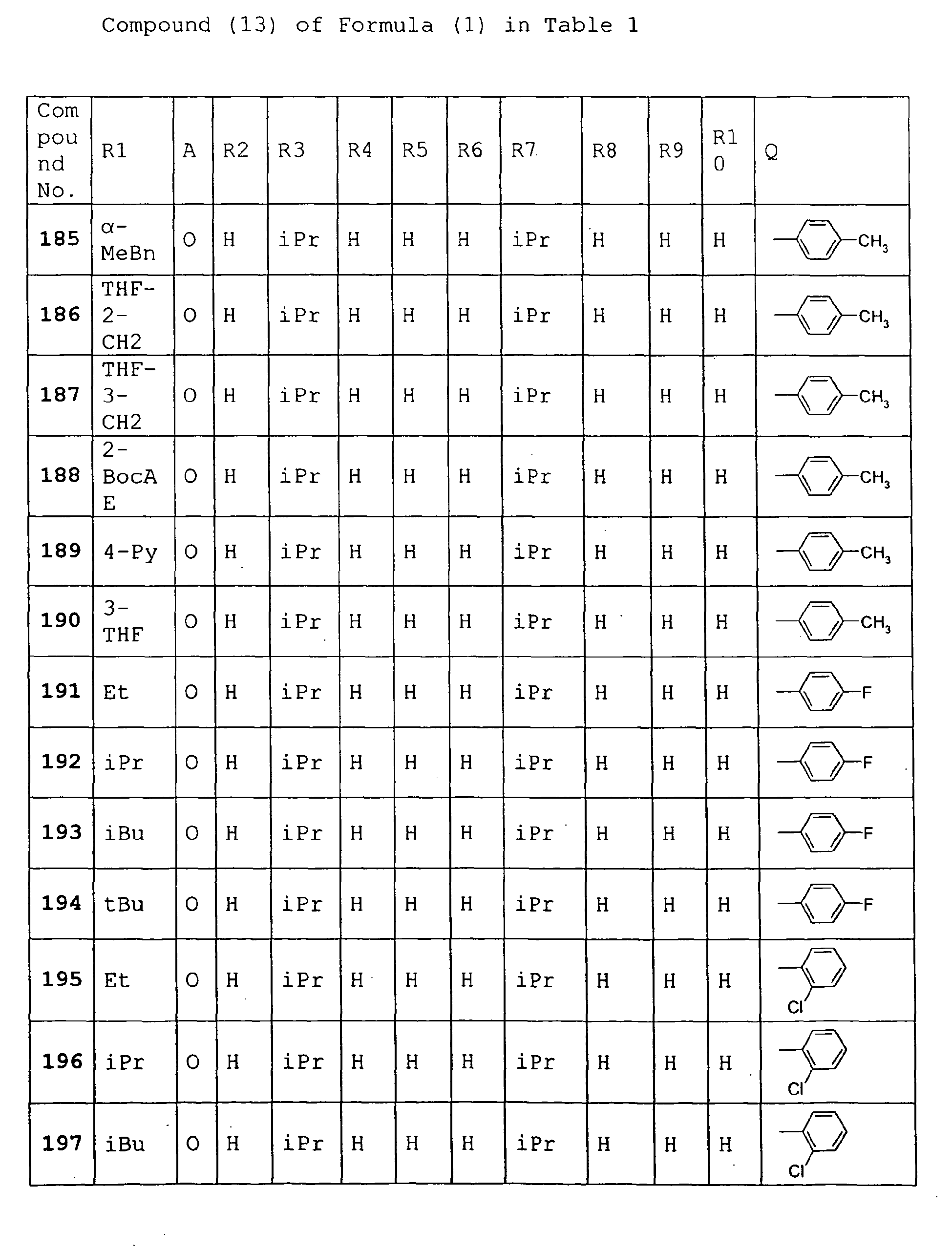

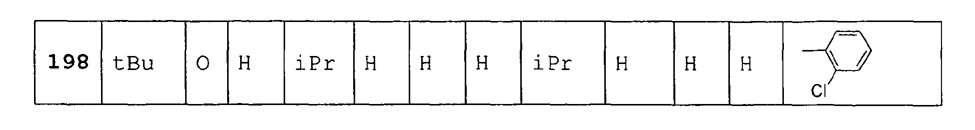

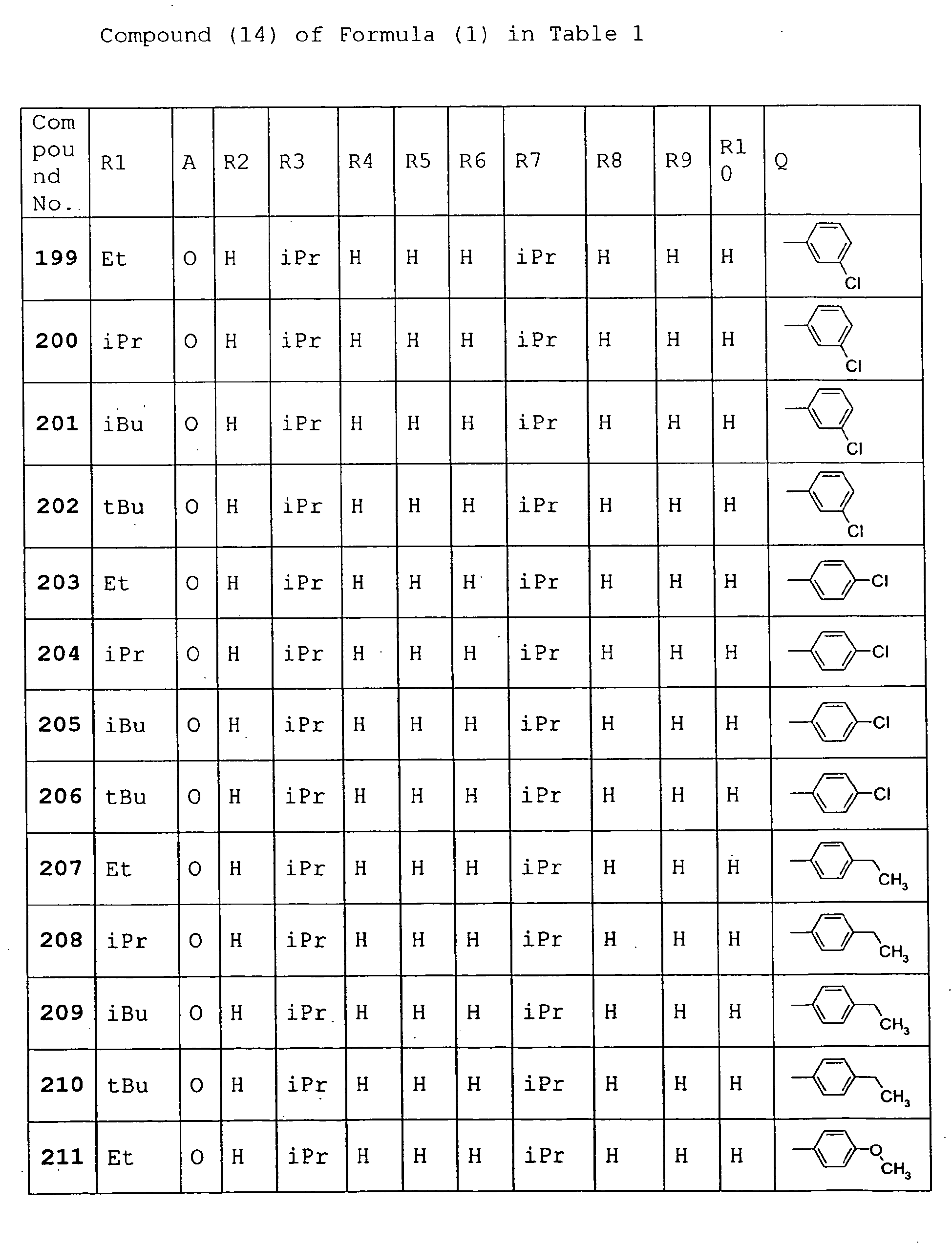

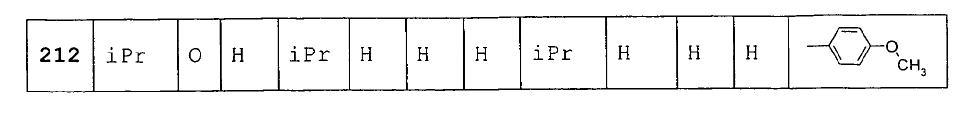

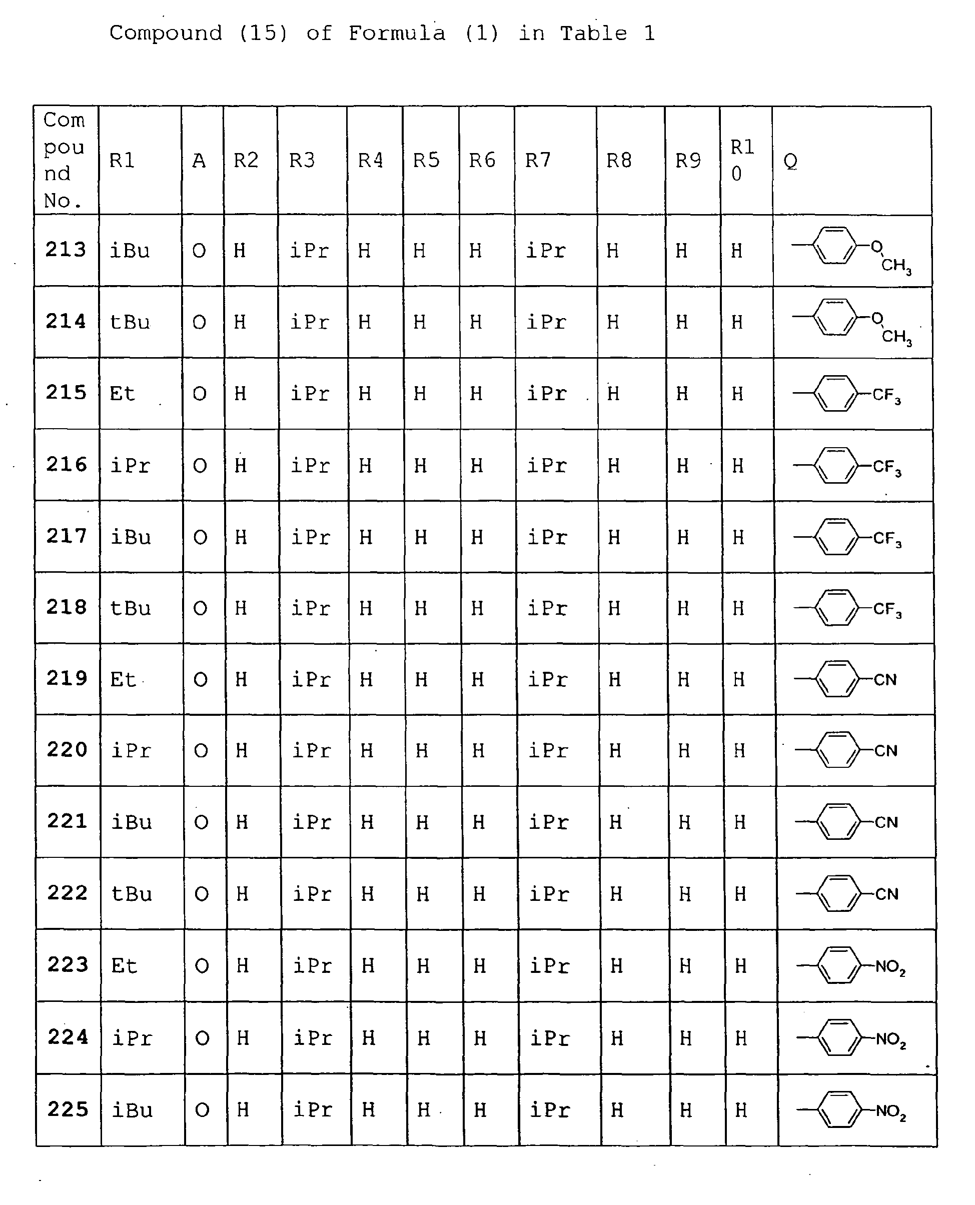

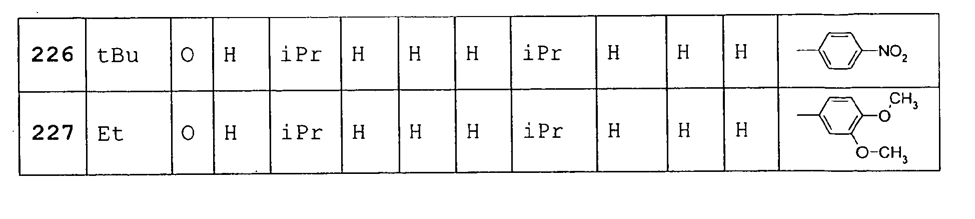

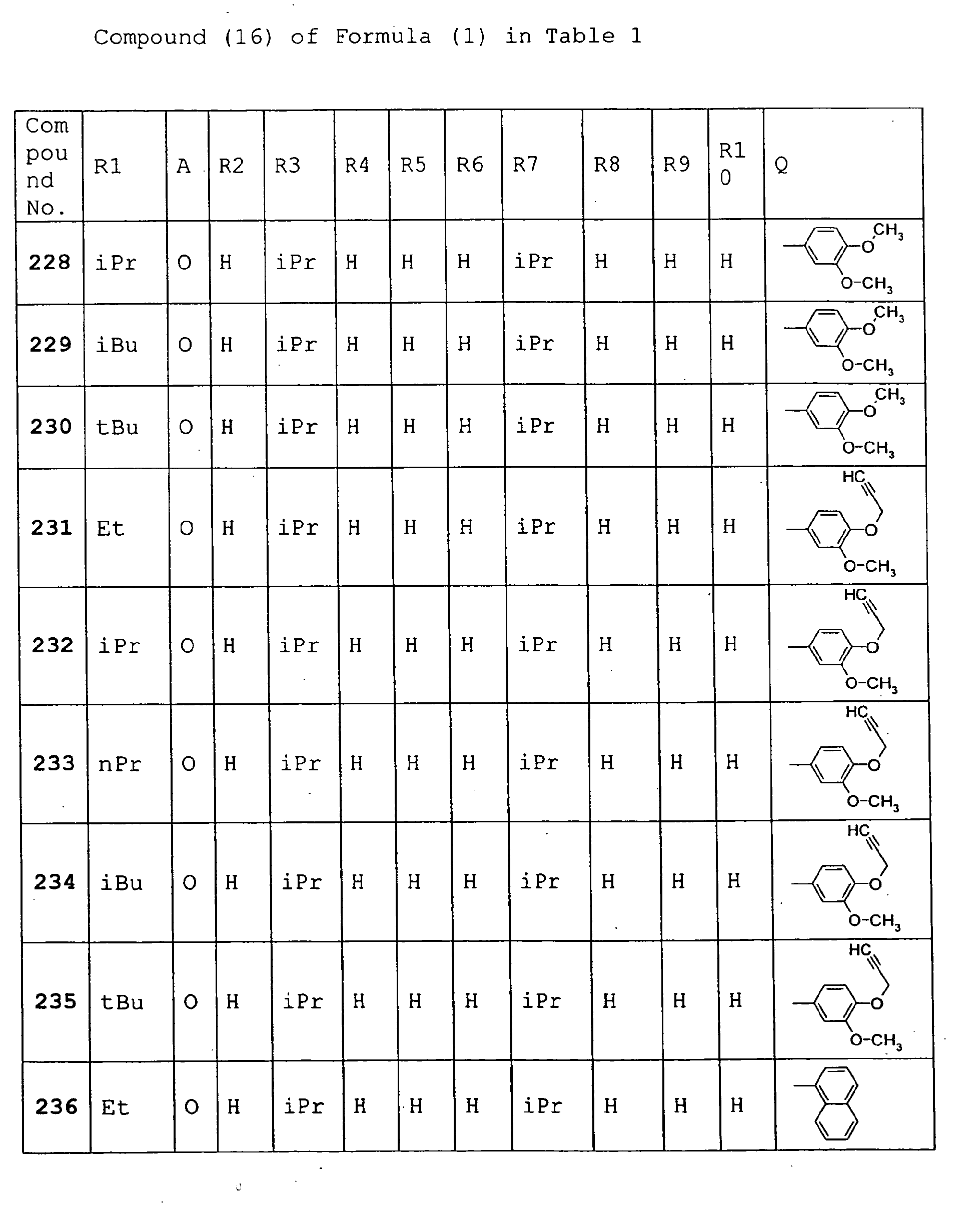

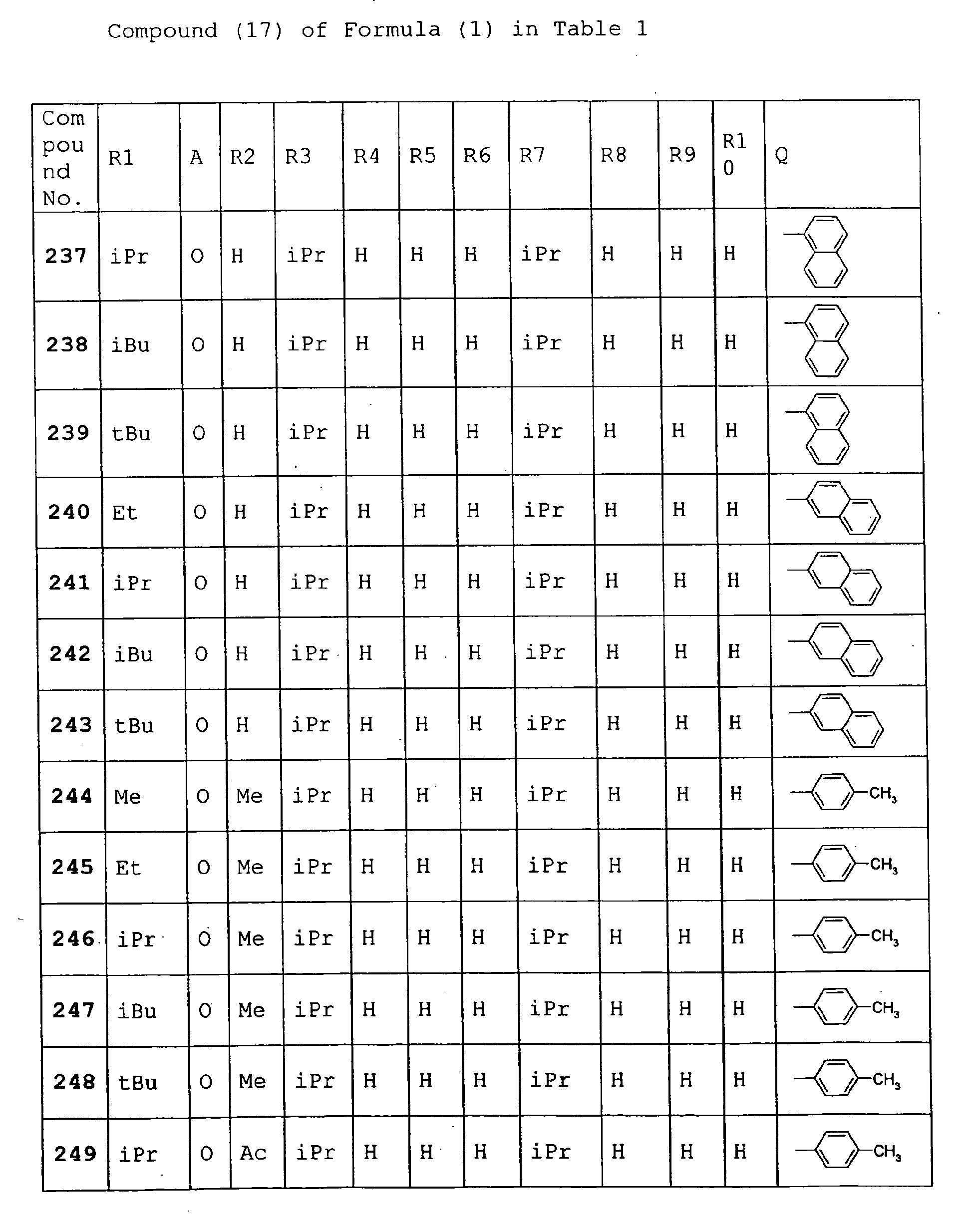

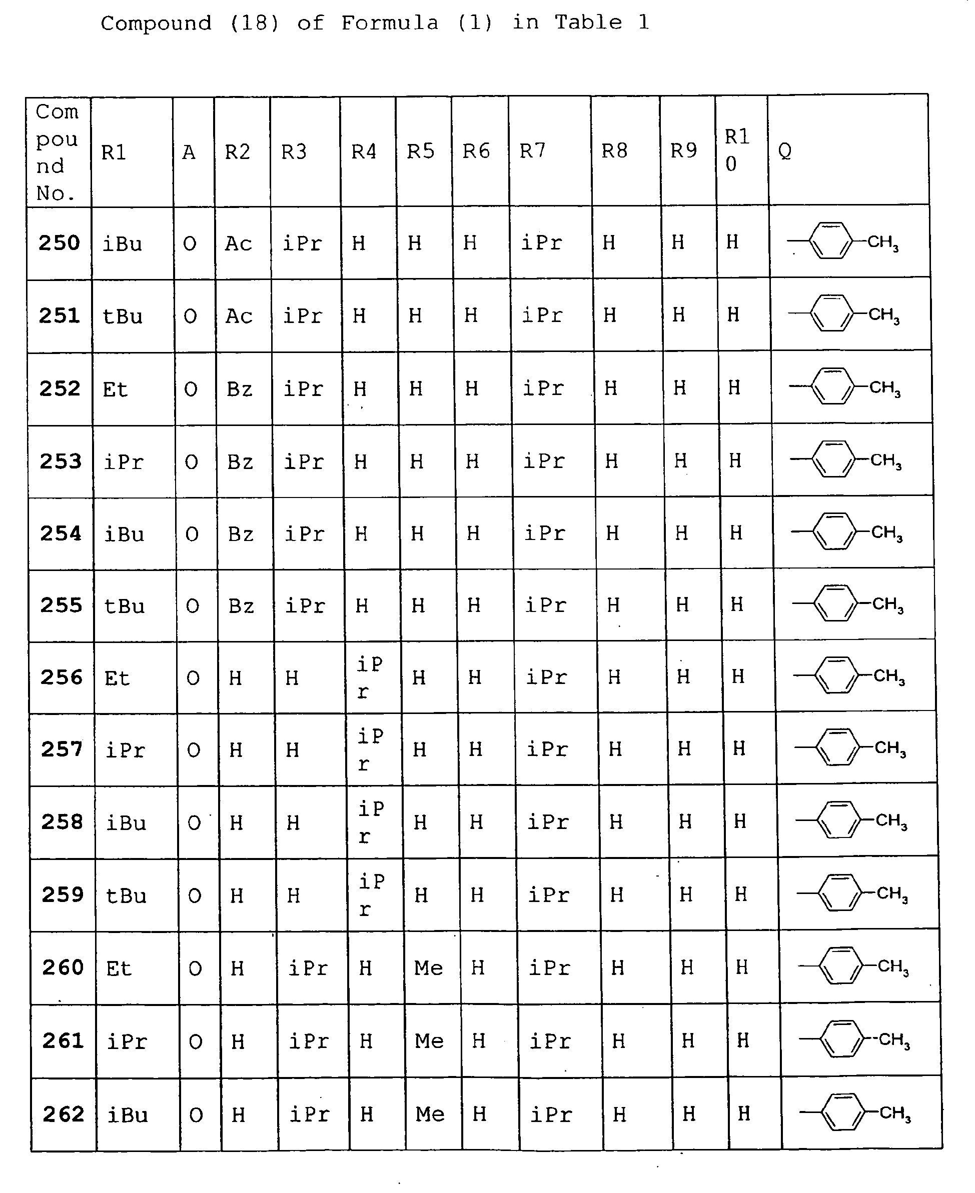

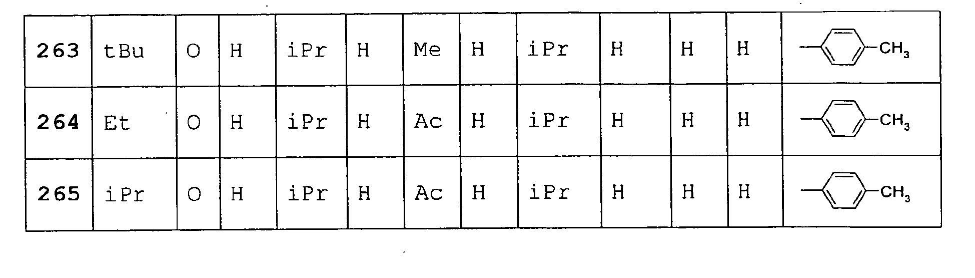

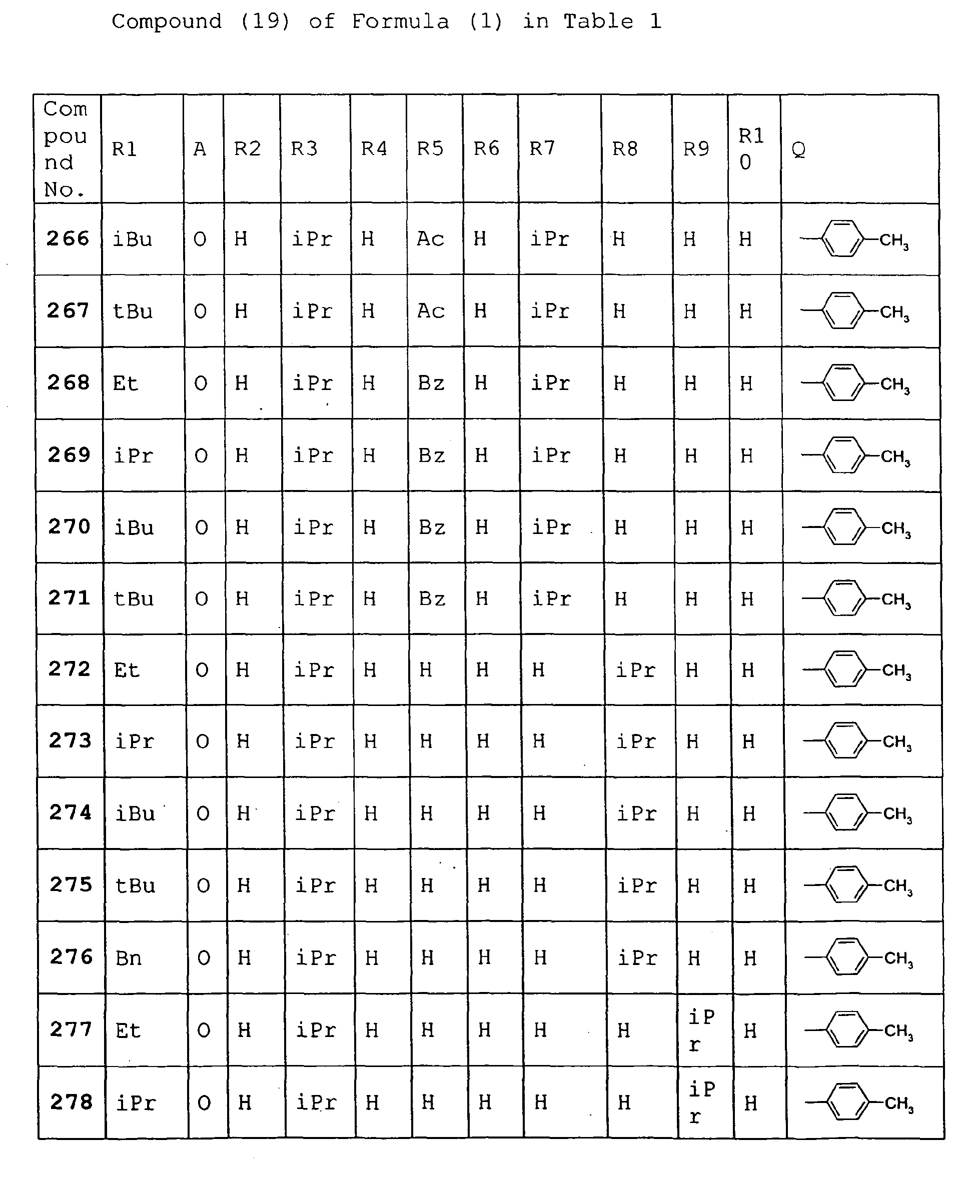

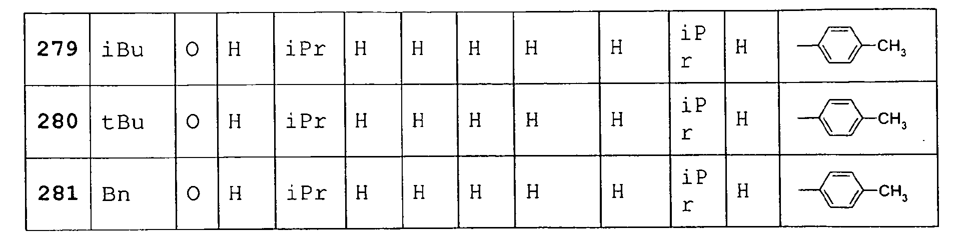

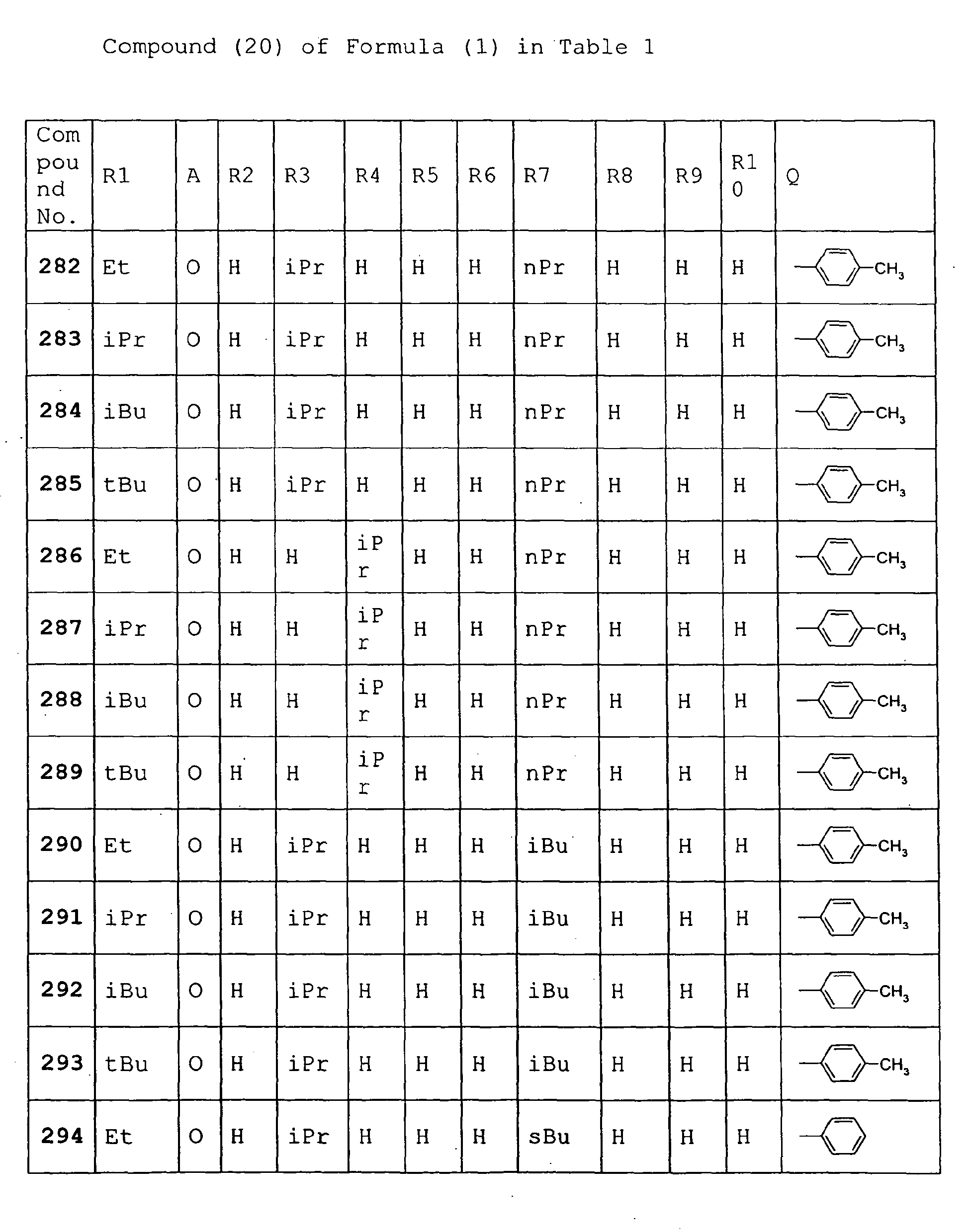

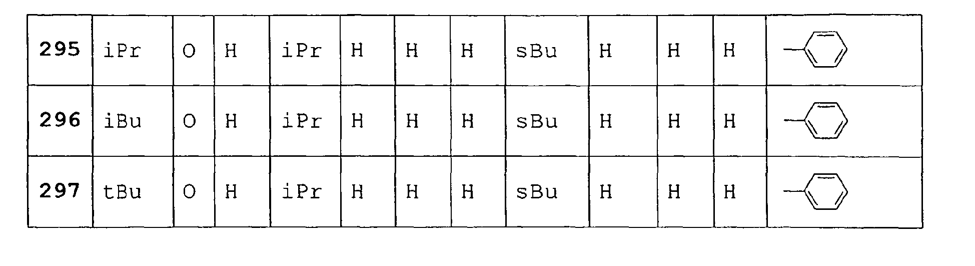

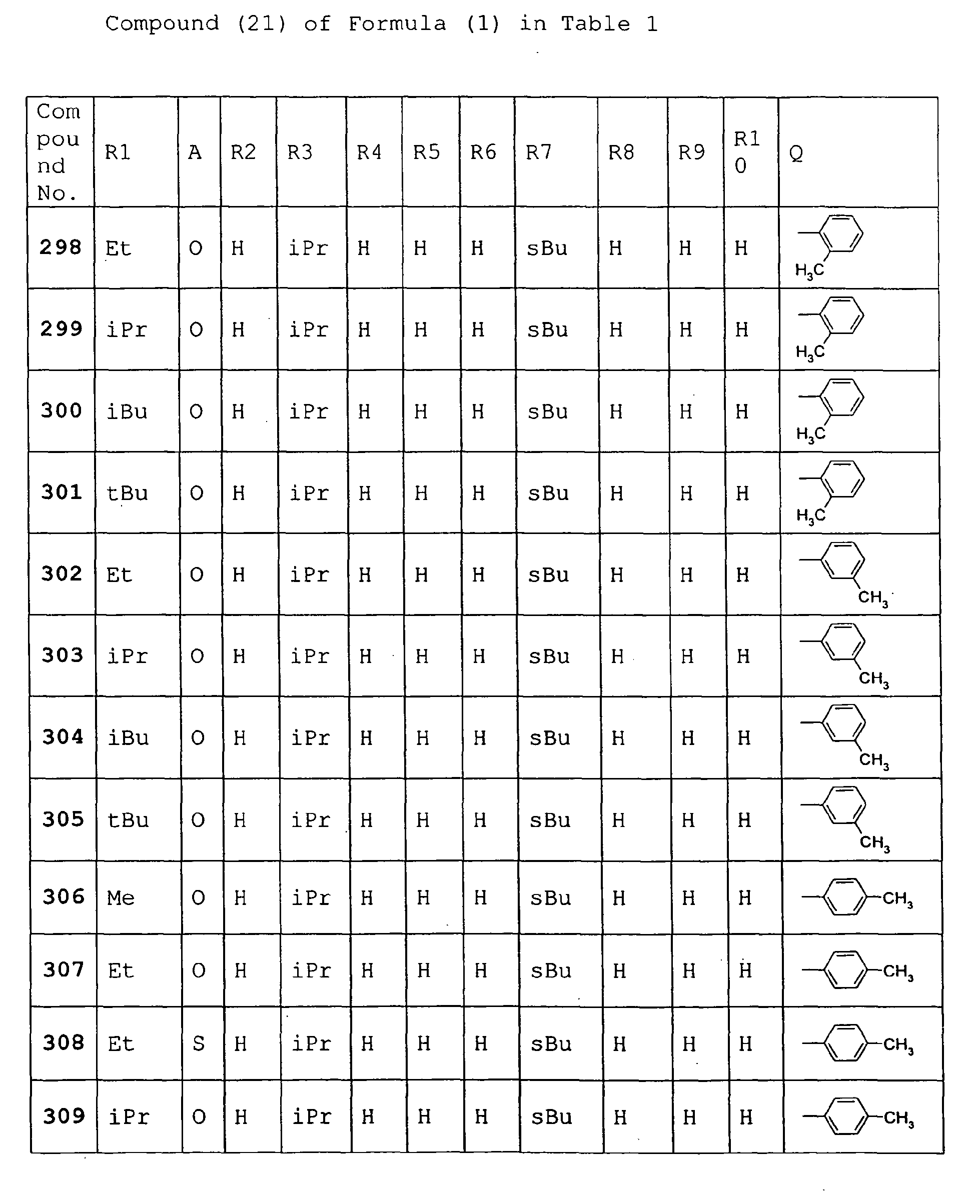

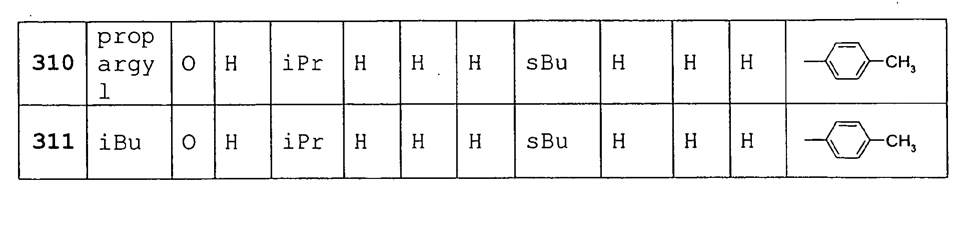

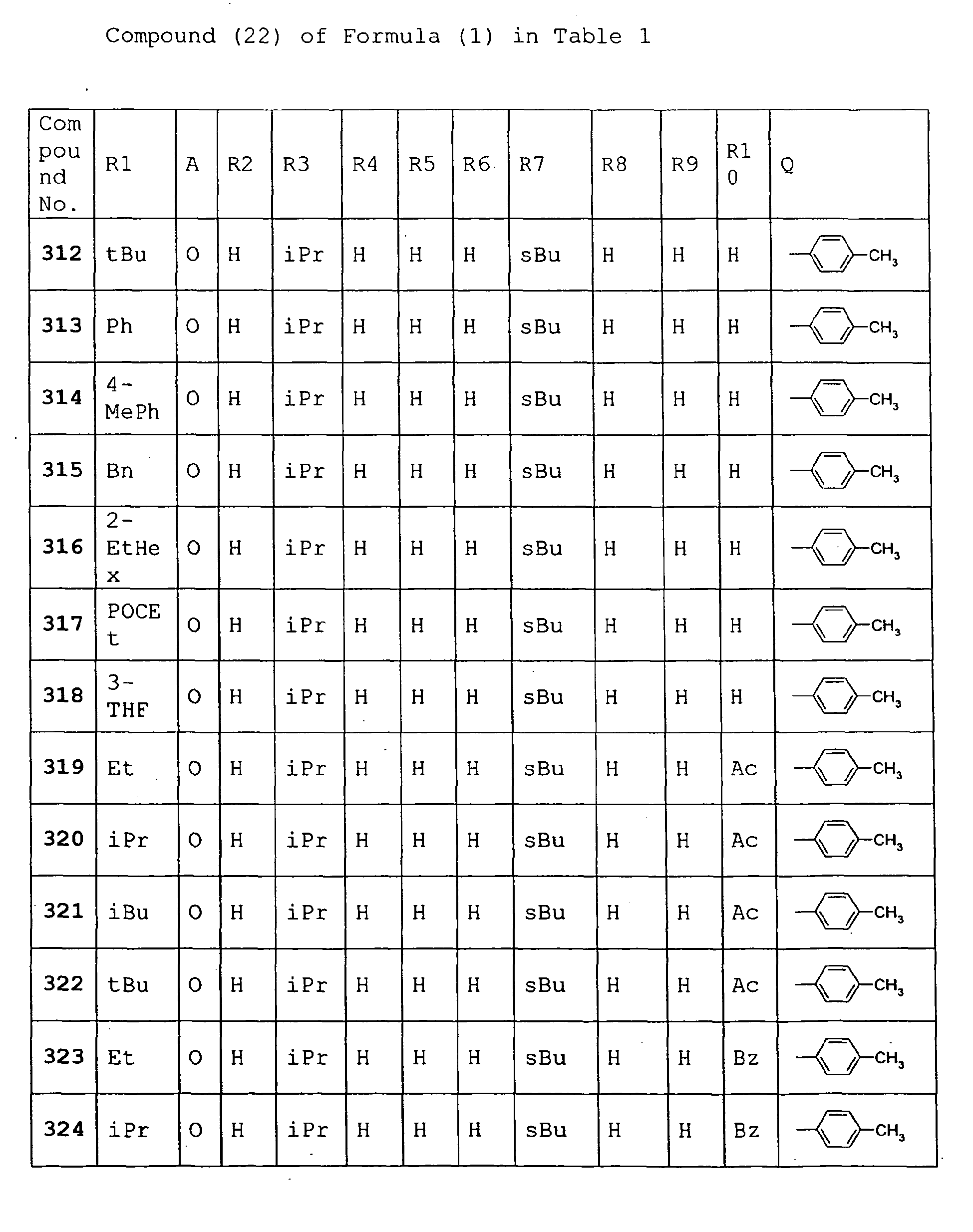

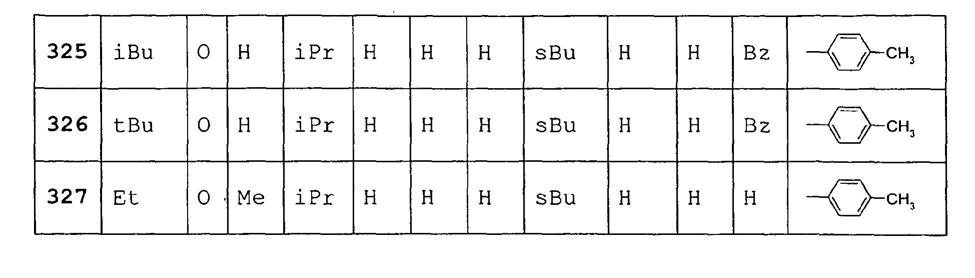

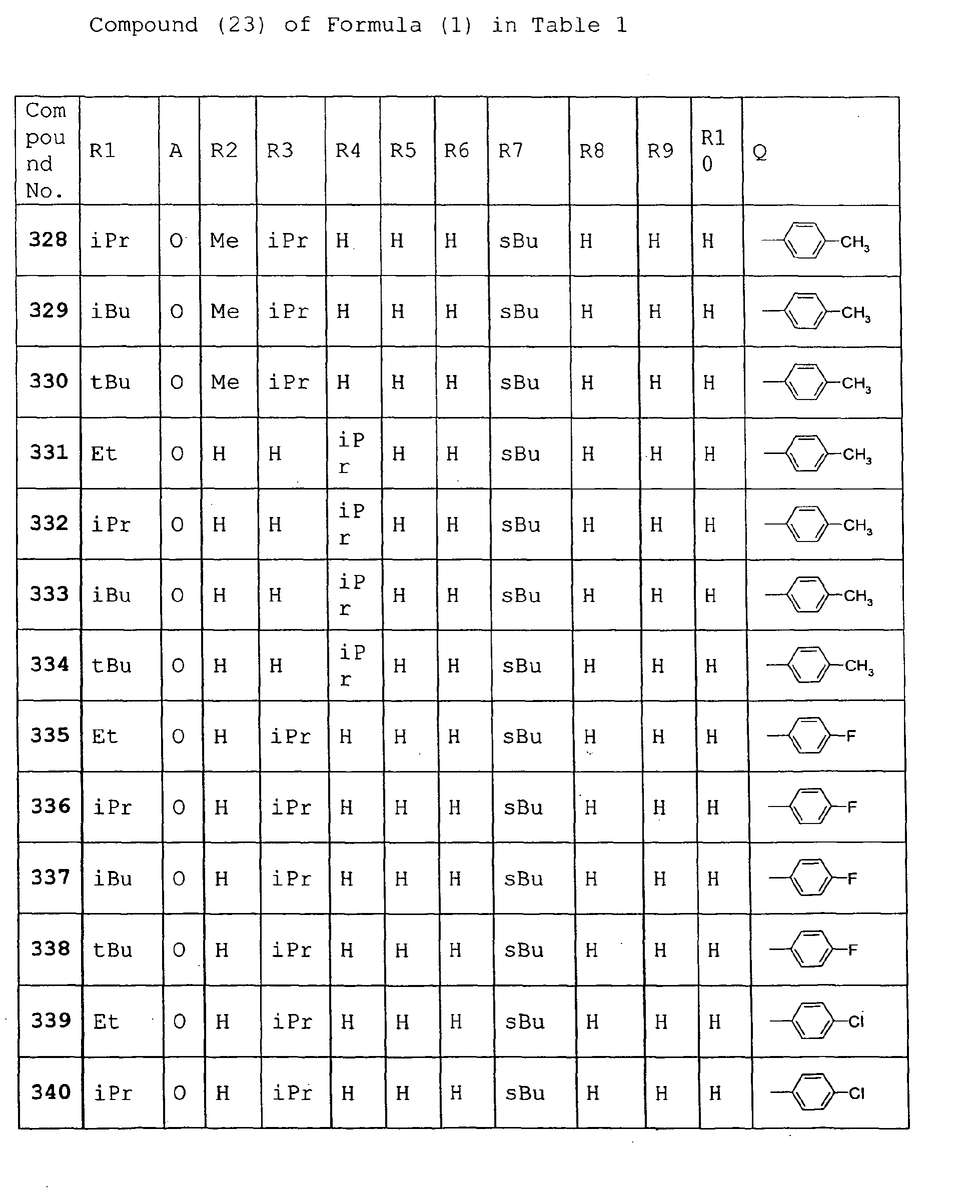

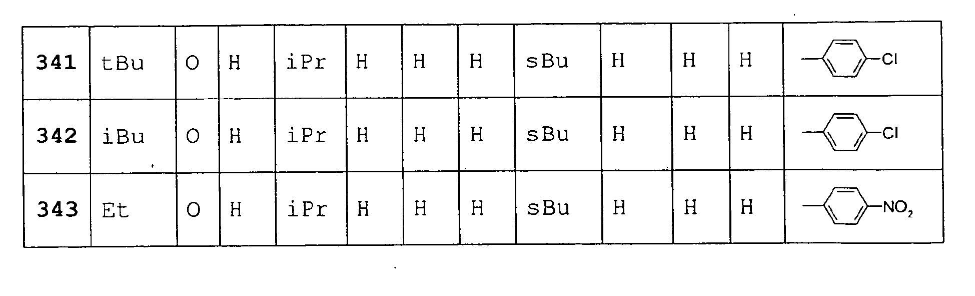

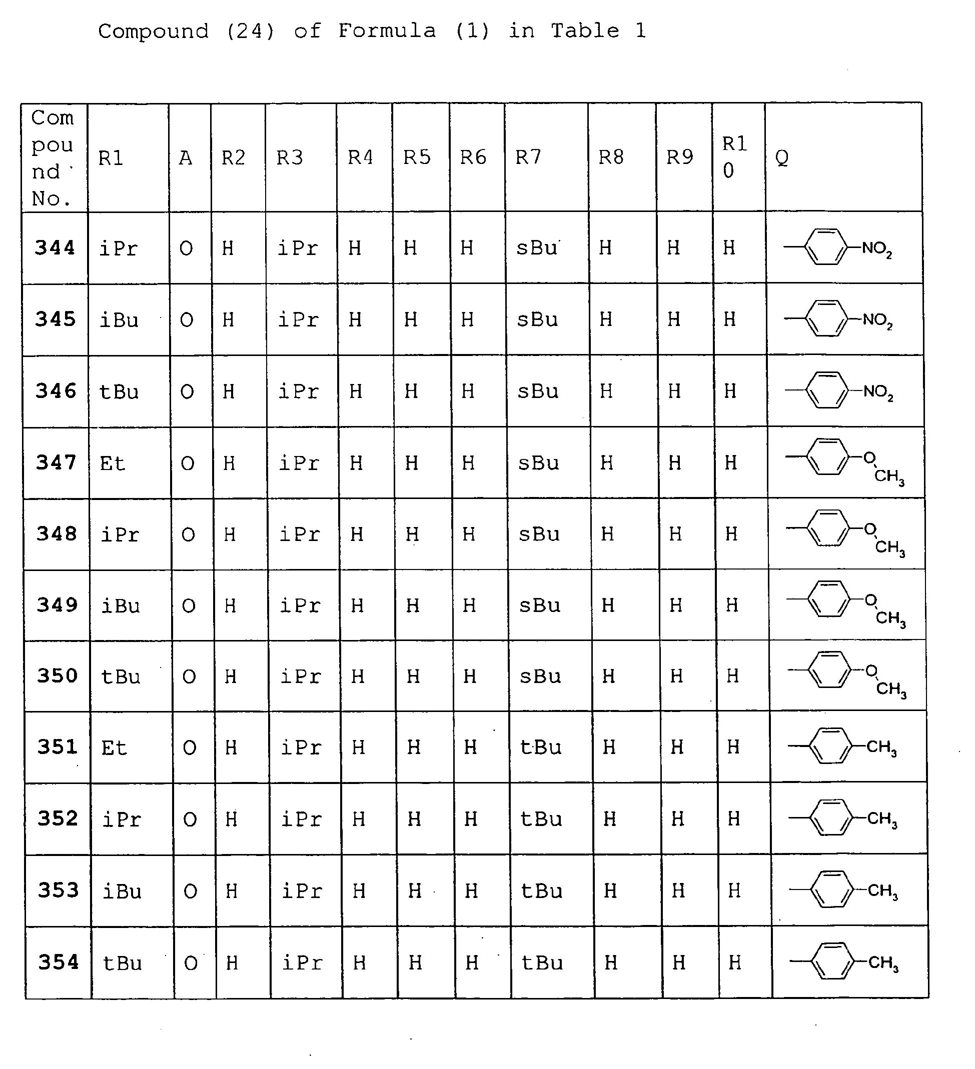

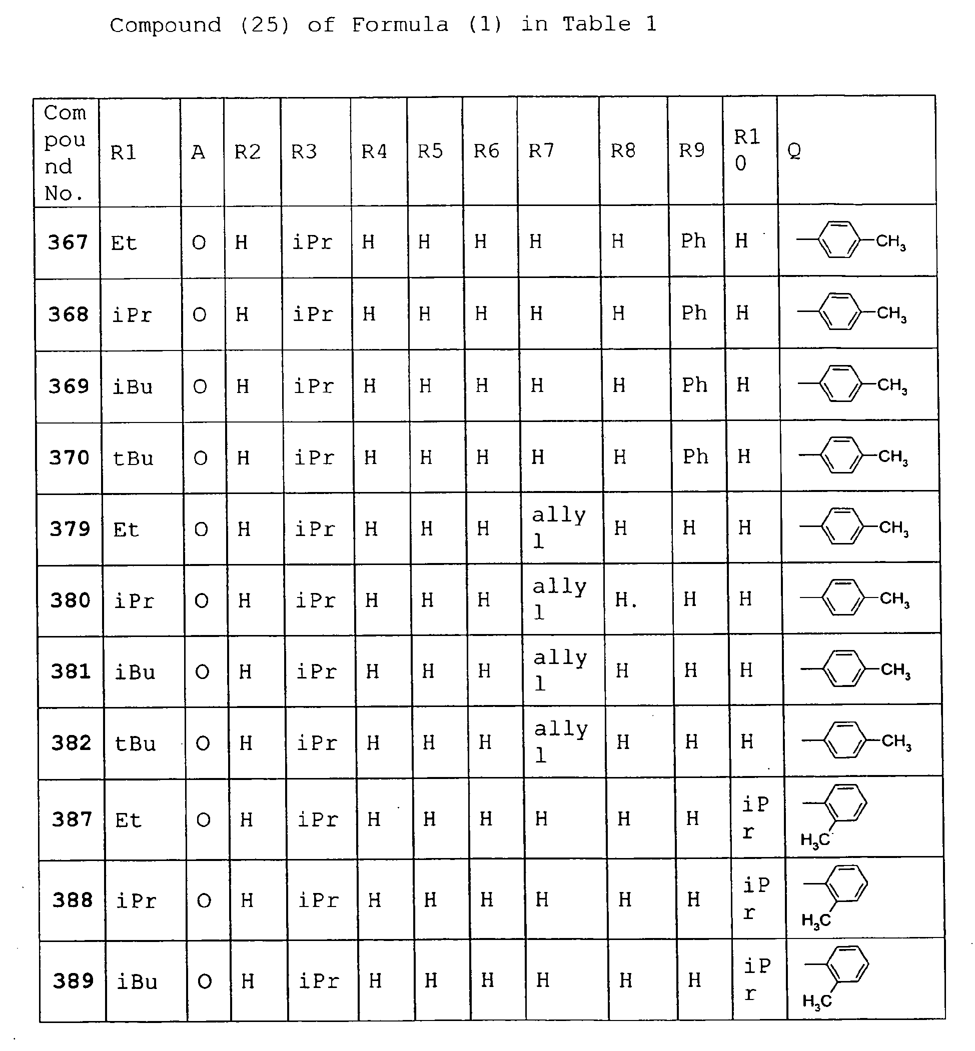

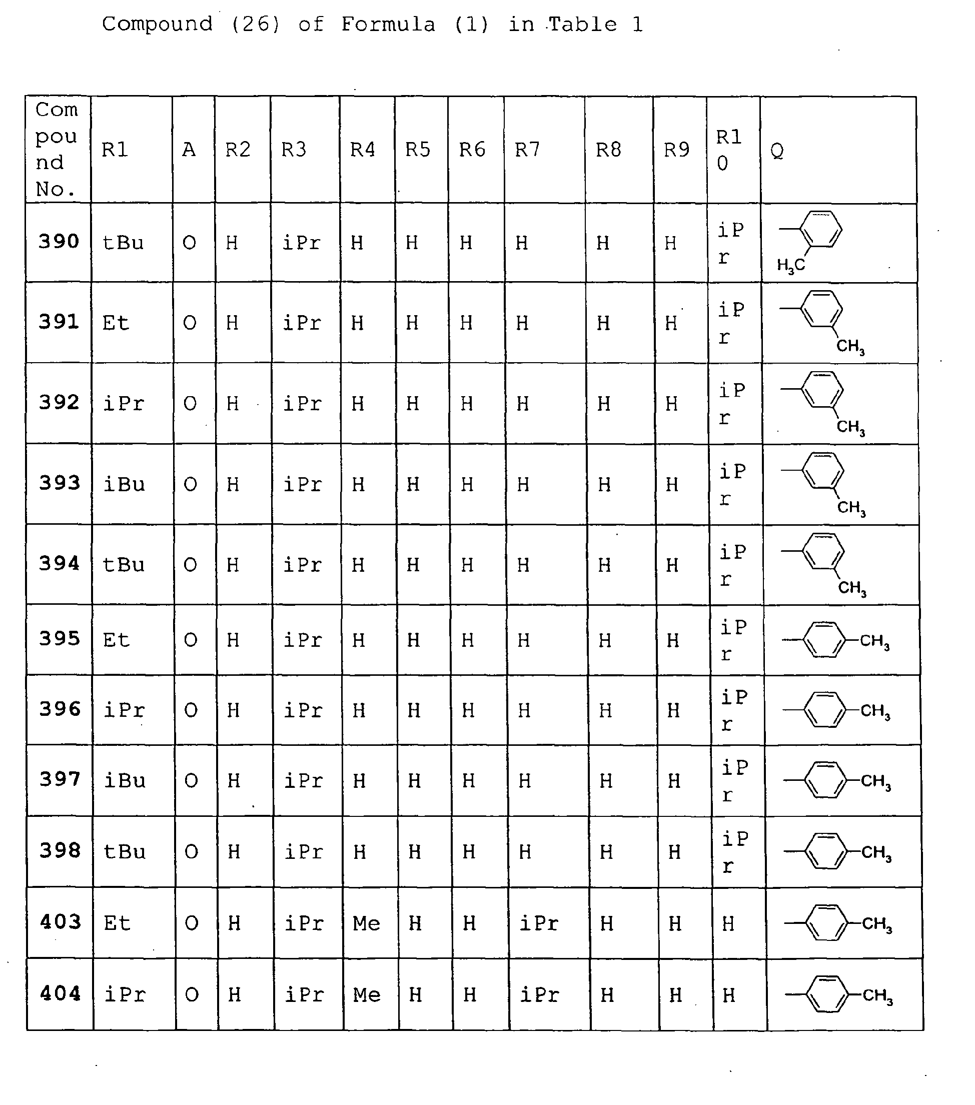

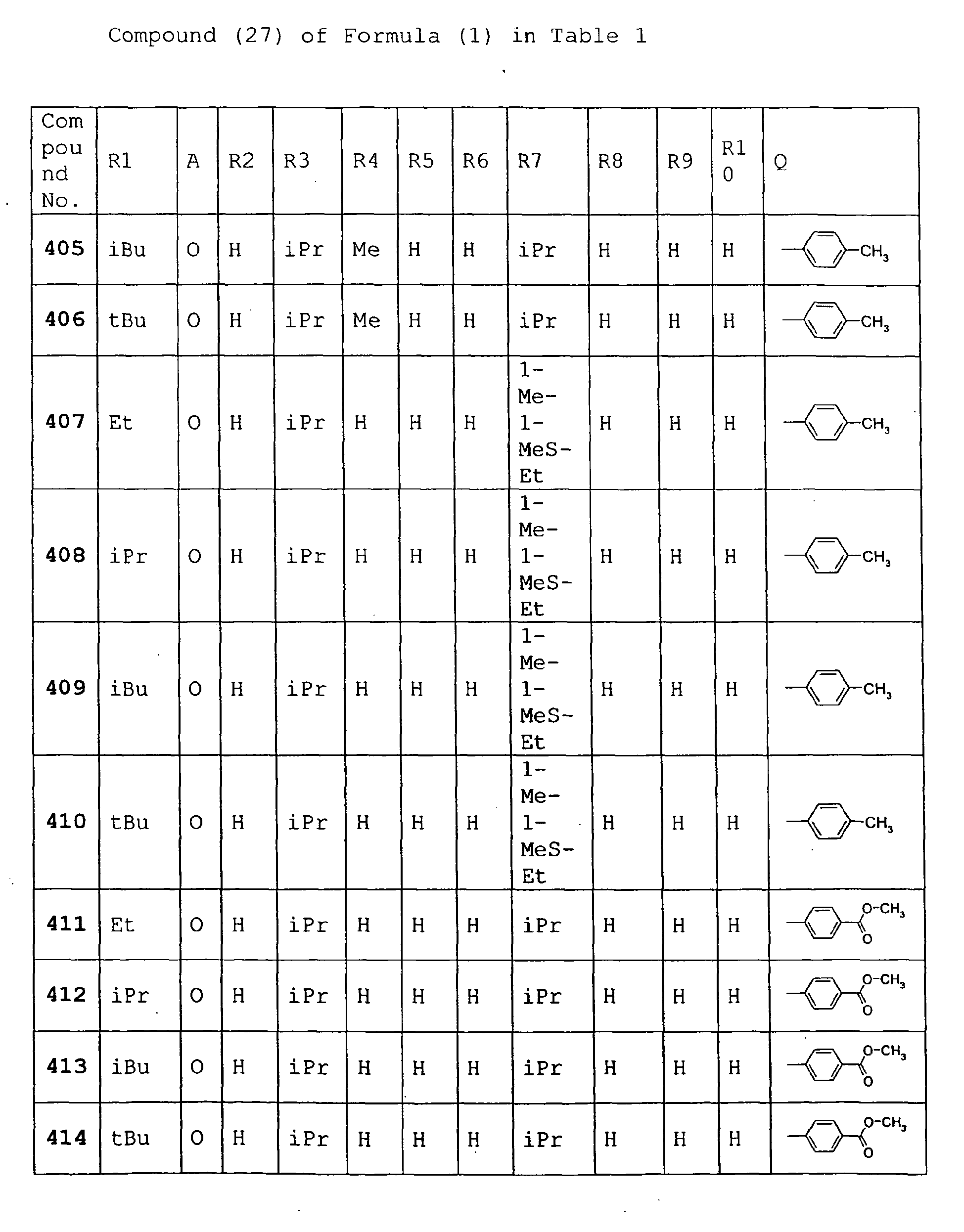

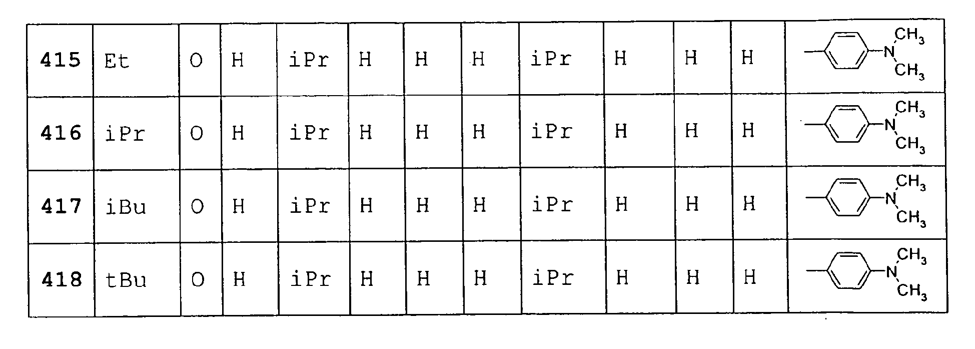

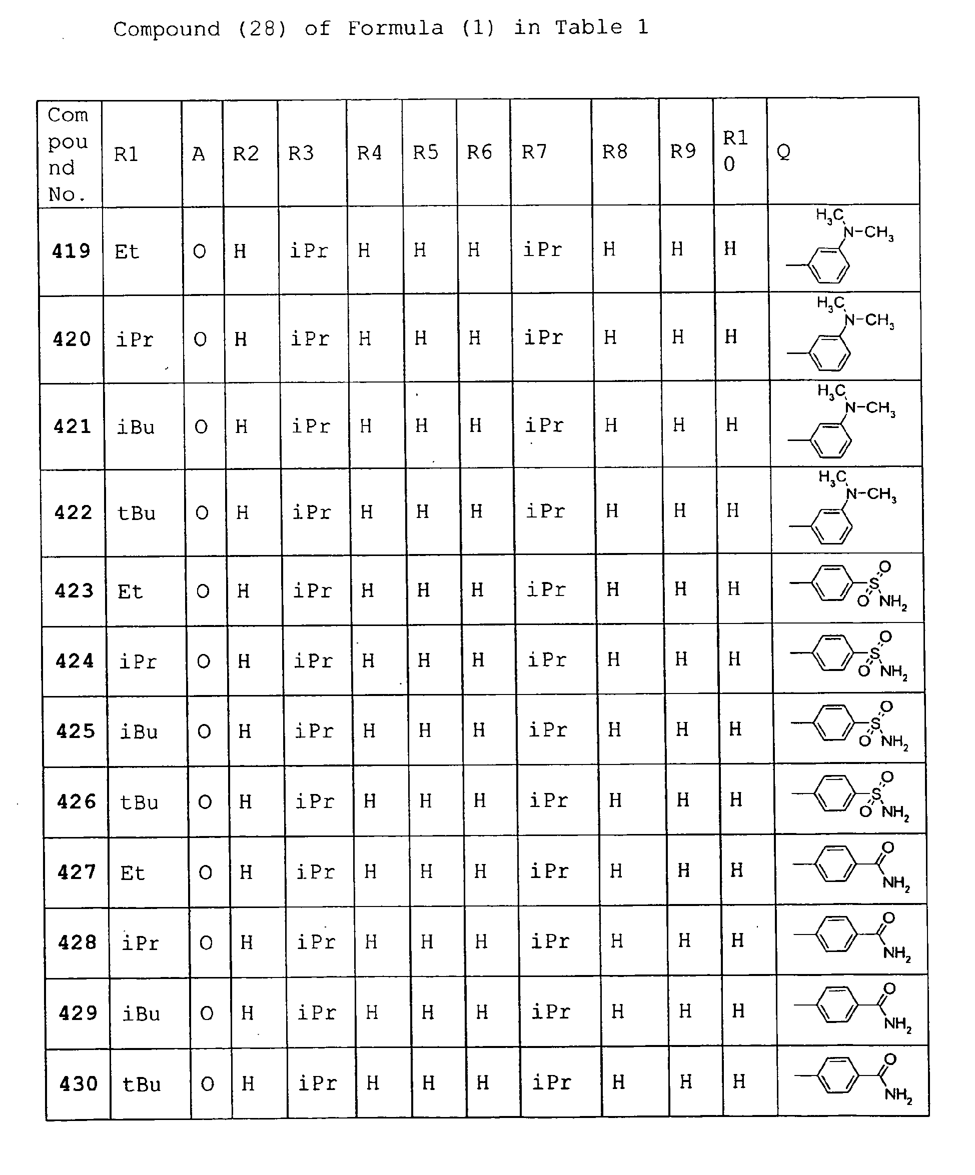

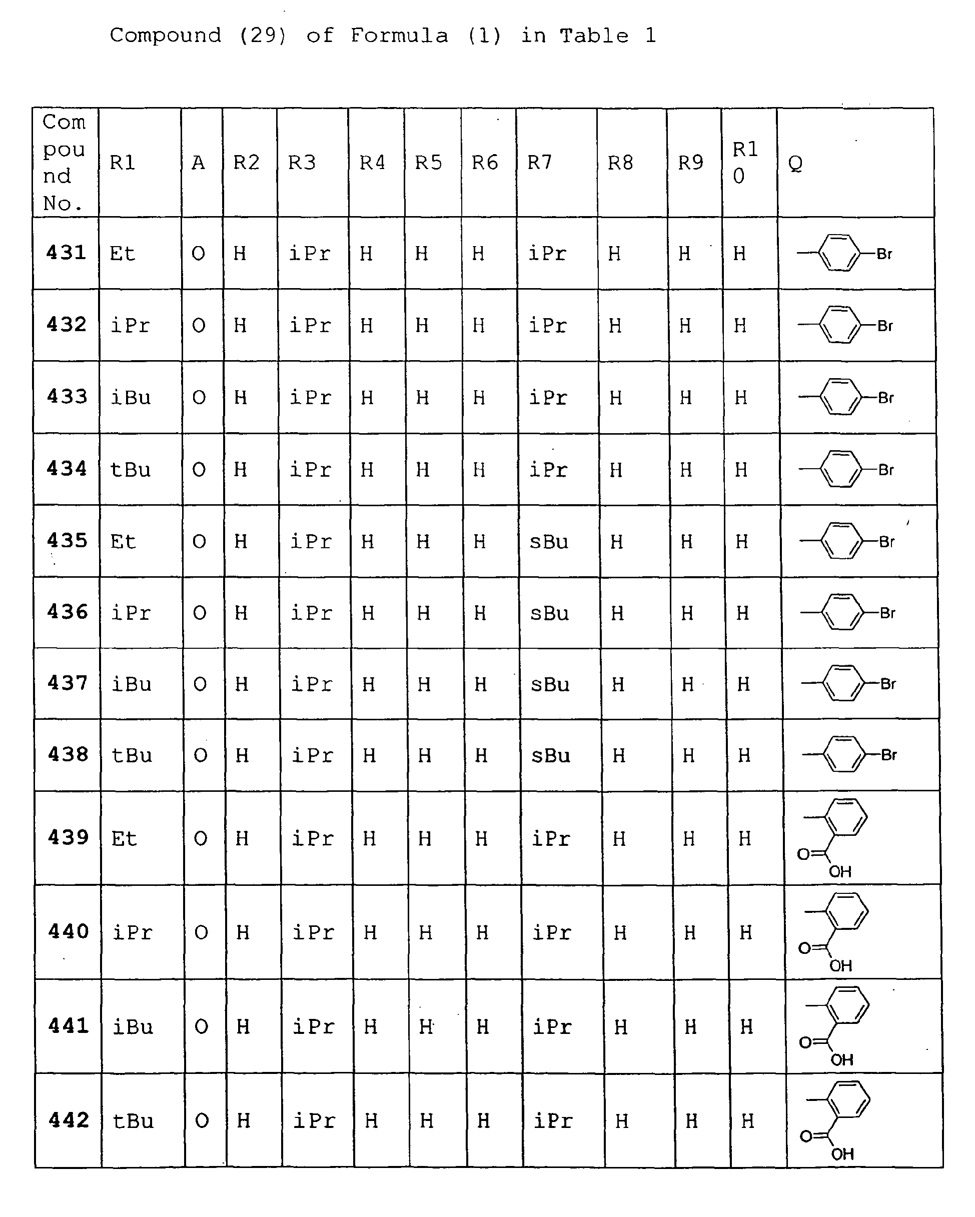

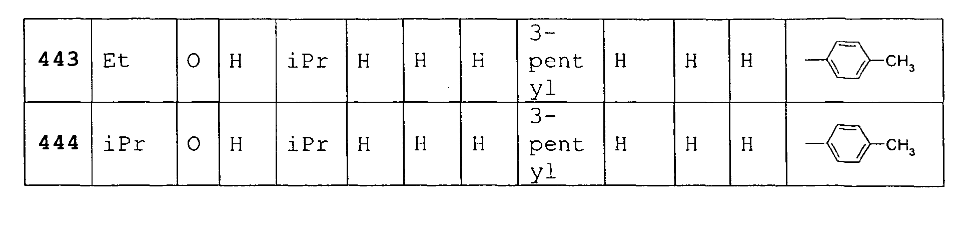

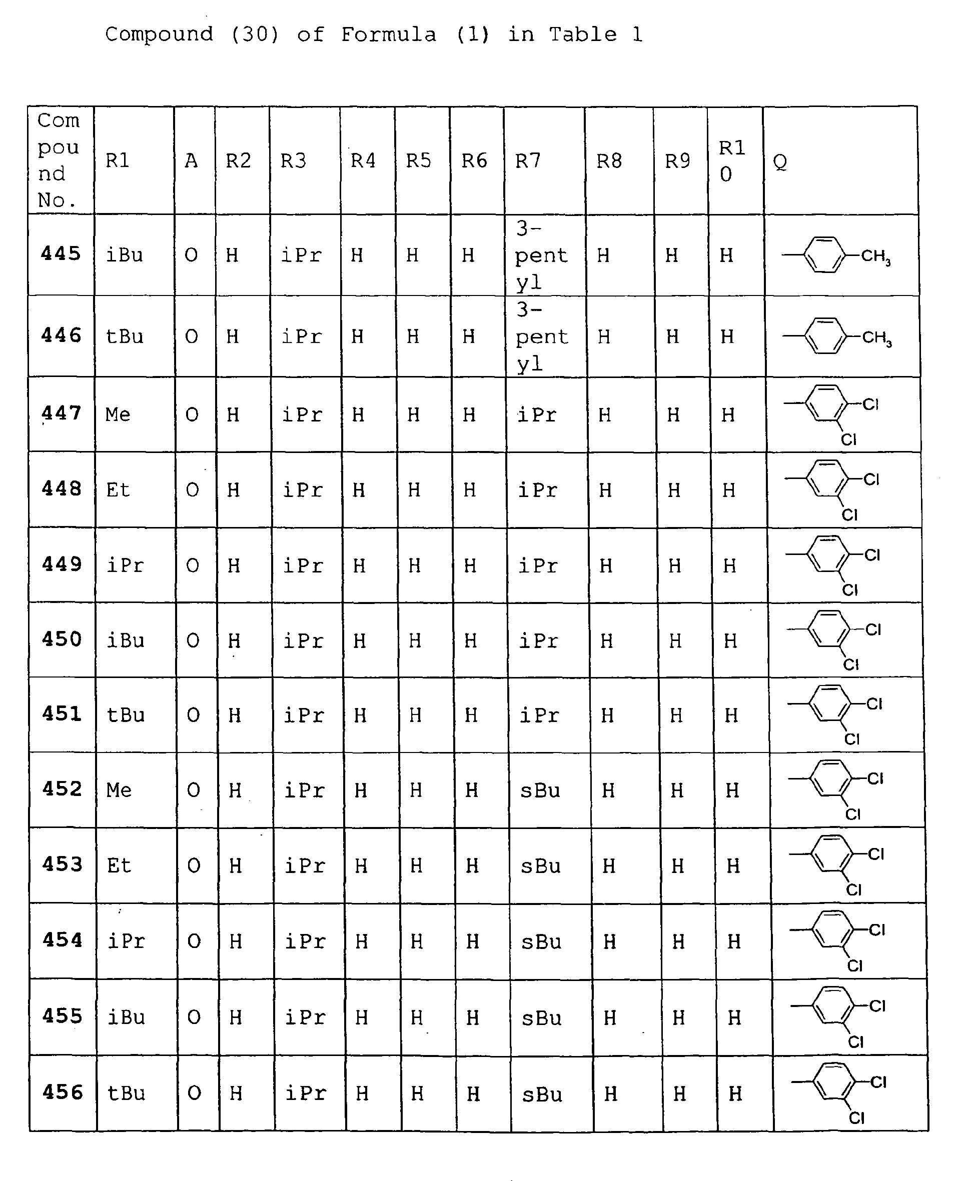

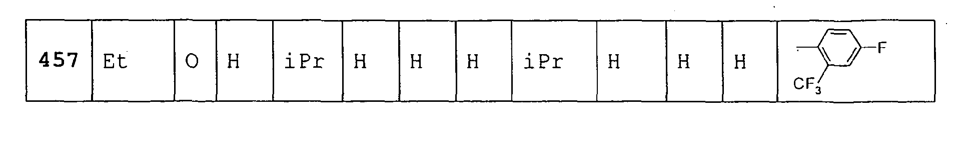

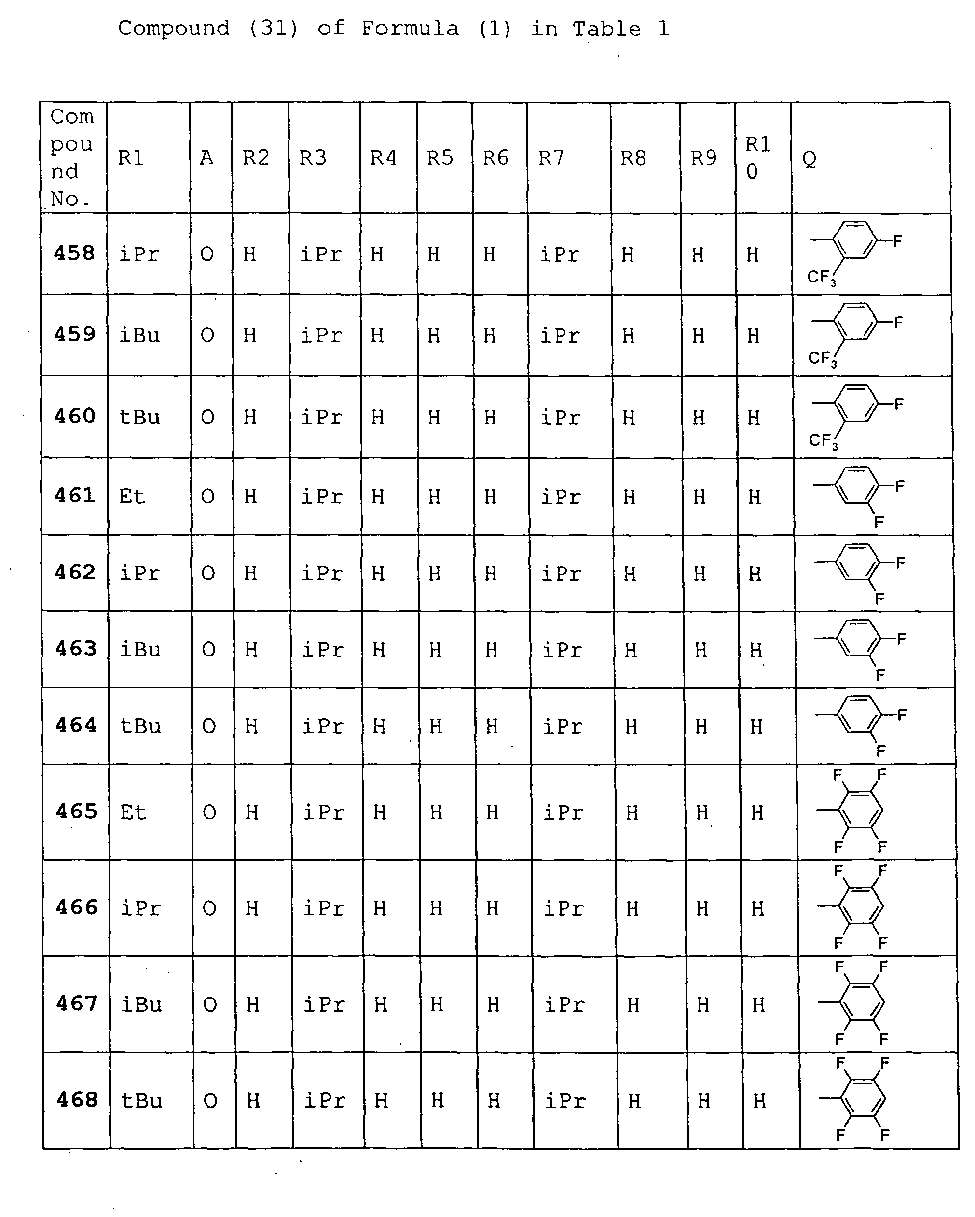

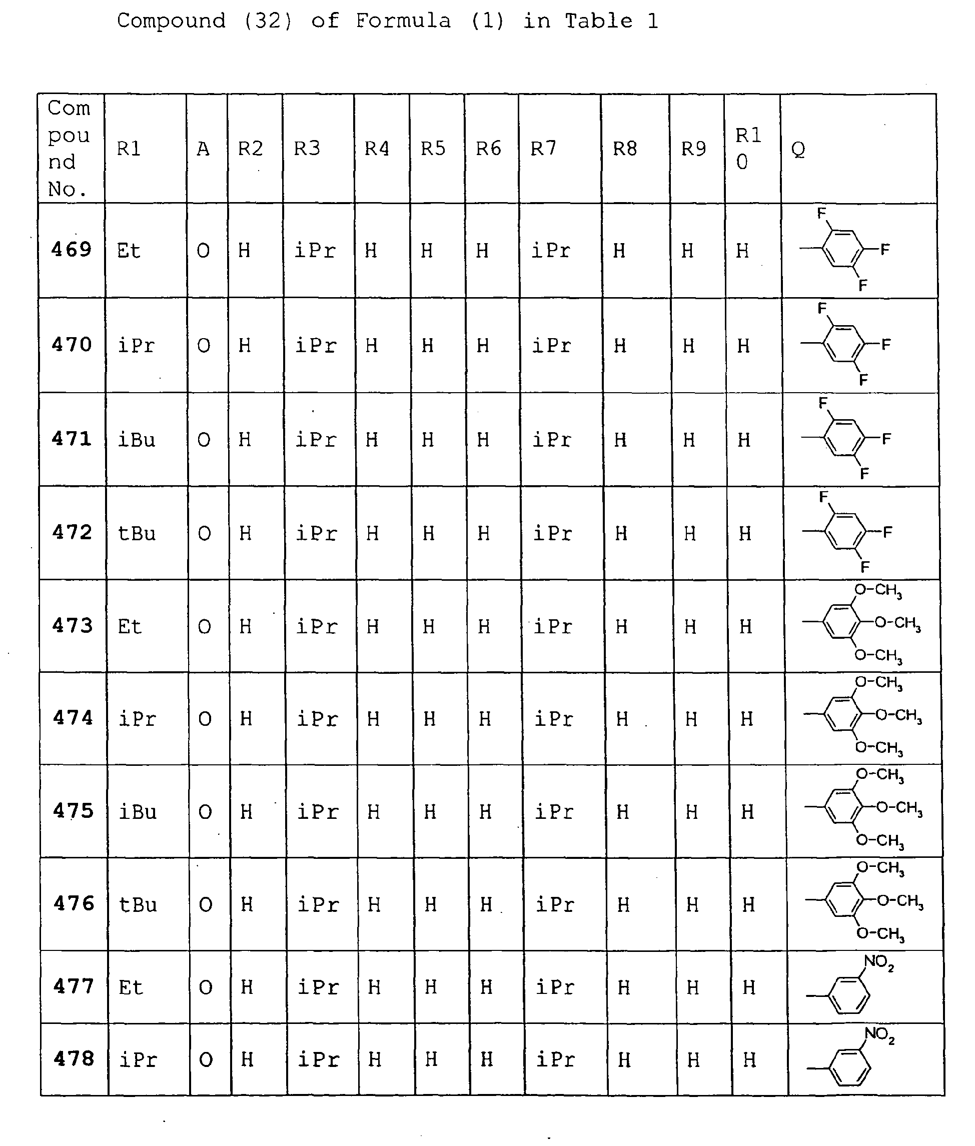

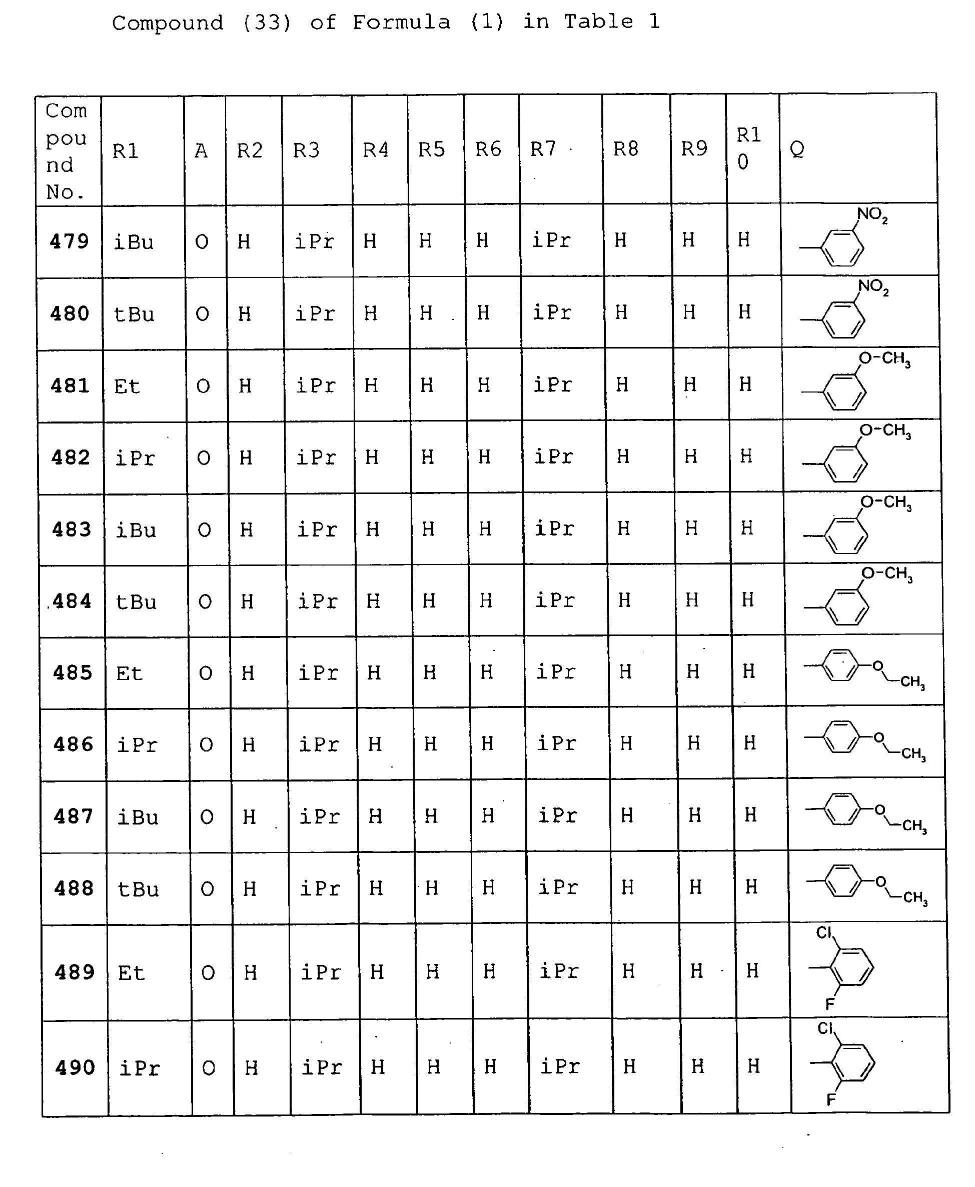

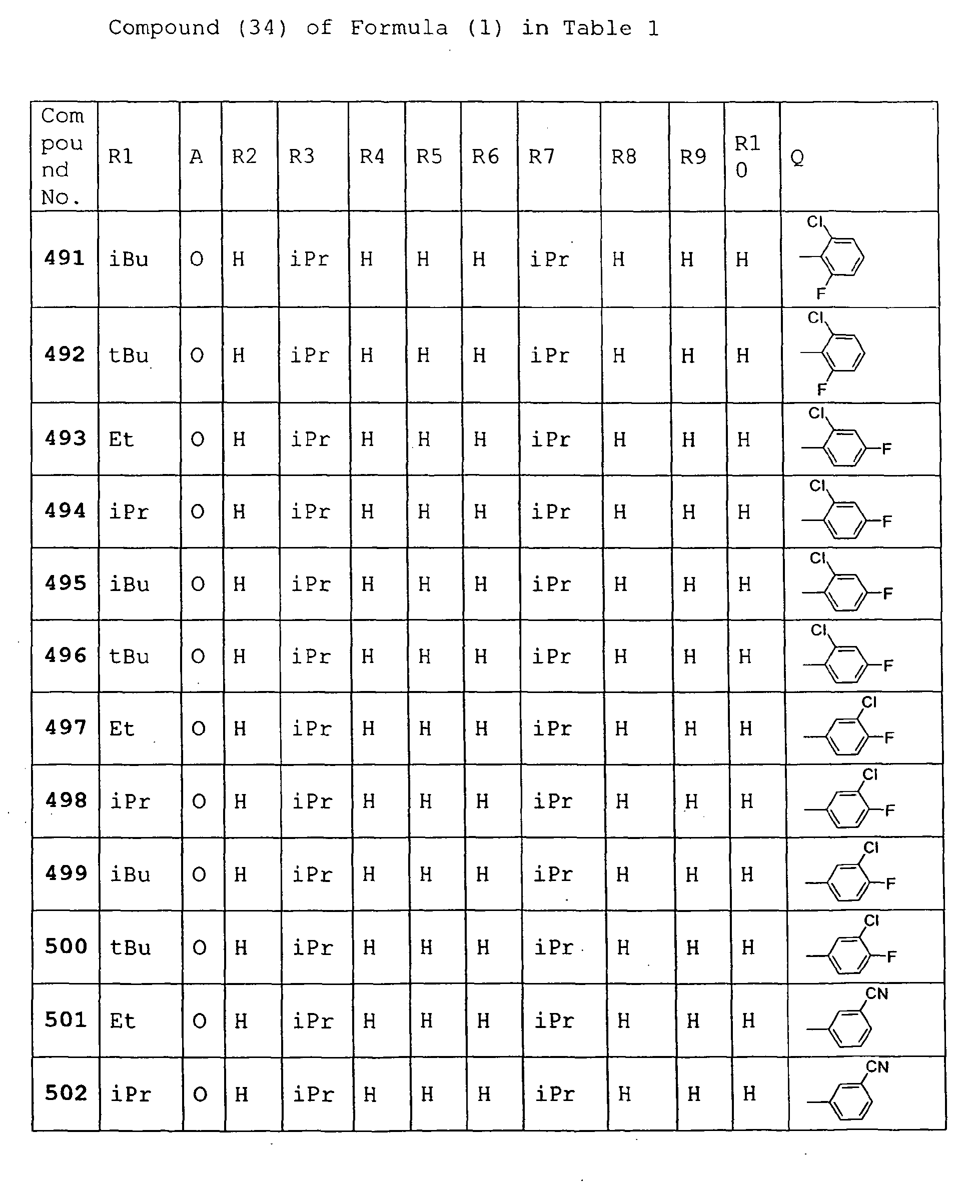

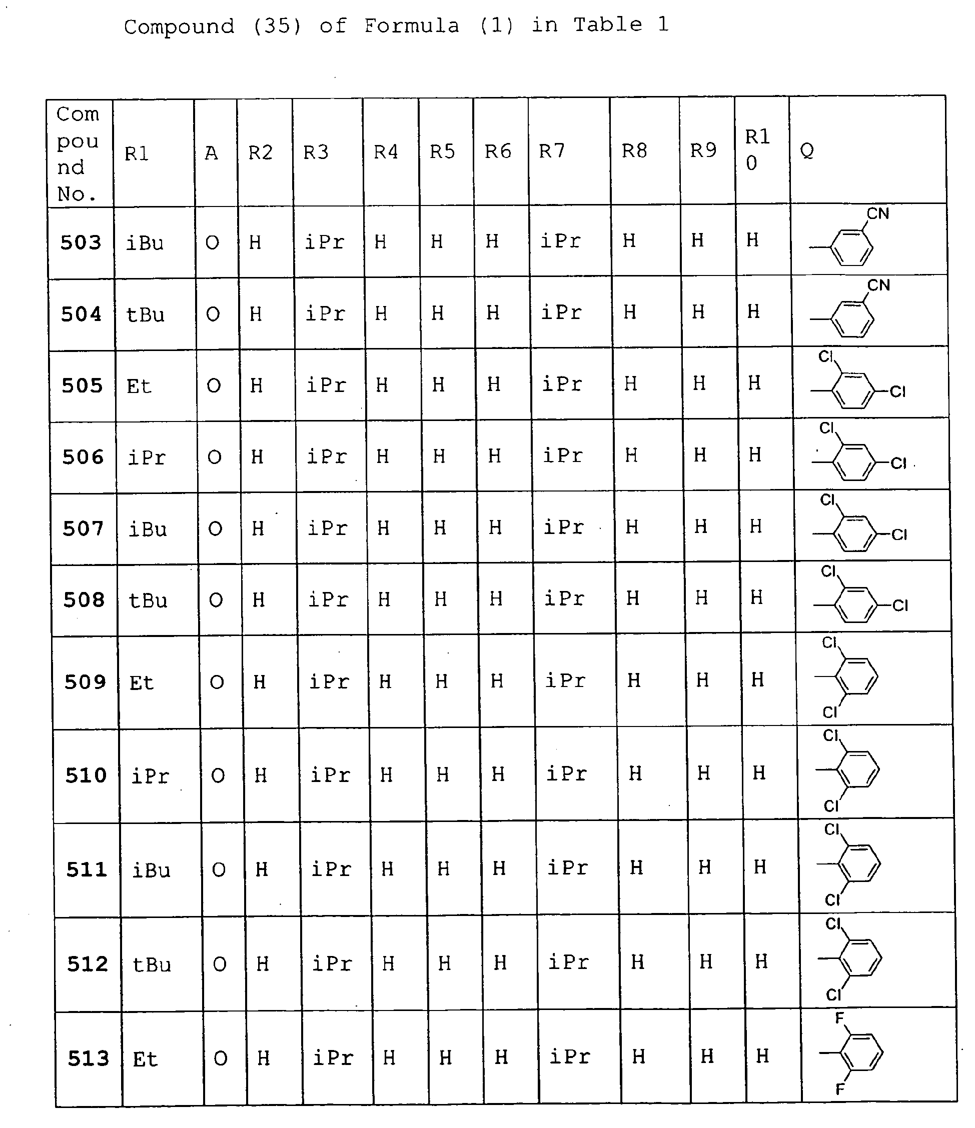

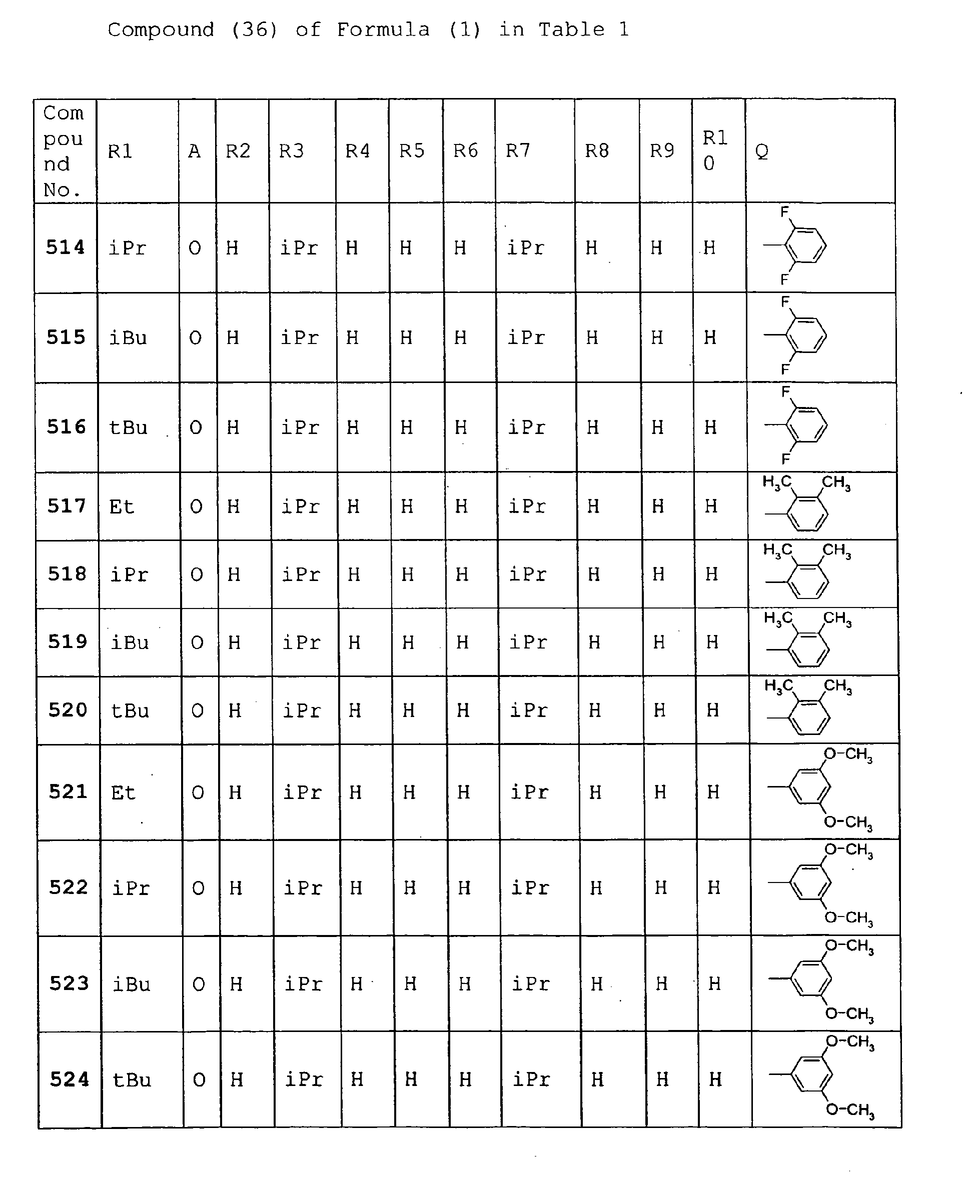

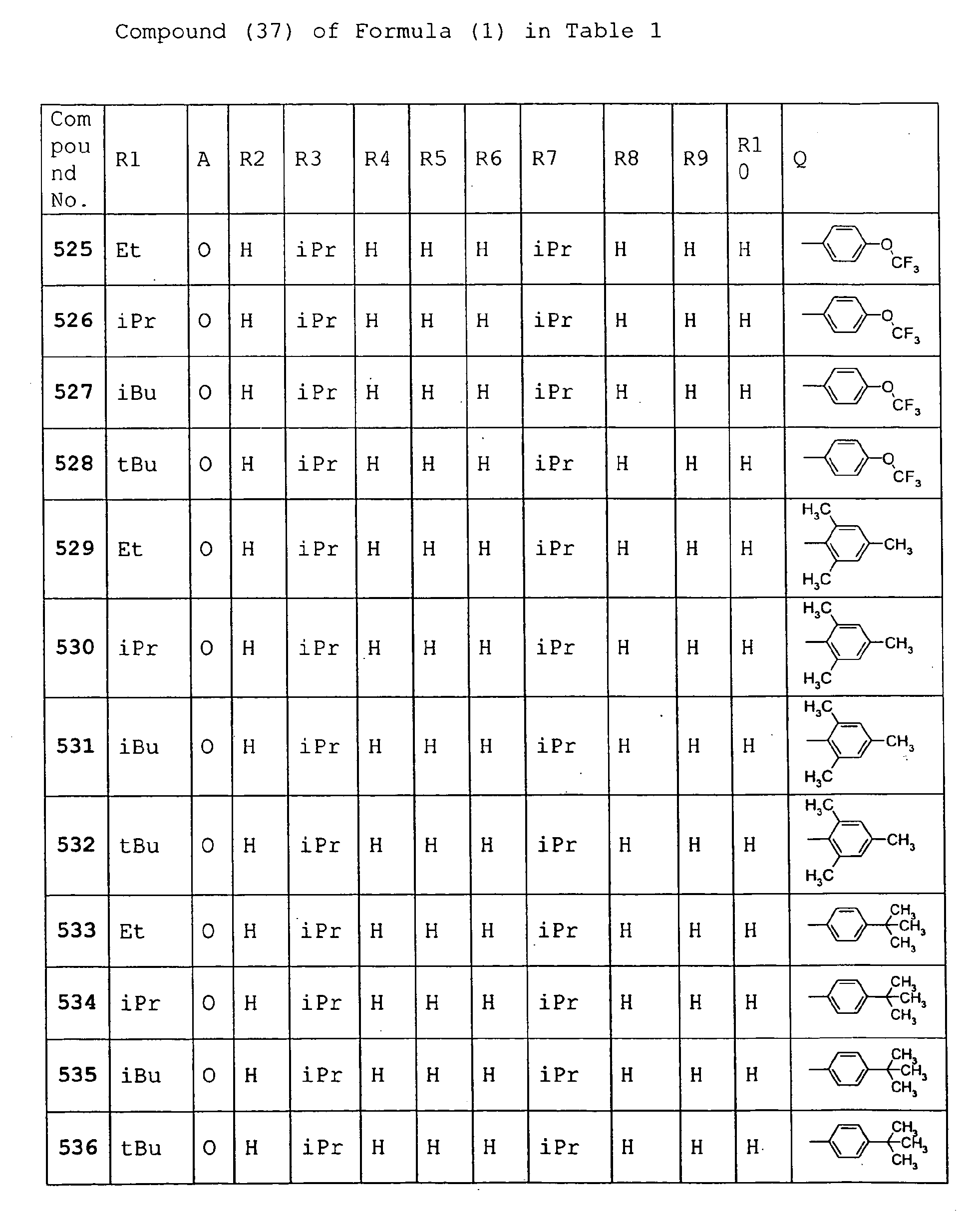

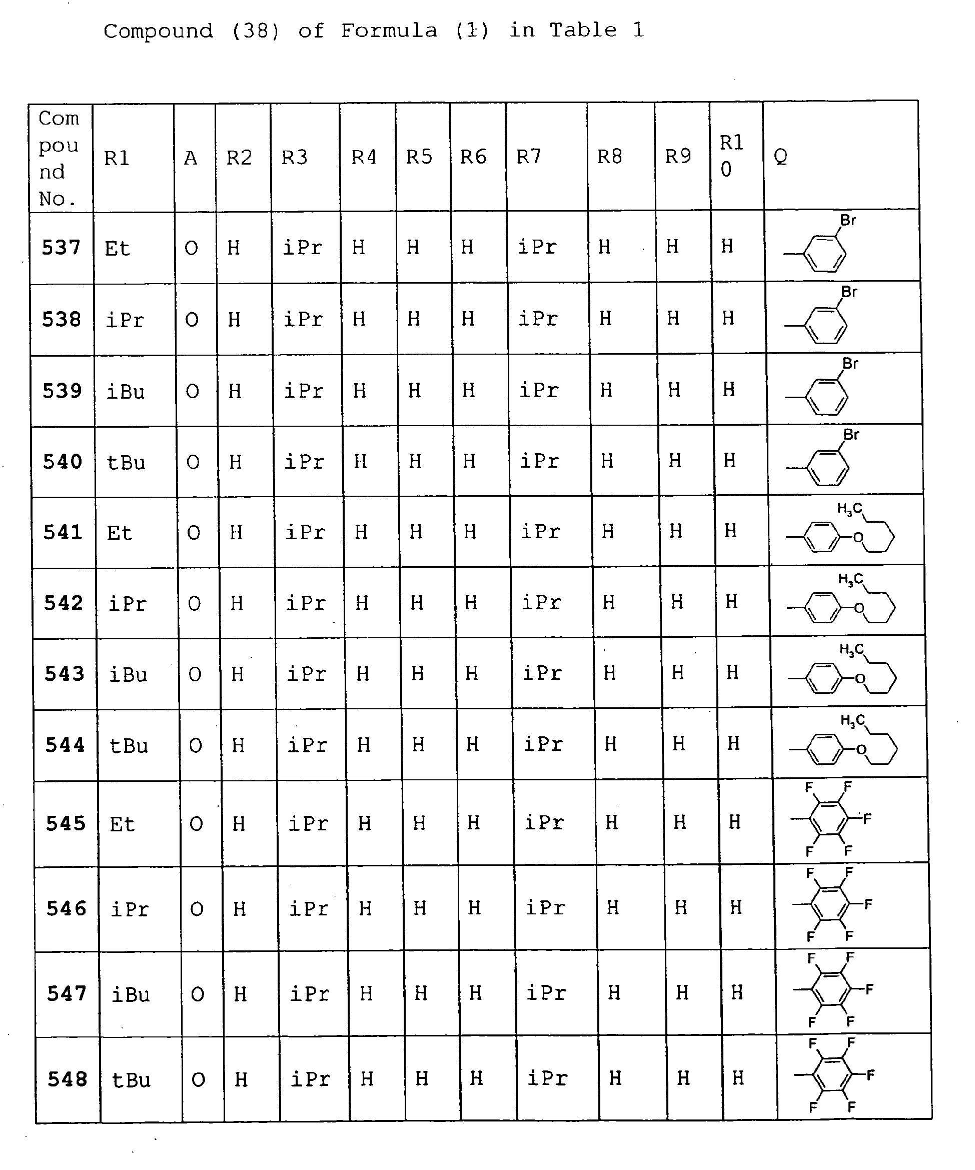

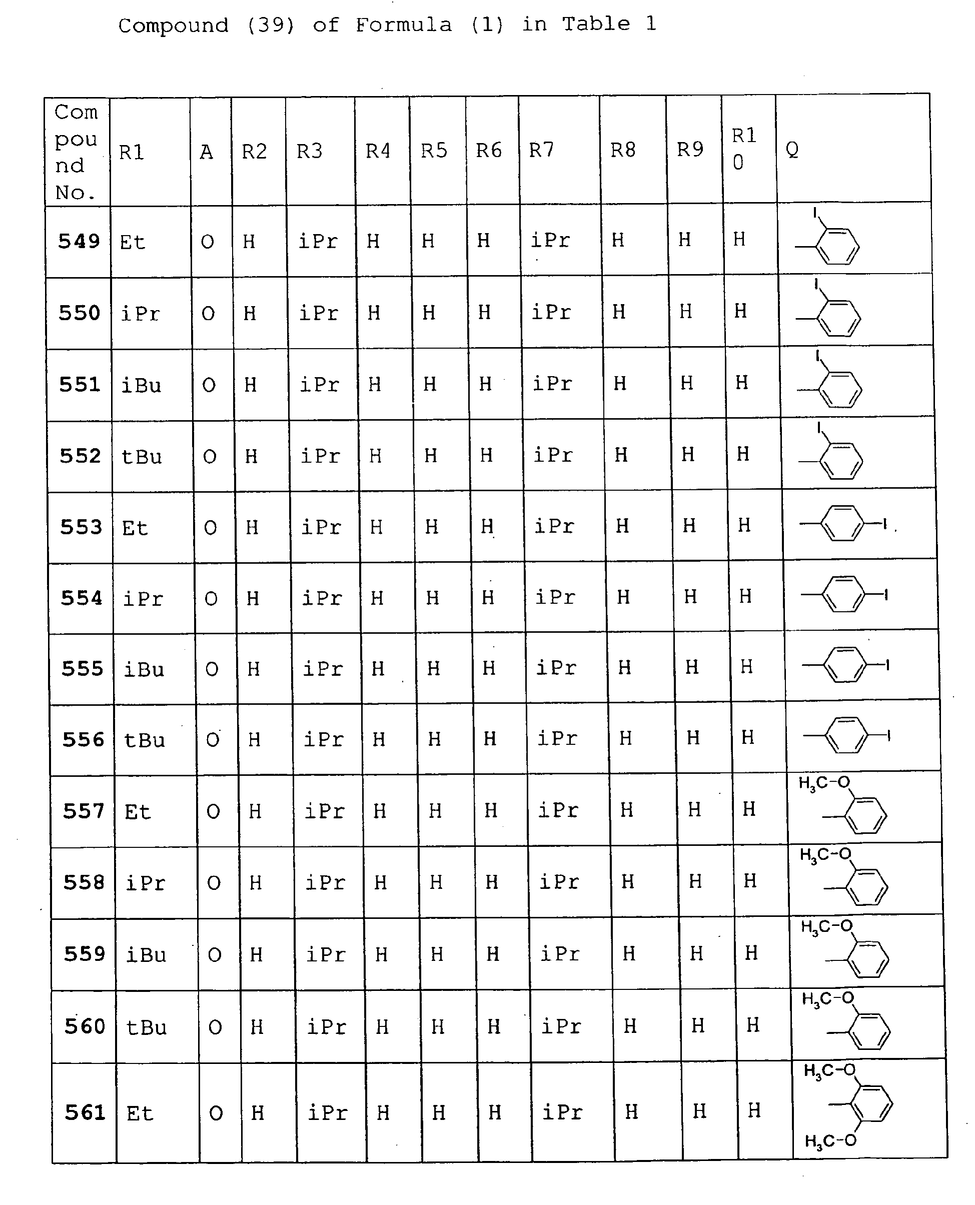

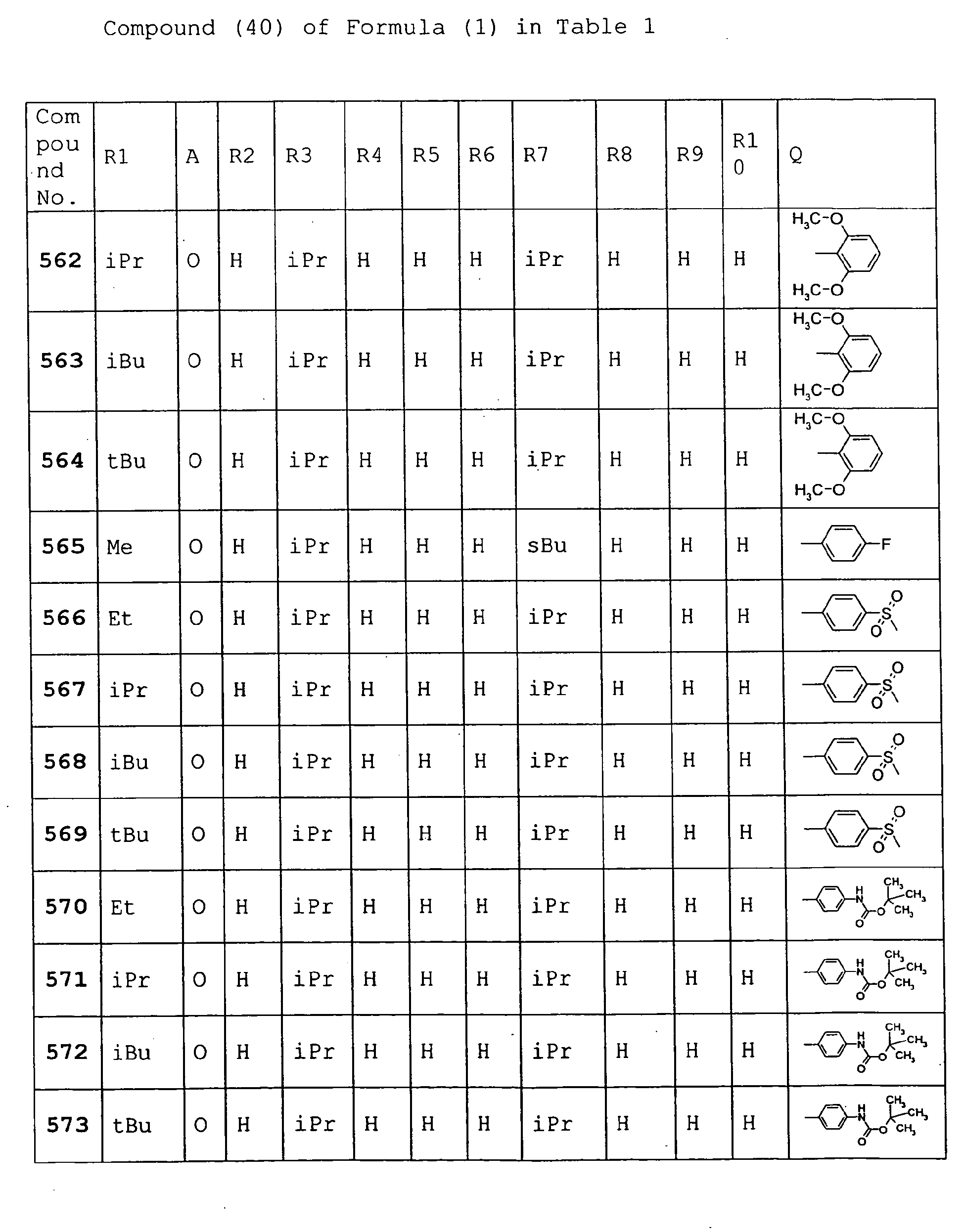

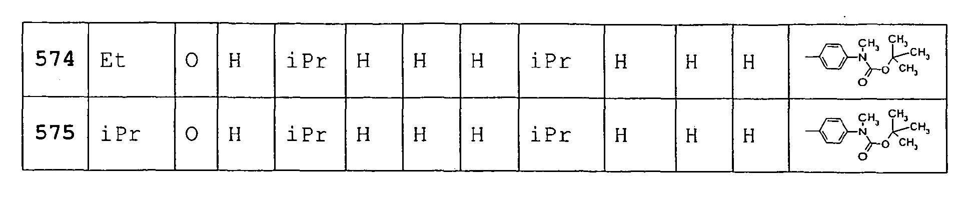

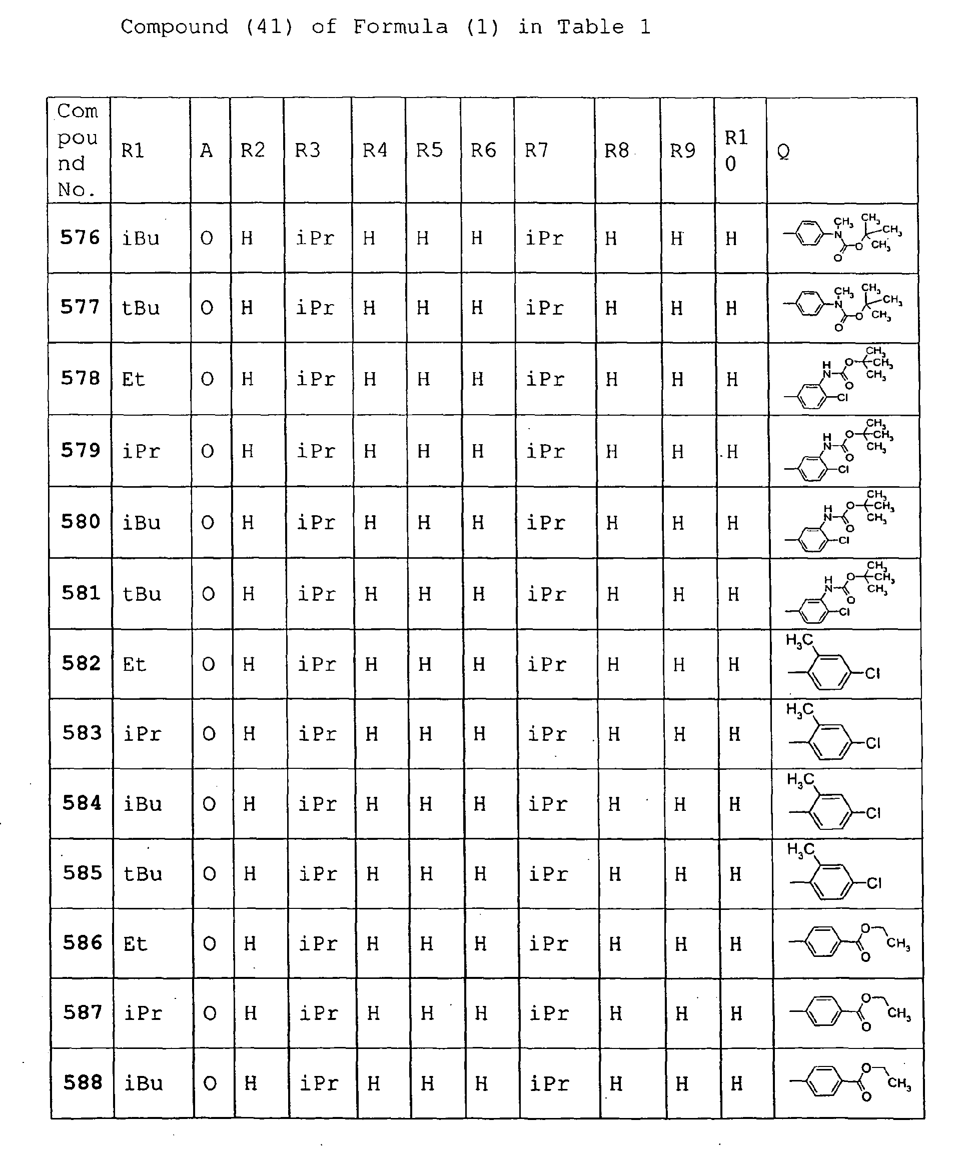

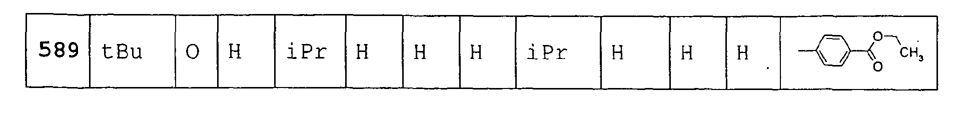

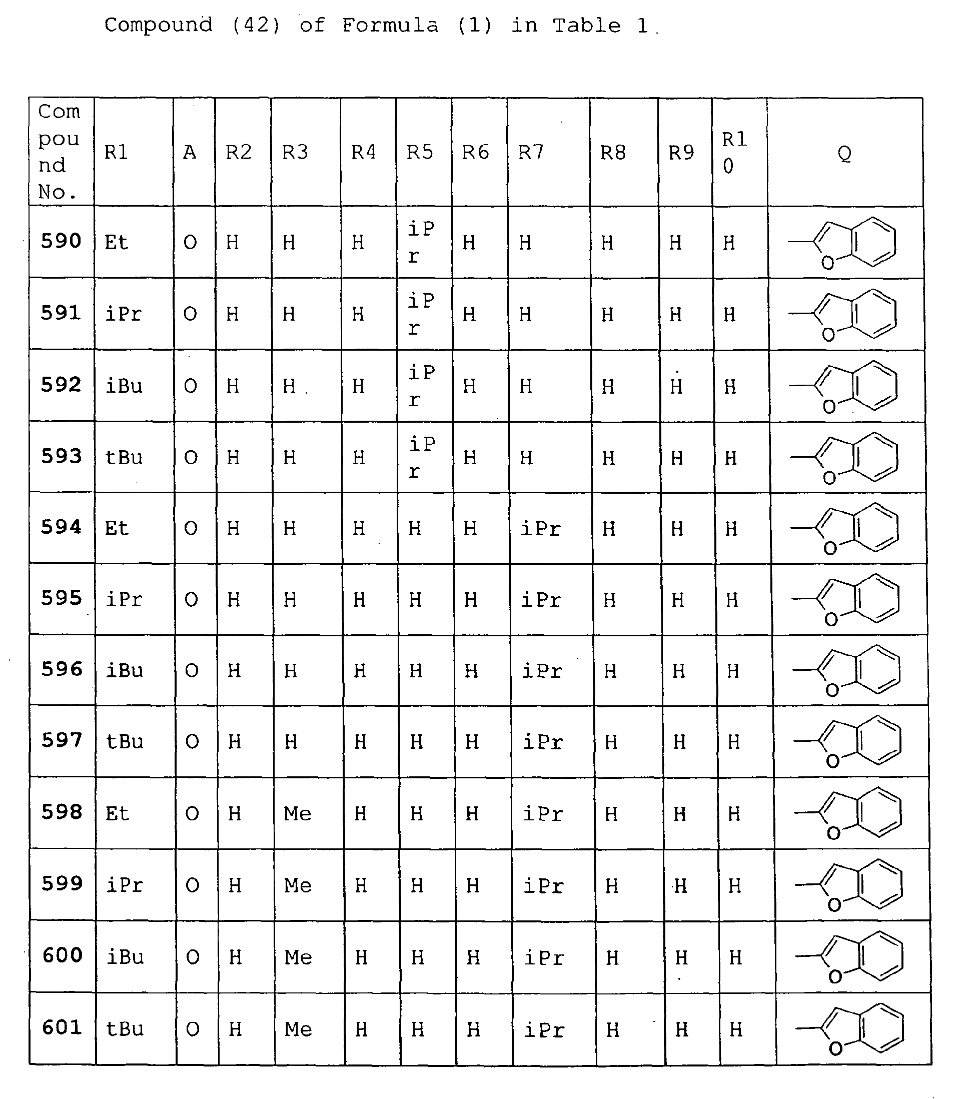

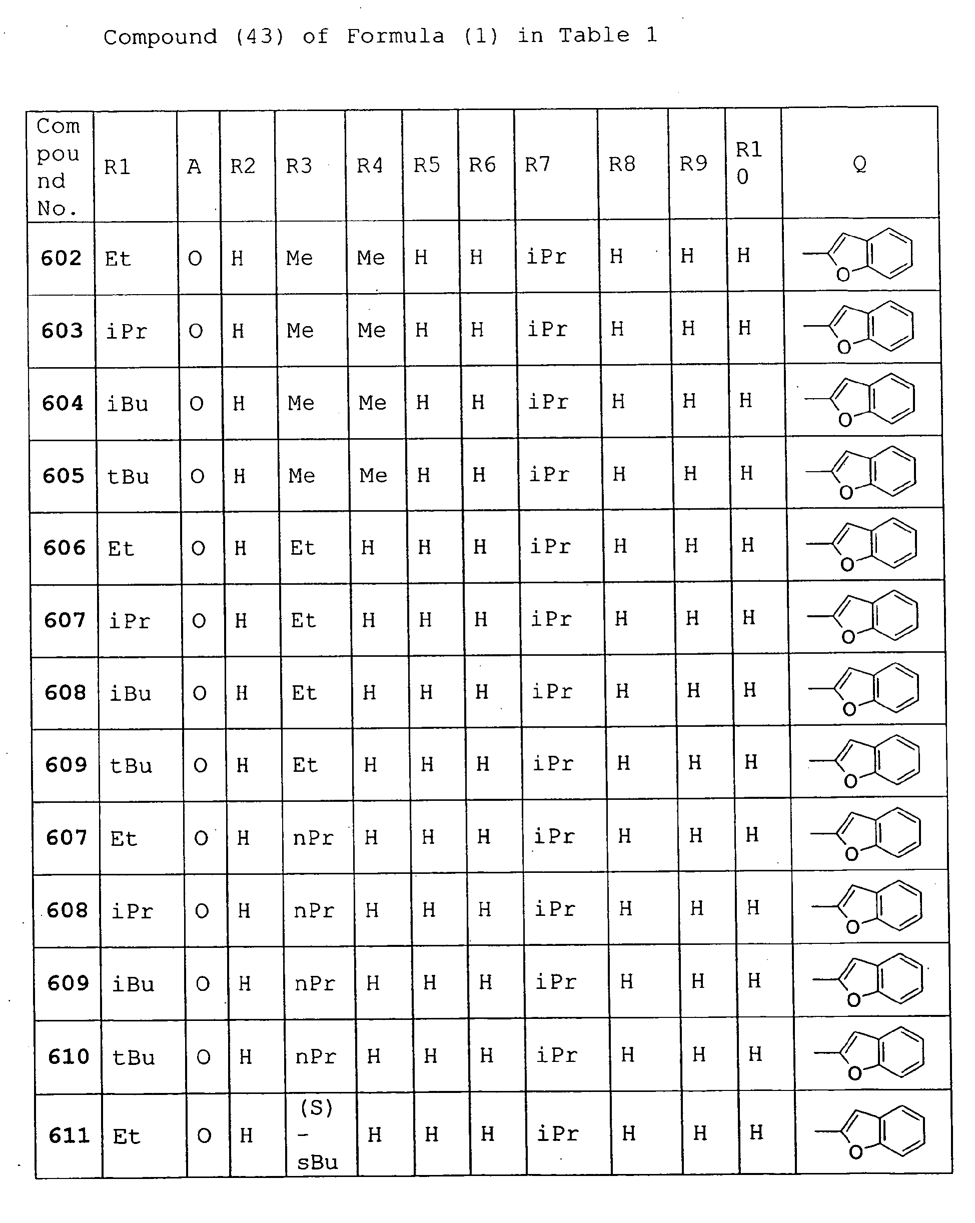

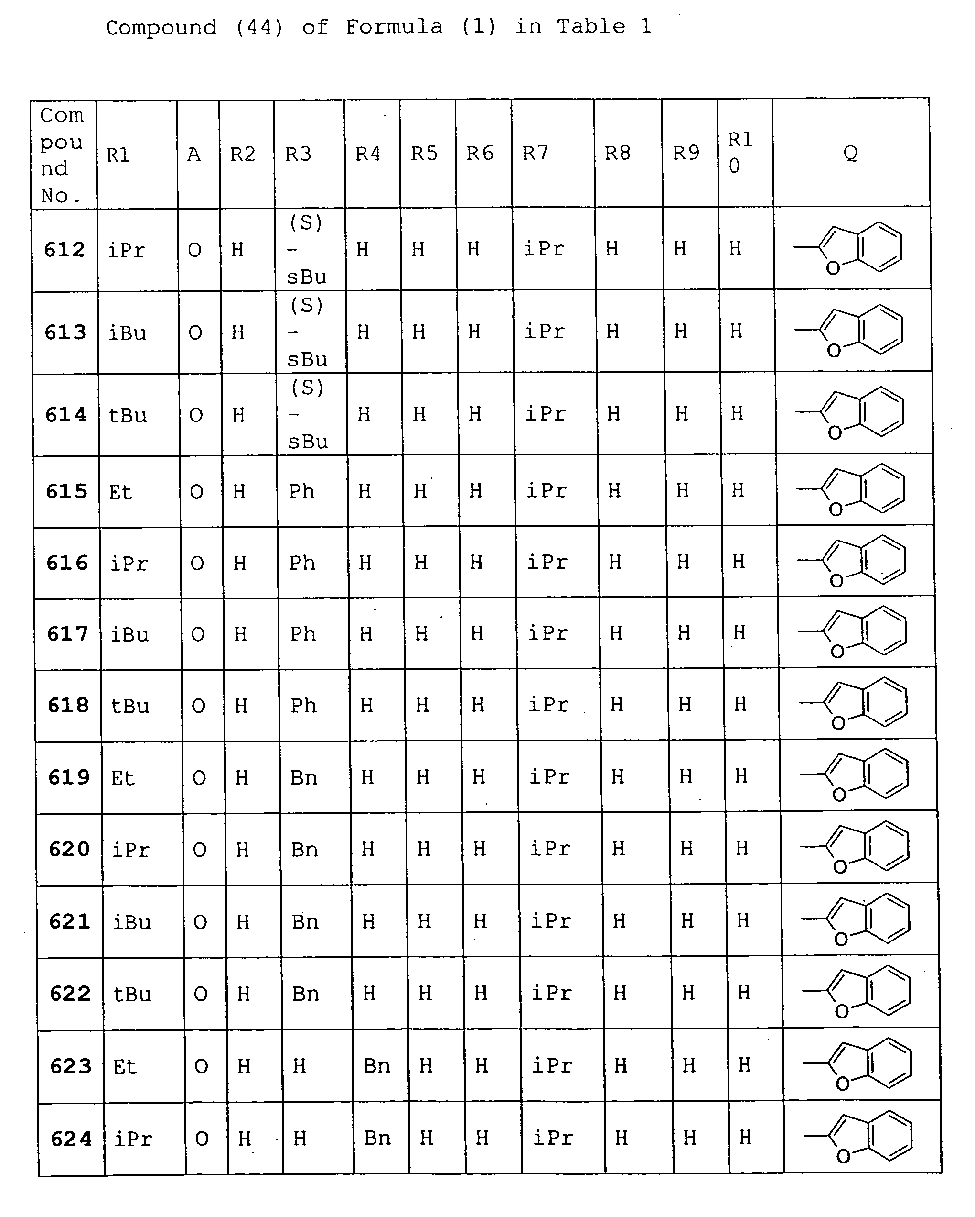

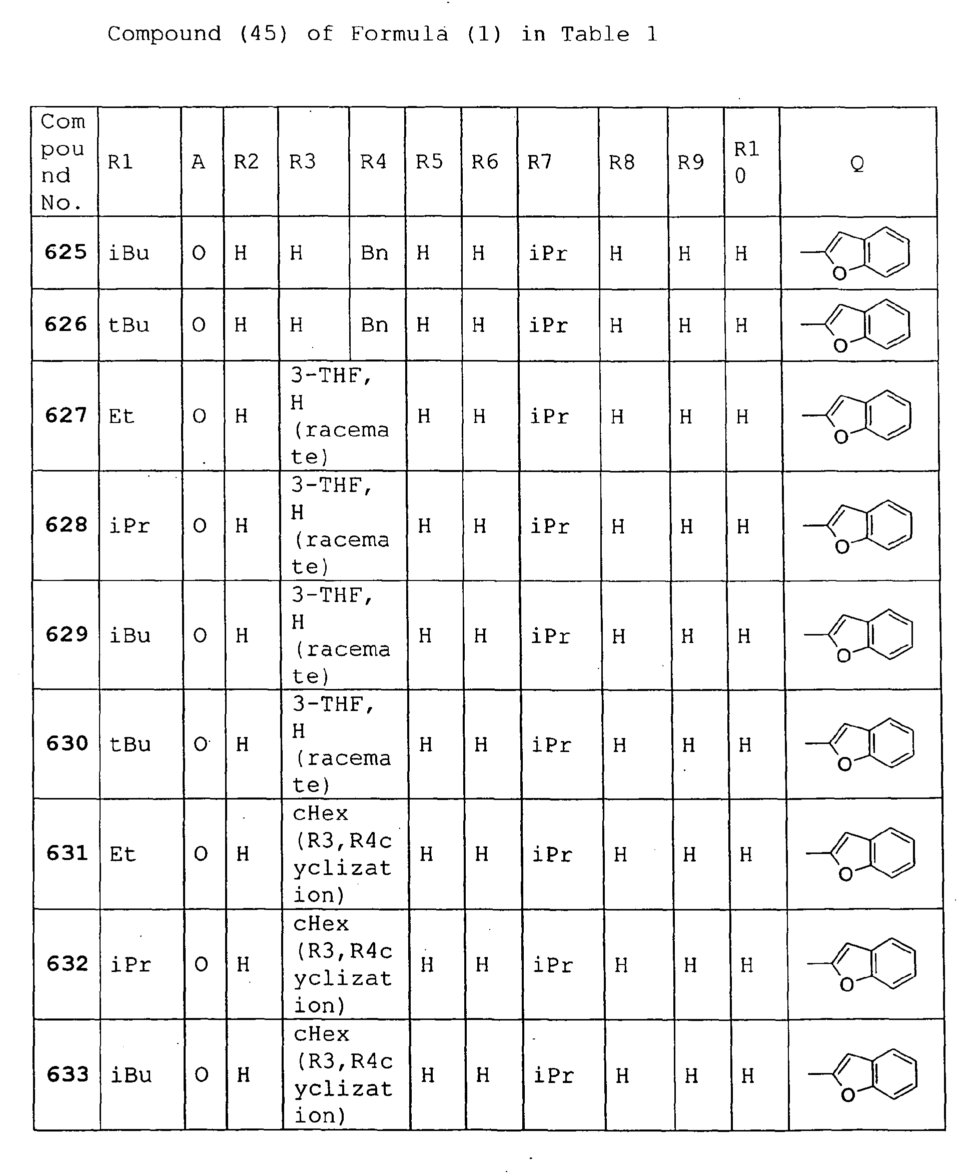

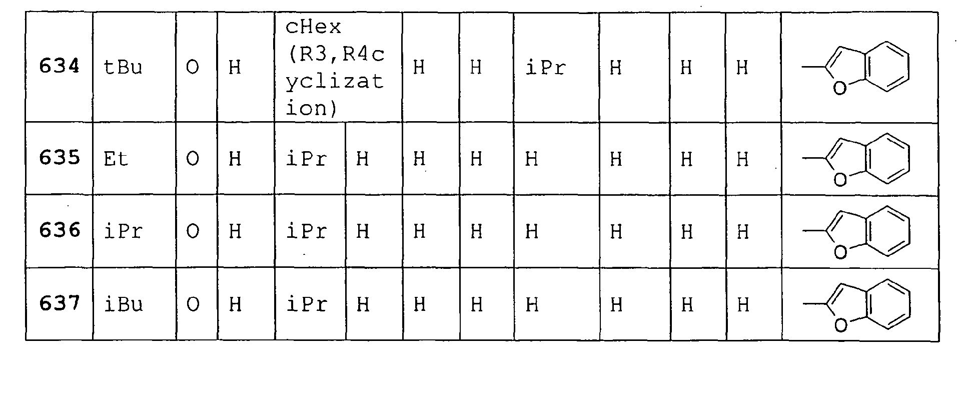

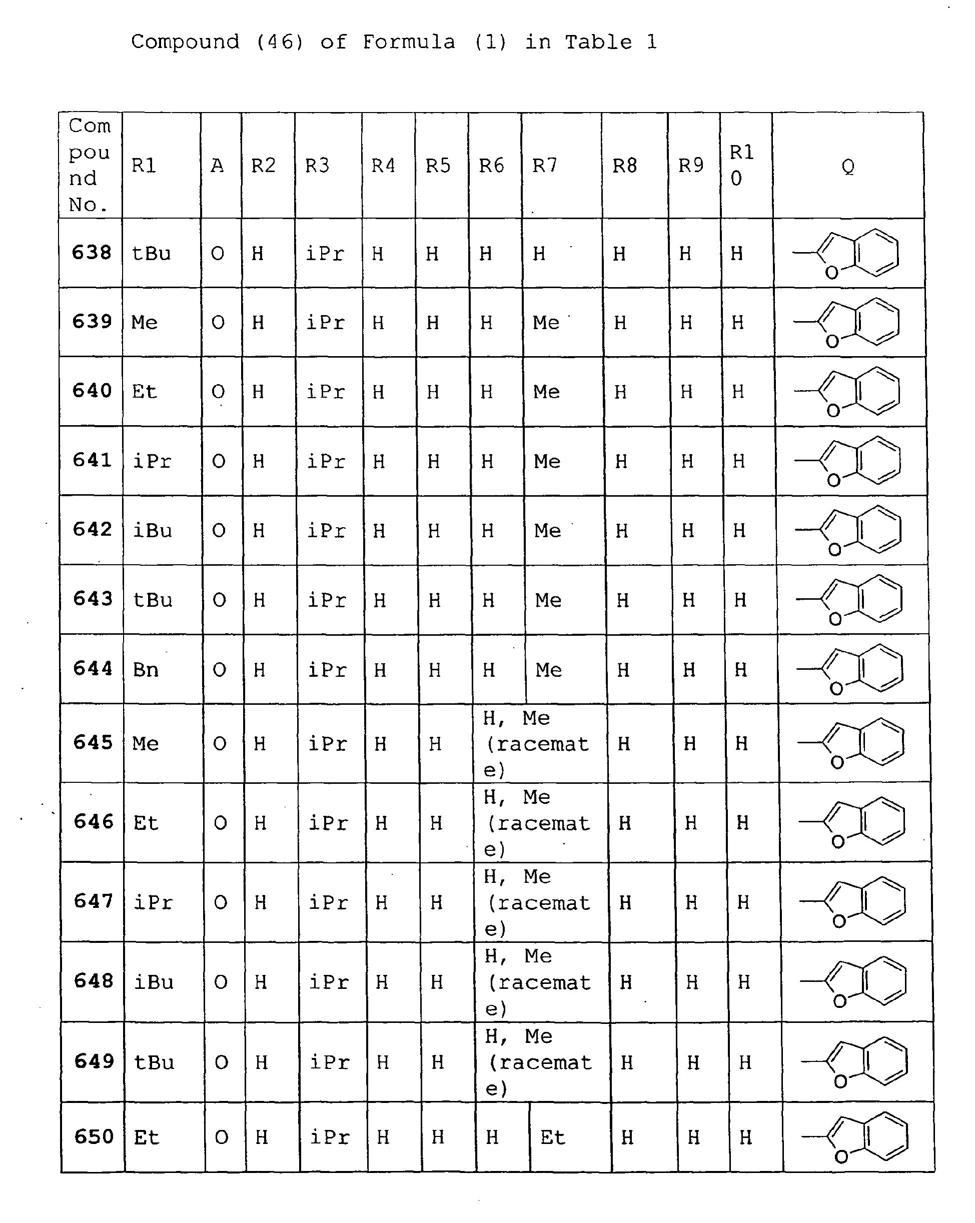

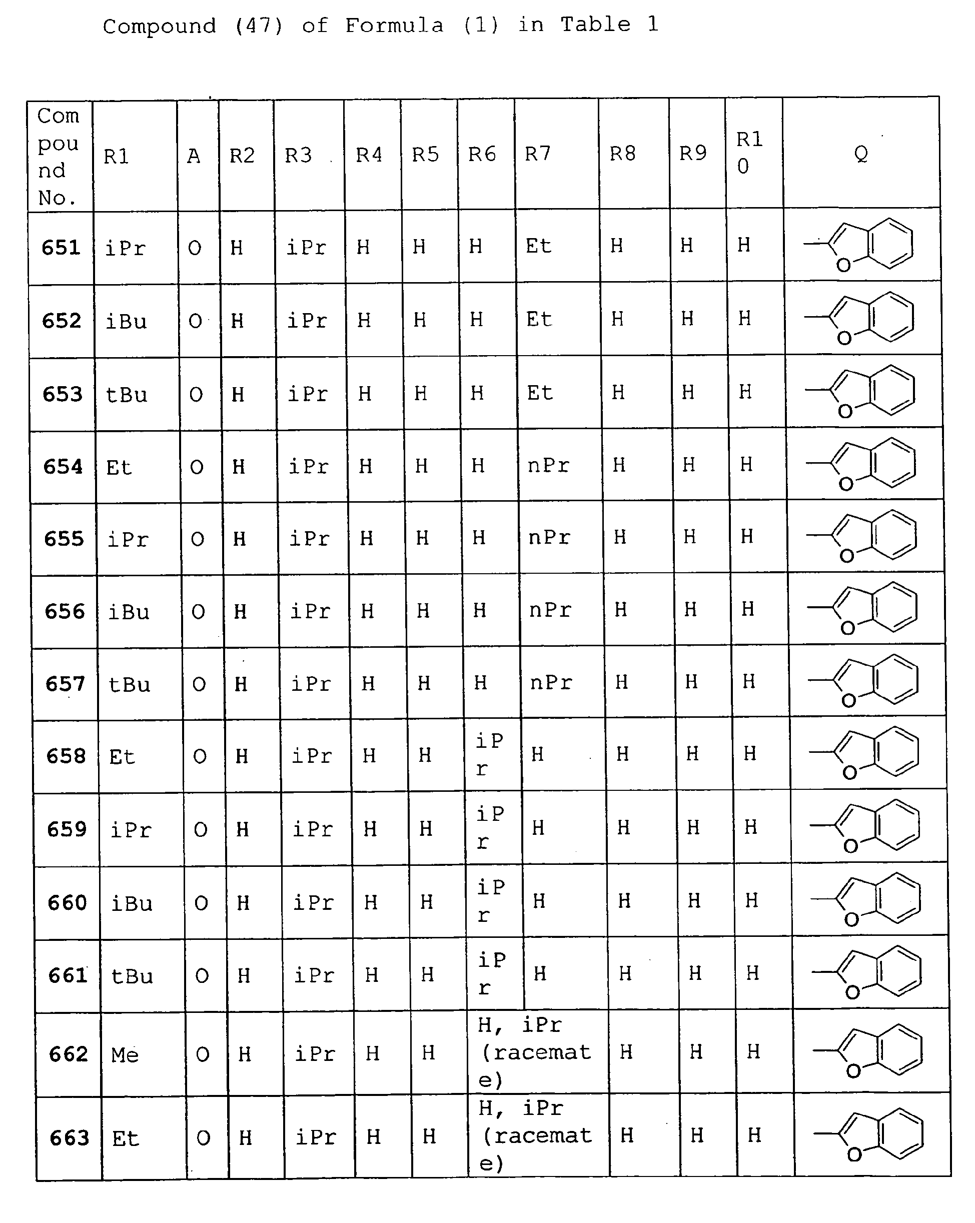

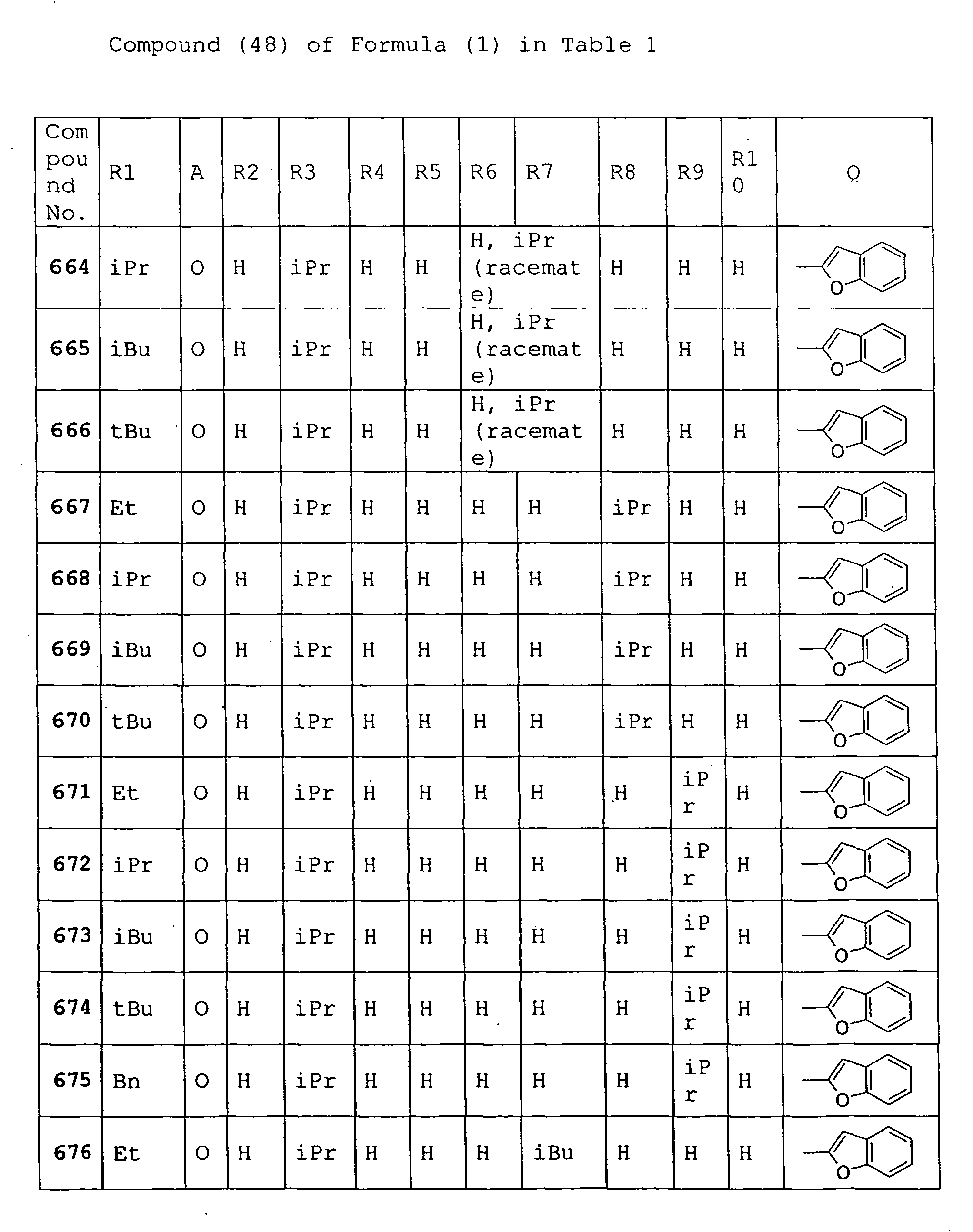

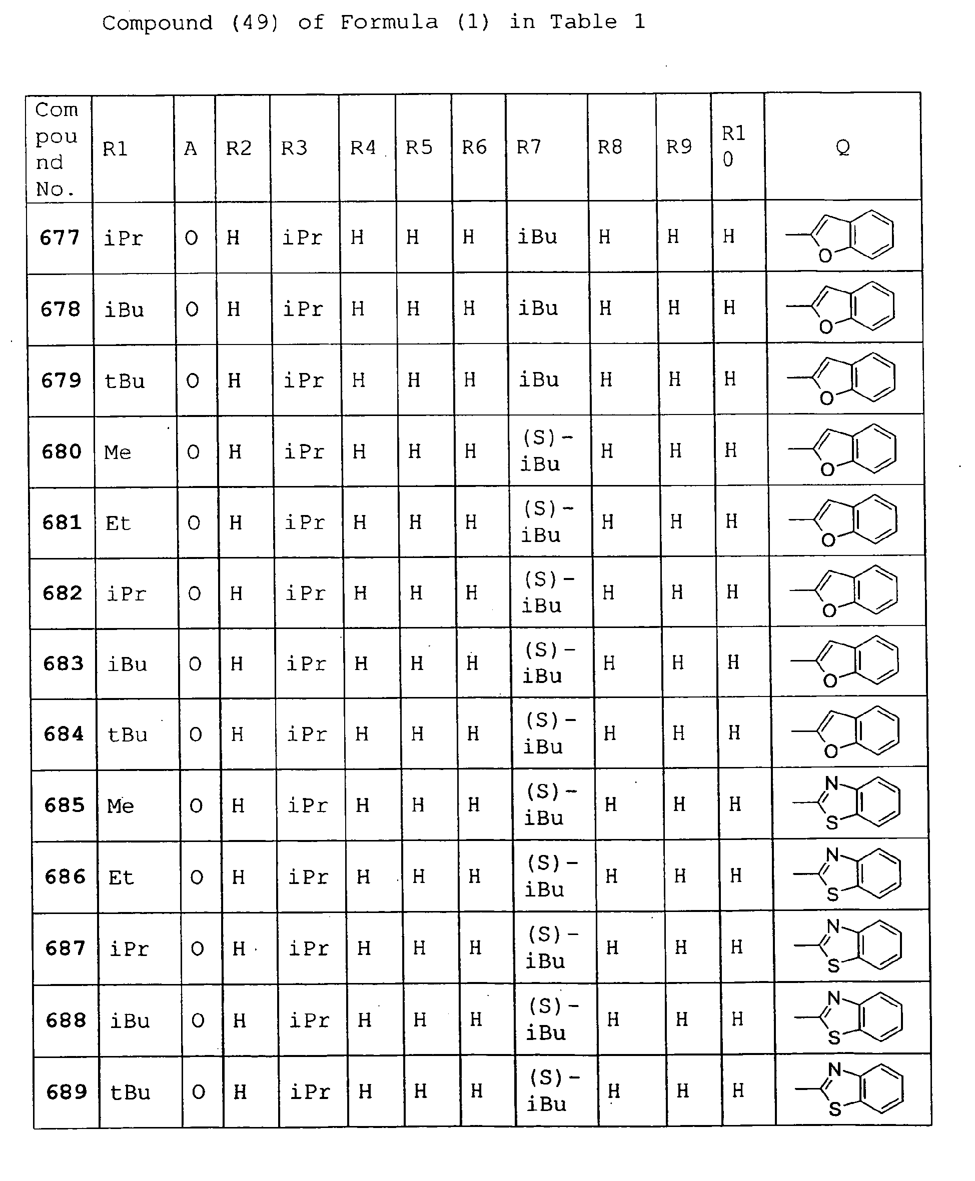

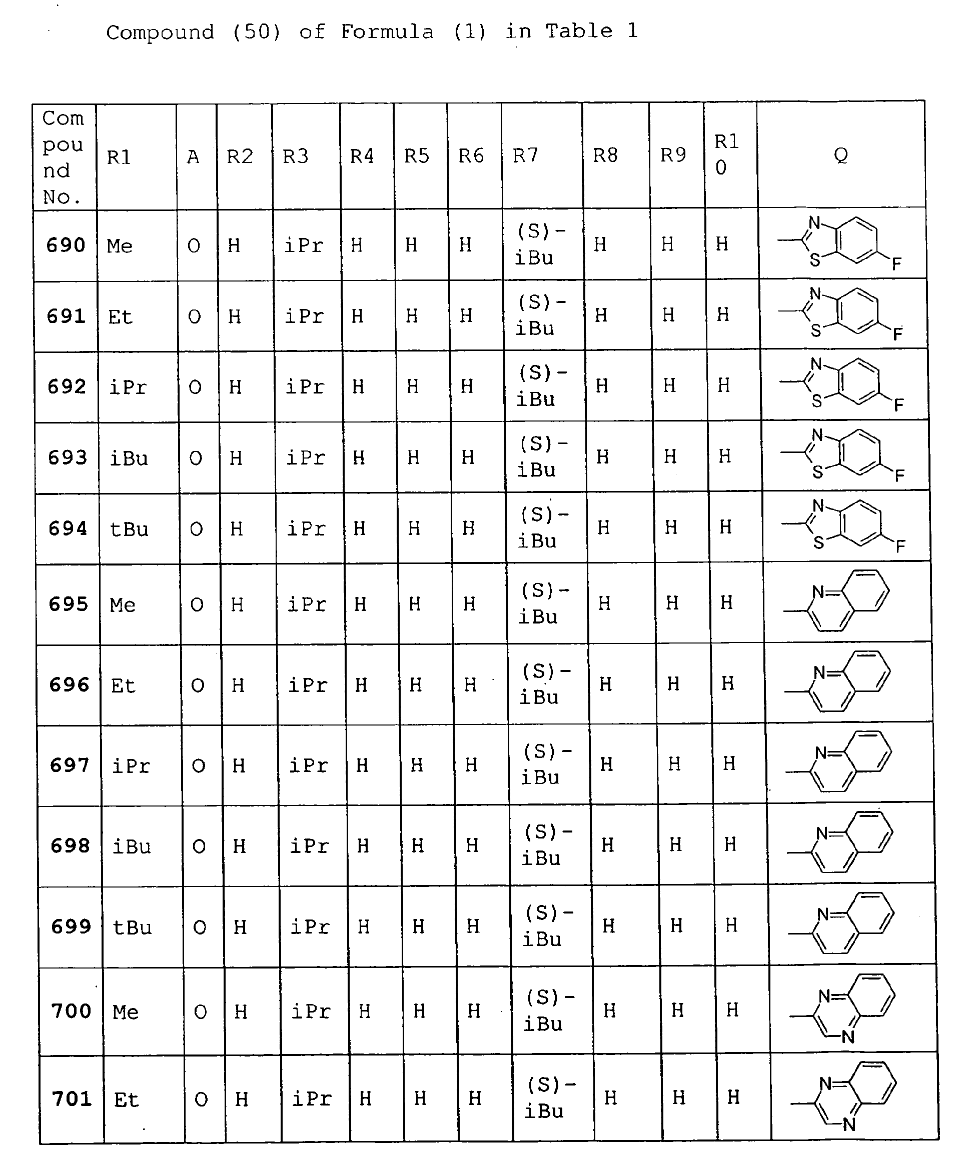

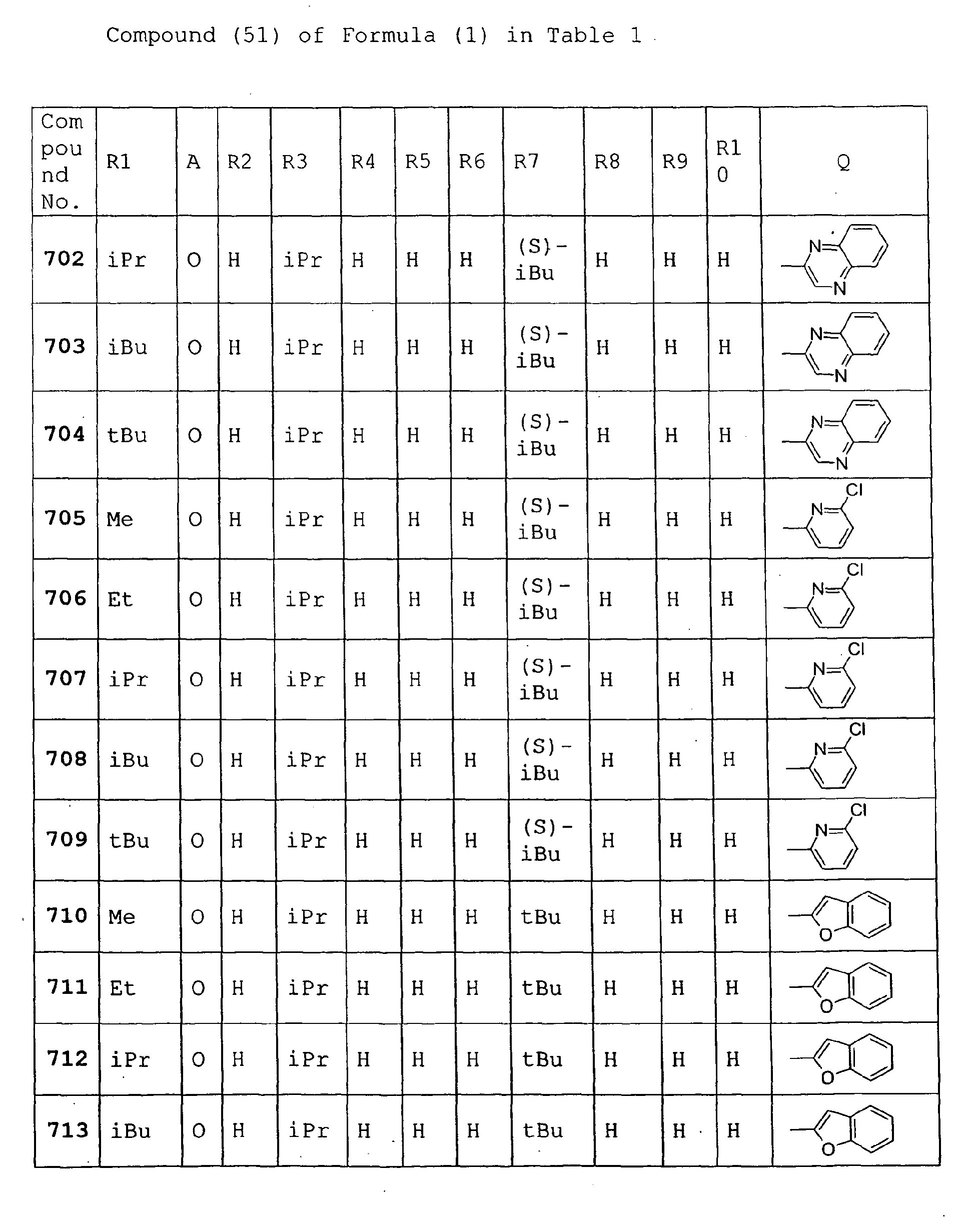

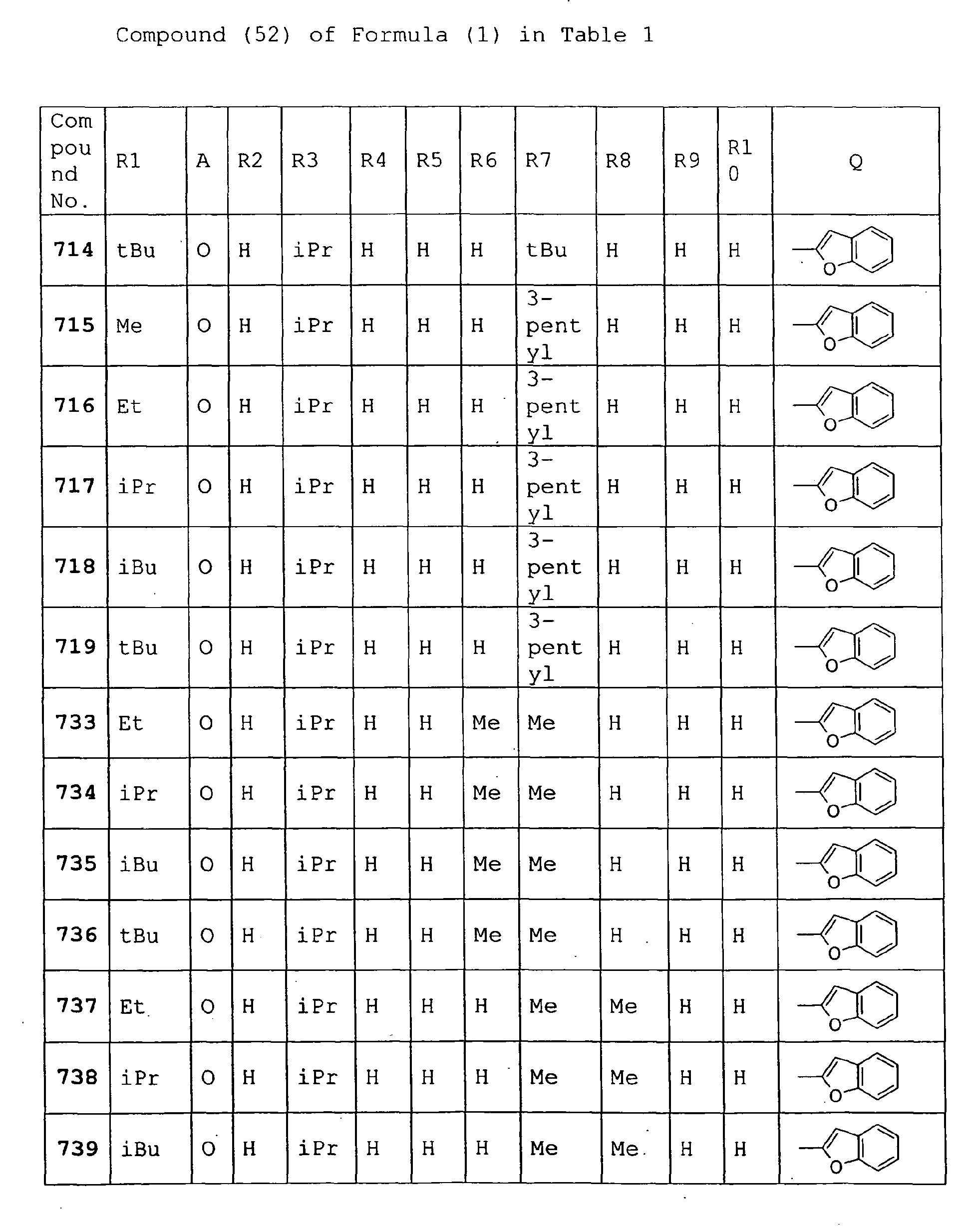

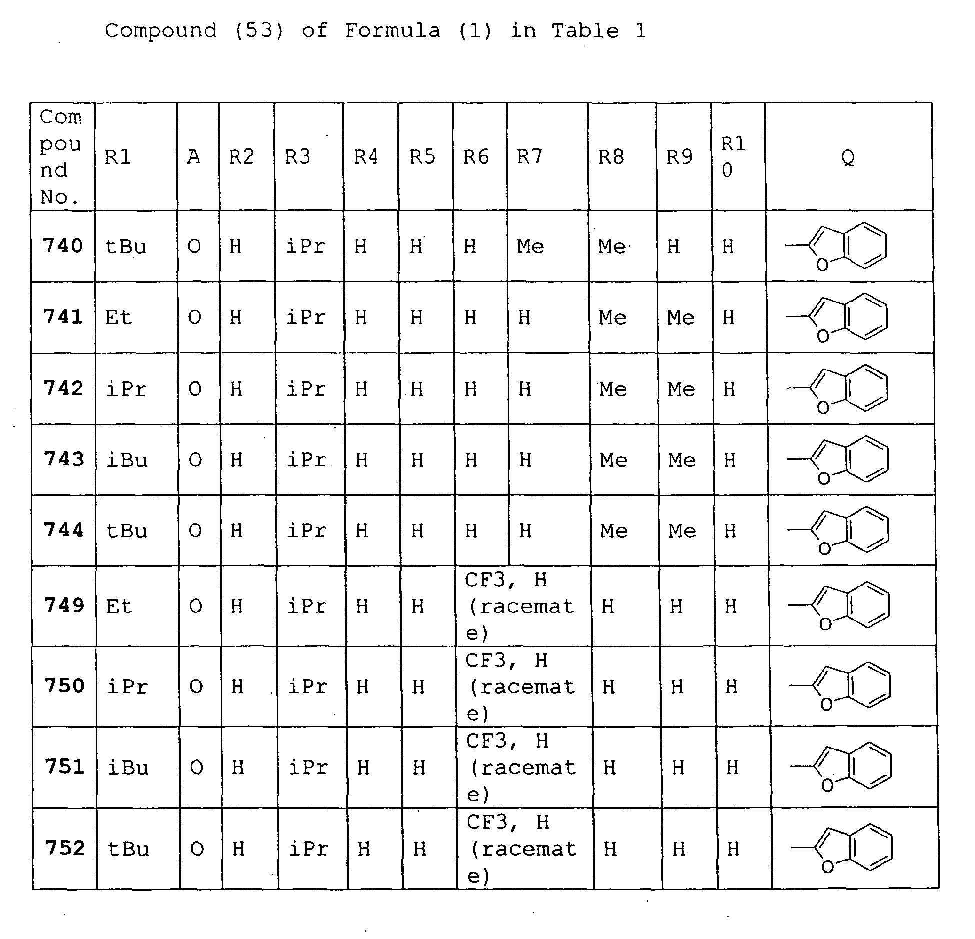

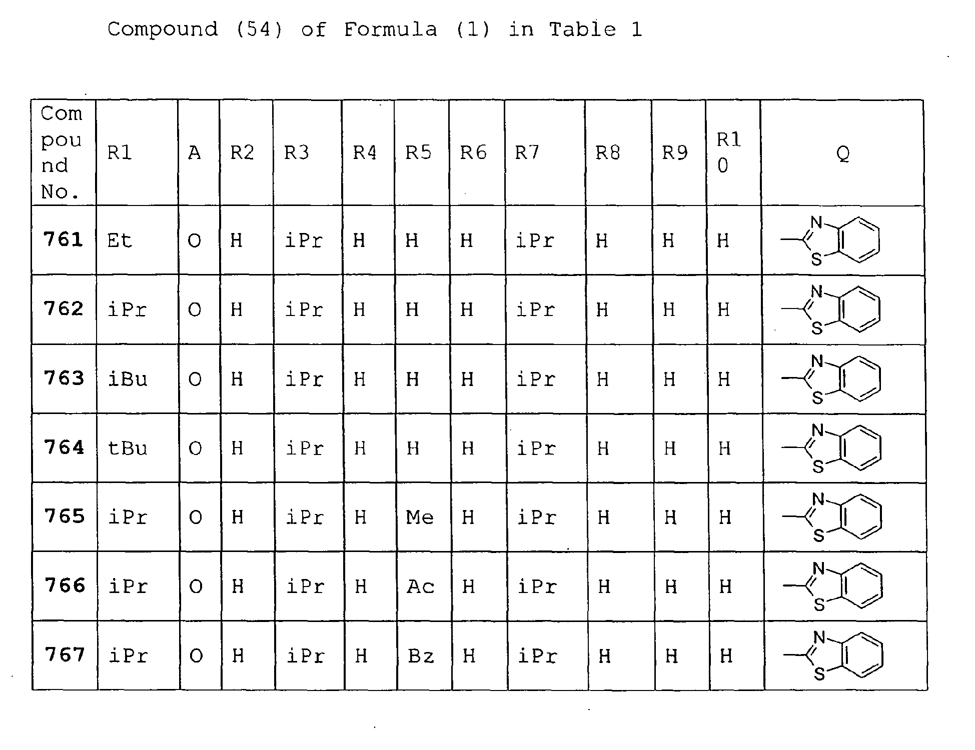

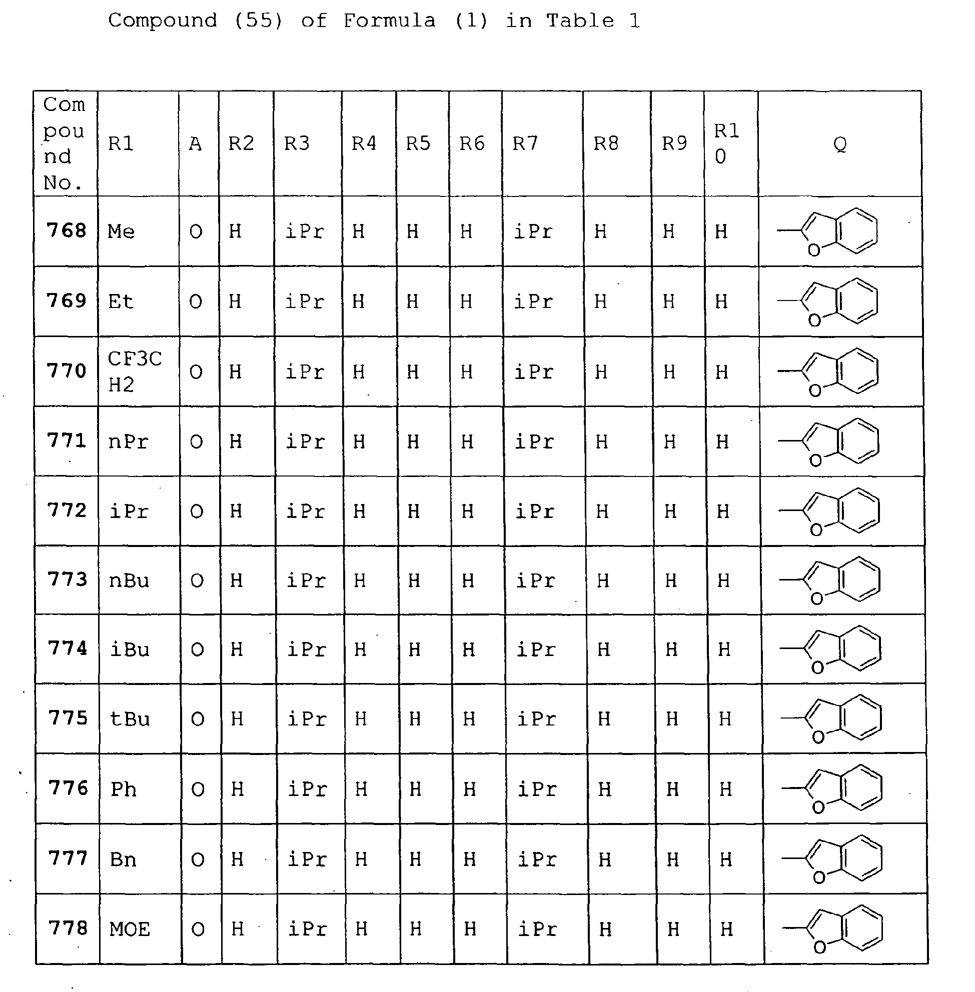

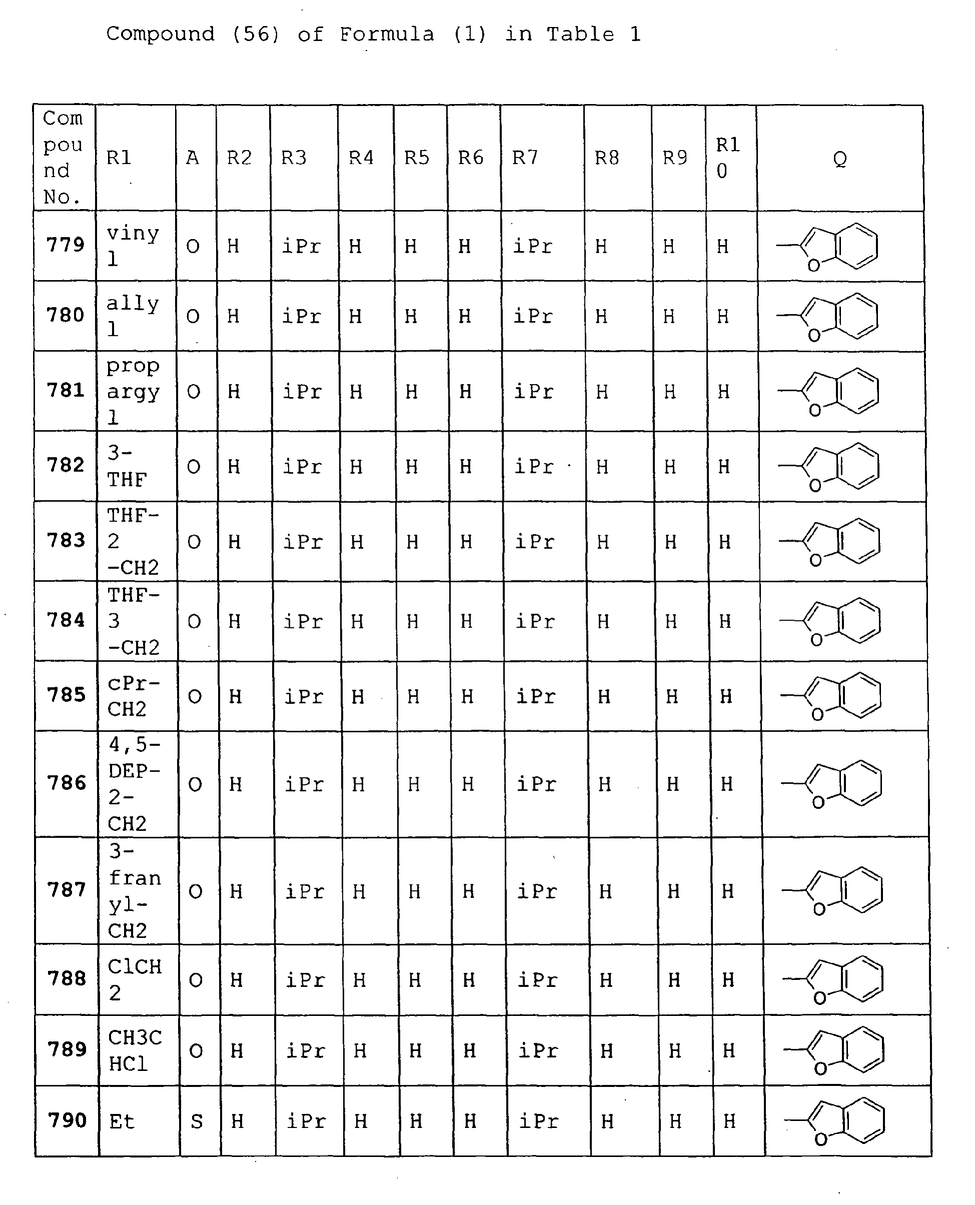

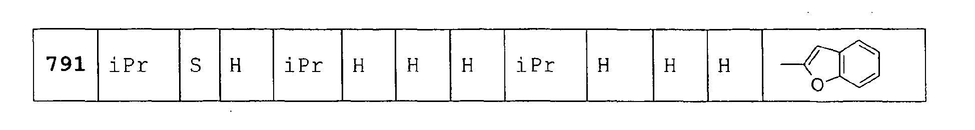

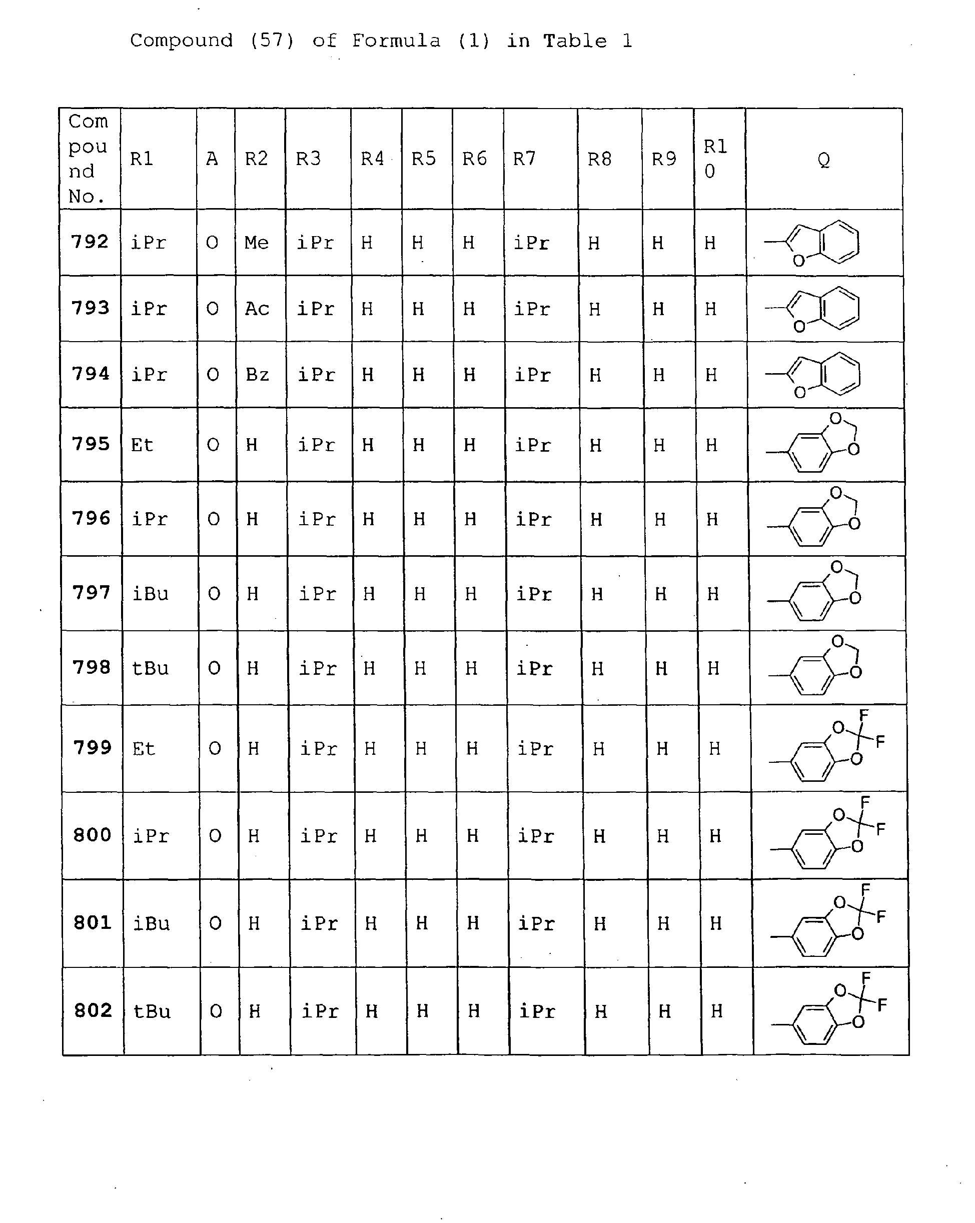

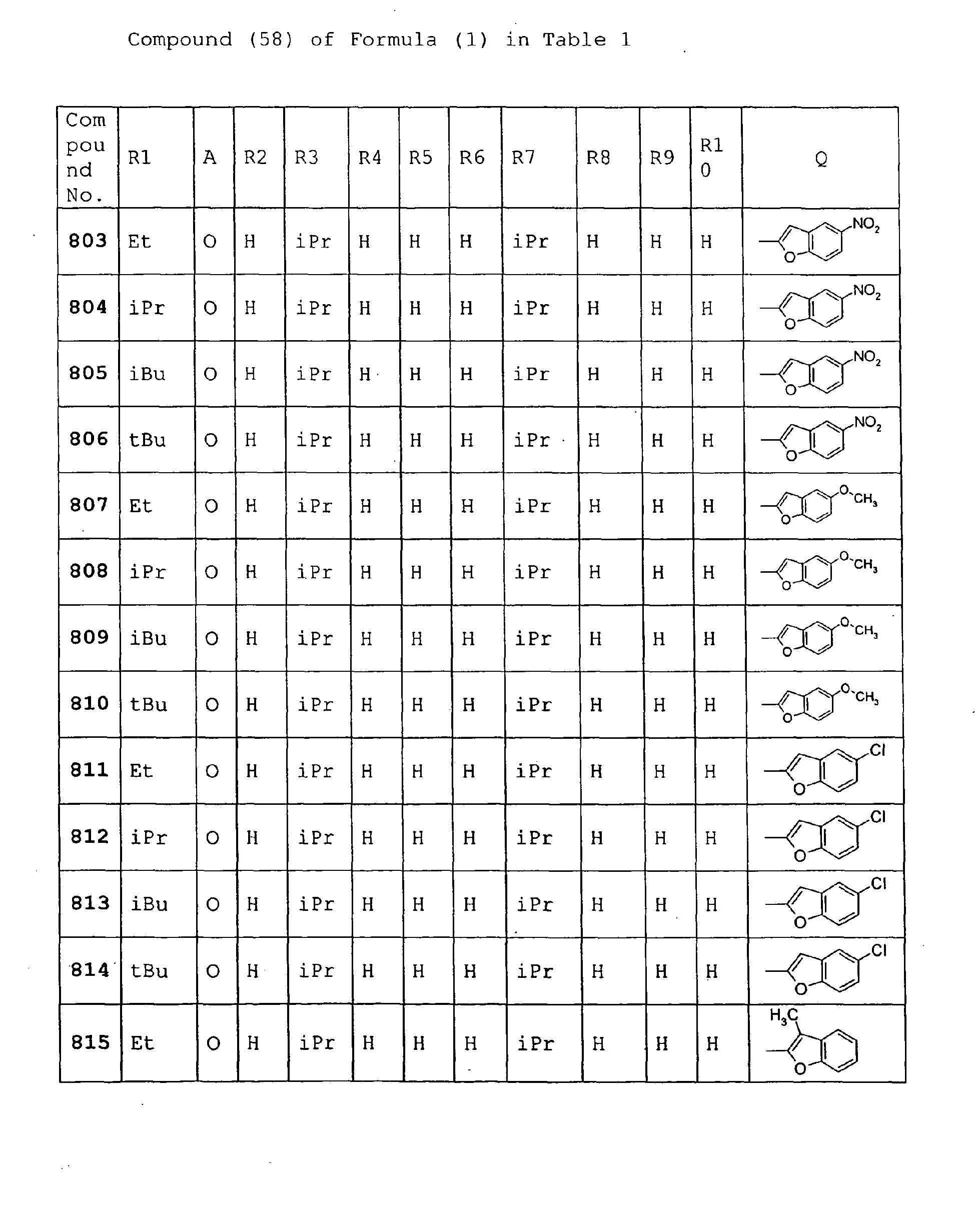

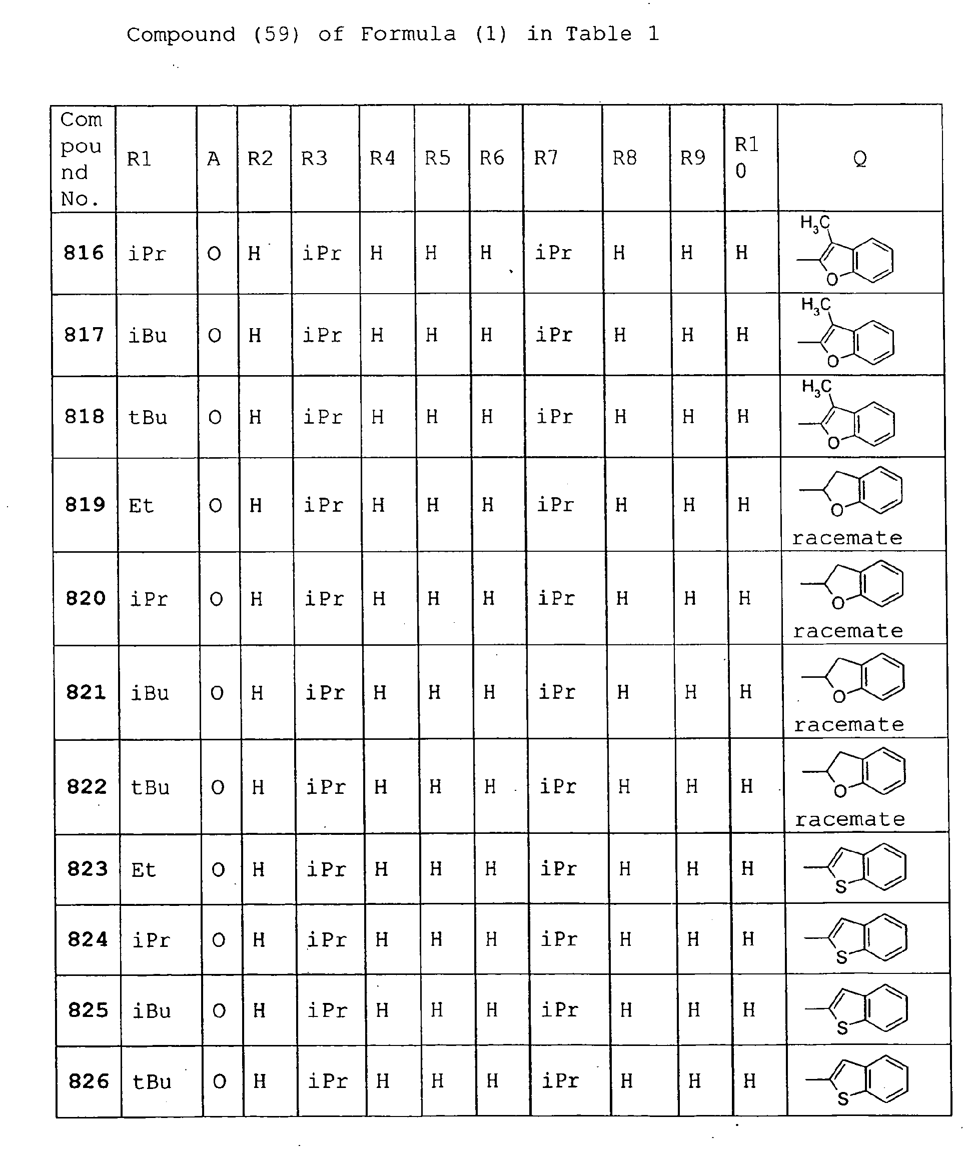

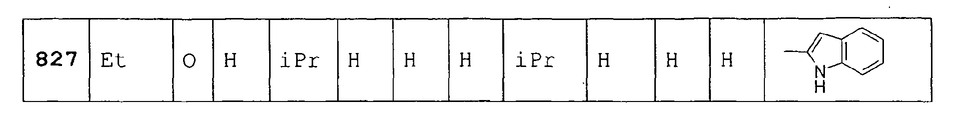

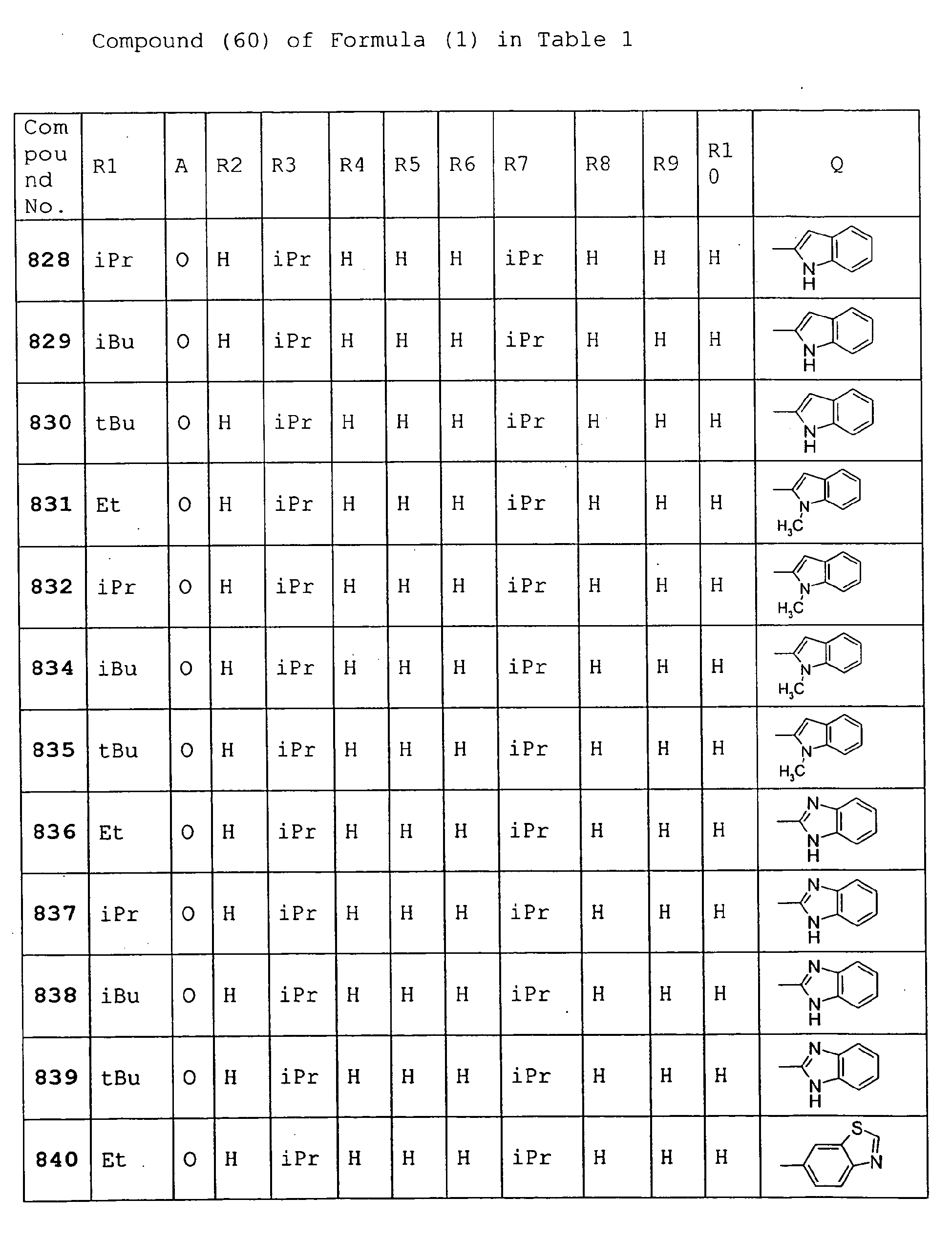

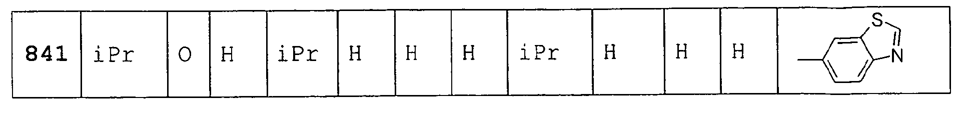

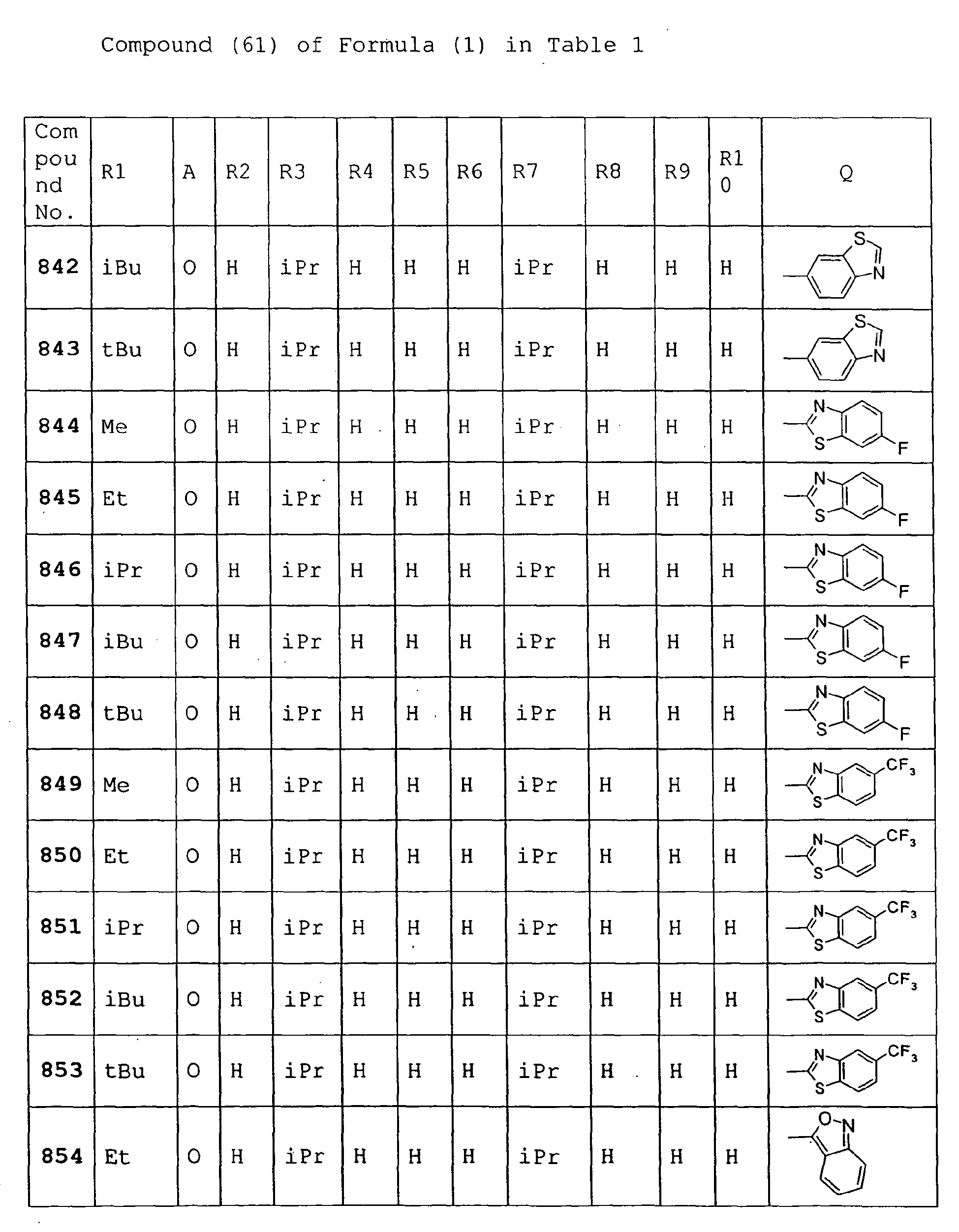

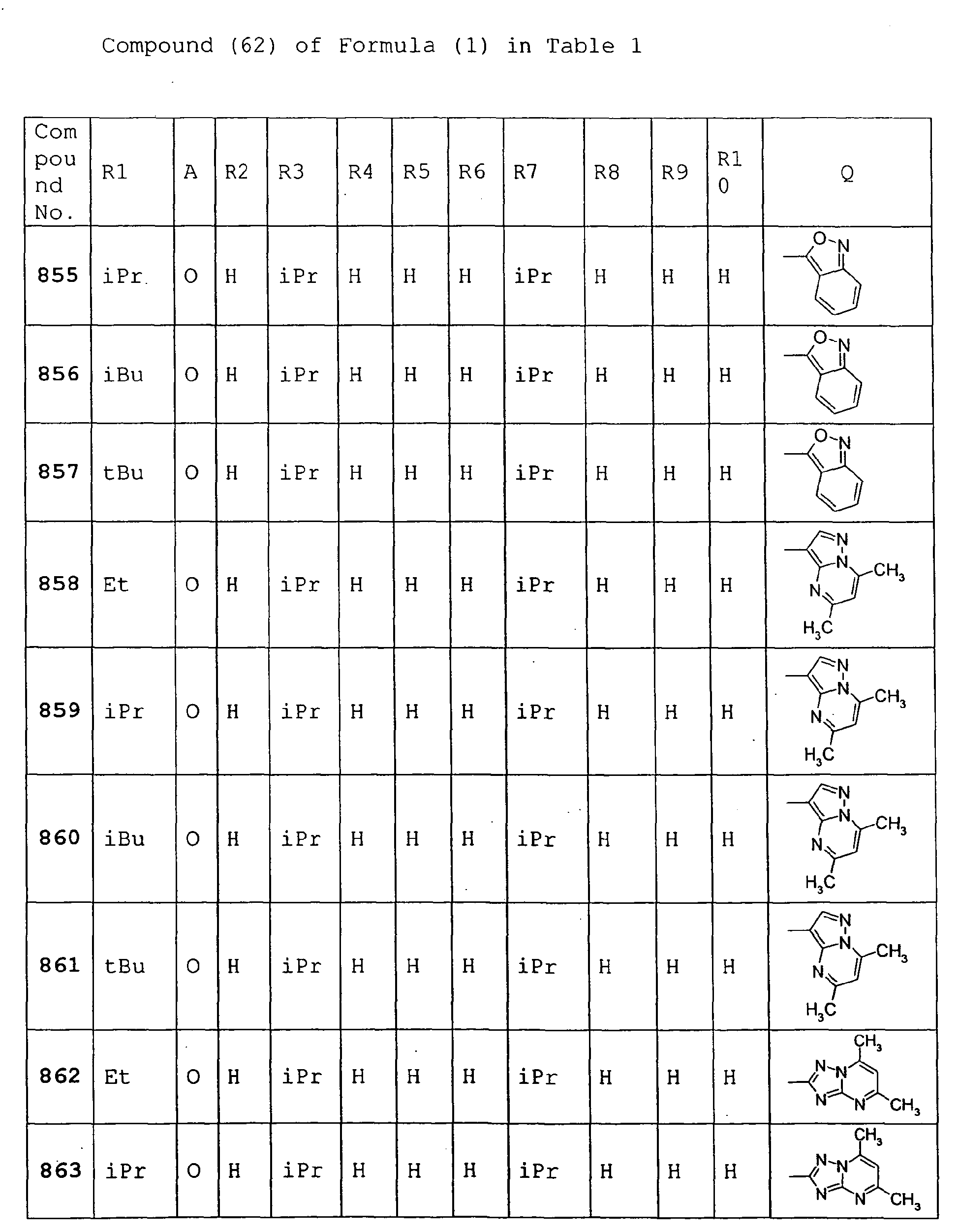

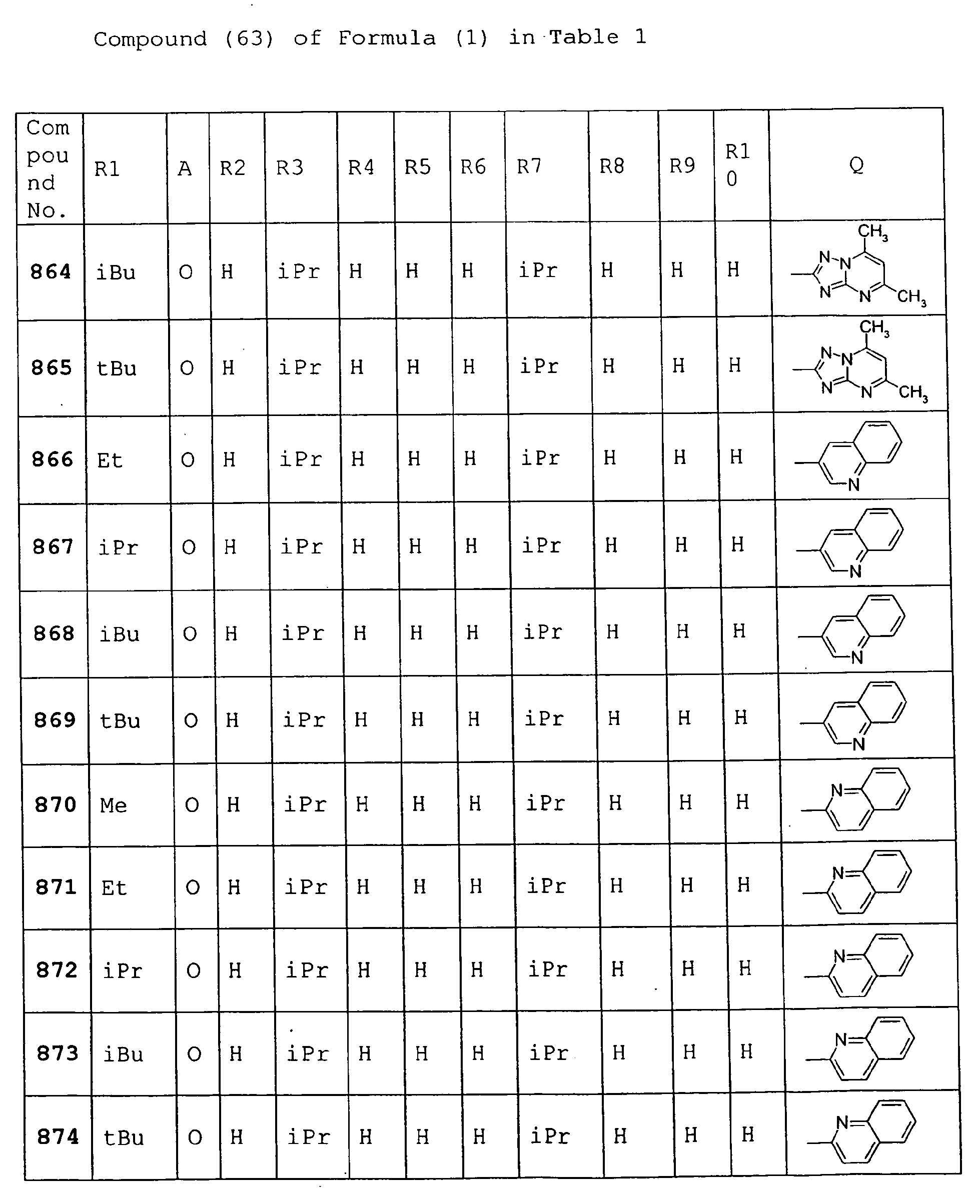

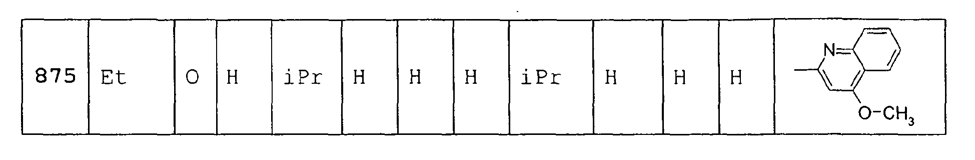

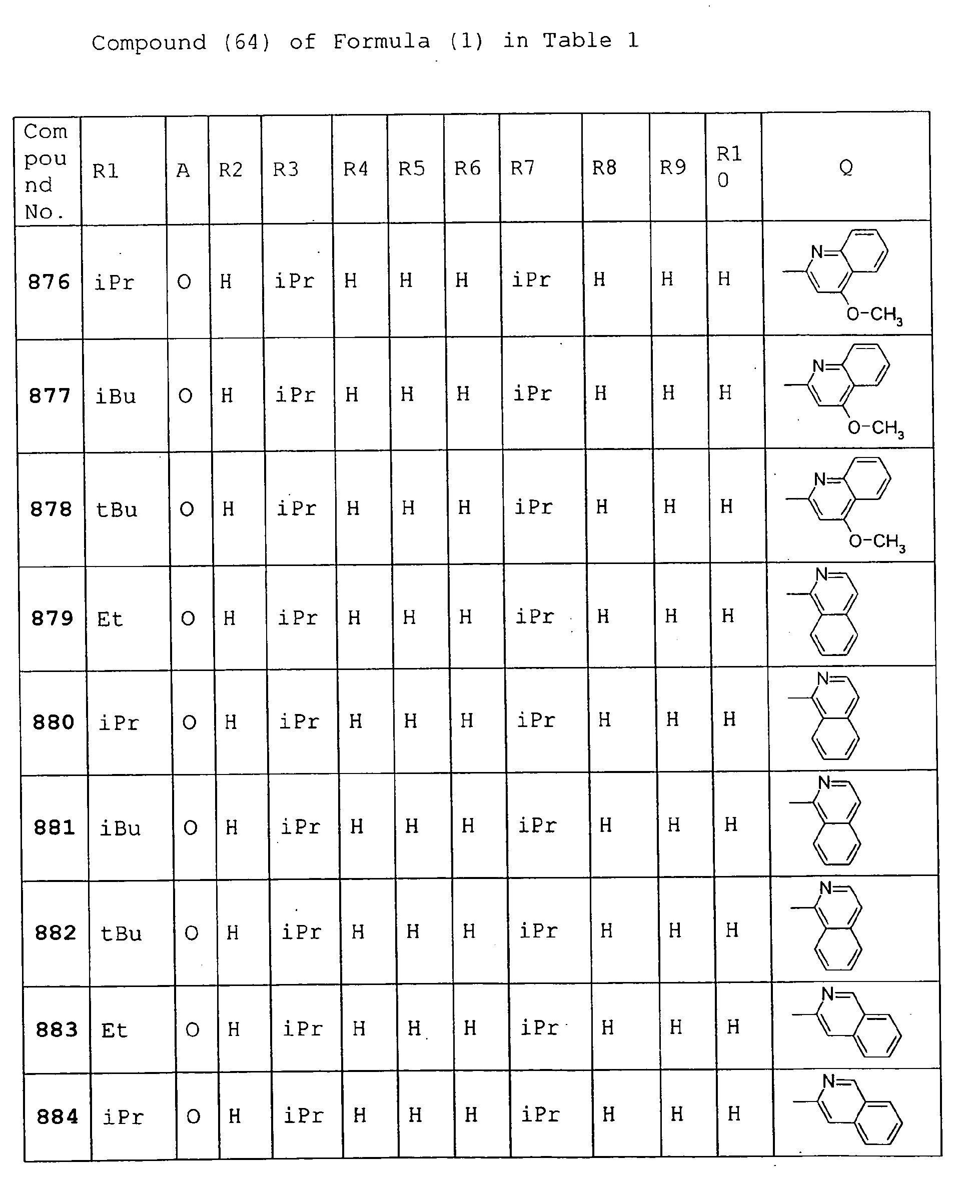

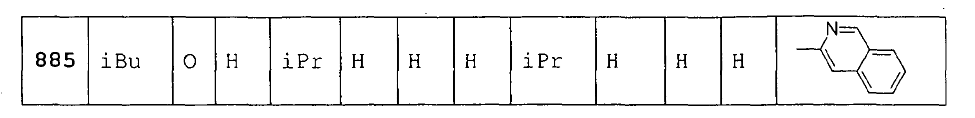

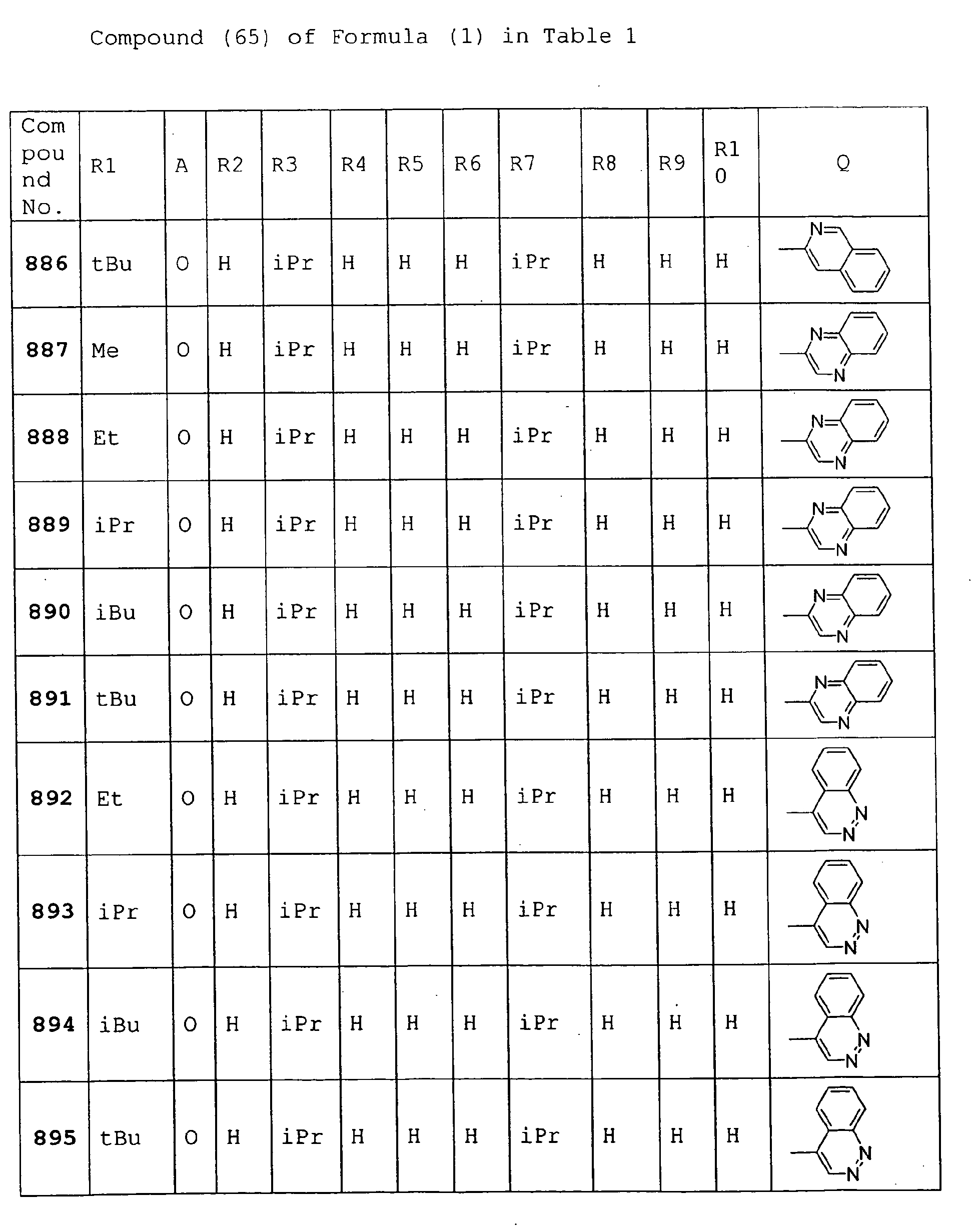

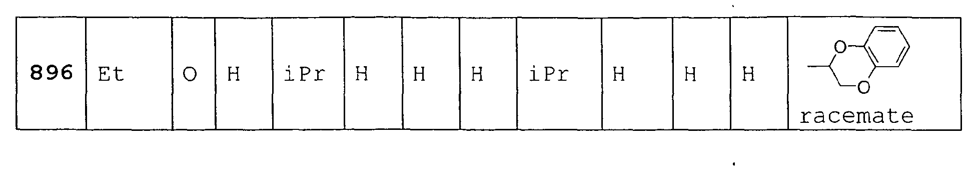

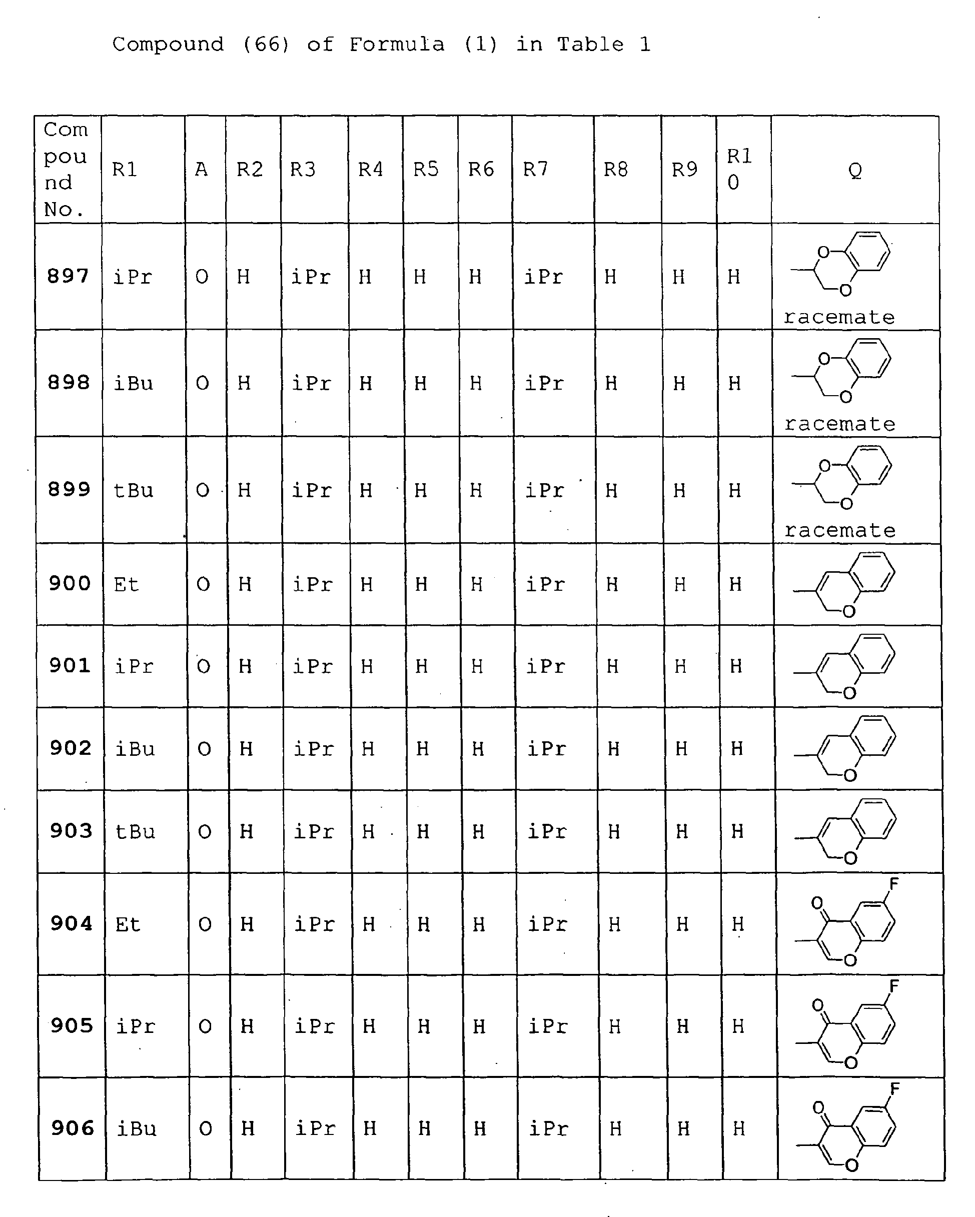

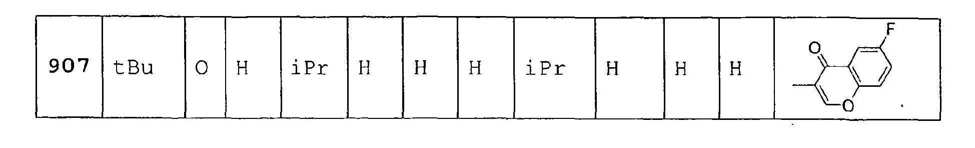

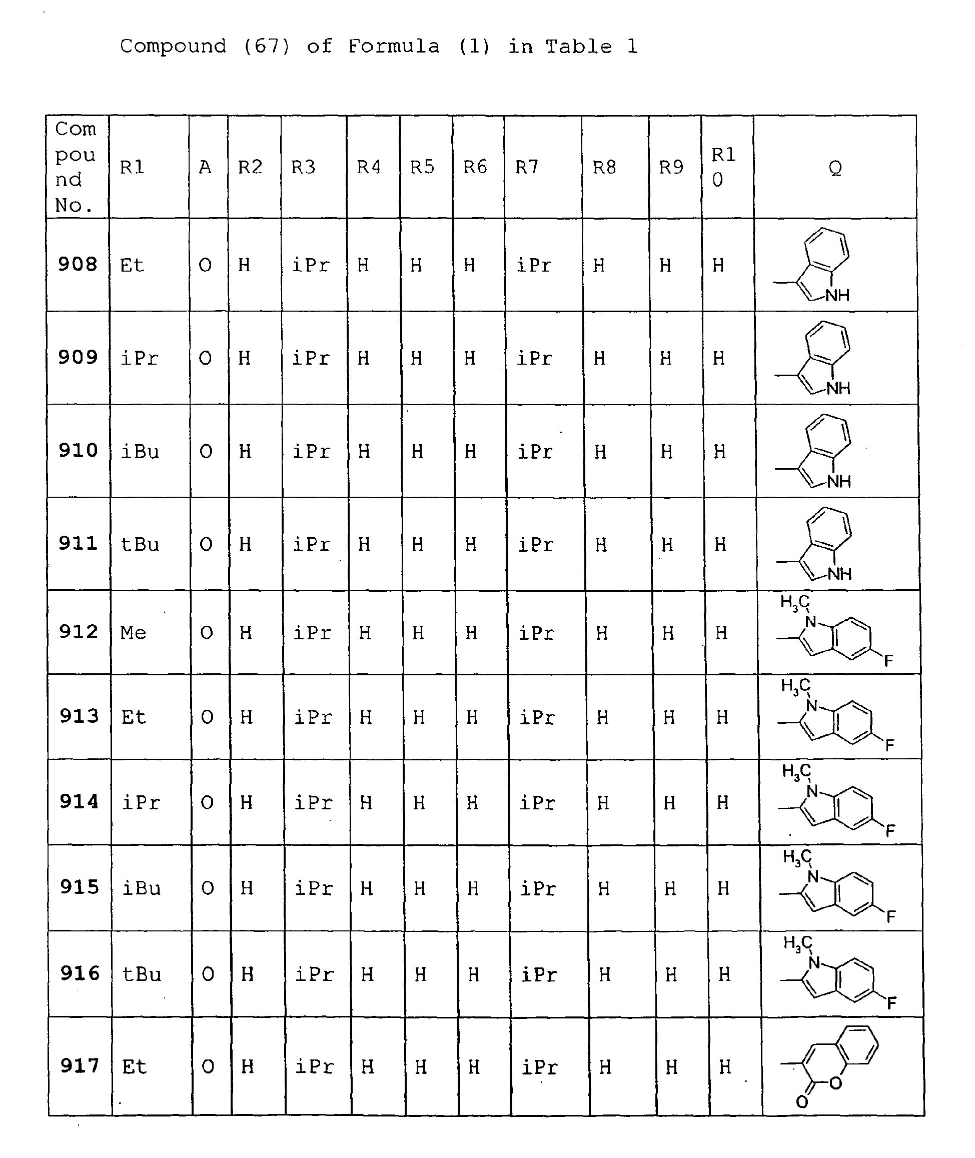

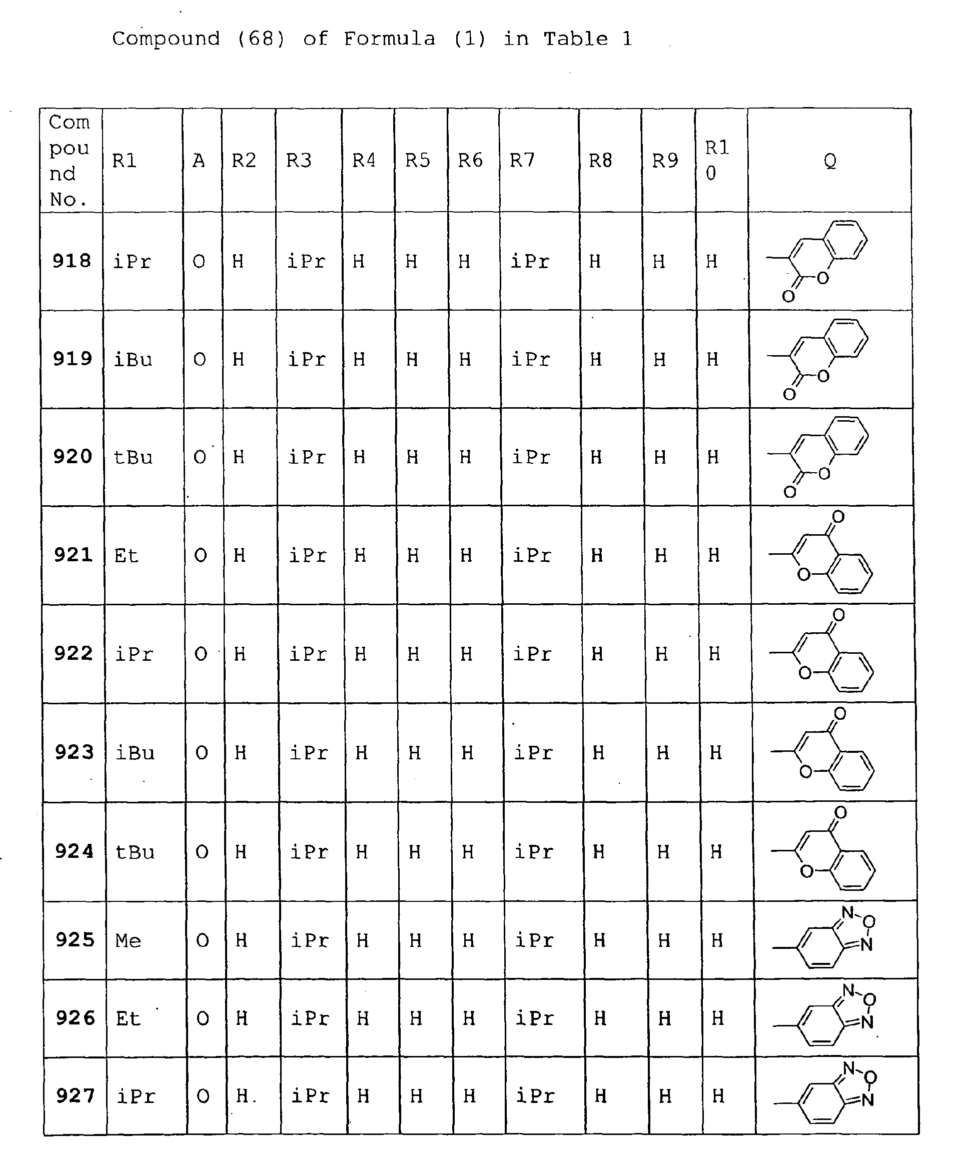

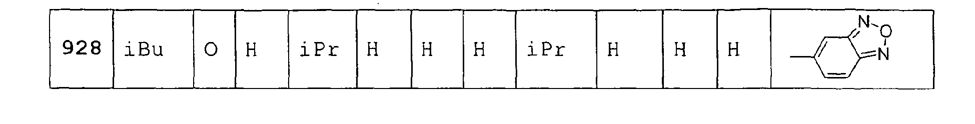

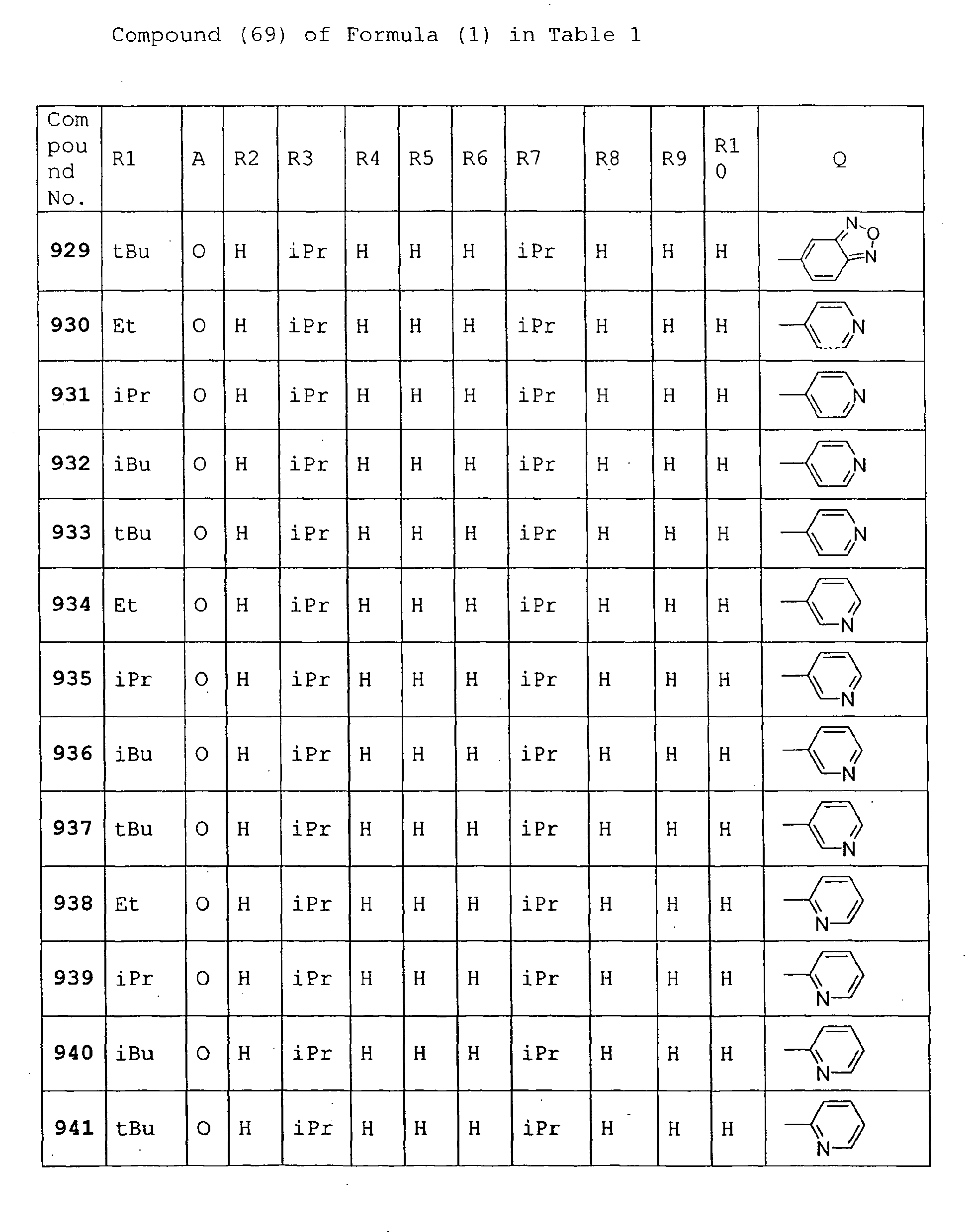

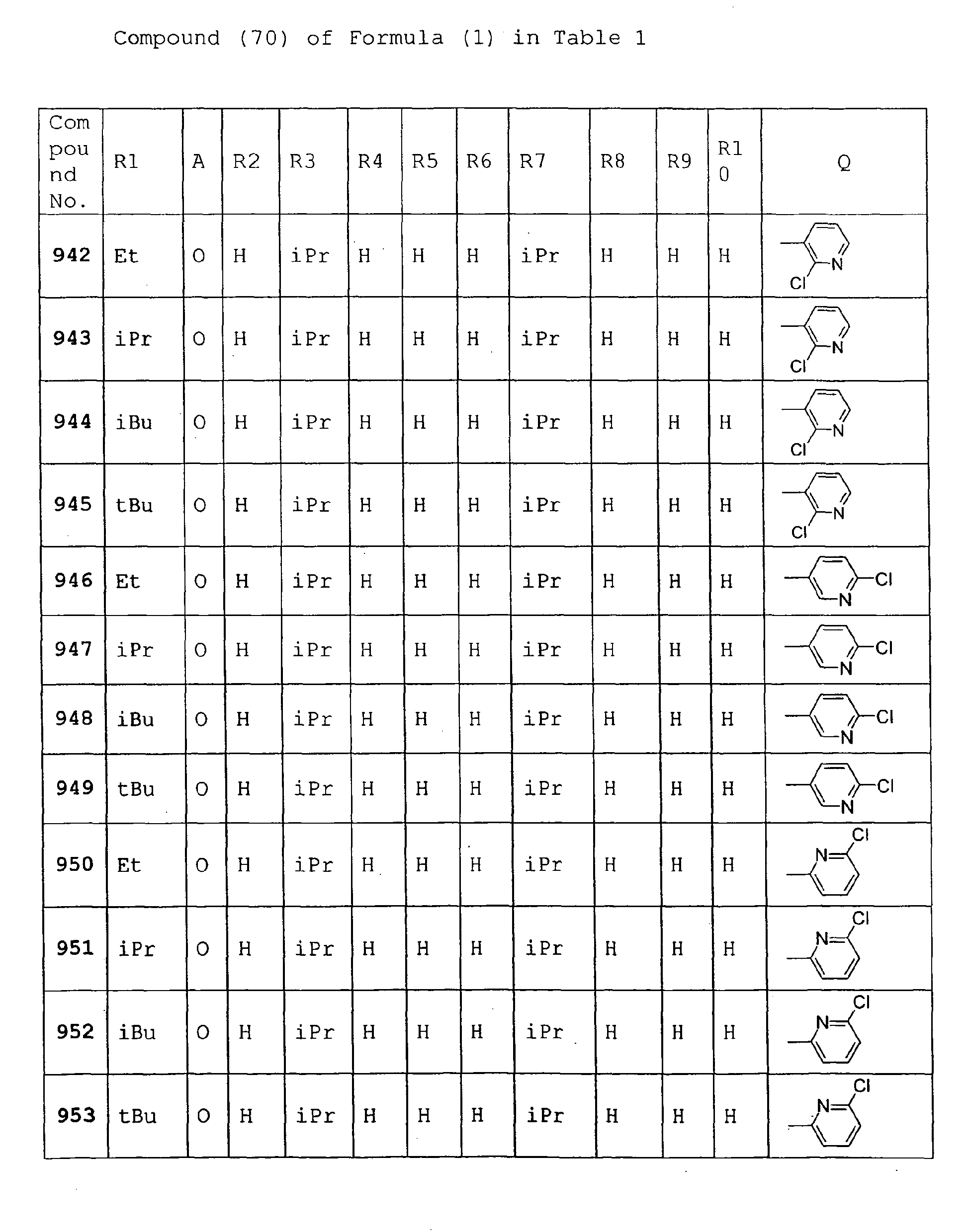

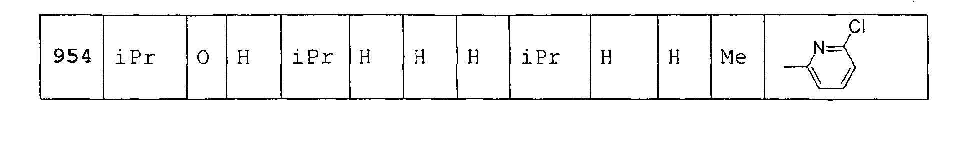

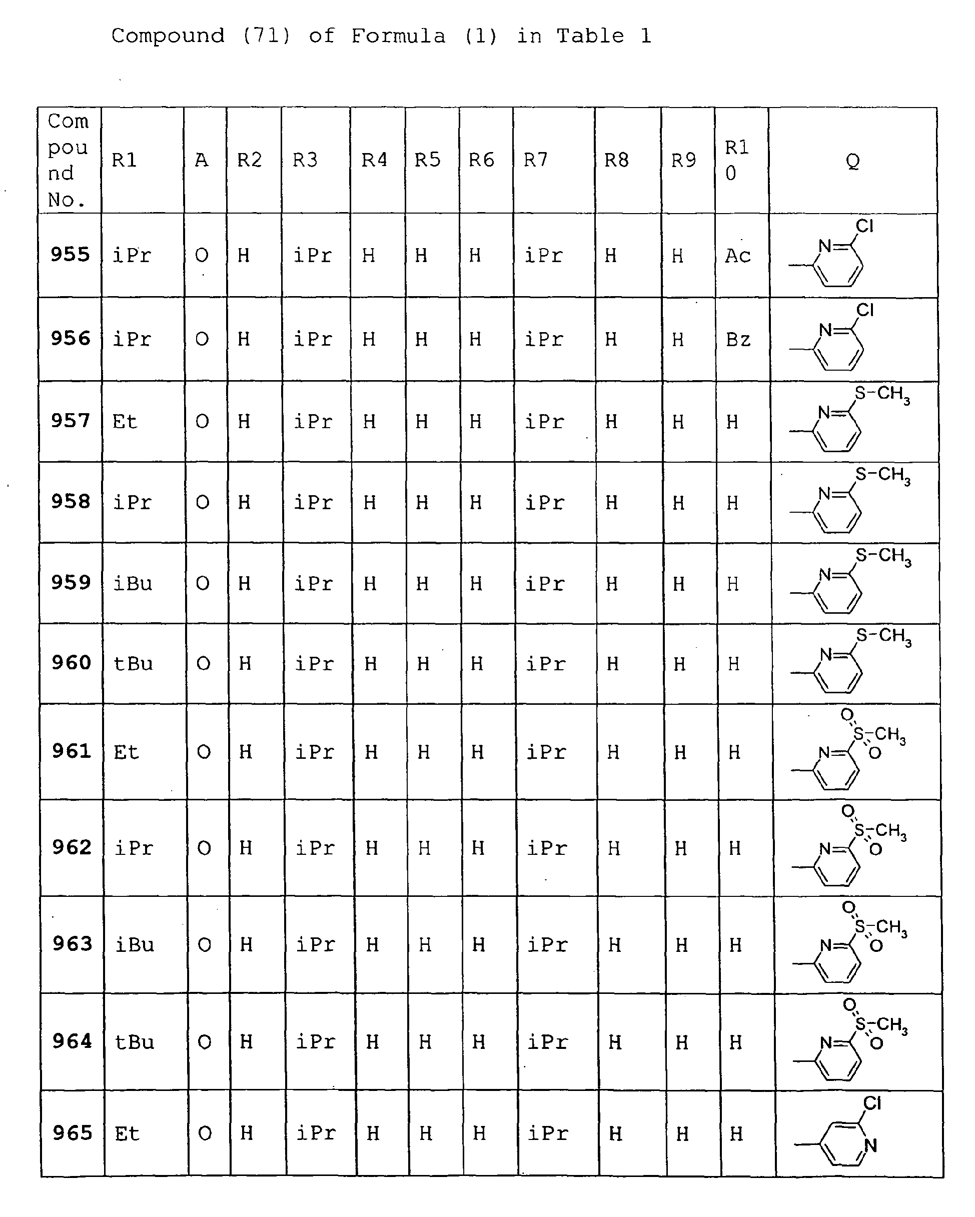

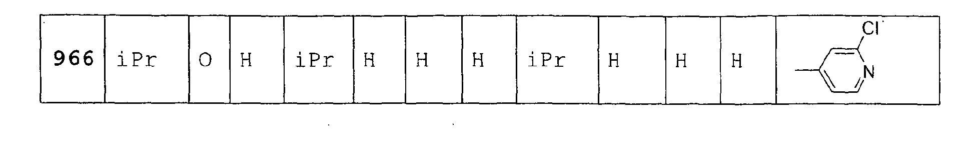

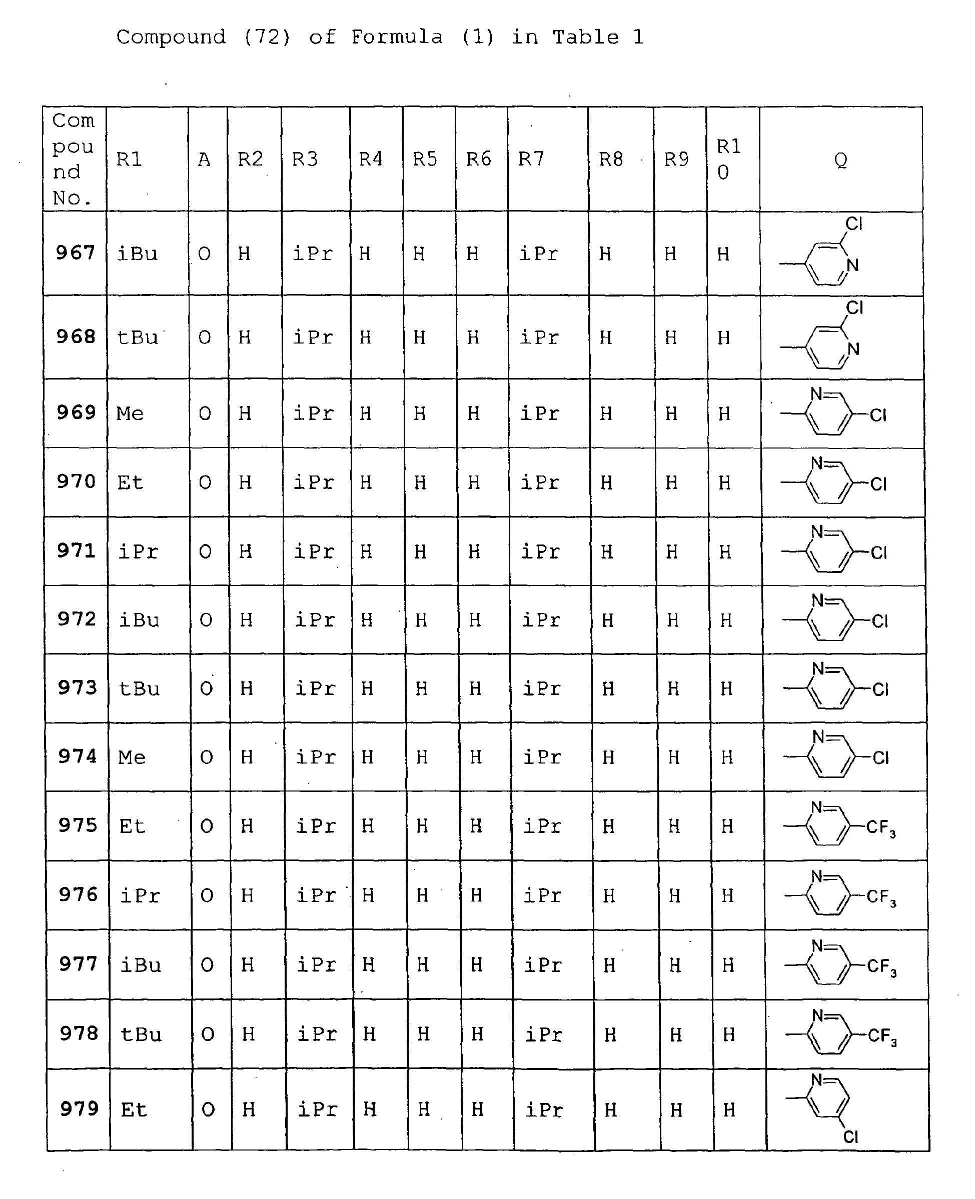

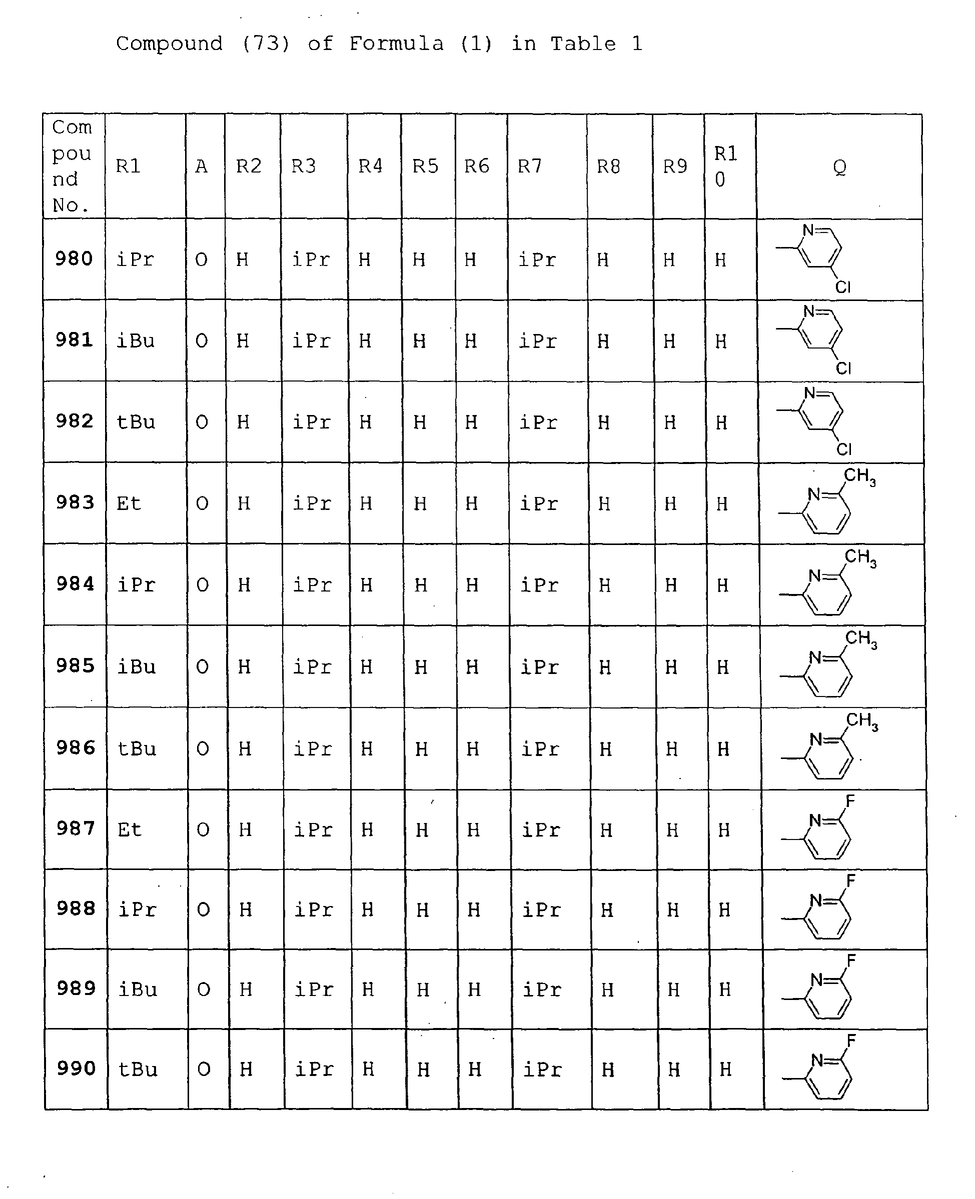

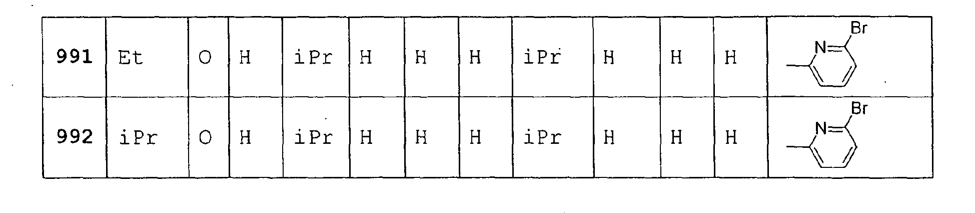

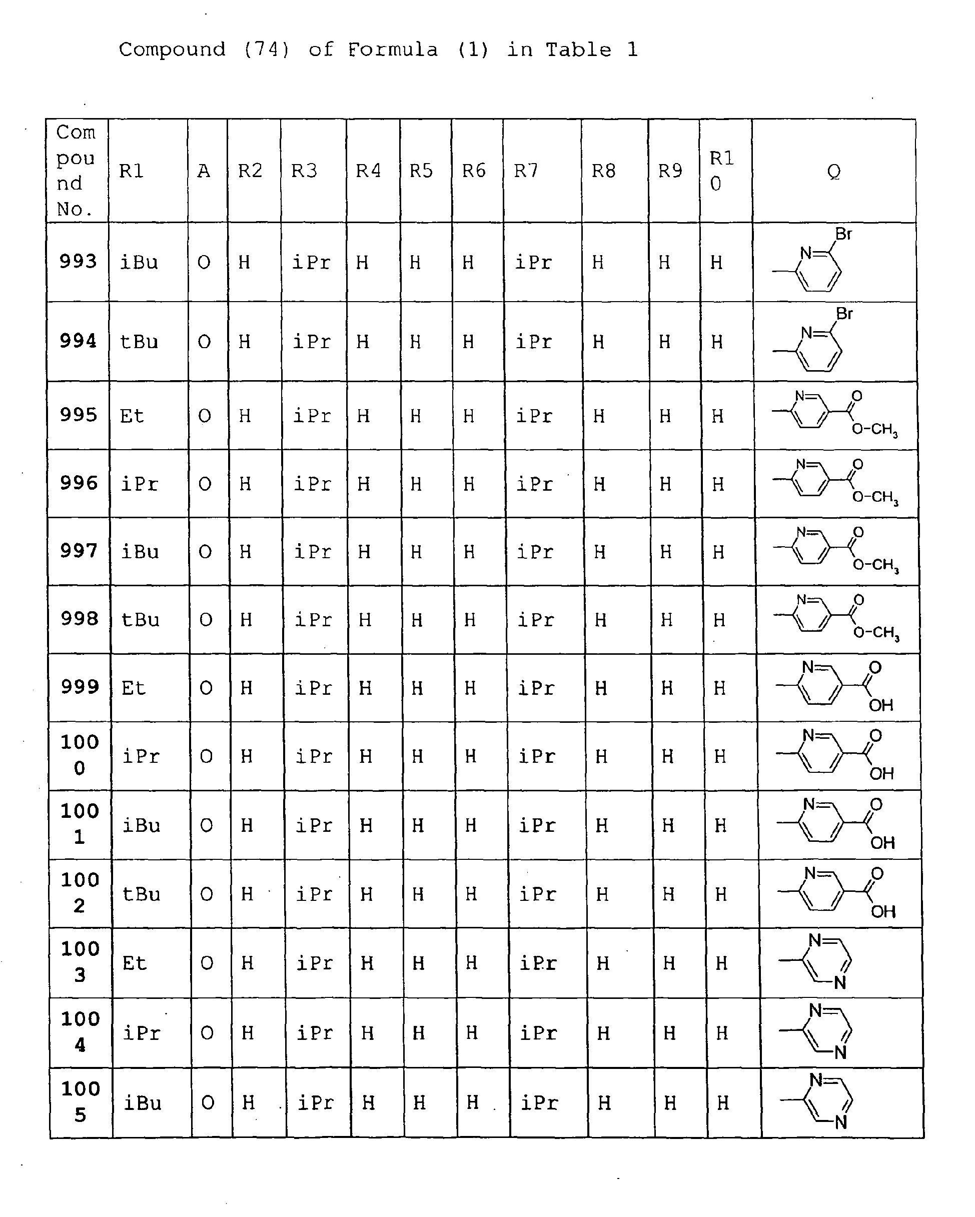

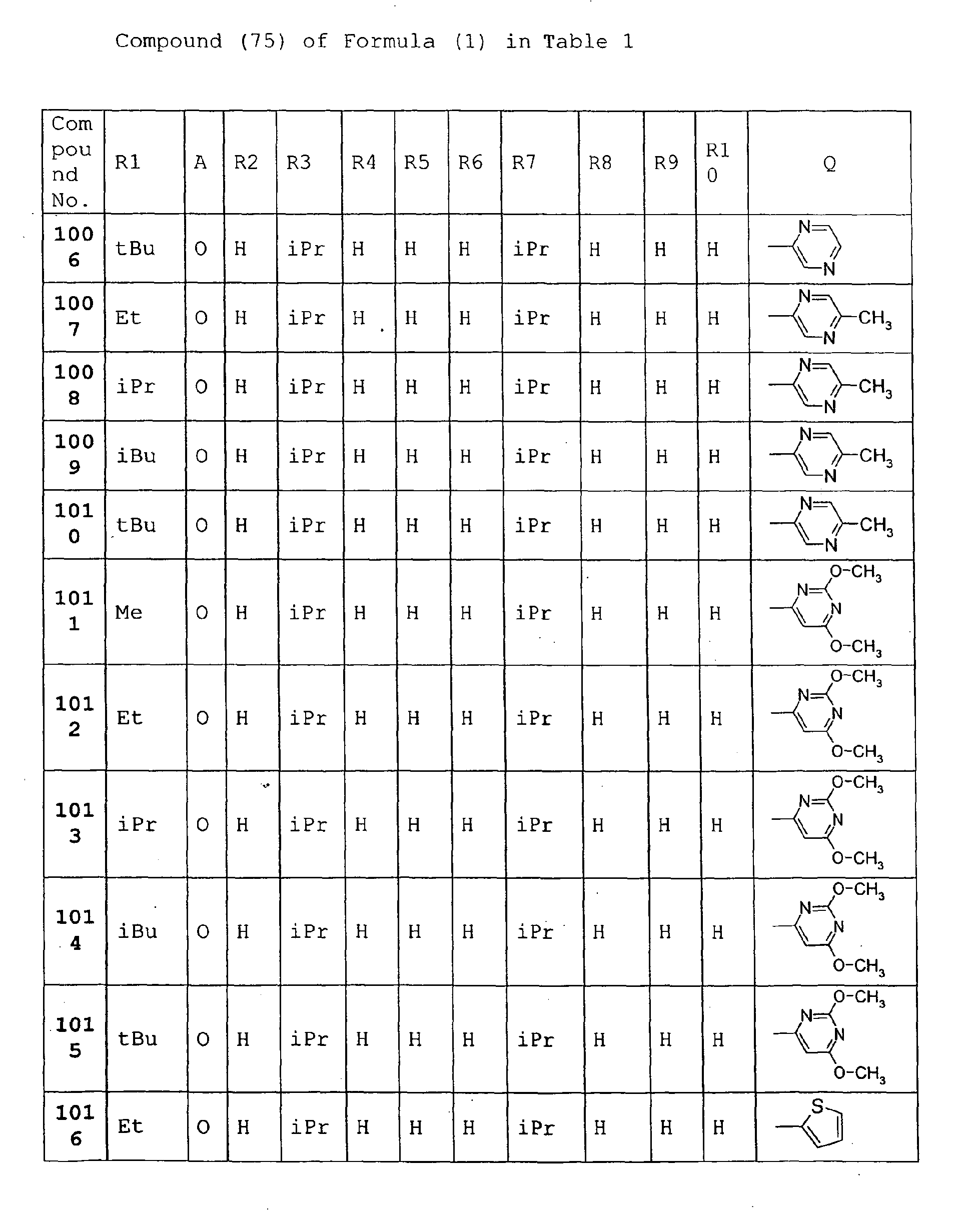

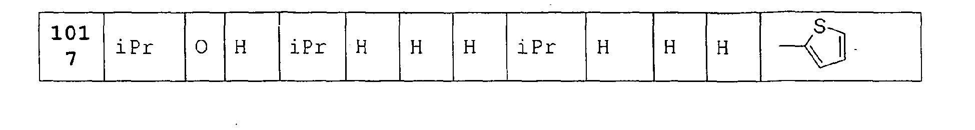

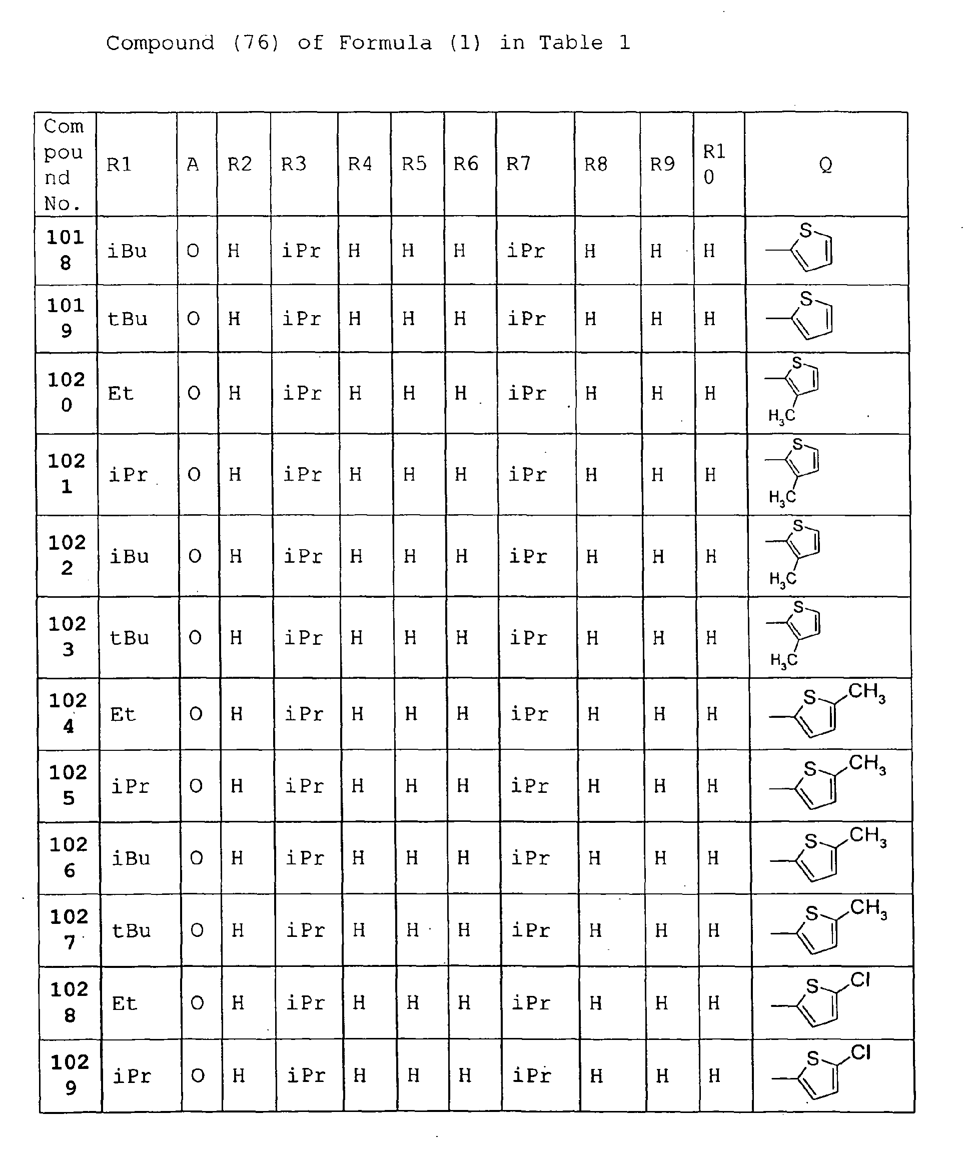

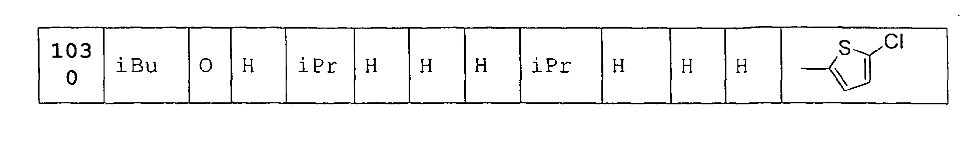

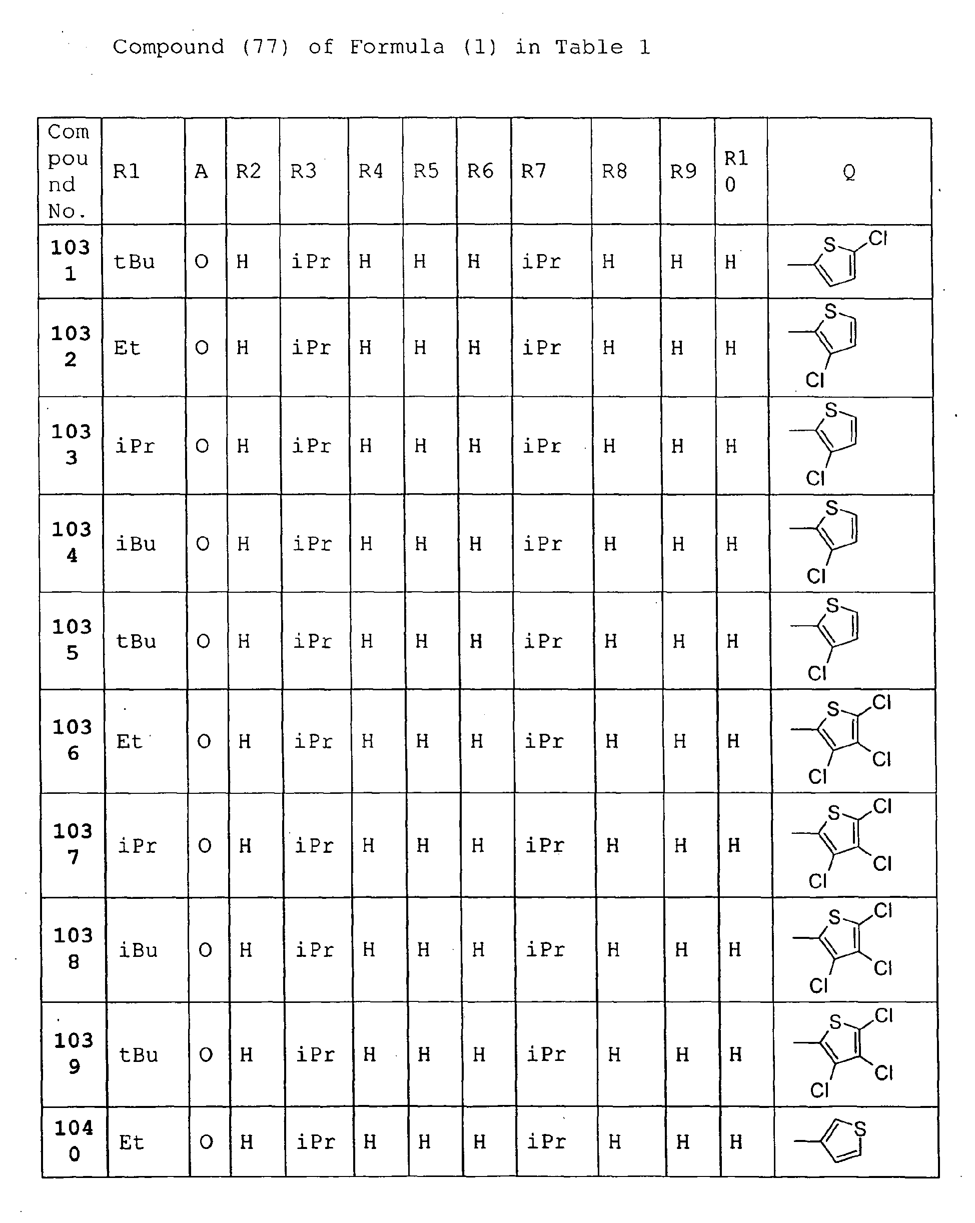

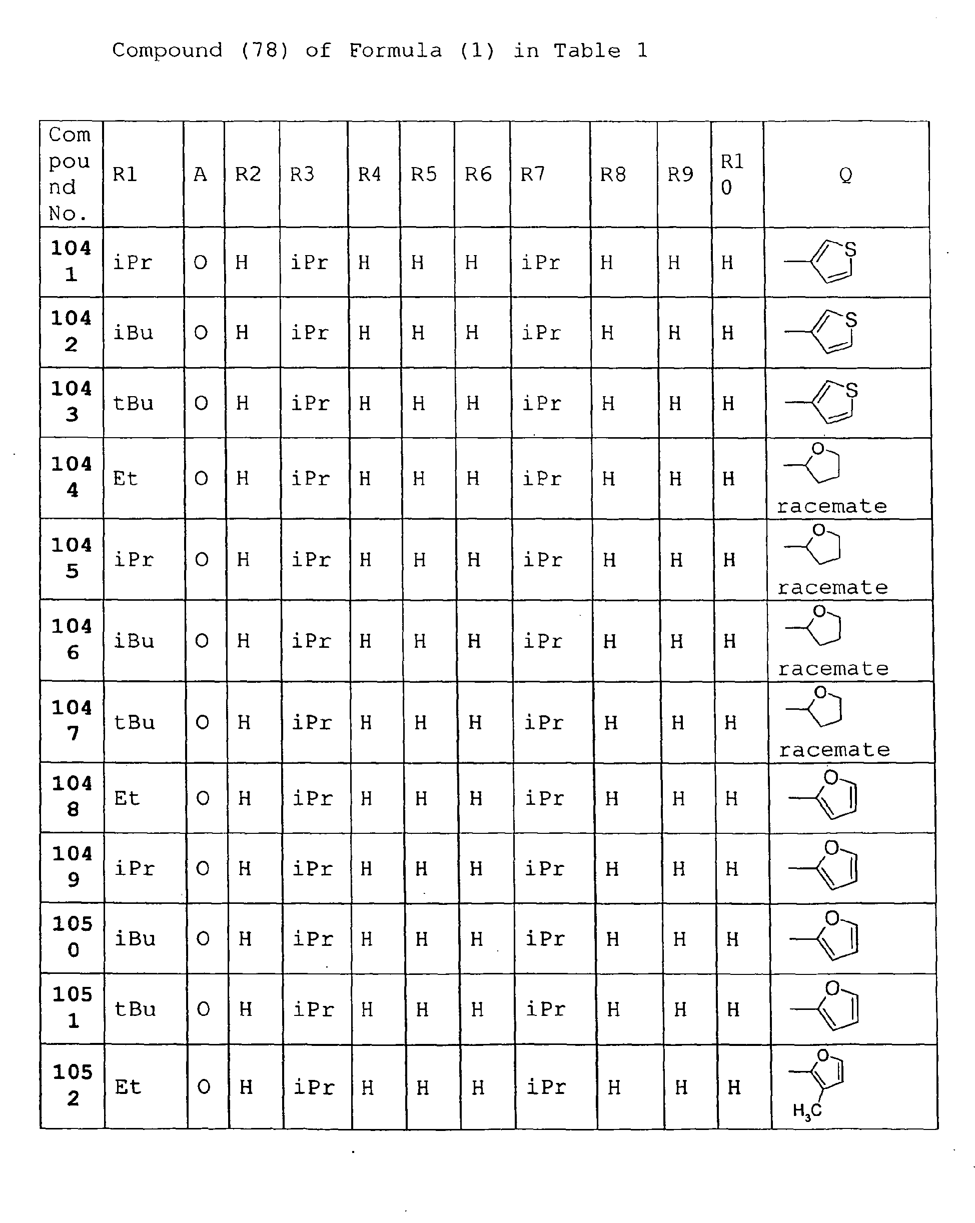

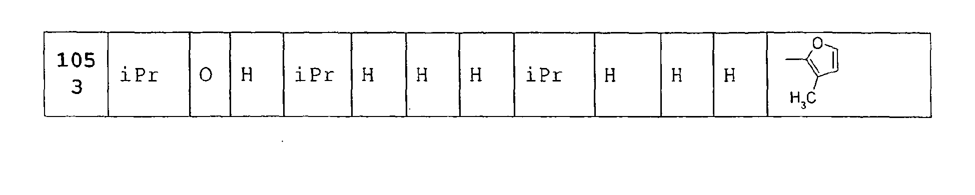

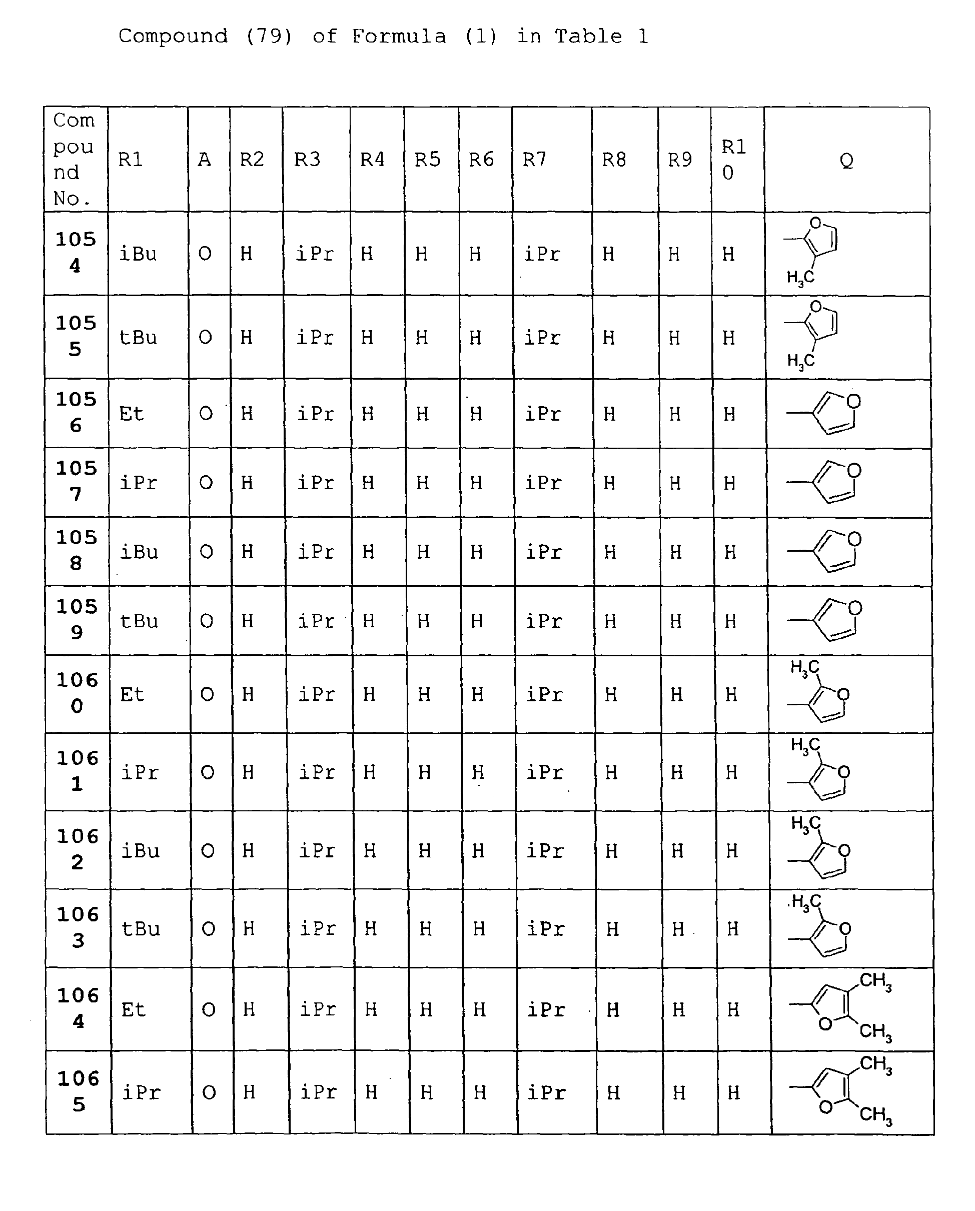

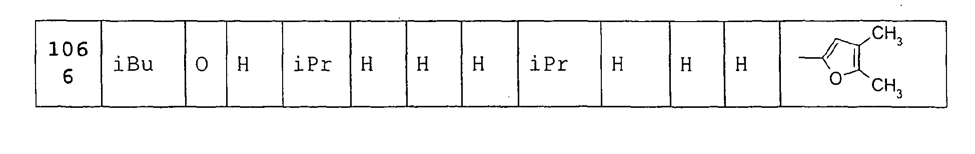

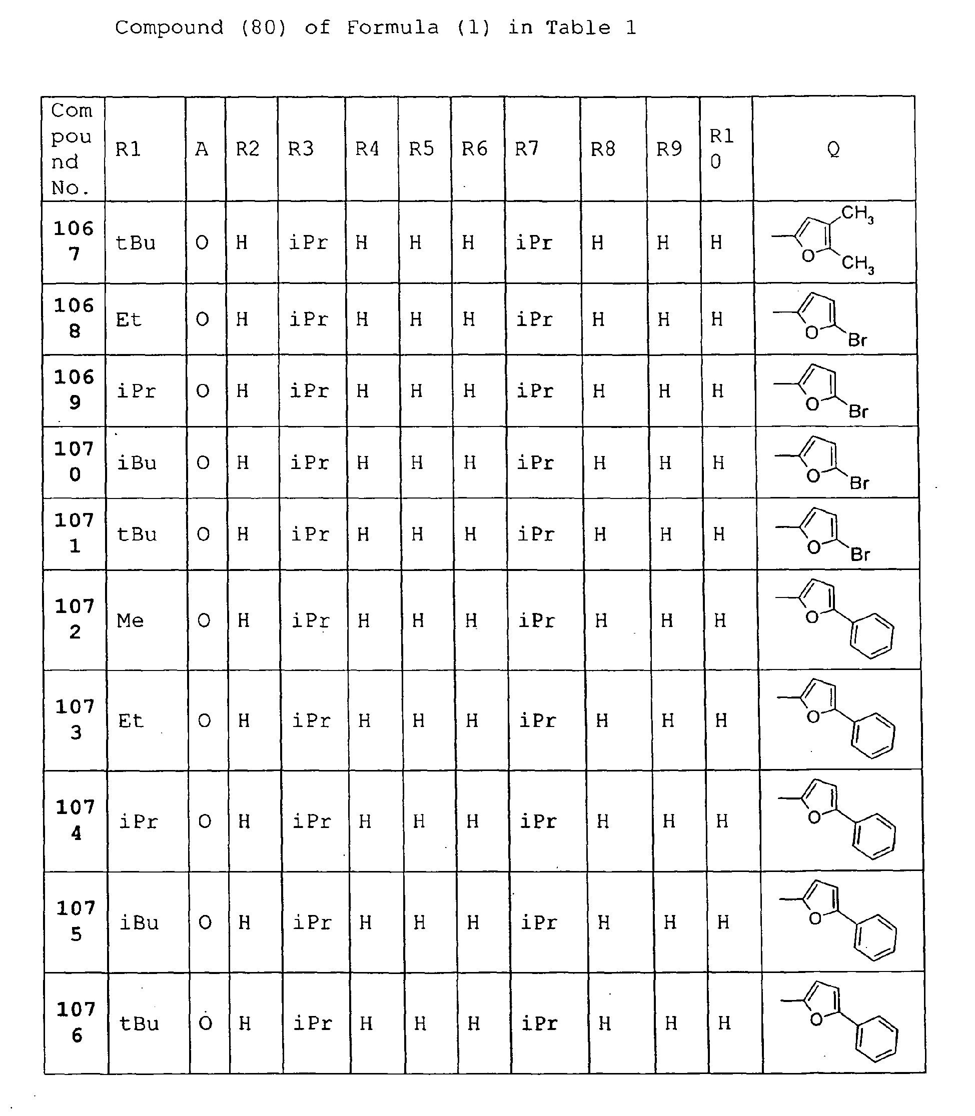

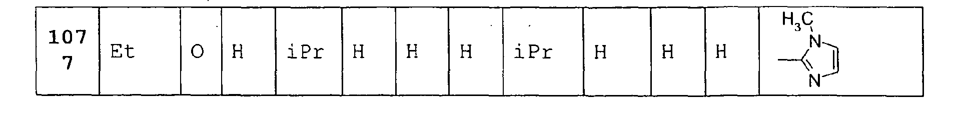

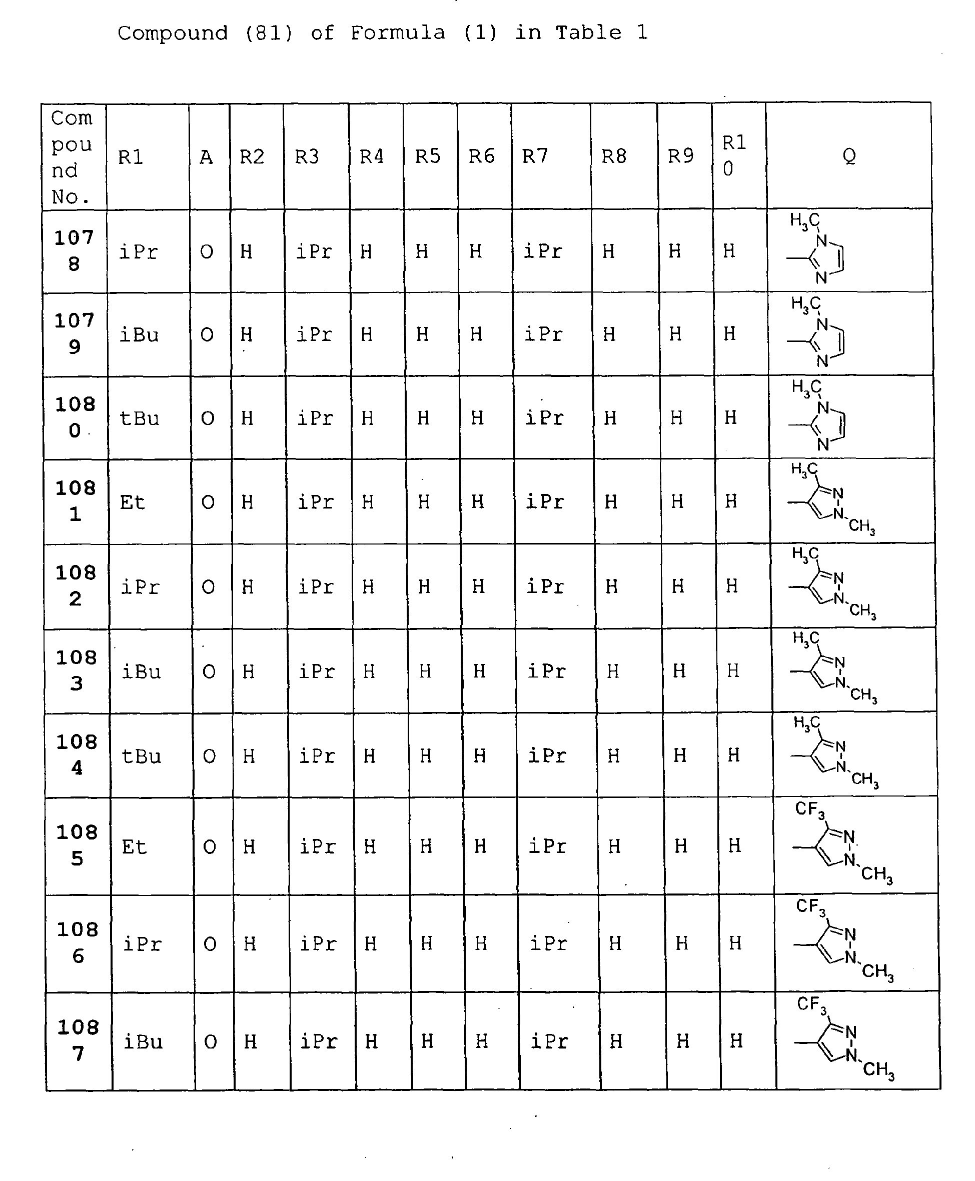

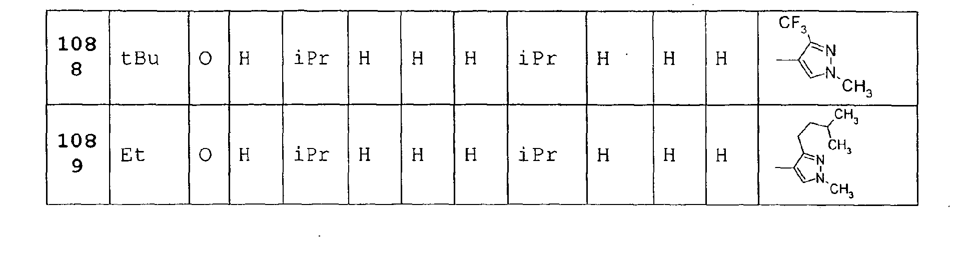

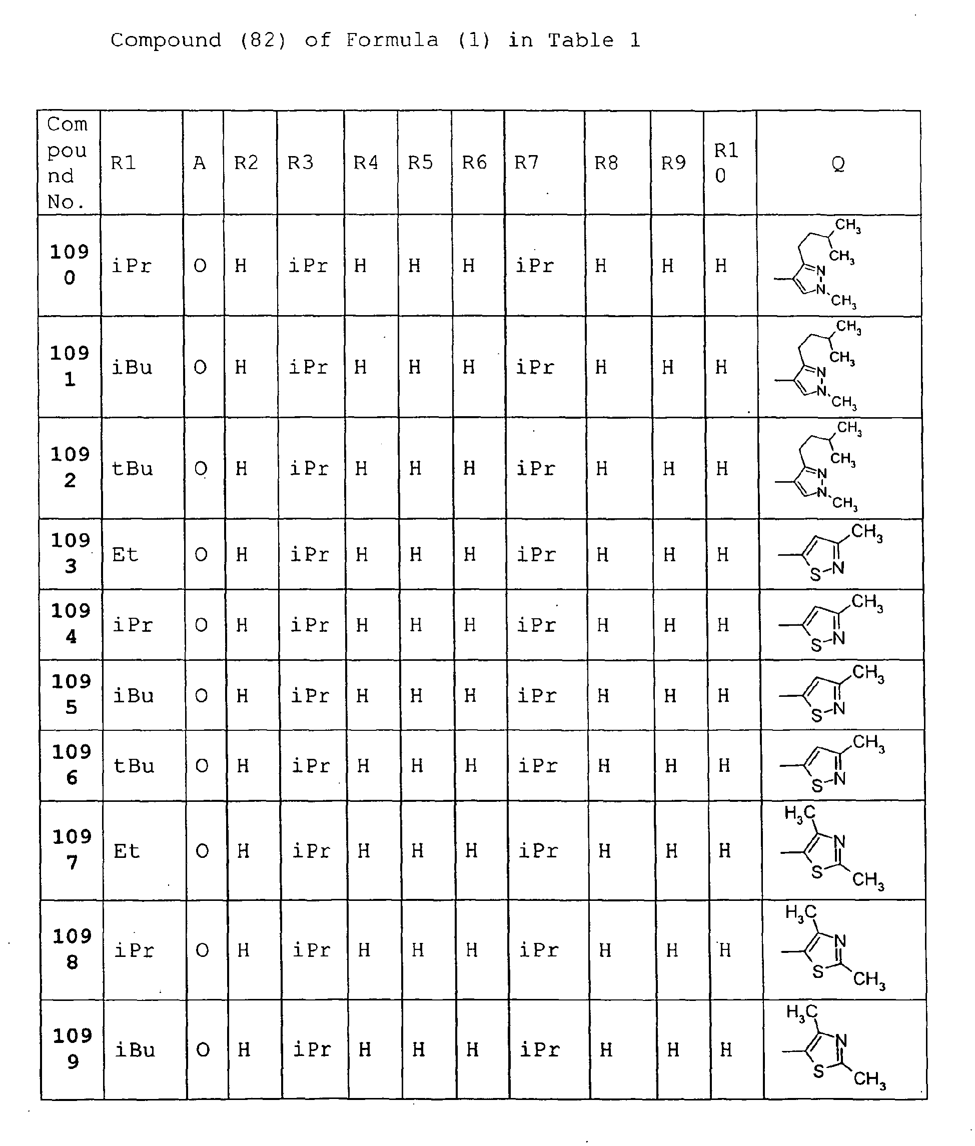

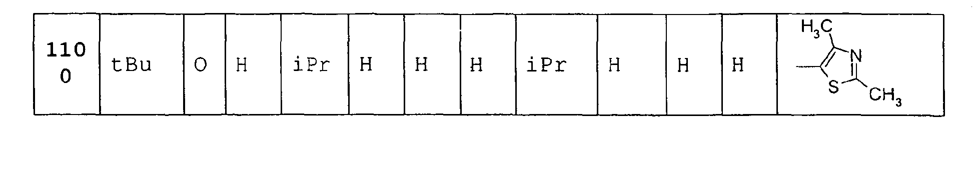

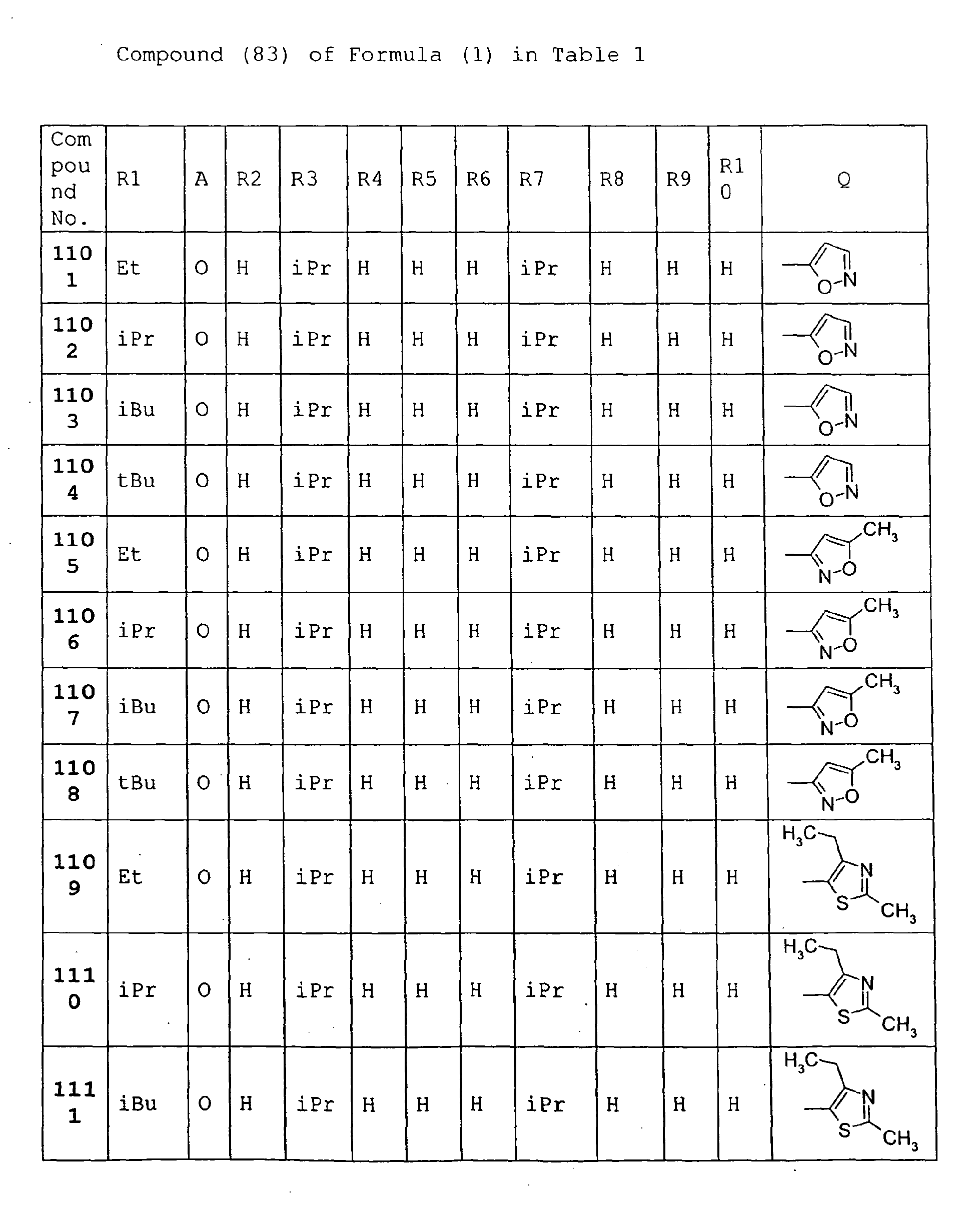

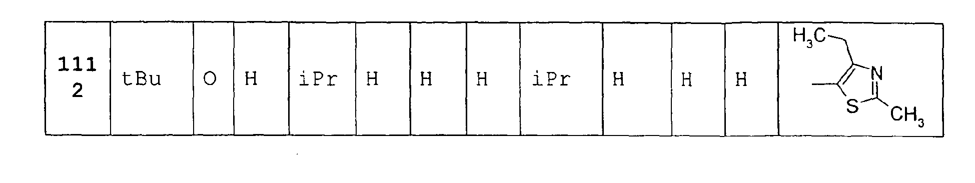

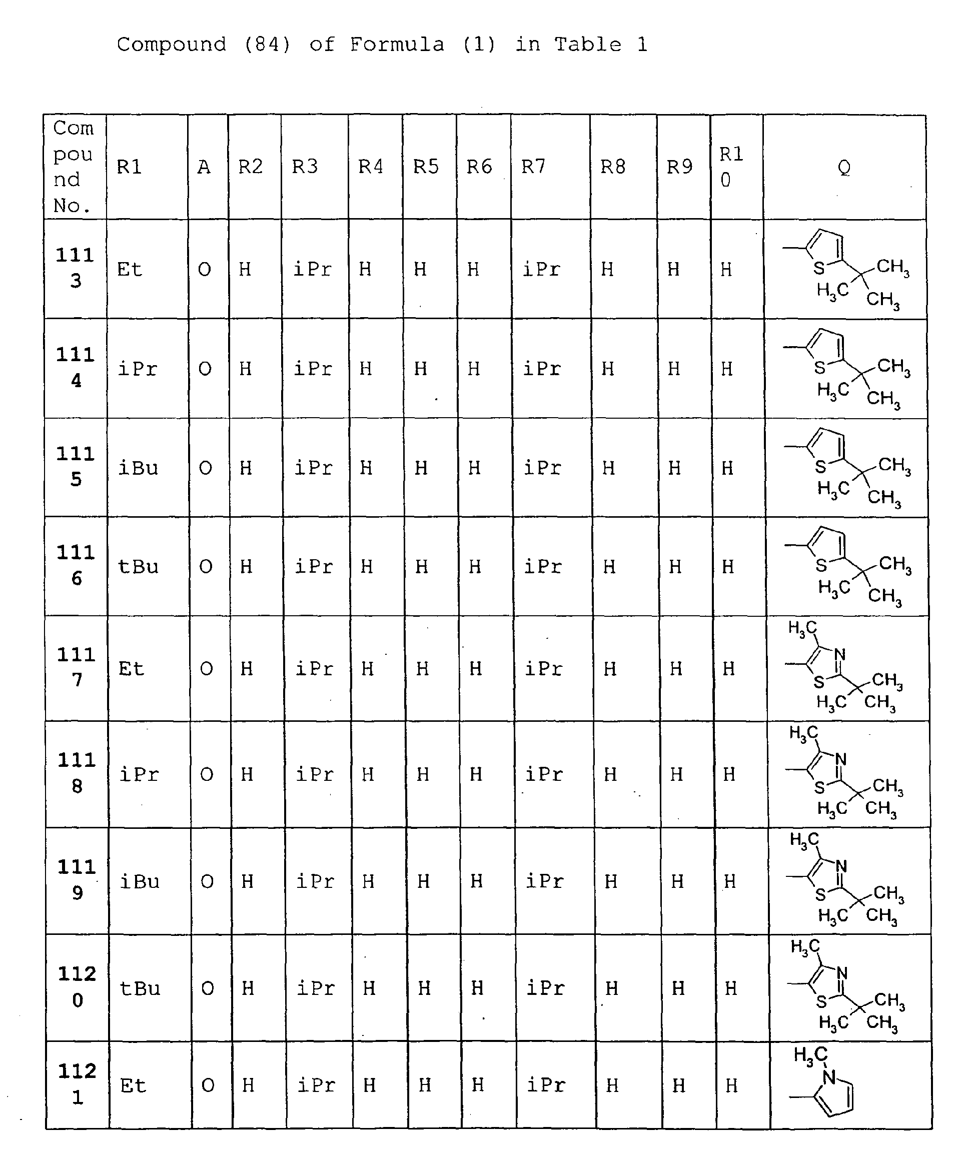

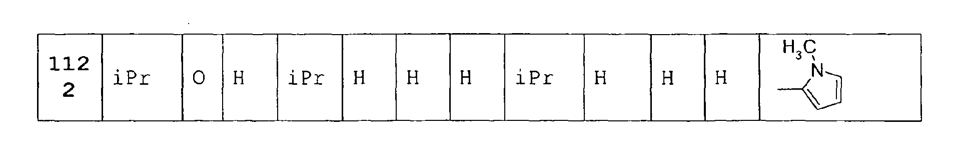

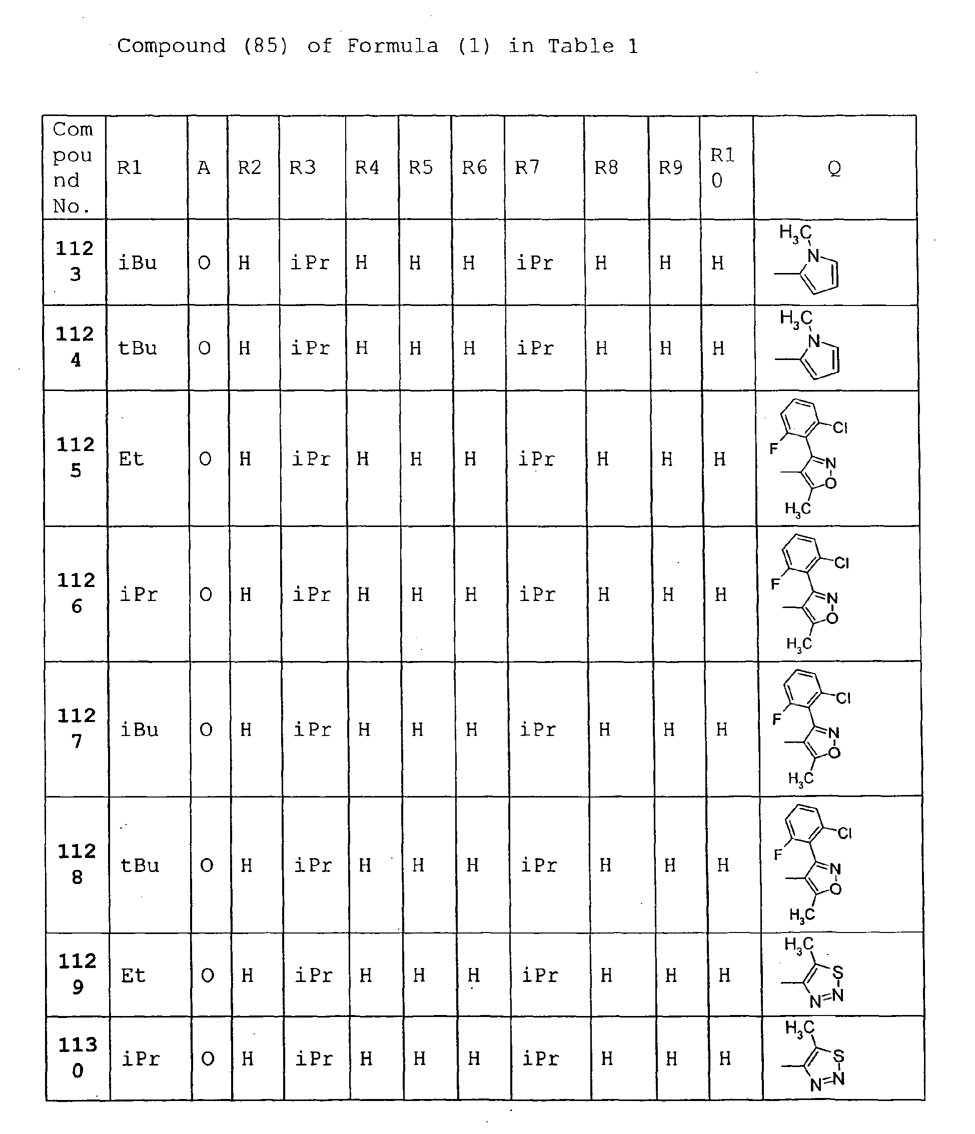

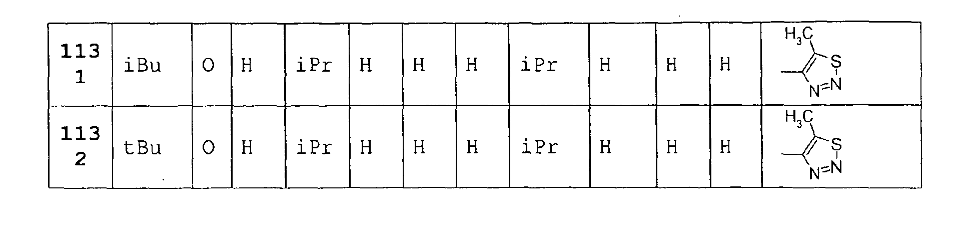

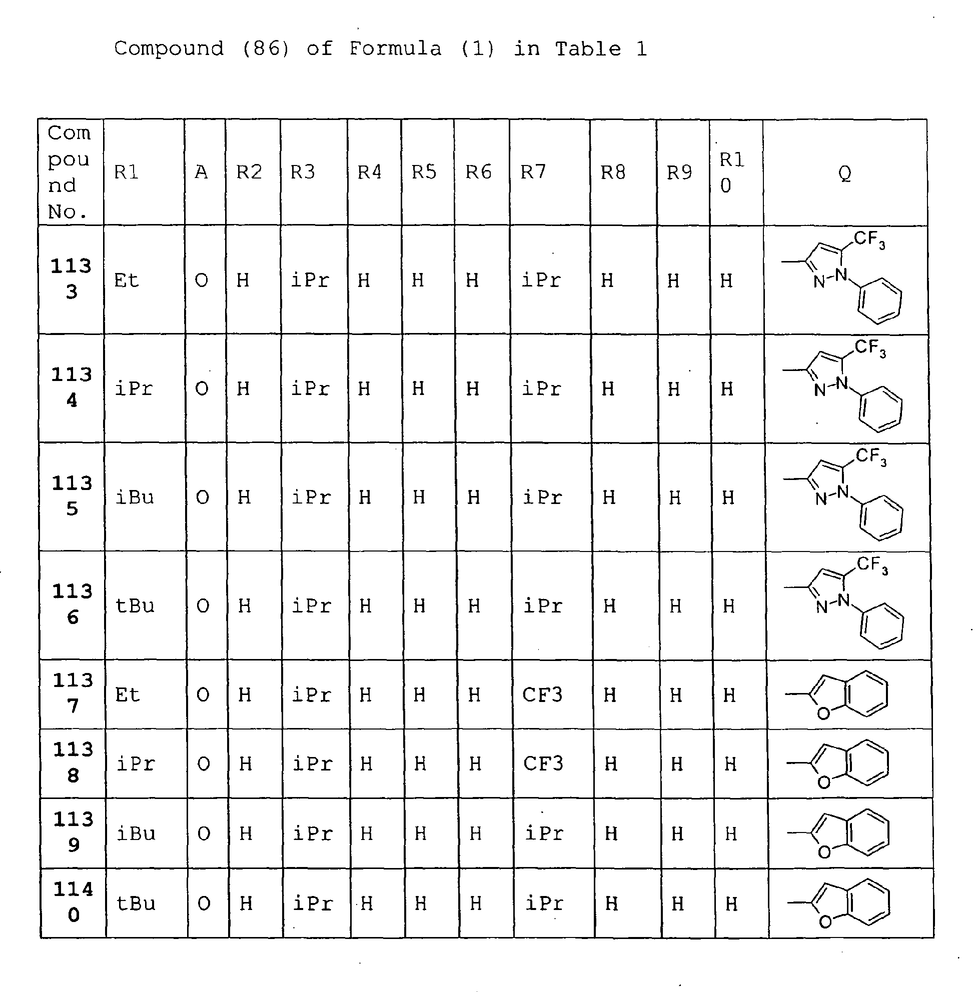

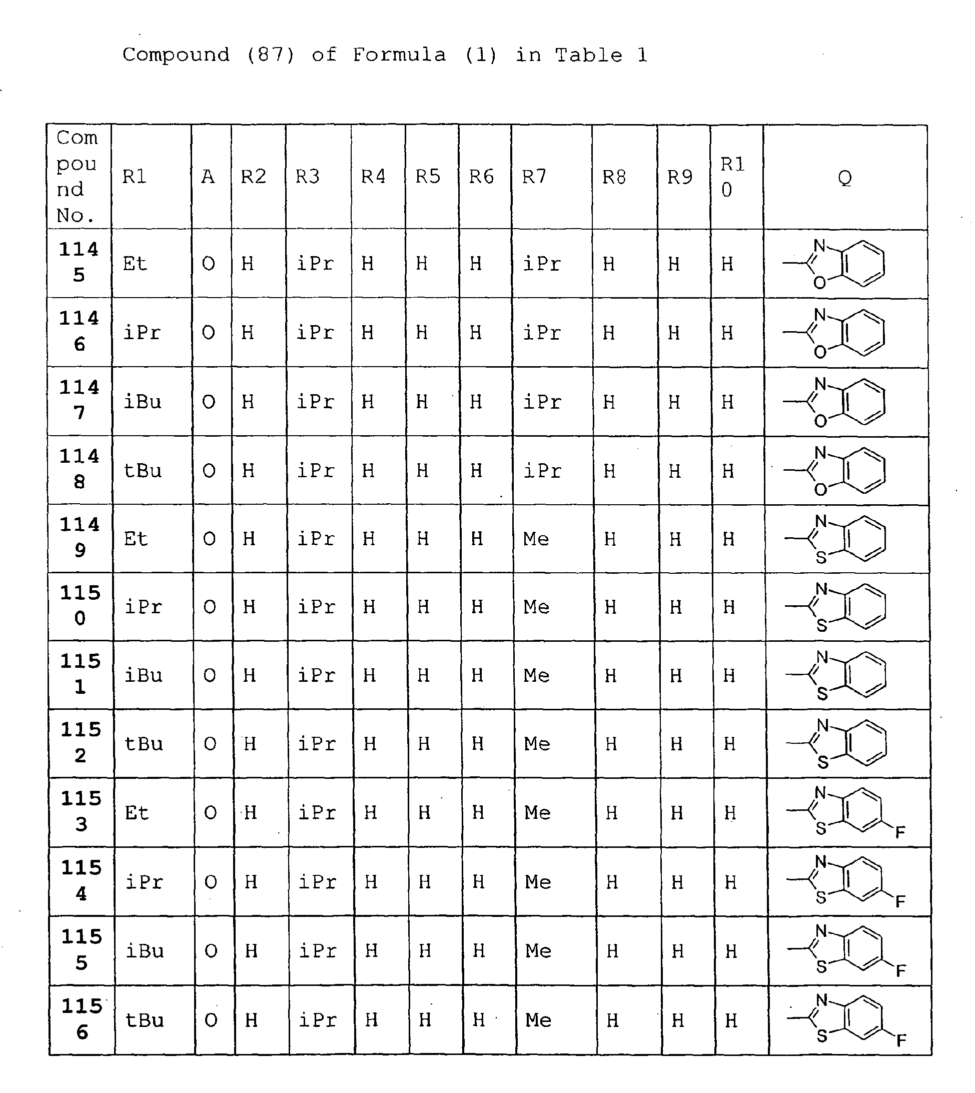

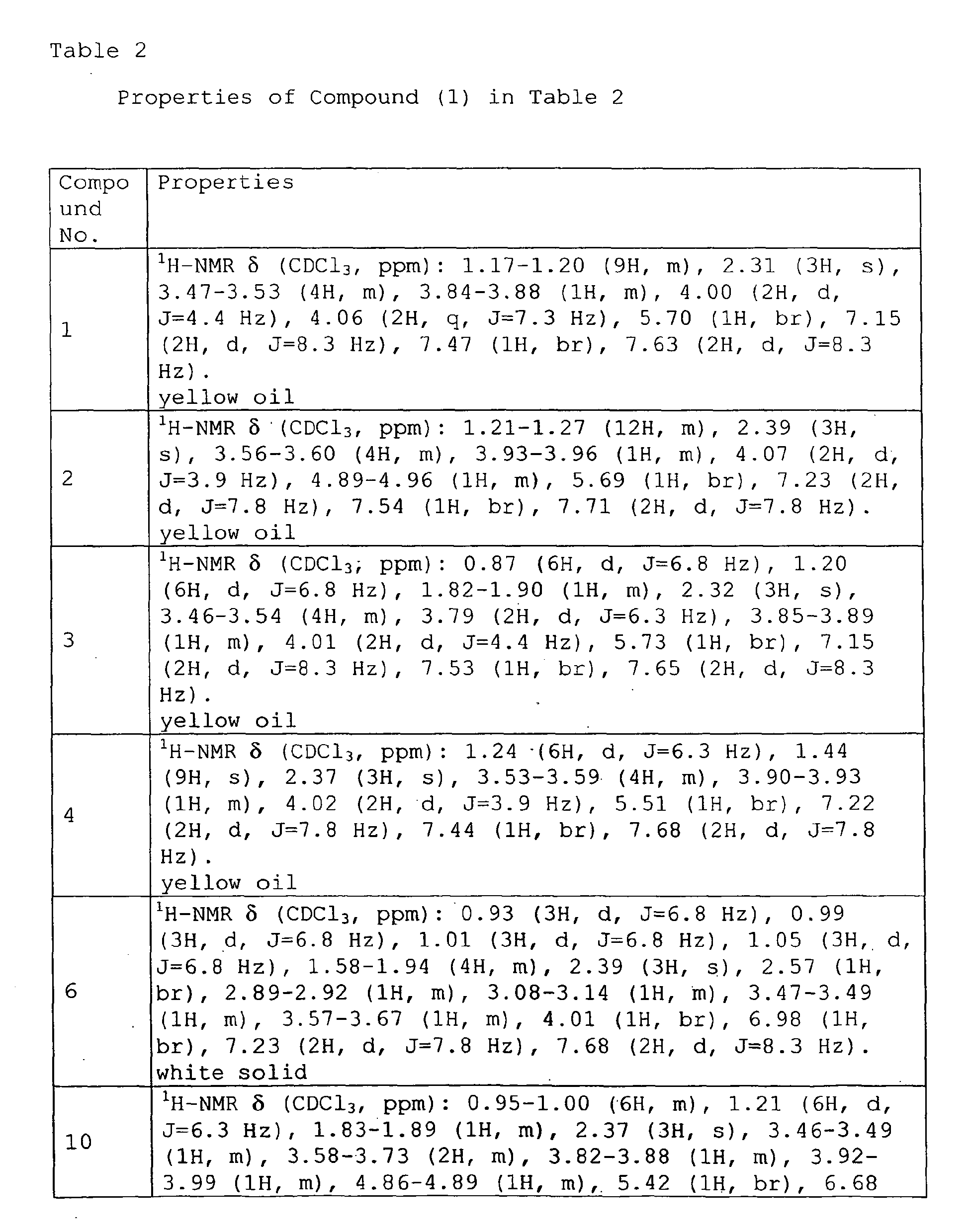

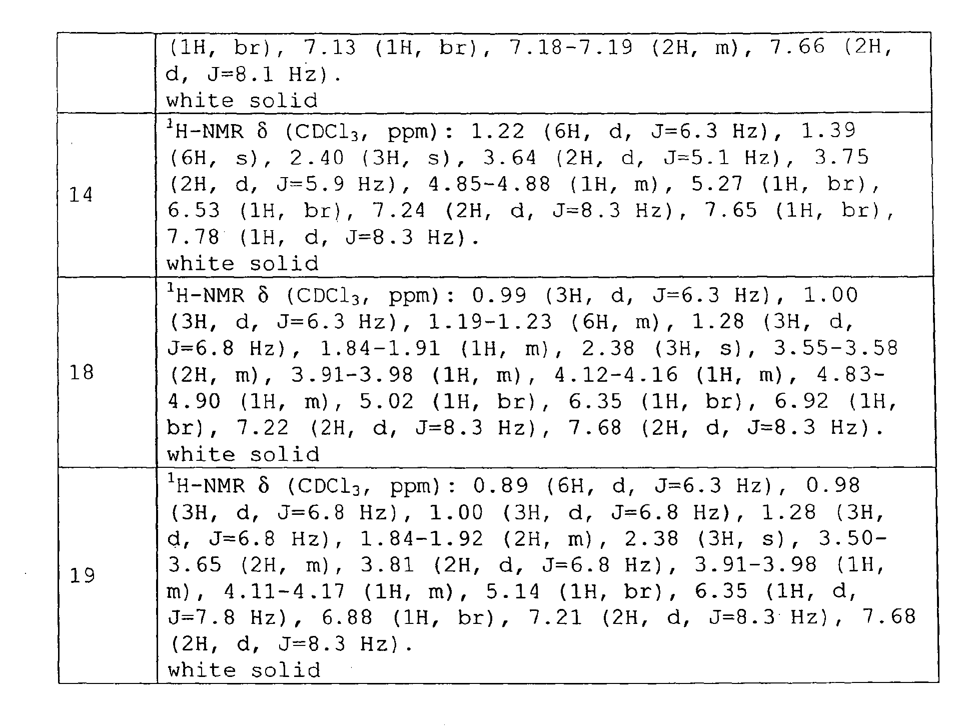

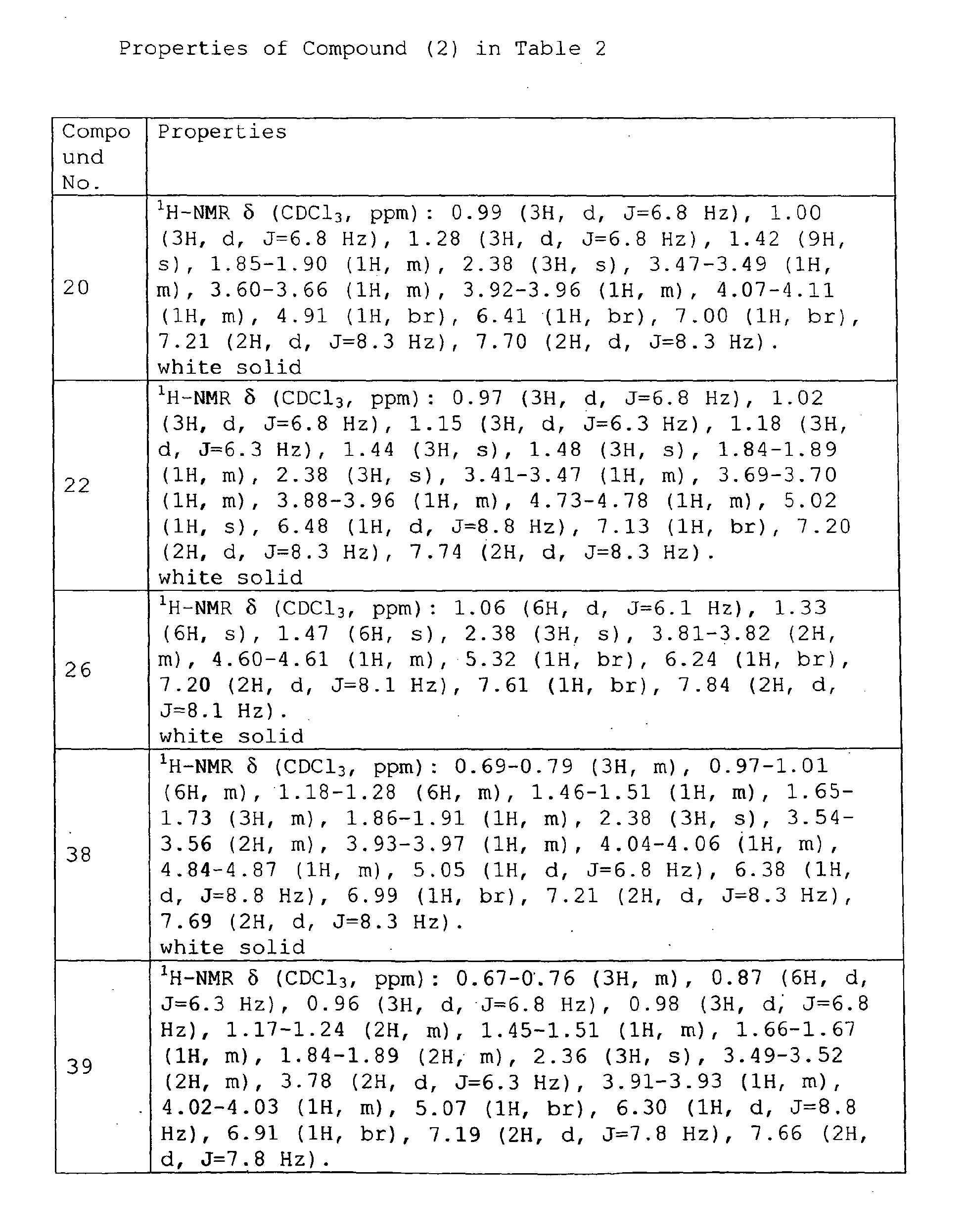

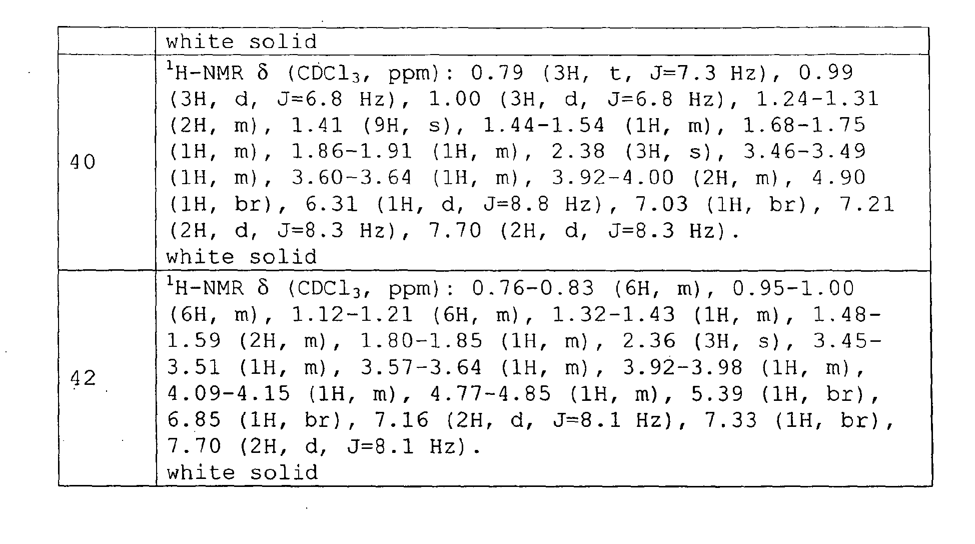

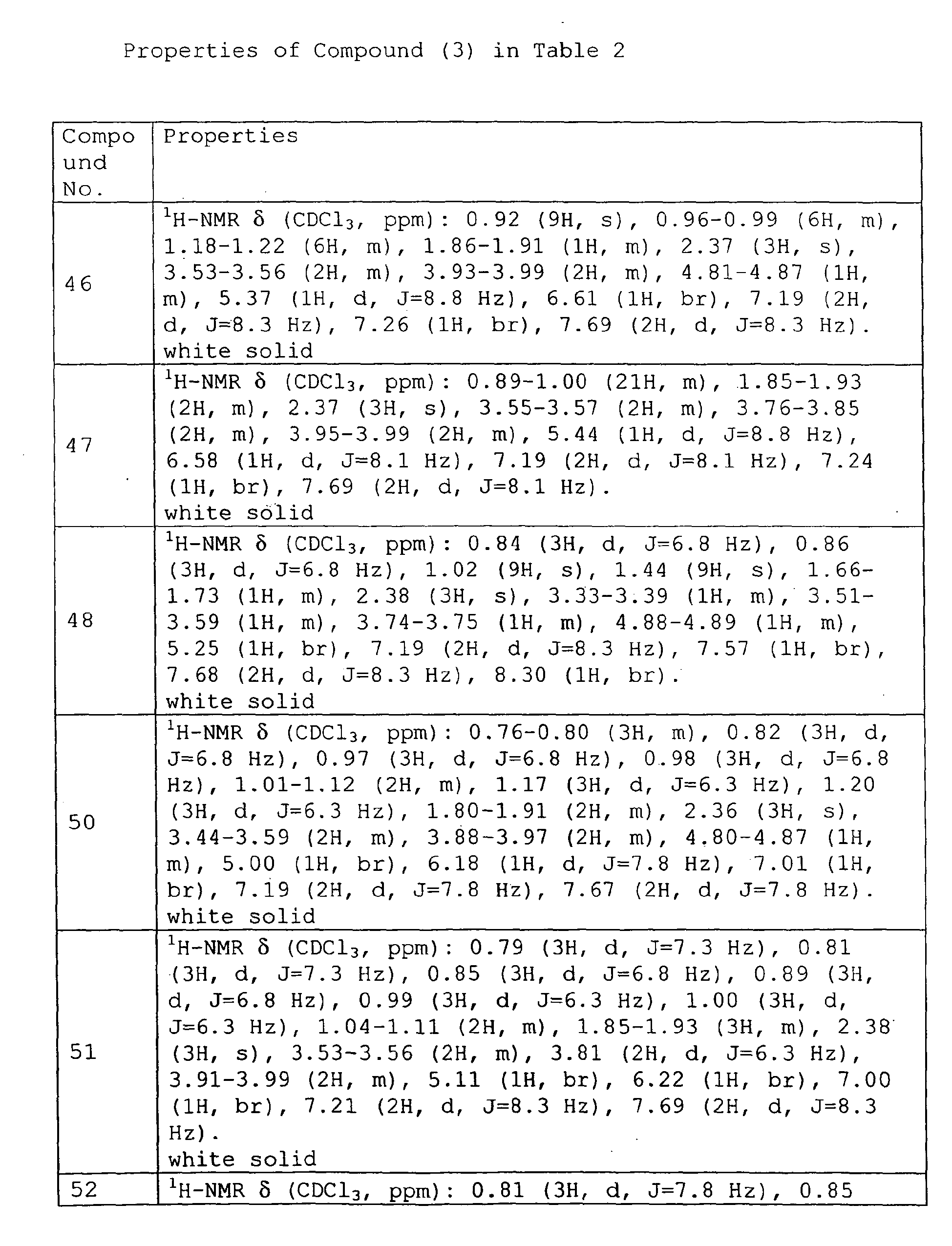

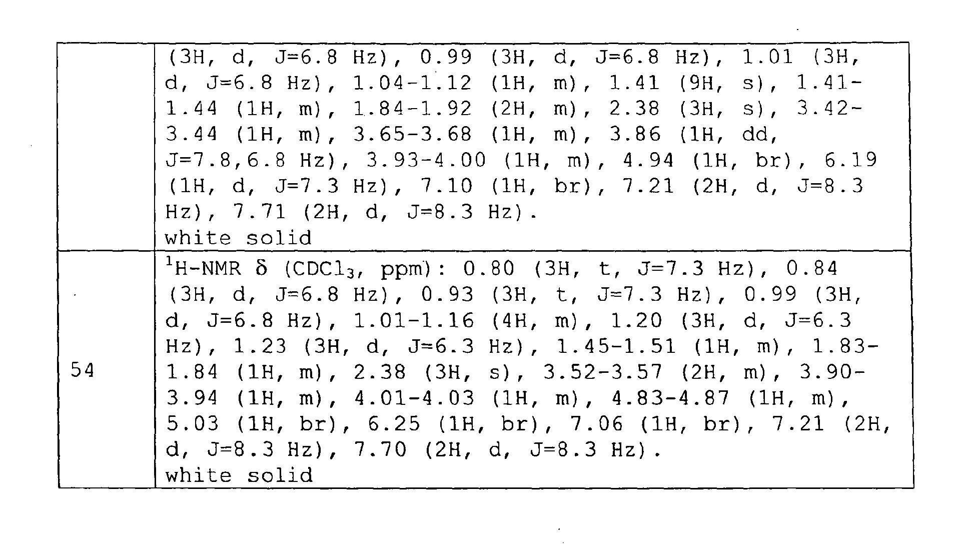

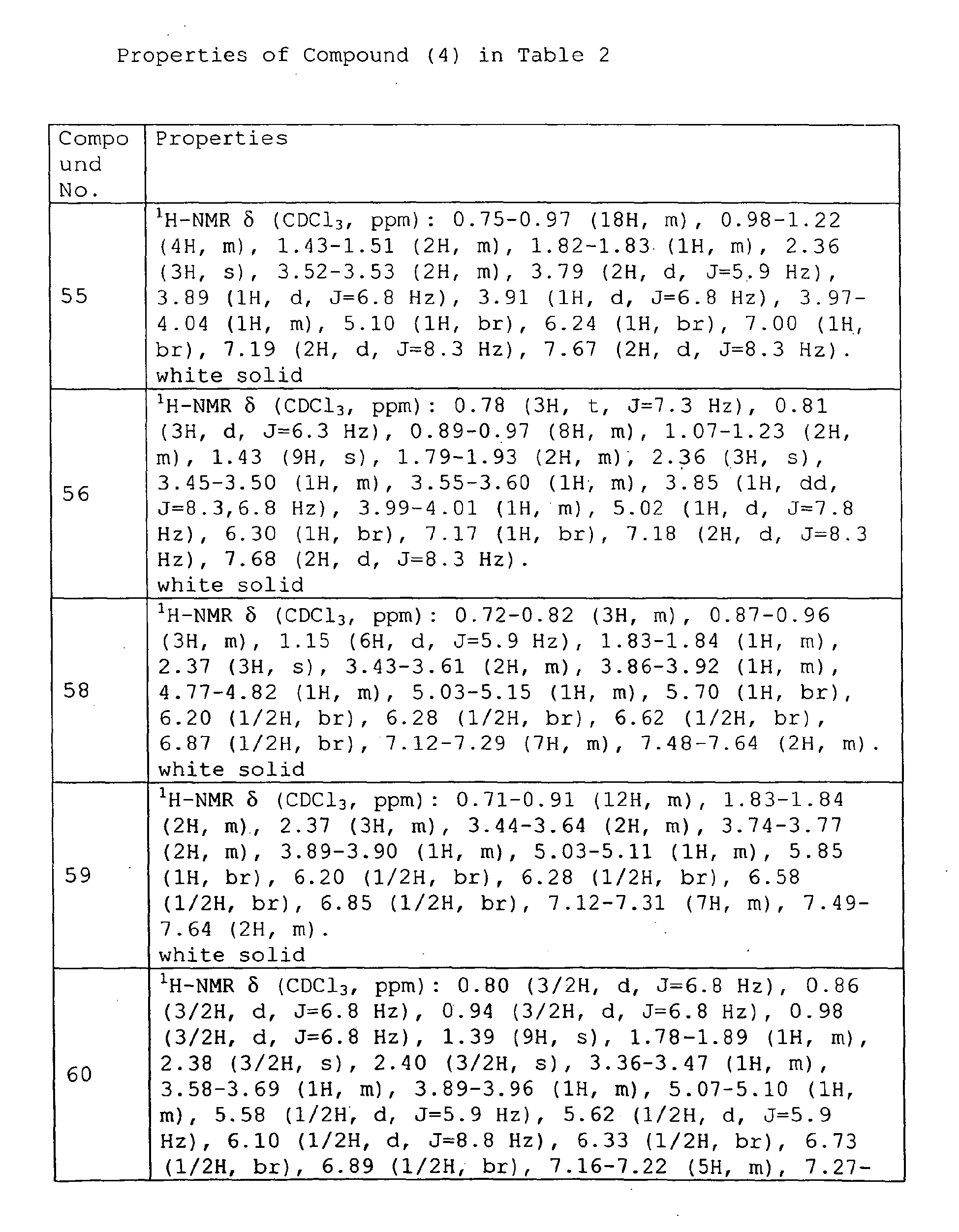

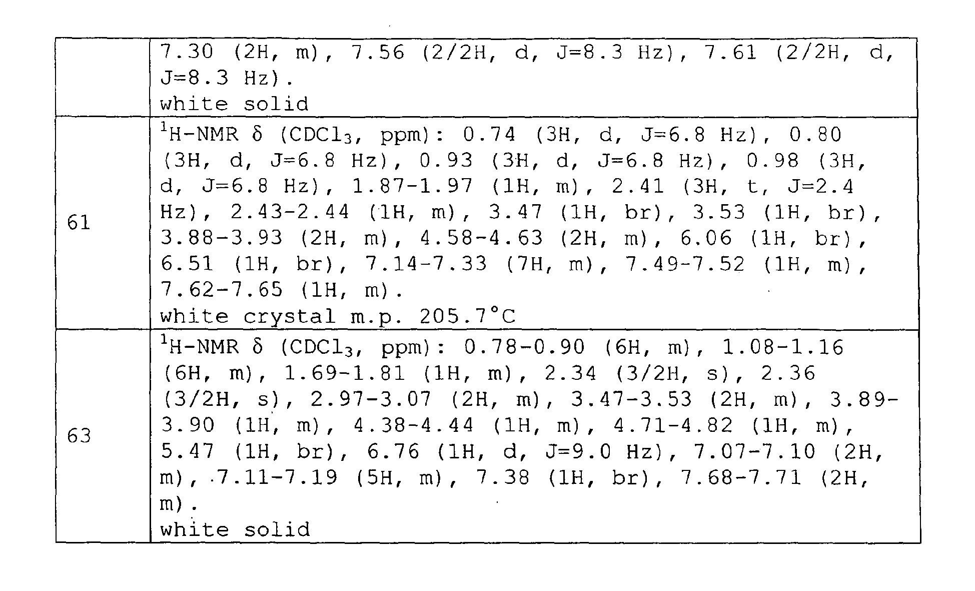

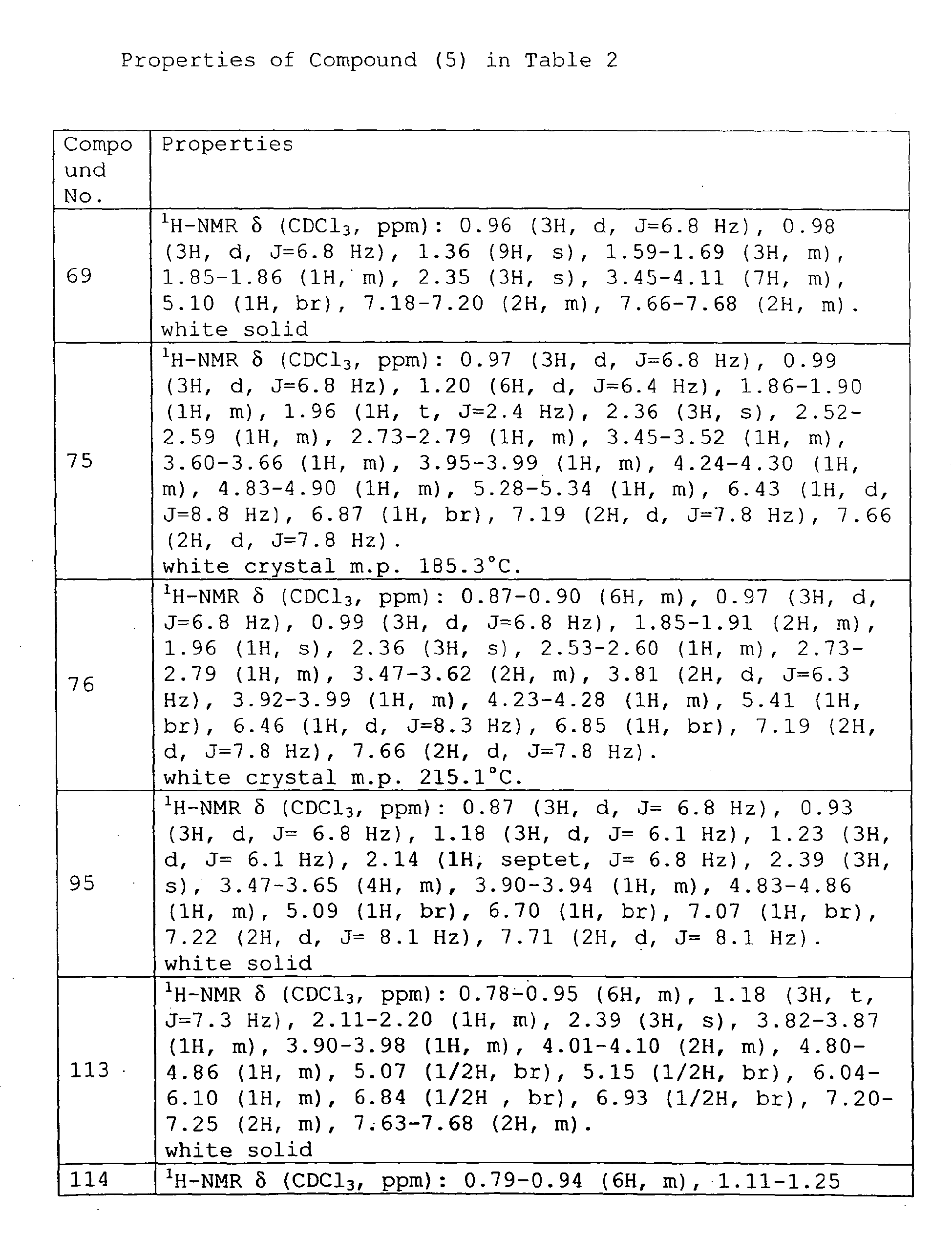

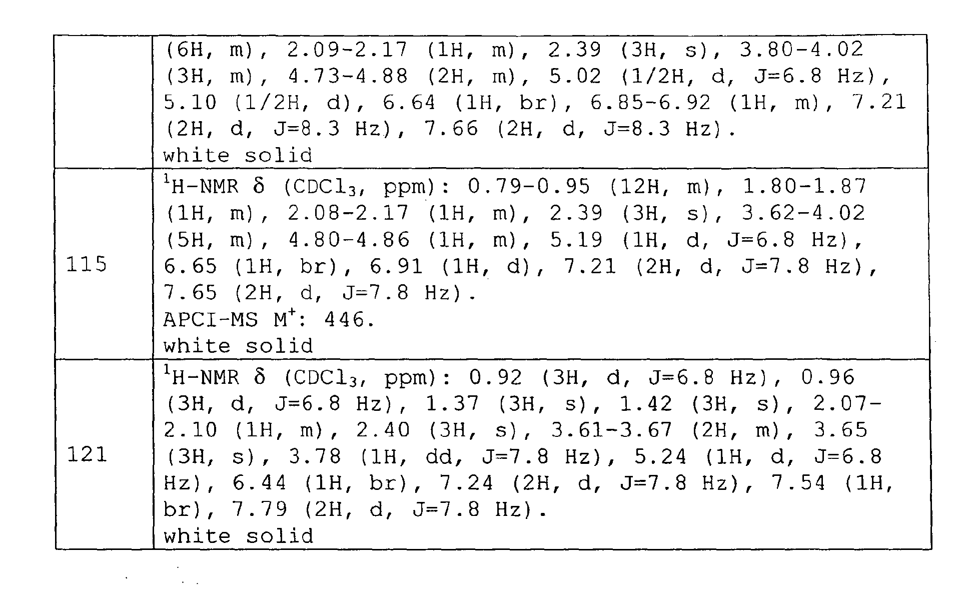

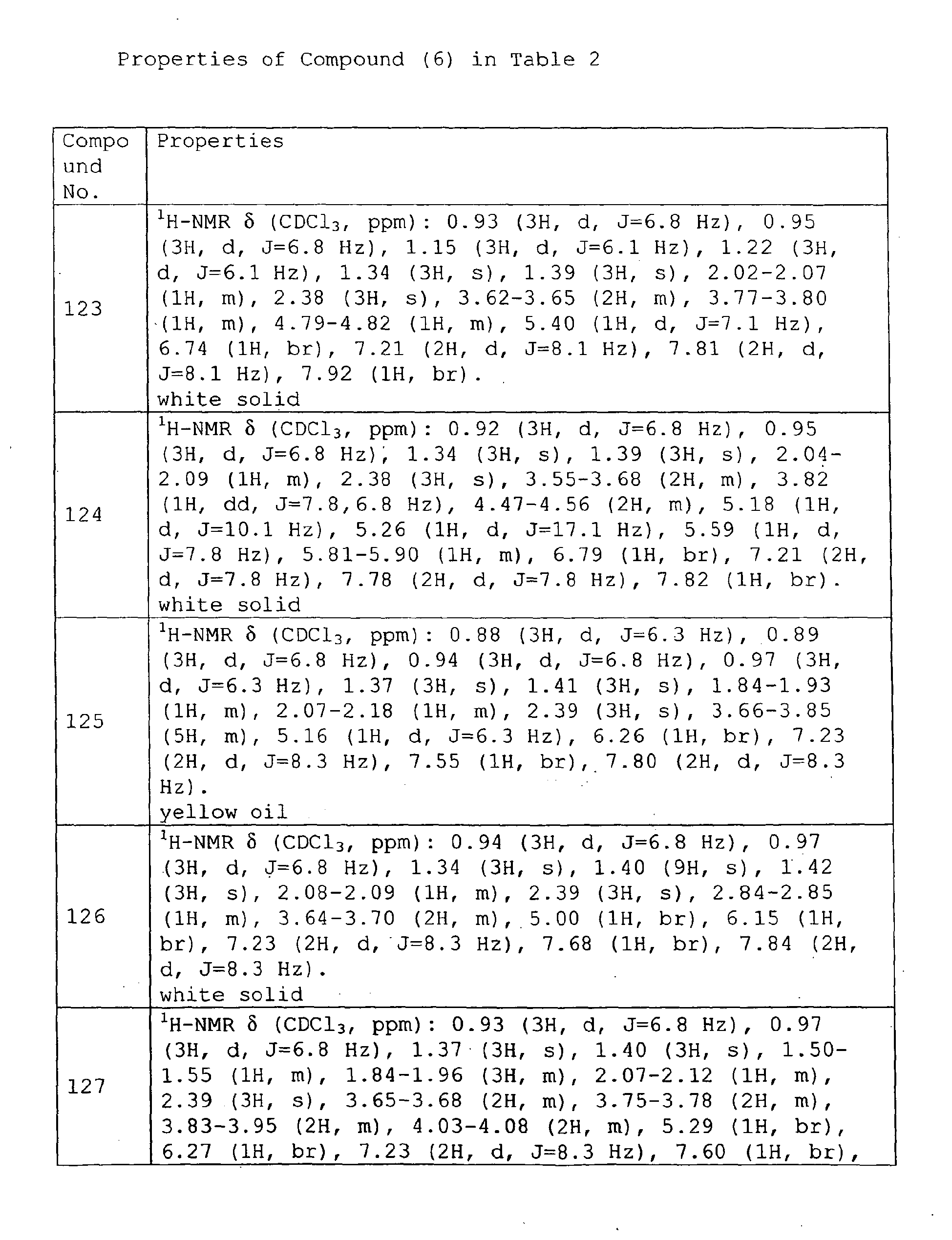

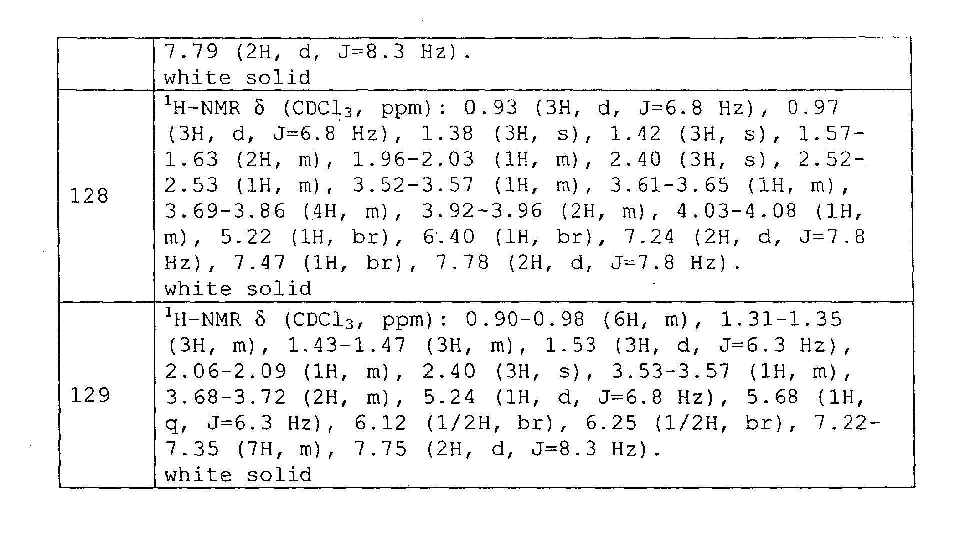

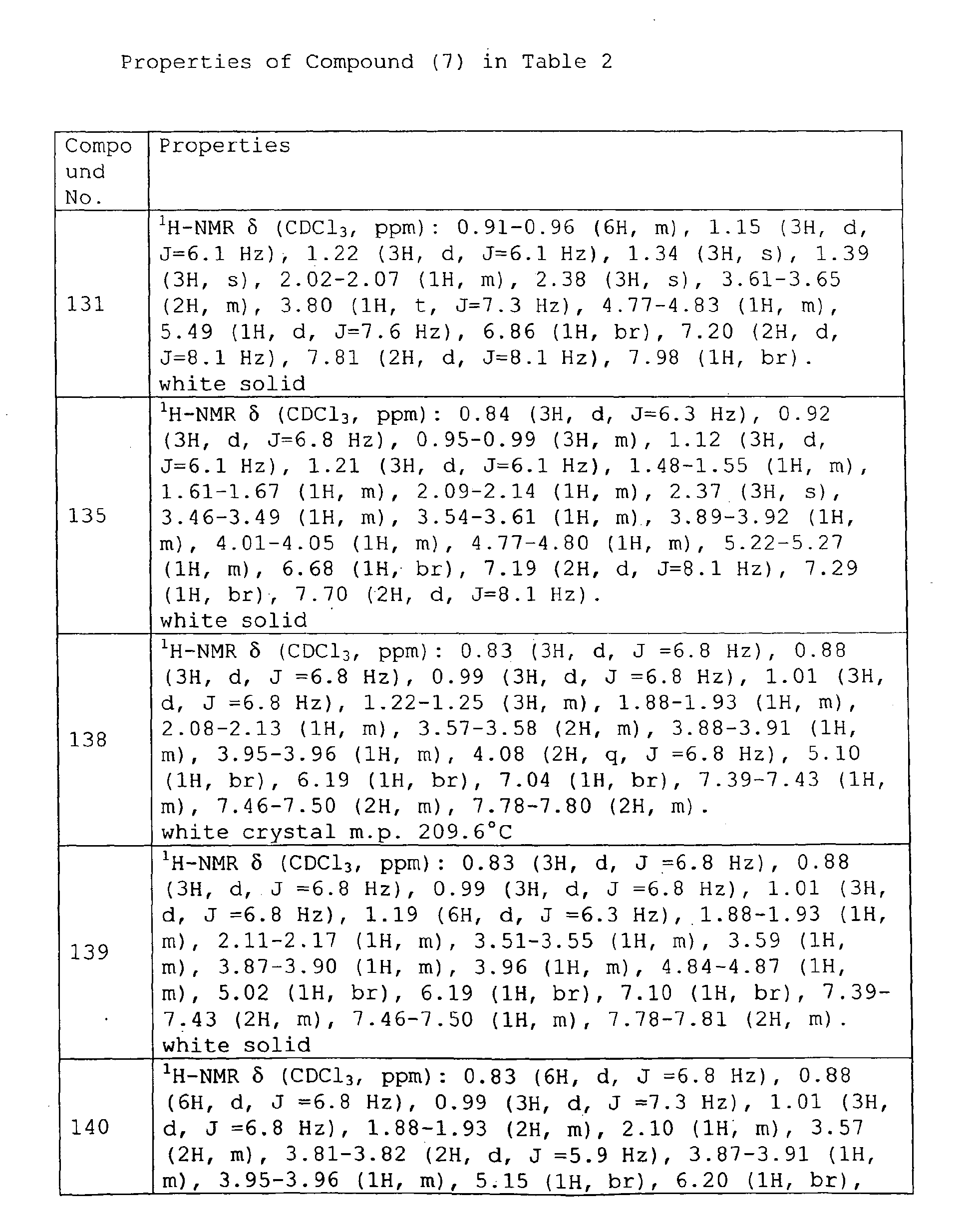

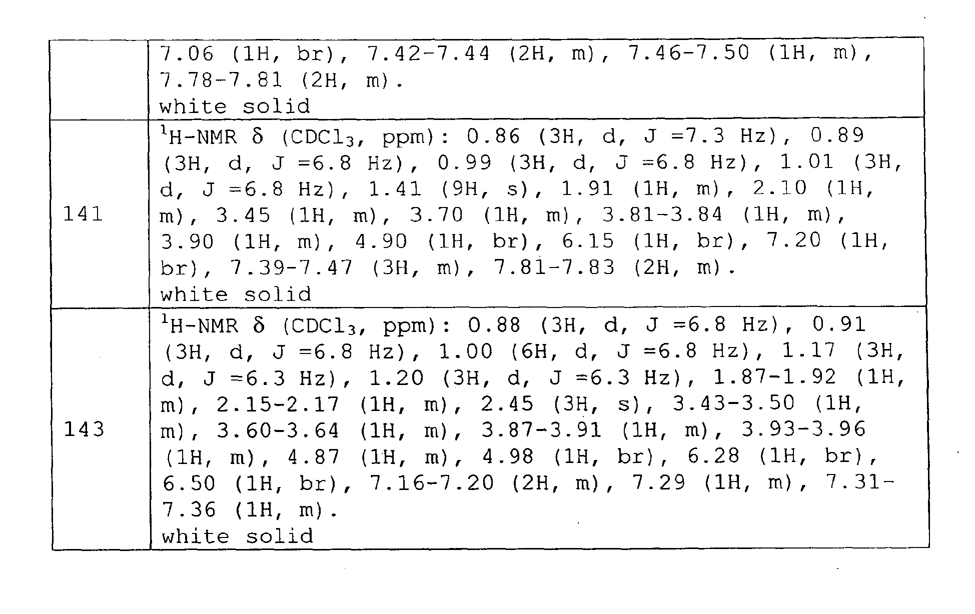

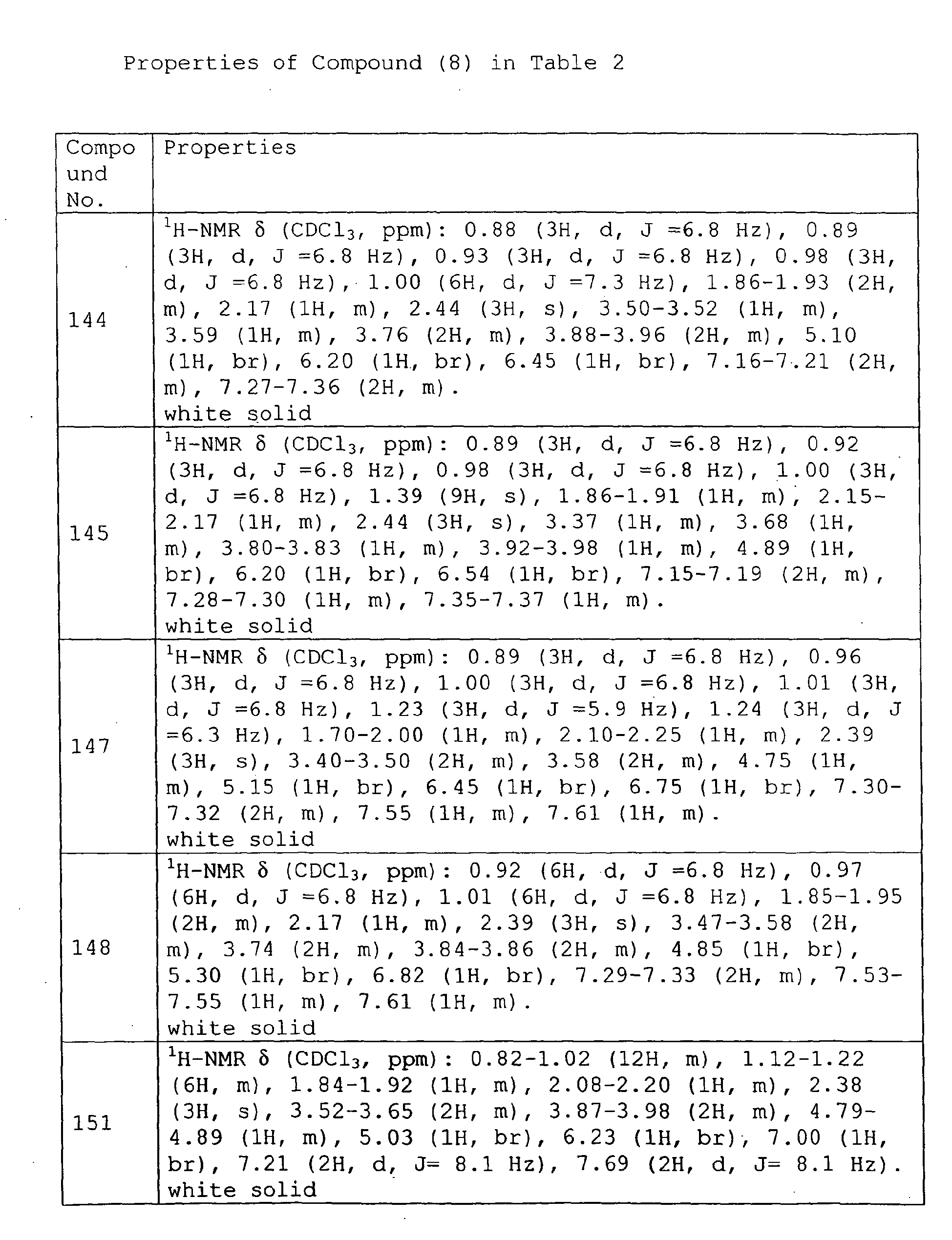

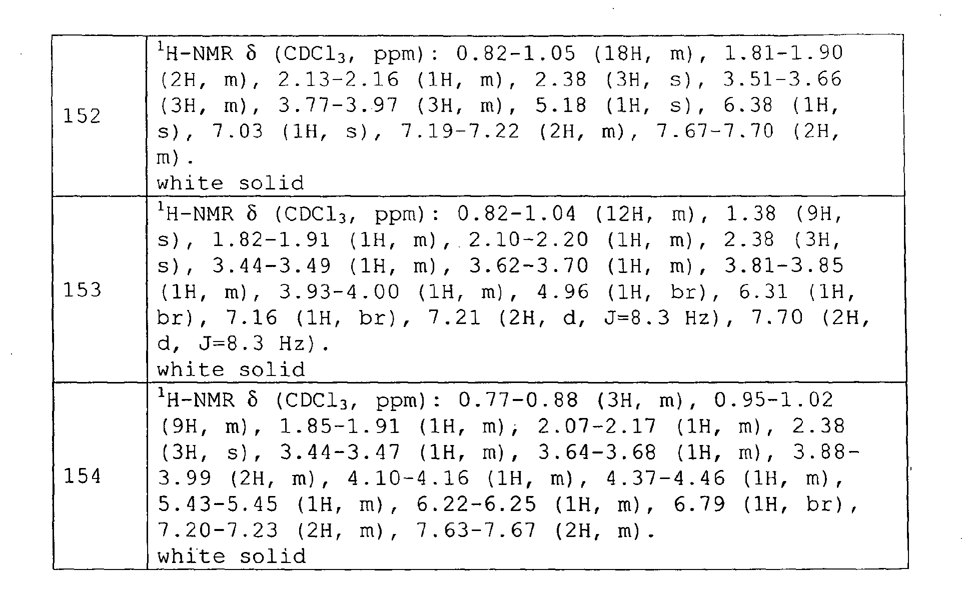

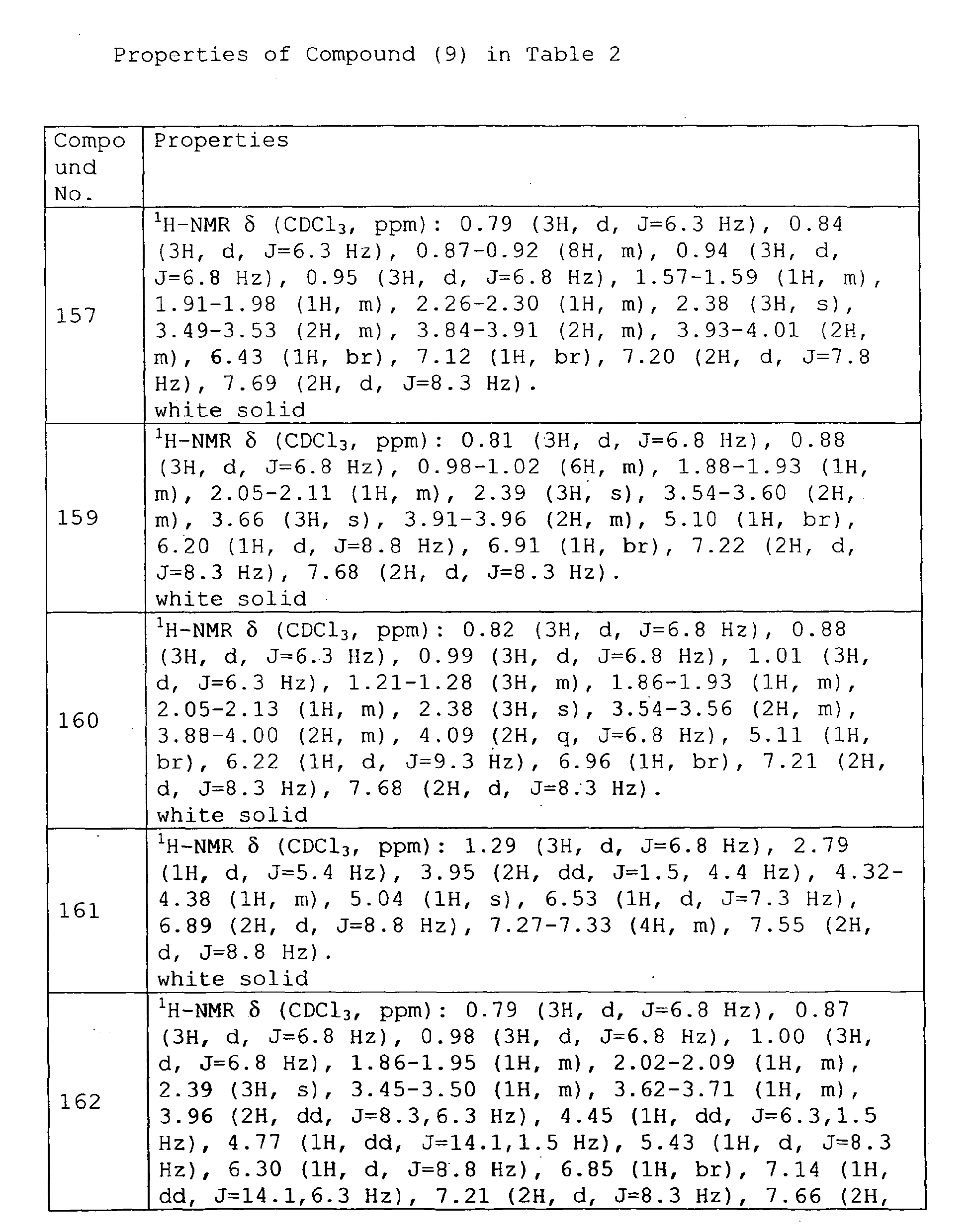

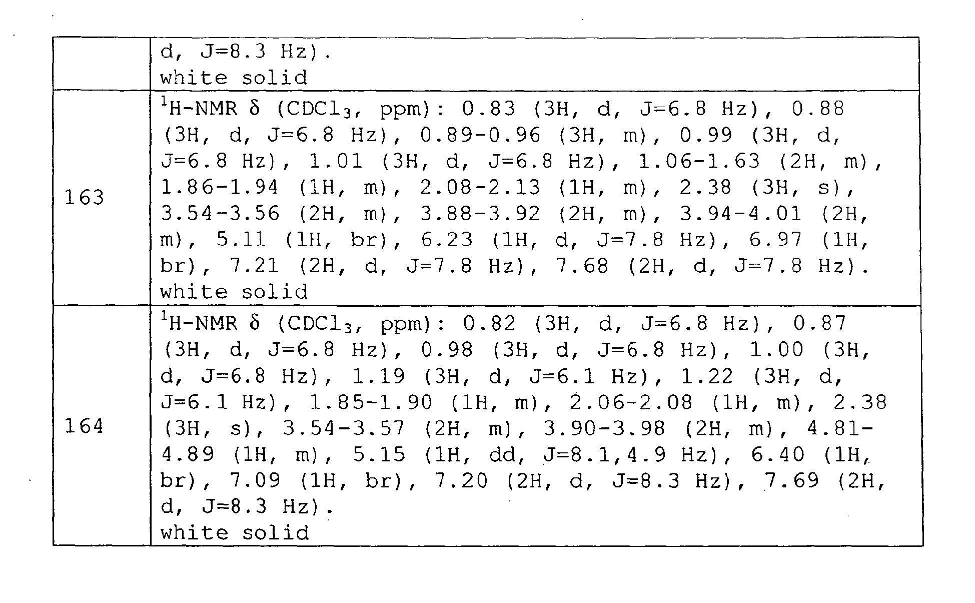

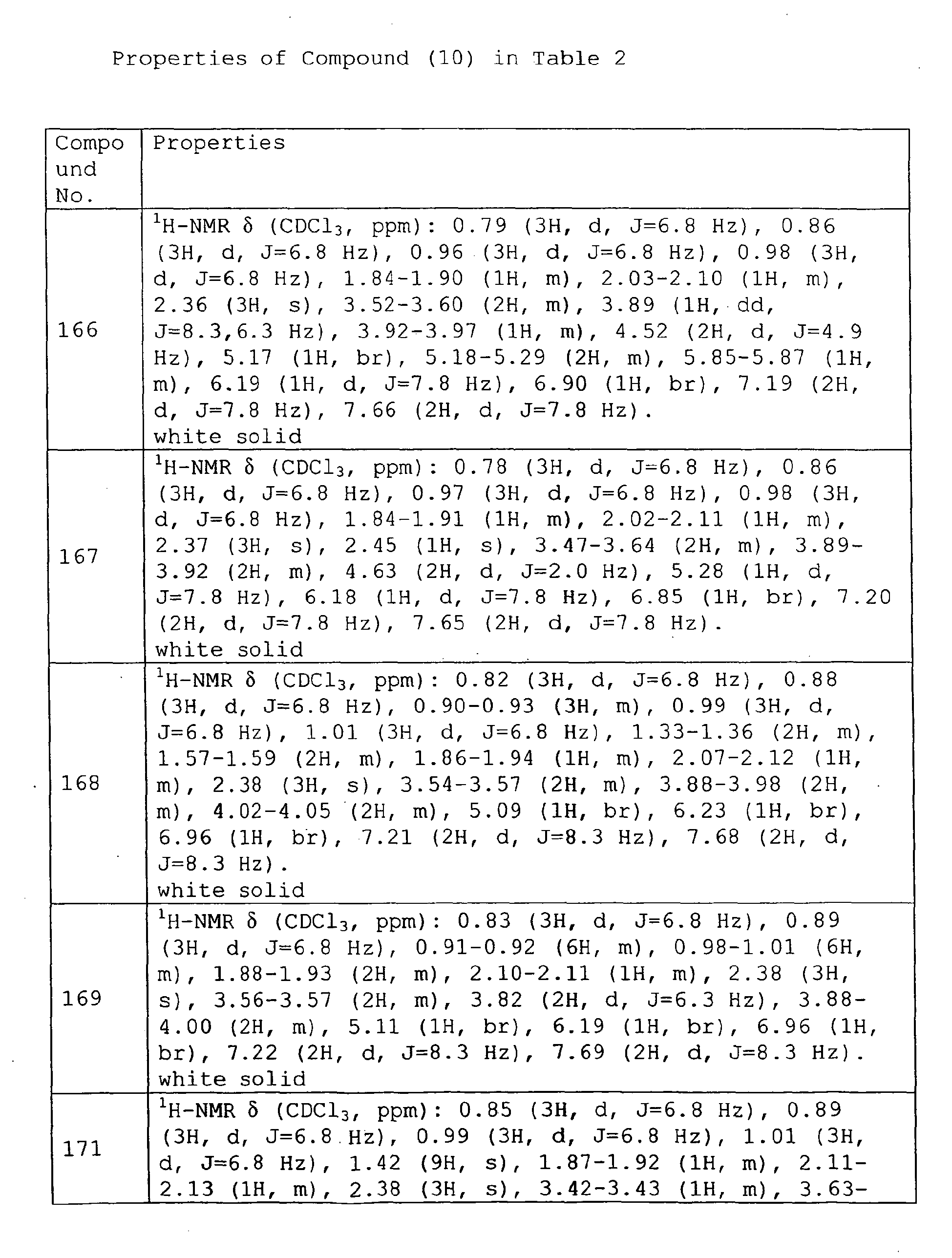

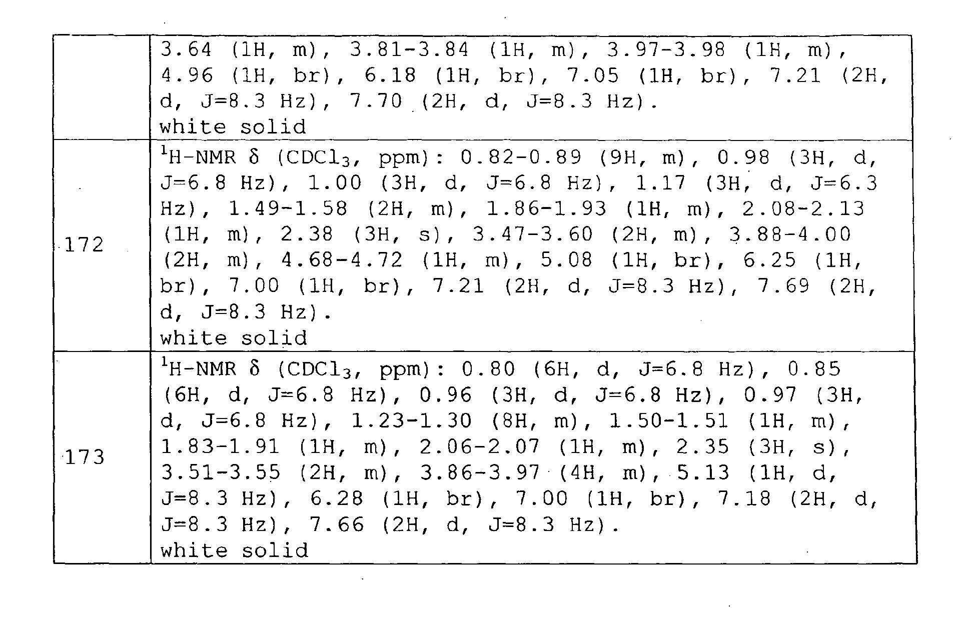

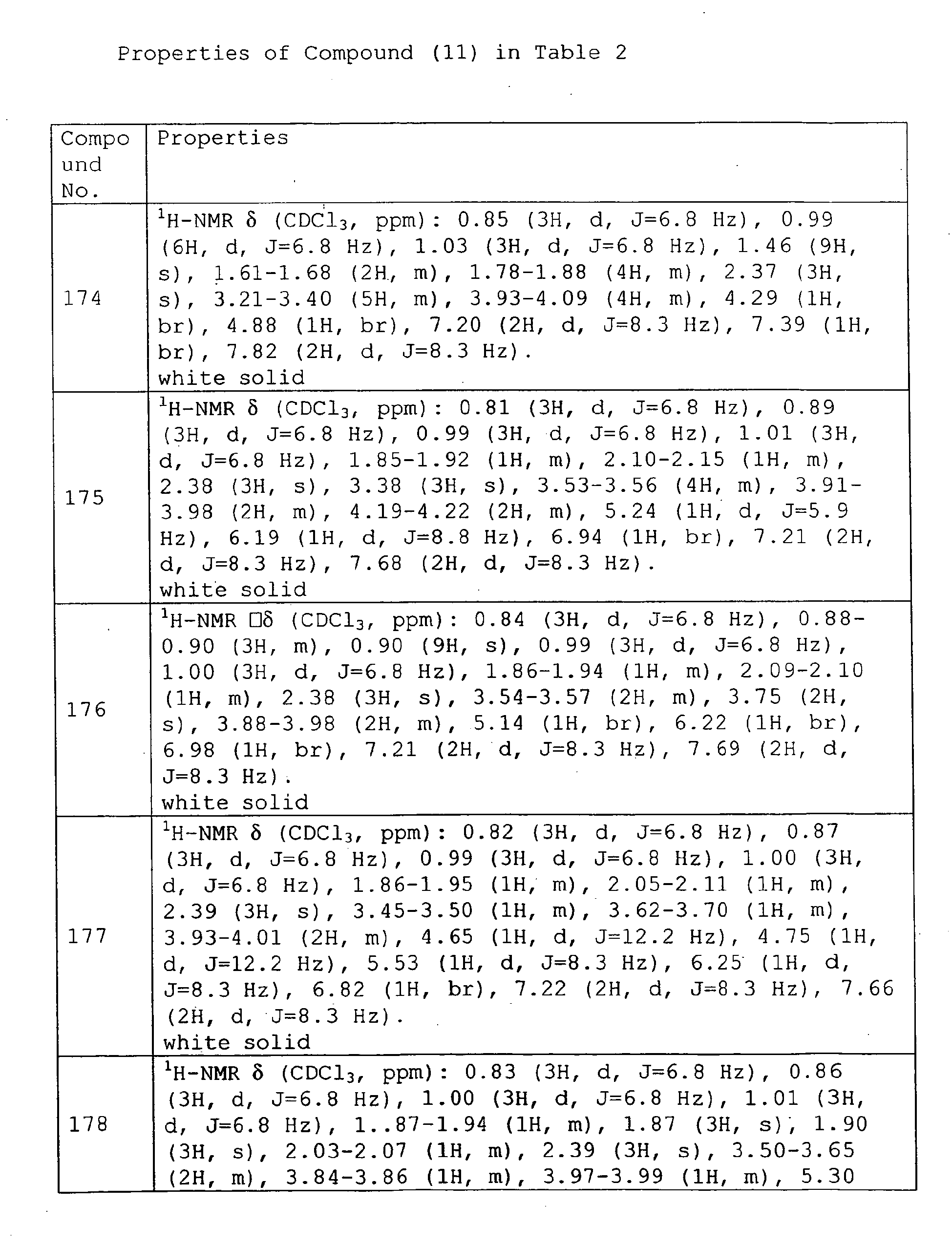

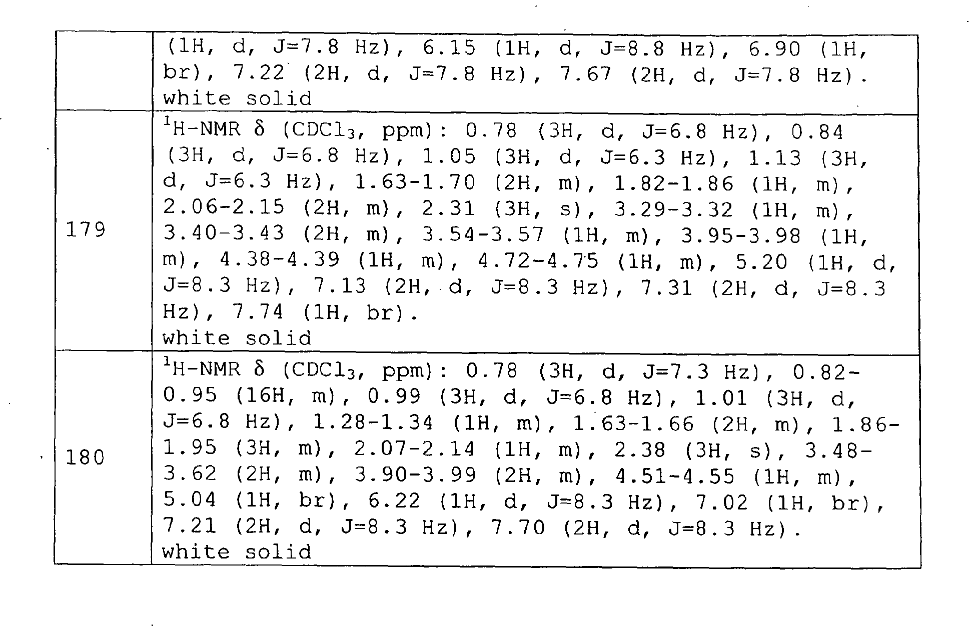

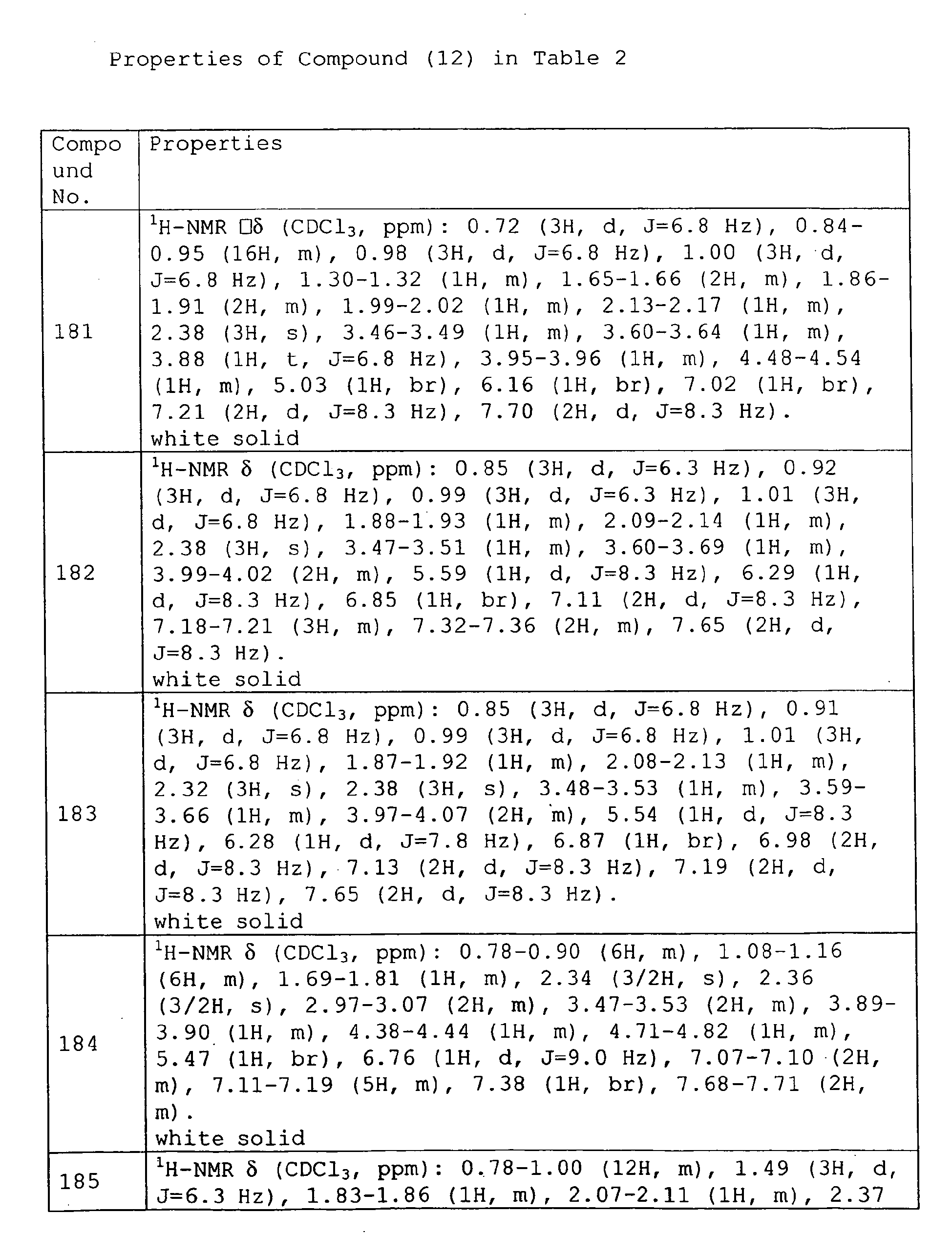

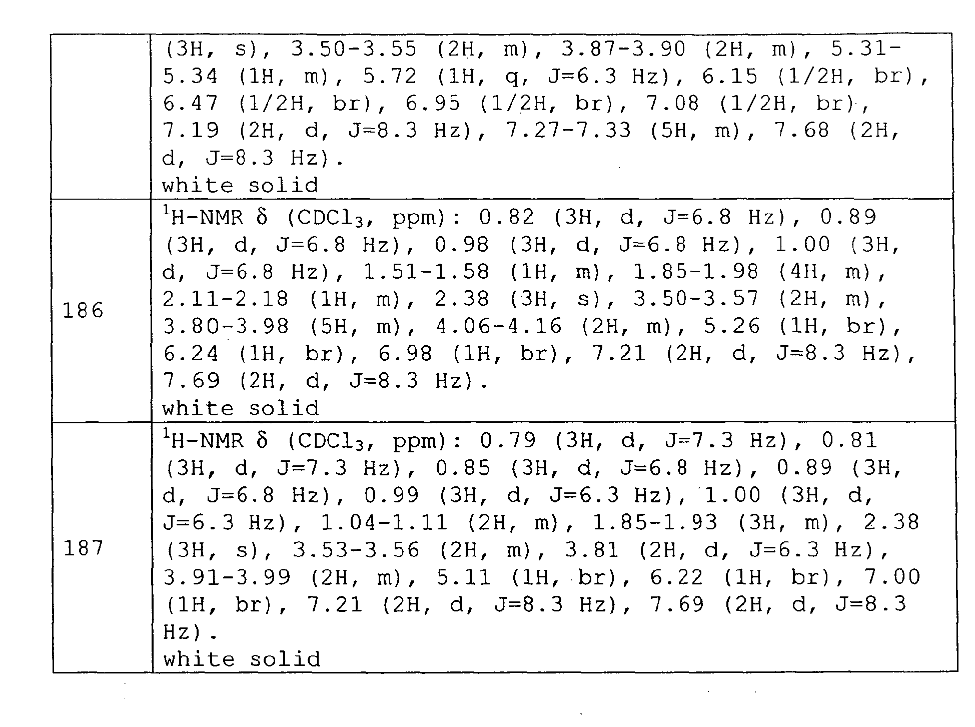

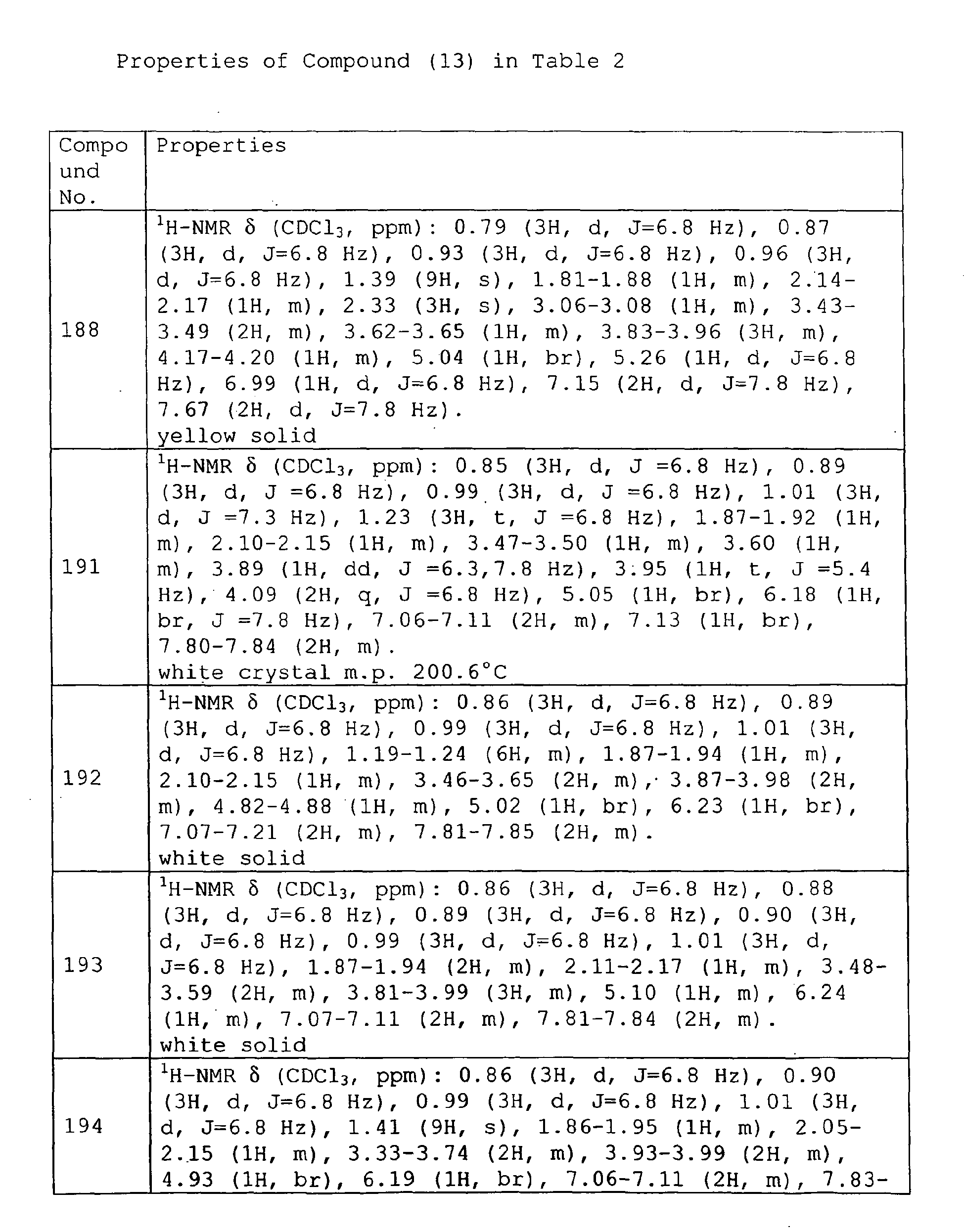

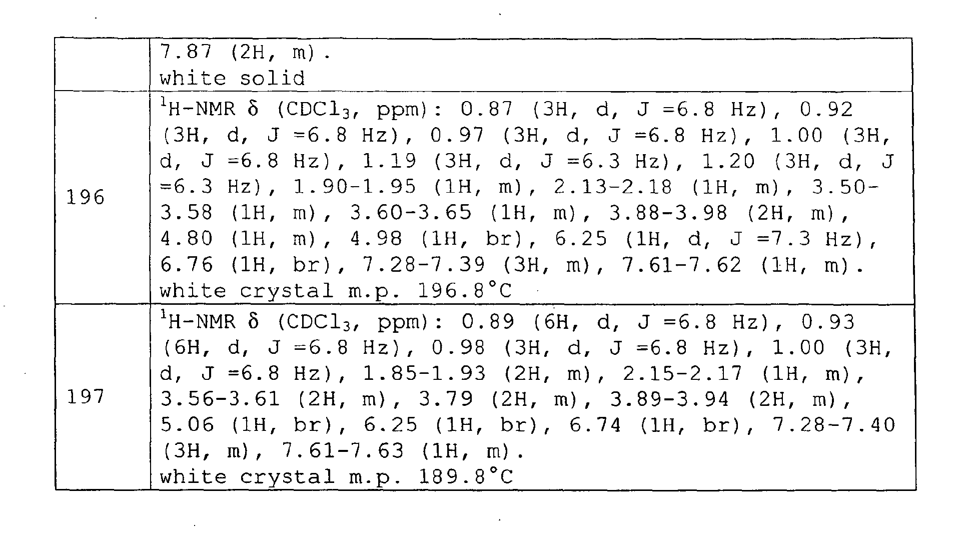

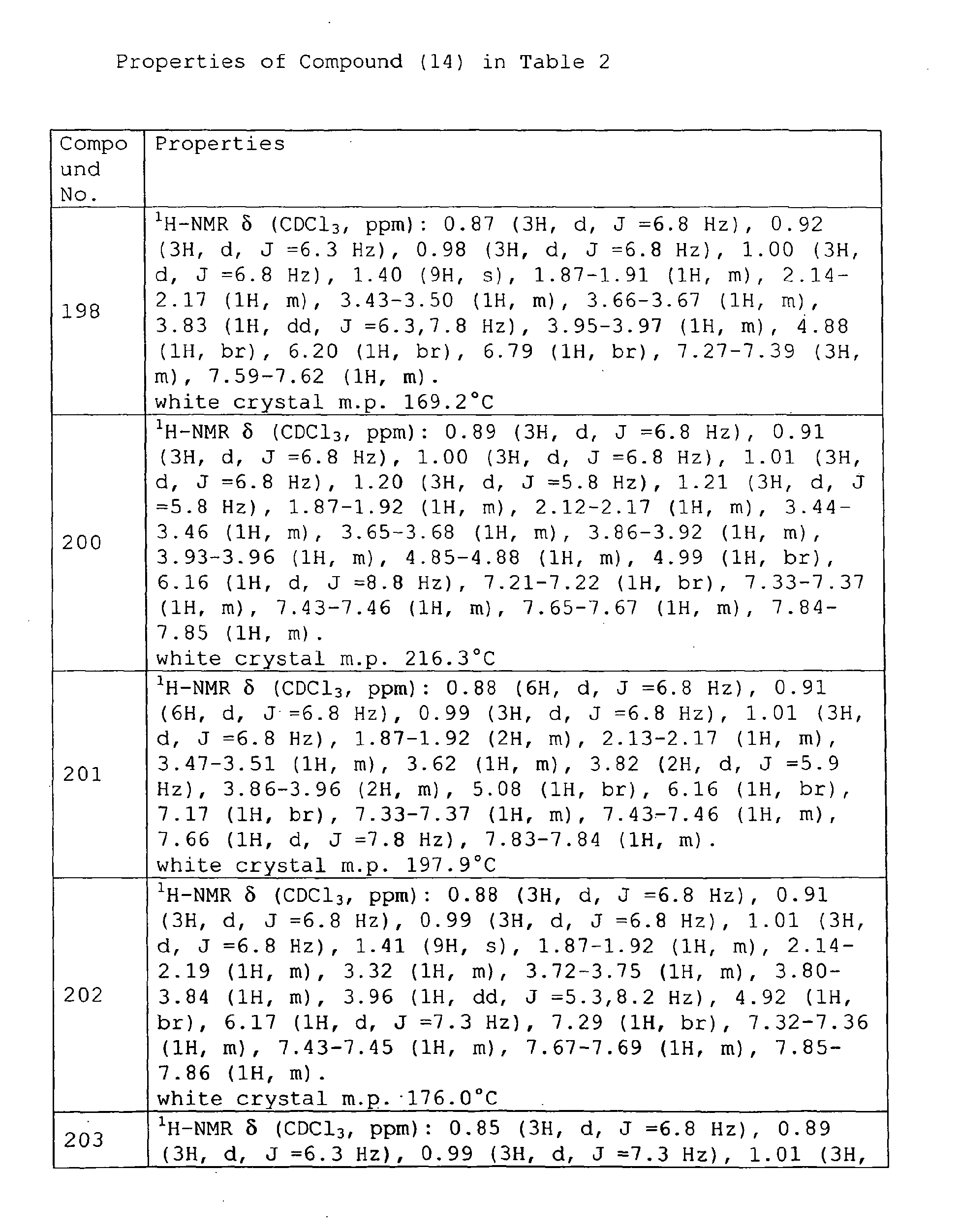

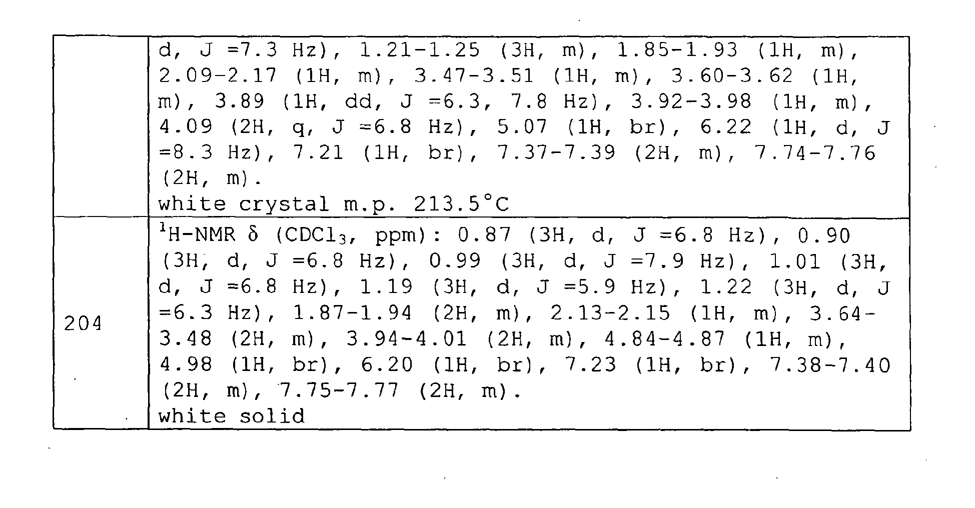

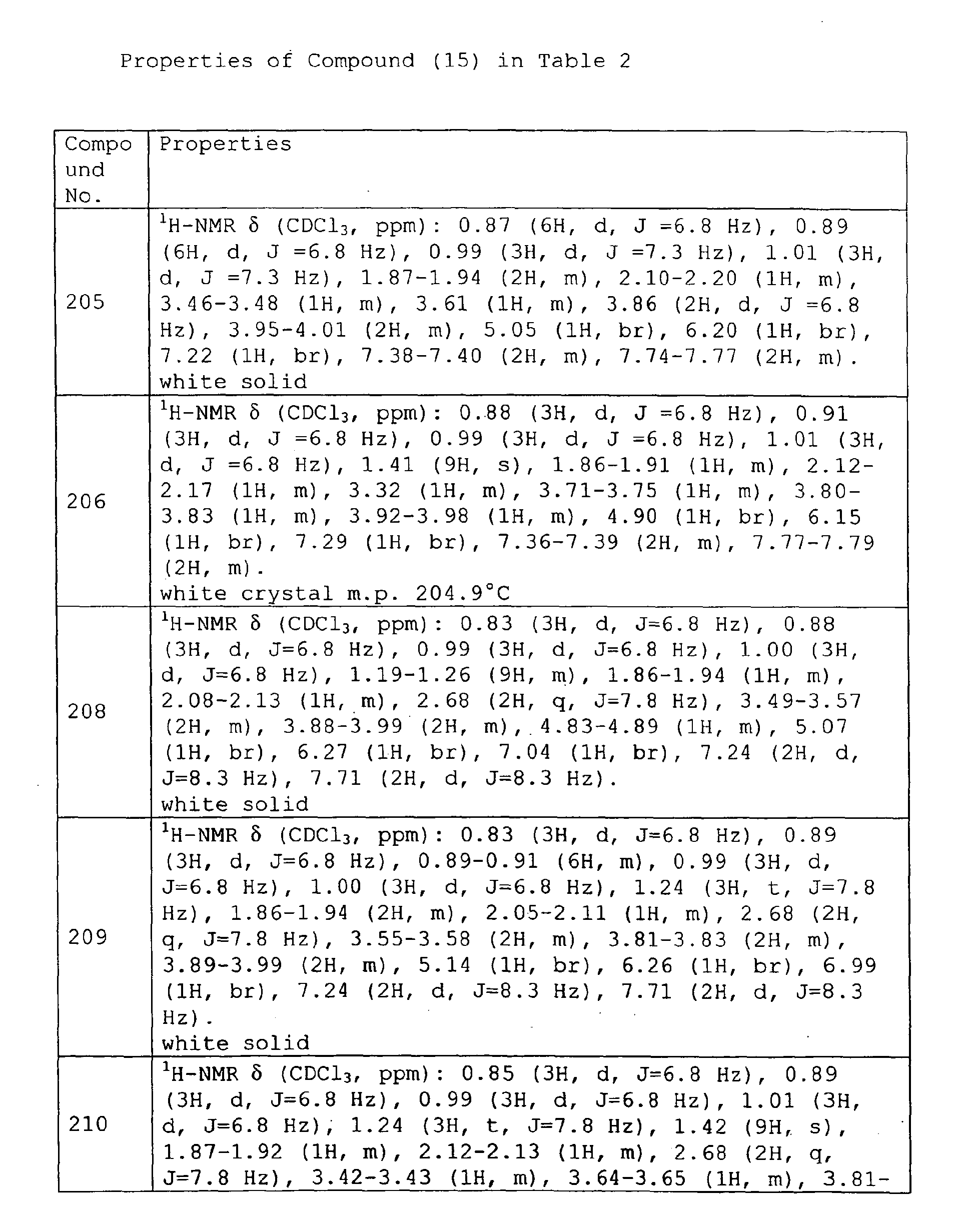

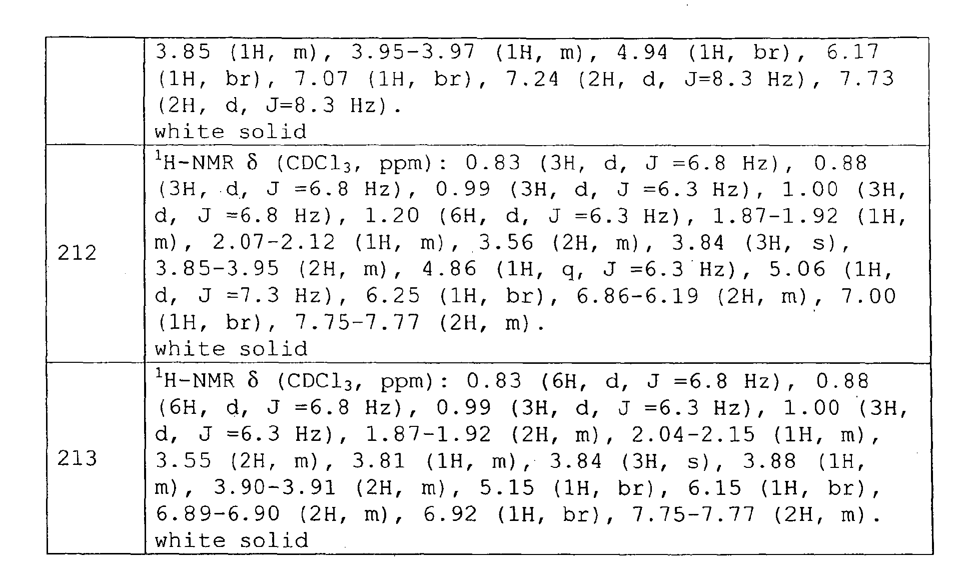

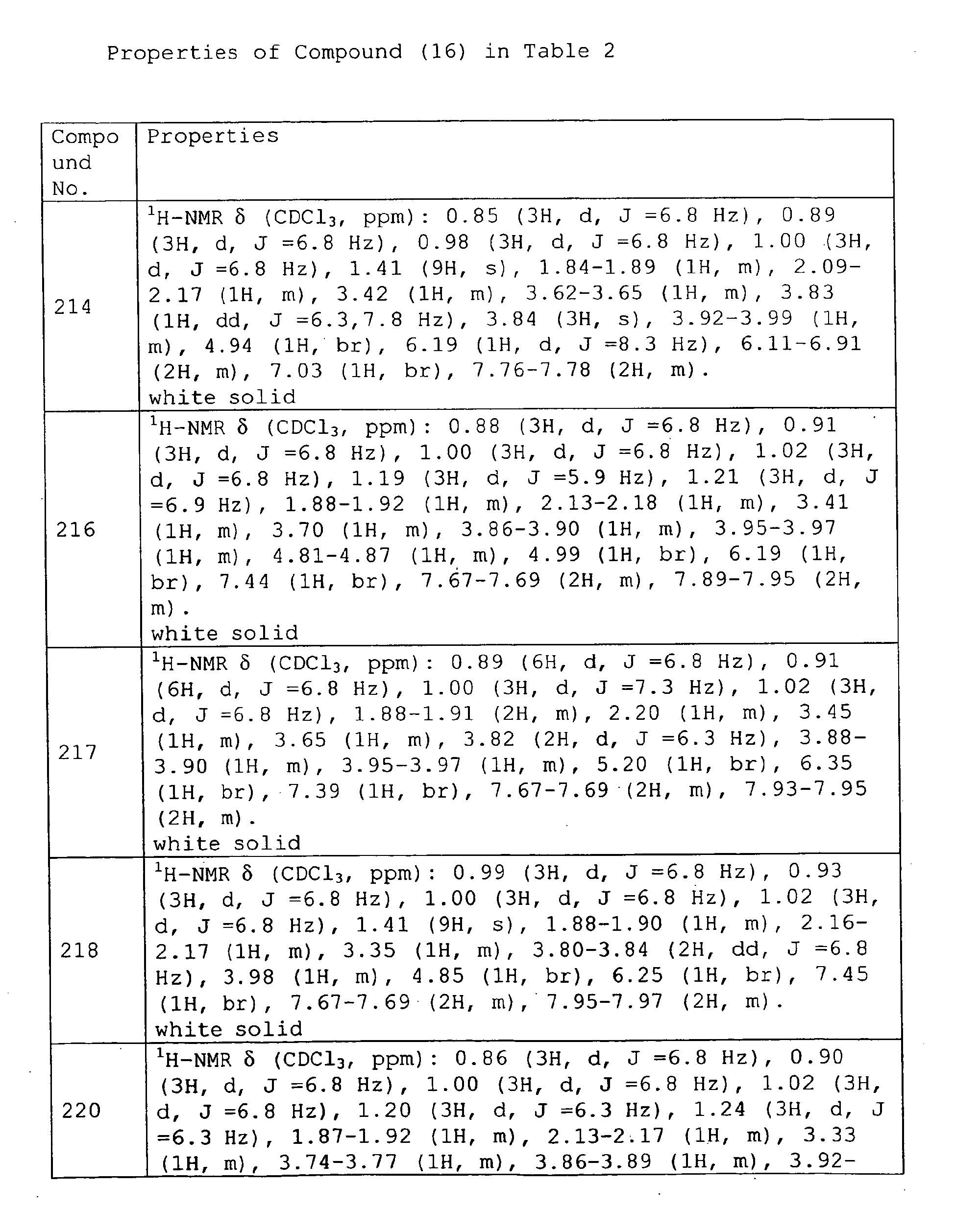

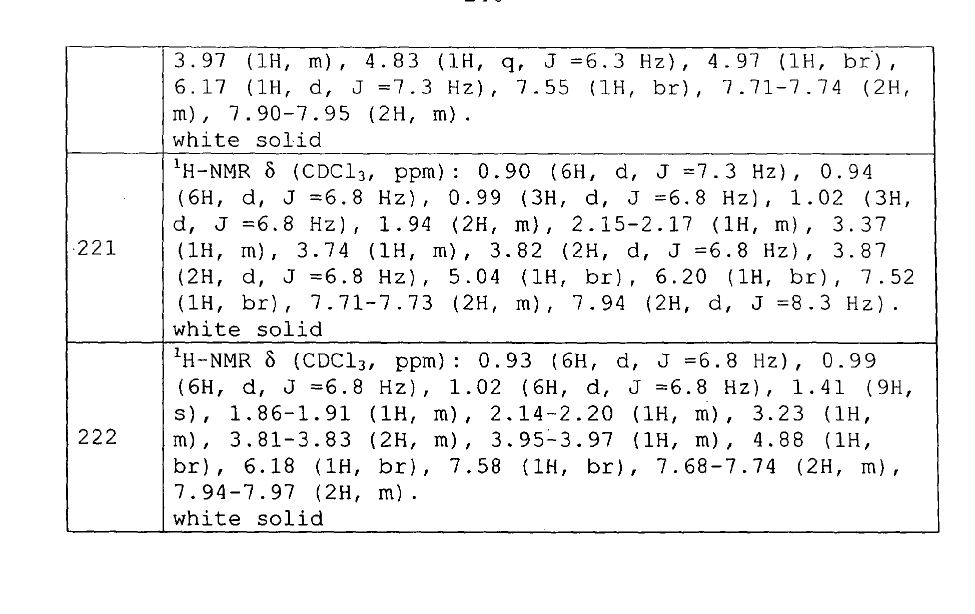

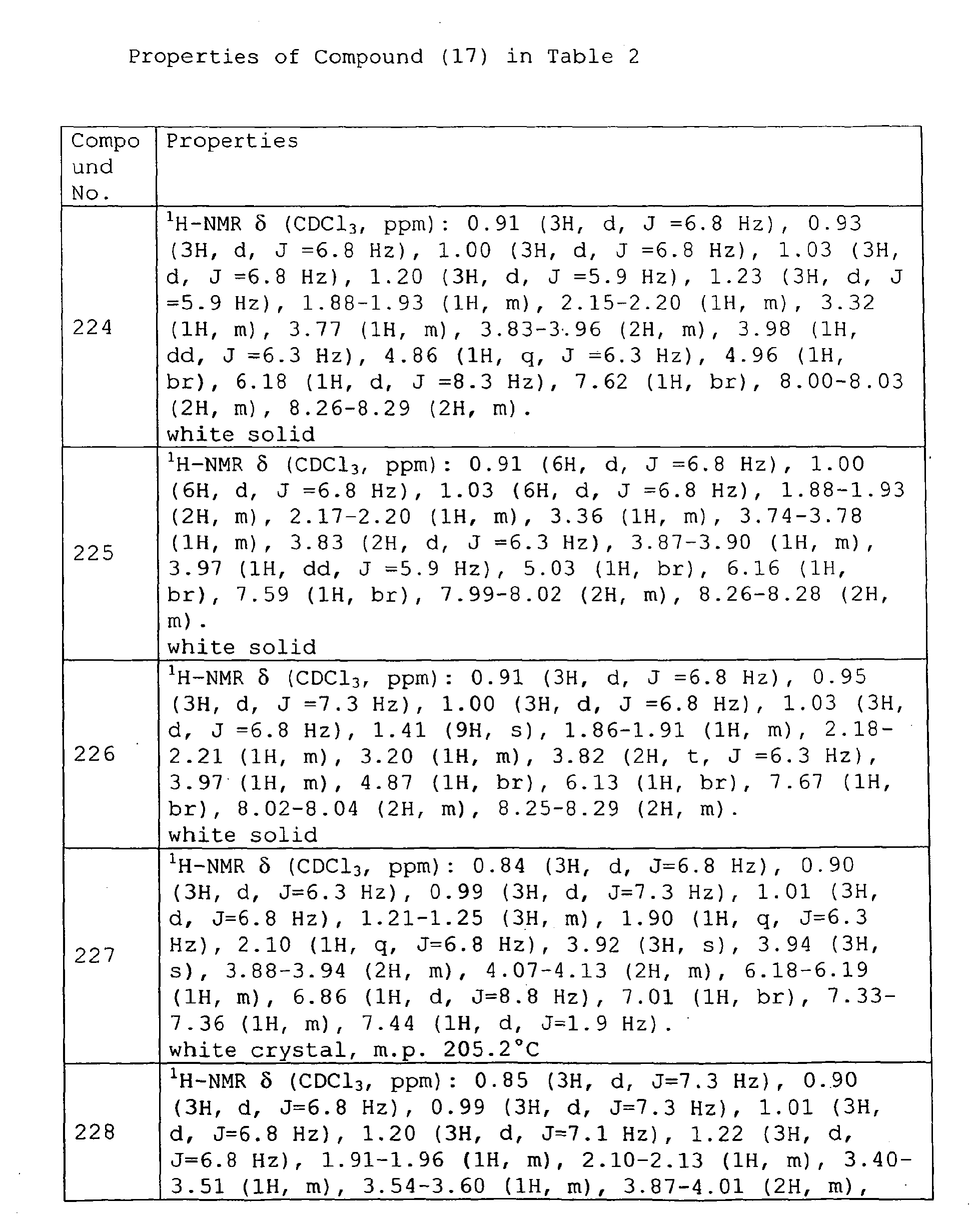

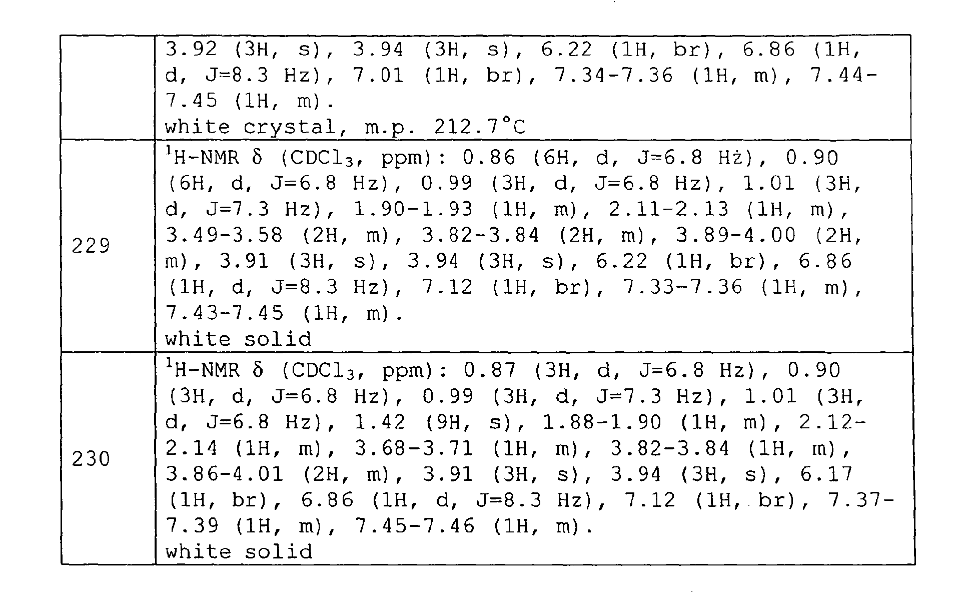

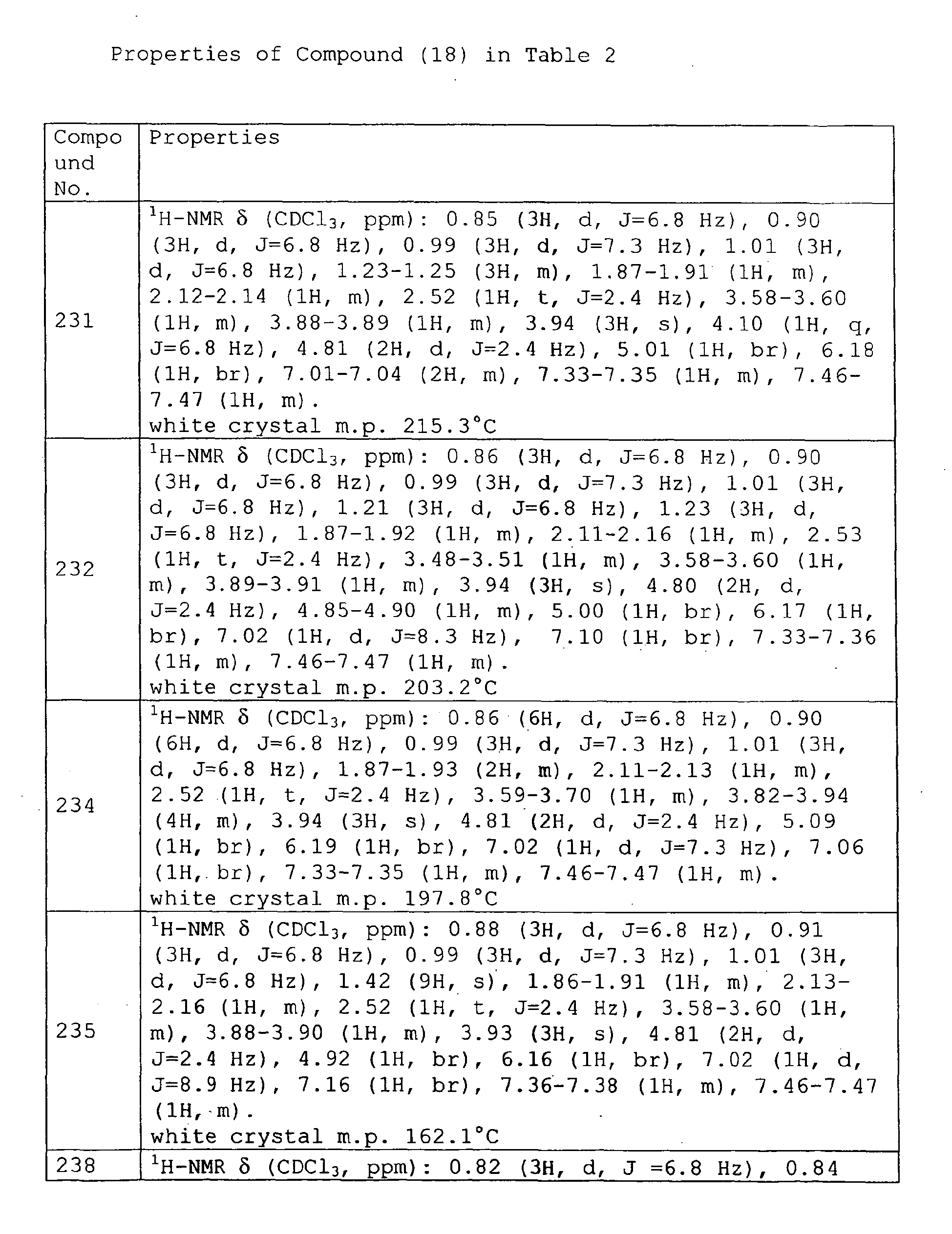

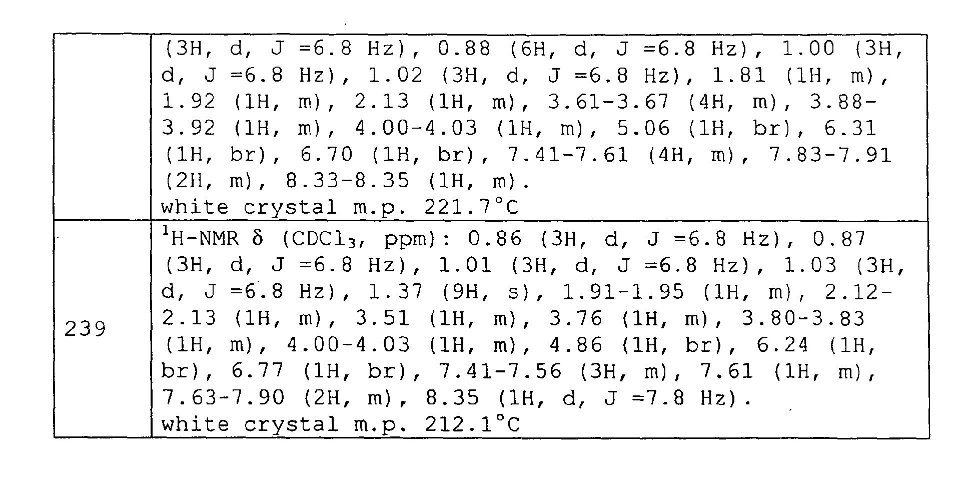

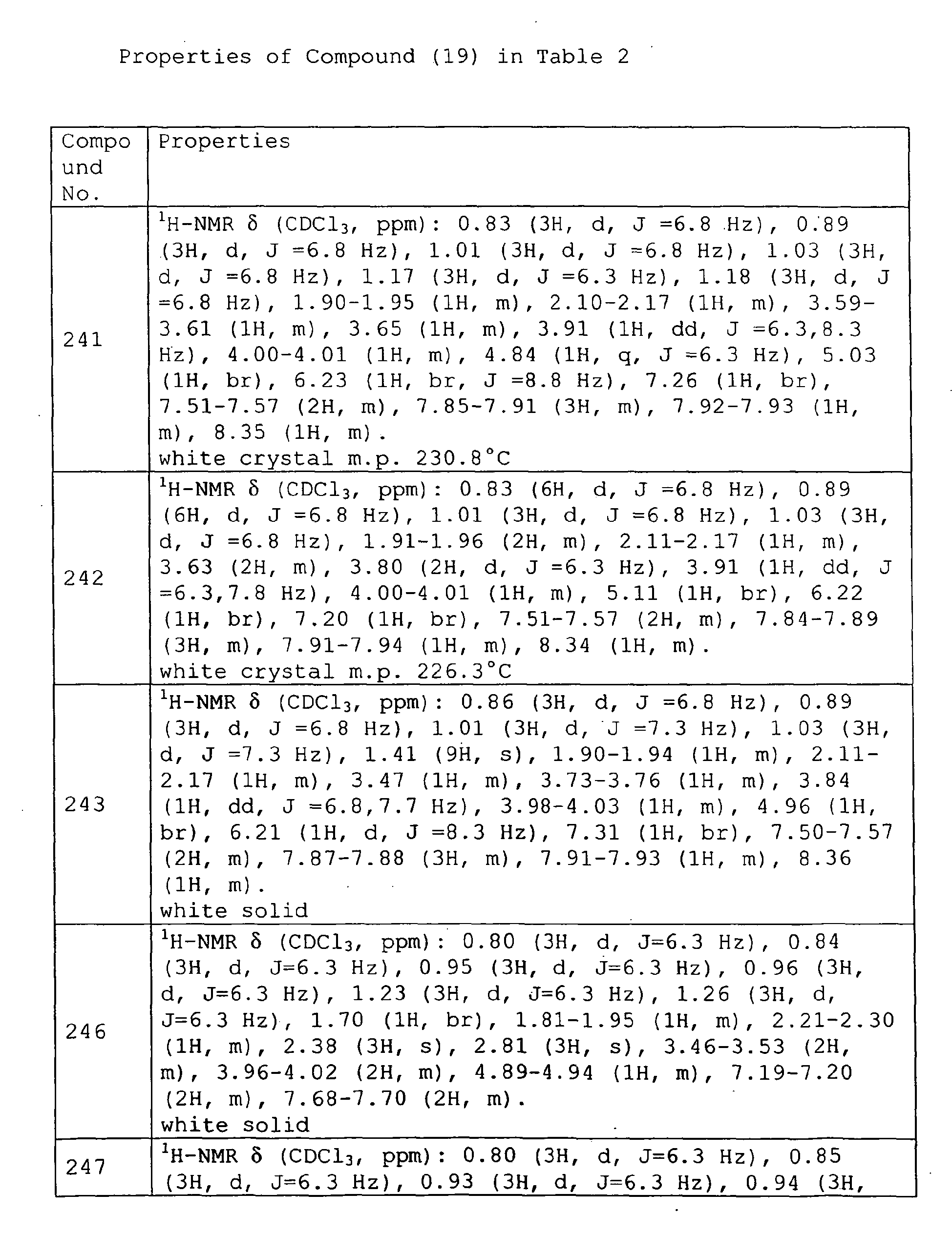

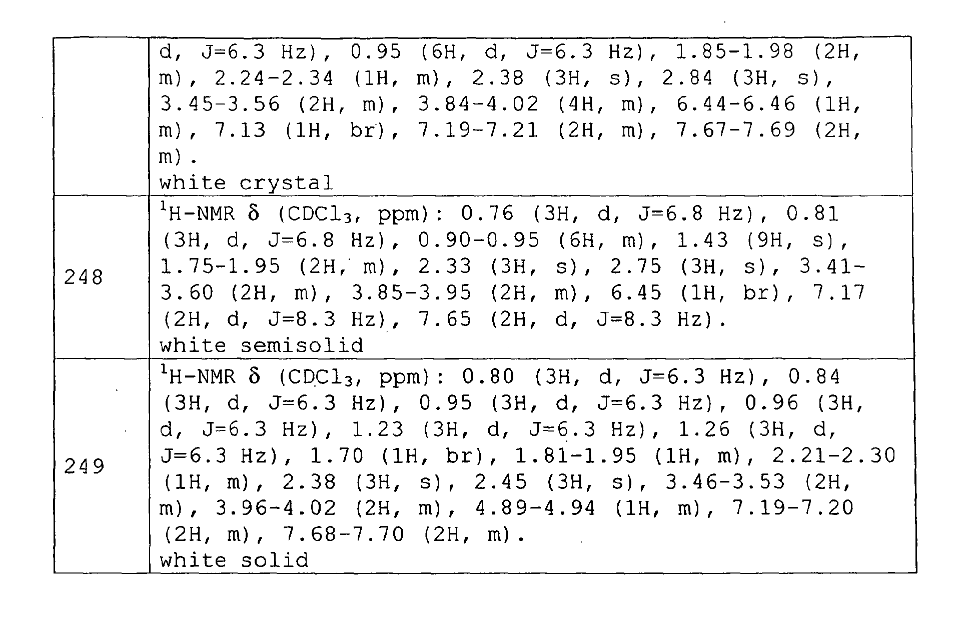

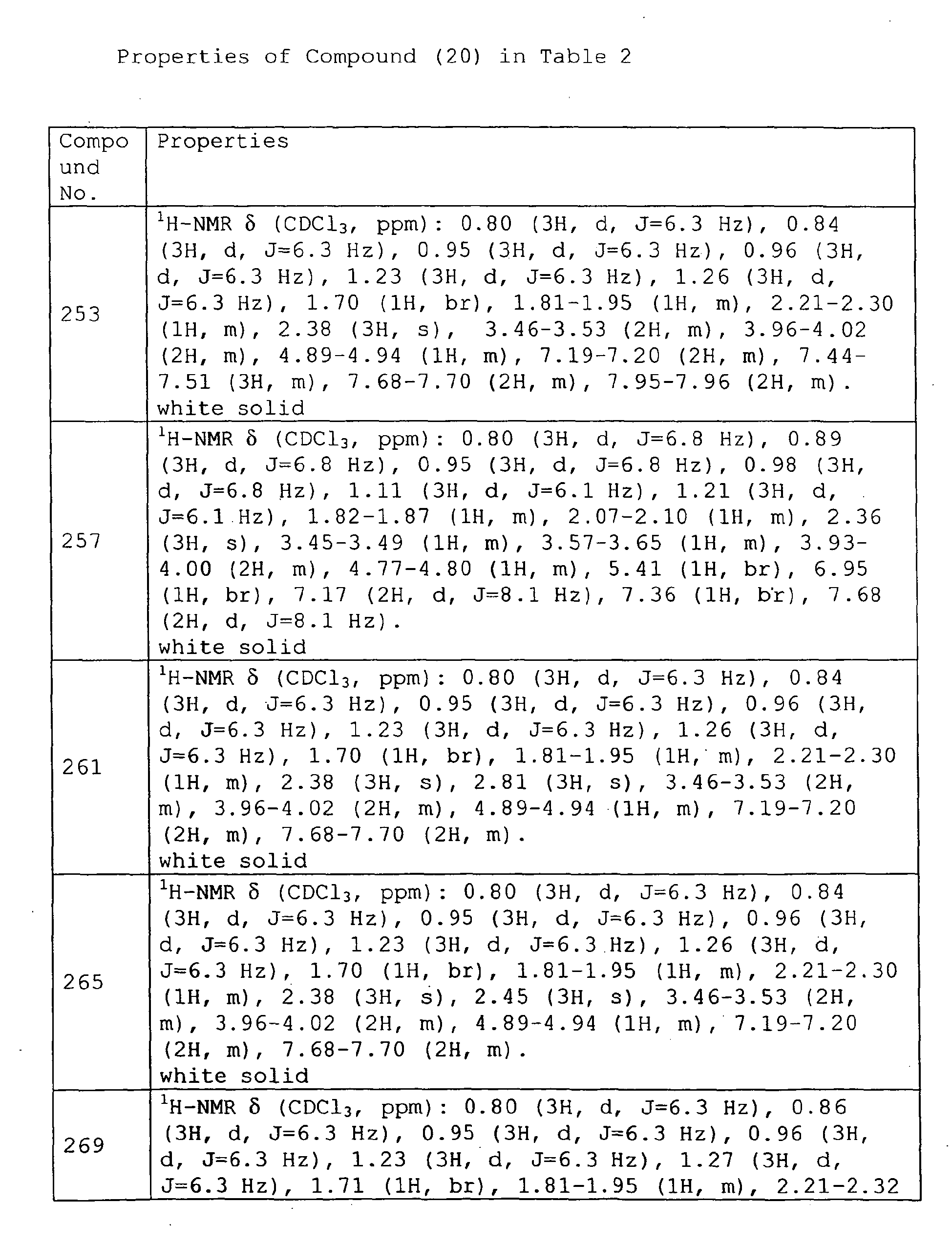

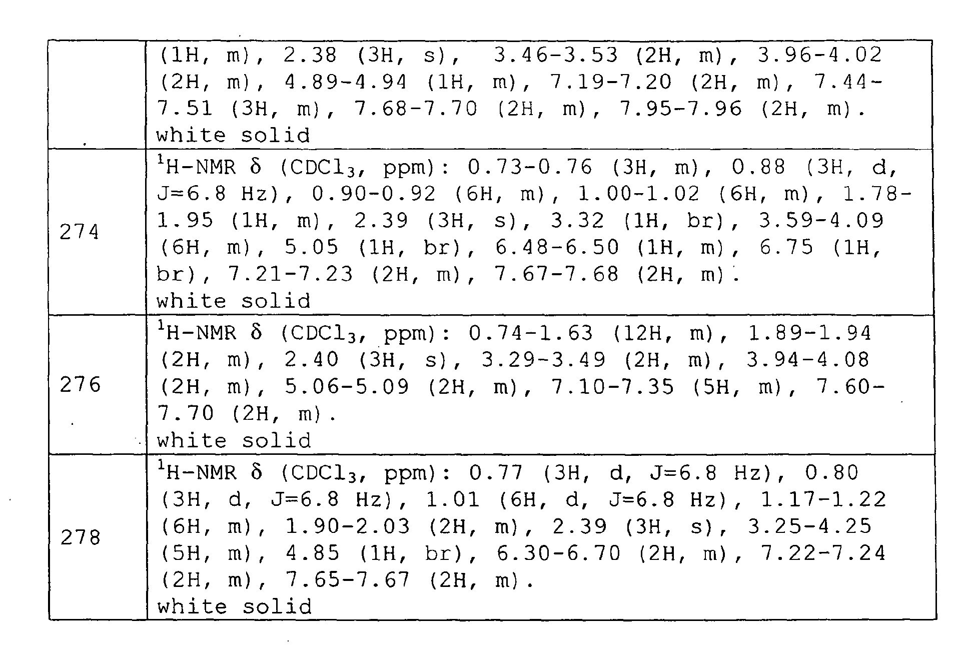

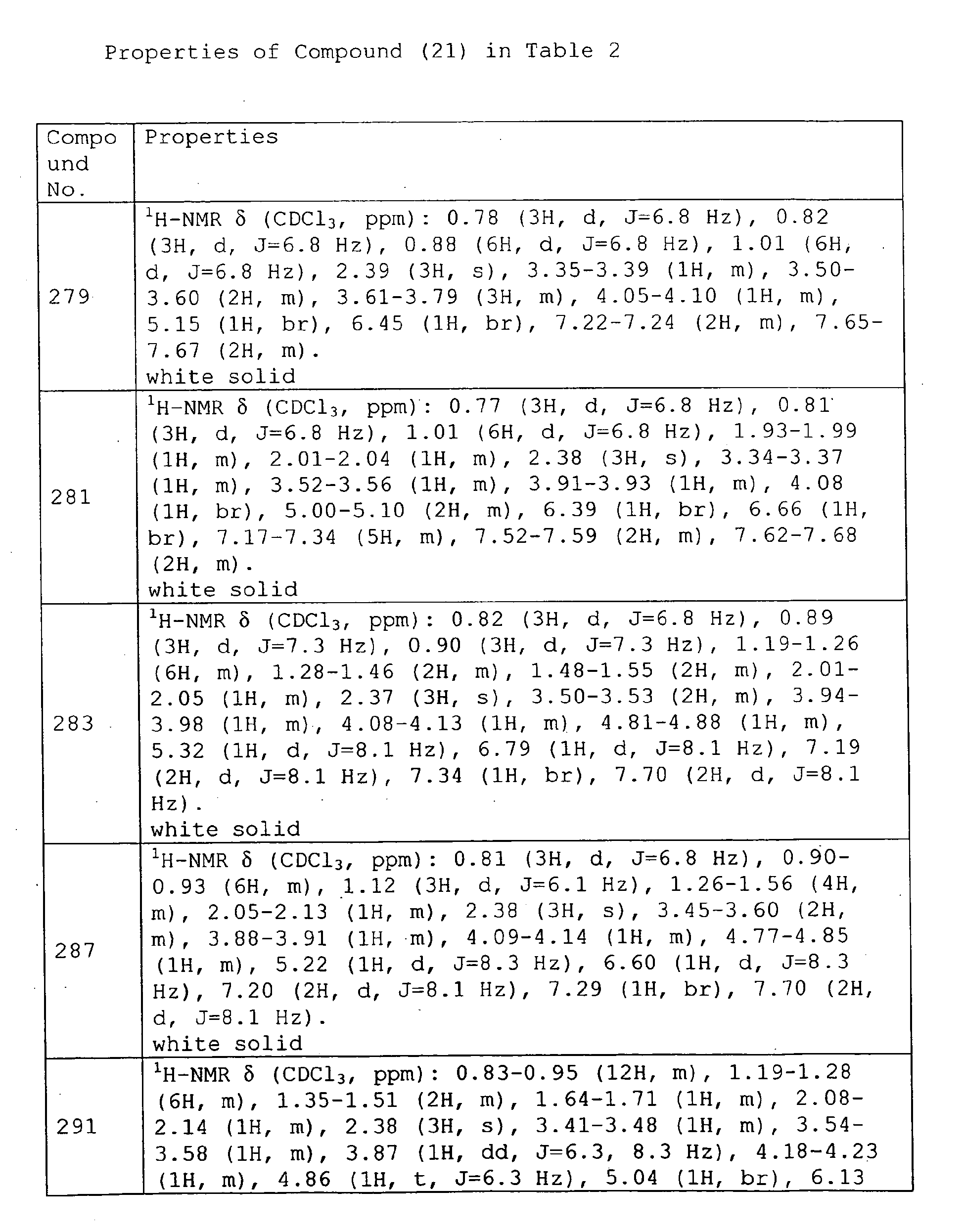

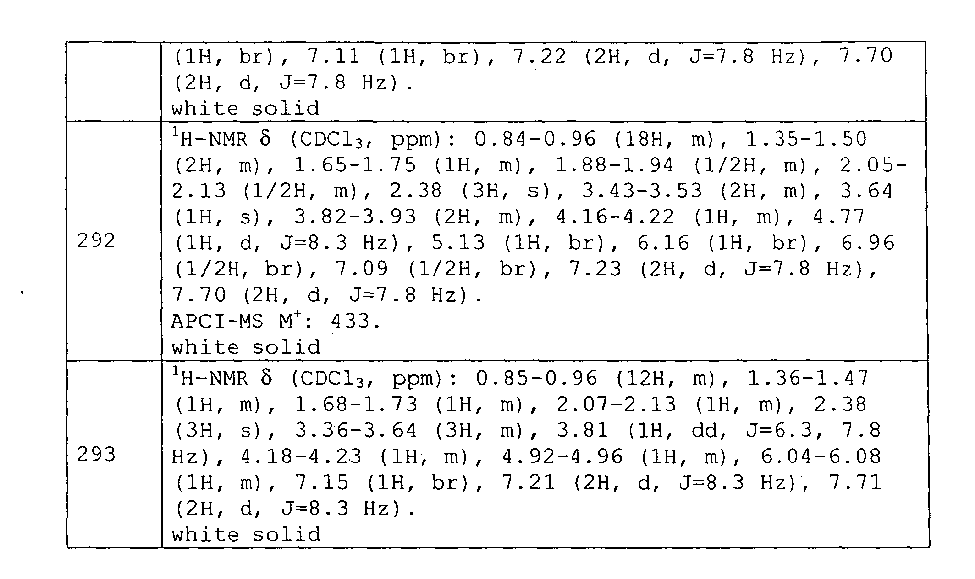

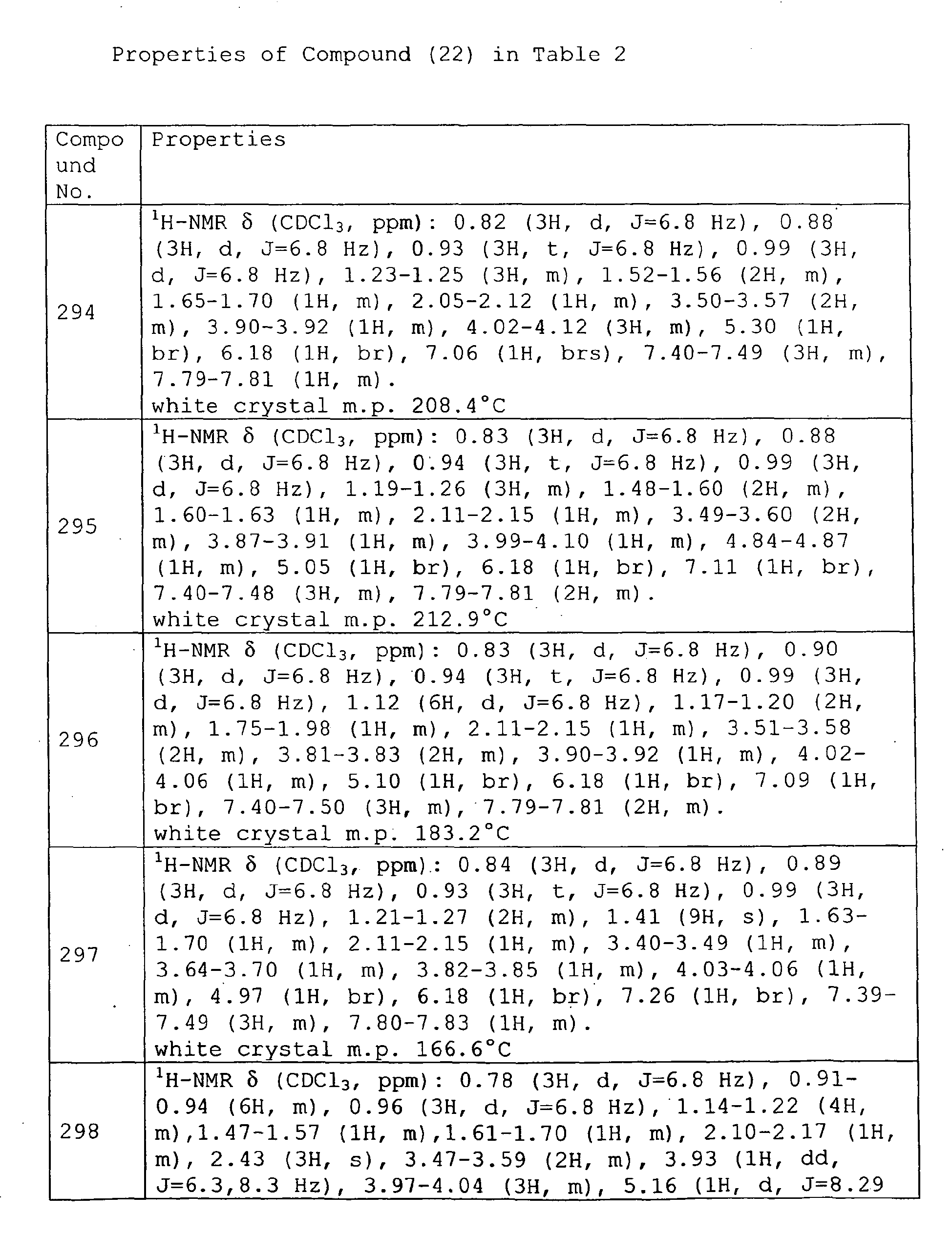

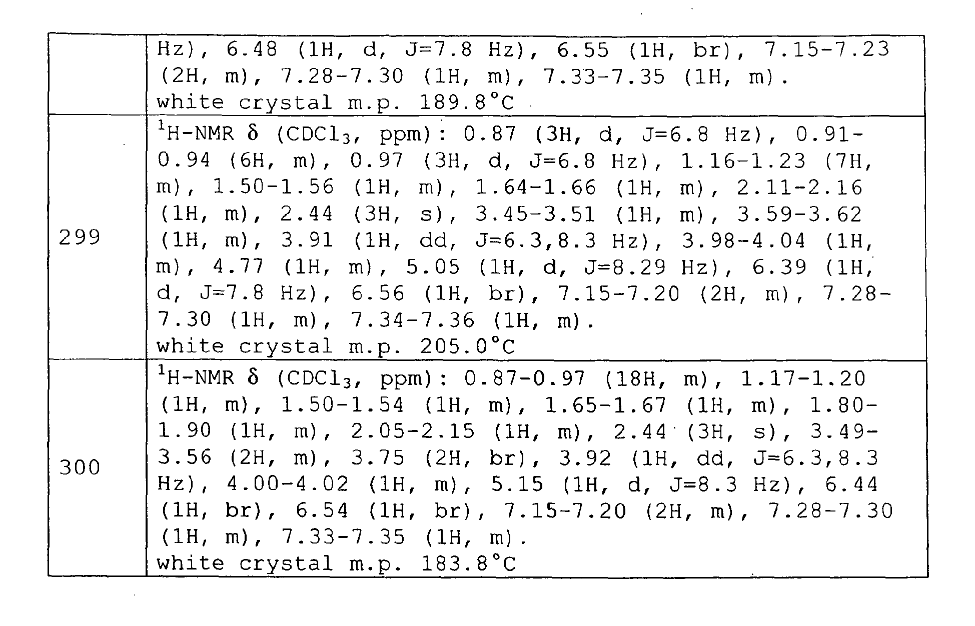

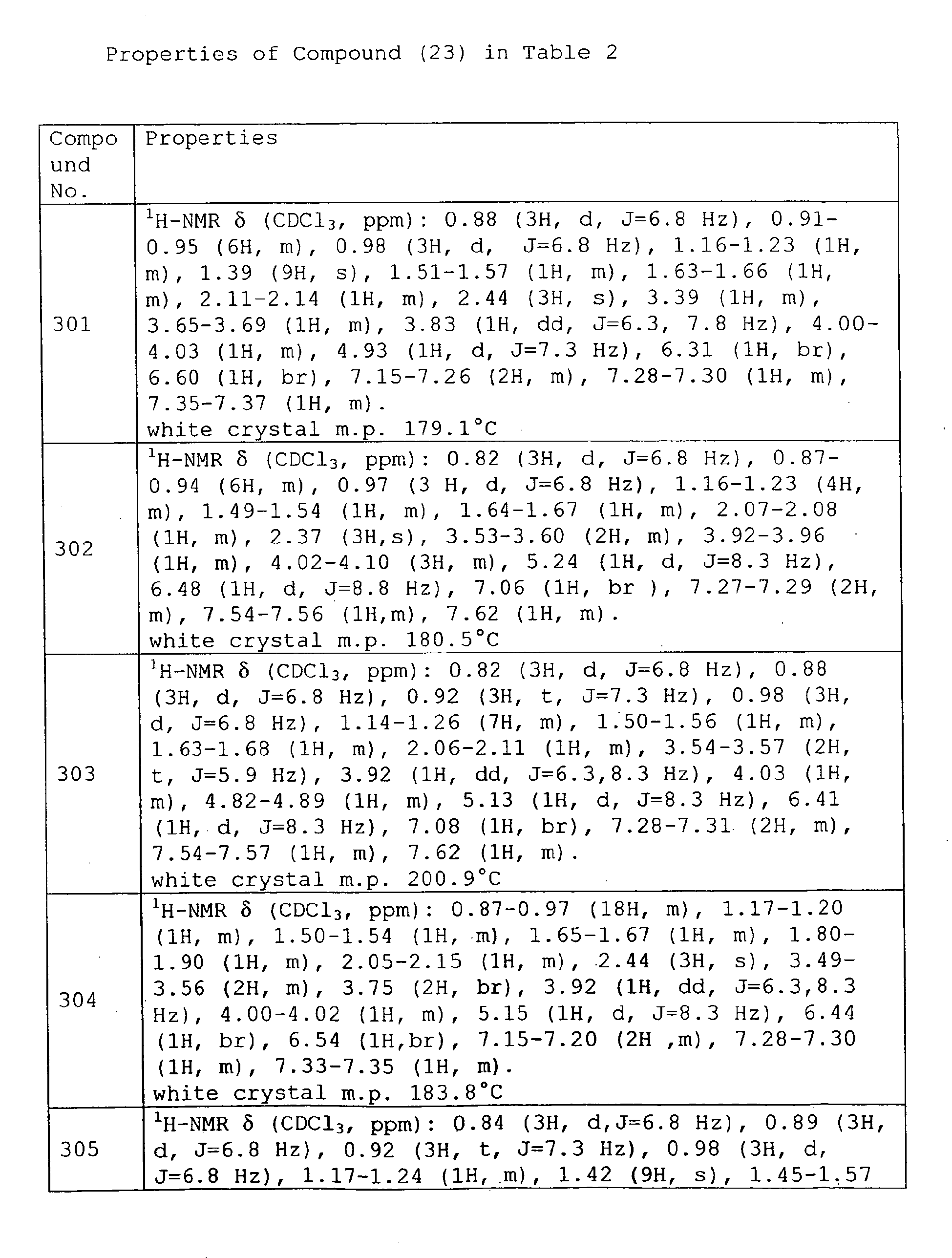

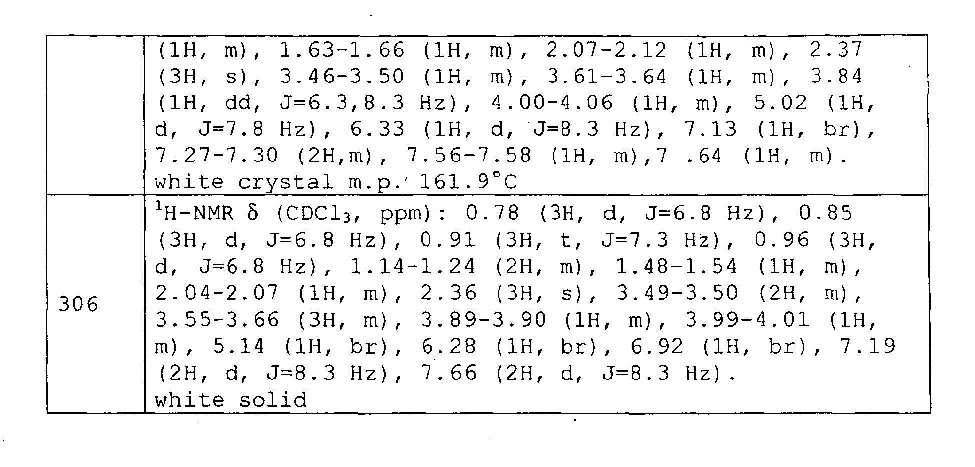

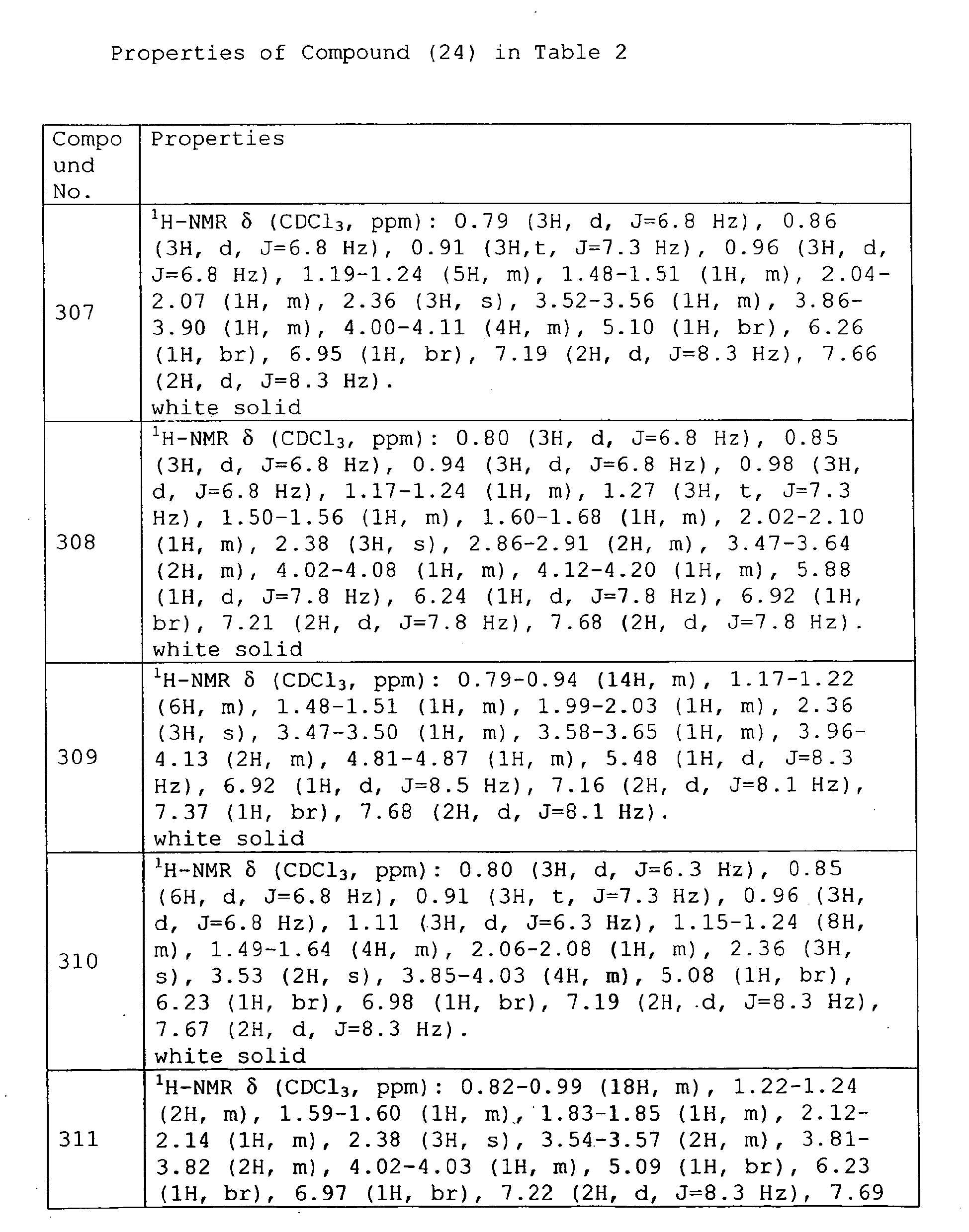

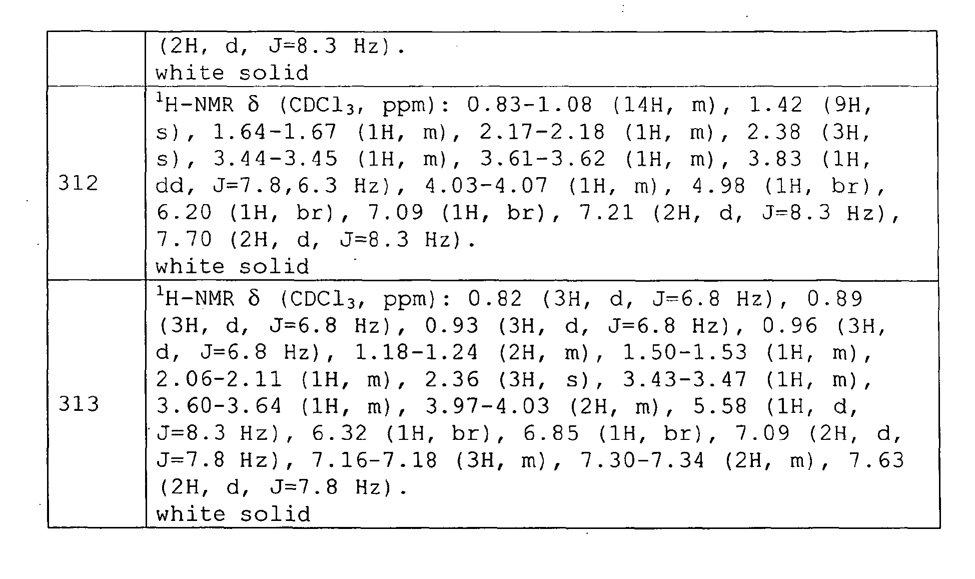

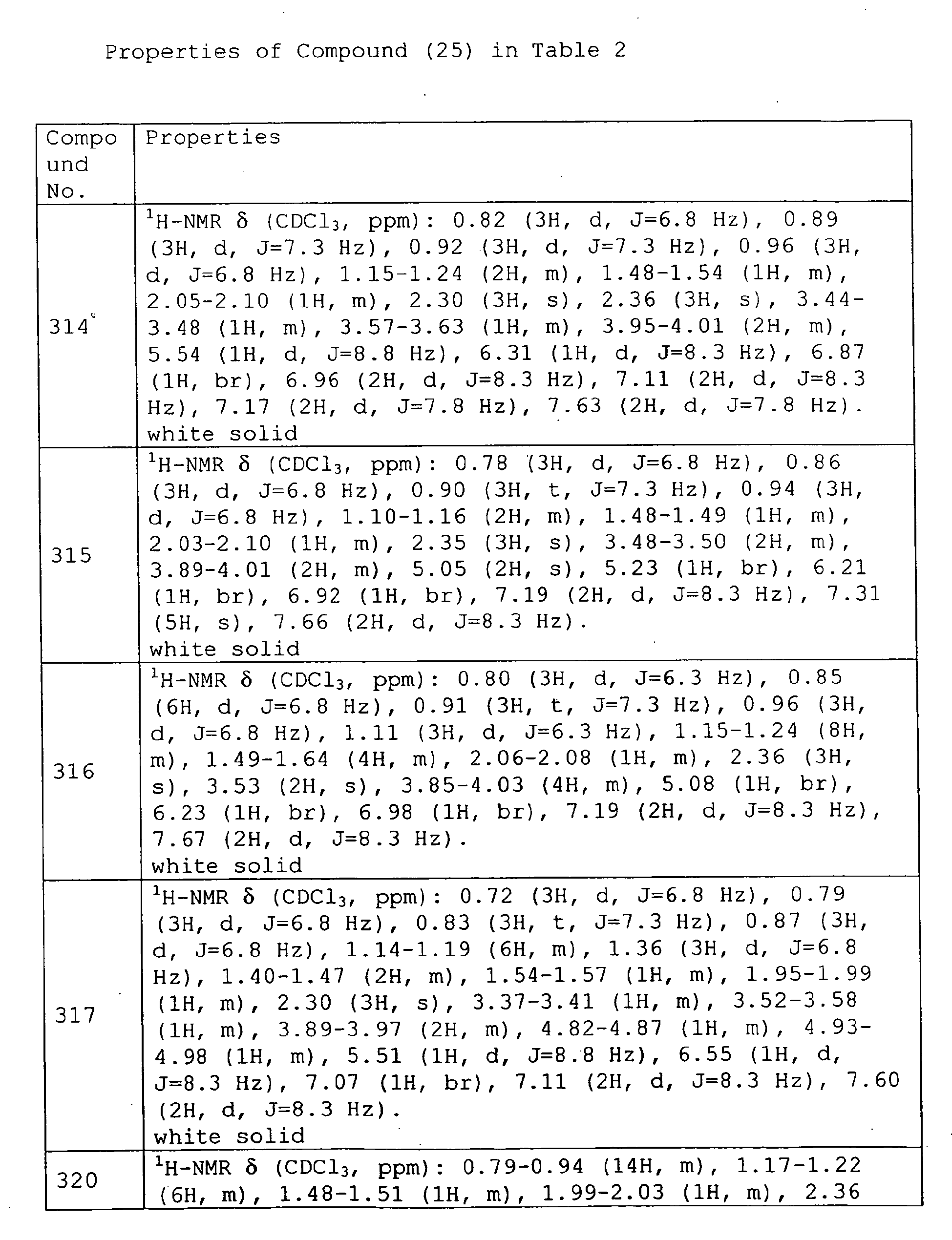

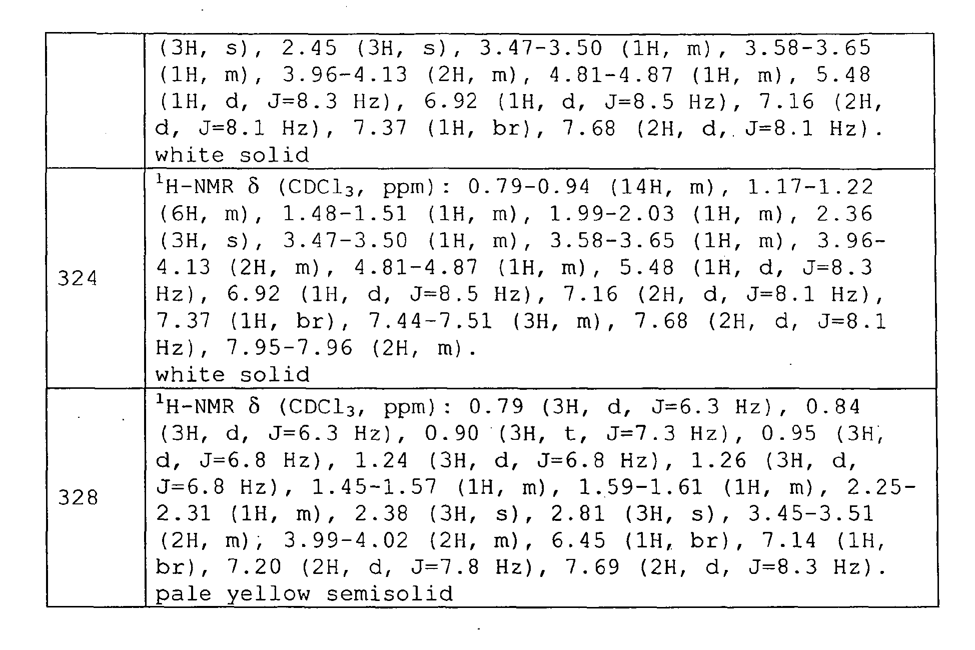

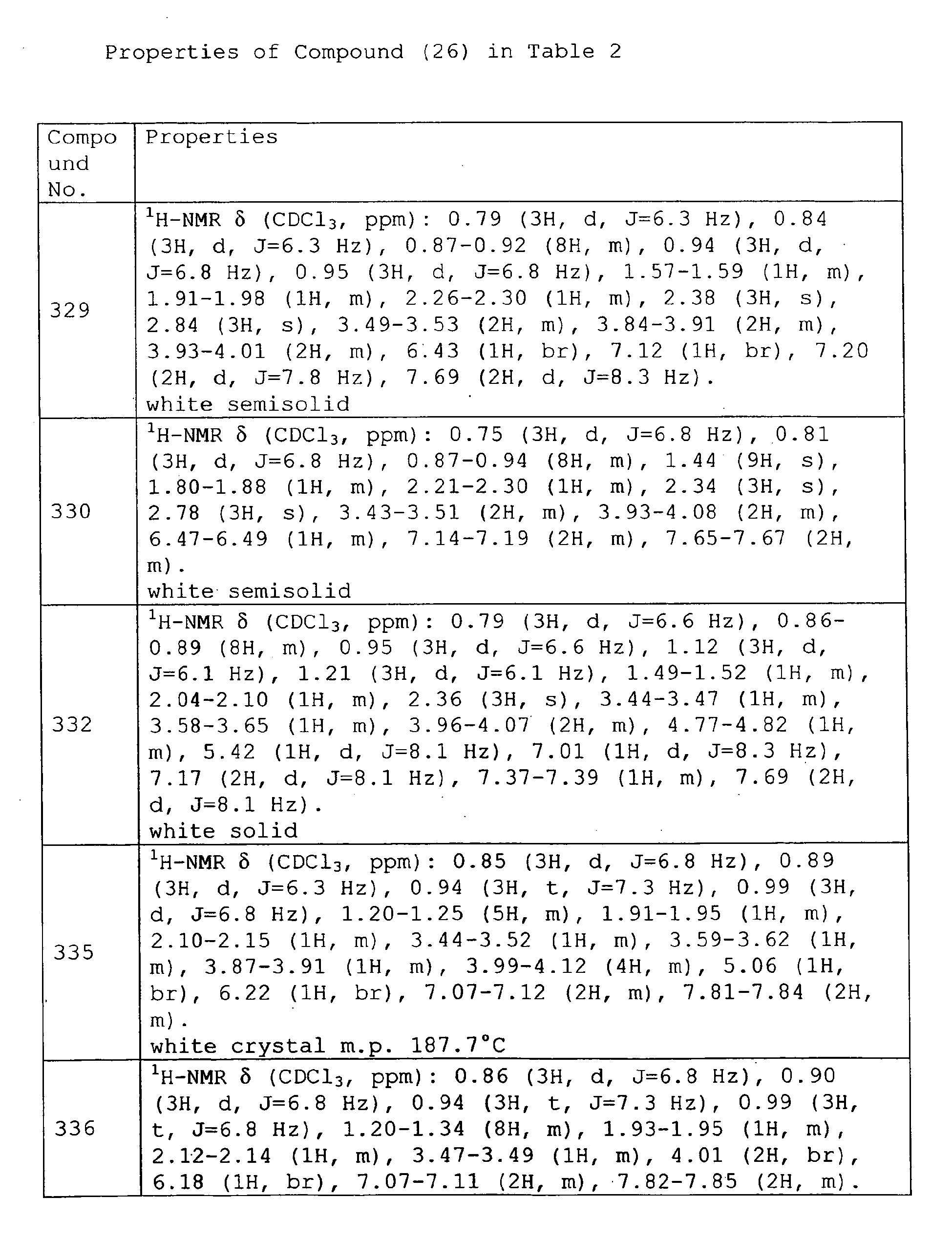

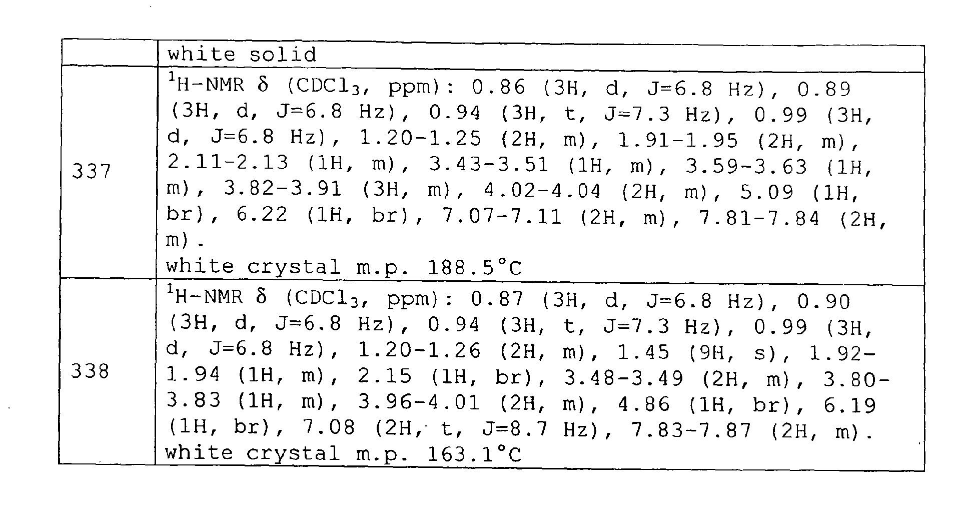

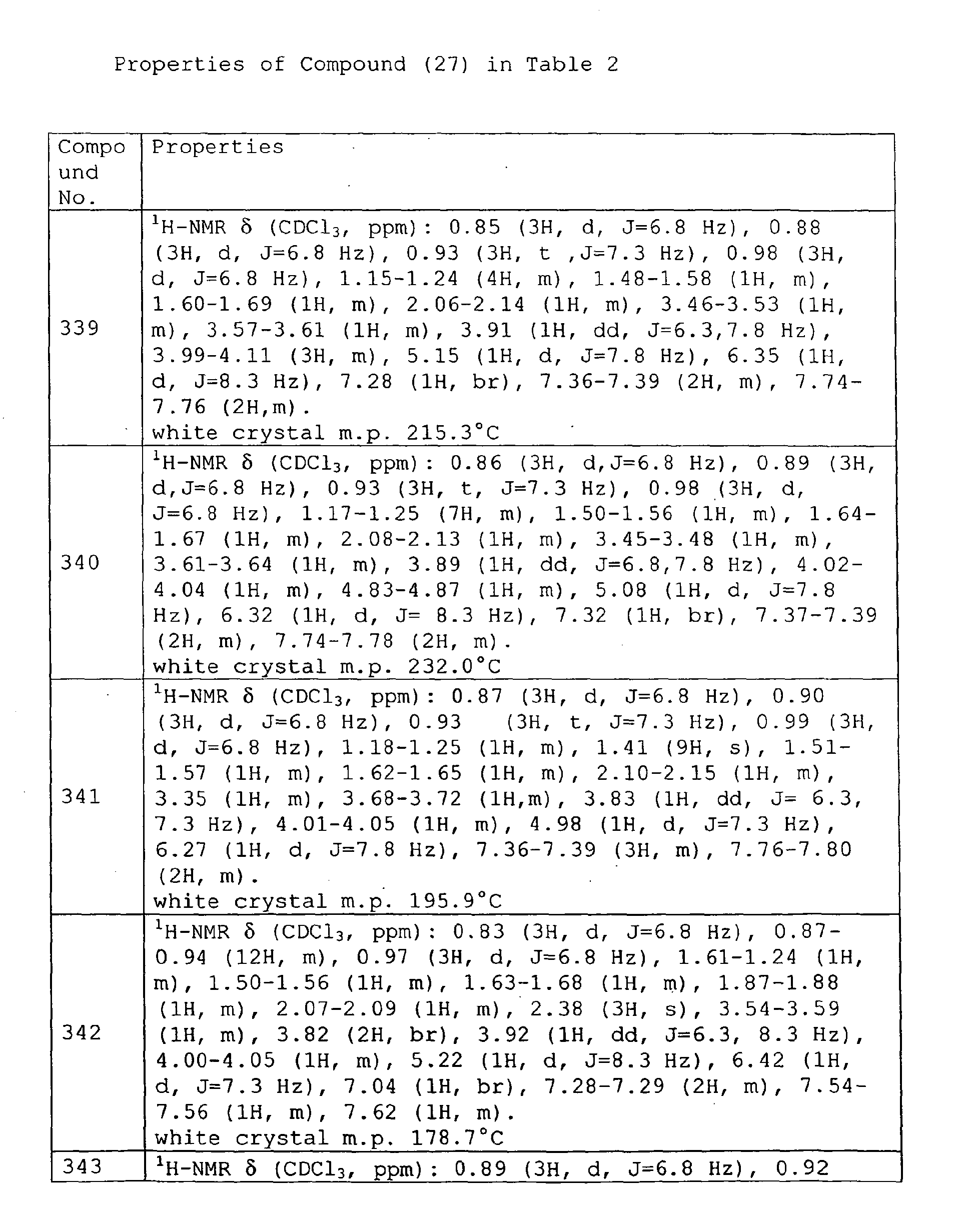

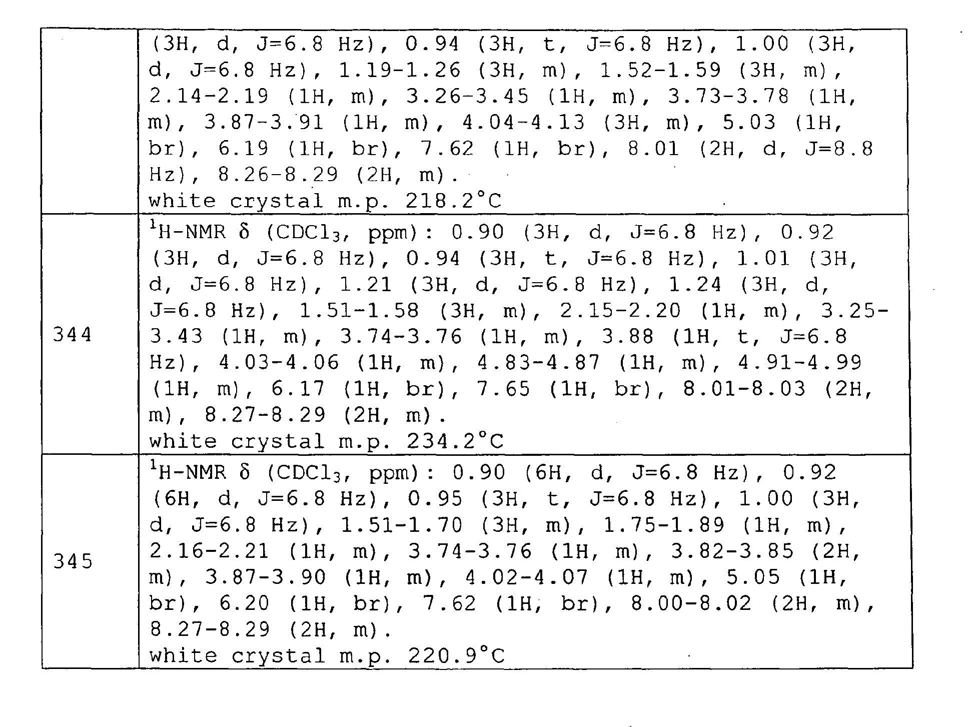

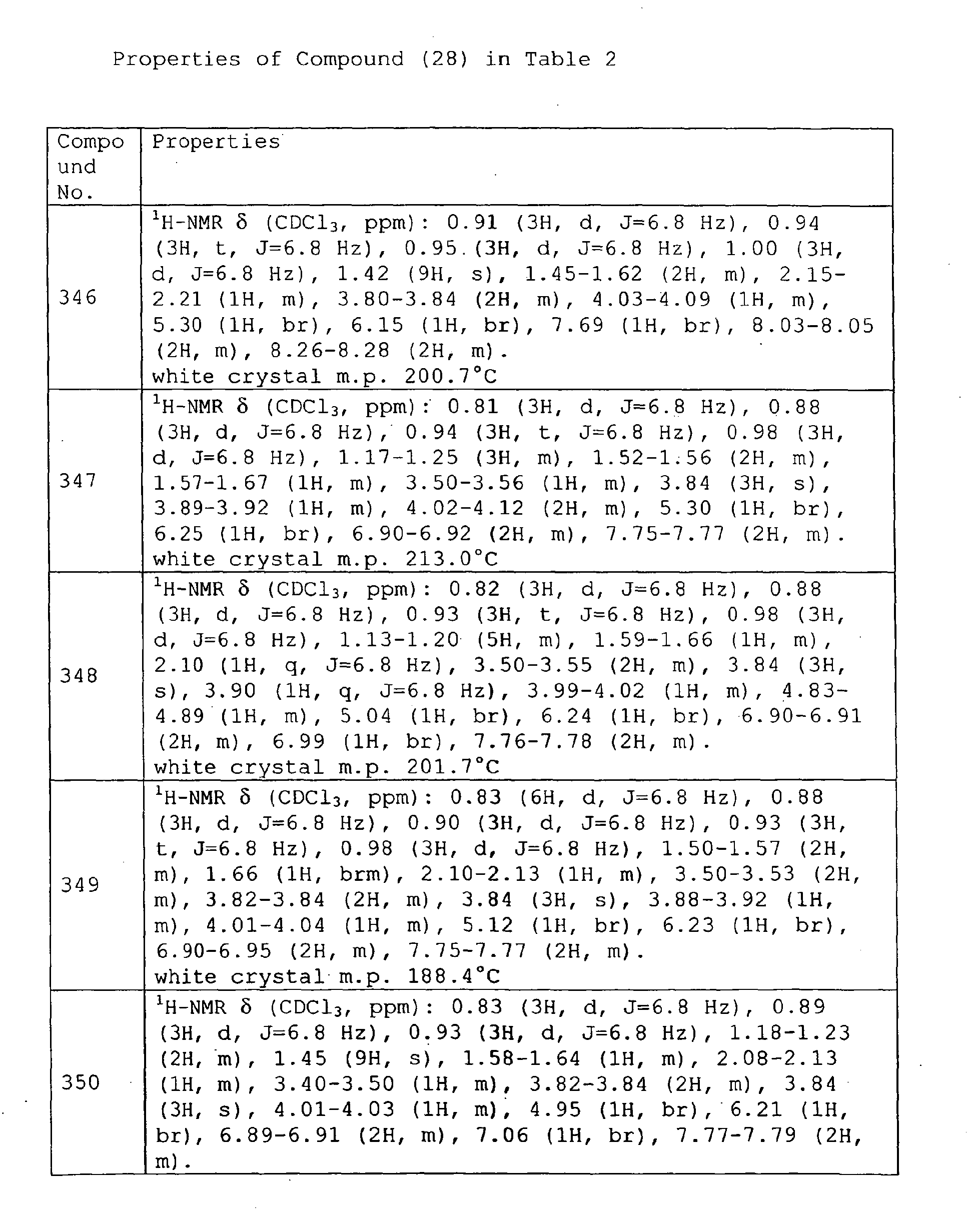

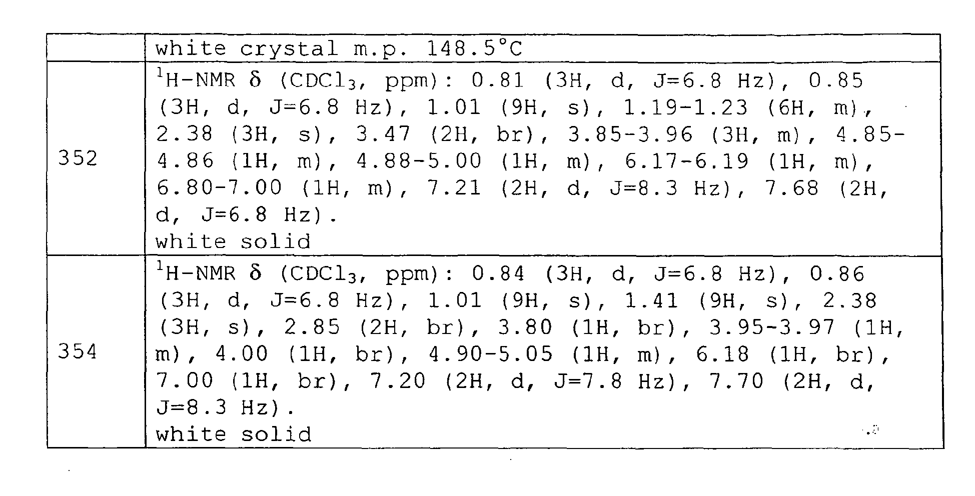

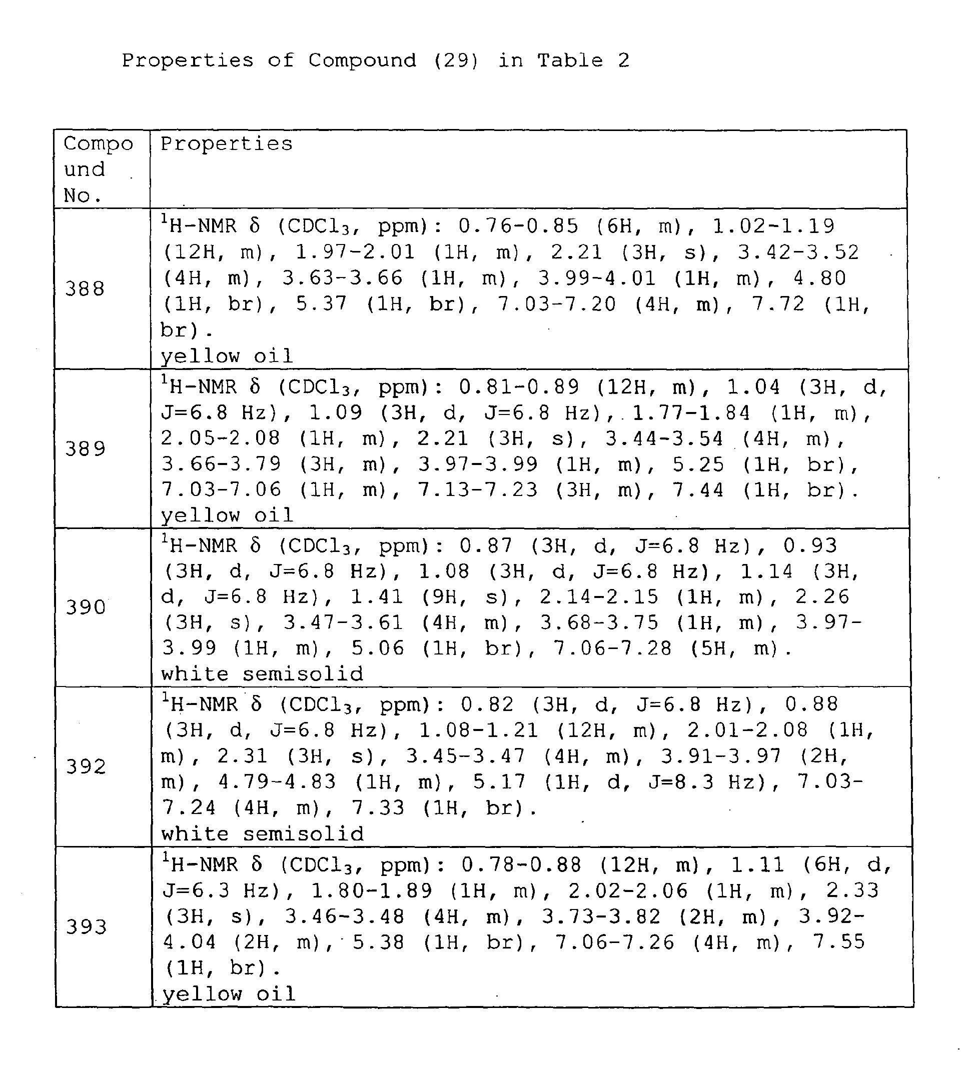

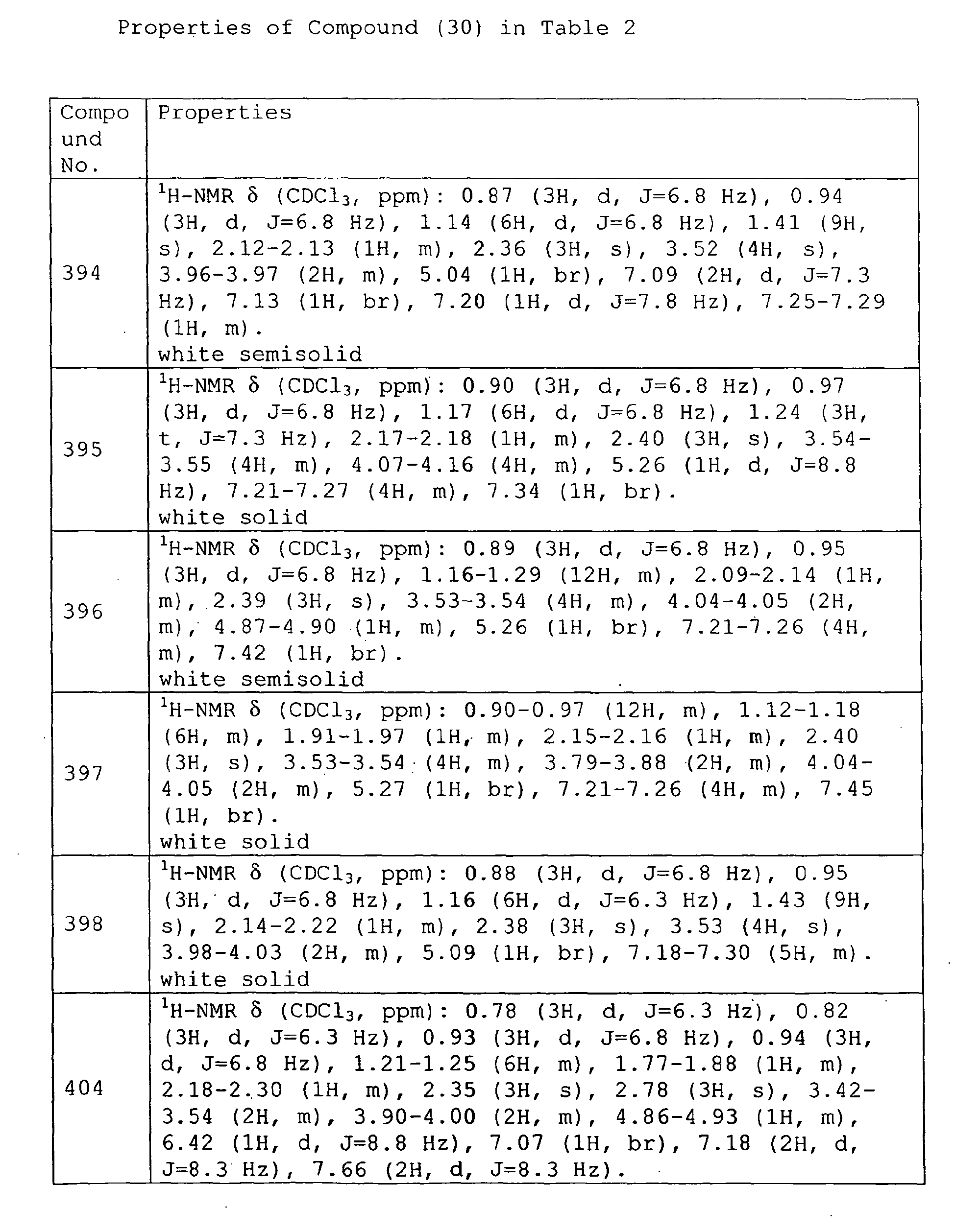

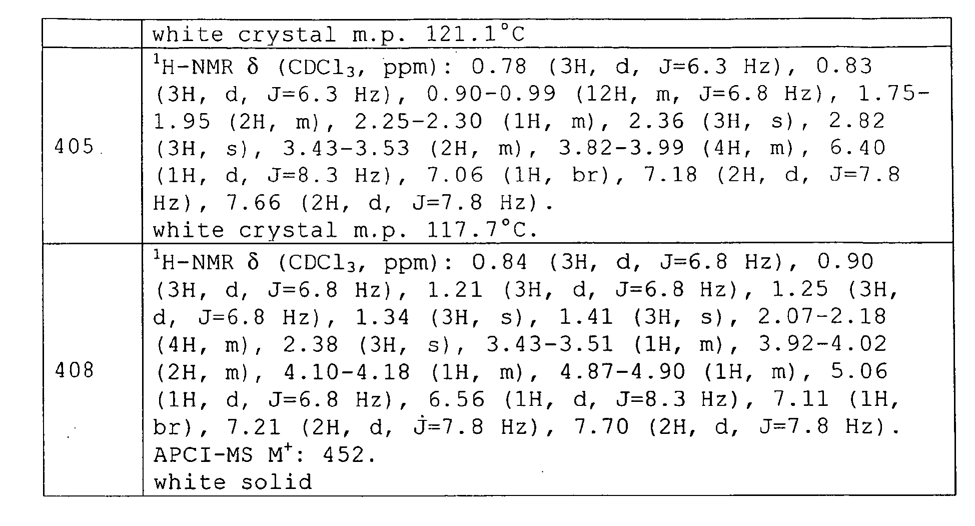

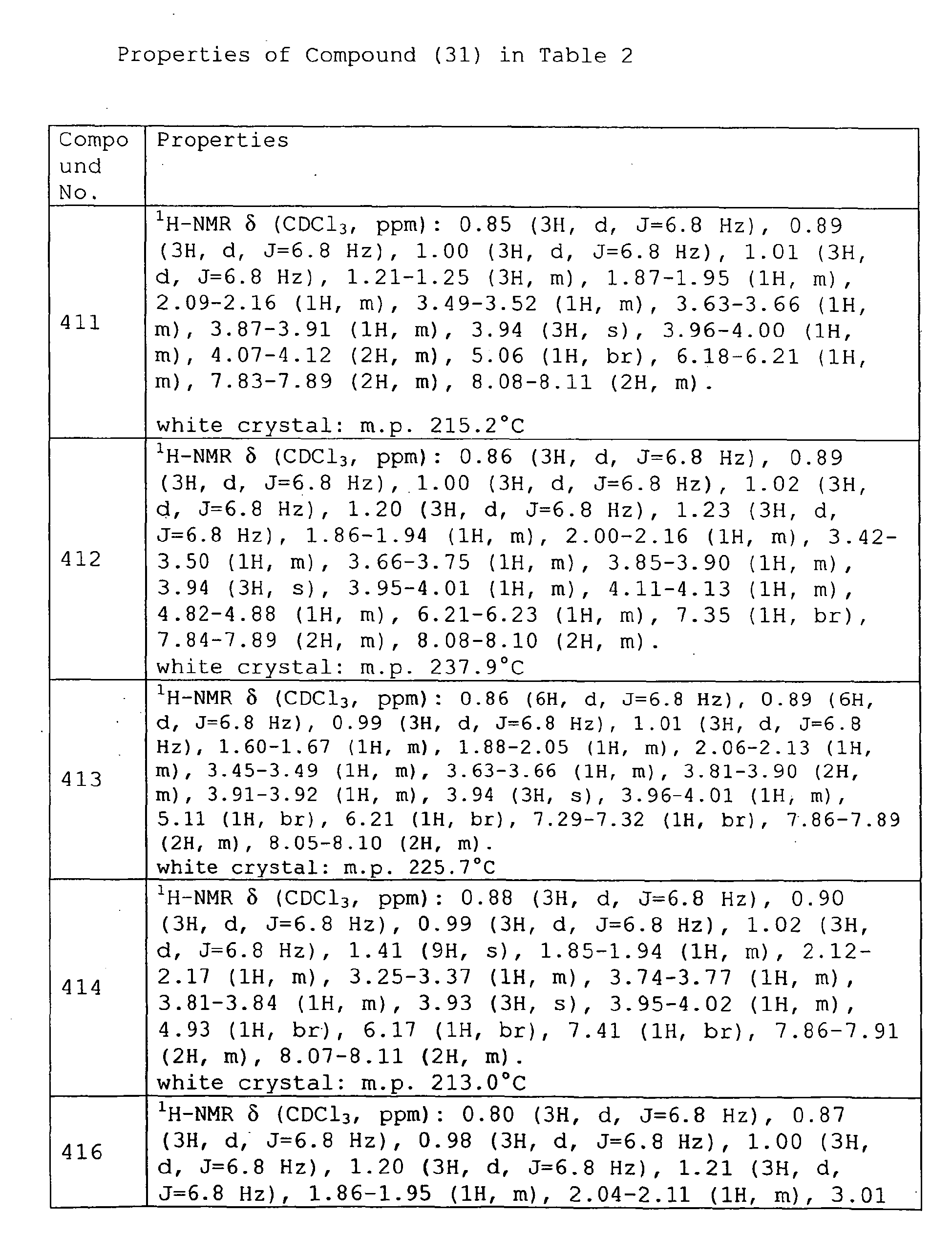

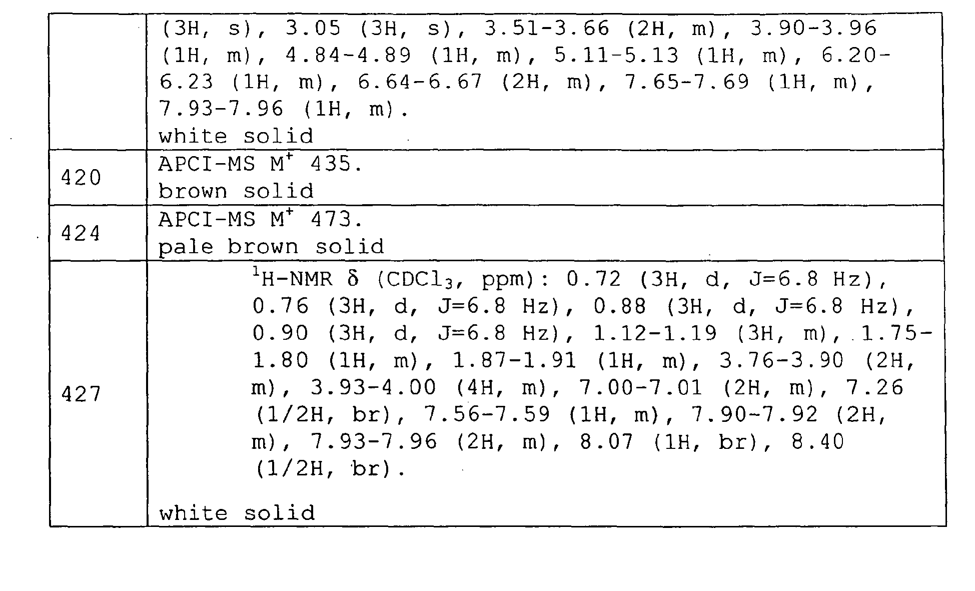

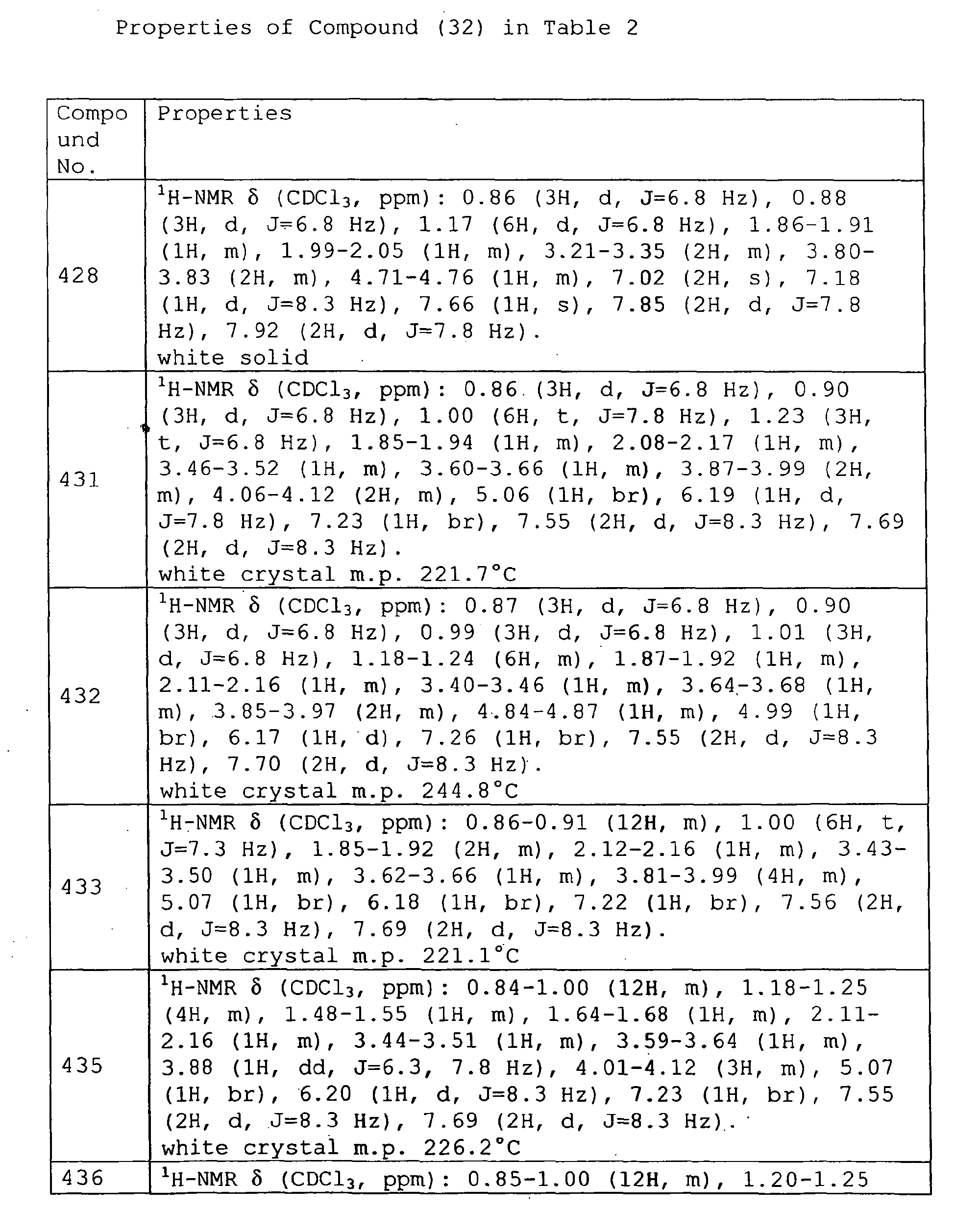

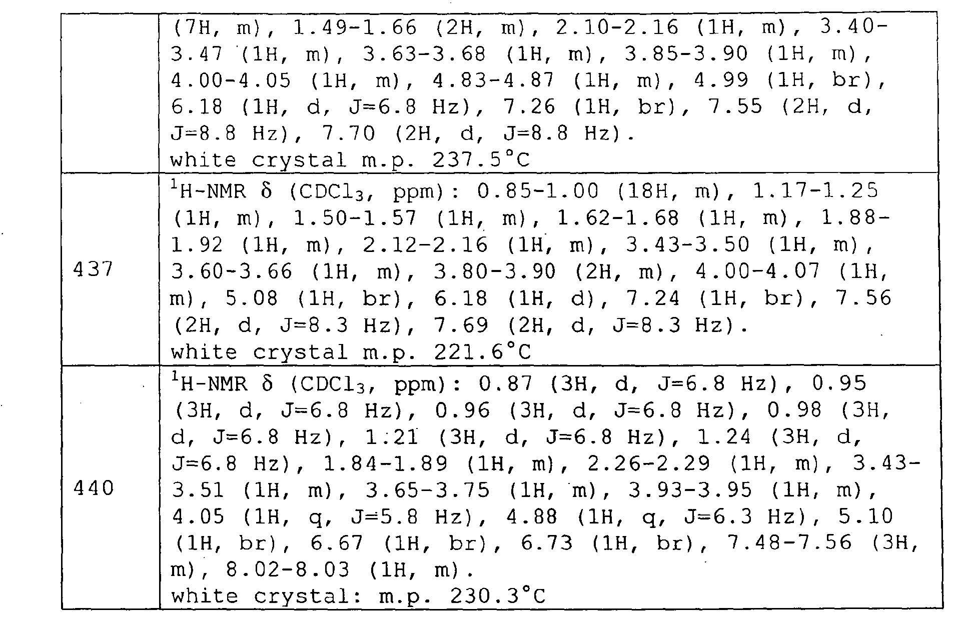

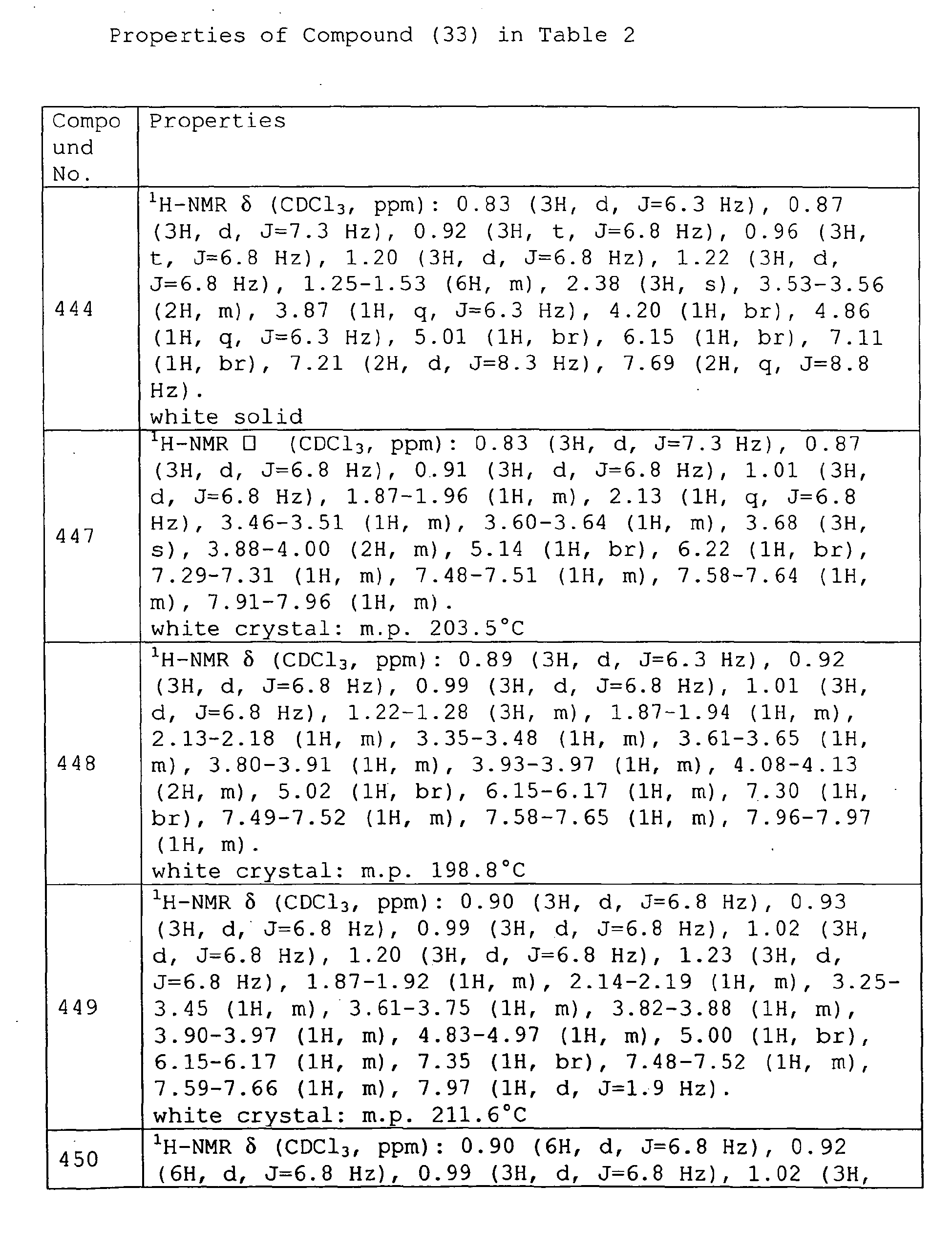

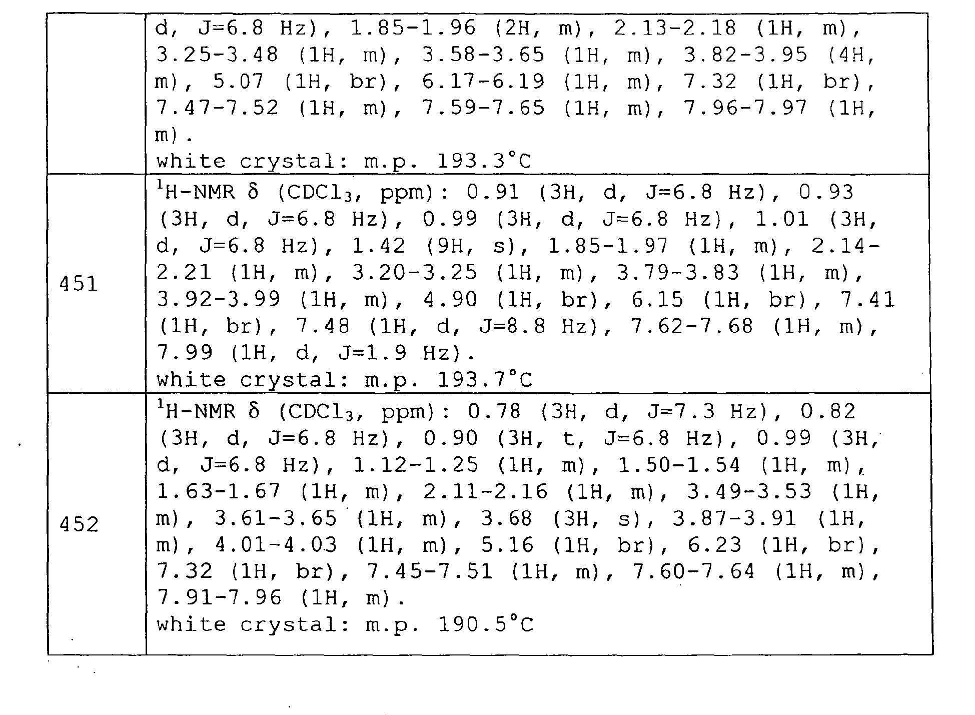

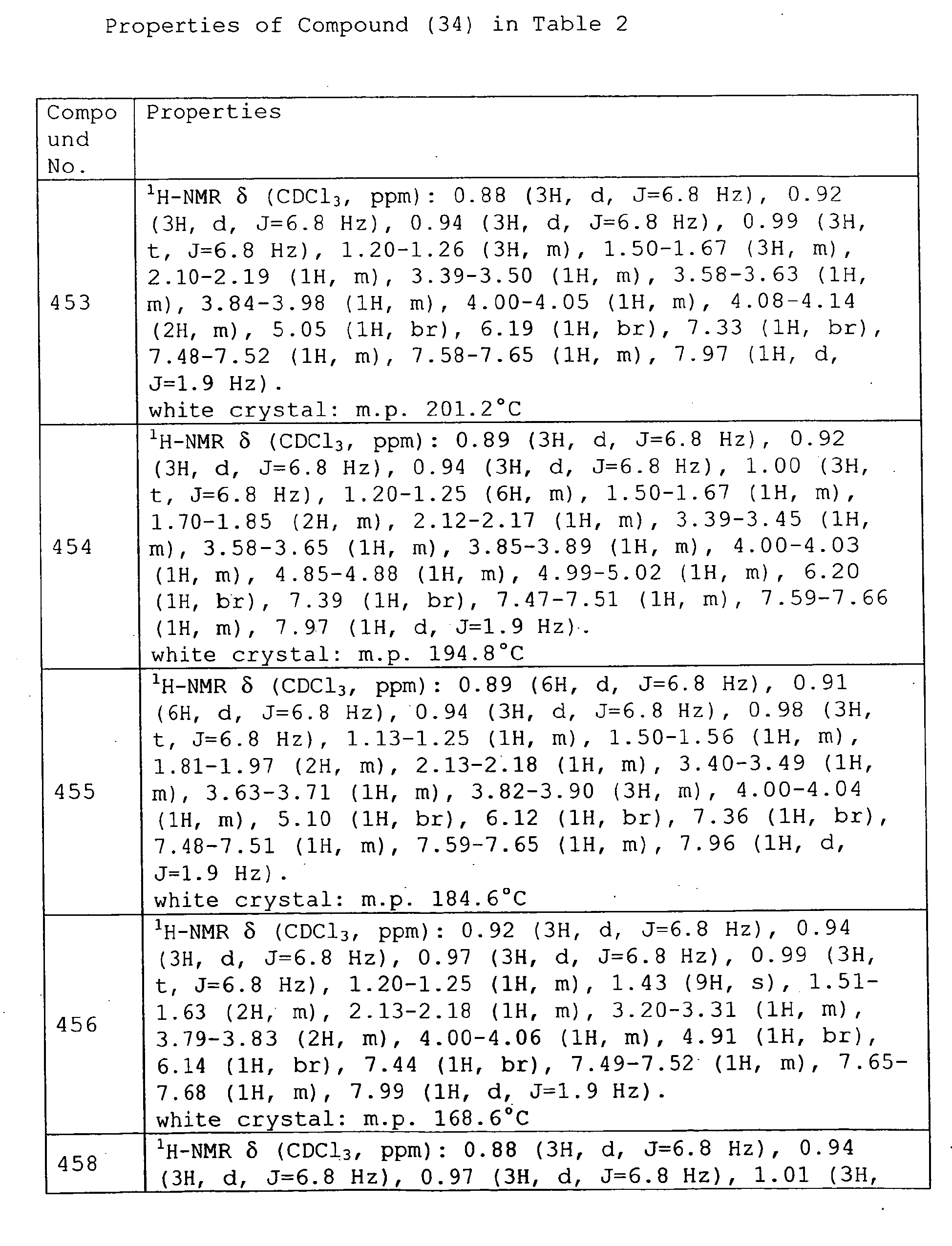

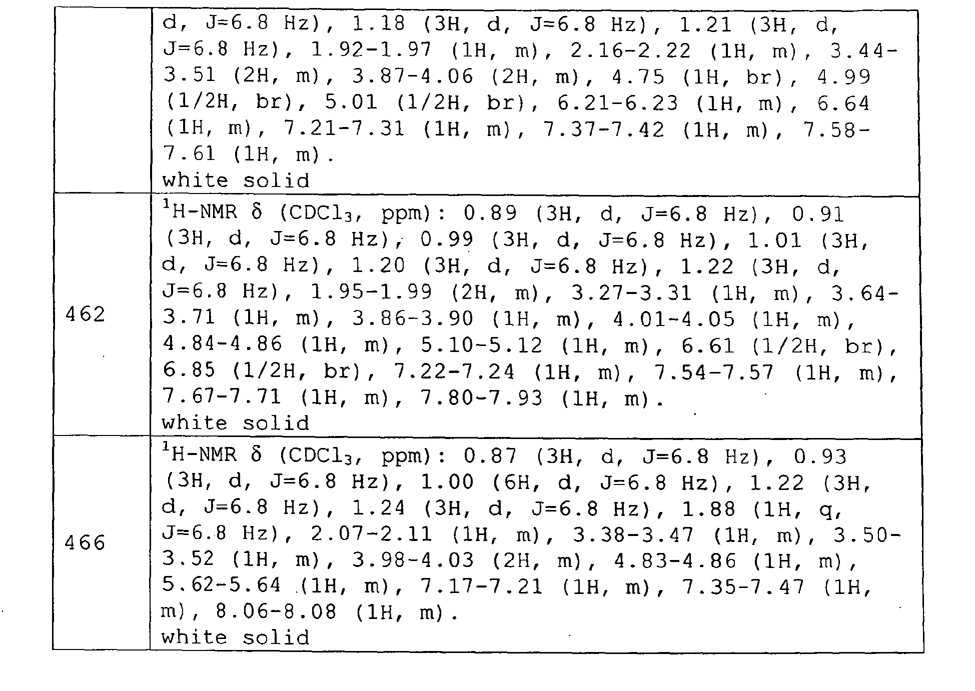

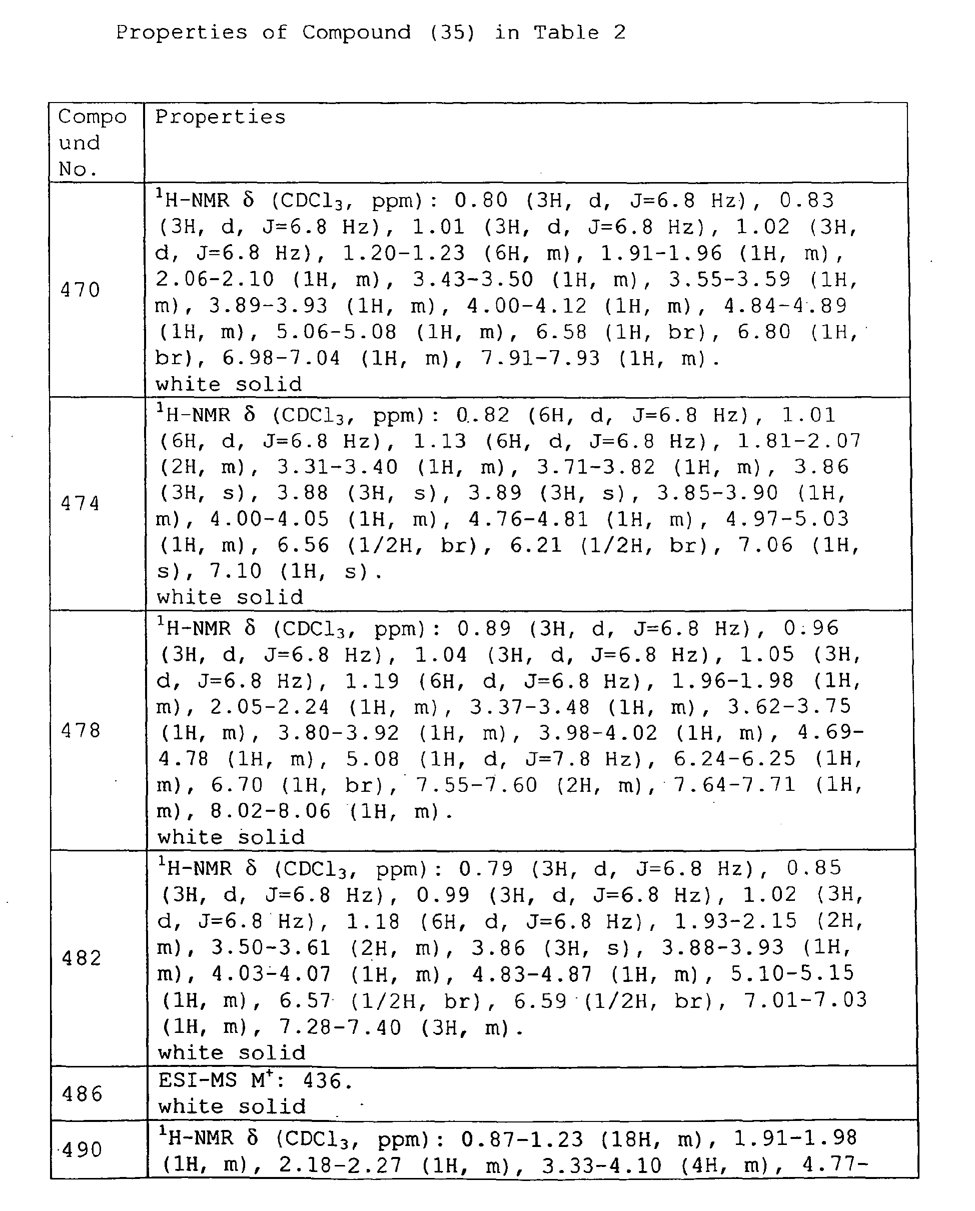

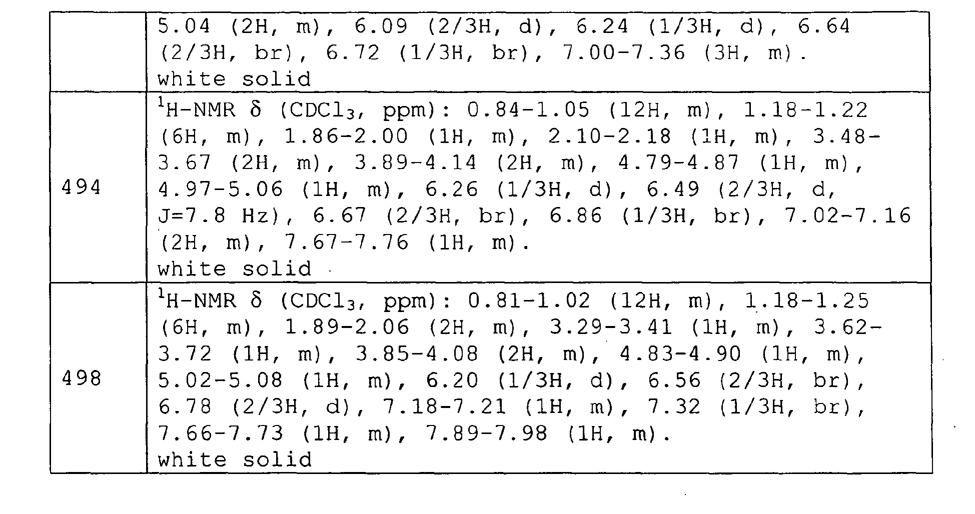

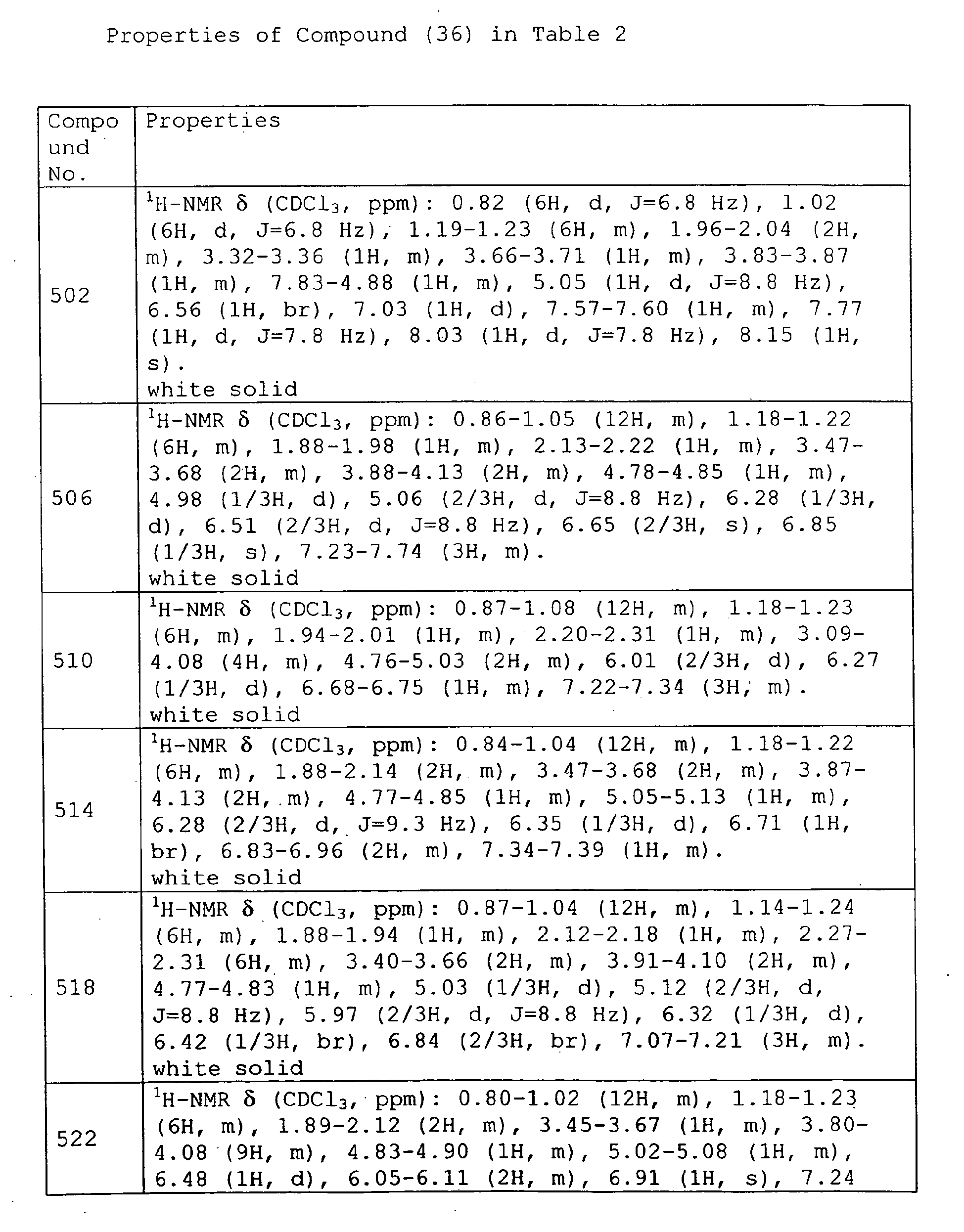

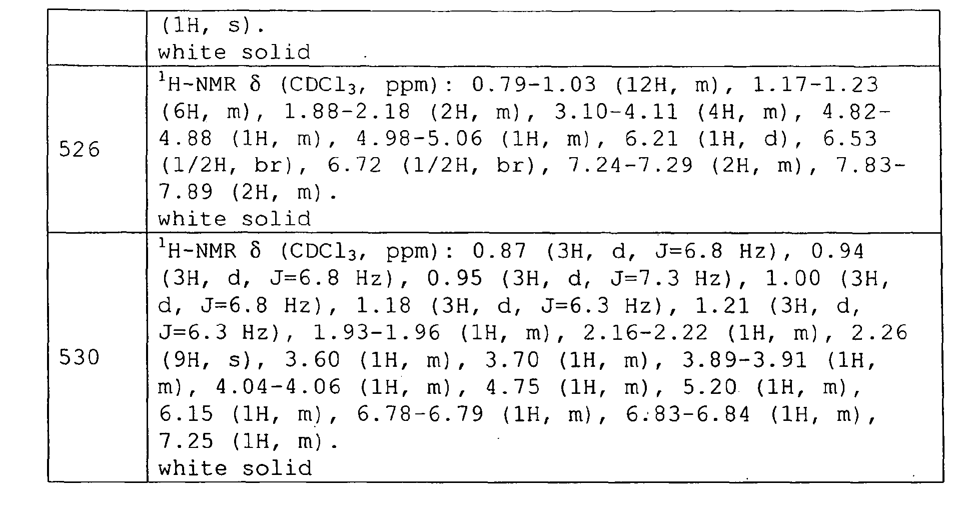

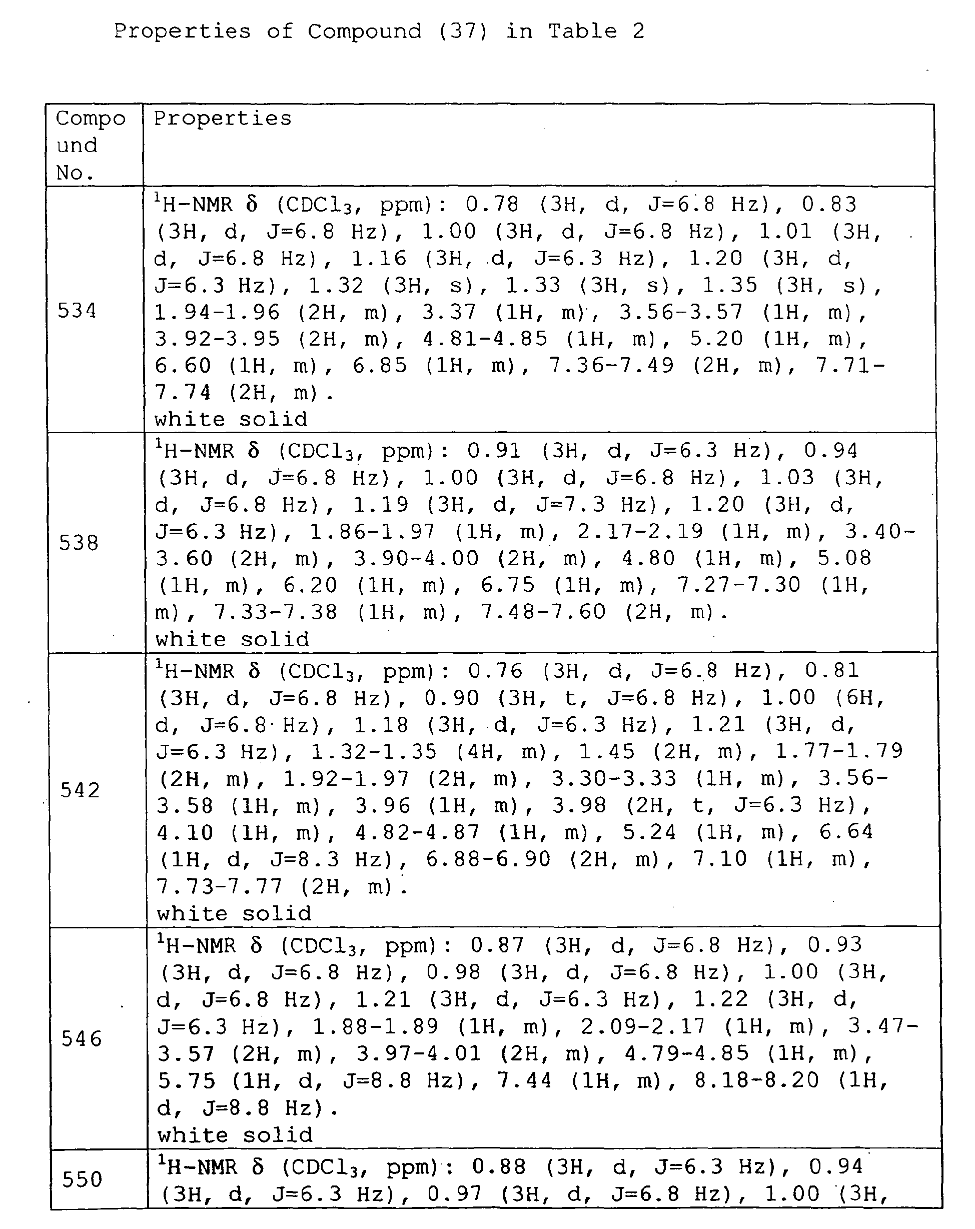

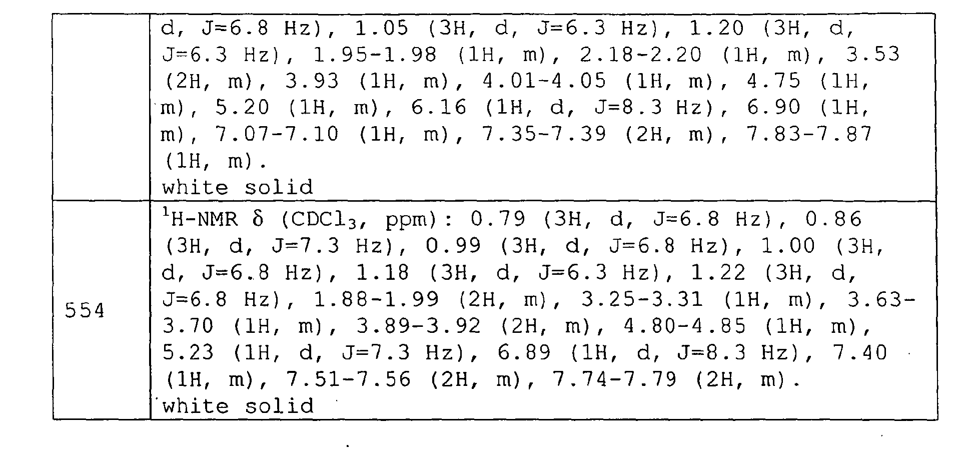

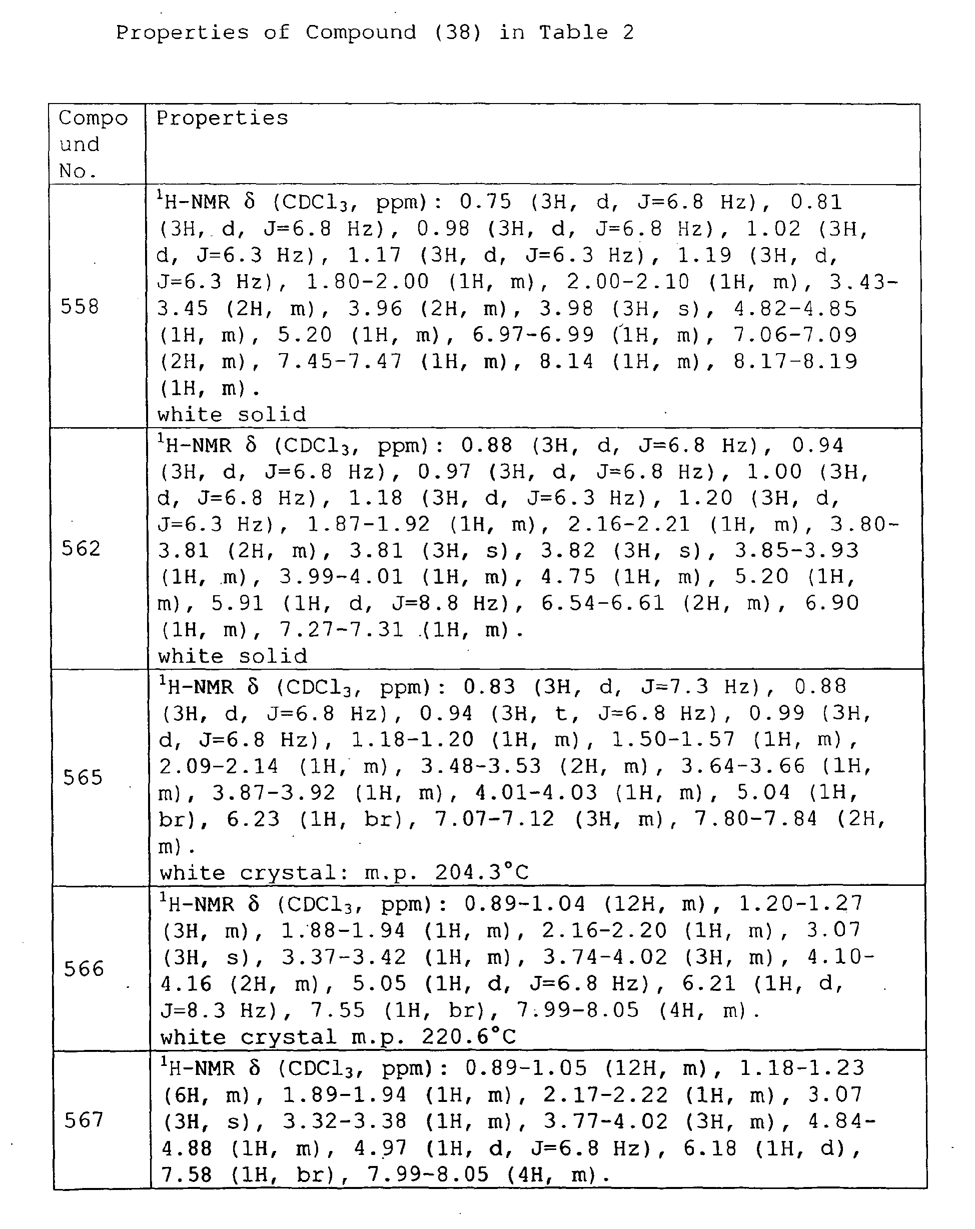

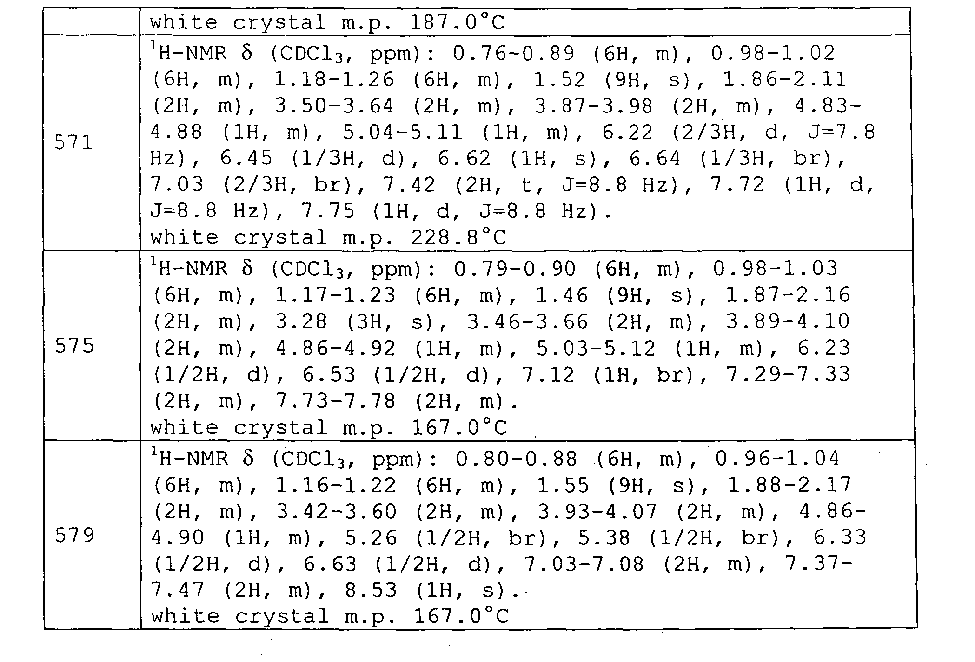

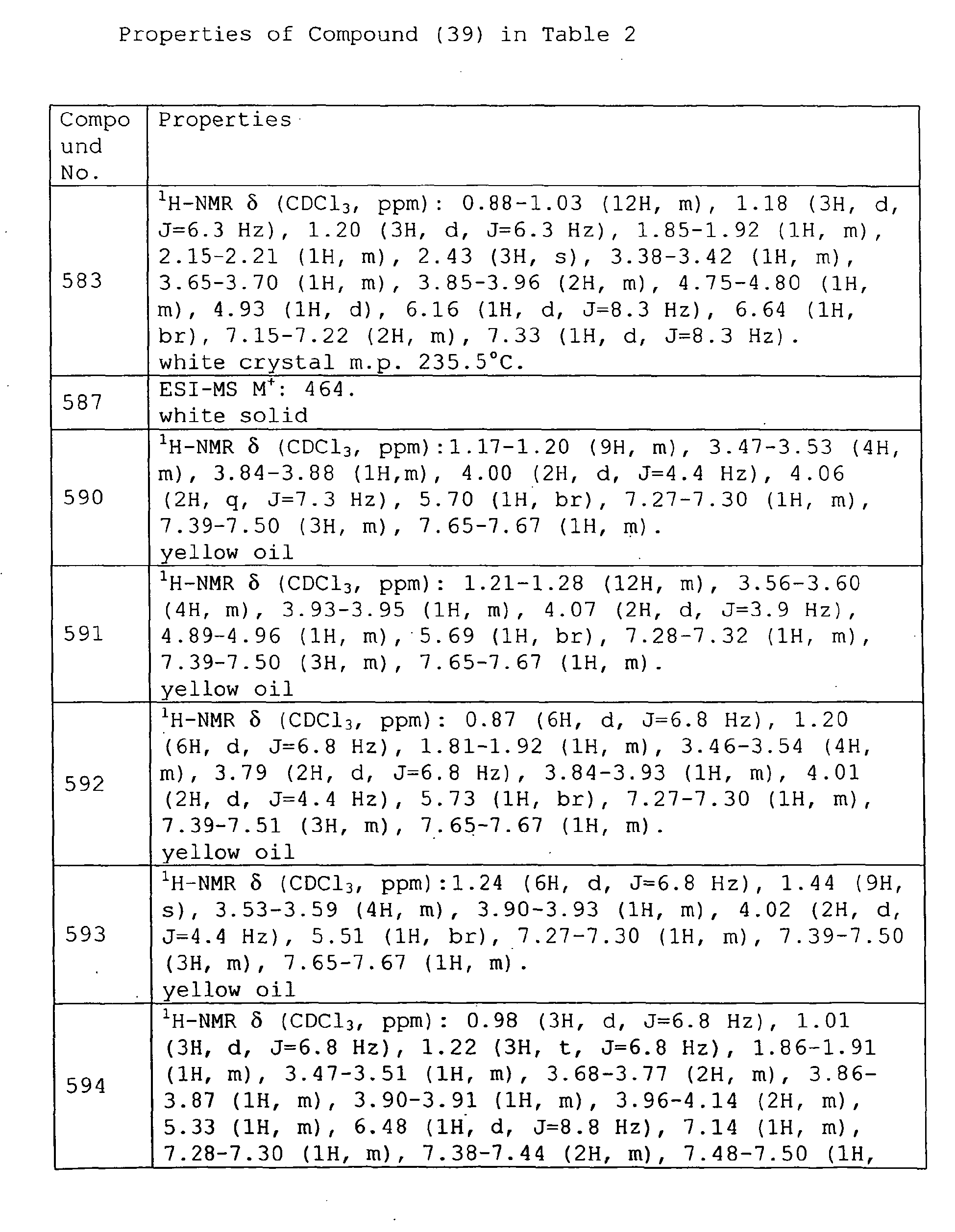

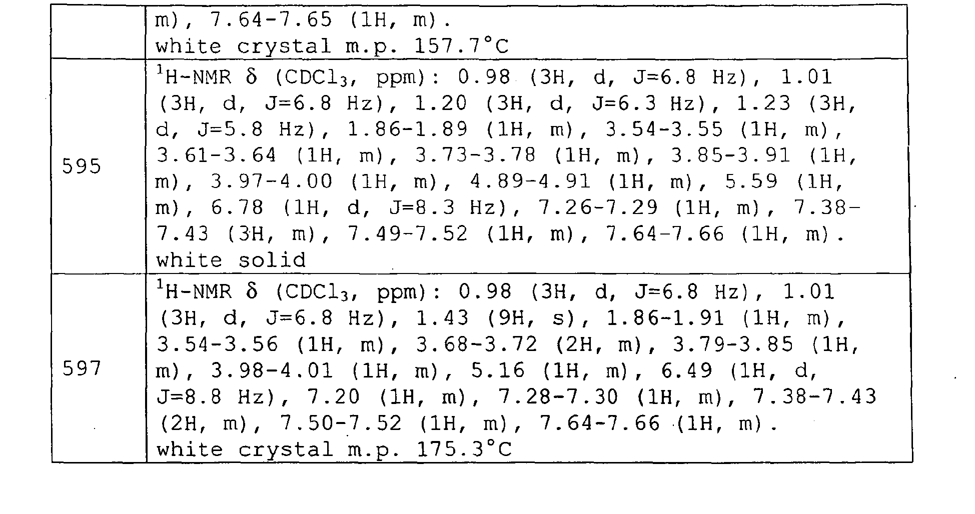

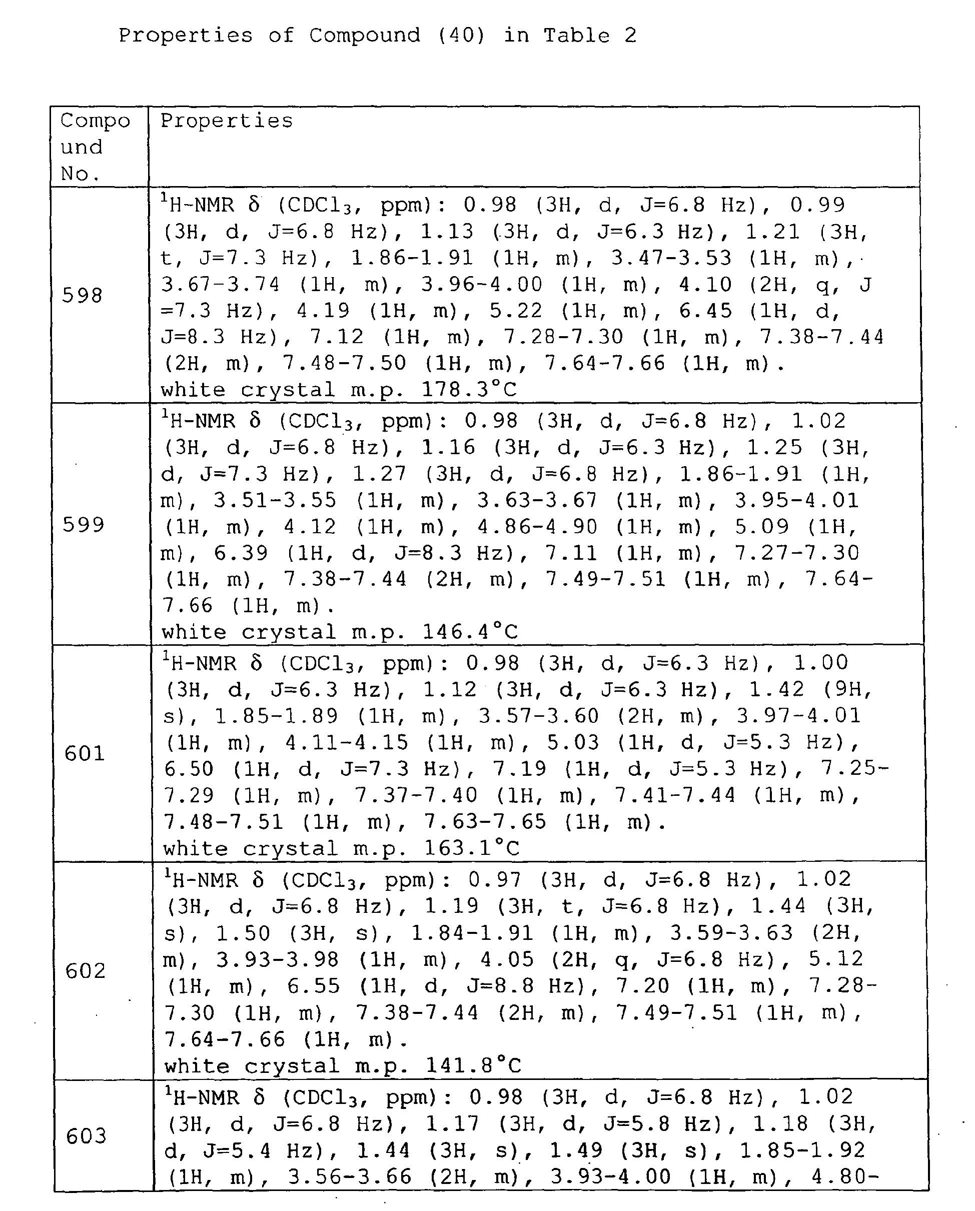

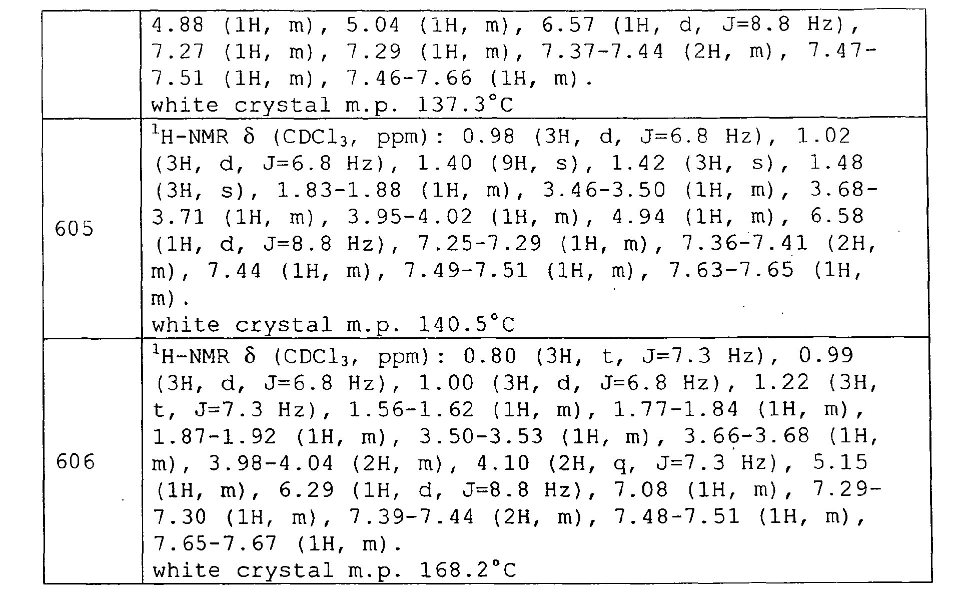

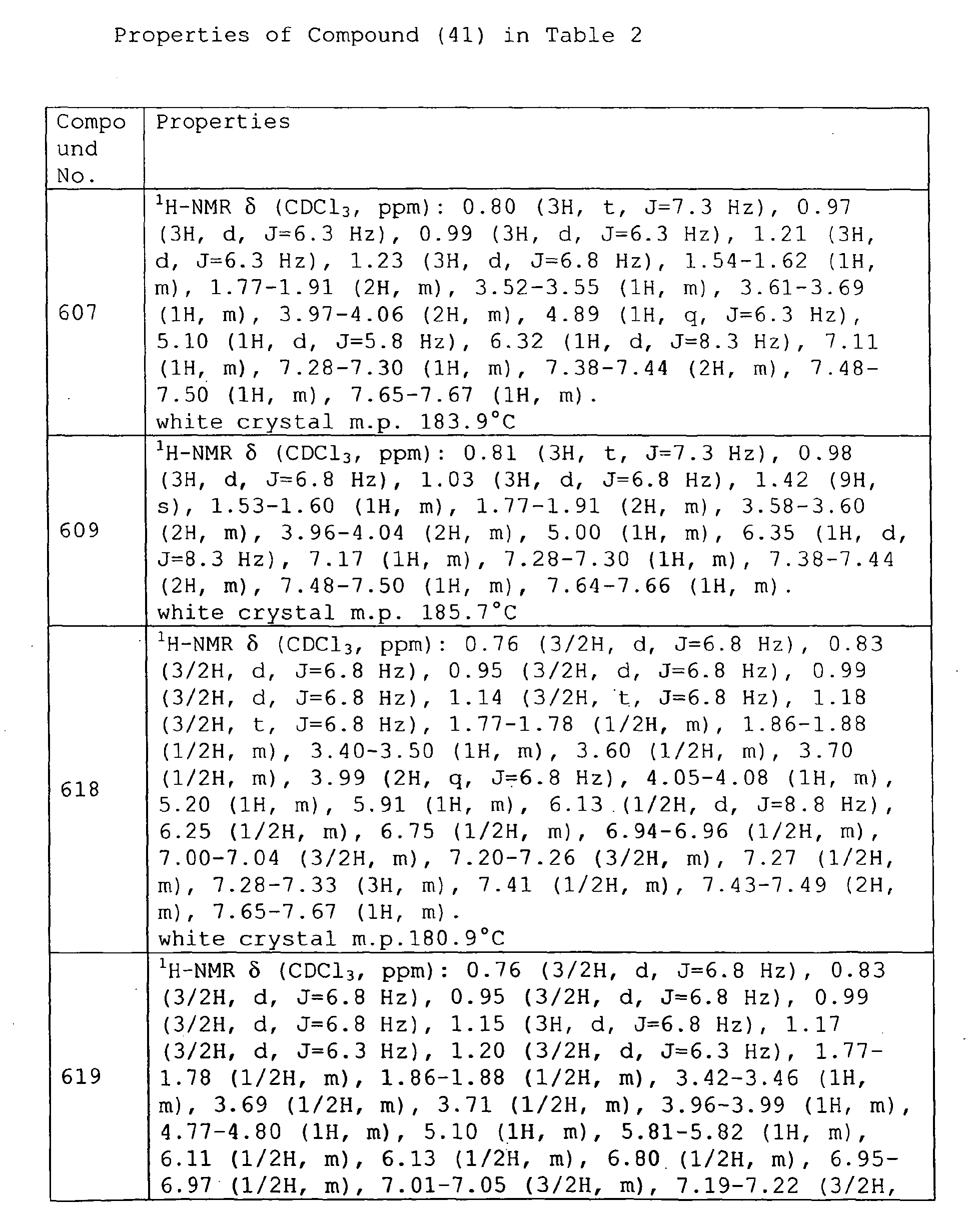

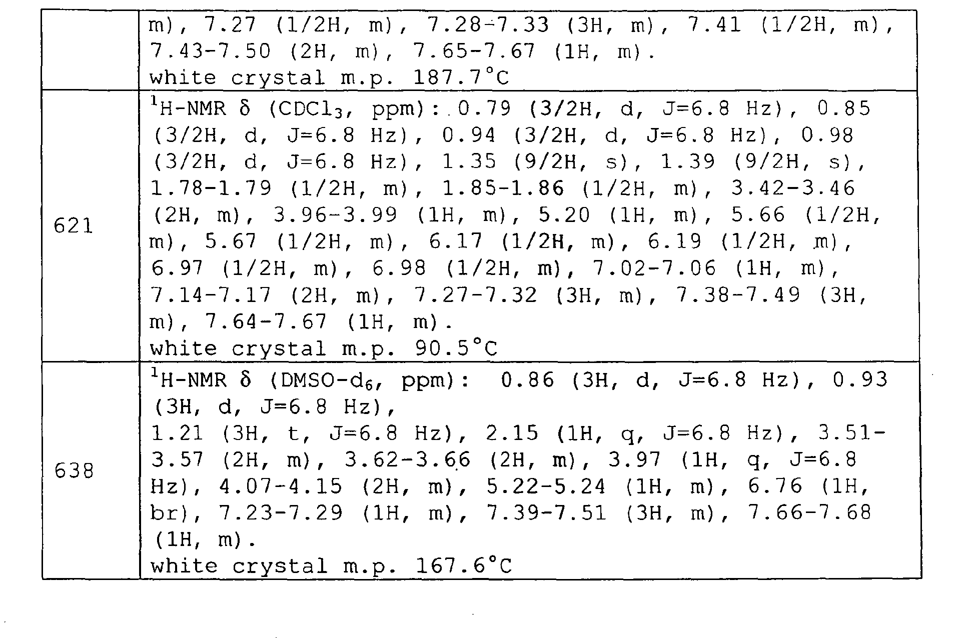

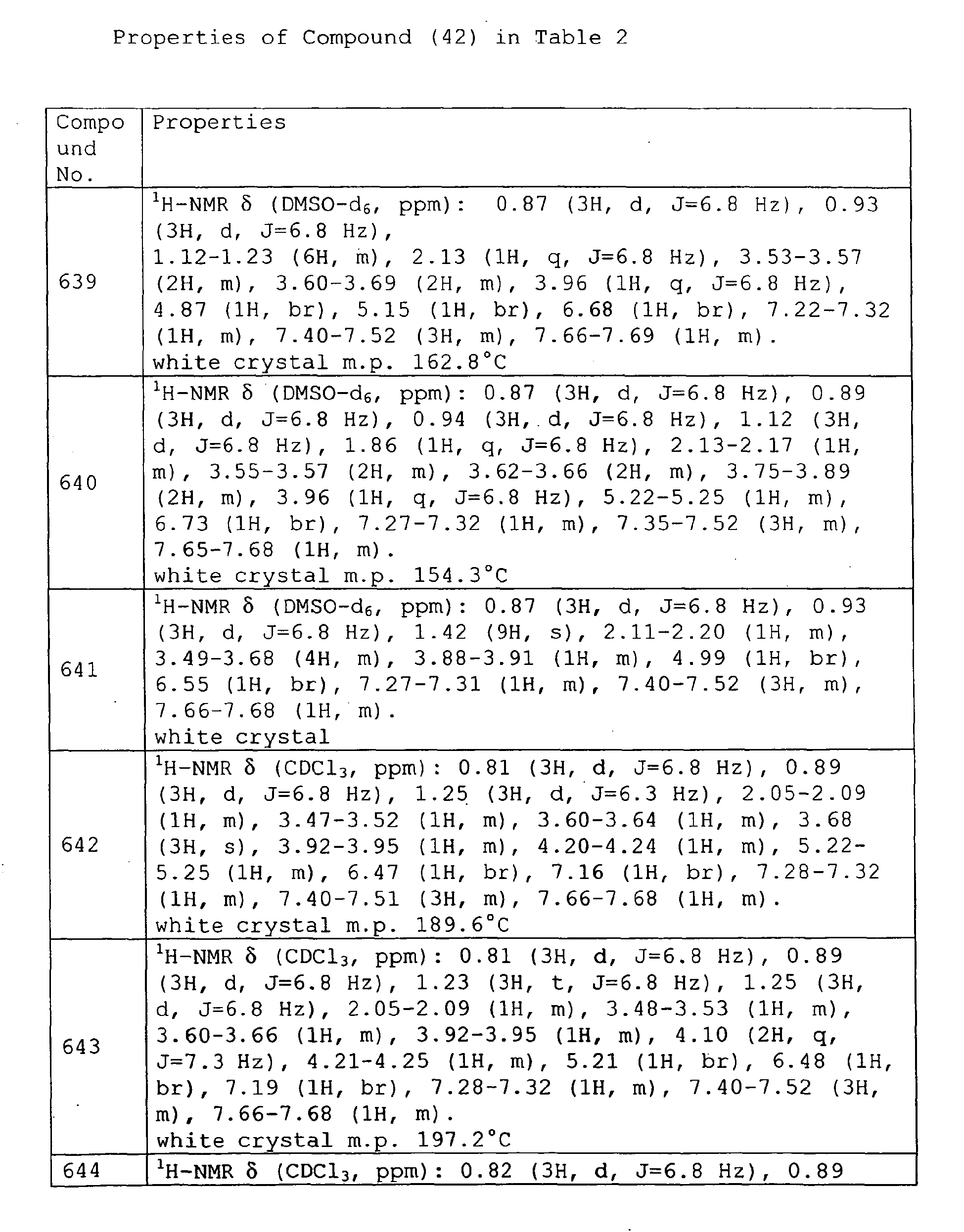

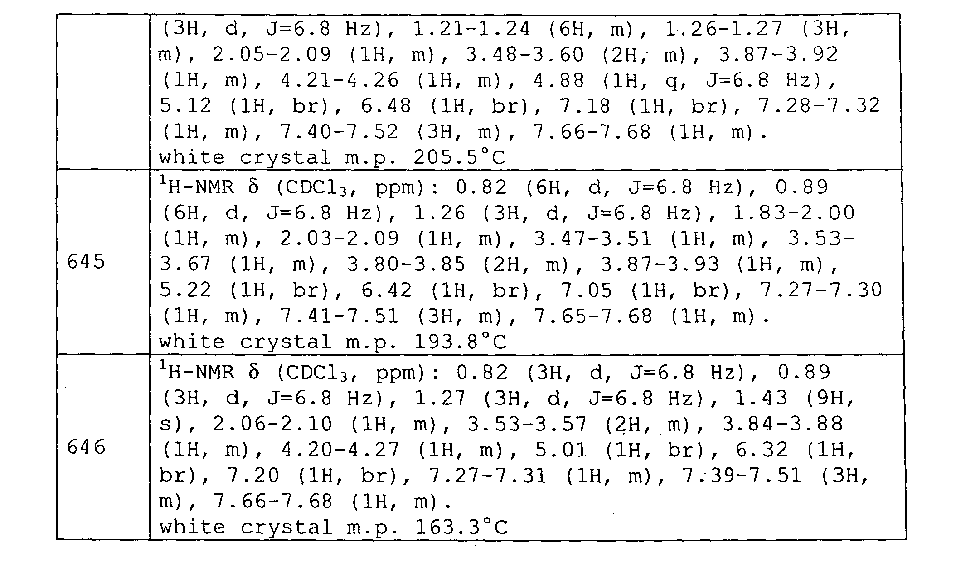

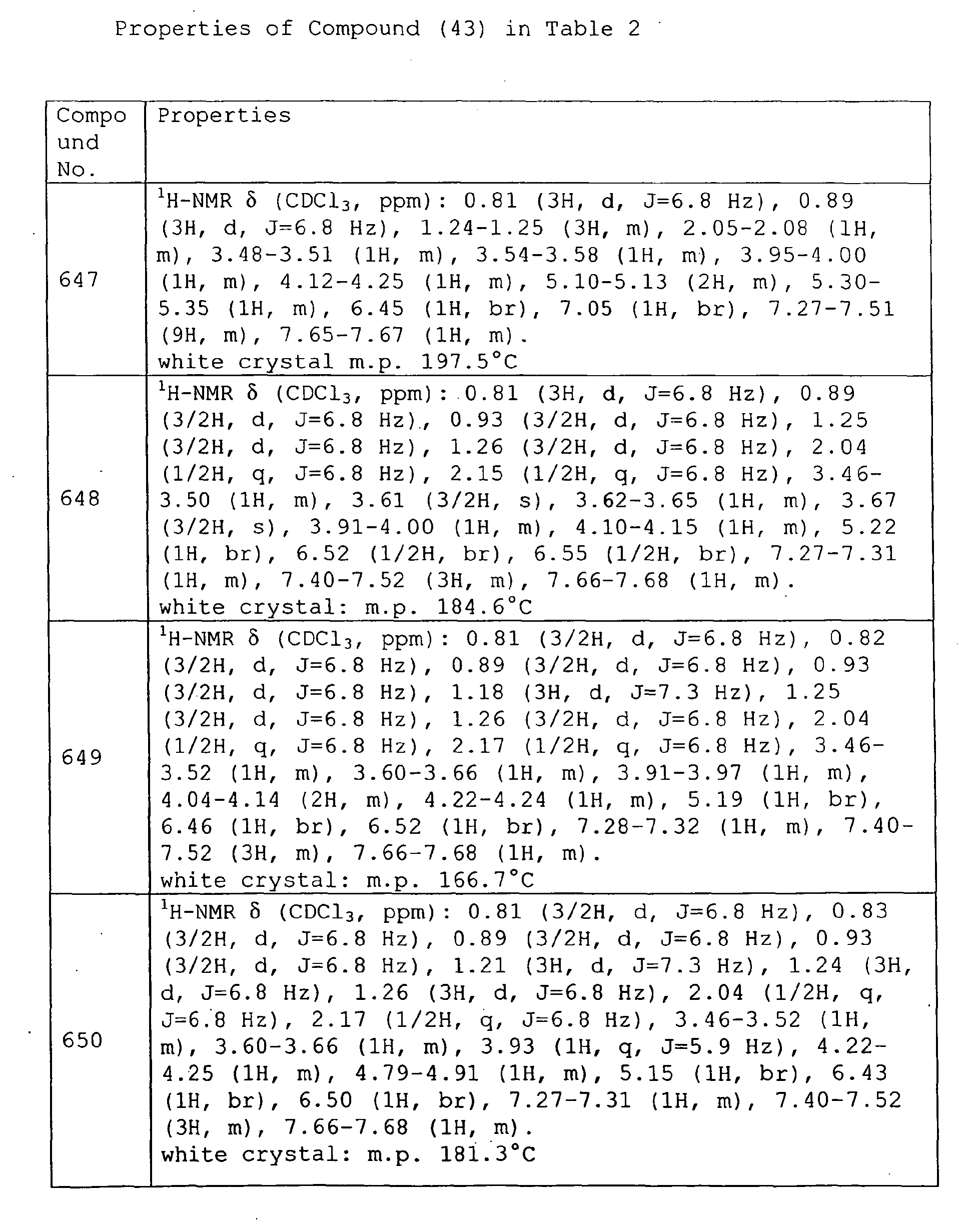

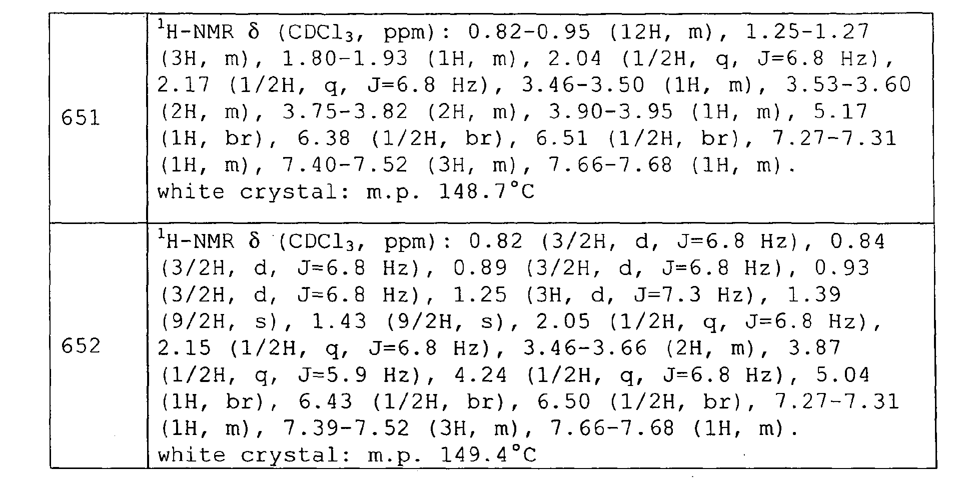

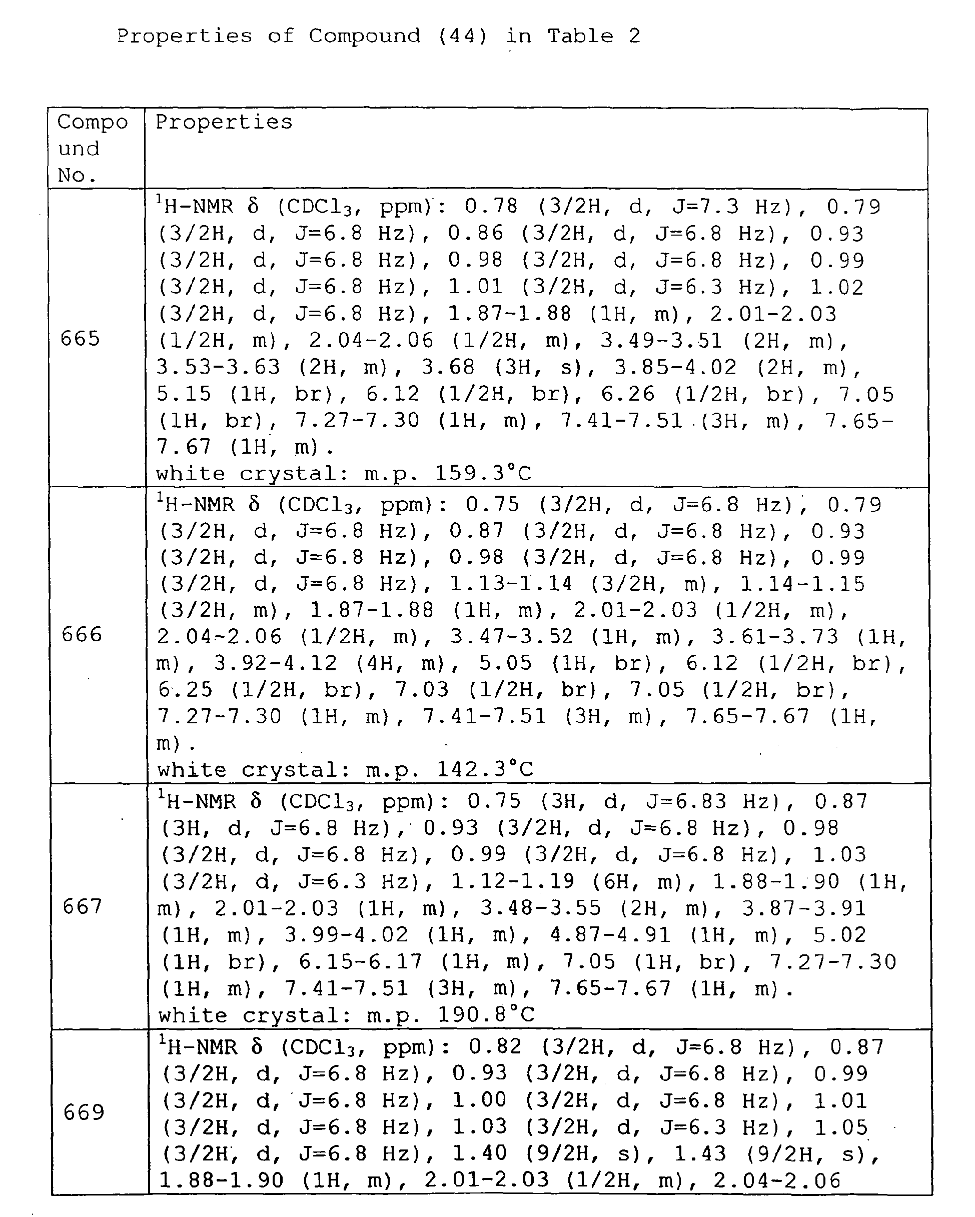

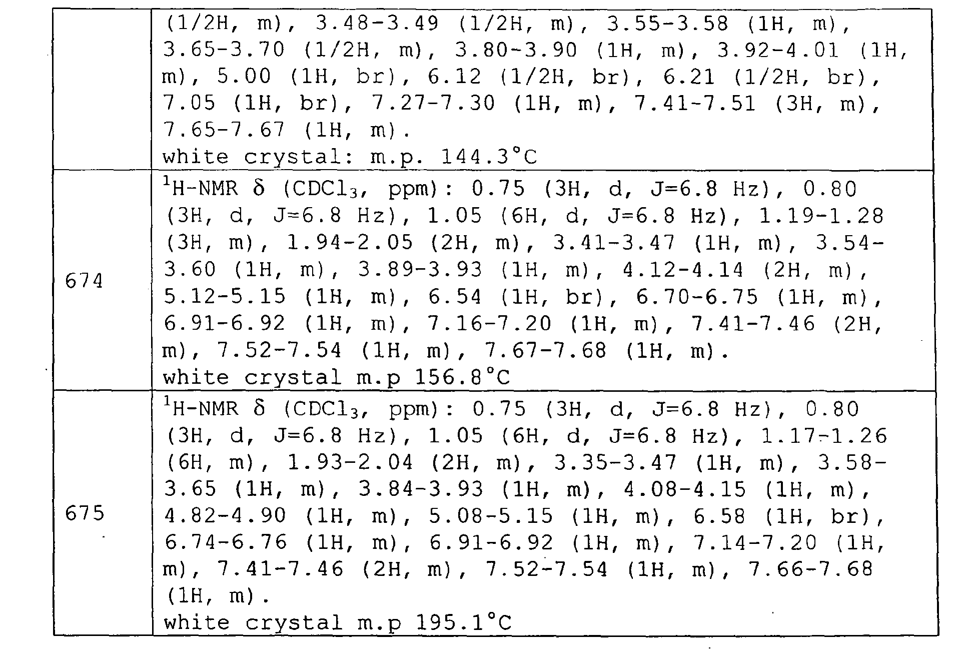

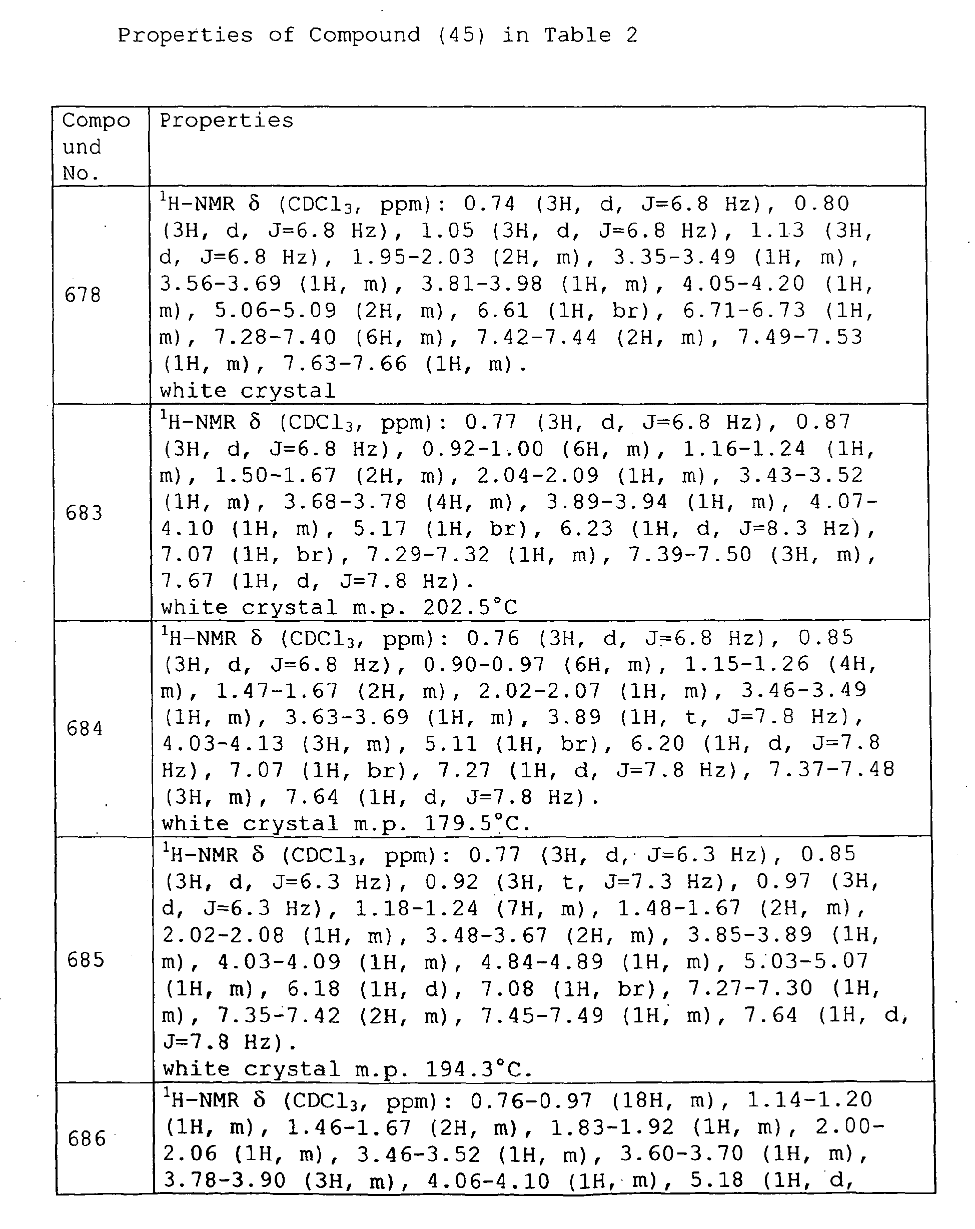

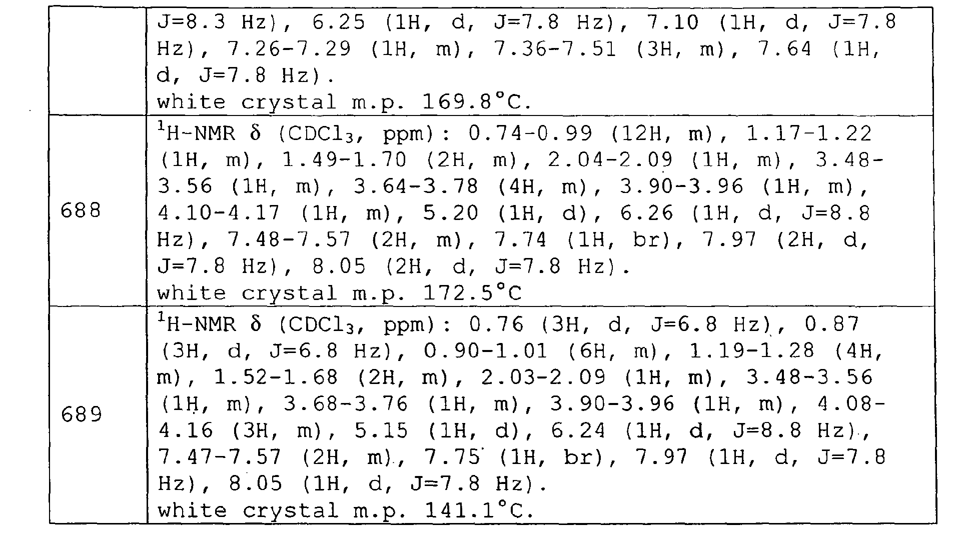

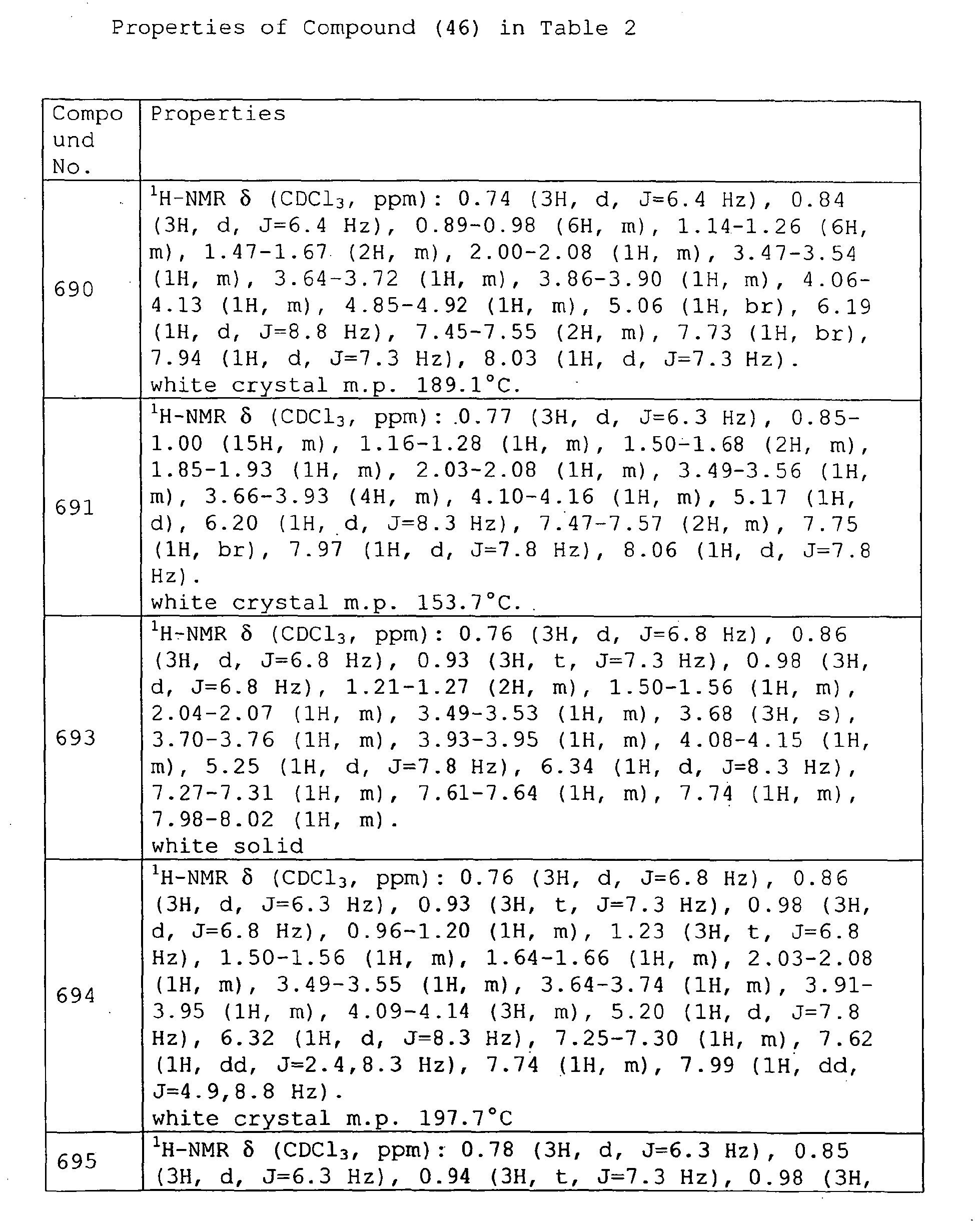

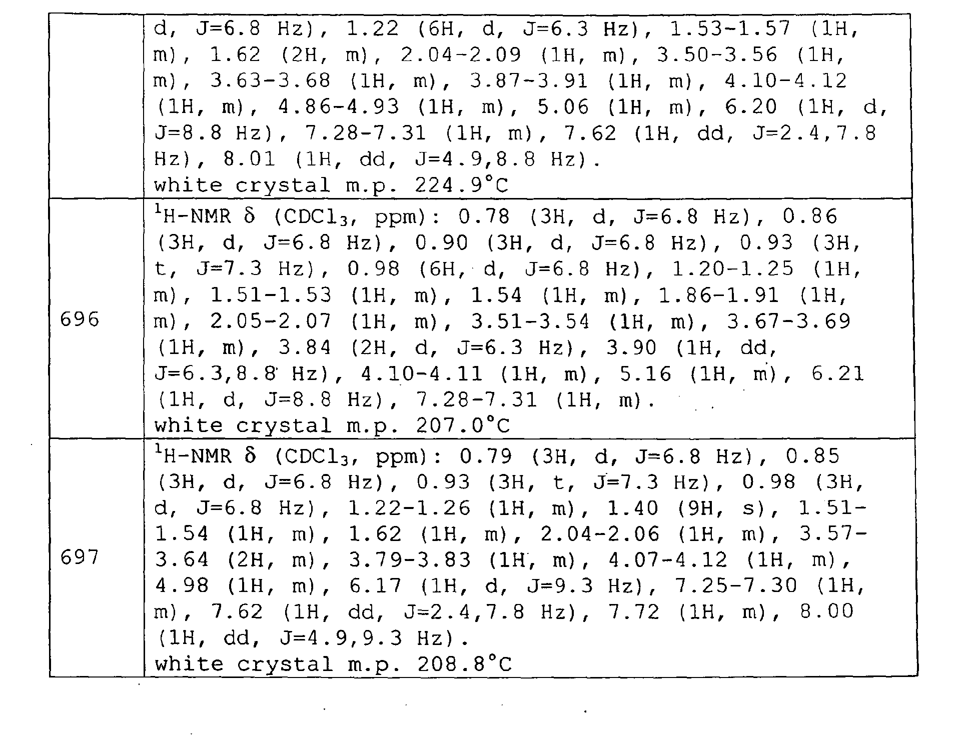

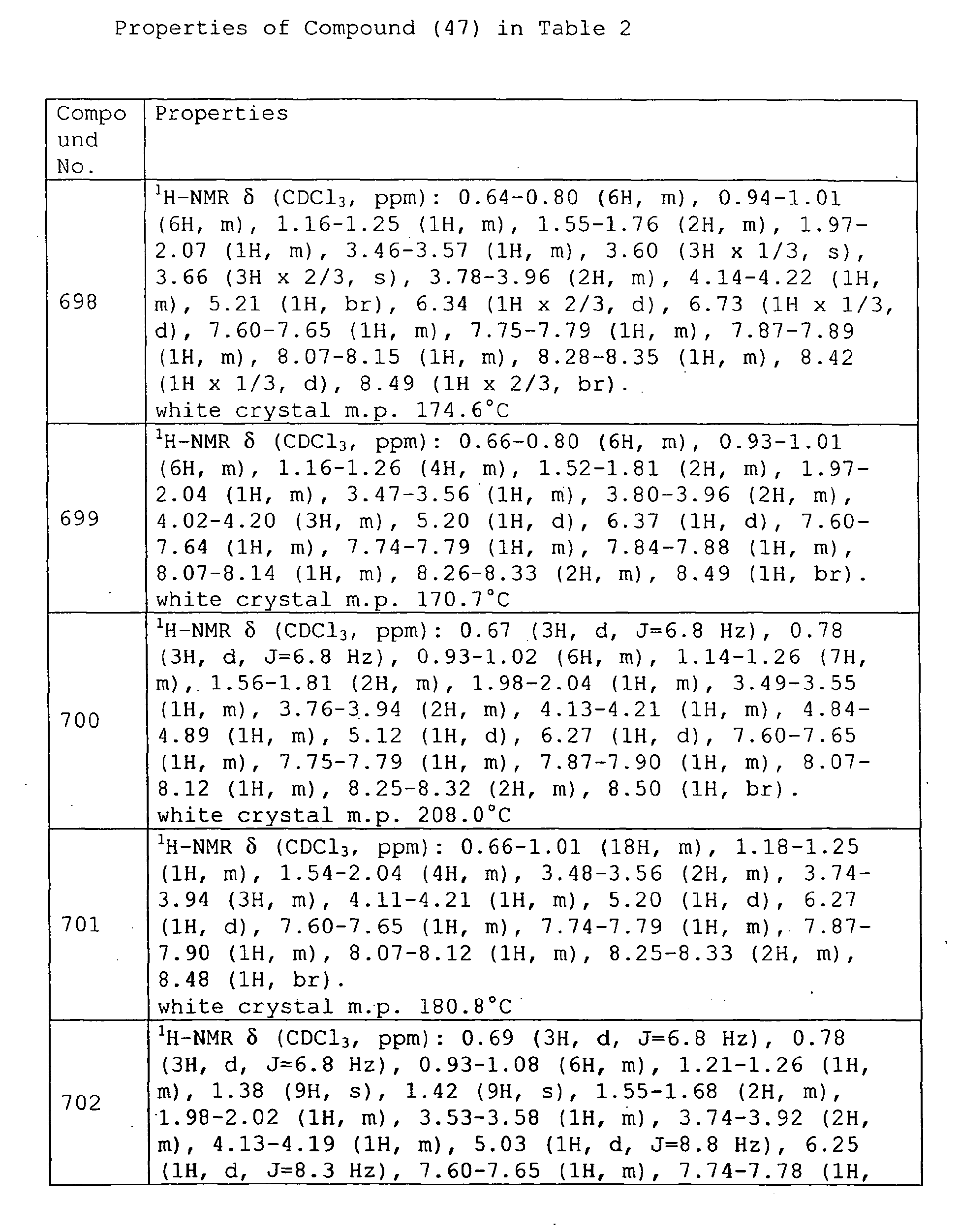

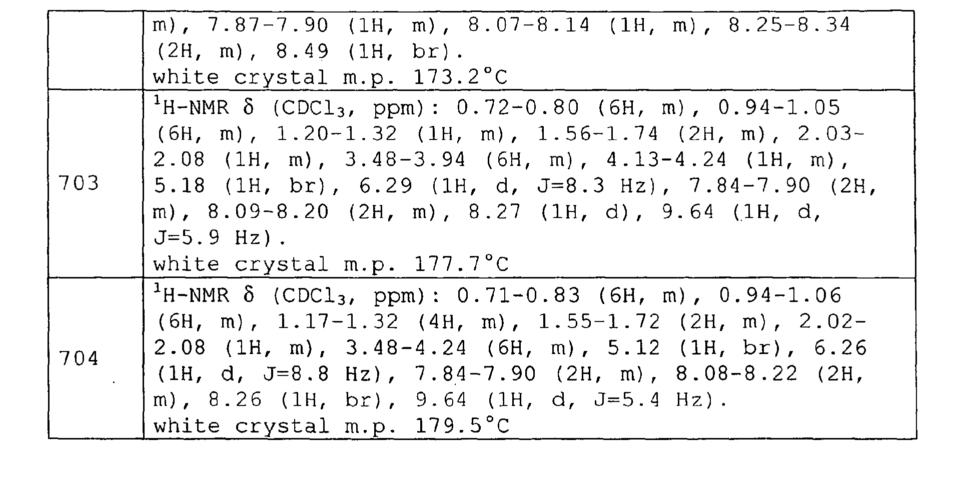

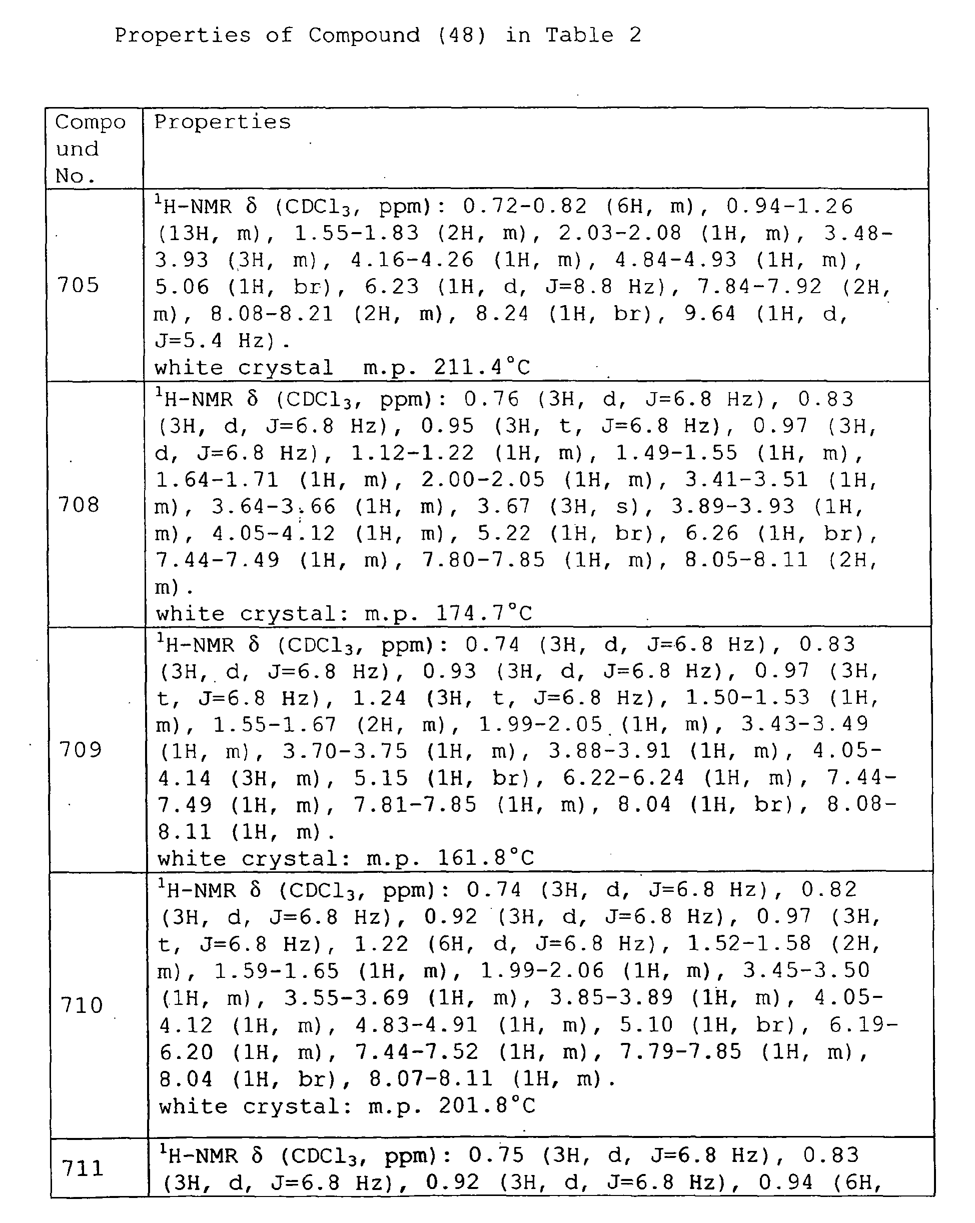

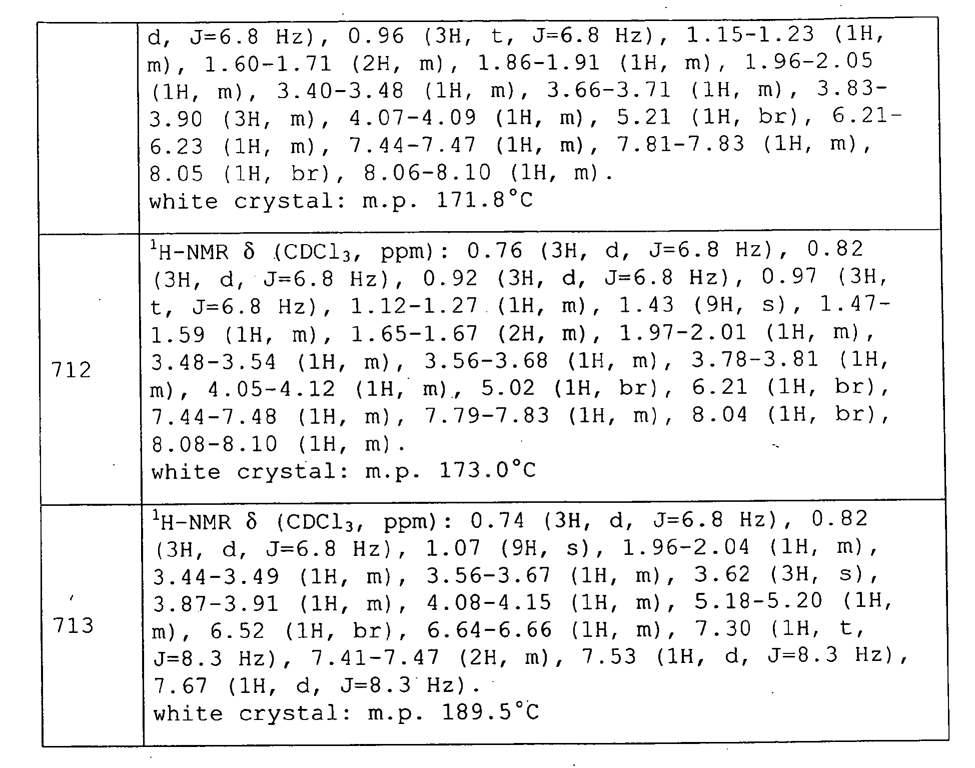

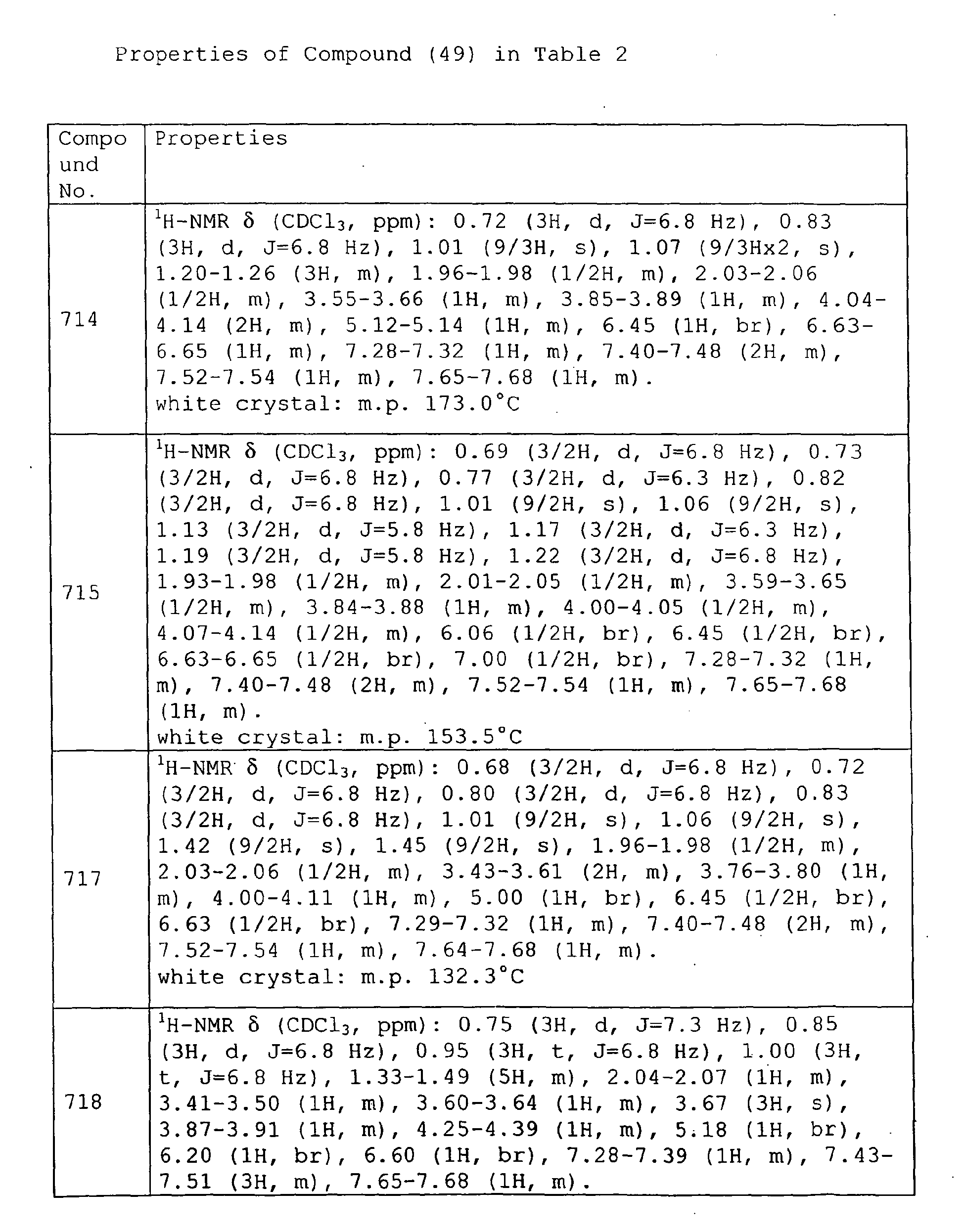

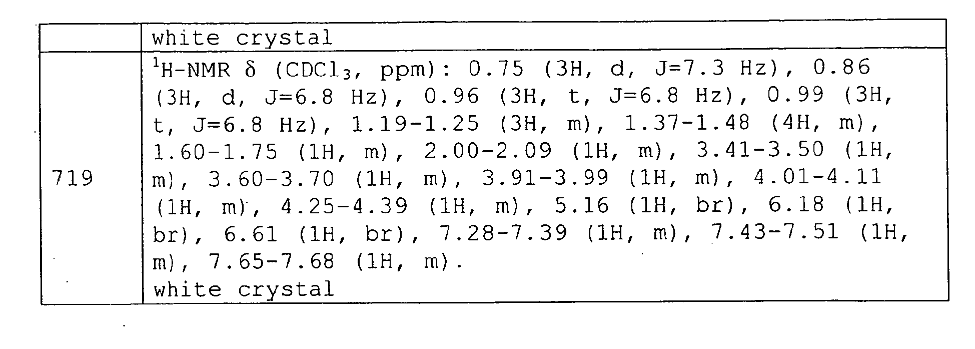

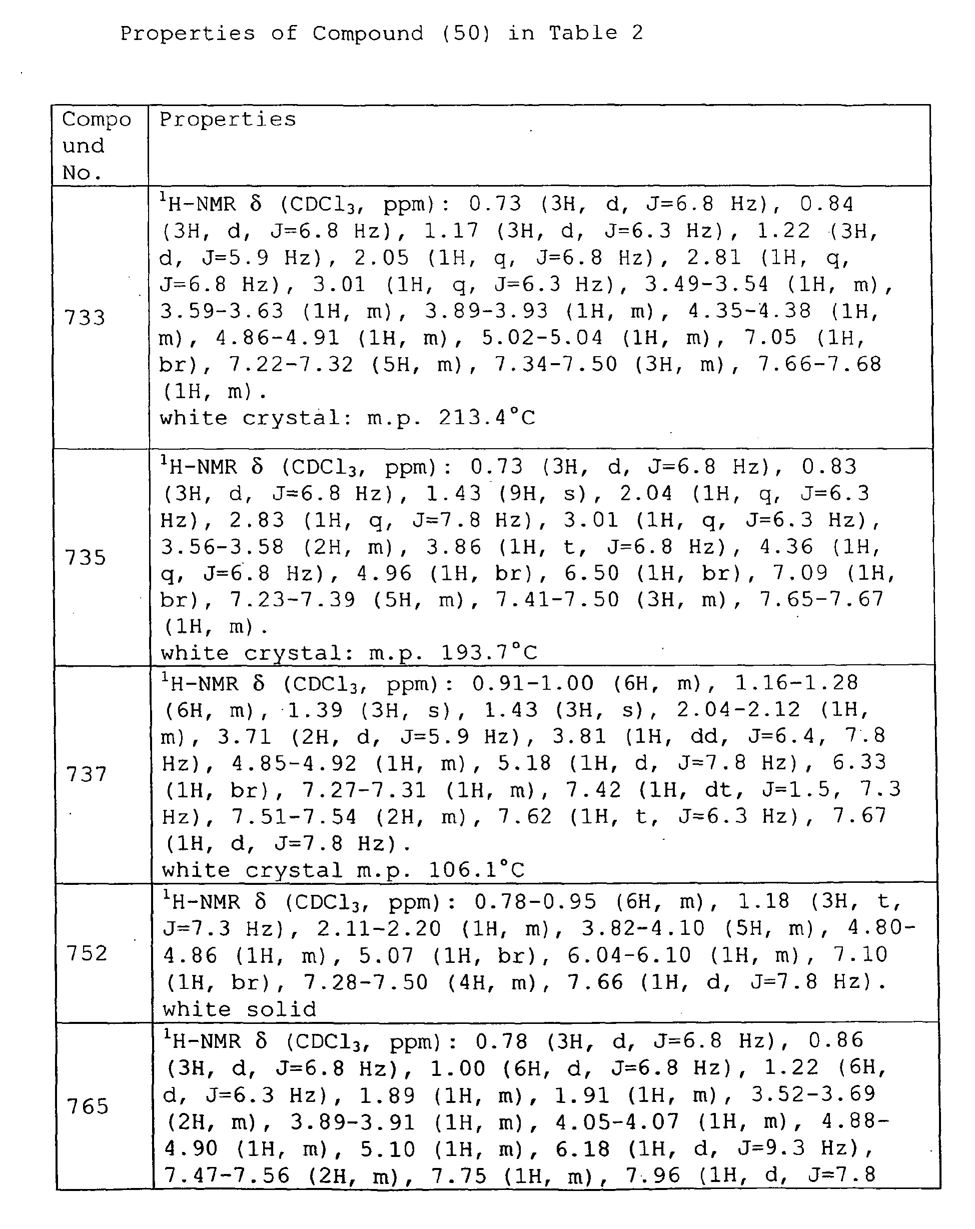

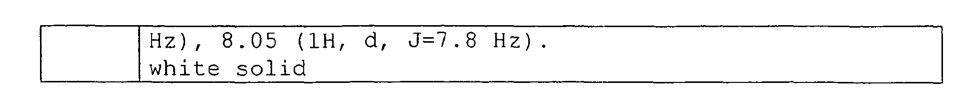

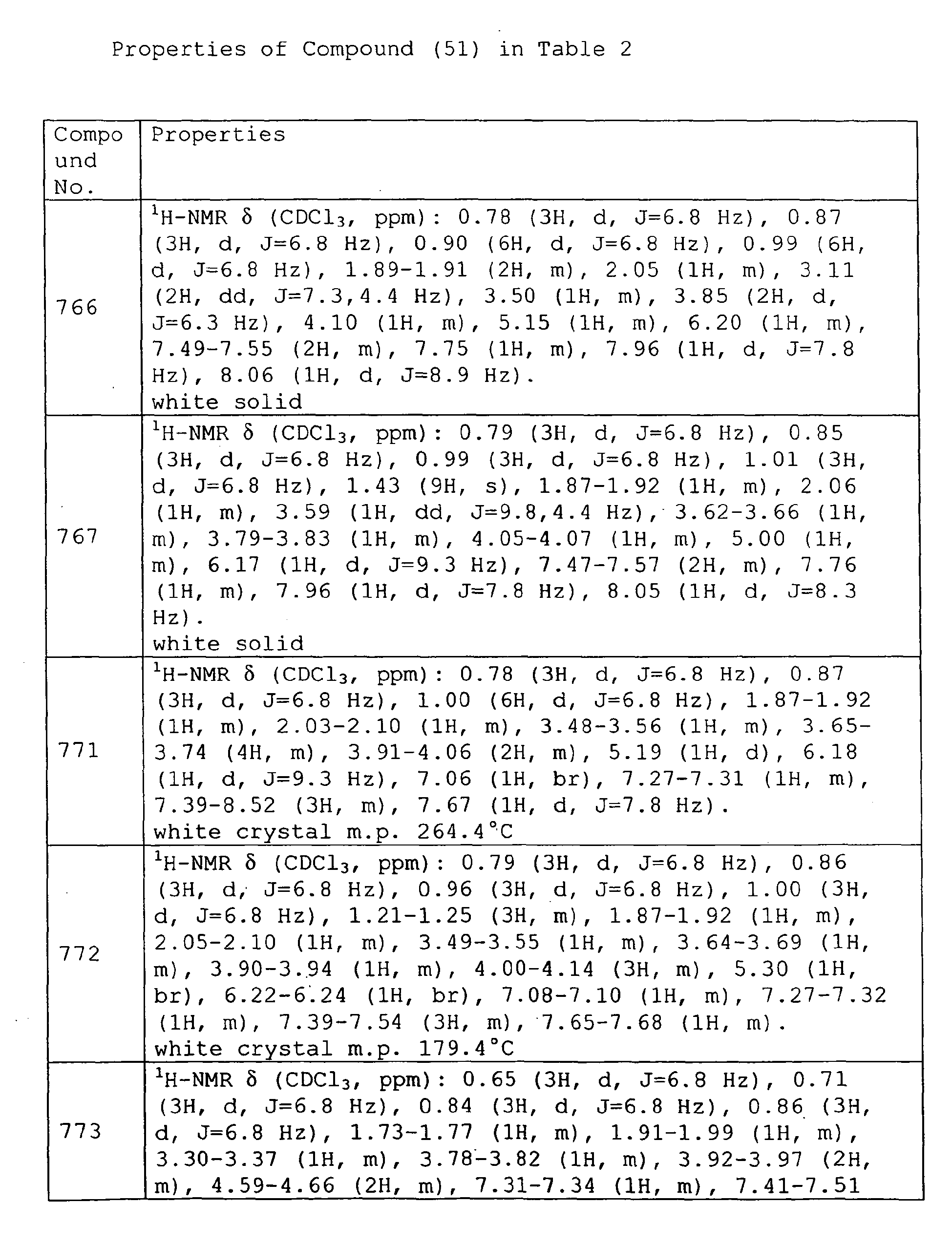

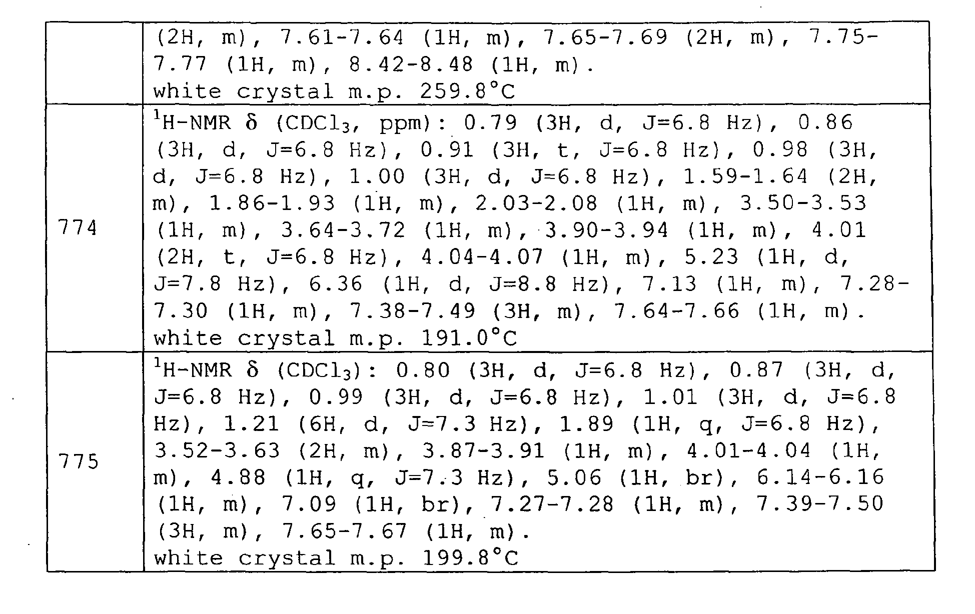

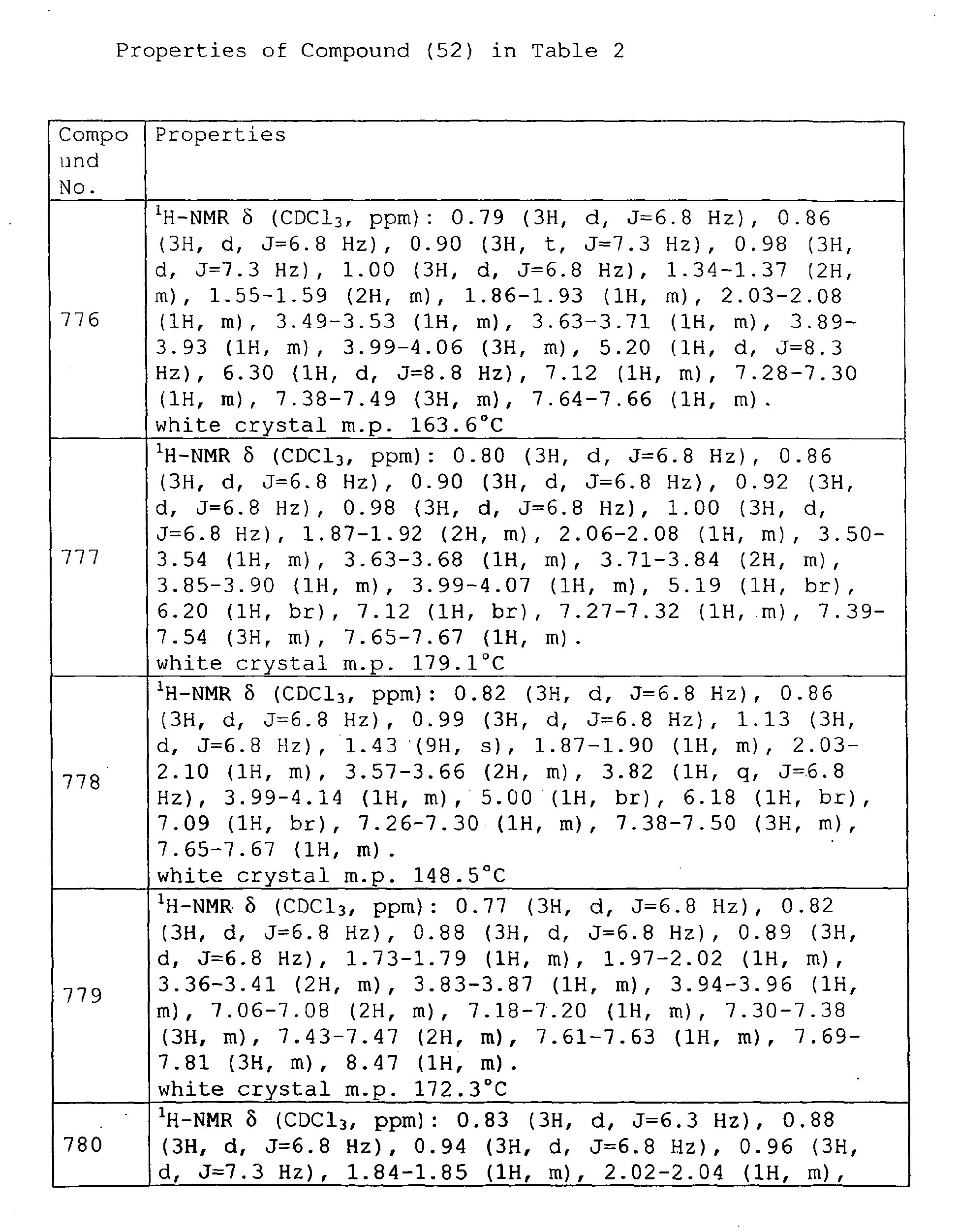

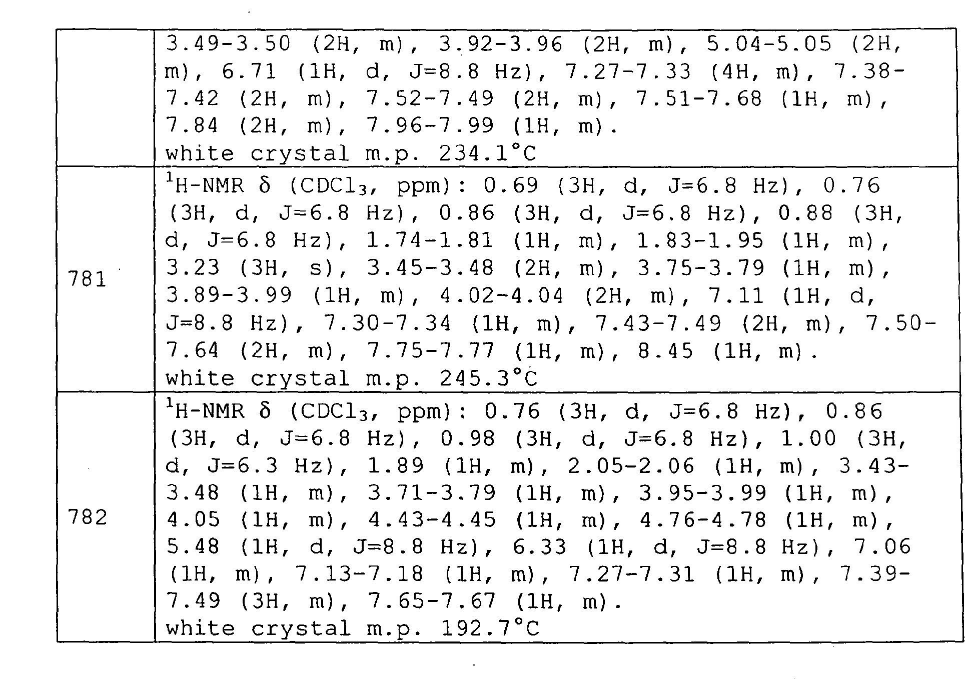

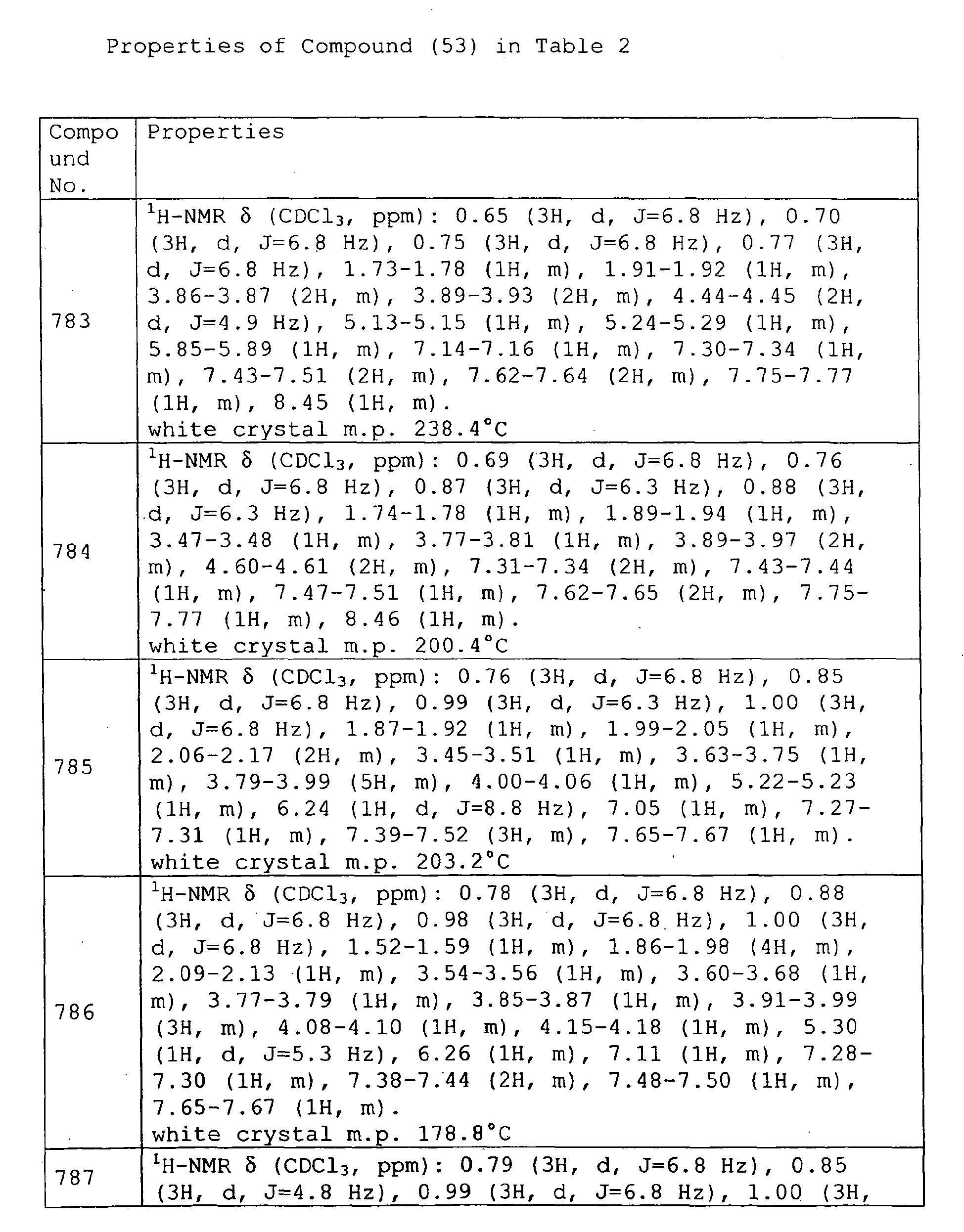

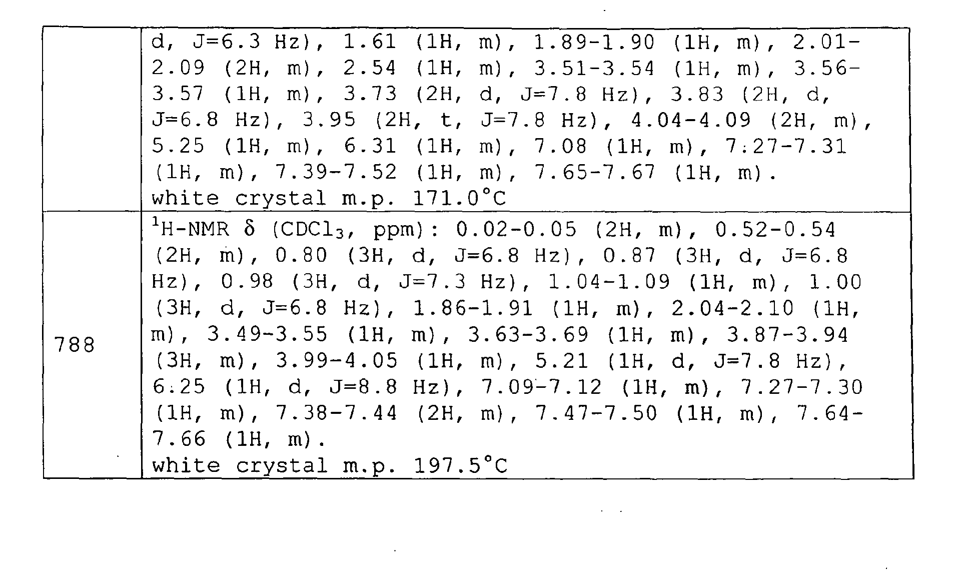

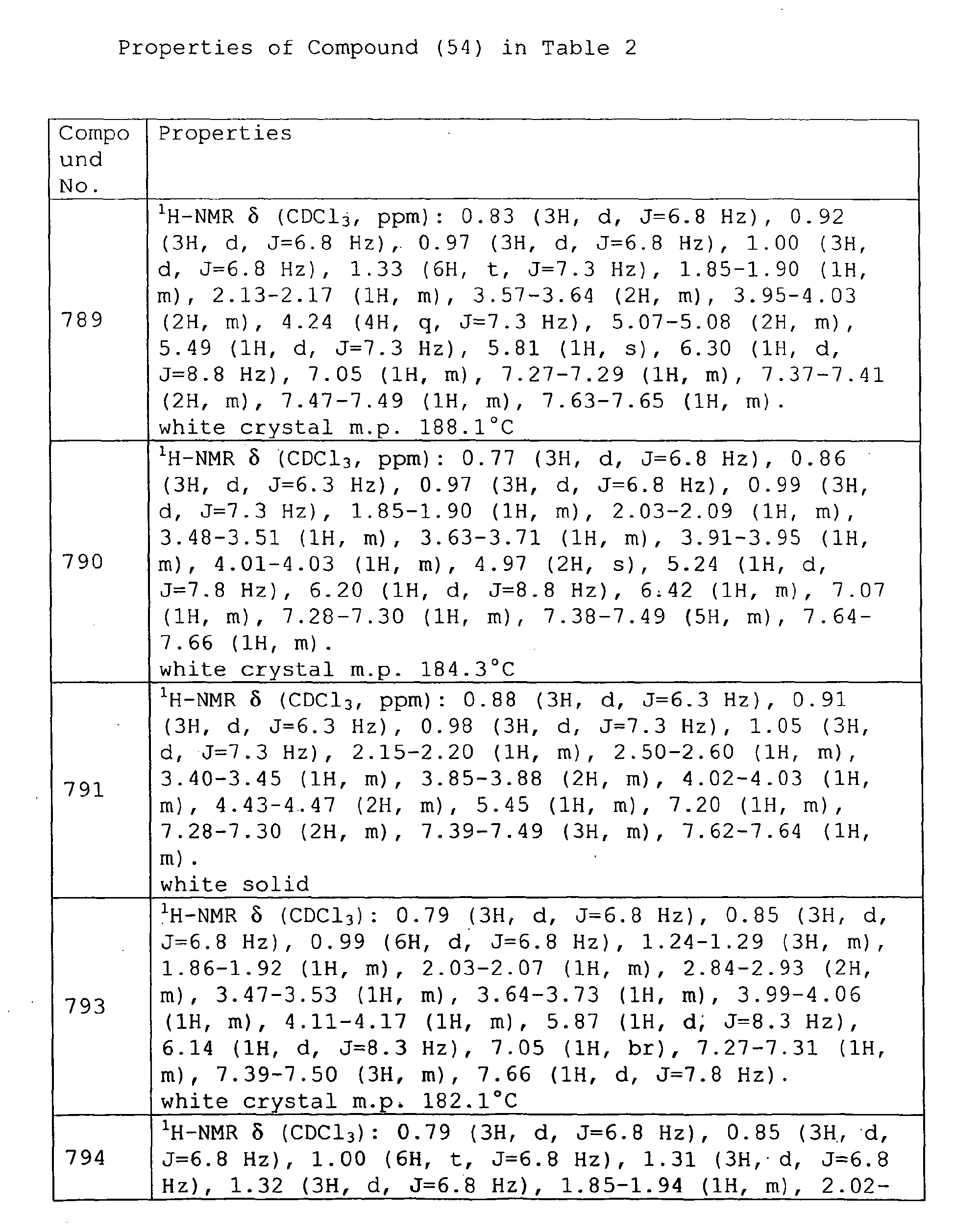

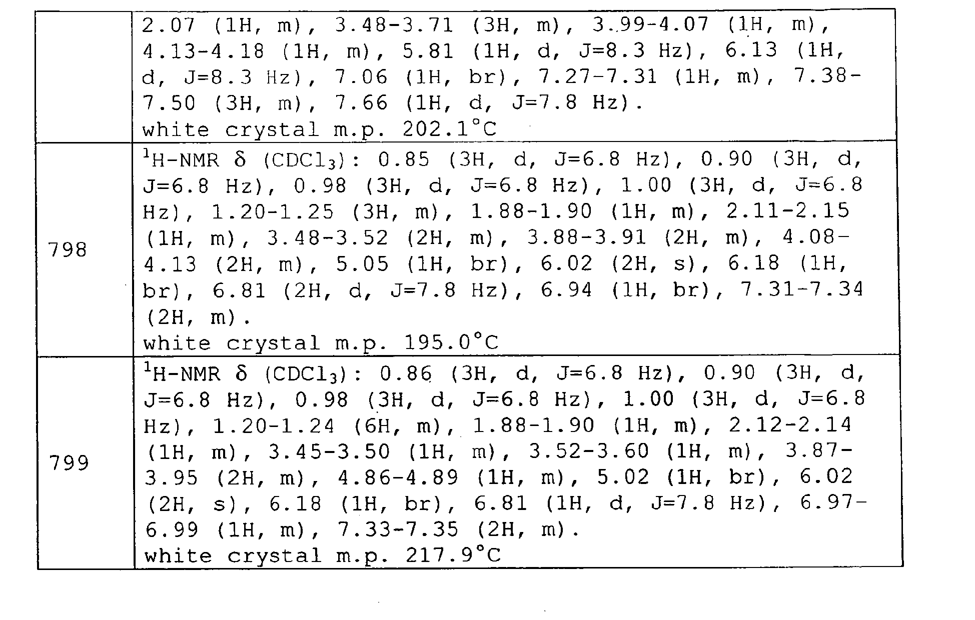

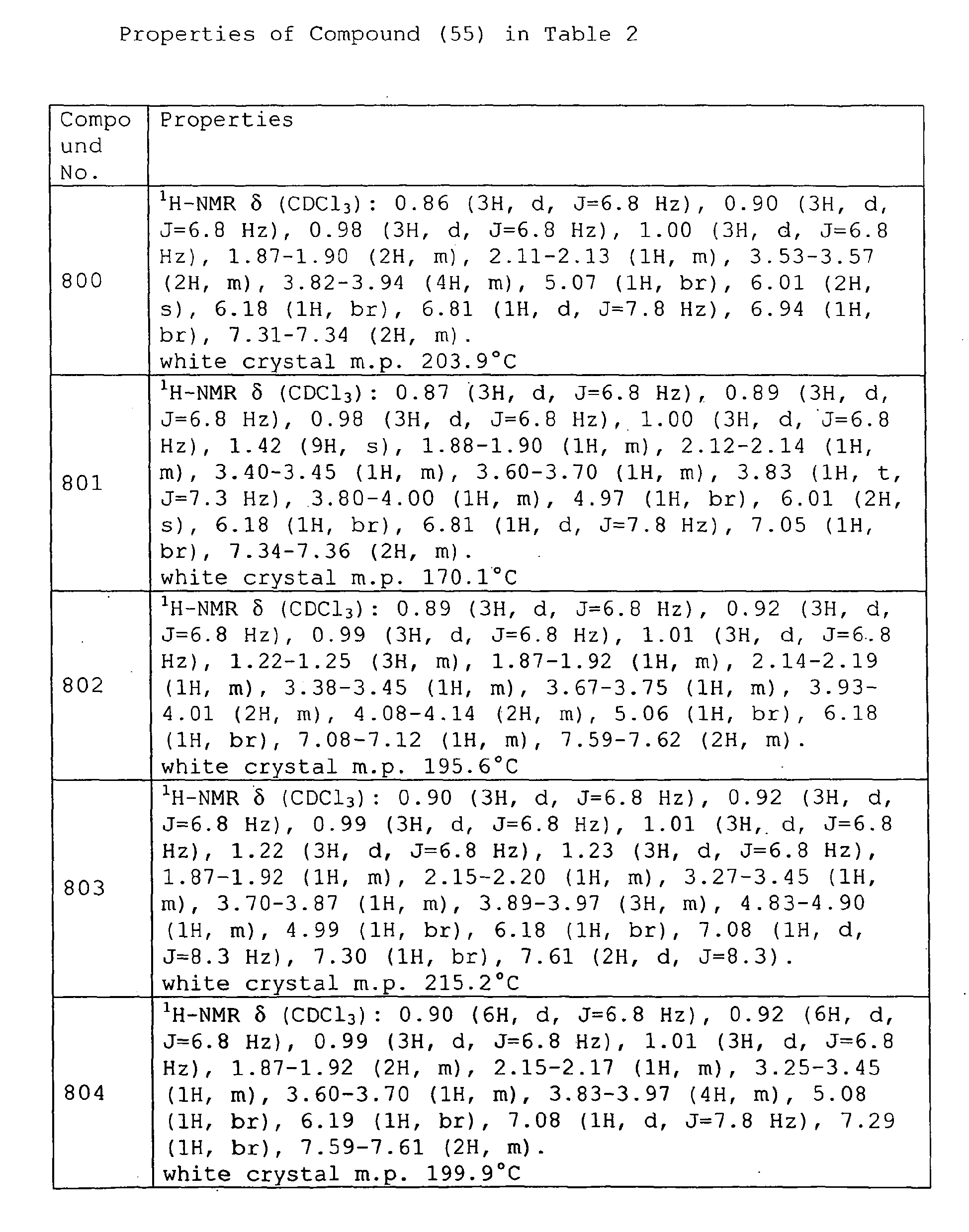

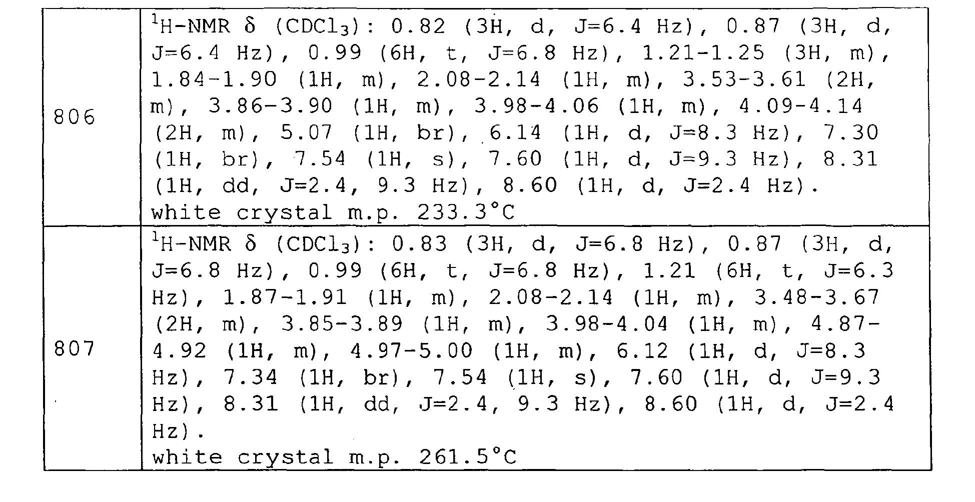

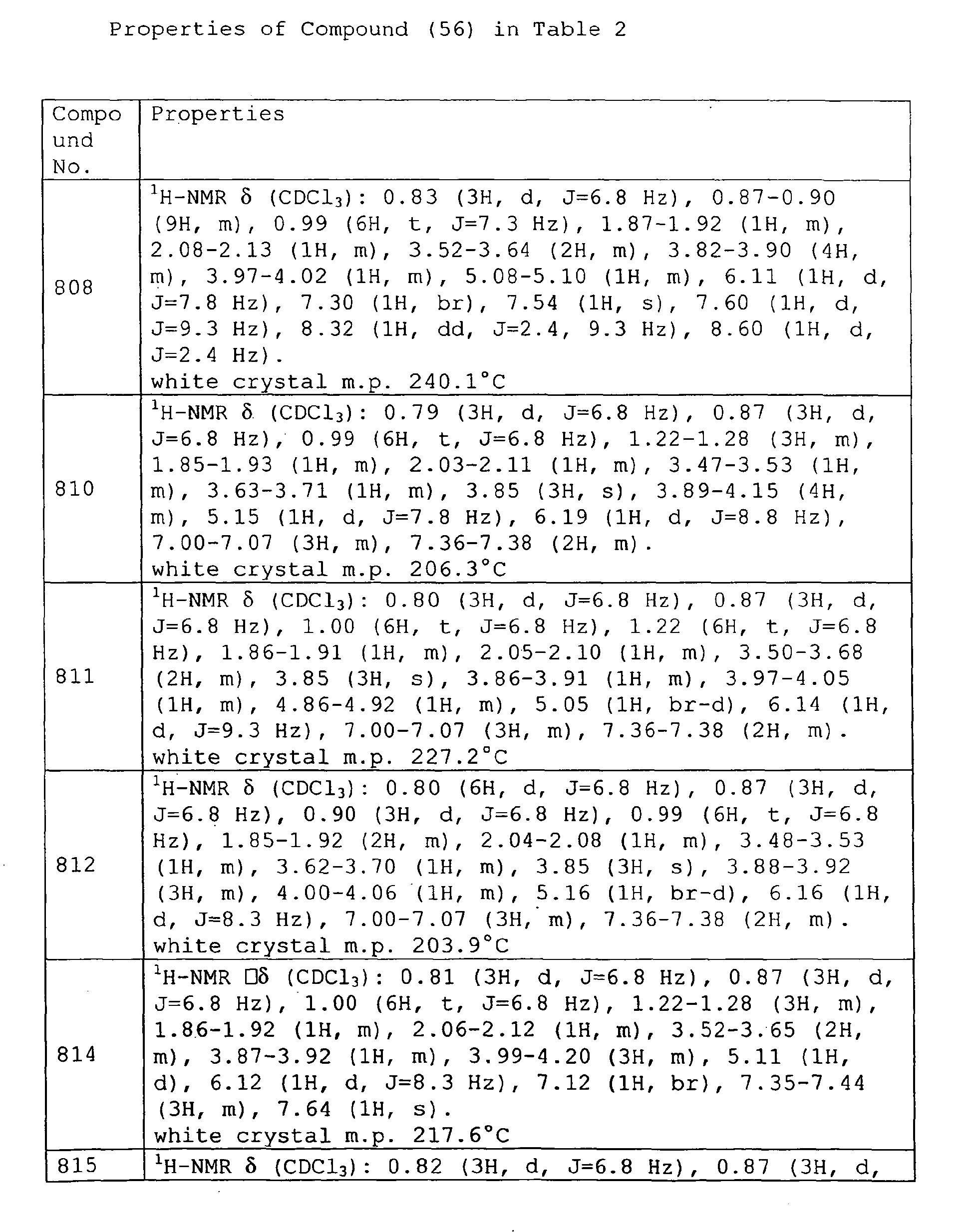

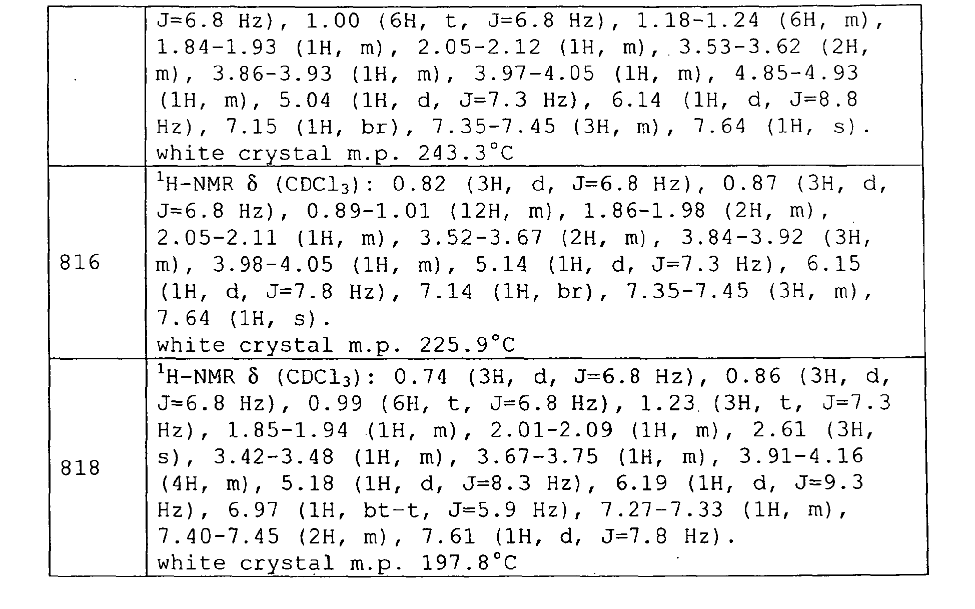

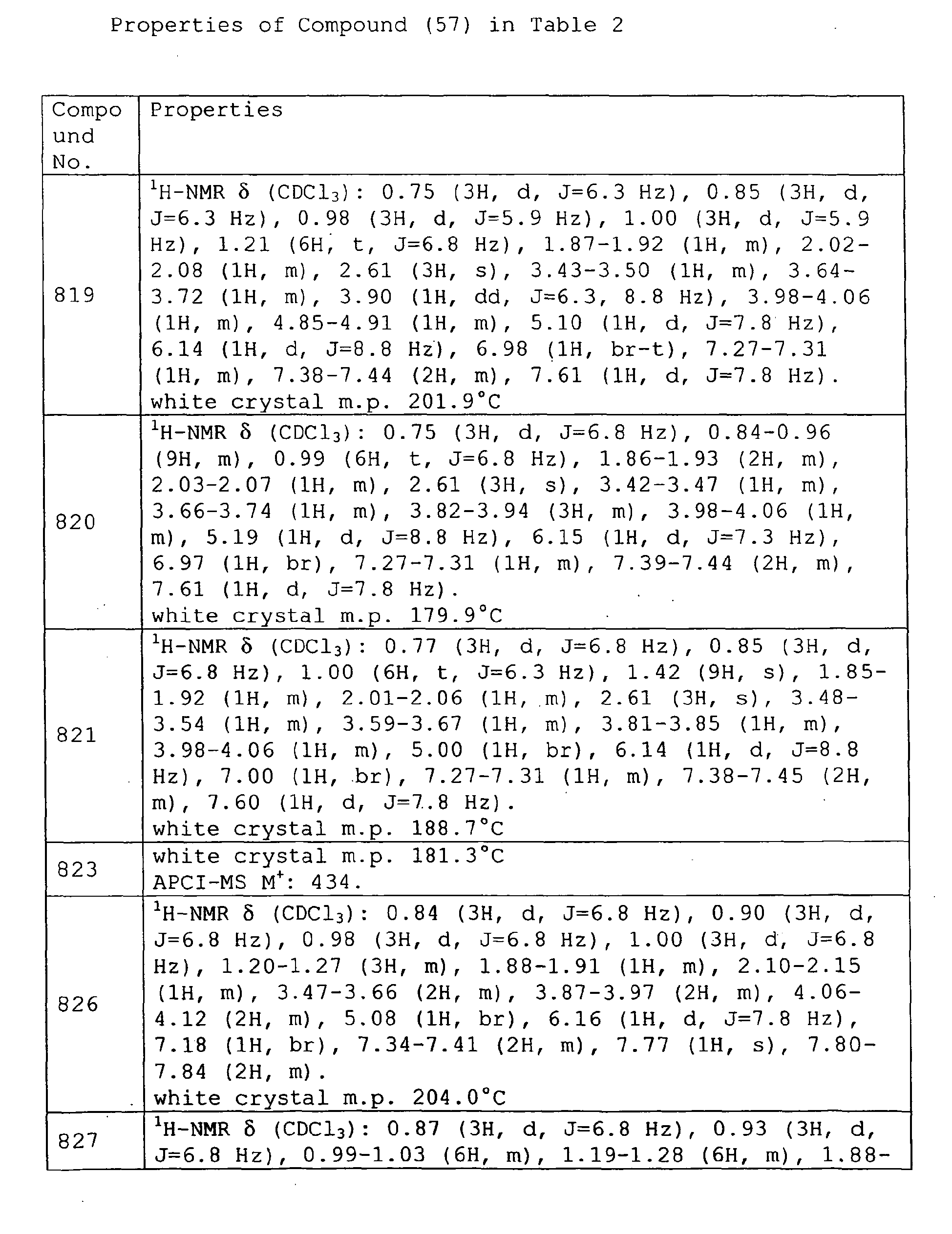

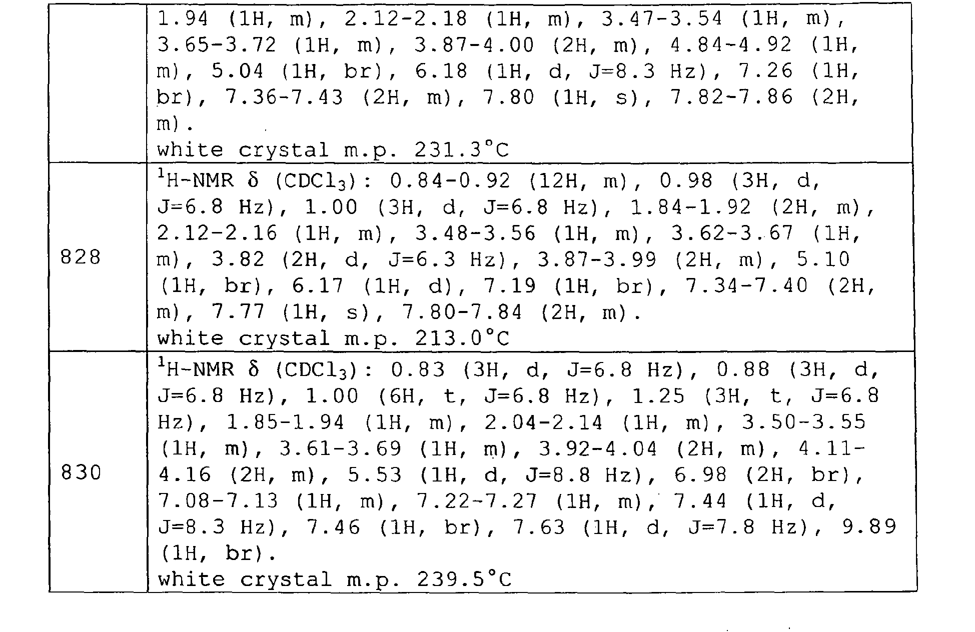

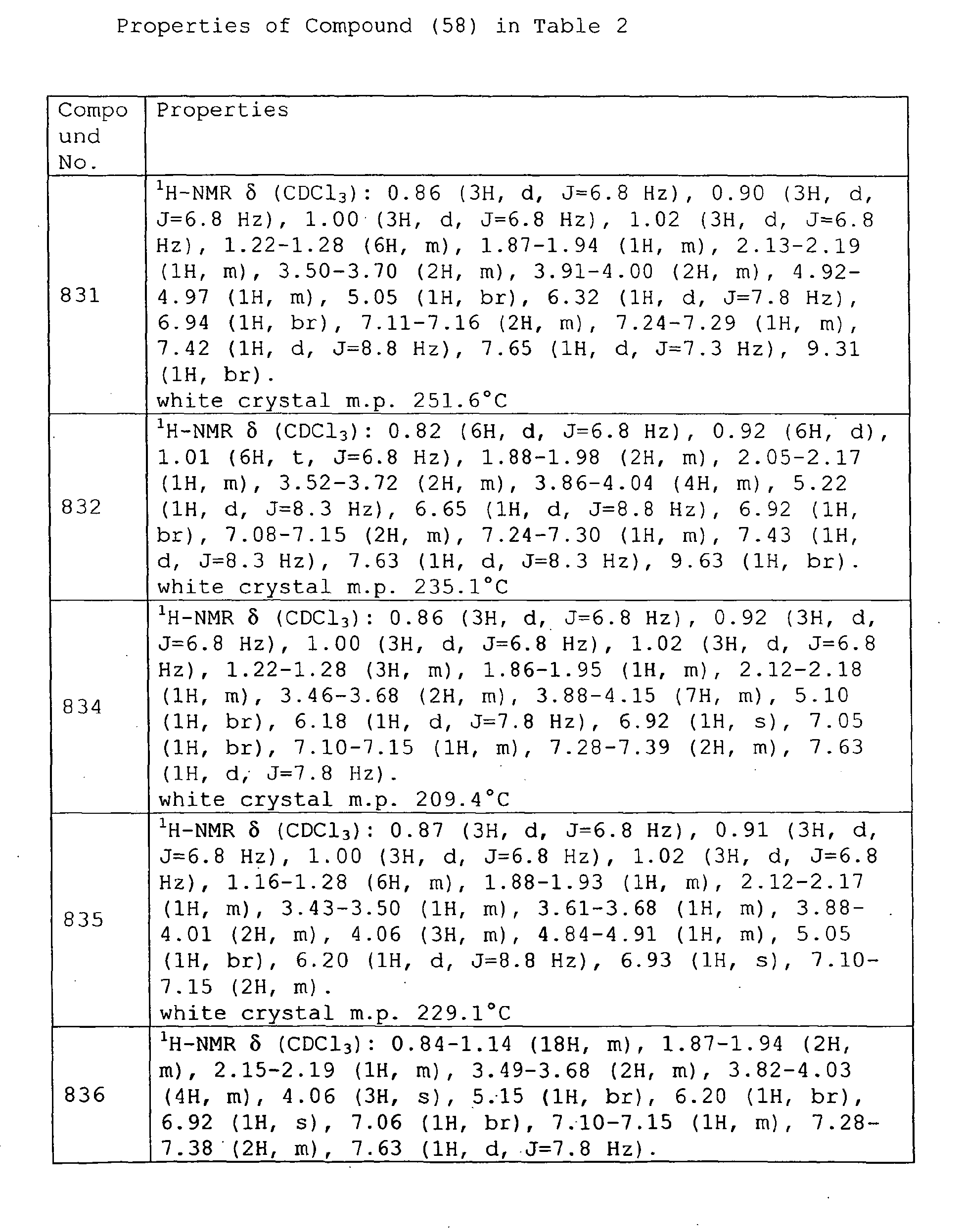

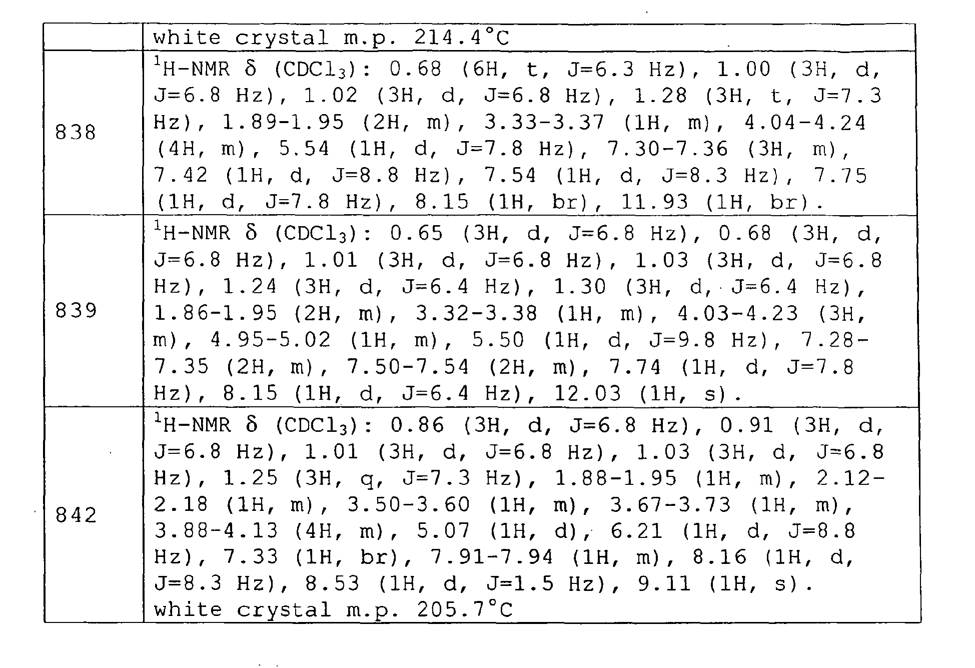

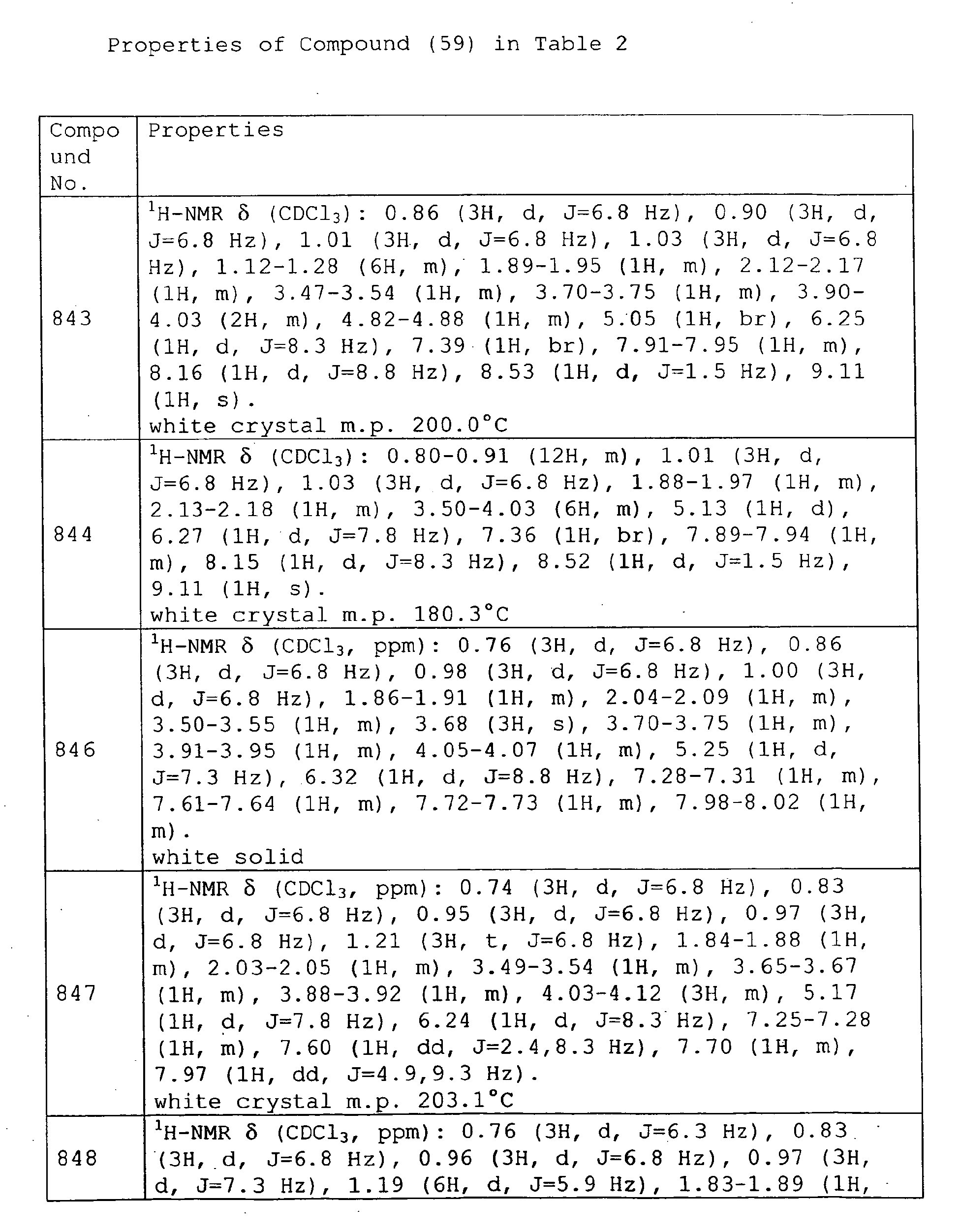

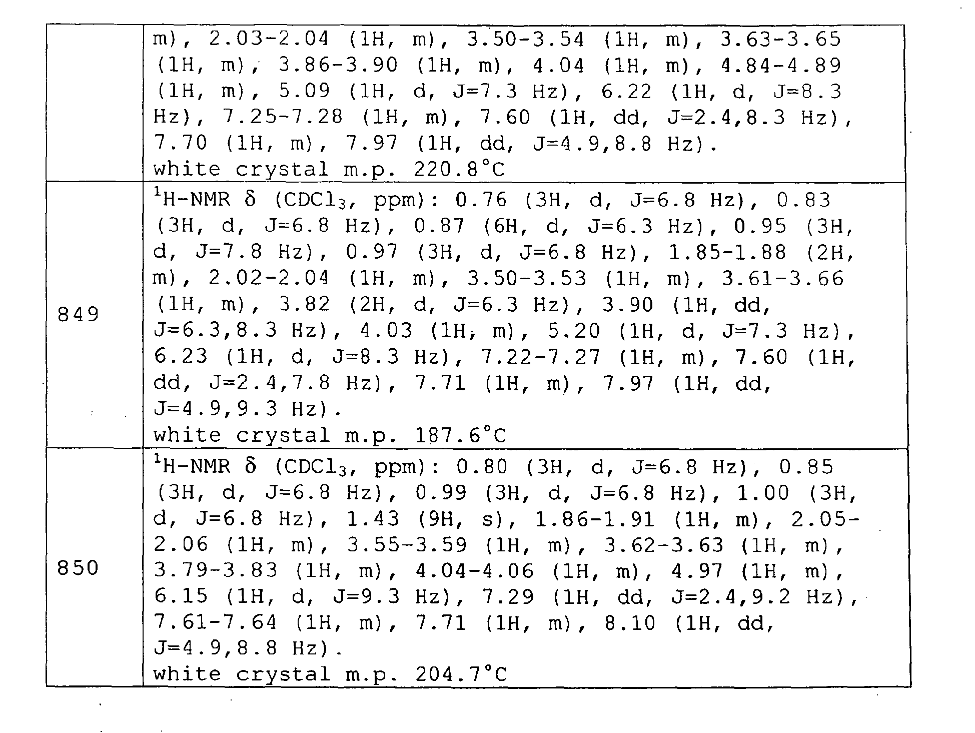

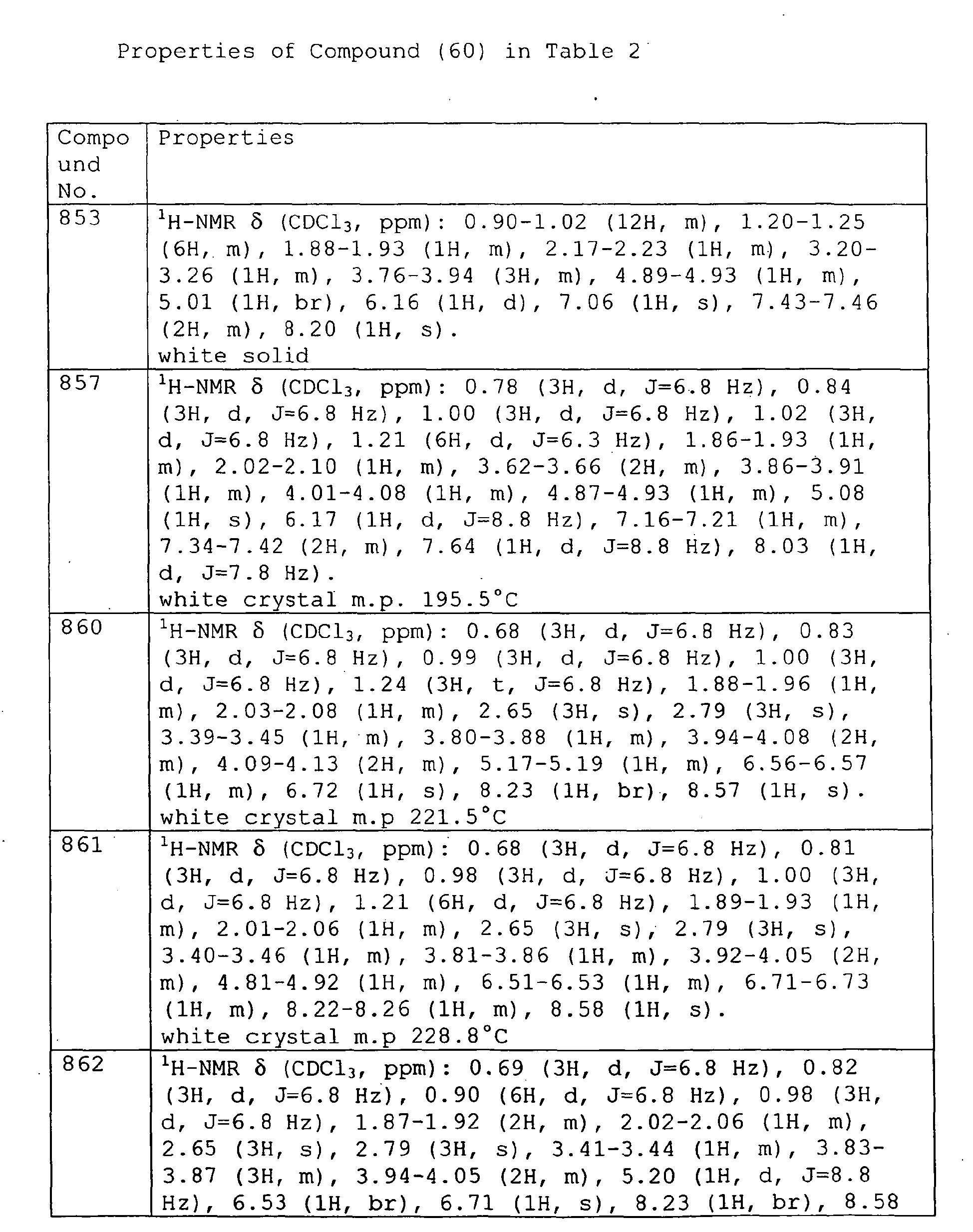

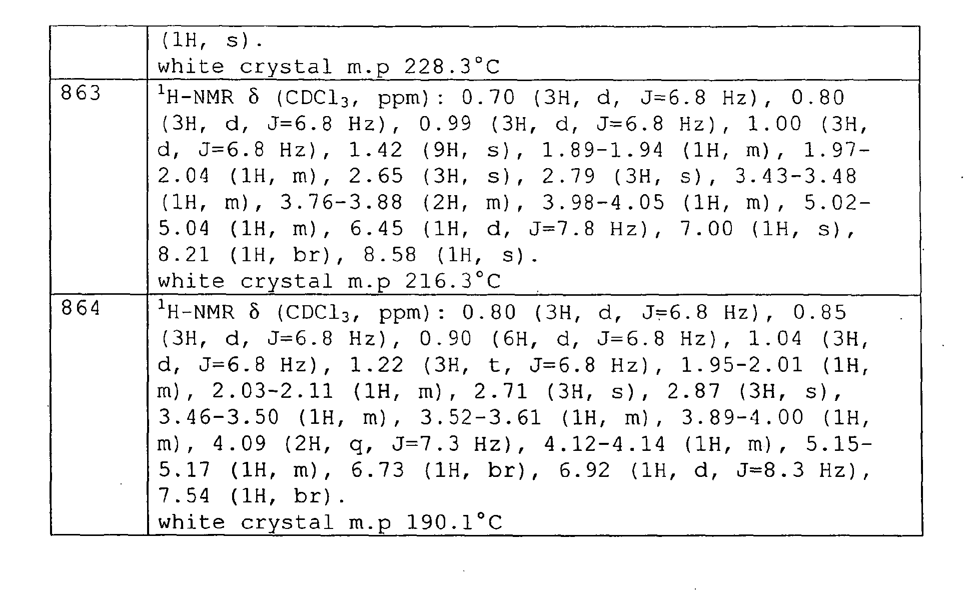

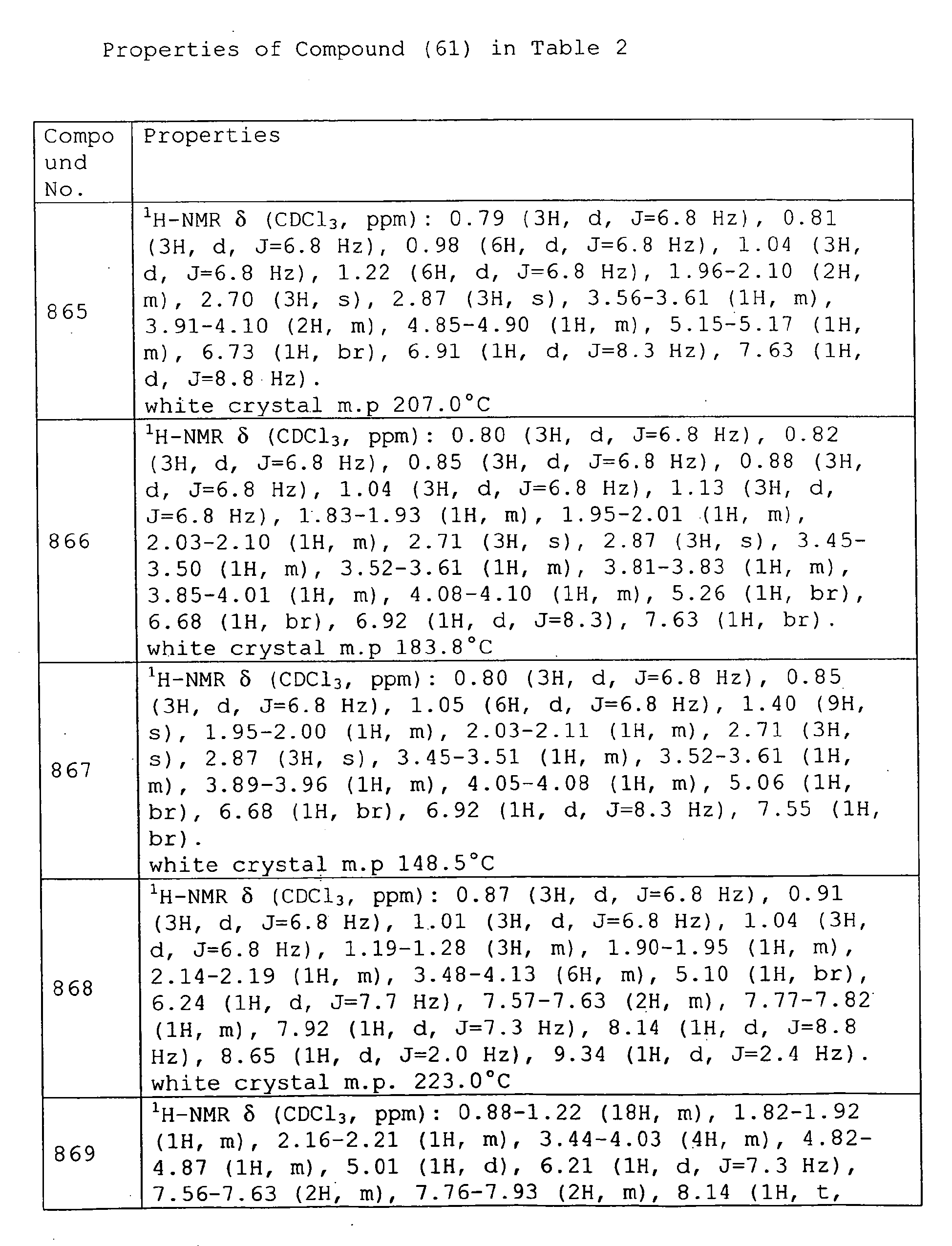

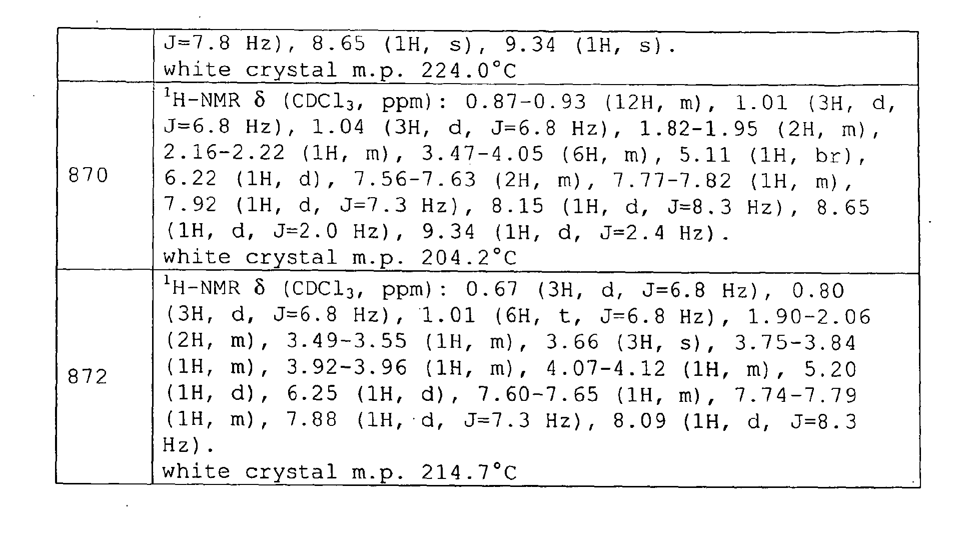

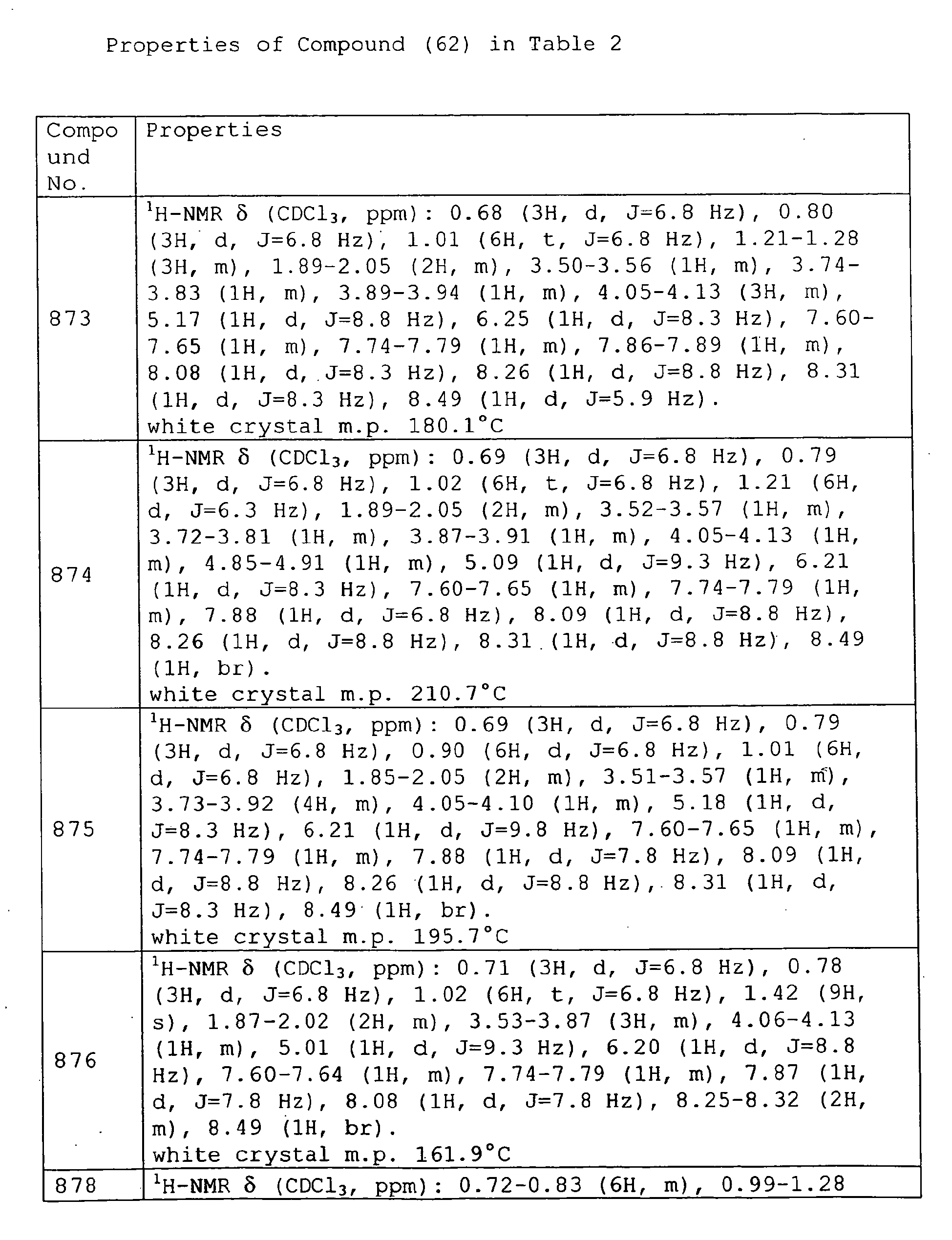

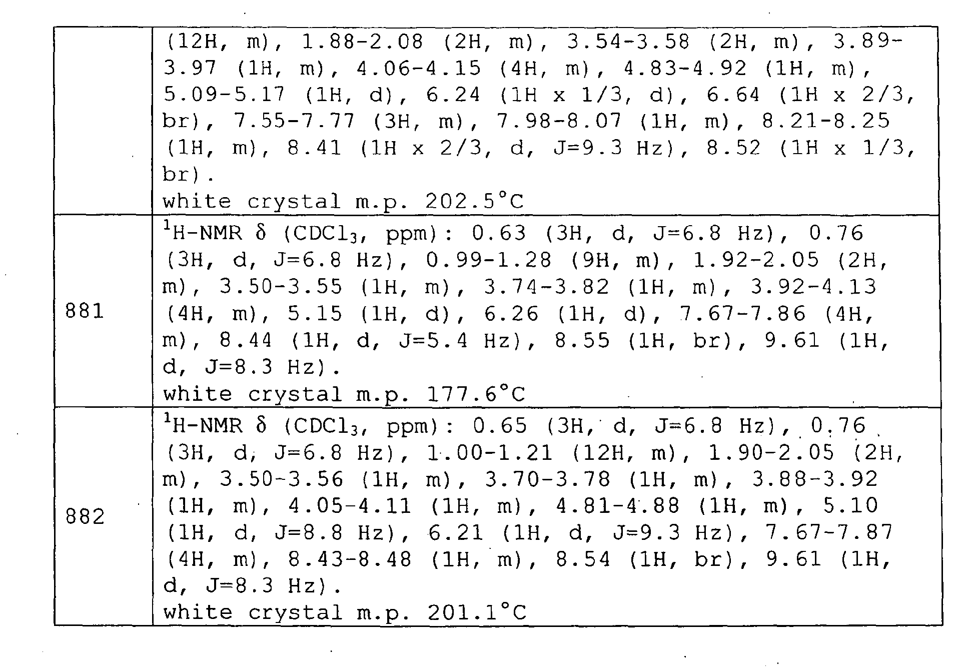

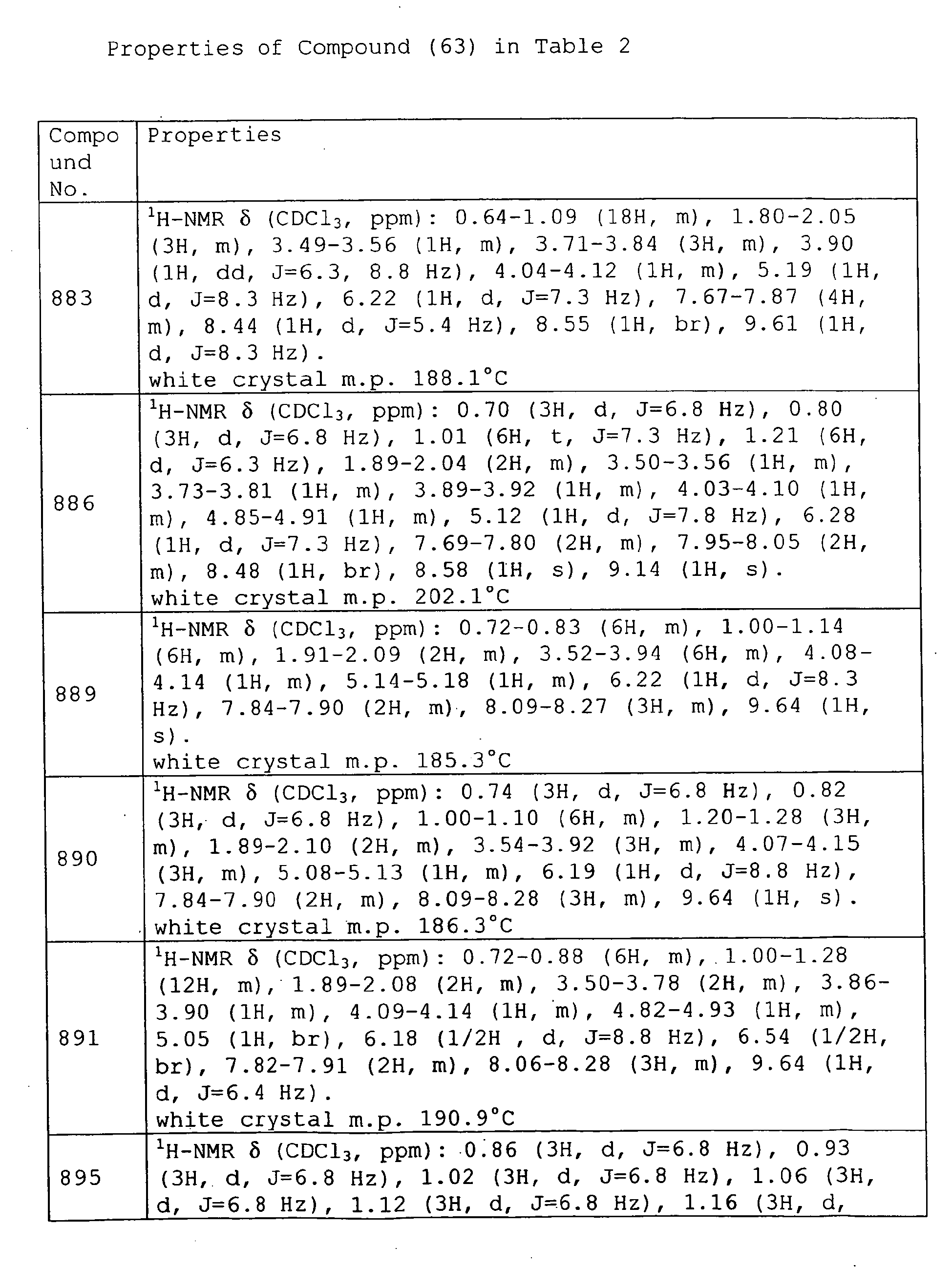

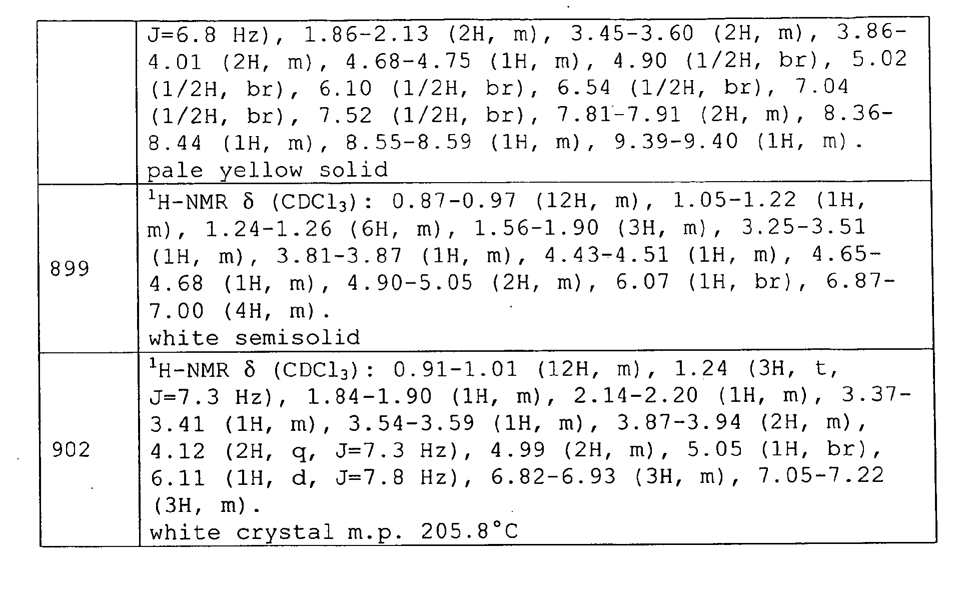

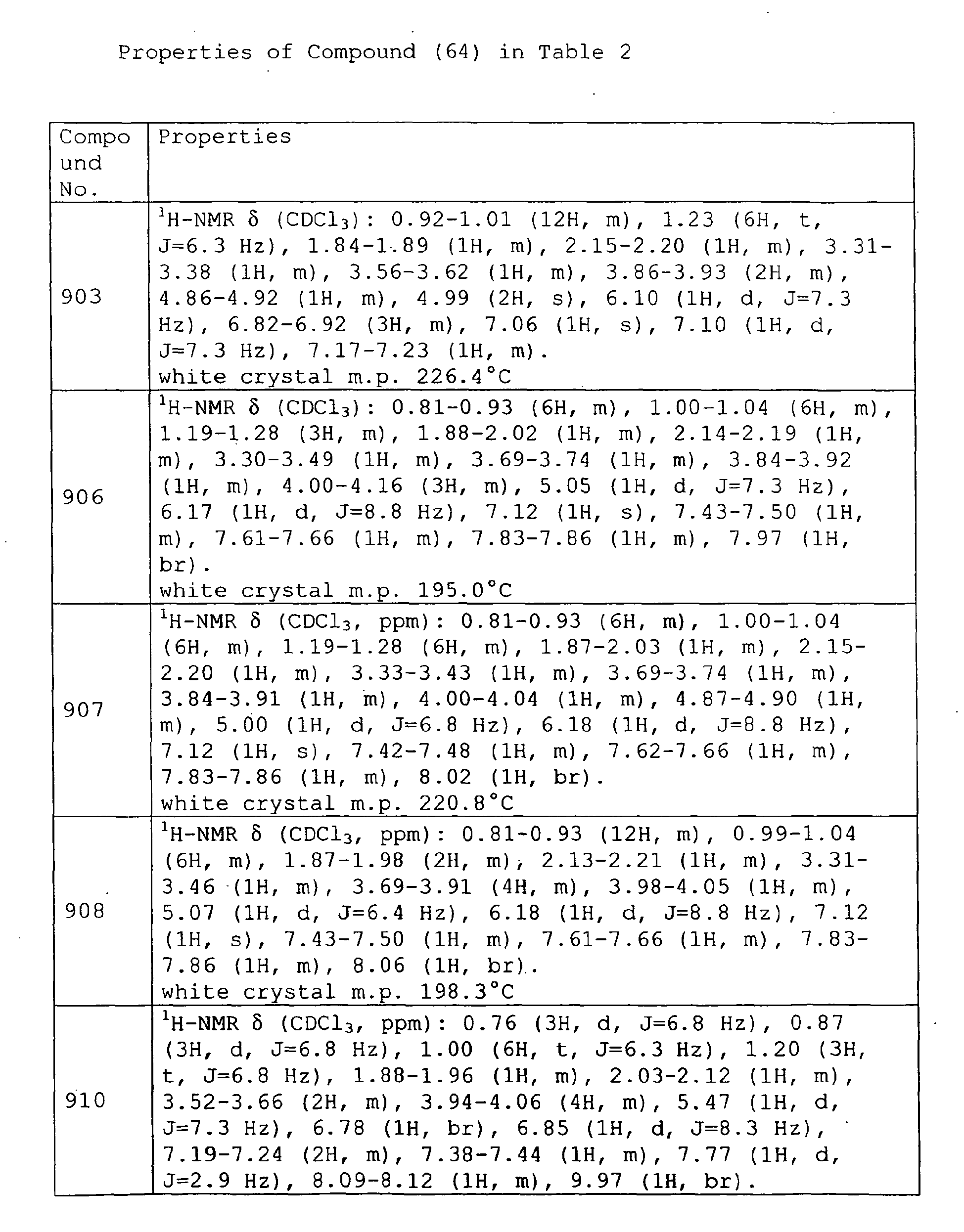

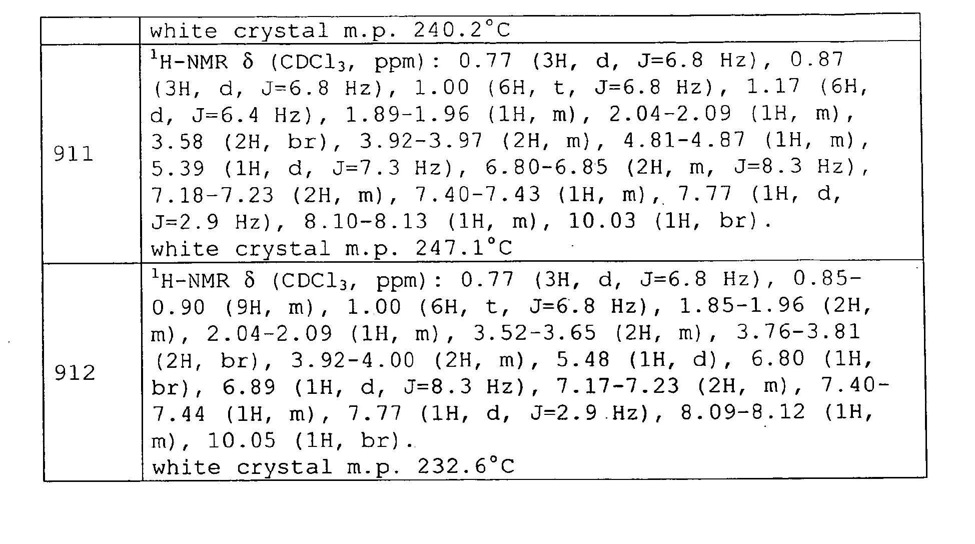

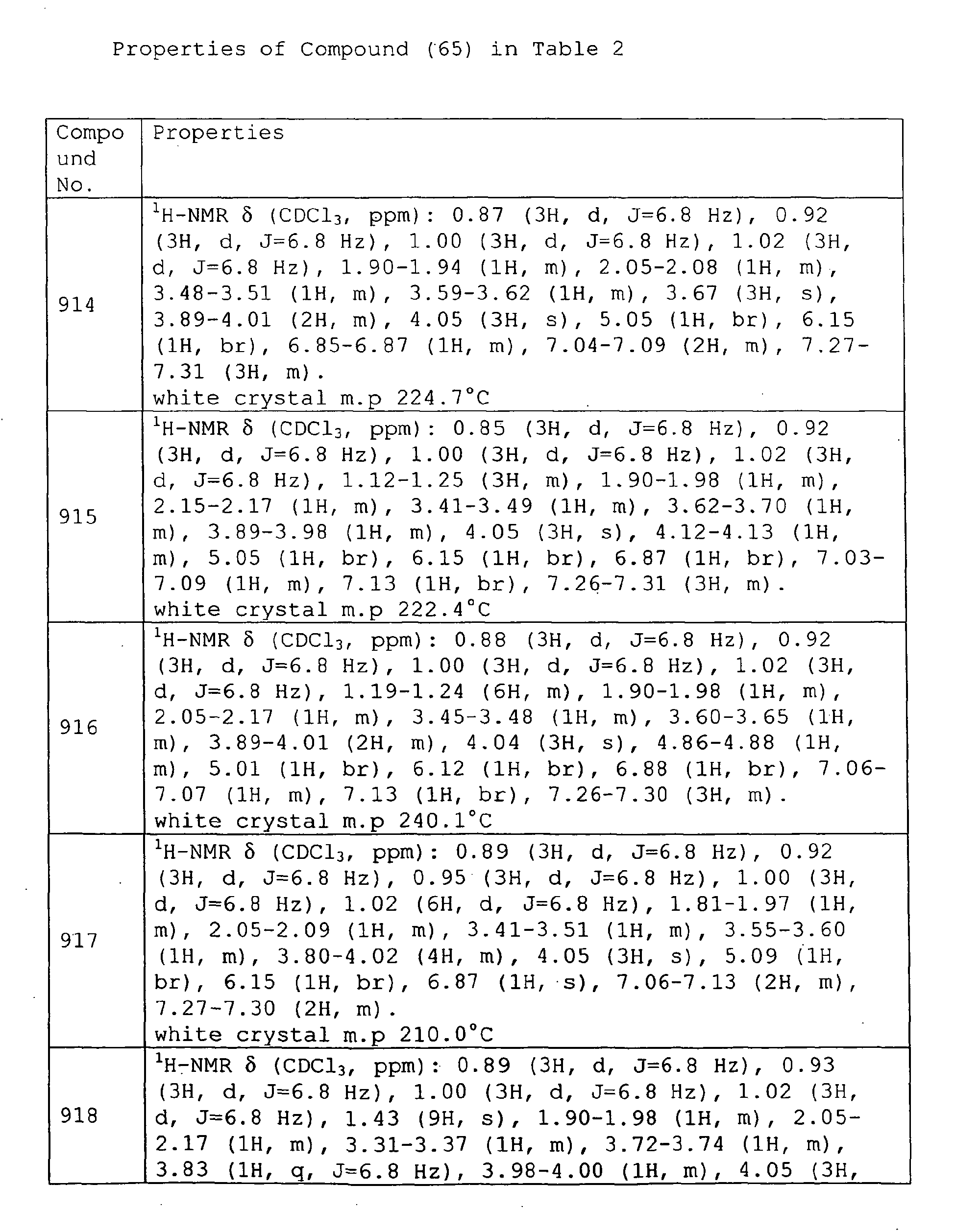

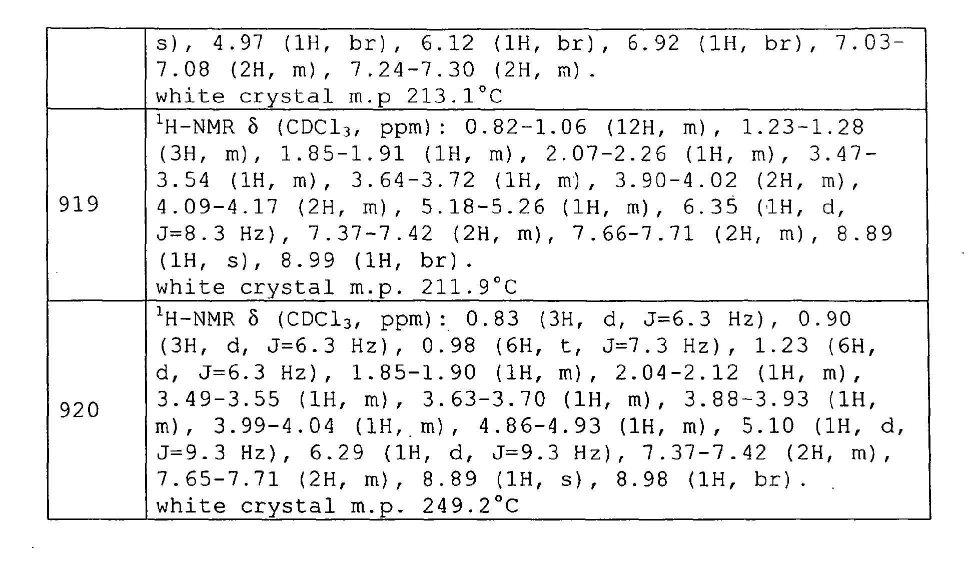

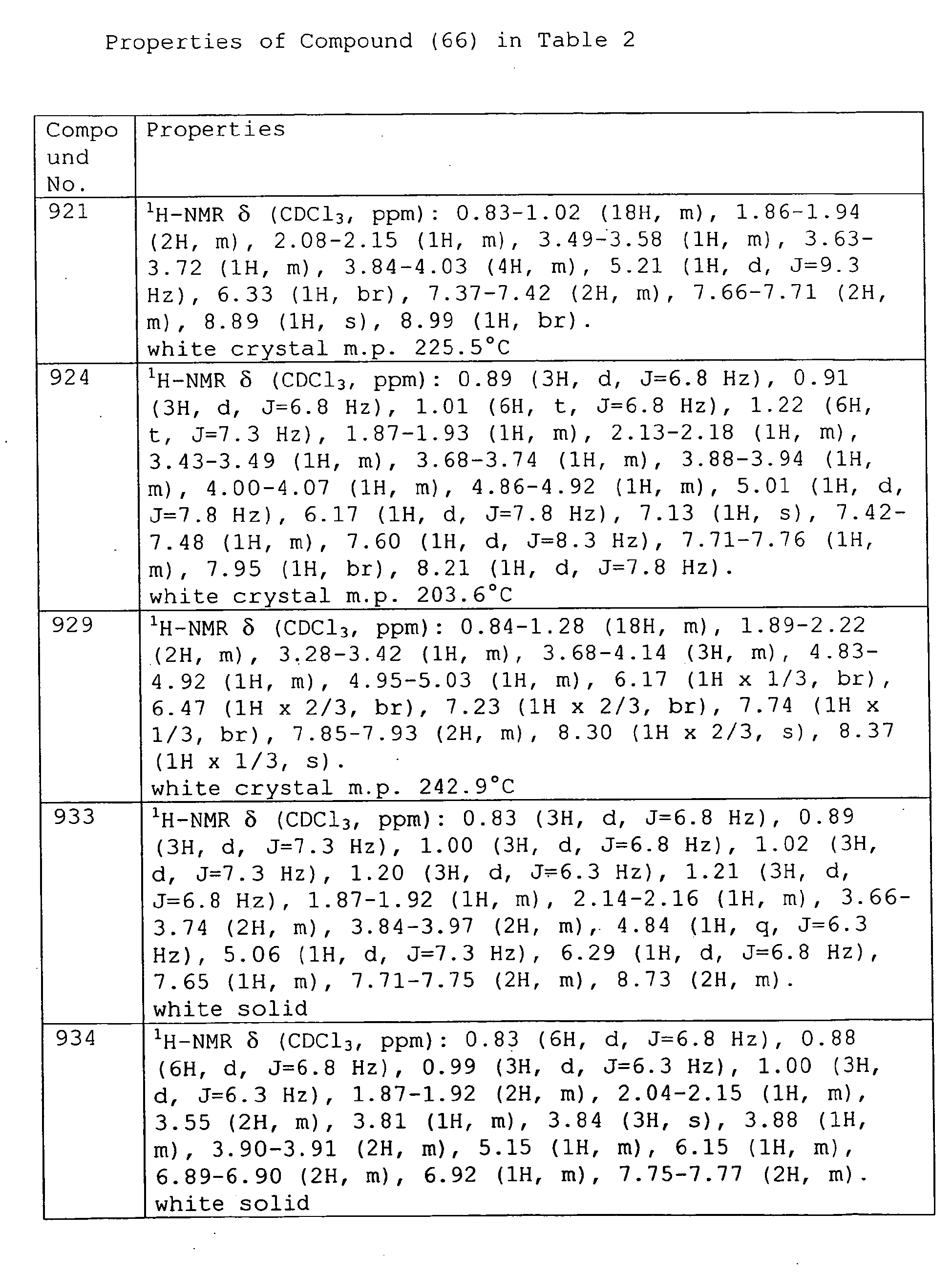

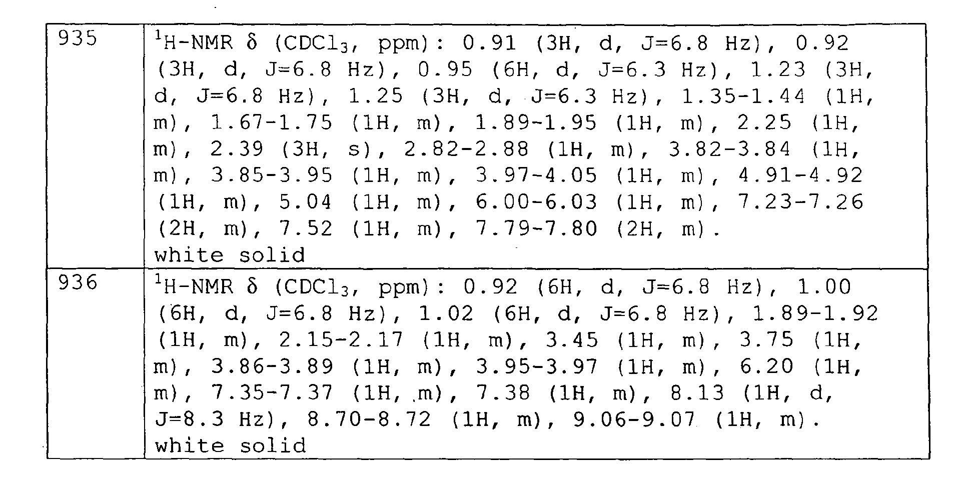

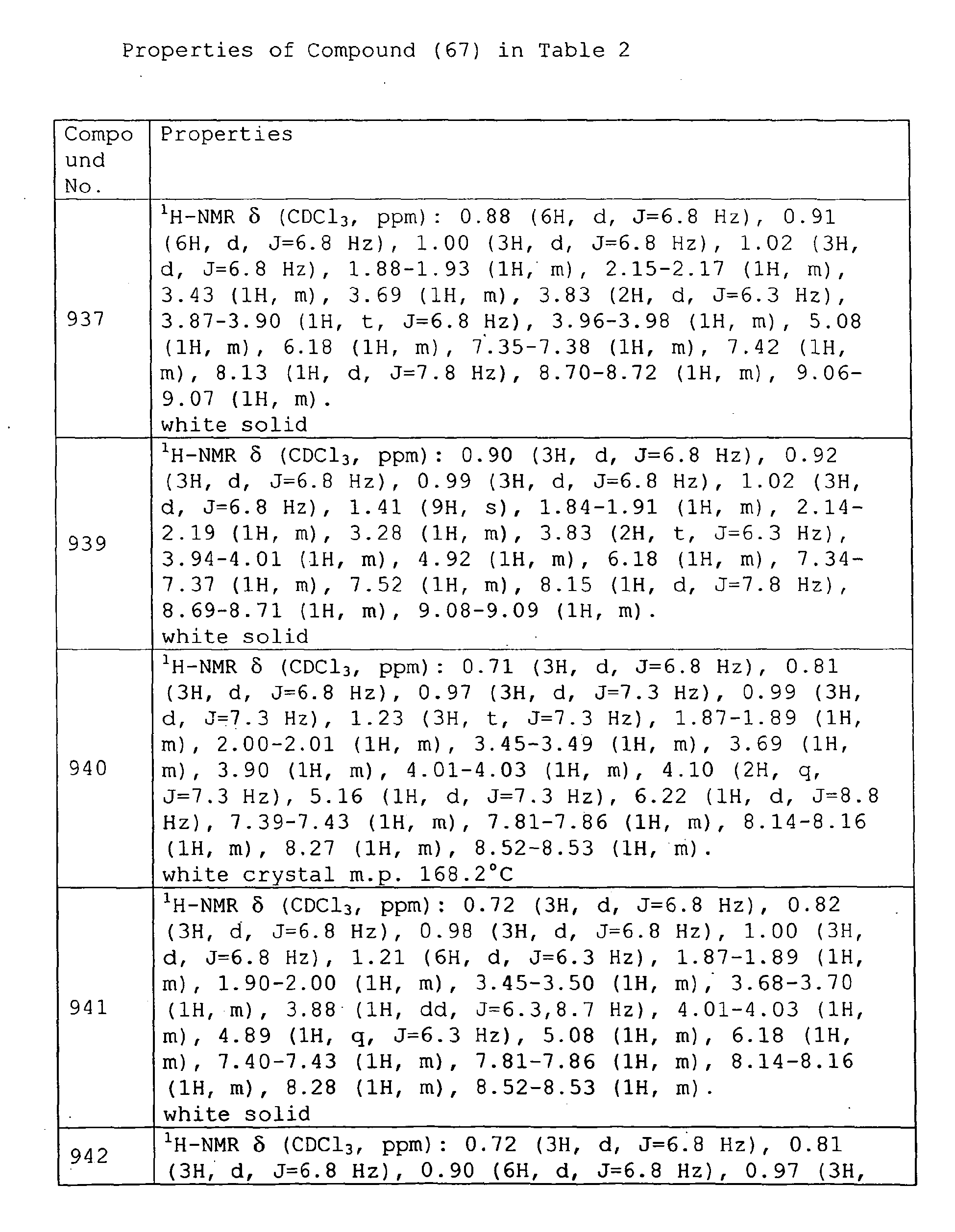

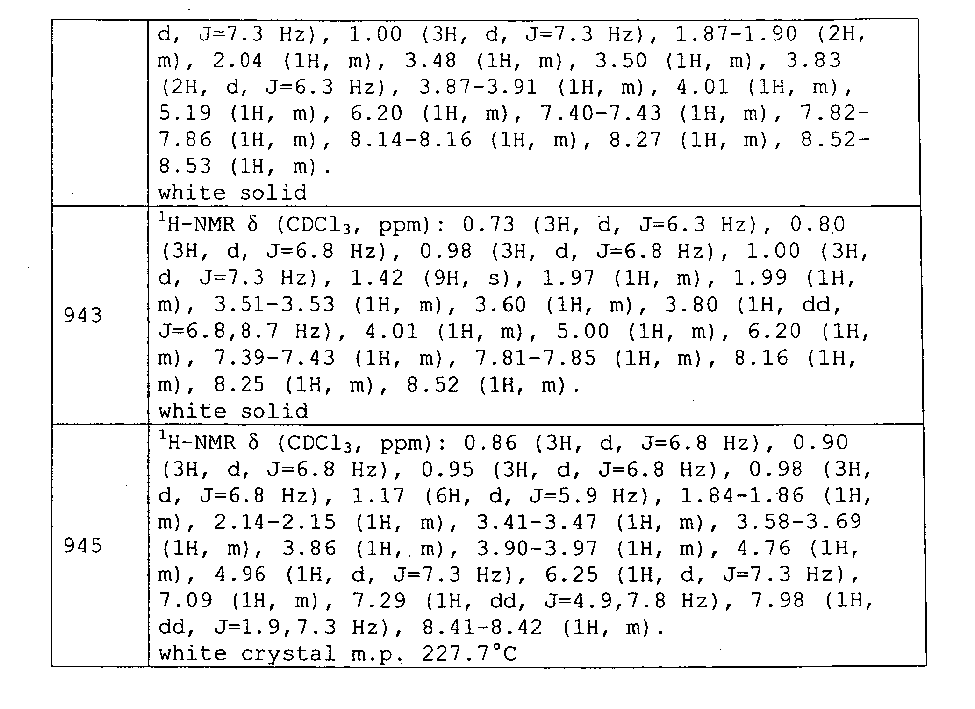

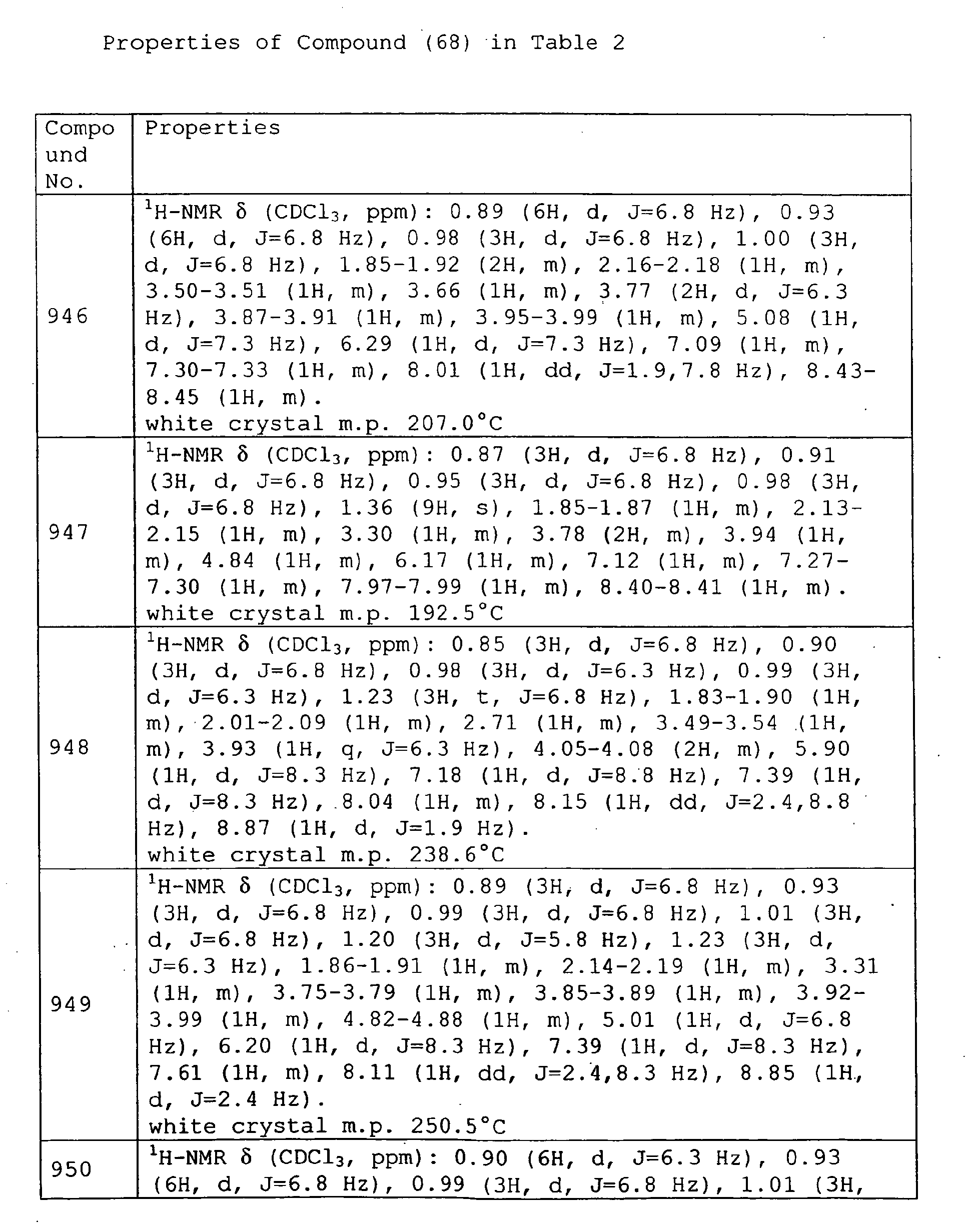

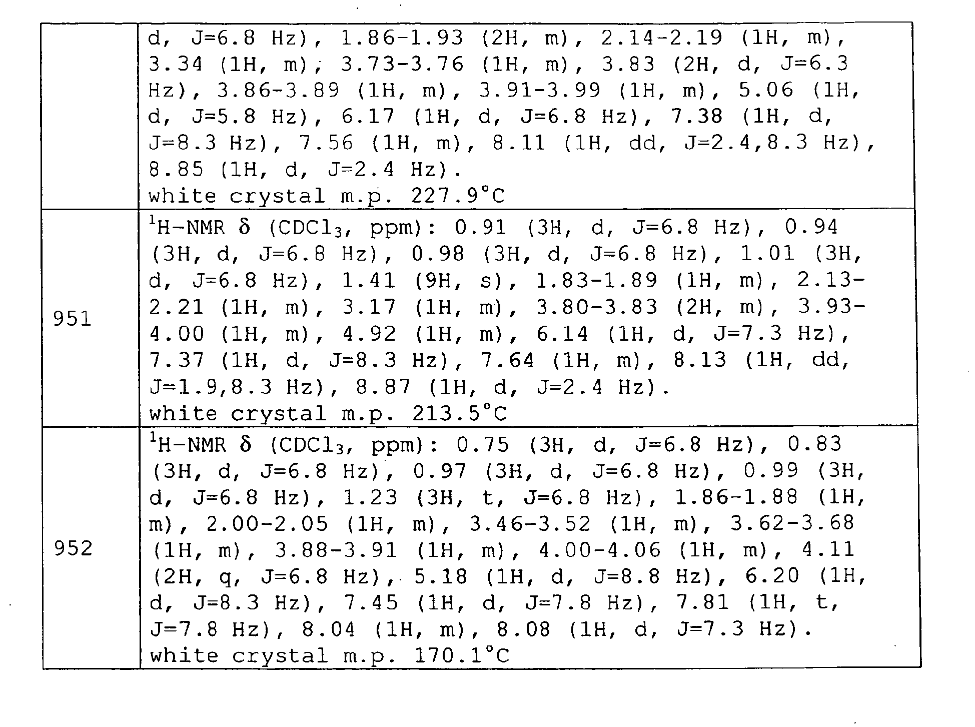

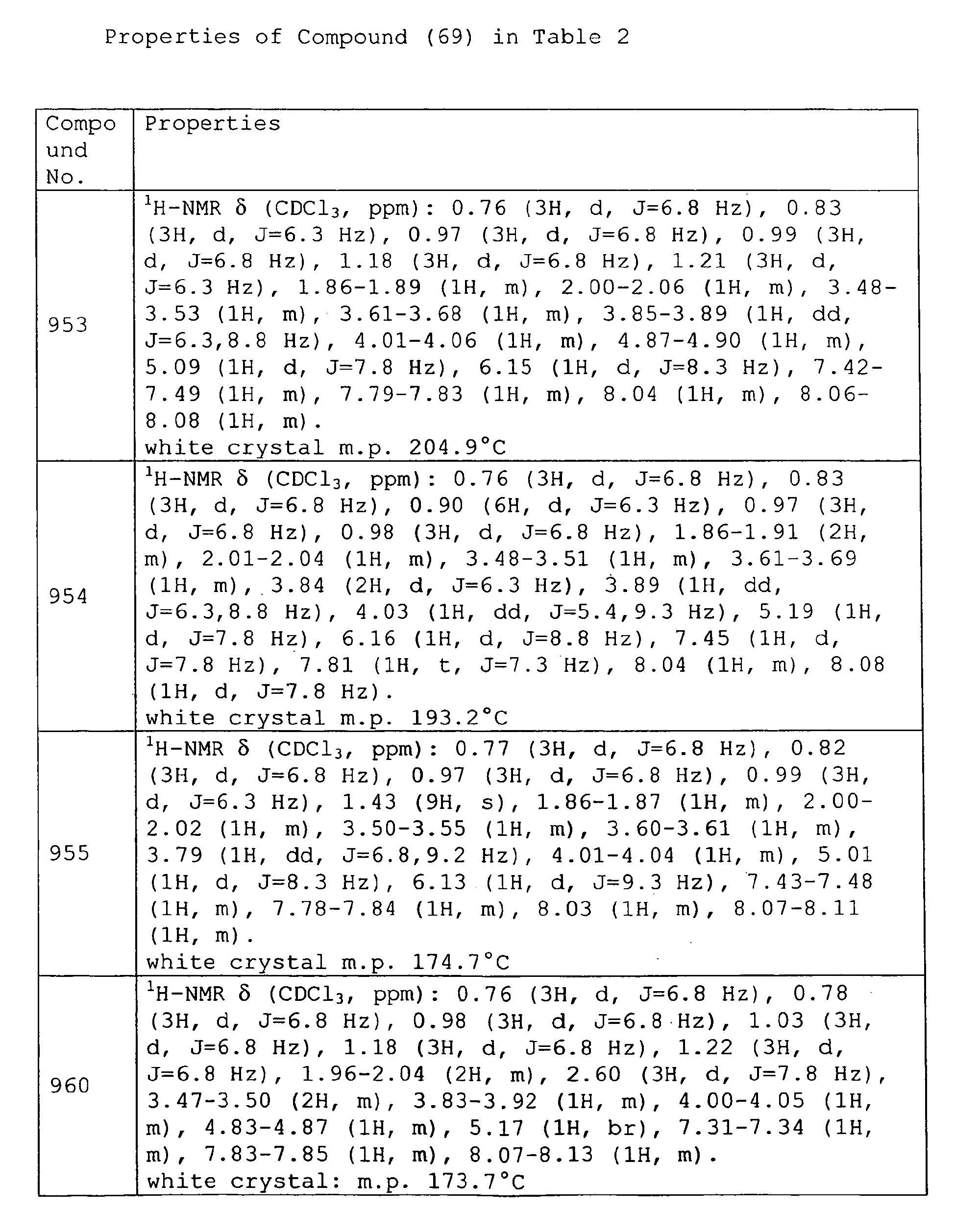

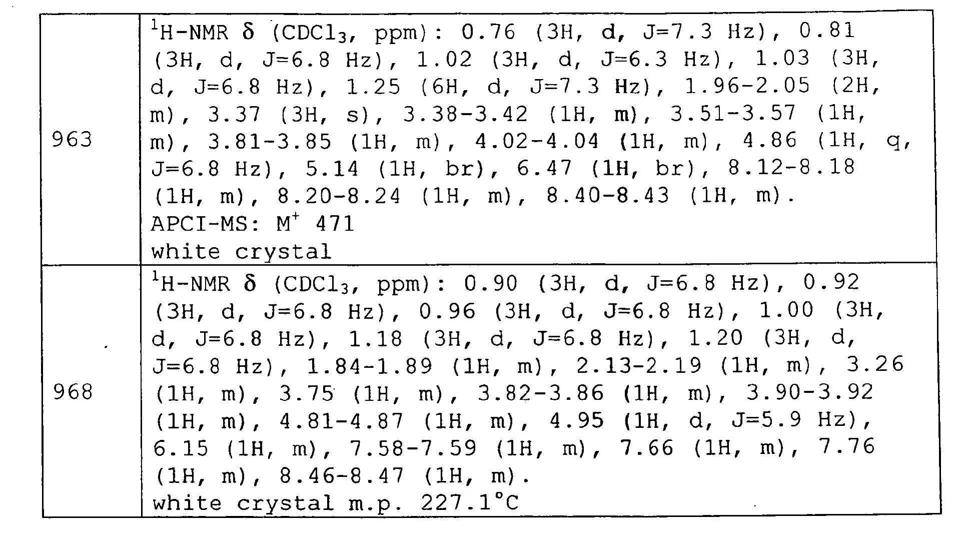

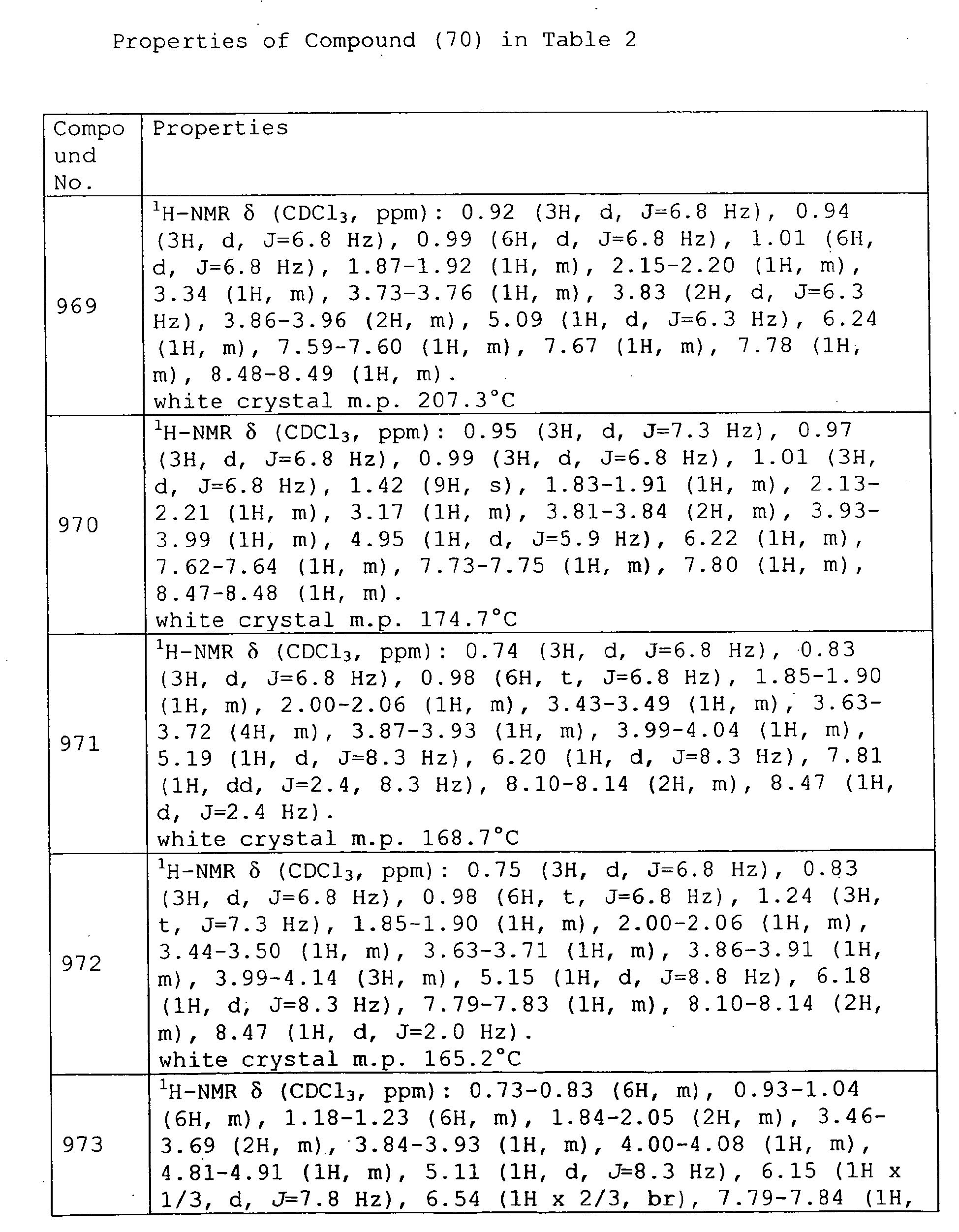

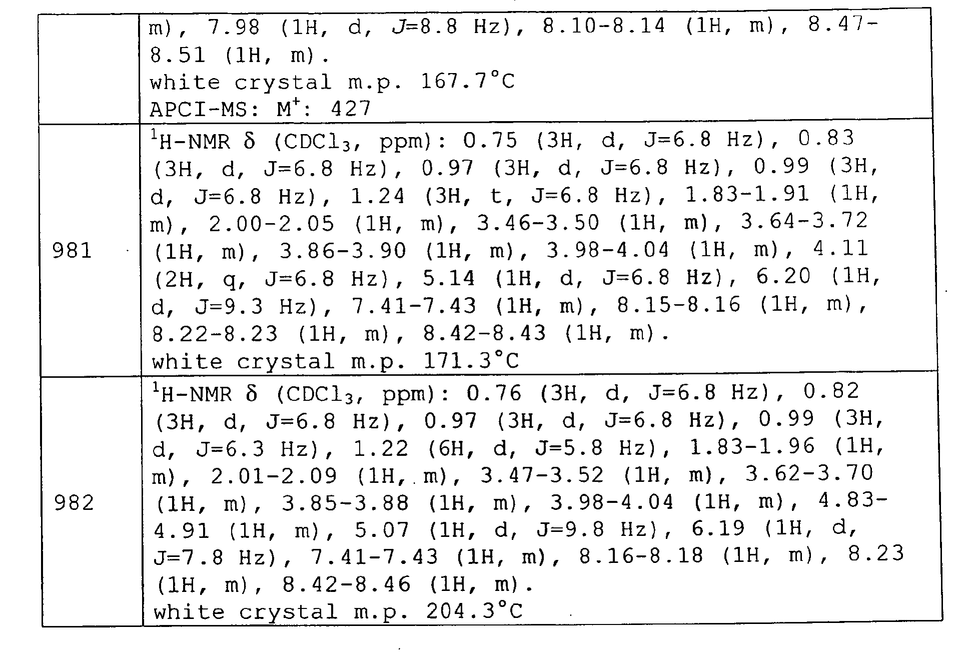

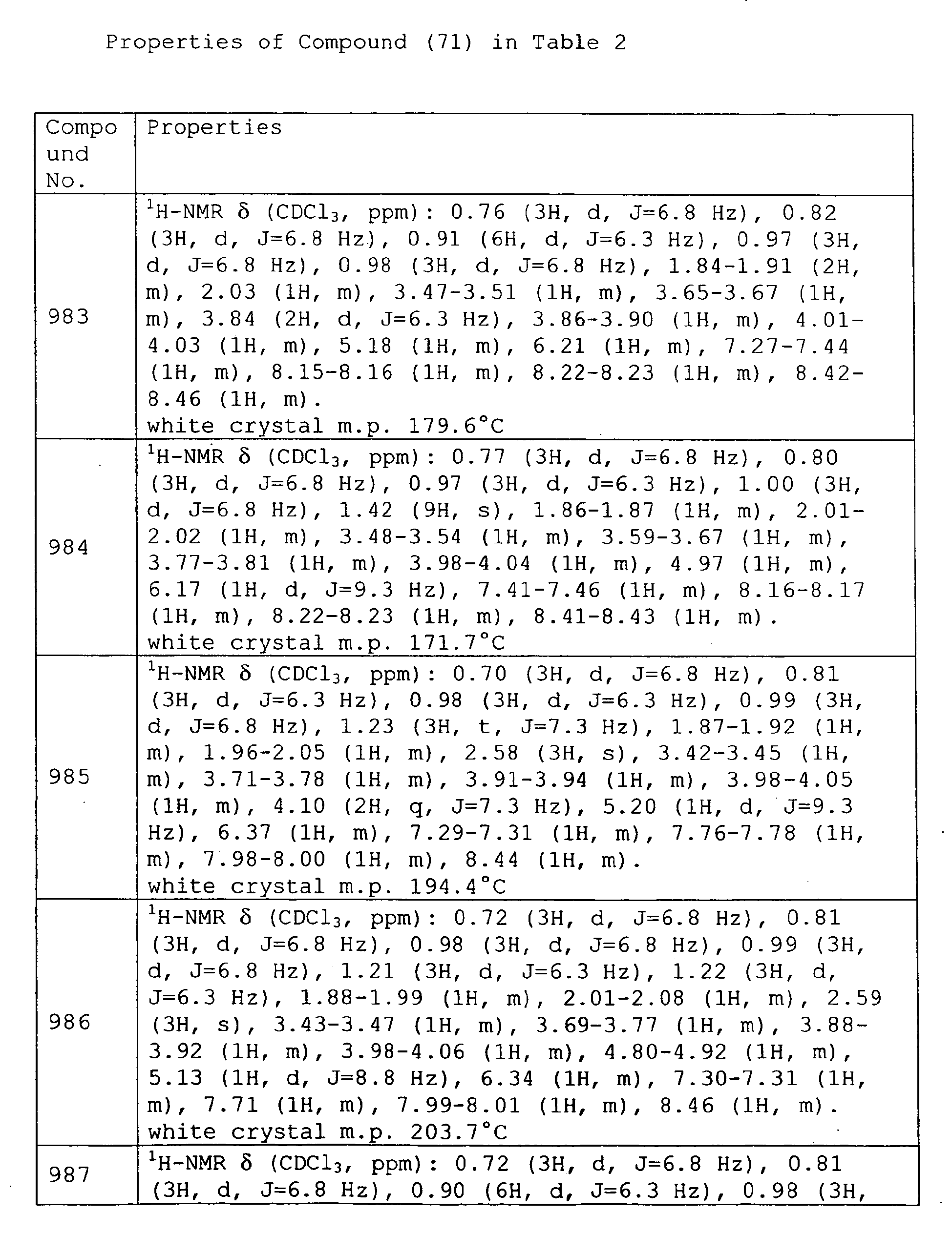

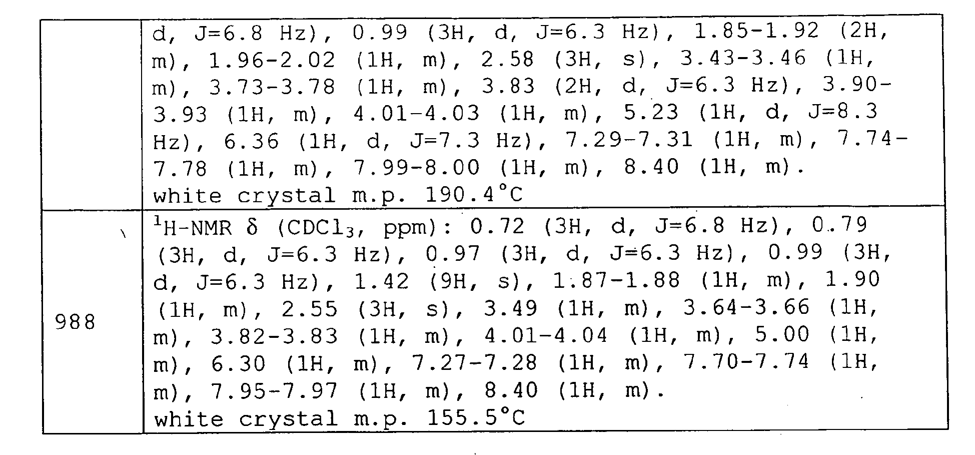

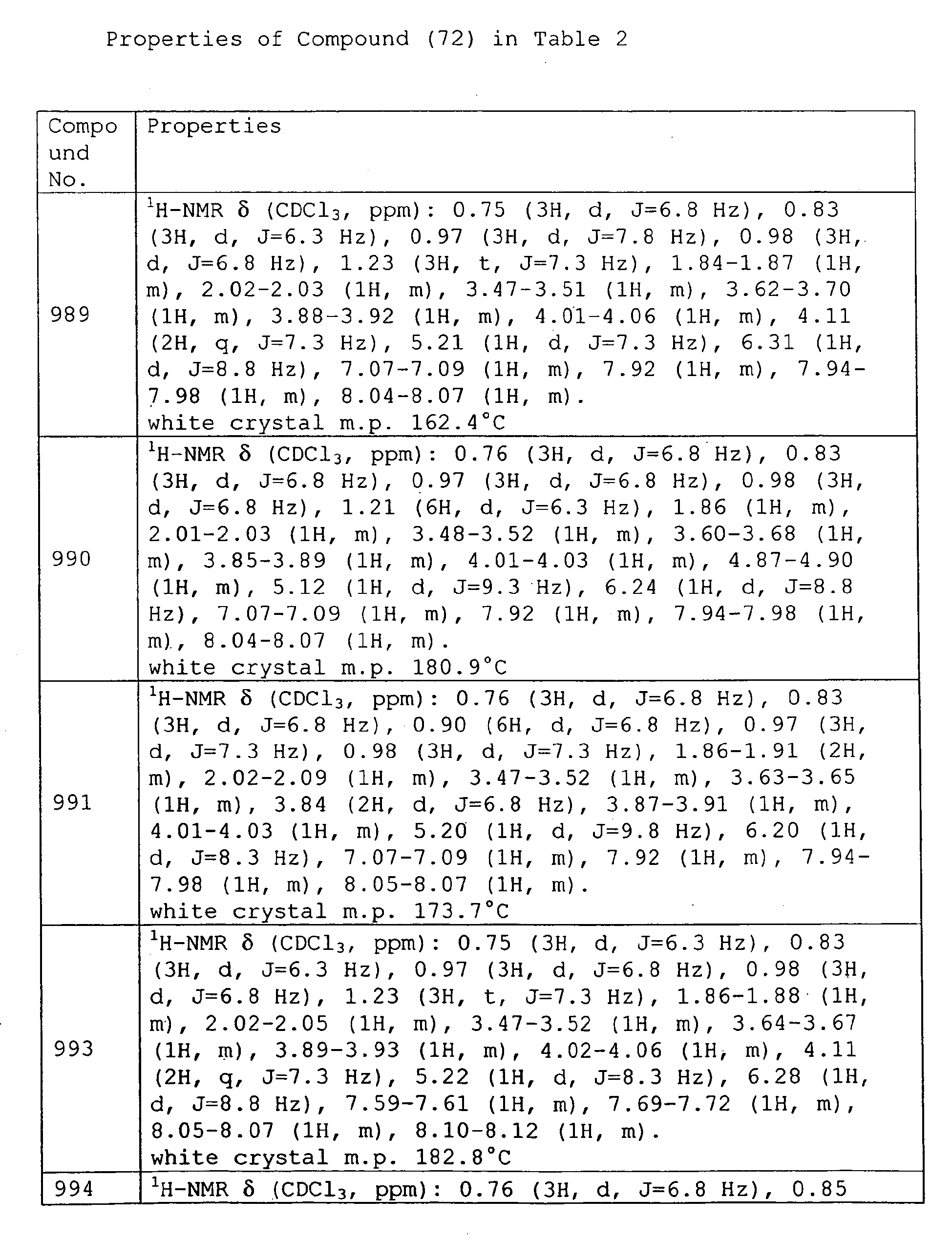

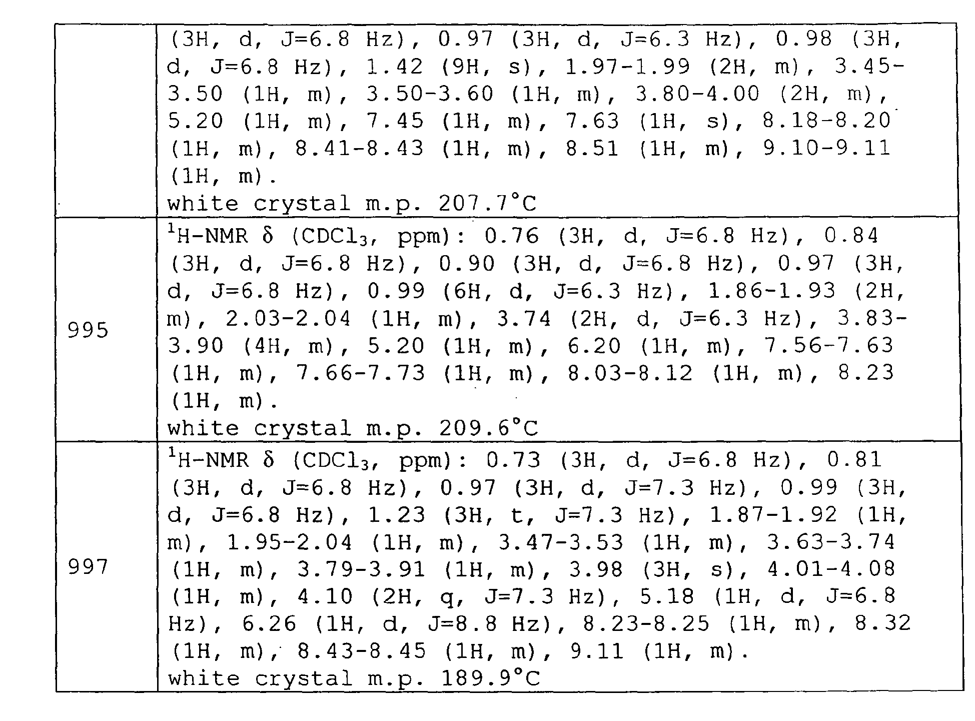

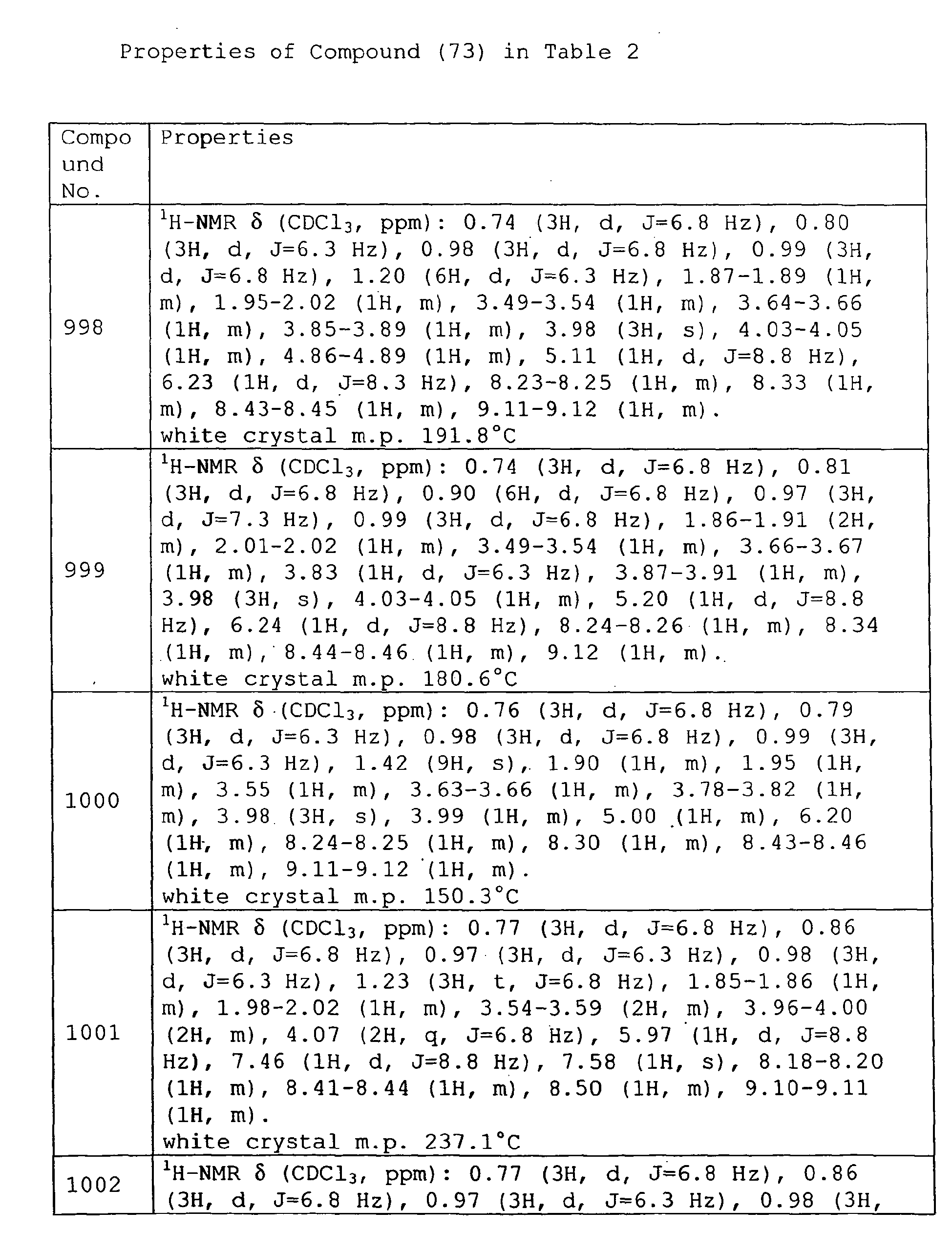

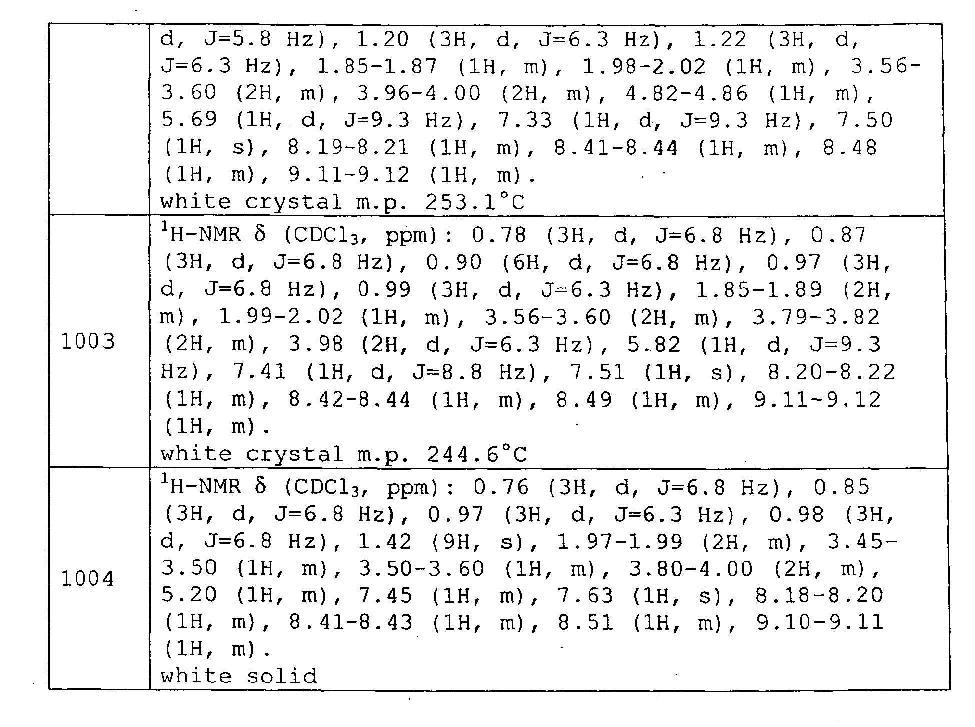

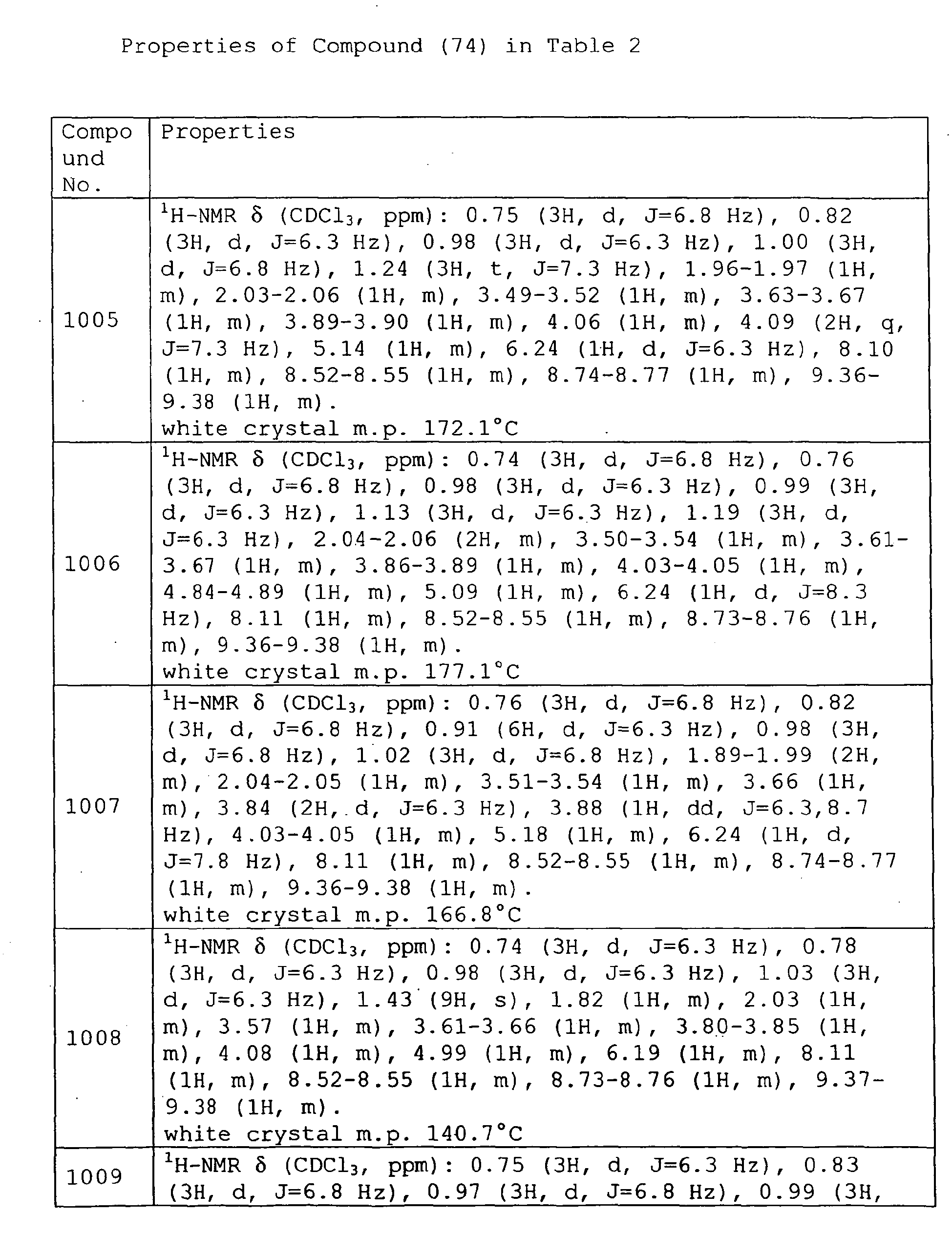

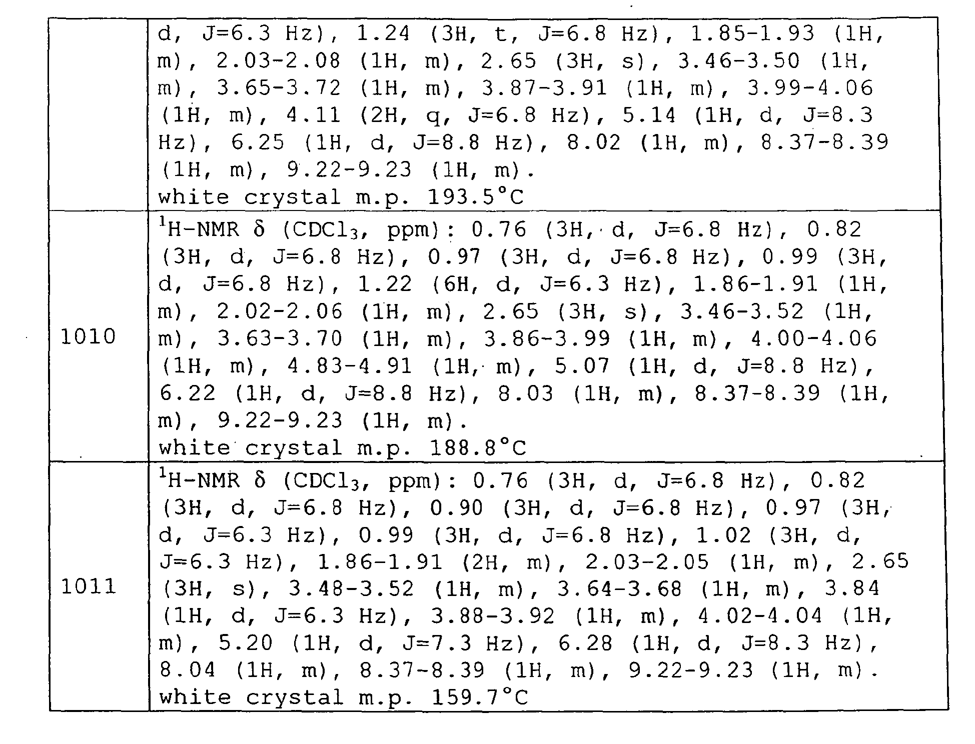

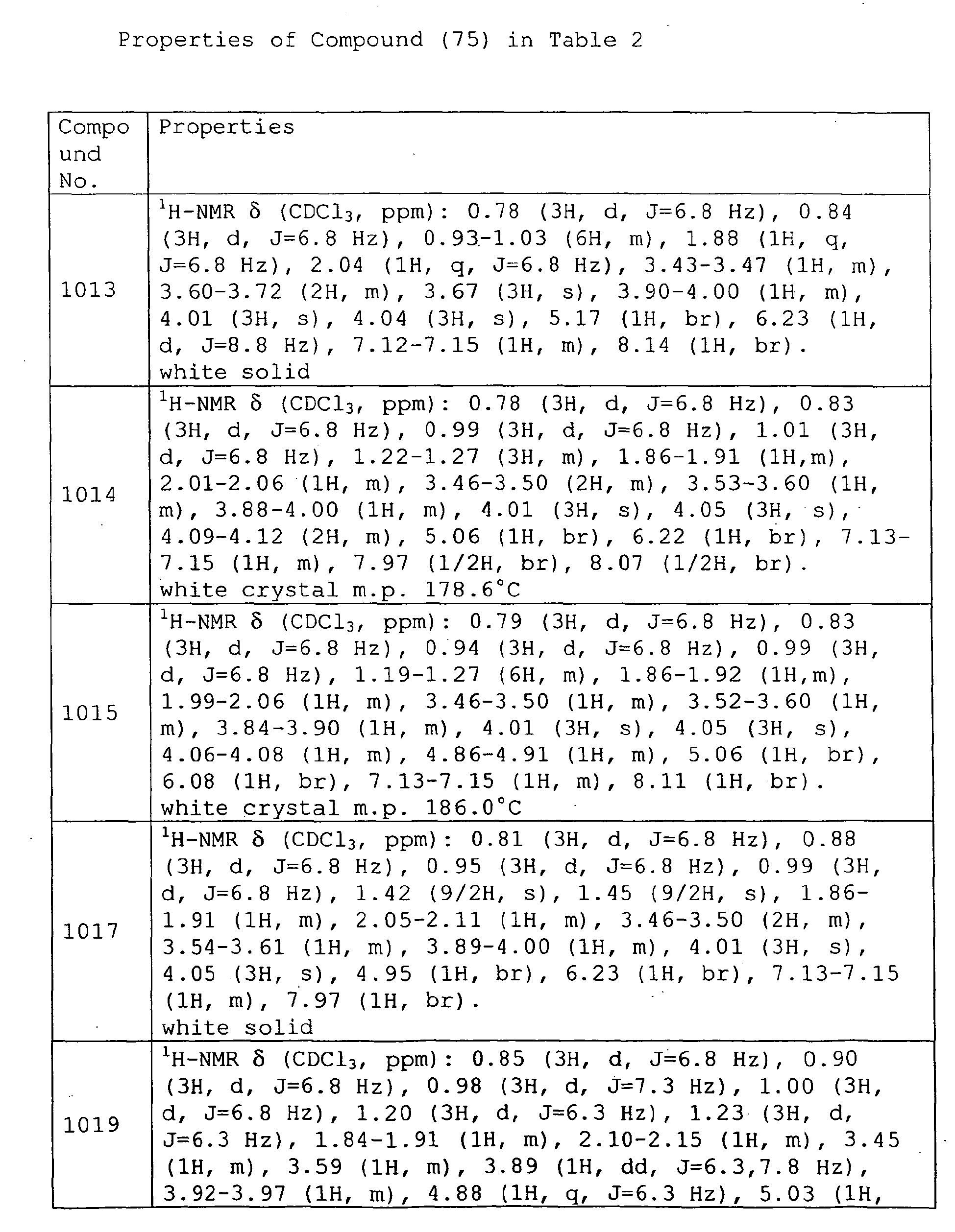

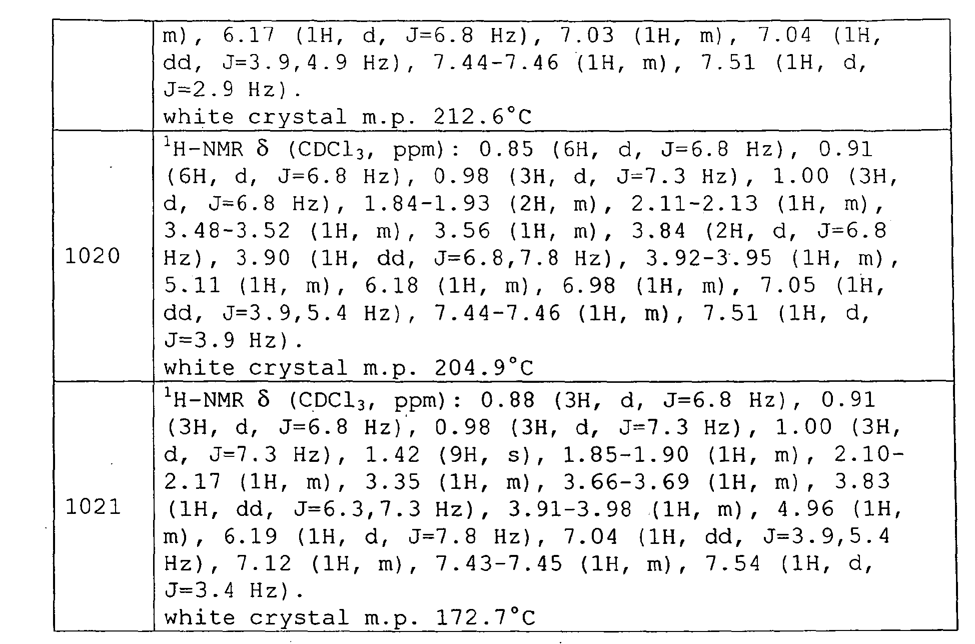

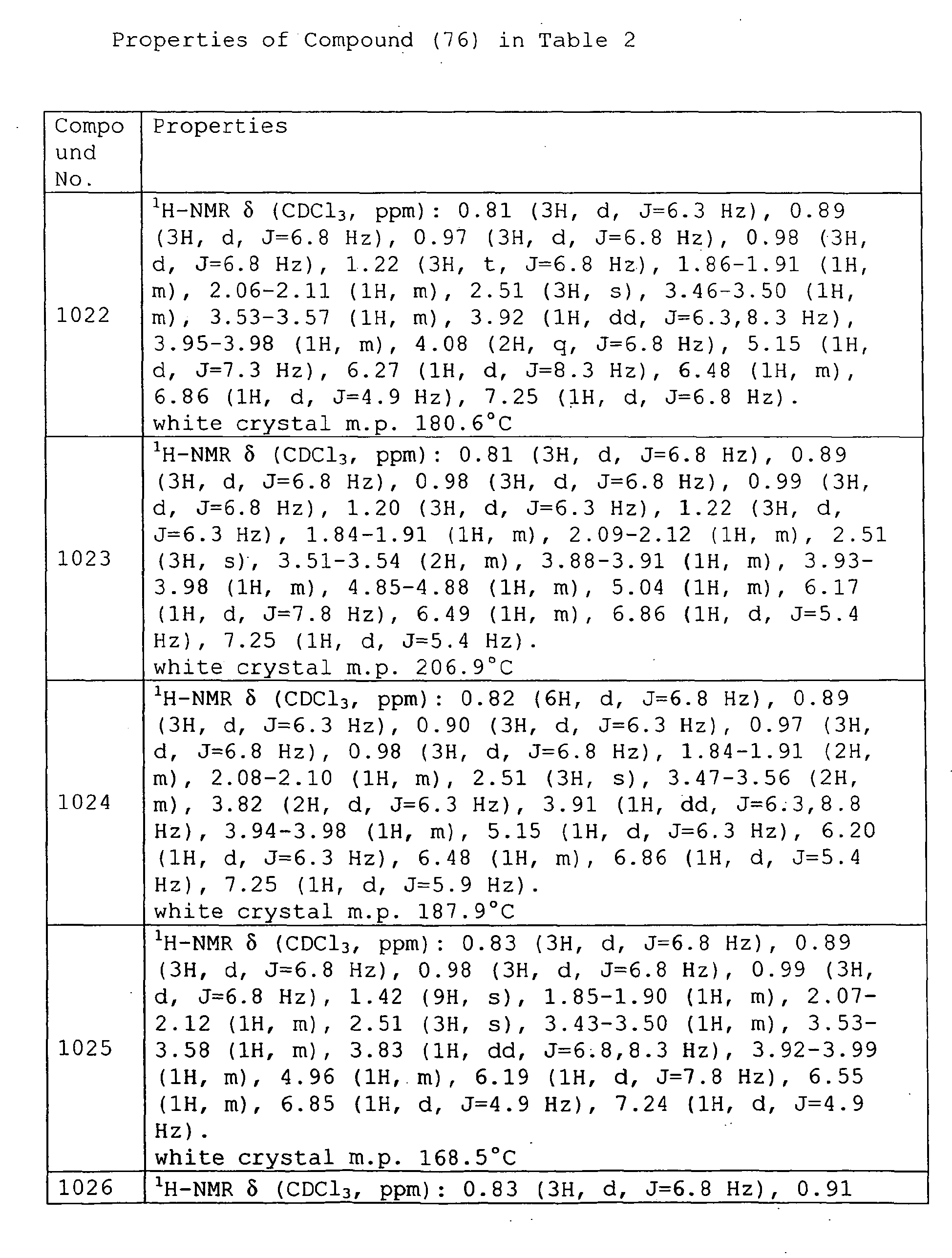

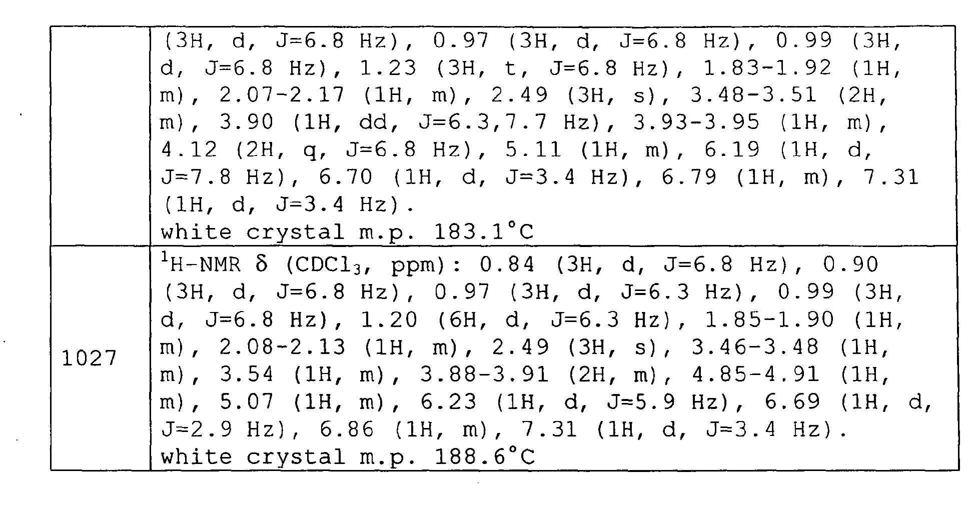

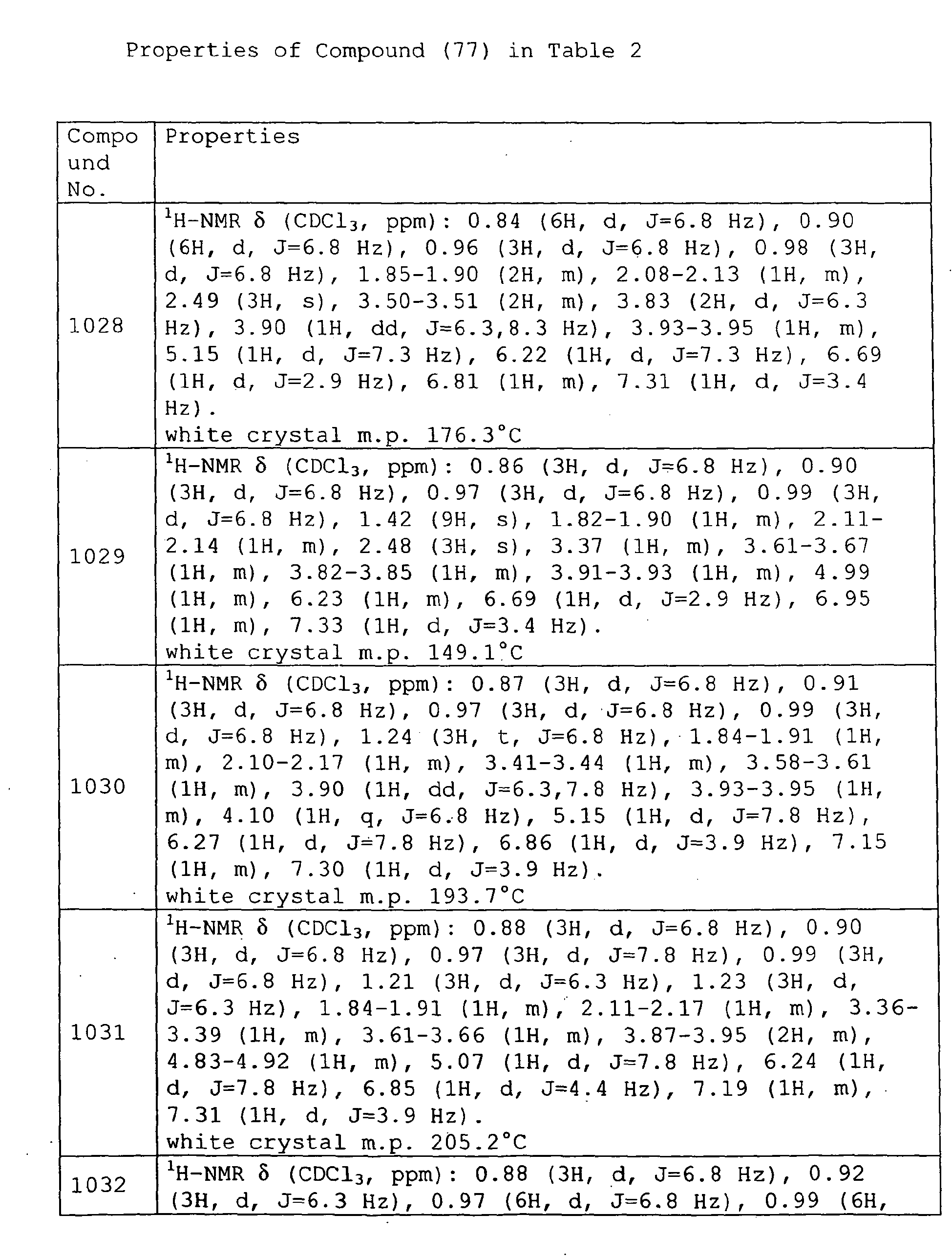

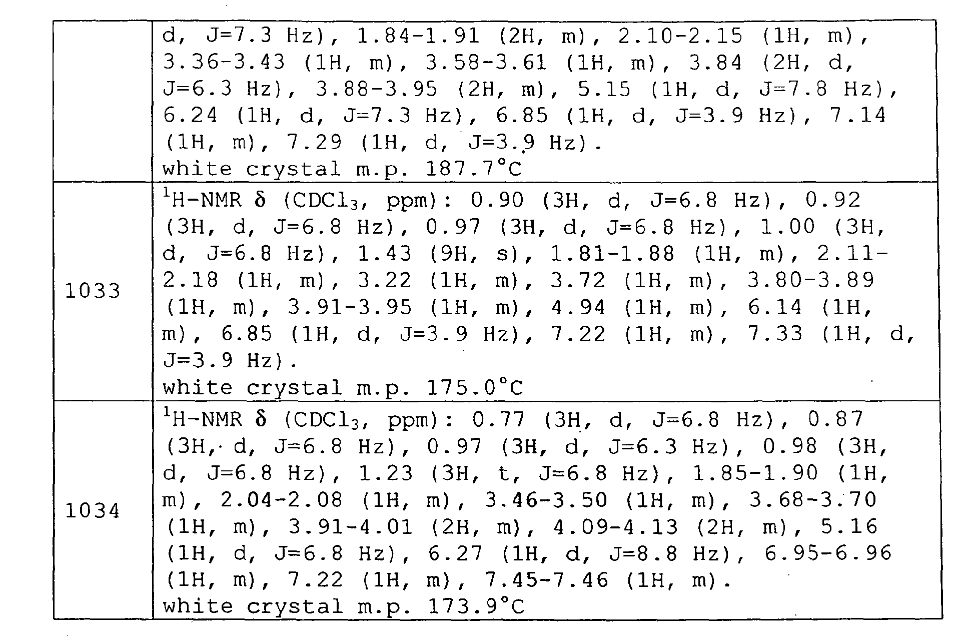

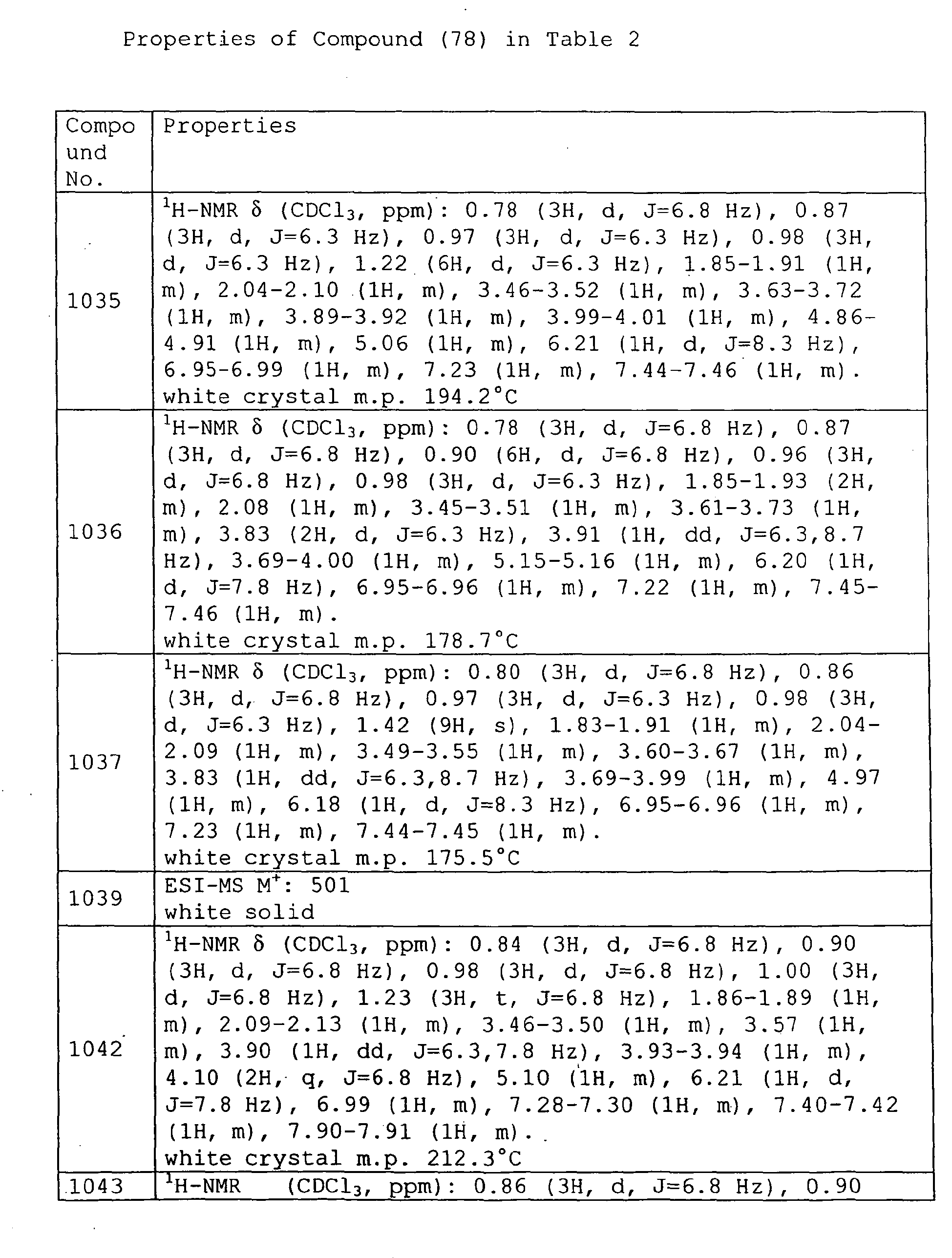

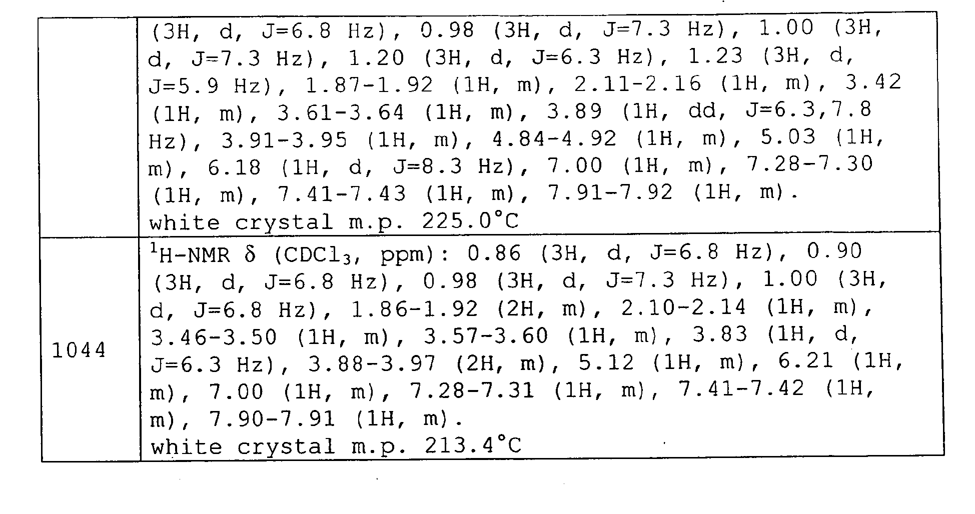

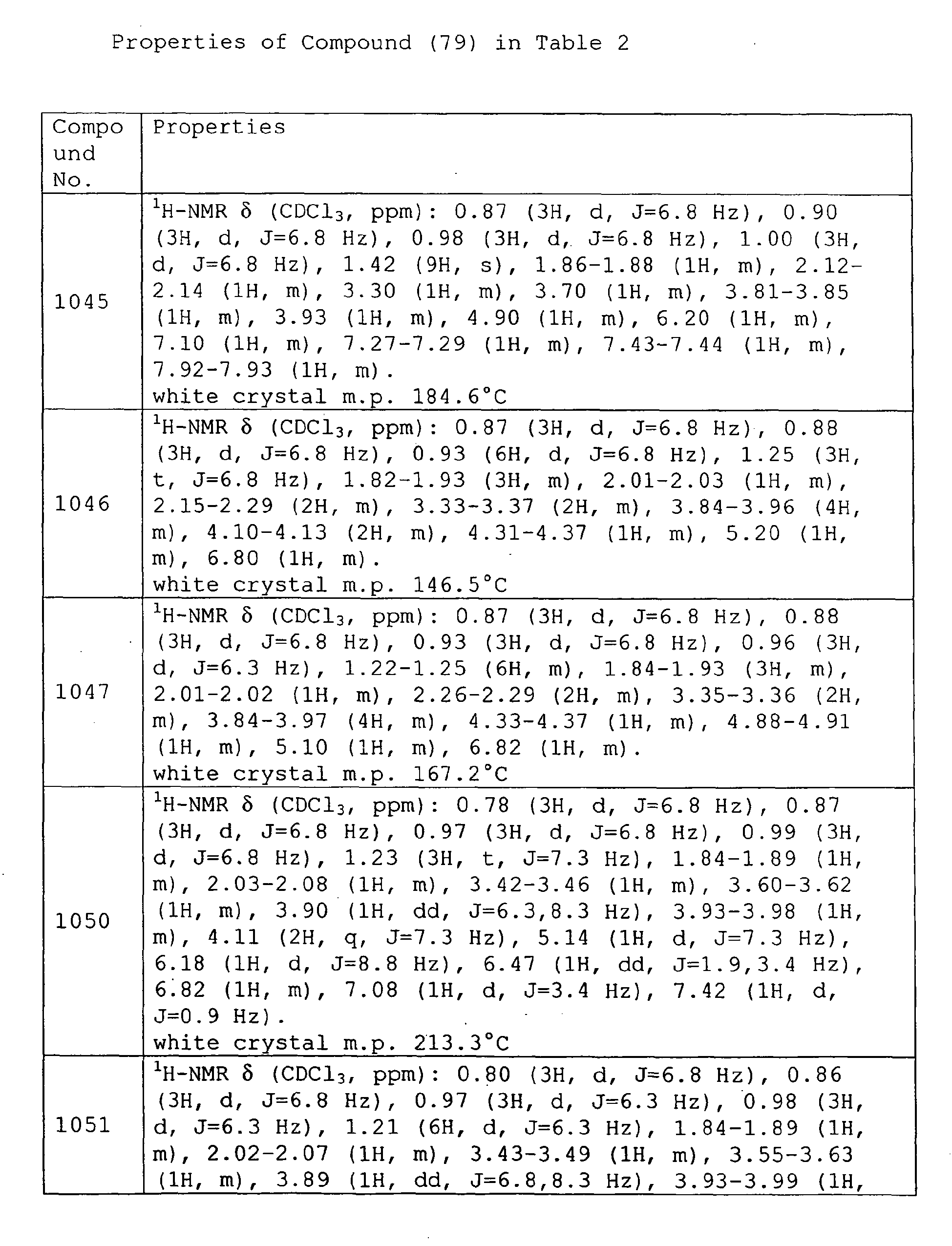

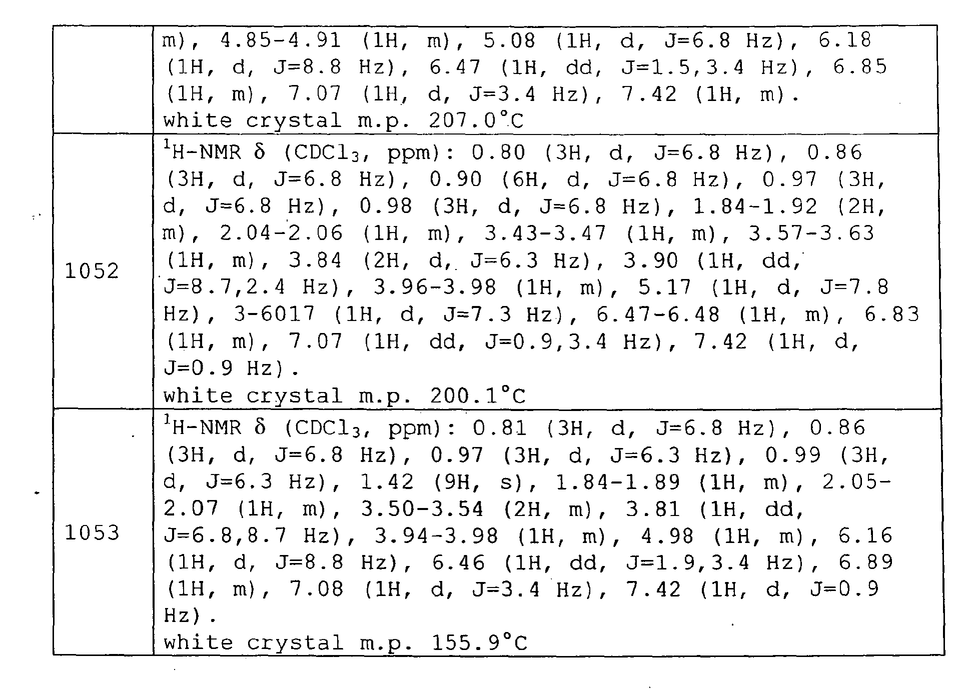

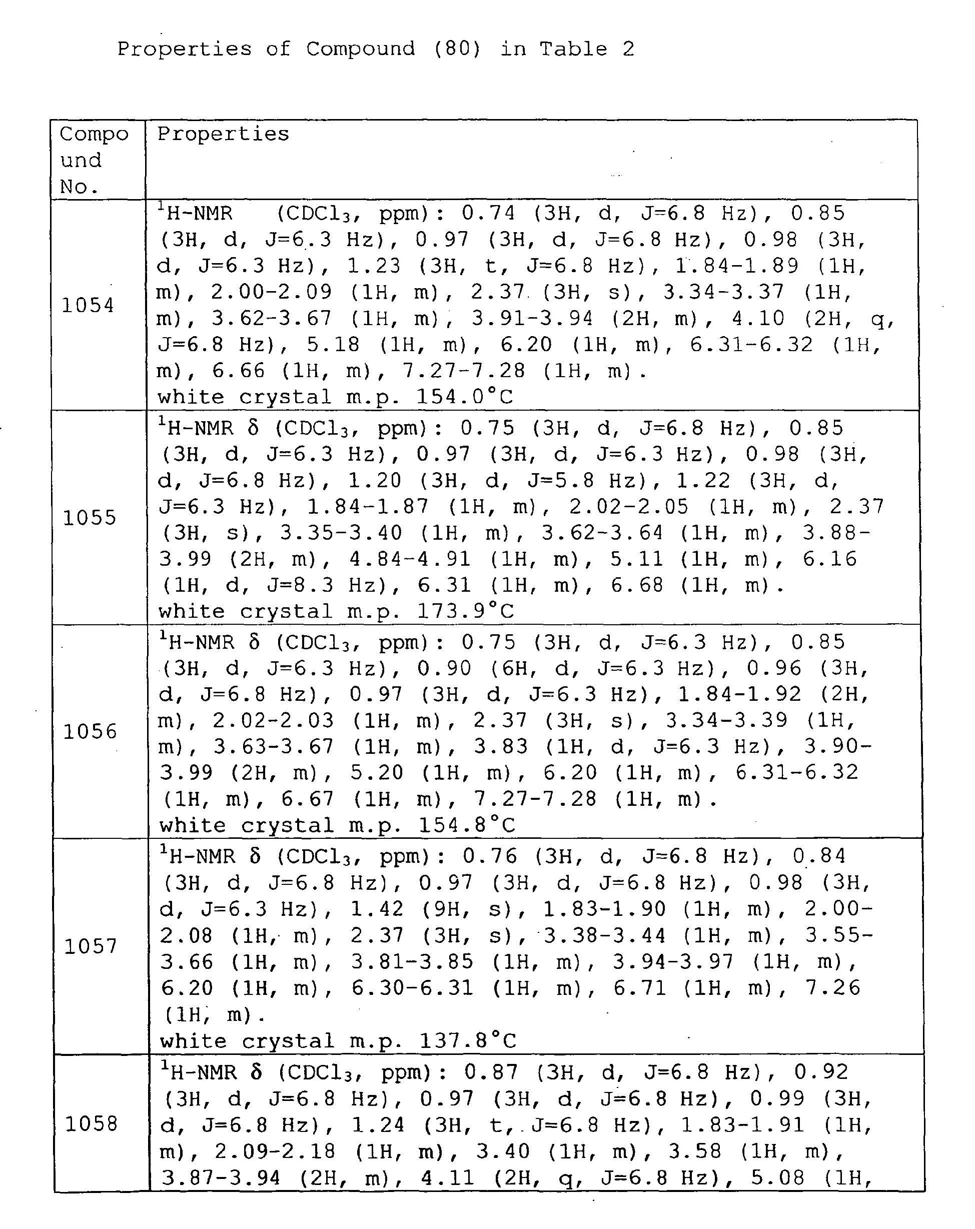

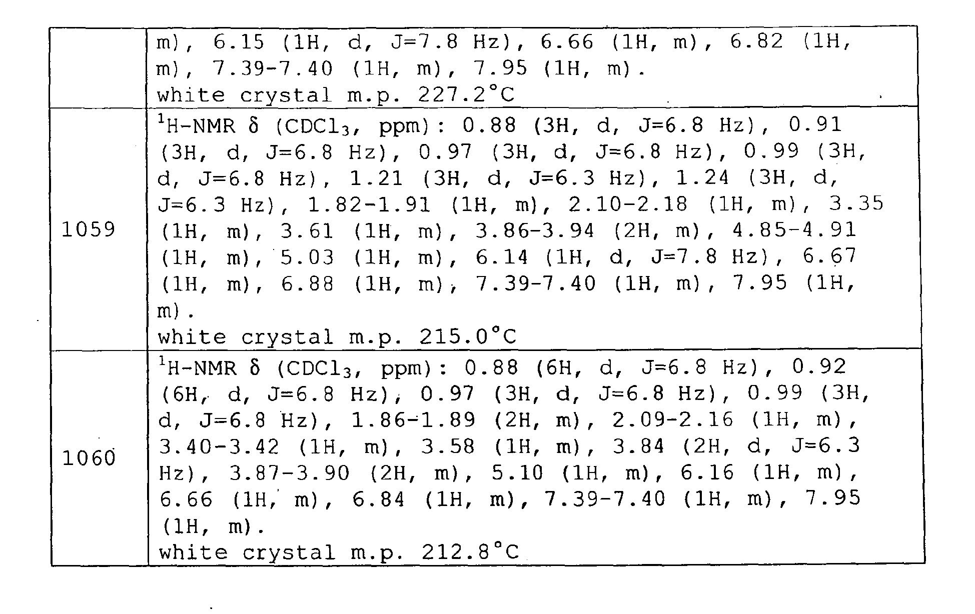

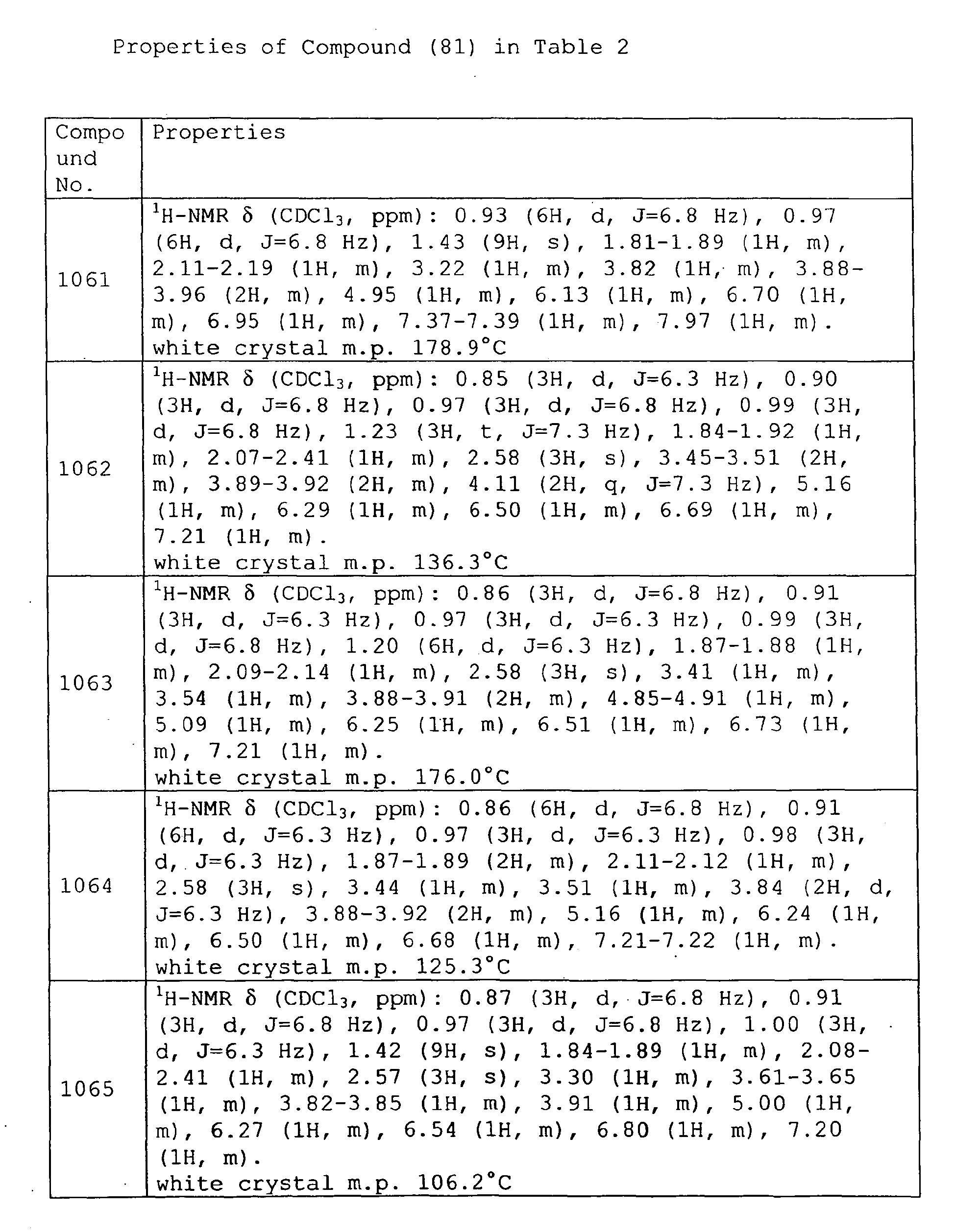

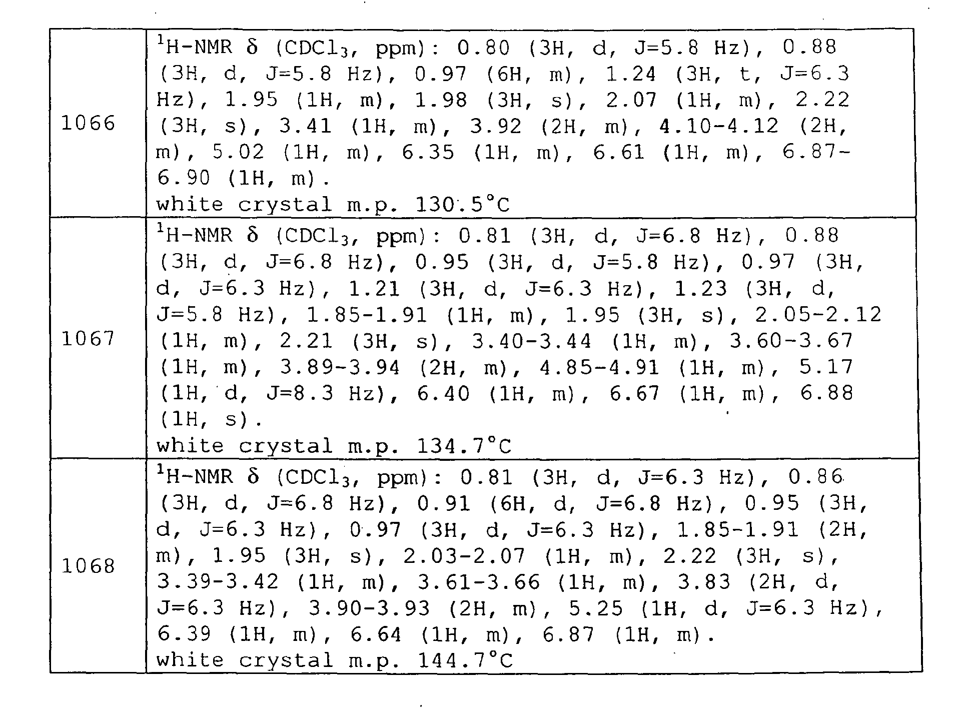

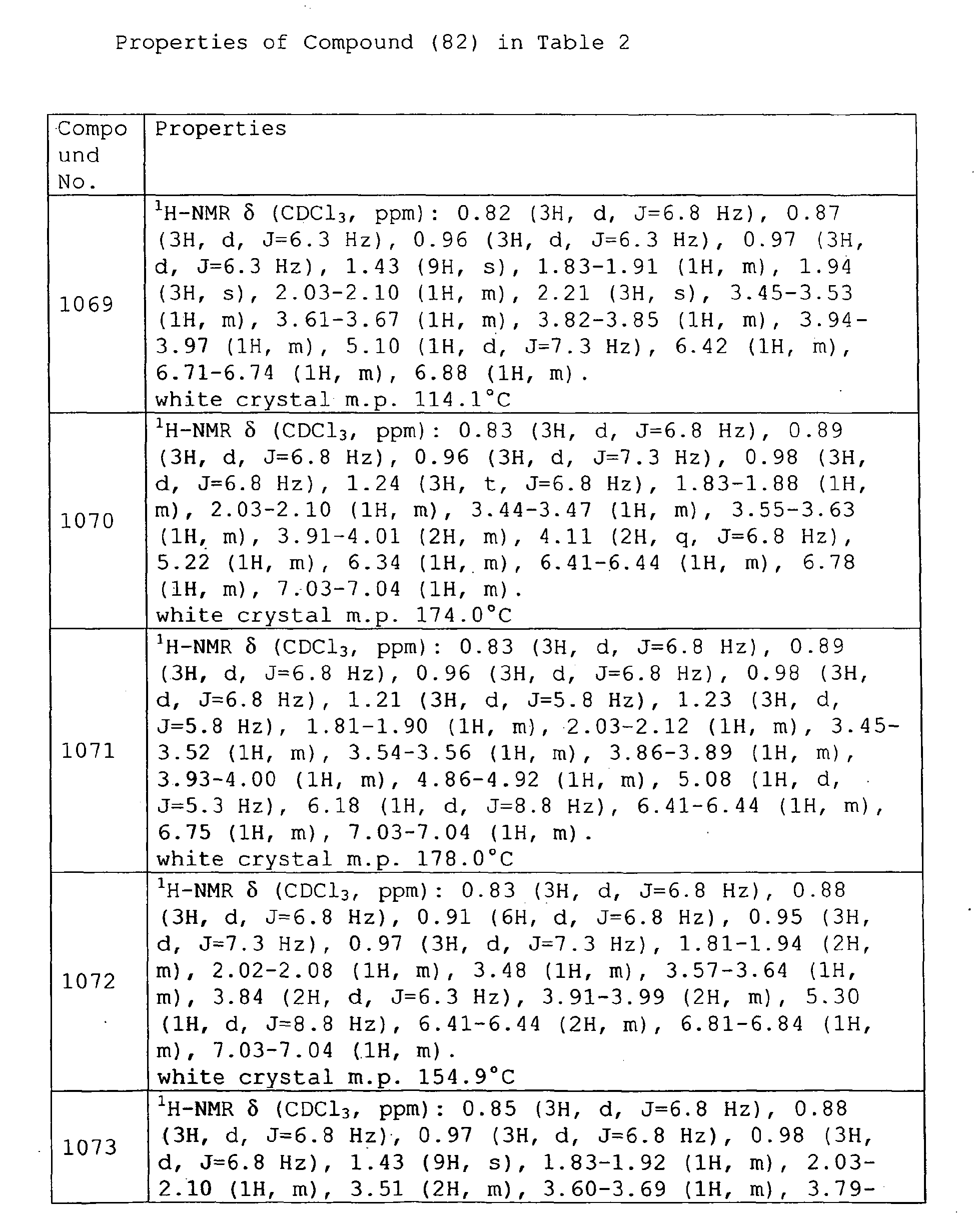

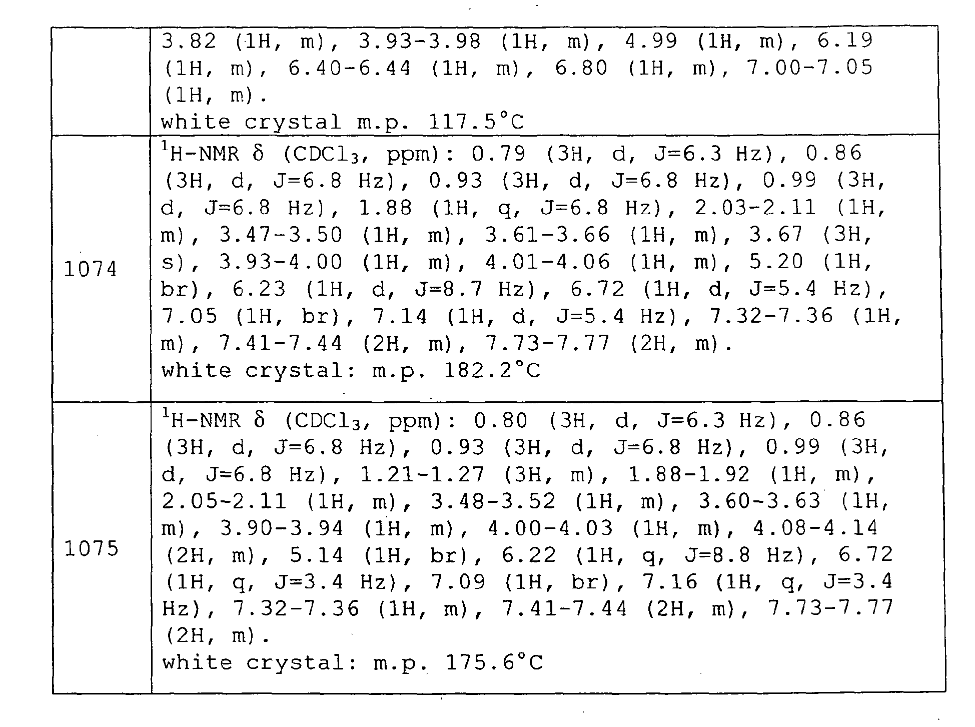

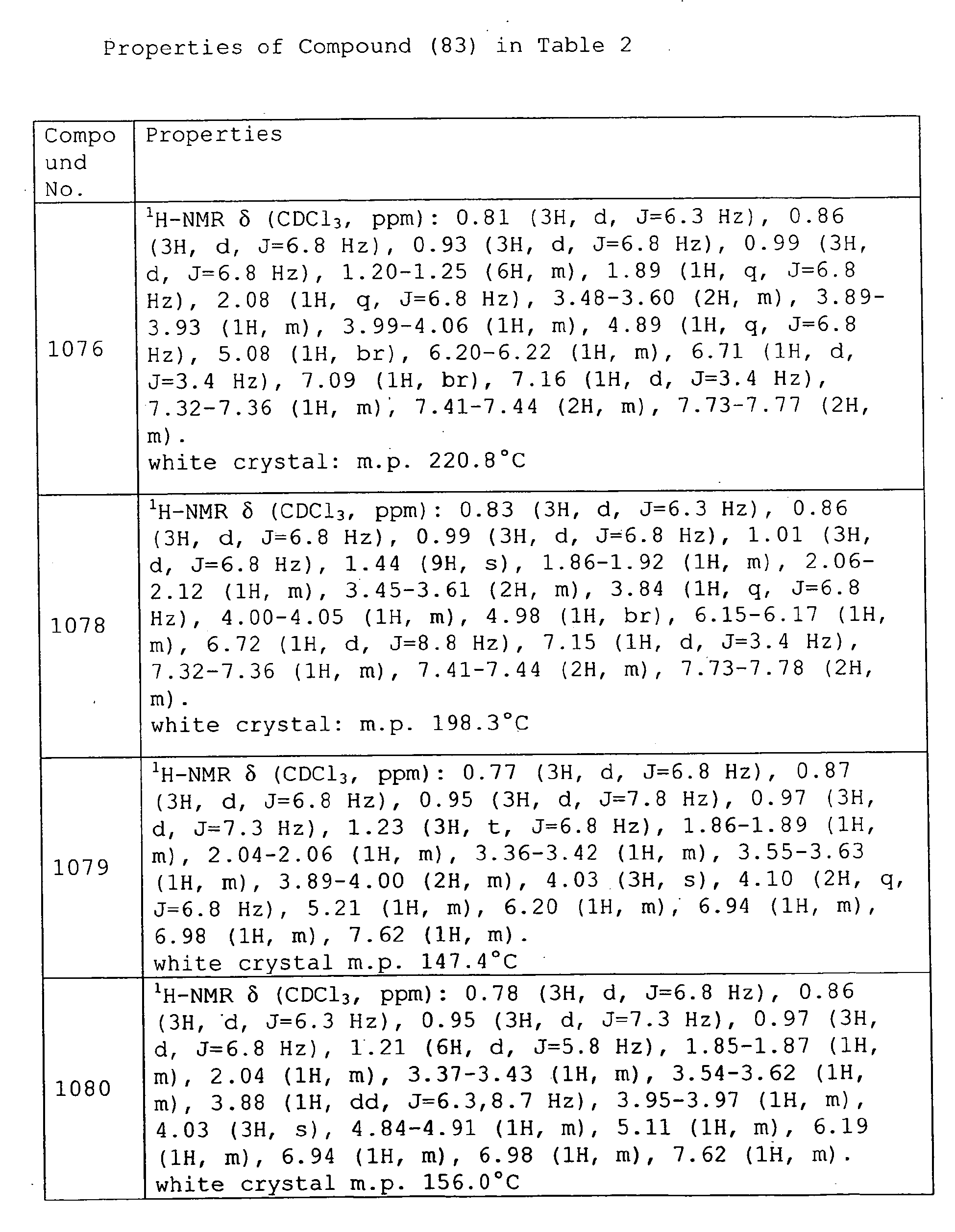

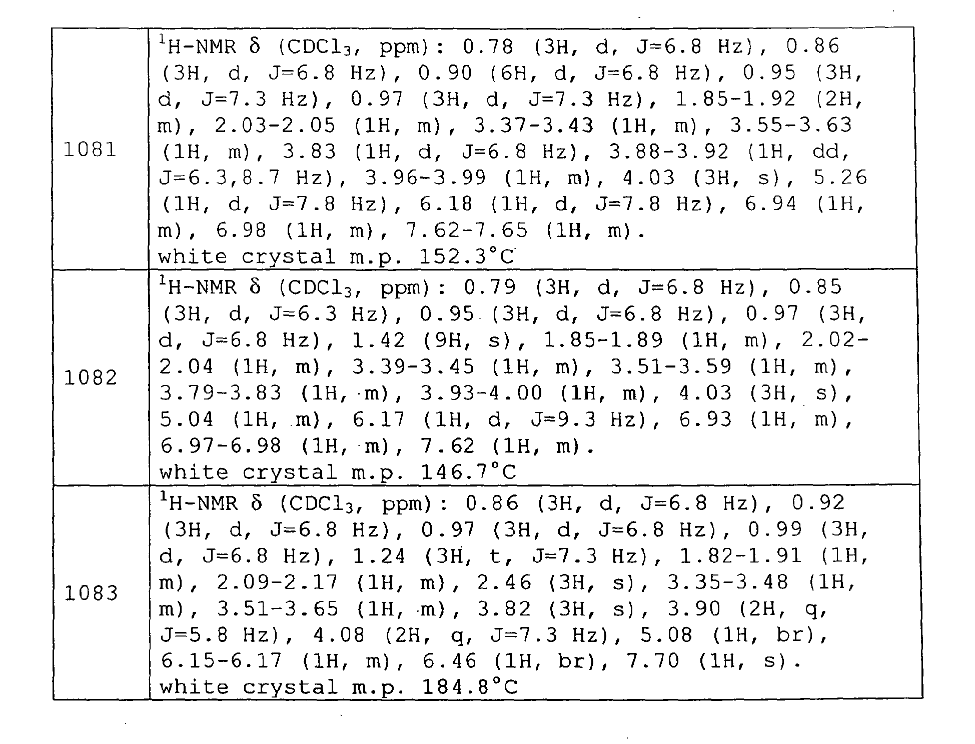

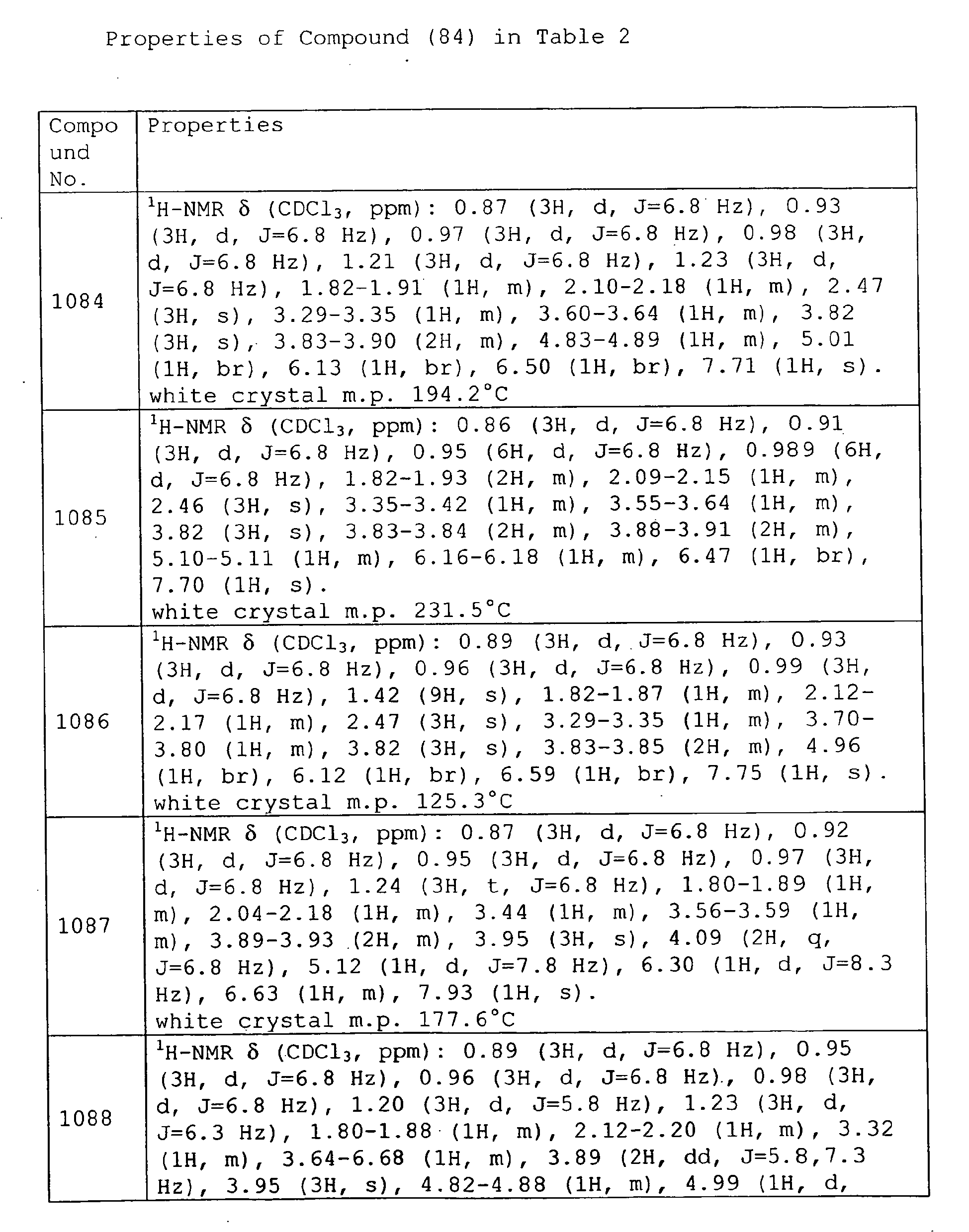

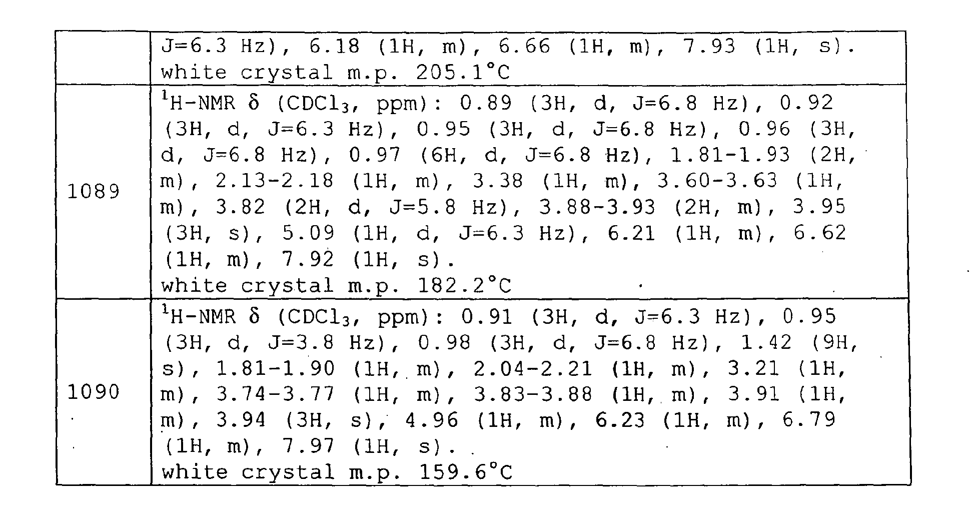

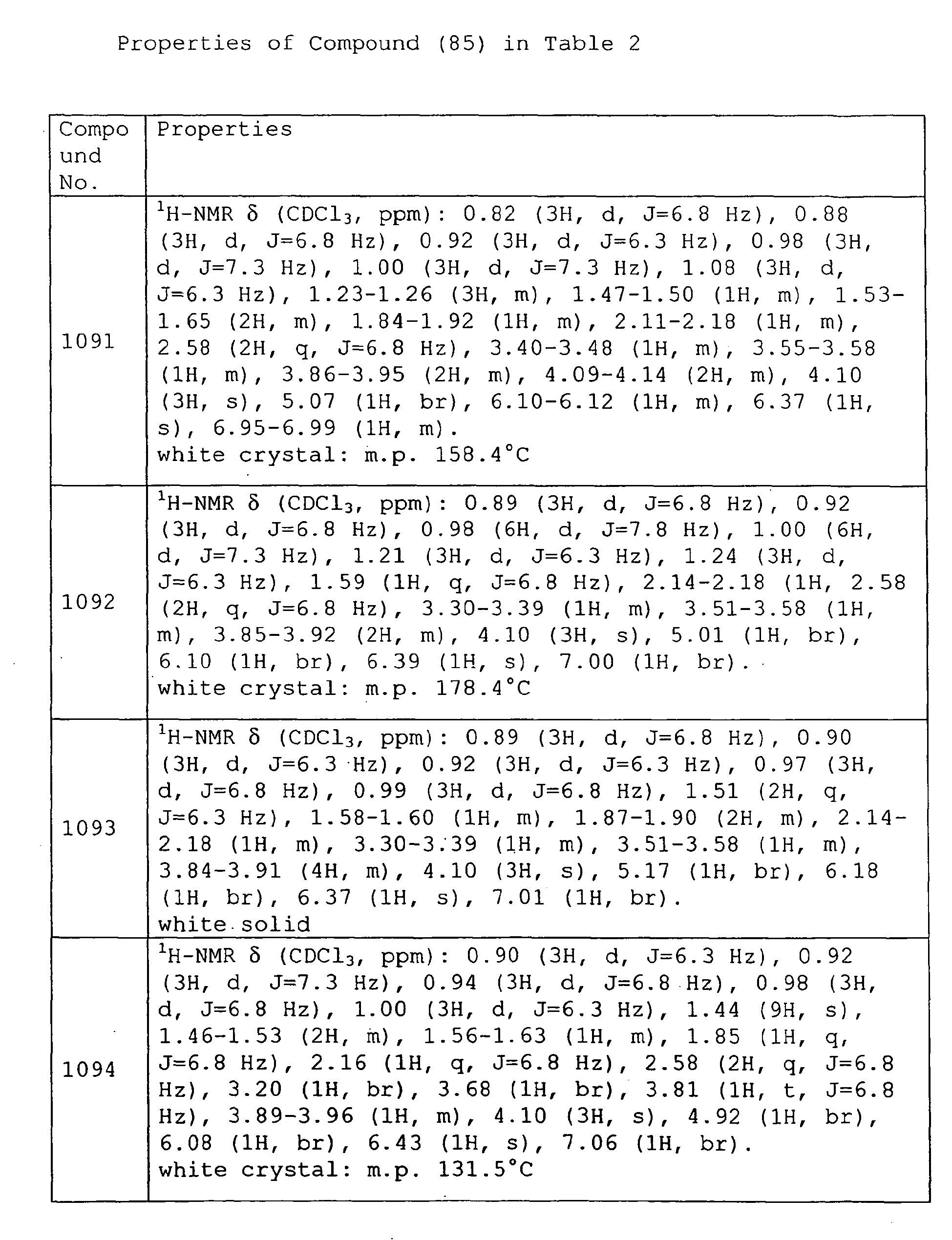

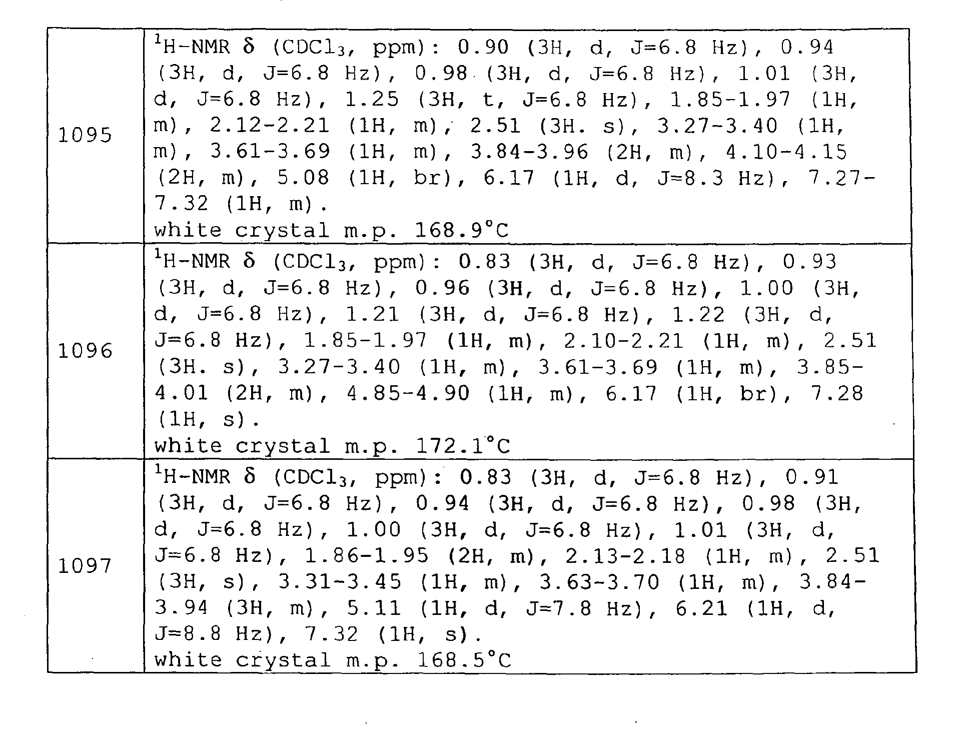

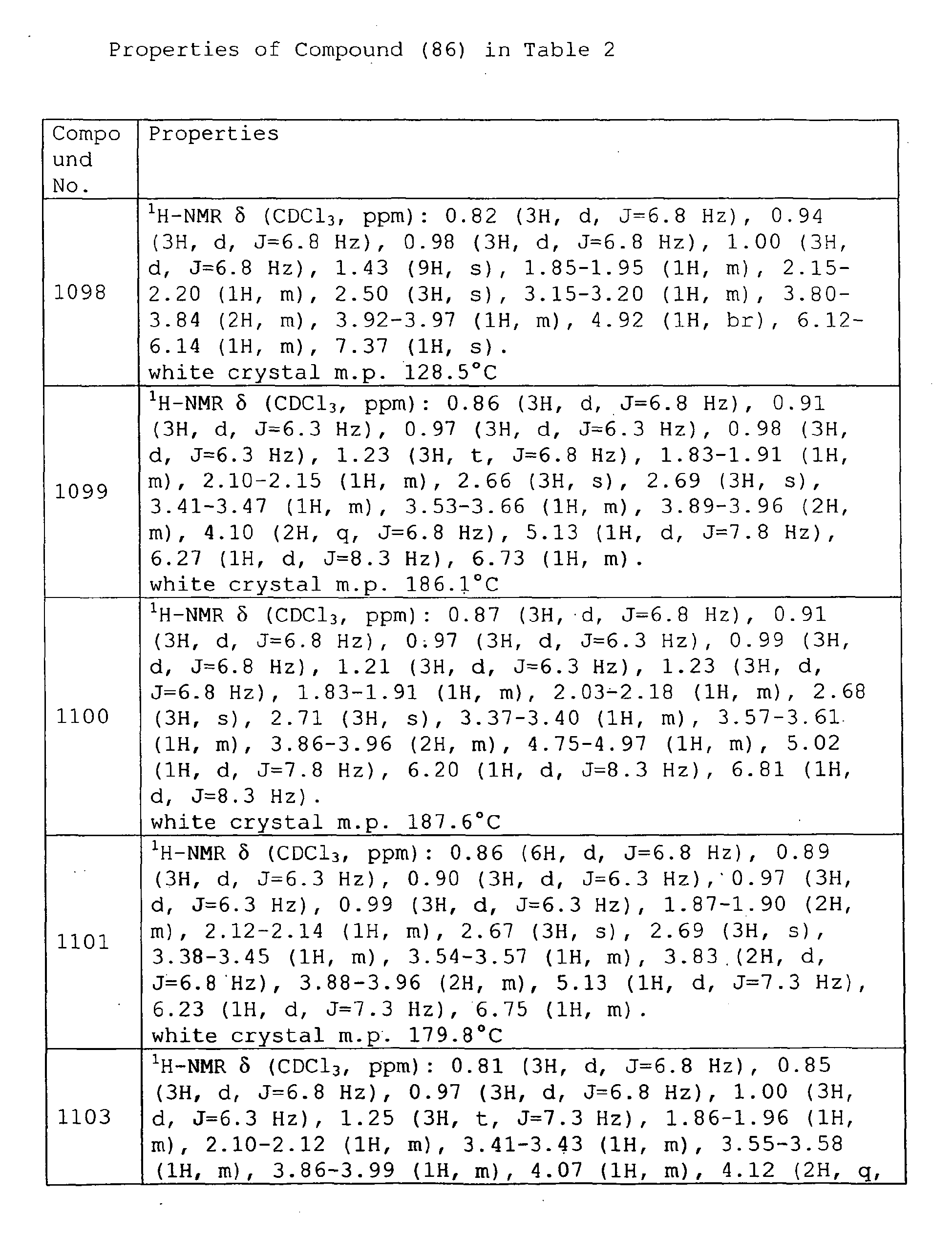

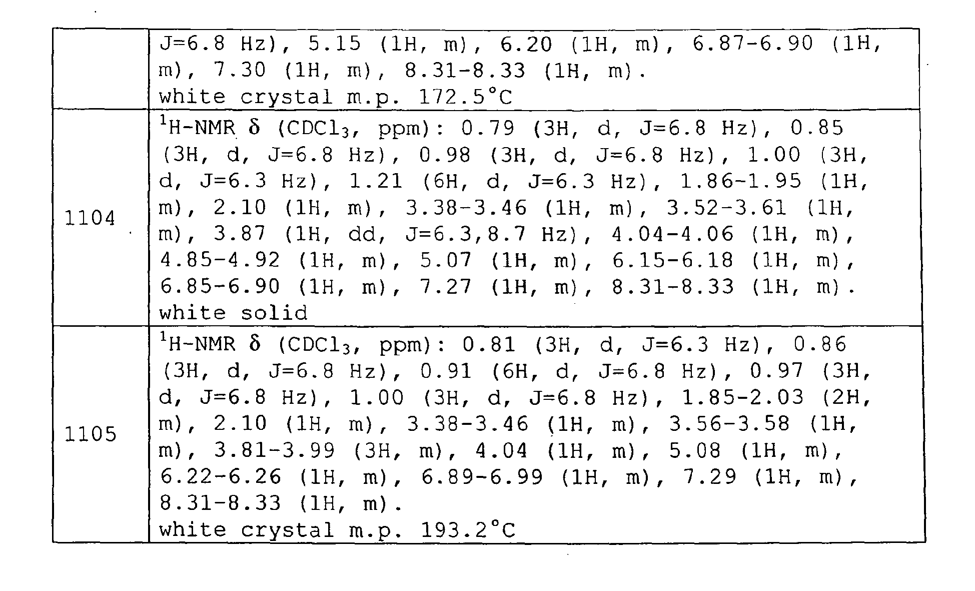

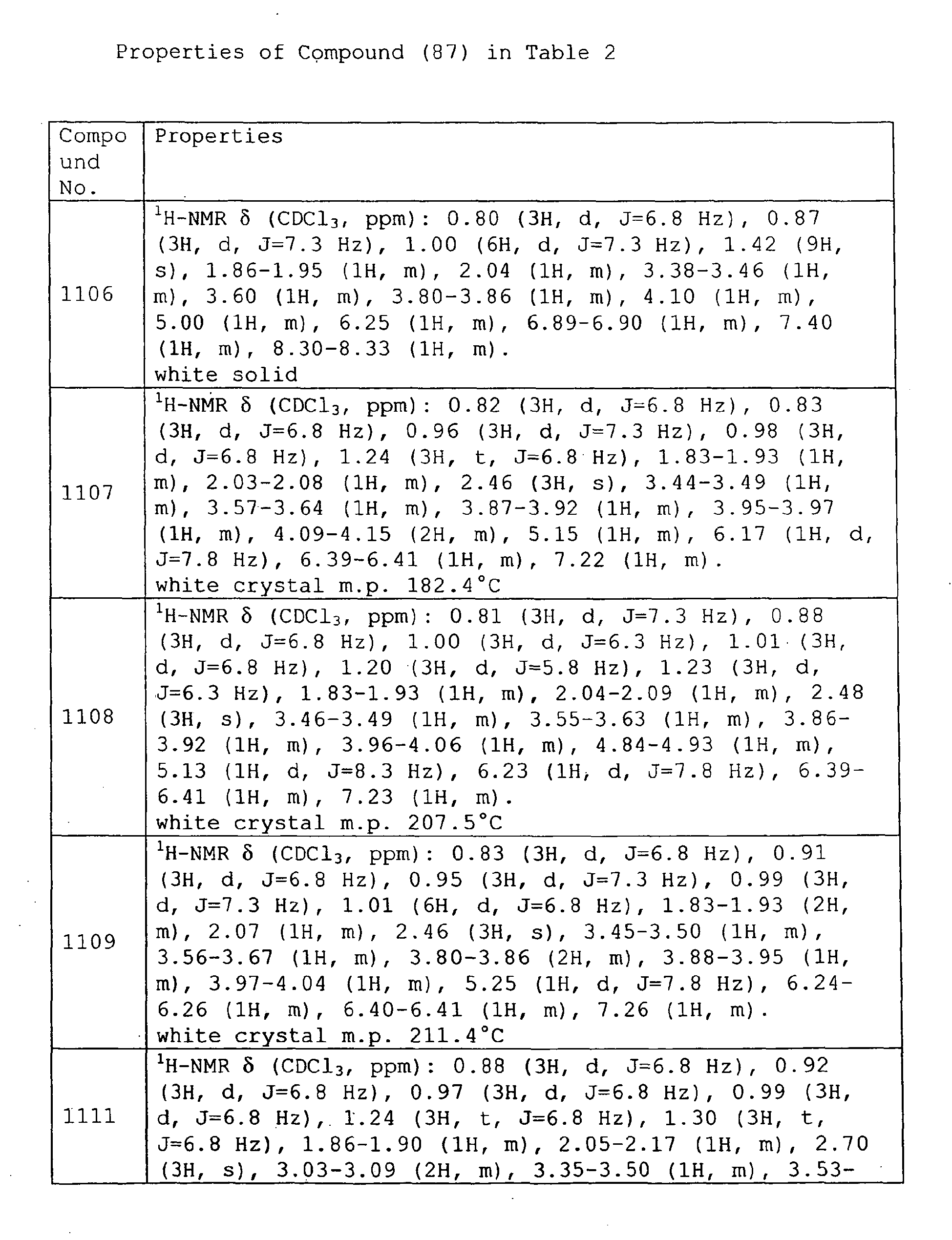

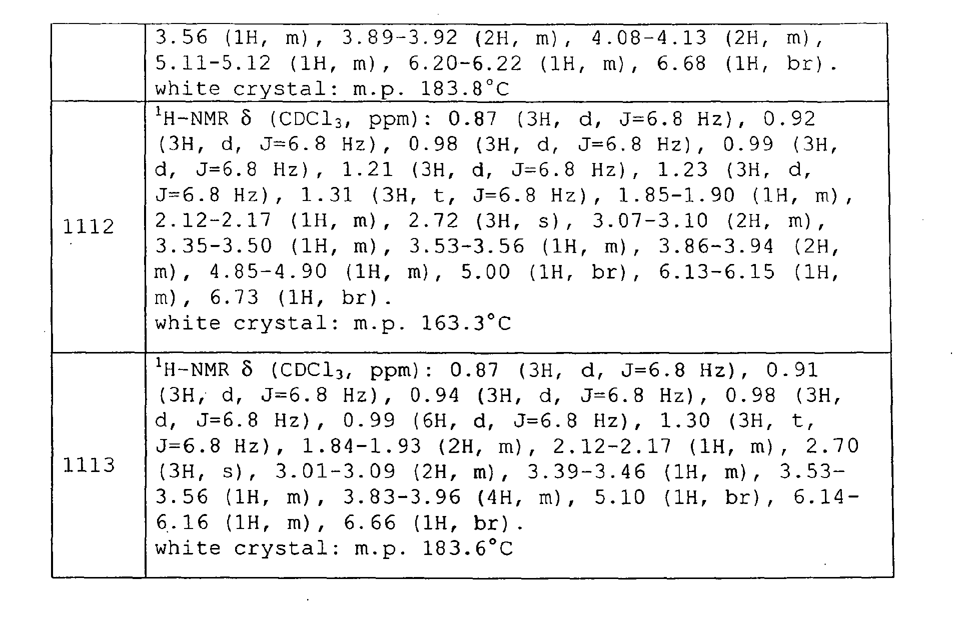

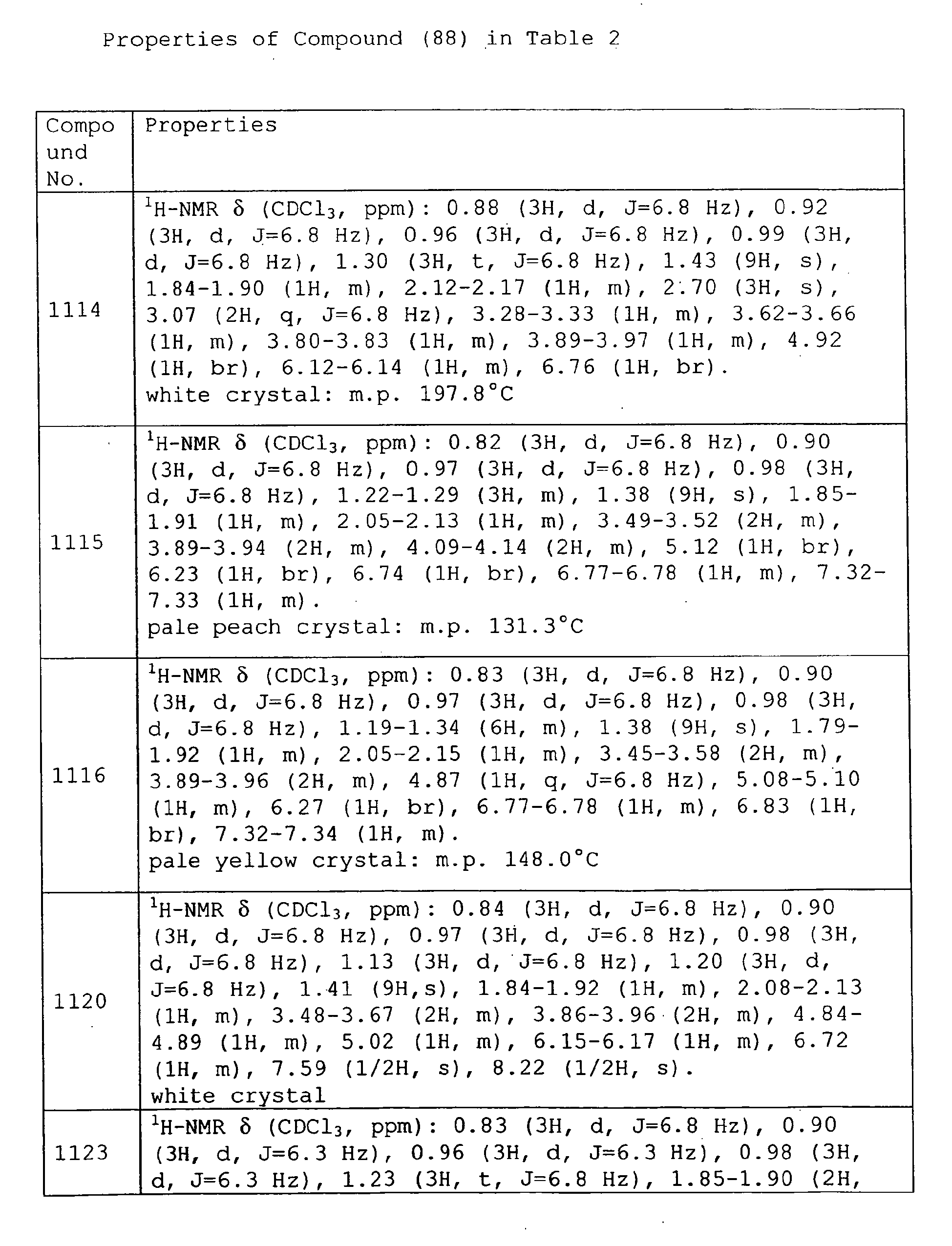

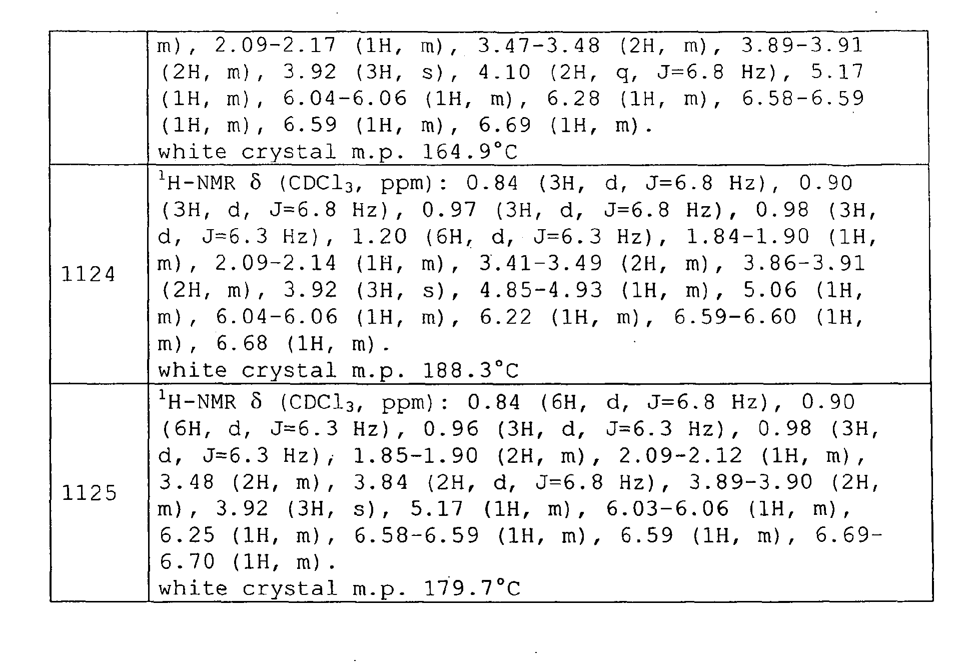

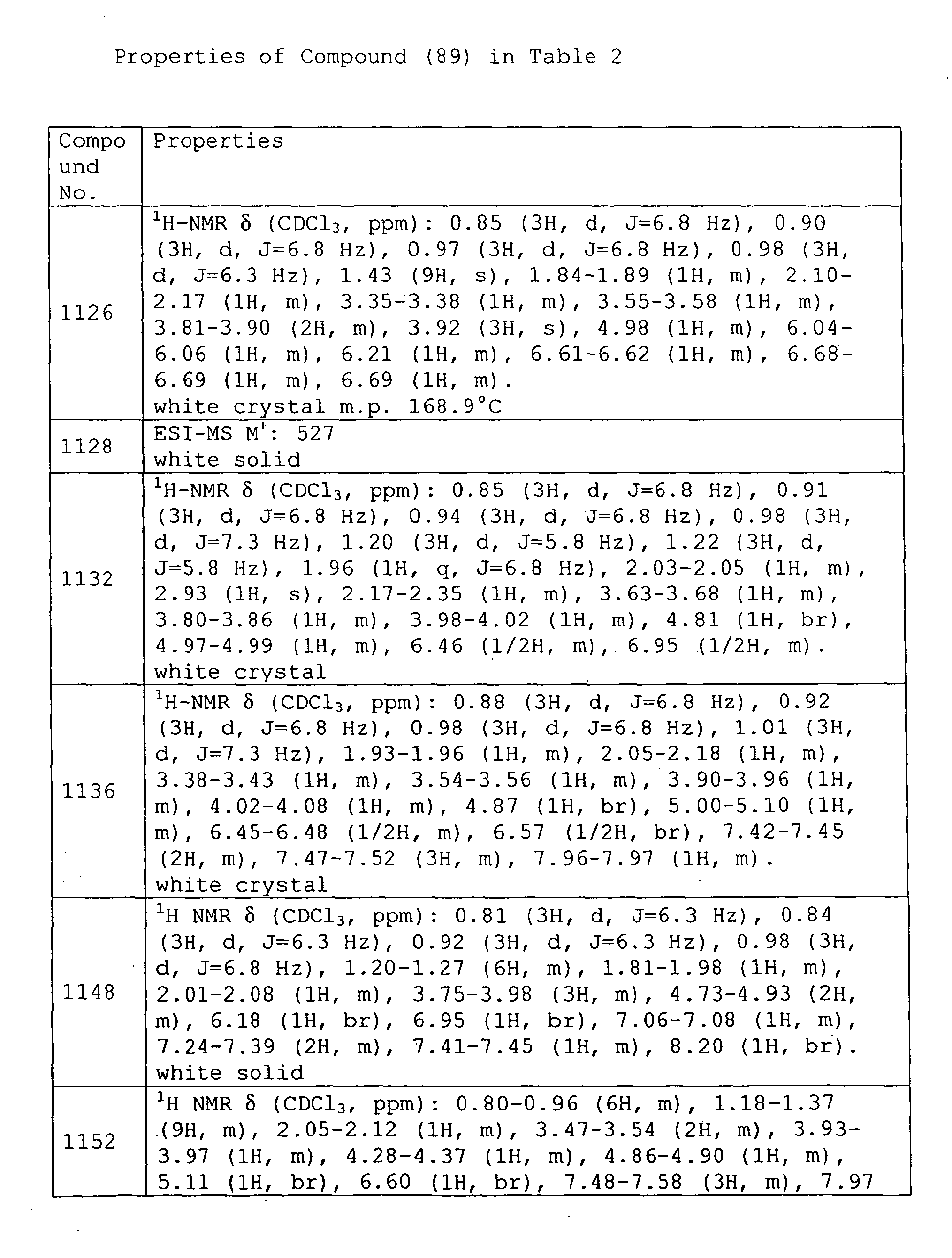

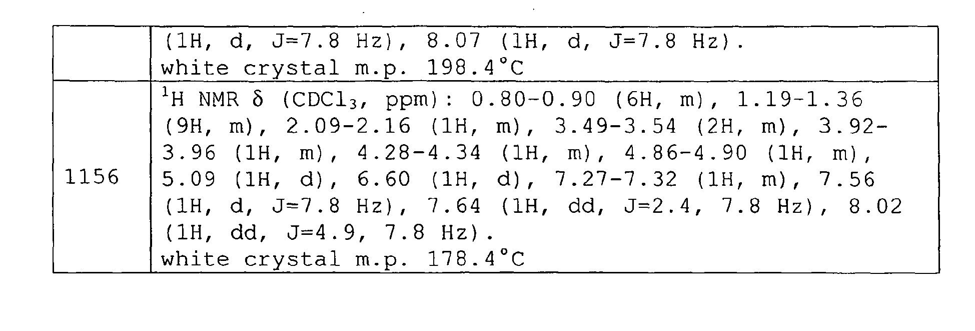

### Preparation Examples and Test Examples

Hereinafter, Preparation Examples and Fungicidal acitivity Test Examples of fungicides according to the invention are illustrated. In the following examples, "parts" refer to "parts by weight" or "% by weight".

### Preparation Example 1 (Powder)

2 parts of Compound No. 38 and 98 parts of clay were uniformly mixed and ground to obtain powder containing 2% of the active ingredients.

### Preparation Example 2 (Wettable powder)

10 parts of Compound No. 54, 70 parts of kaolin, 18 parts of white carbon and 2 parts of calcium alkylbenzene sulfonate were uniformly mixed and ground to obtain wettable powder in the form of uniform, fine powder containing 10% of the active ingredients.

### Preparation Example 3 (Wettable powder)

20 parts of Compound No. 152, 3 parts of calcium alkylbenzene sulfonate, 5 parts of polyoxyethylenenonylphenyl ether and 72 parts of white clay were uniformly mixed and ground to obtain wettable powder in the form of uniform, fine powder containing 20% of the active ingredients.

### Preparation Example 4 (Wettable powder)

50 parts of Compound No. 159, 1 parts of sodium lignin sulfonate, 5 parts of white carbon and 44 parts of diatomaceous earth were mixed and ground to obtain wettable powder containing 50% of the active ingredients.

### Preparation Example 5 (Flowable agent)

5 parts of Compound No. 163, 7 parts of propylene glycol, 4 parts of sodium lignin sulfonate, 2 parts of dioctylsulfosuccinate sodium salt, and 82 parts of water were wet ground with sand grinder to obtain flowable agent containing 5% of the active ingredients.

### Preparation Example 6 (Flowable agent)

10 parts of Compound No. 167, 7 parts of propylene glycol, 2 parts of sodium lignin sulfonate, 2 parts of dioctylsulfosuccinate sodium salt, and 79 parts of water were wet ground with sand grinder to obtain flowable agent containing 10% of the active ingredients.

### Preparation Example 7 (Flowable agent)

25 parts of Compound No. 307, 5 parts of propylene glycol, 5 parts of polyoxyethylene oleic acid ester, 5 parts of polyoxyethylene diallyl ether sulfate, 0.2 parts of antifoaming agent based silicone, and 59.8 parts of water were wet ground with sand grinder to obtain flowable agent containing 25% of the active ingredients.

### Preparation Example 8 (Powder)

2 parts of Compound No. 684 and 98 parts of clay were uniformly mixed and ground to obtain powder containing 2% of the active ingredients.

### Preparation Example 9 (Wettable powder)

10 parts of Compound No. 733, 70 parts of kaolin, 18 parts of white carbon and 2 parts of calcium alkylbenzene sulfonate were uniformly mixed and ground to obtain wettable powder in the form of uniform, fine powder containing 10% of the active ingredients.

### Preparation Example 10 (Wettable powder)

20 parts of Compound No. 696, 3 parts of calcium alkylbenzene sulfonate, 5 parts of polyoxyethylenenonylphenyl ether and 72 parts of white clay were uniformly mixed and ground to obtain wettable powder in the form of uniform, fine powder containing 20% of the active ingredients.

### Preparation Example 11 (Wettable powder)

50 parts of Compound No. 718, 1 parts of sodium lignin sulfonate, 5 parts of white carbon and 44 parts of diatomaceous earth were mixed and ground to obtain wettable powder containing 50% of the active ingredients.

### Preparation Example 12 (Flowable agent)

5 parts of Compound No. 689, 7 parts of propylene glycol, 4 parts of sodium lignin sulfonate, 2 parts of dioctylsulfosuccinate sodium salt, and 82 parts of water were wet ground with sand grinder to obtain flowable agent containing 5% of the active ingredients.

### Preparation Example 13 (Flowable agent)

10 parts of Compound No. 874, 7 parts of propylene glycol, 2 parts of sodium lignin sulfonate, 2 parts of dioctylsulfosuccinate sodium salt, and 79 parts of water were wet ground with sand grinder to obtain flowable agent containing 10% of the active ingredients.

### Preparation Example 14 (Flowable agent)

25 parts of Compound No. 848, 5 parts of propylene glycol, 5 parts of polyoxyethylene oleic acid ester, 5 parts of polyoxyethylene diallyl ether sulfate, 0.2 parts of antifoaming agent based silicone, and 59.8 parts of water were wet ground with sand grinder to obtain flowable agent containing 25% of the active ingredients.

The effect obtained from the fungicides of the invention is specifically illustrated with reference to Test Examples as follows. The agent used for comparision is the compound recited as agricultural and horticultural fungicides in the publication of W003008372, and it was subjected to experiment as prepared in the same manner as the experimental compounds.

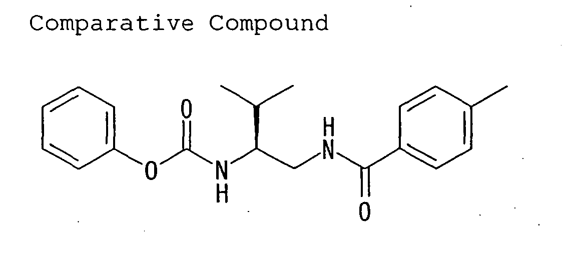

### Test Example 1: Controlling effect against tomato late blight

A solution of the compound in acetone was diluted with water to 250 ppm, and the solution was sprayed to the tomato seedling (breed: Sekaiichi, one per plastic pot having a diameter of 7.5 cm) at 4-leaf stage in an amount of 10 mL of the compound/pot. After air-drying, the pot was transferred to a moist chamber at 18°C (12-hour light-dark light cycle), and inoculated by spray with a zoosporic suspension of Phytophthora infestans. On the 4^{th} day after inoculation the infected area rate for 4 leaves of leaflet was examined. Evaluation was carried out by the following criteria.

The infected area rate for the untreated groups in the examination was not less than 60%.
A: Infected area rate of less than 5%
B: Infected area rate of 5% to less than 50%
C: Infected area rate of not less than 50%

**Table 3**

| Controlling effect against tomato late blight (1) | |
|---|---|
| Compound No. | Effect |
| Comparative Compound | C |
| 1 | B |
| 4 | B |
| 6 | B |
| 18 | B |
| 26 | A |
| 38 | A |
| 39 | B |
| 40 | A |
| 42 | A |
| 47 | B |
| 50 | A |
| 51 | A |
| 52 | A |
| 54 | A |
| 55 | A |
| 56 | A |
| 58 | B |
| 59 | B |
| 60 | B |
| 69 | A |
| 75 | A |
| 76 | A |
| 99 | B |
| 100 | A |
| 101 | B |
| 123 | A |
| 124 | A |
| 125 | A |
| 126 | A |
| 127 | A |
| 128 | A |
| 129 | B |
| 135 | A |
| 138 | A |
| 139 | A |
| 140 | A |
| 141 | A |
| 143 | A |
| 147 | A |
| 148 | B |

| Controlling effect against tomato late blight (2) | |
|---|---|
| Compound No. | Effect |
| 151 | A |
| 152 | A |
| 153 | A |
| 154 | A |
| 157 | A |
| 159 | A |
| 160 | A |
| 161 | A |
| 162 | A |
| 163 | A |
| 164 | A |
| 166 | A |
| 167 | A |
| 168 | A |
| 169 | A |
| 171 | A |
| 172 | A |
| 173 | A |
| 174 | A |
| 175 | A |
| 176 | A |
| 177 | A |
| 178 | B |
| 180 | B |
| 182 | A |
| 183 | A |
| 184 | A |
| 185 | A |
| 186 | B |
| 187 | A |
| 188 | A |
| 191 | A |
| 192 | A |
| 193 | A |
| 194 | A |
| 196 | A |
| 198 | A |
| 200 | A |
| 201 | A |
| 202 | A |
| 203 | A |

| Controlling effect against tomato late blight (3) | |
|---|---|
| Compound No. | Effect |
| 204 | A |
| 205 | A |
| 206 | A |
| 208 | A |
| 209 | B |
| 210 | B |
| 213 | A |
| 214 | A |
| 218 | B |
| 220 | B |
| 221 | B |
| 222 | A |
| 224 | A |
| 225 | A |
| 226 | A |
| 227 | B |
| 228 | B |
| 229 | A |
| 230 | B |
| 231 | B |
| 232 | B |
| 237 | B |
| 238 | B |
| 239 | B |
| 241 | A |
| 242 | B |
| 243 | A |
| 247 | B |
| 248 | B |
| 257 | A |
| 261 | B |
| 274 | A |
| 276 | B |
| 278 | A |
| 279 | A |
| 281 | B |
| 283 | A |
| 287 | A |
| 291 | A |
| 292 | A |
| 293 | A |

| Controlling effect against tomato late blight (4) | |
|---|---|
| Compound No. | Effect |
| 294 | A |
| 295 | A |
| 296 | A |
| 297 | A |
| 298 | B |
| 299 | A |
| 300 | B |
| 301 | B |
| 302 | A |
| 303 | A |
| 304 | A |
| 305 | A |
| 306 | A |
| 307 | A |
| 308 | A |
| 309 | A |
| 310 | A |
| 311 | A |
| 312 | B |
| 313 | A |
| 314 | A |
| 315 | A |
| 316 | B |
| 317 | A |
| 328 | B |
| 329 | B |
| 330 | B |
| 332 | A |
| 335 | A |
| 336 | A |
| 337 | A |
| 338 | A |
| 339 | A |
| 340 | A |
| 341 | A |
| 342 | A |
| 343 | A |
| 344 | A |
| 345 | A |
| 346 | A |
| 347 | A |

| Controlling effect against tomato late blight (5) | |
|---|---|
| Compound No. | Effect |
| 348 | A |
| 349 | A |
| 350 | A |
| 352 | A |
| 388 | B |
| 389 | B |
| 390 | B |
| 392 | B |
| 393 | B |
| 395 | B |
| 396 | B |
| 397 | B |
| 398 | A |
| 400 | A |
| 404 | B |
| 408 | A |
| 411 | A |
| 412 | A |
| 413 | A |
| 414 | A |
| 416 | A |
| 420 | A |
| 424 | B |
| 427 | B |

| Controlling effect against tomato late blight (6) | |
|---|---|
| Compound No. | Effect |
| 431 | A |
| 432 | A |
| 433 | A |
| 435 | A |
| 436 | A |
| 437 | B |
| 444 | A |
| 447 | A |
| 448 | A |
| 449 | A |
| 450 | A |
| 451 | A |
| 452 | A |
| 453 | A |
| 454 | A |
| 455 | B |
| 456 | A |
| 458 | B |
| 462 | A |
| 466 | B |
| 470 | B |
| 478 | B |
| 482 | B |
| 486 | B |
| 490 | A |
| 494 | A |
| 498 | A |
| 506 | B |
| 514 | A |
| 526 | B |
| 538 | B |
| 554 | A |
| 565 | A |
| 566 | B |
| 575 | B |
| 579 | B |
| 583 | B |
| 587 | A |

| Controlling effect against tomato late blight (7) | |
|---|---|
| Compound No. | Effect |
| 599 | A |
| 601 | B |
| 603 | B |
| 638 | B |
| 639 | A |
| 640 | A |
| 641 | A |
| 642 | B |
| 643 | A |
| 644 | A |
| 645 | A |
| 646 | A |
| 647 | A |
| 649 | A |
| 650 | A |
| 651 | A |
| 652 | A |
| 665 | A |
| 666 | A |
| 667 | A |
| 669 | A |
| 675 | A |
| 678 | B |
| 684 | A |
| 685 | A |
| 686 | A |
| 688 | A |
| 689 | A |
| 690 | A |
| 691 | A |
| 693 | A |
| 694 | A |
| 695 | A |
| 696 | A |
| 697 | A |
| 698 | A |
| 699 | A |
| 700 | A |
| 701 | A |
| 702 | A |

| Controlling effect against tomato late blight (8) | |
|---|---|
| Compound No. | Effect |
| 703 | A |
| 704 | A |
| 705 | A |
| 708 | A |
| 709 | A |
| 710 | A |
| 711 | A |
| 712 | A |
| 713 | A |
| 714 | A |
| 715 | A |
| 717 | A |
| 718 | A |
| 719 | A |
| 733 | A |
| 735 | A |
| 737 | A |
| 765 | A |
| 766 | A |
| 767 | A |
| 771 | A |
| 772 | A |
| 773 | A |
| 774 | A |
| 775 | A |
| 776 | A |
| 777 | A |
| 778 | A |
| 779 | B |
| 780 | A |
| 781 | A |
| 782 | A |
| 783 | A |
| 784 | A |
| 785 | B |
| 786 | A |
| 787 | B |
| 788 | A |

| Controlling effect against tomato late blight (9) | |
|---|---|
| Compound No. | Effect |
| 789 | A |
| 790 | A |
| 793 | A |
| 794 | A |
| 798 | A |
| 799 | A |
| 800 | A |
| 801 | A |
| 802 | A |
| 803 | A |
| 810 | A |
| 811 | A |
| 812 | B |
| 814 | A |
| 815 | A |
| 816 | B |
| 818 | A |
| 819 | A |
| 820 | A |
| 823 | A |
| 826 | A |
| 827 | A |
| 828 | A |
| 830 | A |
| 831 | A |
| 832 | A |
| 834 | A |
| 835 | A |
| 836 | A |
| 838 | A |
| 839 | A |
| 842 | B |
| 843 | A |
| 844 | A |
| 846 | A |
| 847 | A |
| 848 | A |
| 849 | A |
| 850 | A |
| 857 | A |
| 861 | B |

| Controlling effect against tomato late blight (10) | |
|---|---|
| Compound No. | Effect |
| 862 | B |
| 868 | A |
| 869 | A |
| 870 | A |
| 872 | A |
| 873 | A |
| 874 | A |
| 875 | A |
| 876 | A |
| 878 | A |
| 882 | A |
| 886 | A |
| 889 | A |
| 890 | A |
| 891 | A |
| 895 | B |
| 899 | A |
| 902 | A |
| 903 | A |
| 906 | B |
| 907 | A |
| 908 | A |
| 910 | A |
| 911 | A |
| 912 | A |
| 914 | A |
| 915 | A |
| 916 | A |
| 917 | B |
| 921 | B |
| 924 | A |
| 929 | A |
| 933 | A |
| 937 | B |
| 938 | B |
| 939 | B |
| 940 | B |
| 941 | A |
| 942 | A |
| 943 | A |
| 945 | B |

| Controlling effect against tomato late blight (11) | |
|---|---|
| Compound No. | Effect |
| 948 | B |
| 949 | A |
| 951 | A |
| 952 | A |
| 953 | A |
| 954 | A |
| 955 | A |
| 960 | A |
| 963 | A |
| 968 | A |
| 969 | A |
| 970 | A |
| 971 | A |
| 972 | A |
| 973 | A |
| 981 | A |
| 982 | A |
| 983 | A |
| 984 | A |
| 985 | A |
| 986 | A |
| 987 | A |
| 988 | A |
| 989 | A |
| 990 | A |
| 991 | A |
| 993 | A |
| 994 | A |
| 995 | B |
| 997 | A |
| 998 | A |
| 999 | A |
| 1000 | A |
| 1005 | B |
| 1008 | B |
| 1009 | A |
| 1010 | A |
| 1011 | A |
| 1013 | B |
| 1014 | B |
| 1015 | B |

| Controlling effect against tomato late blight (12) | |
|---|---|
| Compound No. | Effect |
| 1017 | B |
| 1019 | A |
| 1020 | B |
| 1021 | B |
| 1022 | B |
| 1023 | B |
| 1024 | B |
| 1025 | B |
| 1026 | A |
| 1027 | A |
| 1028 | A |
| 1029 | A |
| 1030 | A |
| 1031 | A |
| 1032 | A |
| 1033 | A |
| 1035 | B |
| 1042 | B |
| 1043 | A |
| 1044 | A |
| 1045 | A |
| 1050 | B |
| 1051 | A |
| 1052 | B |
| 1053 | A |
| 1055 | A |
| 1057 | B |
| 1066 | A |
| 1067 | A |
| 1068 | A |
| 1069 | A |
| 1070 | A |
| 1071 | A |
| 1072 | A |
| 1073 | A |
| 1074 | A |
| 1075 | A |
| 1076 | A |
| 1078 | A |
| 1080 | A |
| 1082 | B |

| Controlling effect against tomato late blight (13) | |
|---|---|
| Compound No. | Effect |
| 1093 | B |
| 1094 | B |
| 1097 | B |
| 1100 | B |
| 1104 | B |
| 1105 | B |
| 1106 | B |
| 1108 | B |
| 1109 | B |
| 1110 | B |
| 1111 | A |
| 1112 | A |
| 1113 | A |
| 1114 | A |
| 1115 | A |
| 1116 | A |
| 1120 | A |
| 1124 | B |
| 1125 | A |
| 1126 | B |
| 1148 | A |
| 1152 | A |
| 1156 | A |

### Test Example 2: Controlling effect against cucumber downy mildew

A solution of the compound in acetone was diluted with water to 250ppm, and the solution was sprayed to the cucumber seedling (breed: Sagamihangbak, two per plastic pot having a diameter of 7.5 cm) at 1-leaf stage in an amount of 10mL of the compound/pot. After air-drying, the pot was transferred to a moist chamber provided in the greenhouse, and inoculated by spray with a zoosporic suspension of Pseudoperonospora cubensis. On the 7^{th} day after inoculation the infected area rate for the leaves was examined. Evaluation was carried out by the following criteria.

The infected area rate for untreated groups in the examination was 60 to 80%.
A: the infected area rate of less than 5%
B: the infected area rate of 5% to less than 50%
C: the infected area rate of not less than 50%

**Table 4**

| Controlling effect against cucumber downy mildew (1) | |
|---|---|
| Compound No. | Effect |
| Comparative Compound | C |
| 1 | B |
| 2 | B |
| 3 | B |
| 4 | B |
| 6 | B |
| 10 | A |
| 22 | B |
| 38 | A |
| 39 | B |
| 40 | A |
| 42 | A |
| 47 | B |
| 50 | A |
| 51 | A |
| 52 | A |
| 54 | A |
| 55 | A |
| 56 | A |
| 69 | A |
| 75 | B |
| 121 | B |
| 123 | A |
| 124 | A |
| 125 | A |
| 126 | A |
| 129 | B |
| 131 | A |
| 135 | B |
| 138 | A |
| 139 | A |
| 140 | A |
| 141 | B |
| 143 | B |
| 145 | B |
| 147 | B |
| 148 | B |
| 151 | B |
| 152 | A |
| 153 | A |
| 154 | A |

| Controlling effect against cucumber downy mildew (2) | |
|---|---|
| Compound No. | Effect |
| 157 | A |
| 159 | A |
| 160 | A |
| 161 | A |
| 162 | A |
| 163 | A |
| 164 | A |
| 166 | A |
| 167 | A |
| 168 | A |
| 169 | A |
| 171 | A |
| 172 | A |
| 173 | A |
| 174 | A |
| 175 | B |
| 176 | A |
| 177 | A |
| 182 | A |
| 183 | A |
| 184 | B |
| 185 | A |
| 186 | A |
| 187 | A |
| 188 | A |
| 191 | B |
| 192 | A |
| 193 | A |
| 194 | A |
| 196 | B |
| 197 | B |
| 198 | B |
| 200 | A |
| 201 | A |
| 202 | A |
| 203 | A |
| 204 | A |
| 205 | A |
| 206 | A |
| 208 | A |
| 209 | B |

| Controlling effect against cucumber downy mildew (3) | |
|---|---|
| Compound No. | Effect |
| 210 | B |
| 213 | A |
| 214 | A |
| 218 | B |
| 221 | B |
| 222 | B |
| 224 | A |
| 225 | A |
| 226 | A |
| 227 | B |
| 228 | B |
| 229 | B |
| 230 | B |
| 231 | B |
| 232 | B |
| 237 | B |
| 238 | B |
| 239 | B |
| 241 | A |
| 242 | B |
| 243 | A |
| 247 | B |
| 248 | B |
| 261 | B |
| 274 | B |
| 278 | A |
| 279 | A |
| 281 | B |
| 283 | A |
| 287 | B |
| 291 | A |
| 292 | A |
| 293 | A |
| 294 | B |
| 295 | A |
| 296 | B |
| 297 | A |
| 298 | B |
| 299 | A |
| 300 | B |
| 301 | B |

| Controlling effect against cucumber downy mildew (4) | |
|---|---|
| Compound No. | Effect |
| 302 | A |
| 303 | A |
| 304 | A |
| 305 | A |
| 306 | A |
| 307 | A |
| 308 | A |
| 309 | A |
| 310 | A |
| 311 | A |
| 312 | B |
| 313 | A |
| 314 | A |
| 315 | A |
| 316 | A |
| 317 | B |
| 328 | B |
| 329 | B |
| 330 | B |
| 332 | A |
| 335 | A |
| 336 | A |
| 337 | A |
| 338 | A |
| 339 | A |
| 340 | A |
| 341 | A |
| 342 | A |
| 343 | A |
| 344 | B |
| 345 | B |
| 346 | B |
| 347 | B |
| 348 | B |
| 349 | B |
| 350 | B |
| 352 | A |

| Controlling effect against cucumber downy mildew (5) | |
|---|---|
| Compound No. | Effect |
| 388 | B |
| 389 | B |
| 390 | B |
| 392 | B |
| 394 | B |
| 396 | B |
| 397 | B |
| 400 | A |
| 408 | A |
| 412 | A |
| 416 | A |
| 431 | A |
| 432 | A |
| 433 | A |
| 435 | A |
| 436 | A |
| 437 | A |
| 444 | A |
| 447 | A |
| 448 | A |
| 449 | A |
| 450 | A |
| 451 | A |
| 452 | A |
| 453 | A |
| 454 | A |
| 455 | A |
| 456 | A |
| 554 | A |
| 565 | A |
| 567 | B |
| 575 | B |

| Controlling effect against cucumber downy mildew (6) | |
|---|---|
| Compound No. | Effect |
| 595 | B |
| 599 | A |
| 601 | A |
| 639 | B |
| 640 | B |
| 641 | B |
| 643 | A |
| 644 | A |
| 645 | A |
| 646 | B |
| 647 | B |
| 648 | B |
| 649 | A |
| 650 | A |
| 651 | A |
| 652 | A |
| 665 | A |
| 666 | A |
| 667 | A |
| 669 | A |
| 674 | A |
| 675 | B |
| 678 | A |
| 684 | A |
| 685 | A |
| 686 | A |
| 688 | A |
| 689 | A |
| 690 | B |
| 691 | A |
| 693 | A |
| 694 | A |
| 695 | A |
| 696 | B |
| 697 | B |
| 698 | A |
| 699 | A |
| 700 | A |
| 701 | A |
| 702 | A |

| Controlling effect against cucumber downy mildew (7) | |
|---|---|
| Compound No. | Effect |
| 703 | A |
| 704 | A |
| 705 | A |
| 708 | A |
| 709 | A |
| 710 | A |
| 711 | A |
| 712 | A |
| 713 | A |
| 714 | A |
| 715 | A |
| 717 | A |
| 718 | A |
| 719 | A |
| 733 | A |
| 735 | A |
| 737 | A |
| 765 | A |
| 766 | A |
| 767 | A |
| 771 | A |
| 772 | A |
| 773 | A |
| 774 | A |
| 775 | A |
| 776 | A |
| 777 | A |
| 778 | A |
| 780 | A |
| 781 | A |
| 782 | A |
| 783 | A |
| 784 | A |
| 785 | B |
| 786 | A |
| 787 | A |
| 788 | A |
| 789 | A |

| Controlling effect against cucumber downy mildew (8) | |
|---|---|
| Compound No. | Effect |
| 793 | A |
| 794 | A |
| 798 | B |
| 799 | A |
| 800 | B |
| 801 | B |
| 802 | B |
| 803 | B |
| 810 | A |
| 811 | A |
| 812 | A |
| 814 | A |
| 815 | A |
| 816 | A |
| 818 | A |
| 819 | A |
| 820 | A |
| 823 | A |
| 826 | A |
| 827 | B |
| 828 | B |
| 830 | A |
| 831 | A |
| 832 | A |
| 833 | A |
| 834 | A |
| 836 | A |
| 838 | A |
| 839 | A |
| 842 | B |
| 843 | A |
| 844 | A |
| 846 | A |
| 847 | A |
| 848 | A |
| 849 | B |
| 850 | B |
| 853 | B |
| 857 | B |
| 868 | A |
| 869 | A |

| Controlling effect against cucumber downy mildew (9) | |
|---|---|
| Compound No. | Effect |
| 870 | A |
| 872 | A |
| 873 | A |
| 874 | A |
| 875 | A |
| 876 | A |
| 878 | B |
| 882 | B |
| 886 | A |
| 889 | A |
| 890 | A |
| 891 | A |
| 899 | A |
| 902 | A |
| 903 | A |
| 906 | B |
| 907 | B |
| 908 | A |
| 911 | B |
| 912 | B |
| 914 | A |
| 915 | A |
| 916 | A |
| 917 | A |
| 924 | A |
| 929 | A |
| 933 | B |
| 934 | B |
| 937 | A |
| 938 | B |
| 939 | B |
| 940 | B |
| 941 | A |
| 942 | A |
| 943 | B |
| 948 | B |
| 949 | A |
| 951 | A |
| 952 | A |
| 953 | A |
| 954 | A |

| Controlling effect against cucumber downy mildew (10) | |
|---|---|
| Compound No. | Effect |
| 955 | A |
| 960 | A |
| 968 | B |
| 969 | B |
| 970 | B |
| 971 | A |
| 972 | A |
| 973 | A |
| 981 | A |
| 982 | A |
| 983 | A |
| 984 | A |
| 985 | A |
| 986 | A |
| 987 | A |
| 988 | A |
| 989 | B |
| 990 | A |
| 991 | B |
| 993 | A |
| 994 | A |
| 995 | A |
| 998 | A |
| 999 | A |
| 1000 | B |
| 1001 | B |
| 1002 | B |
| 1004 | B |
| 1007 | B |
| 1010 | B |
| 1011 | B |
| 1015 | B |
| 1017 | B |
| 1019 | A |
| 1020 | B |
| 1021 | B |
| 1023 | B |
| 1026 | A |
| 1027 | A |
| 1028 | A |
| 1029 | A |

| Controlling effect against cucumber downy mildew (11) | |
|---|---|
| Compound No. | Effect |
| 1030 | A |
| 1031 | A |
| 1032 | A |
| 1033 | A |
| 1035 | A |
| 1036 | A |
| 1042 | A |
| 1043 | A |
| 1044 | A |
| 1045 | A |
| 1050 | B |
| 1051 | A |
| 1052 | A |
| 1053 | A |
| 1054 | B |
| 1055 | A |
| 1056 | B |
| 1057 | B |
| 1058 | B |
| 1061 | B |
| 1065 | B |
| 1066 | A |
| 1067 | A |
| 1068 | A |
| 1069 | A |
| 1070 | A |
| 1071 | A |
| 1072 | A |
| 1073 | A |
| 1074 | A |
| 1075 | A |
| 1076 | A |
| 1078 | B |
| 1080 | A |
| 1082 | B |
| 1094 | B |
| 1097 | B |
| 1109 | B |
| 1110 | B |
| 1112 | B |

| Controlling effect against cucumber downy mildew (12) | |
|---|---|
| Compound No. | Effect |
| 1113 | B |
| 1114 | B |
| 1115 | A |
| 1116 | A |
| 1124 | B |
| 1125 | A |
| 1126 | B |
| 1148 | A |
| 1152 | A |
| 1156 | A |

### Test Example 3: Controlling effect against grape downy mildew

The developed leaf of grape seedling (breed: Cabernet Sauvignon) grown in 1/2000a pot was cut and prepared a leaf disk having a diameter of 32 mm. A solution of the compound in acetone was diluted with water to 250 ppm, and the leaf disk was immersed for 3 hours. The disk was taken from the agent solution, subjected to air-drying, and then inoculated by spray with a zoosporangia suspension of Plasmopara viticola. The test disk was incubated at 18°C for 12 hours in a light-dark light cycle. On the 10^{th} day after inoculation the infected area rate for the leaf disk was examined. Evaluation was carried out by the following criteria. The infected area rate for the untreated groups in the examination was 60 to 70%.
A: the infected area rate of less than 5%
B: the infected area rate of 5% to less than 50%
C: the infected area rate of not less than 50%

**Table 5**

| Controlling effect against grape downy mildew (1) | |
|---|---|
| Compound No. | Effect |
| Comparative Compound | C |
| 10 | B |
| 38 | B |
| 40 | A |
| 42 | B |
| 50 | A |
| 51 | B |
| 52 | B |
| 54 | A |
| 55 | A |
| 56 | A |
| 69 | B |
| 123 | B |
| 124 | B |
| 125 | B |
| 126 | B |
| 131 | B |
| 138 | B |
| 139 | B |
| 152 | A |
| 153 | A |
| 154 | A |
| 157 | A |
| 159 | A |
| 160 | A |
| 161 | A |
| 162 | A |
| 163 | A |
| 164 | A |
| 166 | B |
| 167 | A |
| 168 | A |
| 169 | A |
| 171 | A |
| 172 | A |
| 173 | A |
| 174 | B |
| 176 | A |
| 177 | A |
| 182 | A |
| 183 | A |

| Controlling effect against grape downy mildew (2) | |
|---|---|
| Compound No. | Effect |
| 185 | A |
| 186 | B |
| 187 | B |
| 188 | B |
| 192 | A |
| 193 | B |
| 194 | B |
| 200 | B |
| 201 | A |
| 202 | A |
| 203 | A |
| 204 | A |
| 205 | B |
| 206 | A |
| 208 | A |
| 213 | B |
| 214 | B |
| 224 | A |
| 225 | B |
| 226 | B |
| 241 | B |
| 243 | A |
| 278 | B |
| 279 | B |
| 283 | A |
| 291 | A |
| 292 | A |
| 293 | A |
| 295 | A |
| 297 | A |
| 299 | B |
| 302 | A |
| 303 | A |
| 304 | A |
| 305 | A |
| 306 | A |
| 307 | A |
| 308 | A |
| 309 | A |
| 310 | A |
| 311 | A |

| Controlling effect against grape downy mildew (3) | |
|---|---|
| Compound No. | Effect |
| 313 | A |
| 314 | A |
| 315 | A |
| 316 | B |
| 335 | A |
| 336 | A |
| 337 | A |
| 338 | A |
| 339 | A |
| 340 | A |
| 341 | A |
| 342 | A |
| 343 | B |
| 352 | A |
| 400 | A |
| 408 | A |
| 412 | B |
| 431 | A |
| 432 | A |
| 435 | A |
| 436 | A |
| 437 | B |
| 444 | A |
| 447 | A |
| 448 | A |
| 449 | A |
| 450 | B |
| 451 | A |
| 452 | A |
| 453 | A |
| 454 | A |
| 456 | A |
| 554 | A |
| 565 | A |

| Controlling effect against grape downy mildew (4) | |
|---|---|
| Compound No. | Effect |
| 599 | A |
| 601 | B |
| 643 | A |
| 644 | A |
| 645 | A |
| 649 | B |
| 650 | B |
| 651 | B |
| 652 | B |
| 665 | A |
| 666 | A |
| 667 | A |
| 669 | A |
| 674 | B |
| 678 | B |
| 684 | A |
| 685 | A |
| 686 | A |
| 688 | A |
| 689 | A |
| 691 | A |
| 693 | A |
| 694 | A |
| 695 | A |
| 698 | B |
| 699 | A |
| 700 | A |
| 701 | A |
| 702 | A |
| 703 | A |
| 704 | A |
| 705 | A |
| 708 | A |
| 709 | B |
| 710 | A |
| 711 . | A |
| 712 | A |
| 713 | A |
| 714 | A |
| 715 | A |

| Controlling effect against grape downy mildew (5) | |
|---|---|
| Compound No. | Effect |
| 717 | A |
| 718 | A |
| 719 | A |
| 733 | A |
| 735 | A |
| 737 | A |
| 765 | A |
| 766 | B |
| 767 | A |
| 771 | A |
| 772 | A |
| 773 | A |
| 774 | A |
| 775 | A |
| 776 | A |
| 777 | A |
| 778 | A |
| 780 | A |
| 781 | B |
| 782 | A |
| 783 | A |
| 784 | A |
| 785 | B |
| 787 | B |
| 788 | A |
| 789 | B |
| 793 | A |
| 794 | B |
| 799 | A |
| 810 | B |
| 811 | B |
| 812 | B |
| 814 | A |
| 815 | A |
| 816 | B |
| 818 | A |
| 819 | A |
| 820 | B |

| Controlling effect against grape downy mildew (6) | |
|---|---|
| Compound No. | Effect |
| 823 | B |
| 826 | A |
| 830 | A |
| 831 | A |
| 832 | A |
| 834 | A |
| 835 | A |
| 836 | A |
| 838 | A |
| 839 | A |
| 843 | B |
| 844 | B |
| 846 | A |
| 847 | A |
| 848 | A |
| 868 | B |
| 869 | A |
| 870 | A |
| 872 | B |
| 873 | A |
| 874 | A |
| 875 | A |
| 876 | A |
| 886 | A |
| 889 | A |
| 890 | A |
| 891 | A |
| 899 | B |
| 902 | A |
| 903 | A |
| 908 | B |
| 914 | A |
| 915 | A |
| 916 | A |
| 917 | B |
| 924 | B |
| 929 | B |
| 937 | B |
| 941 | A |
| 942 | B |
| 949 | B |

| Controlling effect against grape downy mildew (7) | |
|---|---|
| Compound No. | Effect |
| 951 | B |
| 952 | A |
| 953 | A |
| 954 | A |
| 955 | A |
| 960 | B |
| 971 | A |
| 972 | A |
| 973 | A |
| 981 | B |
| 982 | A |
| 983 | A |
| 984 | B |
| 985 | B |
| 986 | A |
| 987 | A |
| 988 | B |
| 990 | A |
| 993 | A |
| 994 | B |
| 995 | B |
| 998 | B |
| 999 | B |
| 1019 | B |
| 1026 | B |
| 1027 | A |
| 1028 | B |
| 1029 | B |
| 1030 | B |
| 1031 | A |
| 1032 | B |
| 1033 | A |
| 1035 | B |
| 1036 | B |
| 1042 | B |
| 1043 | B |
| 1044 | B |
| 1045 | B |
| 1051 | B |
| 1052 | B |
| 1053 | B |

| Controlling effect against grape downy mildew (8) | |
|---|---|
| Compound No. | Effect |
| 1055 | B |
| 1066 | B |
| 1067 | B |
| 1068 | B |
| 1069 | B |
| 1070 | A |
| 1071 | A |
| 1072 | A |
| 1073 | A |
| 1074 | B |
| 1075 | B |
| 1076 | A |
| 1080 | B |
| 1115 | B |
| 1116 | B |

### Test Example 4: Controlling effect against rice blast disease

A solution of the compound in acetone was diluted with water to 250 ppm, and the test compound was sprayed to the rice plant (breed: Tsukimimochi, 50 planted per plastic pot having a diameter of 6 cm) at 4-leaf stage in an amount of 10 mL of the compound/pot. After air-drying, the pot was transferred to a moist chamber at 25°C (12-hour light-dark light cycle), and inoculated by spray with conidium suspension of Pyricularia oryzae. On the 7^{th} to 10^{th} days after inoculation, the number of blotch in all the pots was examined. Evaluation was carried out by the following criteria.

The number of blotch per pot for the untreated groups in the examination was not less than 60.
A: The number of lesions of less than 5
B: The number of lesions of not less than 6 to less than 50
C: The number of lesions of not less than 50

**Table 6**

| Controlling effect against rice blast disease | |
|---|---|
| Compound No. | Effect |
| Comparative Compound | A |
| 47 | A |
| 100 | A |
| 113 | A |
| 114 | A |
| 115 | A |
| 147 | A |
| 148 | A |
| 397 | A |
| 405 | A |
| 752 | A |
| 766 | A |
| 835 | A |
| 881 | A |

### Test Example 5: Inhibiting effect of extension of hyphae against Pythium aphanidermatum

Potato Dextrose Agar (hereinafter referred to as PDA) was heated to 40°C, and then a solution of the compound in acetone was added to 100 ppm. 15 mL of the compound was injected to a Petri dish having a diameter of 9 cm, and cooled to solidify. A mycelial fragment of Pythium aphanidermatum preliminarily grown in the PDA, was perforated with cork borer having 6mm of diameter and transplanted to the agent-containing PDA, while allowing the hyphae ascus plane to be directed downward. After incubation of the dark room at 25°C for 24 hours, the diameter of the extended hyphae was measured and the evaluation was carried out by the following criteria.
A: The inhibiting rate compared with the group untreated with the agent of not less than 95%
B: The inhibiting rate compared with the group untreated with the agent of not less than 50 to less than 95%
C: The inhibiting rate compared the group untreated with the agent of less than 50%

**Table 7**

| Inhibiting effect of extension of hyphae against Pythium aphanidermatum (1) | |
|---|---|
| Compound No. | Effect |
| Comparative Compound | C |
| 6 | B |
| 55 | B |
| 129 | B |
| 159 | B |
| 160 | A |
| 163 | A |
| 164 | B |
| 167 | A |
| 169 | B |
| 171 | B |
| 172 | B |
| 176 | A |
| 182 | A |
| 183 | A |
| 186 | B |
| 188 | B |
| 214 | B |
| 279 | B |
| 292 | A |
| 306 | A |
| 307 | B |
| 309 | B |
| 310 | B |
| 311 | A |
| 312 | A |
| 313 | A |
| 314 | A |
| 315 | A |
| 316 | B |
| 317 | B |
| 348 | B |
| 350 | A |
| 408 | A |
| 416 | A |
| 444 | A |
| 452 | A |
| 565 | B |

| Inhibiting effect of extension of hyphae against Pythium aphanidermatum (2) | |
|---|---|
| Compound No. | Effect |
| 649 | B |
| 650 | A |
| 651 | A |
| 652 | A |
| 674 | A |
| 675 | A |
| 678 | A |
| 684 | A |
| 685 | A |
| 686 | A |
| 688 | A |
| 689 | A |
| 690 | A |
| 691 | A |
| 693 | A |
| 694 | A |
| 698 | A |
| 699 | A |
| 700 | B |
| 701 | B |
| 702 | B |
| 703 | A |
| 704 | A |
| 705 | B |
| 708 | B |
| 709 | B |
| 710 | B |
| 711 | B |
| 712 | B |
| 713 | A |
| 714 | A |
| 715 | A |
| 717 | A |
| 718 | A |
| 719 | A |
| 733 | A |
| 735 | A |

| Inhibiting effect of extension of hyphae against Pythium aphanidermatum (3) | |
|---|---|
| Compound No. | Effect |
| 765 | A |
| 766 | B |
| 767 | A |
| 771 | A |
| 772 | A |
| 775 | A |
| 777 | A |
| 778 | A |
| 781 | A |
| 782 | A |
| 783 | A |
| 784 | A |
| 785 | A |
| 786 | A |
| 787 | A |
| 788 | A |
| 789 | A |
| 790 | A |
| 793 | A |
| 794 | A |
| 799 | B |
| 800 | B |
| 814 | B |
| 818 | B |
| 819 | B |
| 826 | A |
| 827 | A |
| 828 | B |
| 830 | A |
| 831 | A |
| 832 | A |
| 834 | A |
| 835 | A |
| 836 | B |
| 846 | A |
| 847 | A |
| 848 | B |
| 849 | B |
| 872 | B |
| 873 | B |

| Inhibiting effect of extension of hyphae against Pythium aphanidermatum (4) | |
|---|---|
| Compound No. | Effect |
| 874 | B |
| 876 | A |
| 886 | B |
| 889 | B |
| 890 | B |
| 891 | B |
| 902 | B |
| 903 | B |
| 911 | B |
| 912 | B |
| 955 | B |
| 994 | A |
| 995 | B |
| 1011 | B |
| 1027 | B |
| 1029 | B |
| 1067 | A |
| 1068 | A |
| 1069 | A |
| 1071 | A |
| 1072 | A |
| 1073 | A |
| 1074 | A |
| 1075 | A |
| 1076 | B |
| 1077 | B |

## Claims

1. A diamine derivative represented by the formula (1): 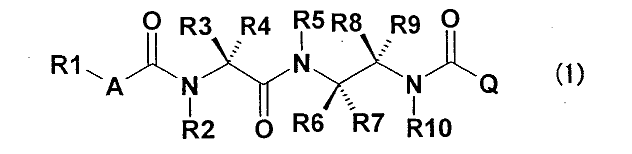 [wherein
R1 is an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, an alkynyl group having 2 to 6 carbon atoms, an aryl group, a heterocycle, an arylalkyl group having 1 to 6 carbon atoms on alkyl moieties, or a heteroarylalkyl group having 1 to 6 carbon atoms on alkyl moieties;
R2 and R5 are each independently a hydrogen atom, an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, an alkynyl group having 2 to 6 carbon atoms, an acyl group, an aryl group, a heterocycle, an arylalkyl group having 1 to 6 carbon atoms on alkyl moieties, or a heteroarylalkyl group having 1 to 6 carbon atoms on alkyl moieties;
R3 and R4 are each independently a hydrogen atom, an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, an alkynyl group having 2 to 6 carbon atoms, an aryl group, a heterocycle, an arylalkyl group having 1 to 6 carbon atoms on alkyl moieties, or a heteroarylalkyl group having 1 to 6 carbon atoms on alkyl moieties, or R3 and R4 may be bonded to each other to form a hydrocarbon ring having 3 to 6 carbon atoms;
R6, R7, R8 and R9 are each independently a hydrogen atom, an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, or an alkynyl group having 2 to 6 carbon atoms, provided that at least one substituent represents an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, or an alkynyl group having 2 to 6 carbon atoms;
R10 is a hydrogen atom, an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, an alkynyl group having 2 to 6 carbon atoms, or an acyl group;
A is oxygen atom or sulfur atom; and
Q is an aryl group or a heterocycle].

2. The diamine derivative according to claim 1, wherein at least one of R3 and R4 is an alkyl group having 1 to 6 carbon atoms, a cycloalkyl group having 3 to 6 carbon atoms, an alkenyl group having 2 to 6 carbon atoms, a cycloalkenyl group having 3 to 6 carbon atoms, an alkynyl group having 2 to 6 carbon atoms, an aryl group, heterocycle, an arylalkyl group having 1 to 6 carbon atoms on alkyl moieties, or a heteroarylalkyl group having 1 to 6 carbon atoms on alkyl moieties, or R3 and R4 may be bonded to each other to form a hydrocarbon ring having 3 to 6 carbon atoms.

3. The diamine derivative according to claim 2, wherein R10 is a hydrogen atom.

4. The diamine derivative according to claim 3, wherein R2 and R5 are hydrogen atoms.

5. A fungicide comprising the diamine derivative according to any one of claims 1 to 4 as an active ingredient.

6. An agricultural and horticultural fungicides comprising the diamine derivative according to any one of claims 1 to 4 as an active ingredient.

7. A process for preparing the diamine derivative according to in any one of claims 1 to 4, comprising reacting a compound represented by the formula (2) 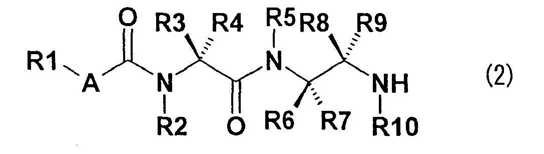 [wherein R1, R2, R3, R4, R5, R6, R7, R8, R9, R10 and A have the same meanings as in claim 1] with a compound represented by the formula (3) 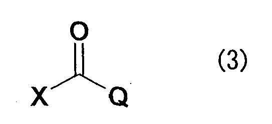 [wherein Q has the same meaning as in claim 1, and X is a leaving group].

8. A process for preparing the diamine derivative as described in any one of claims 1 to 4, comprising reacting the compound represented by the formula (2) with a compound represented by the formula (4) 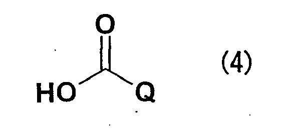 [wherein Q has the same meaning as in claim 1].

9. A process for preparing the diamine derivative as described in any one of claims 1 to 4, comprising reacting a compound represented by the formula (5) 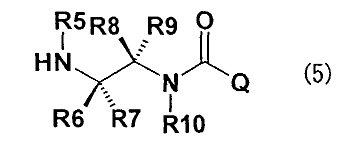 [wherein R5, R6, R7, R8, R9, R10 and Q have the same meanings as in claim 1] with a compound represented by the formula (6) 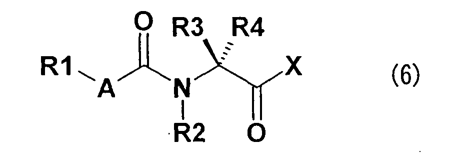 [wherein R1, R2, R3, R4 and A have the same meanings as in claim 1, and X is a leaving group].

10. A process for preparing the diamine derivative as described in any one of claims 1 to 4, comprising reacting the compound represented by the formula (5) with a compound represented by the formula (7) 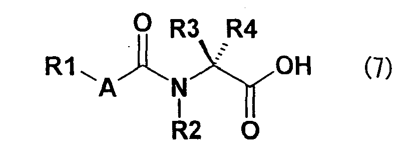 wherein R1, R2, R3, R4 and A have the same meanings as in claim 1.

11. A process for preparing the diamine derivative as described in any one of claims 1 to 4, comprising reacting a compound of formula (8) 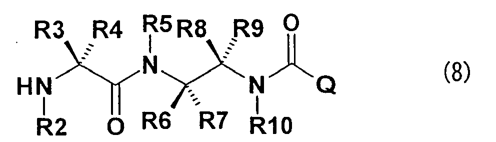 [wherein R2, R3, R4, R5, R6, R7, R8, R9, R10 and Q have the same meanings as in claim 1] with a compound represented by the formula (9) 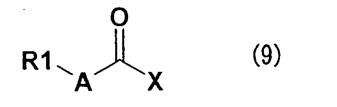 [wherein R1 and A have the same meanings as in claim 1 and X is a leaving group].
